(19) Europäisches Patentamt / European Patent Office / Office européen des brevets

(11) EP 4 458 795 A1

(12) **EUROPEAN PATENT APPLICATION**
published in accordance with Art. 153(4) EPC

(43) Date of publication:
**06.11.2024 Bulletin 2024/45**

(21) Application number: **22916068.4**

(22) Date of filing: **26.12.2022**

(51) International Patent Classification (IPC):
***C07C 15/38*** (2006.01) ***C09K 11/06*** (2006.01)
***C07D 307/77*** (2006.01) ***H05B 33/12*** (2006.01)
***H10K 50/00*** (2023.01) ***H10K 50/16*** (2023.01)
***H10K 50/15*** (2023.01)

(52) Cooperative Patent Classification (CPC):
**C07D 307/77; C09K 11/06; H05B 33/12; H10K 50/00; H10K 50/15; H10K 50/16;** C07C 15/38

(86) International application number:
**PCT/JP2022/048063**

(87) International publication number:
**WO 2023/127845 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2021 JP 2021212989**
**05.08.2022 JP 2022125649**

(71) Applicant: **Idemitsu Kosan Co.,Ltd.**
**Tokyo 100-8321 (JP)**

(72) Inventors:
- **KUDO Yu**
  **Tokyo 100-8321 (JP)**
- **SHIRASAKI Yoshinao**
  **Tokyo 100-8321 (JP)**
- **MITANI Masato**
  **Tokyo 100-8321 (JP)**
- **HASHIMOTO Sigma**
  **Tokyo 100-8321 (JP)**
- **YOSHIDA Kei**
  **Tokyo 100-8321 (JP)**
- **BAN Shintaro**
  **Tokyo 100-8321 (JP)**
- **TERADA Kazuki**
  **Tokyo 100-8321 (JP)**
- **ITOI Hiroaki**
  **Tokyo 100-8321 (JP)**

(74) Representative: **Gille Hrabal**
**Partnerschaftsgesellschaft mbB**
**Patentanwälte**
**Brucknerstraße 20**
**40593 Düsseldorf (DE)**

(54) **COMPOUND, ORGANIC ELECTROLUMINESCENT ELEMENT, AND ELECTRONIC DEVICE**

(57) A compound represented by a formula (1C-A) below.

EP 4 458 795 A1

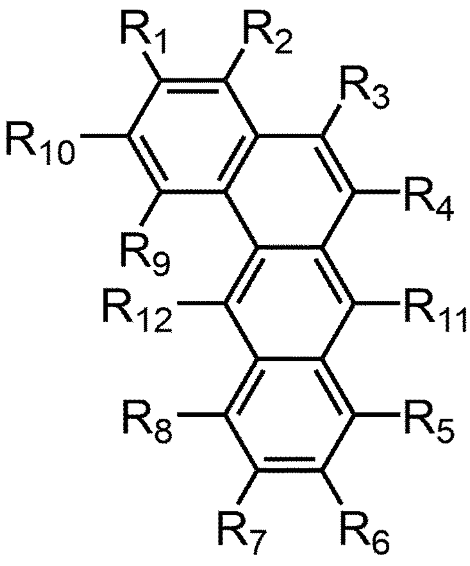

(1C-A)

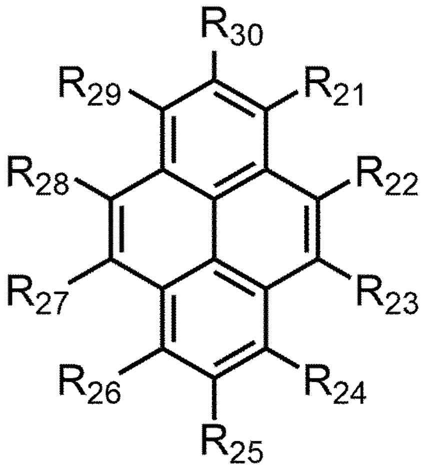

(1D-A)

In the formula (1C-A): one of $R_4$ to $R_8$ and $R_{10}$ to $R_{12}$ is a group represented by a formula (1D-A) above; $R_1$ to $R_3$, $R_9$, and $R_4$ to $R_8$ and $R_{10}$ to $R_{12}$ not being the group represented by the formula (1D-A) are each a hydrogen atom, a substituent, or the like; at least one of $R_{21}$ to $R_{30}$ is other than a hydrogen atom; when $R_{11}$ is a group represented by a formula (1D), $R_{12}$ is a hydrogen atom or a phenyl group; when $R_{12}$ is a group represented by the formula (1D), $R_{11}$ is a hydrogen atom or a phenyl group; in the formula (1D), one of $R_{21}$ to $R_{30}$ represents a bonding position to a benz[a]anthracene ring in the formula (1C), and $R_{21}$ to $R_{30}$ in the formula (1D) not being the bonding position are each a hydrogen atom, a substituent, or the like.

## Description

TECHNICAL FIELD

The present invention relates to a compound, an organic electroluminescence device, and an electronic device.

BACKGROUND ART

An organic electroluminescence device (hereinafter, occasionally referred to as "organic EL device") has found its application in a full-color display for mobile phones, televisions, and the like. When voltage is applied to an organic EL device, holes are injected from an anode and electrons are injected from a cathode into an emitting layer. The injected holes and electrons are recombined in the emitting layer to form excitons. Specifically, according to the electron spin statistics theory, singlet excitons and triplet excitons are generated at a ratio of 25%:75%.

Various studies have been made on a compound to be used for an organic EL device in order to enhance the performance of the organic EL device (see, for instance, Patent Literatures 1 and 2). The performance of the organic EL device is evaluable in terms of, for instance, luminance, emission wavelength, chromaticity, luminous efficiency, drive voltage, and lifetime.

CITATION LIST

PATENT LITERATURE(S)

Patent Literature 1 International Publication No. WO 2021/132535
Patent Literature 2 International Publication No. WO 2009/081774

SUMMARY OF THE INVENTION

PROBLEM(S) TO BE SOLVED BY THE INVENTION

An object of the invention is to provide a compound capable of extending a lifetime of an organic electroluminescence device, an organic electroluminescence device containing the compound, and an electronic device including the organic electroluminescence device.

Another object of the invention is to provide an organic electroluminescence device with an improved lifetime and an electronic device including the organic electroluminescence device.

MEANS FOR SOLVING THE PROBLEM(S)

According to an aspect of the invention, there is provided a compound represented by a formula (1C-A) below.

[Formula 1]

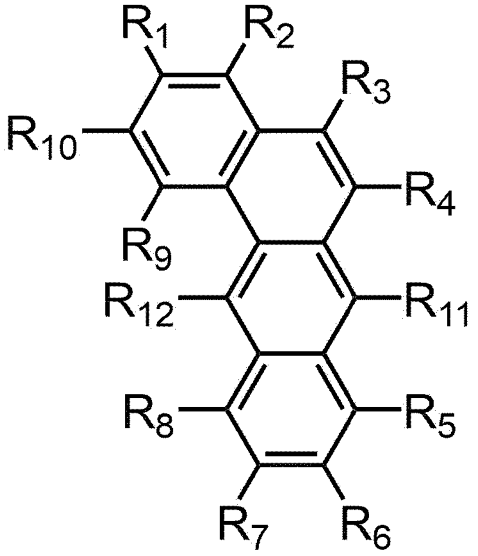

(1C-A)

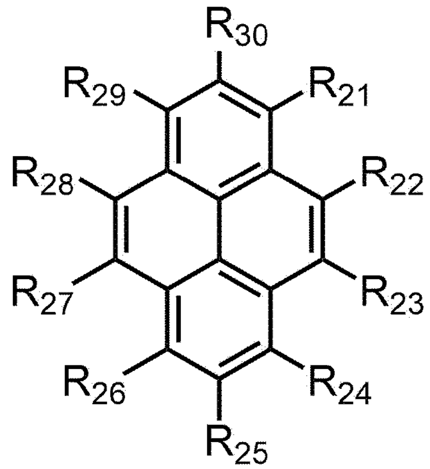

(1D-A)

In the formula (1C-A):

one of $R_4$ to $R_8$ and $R_{10}$ to $R_{12}$ is a group represented by a formula (1D-A) above;
$R_1$ to $R_3$, $R_9$, and $R_4$ to $R_8$ and $R_{10}$ to $R_{12}$ not being the group represented by the formula (1D-A) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a substituted or unsubstituted aralkyl group having 7 to 50 carbon atoms, a group represented by - C(=O)$R_{801}$, a group represented by -COO$R_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;
when $R_{11}$ is a group represented by the formula (1D-A), $R_{12}$ is a hydrogen atom or a substituted or unsubstituted phenyl group;
when $R_{12}$ is a group represented by the formula (1D-A), $R_{11}$ is a hydrogen atom or a substituted or unsubstituted phenyl group;
in the formula (1D-A), one of $R_{21}$ to $R_{30}$ represents a bonding position to a benz[a]anthracene ring in the formula (1C-A), and at least one combination of adjacent two or more of $R_{21}$ to $R_{30}$ not being the bonding position in the formula (1D-A) are mutually bonded to form a substituted or unsubstituted monocyclic ring, mutually bonded to form a substituted or unsubstituted fused ring, or not mutually bonded;
$R_{21}$ to $R_{30}$ not being the bonding position to the benz[a]anthracene ring in the formula (1C-A) and forming neither the substituted or unsubstituted monocyclic ring nor the substituted or unsubstituted fused ring in the formula (1D-A) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and
at least one of $R_{21}$ to $R_{30}$ is other than a hydrogen atom;
in the compound represented by the formula (1C-A), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

According to another aspect of the invention, there is provided a compound represented by a formula (1C-B) below.

[Formula 2]

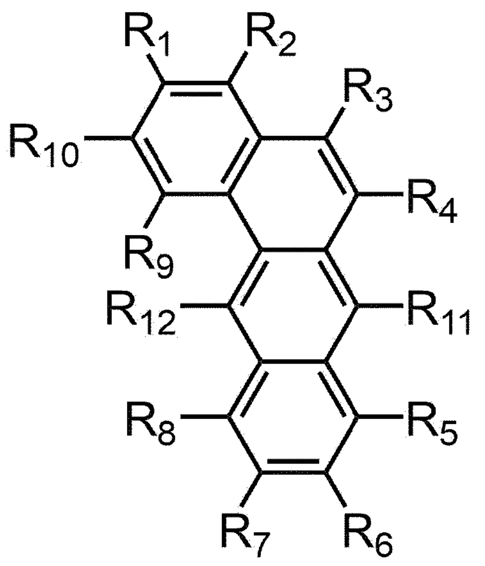

(1C-B)

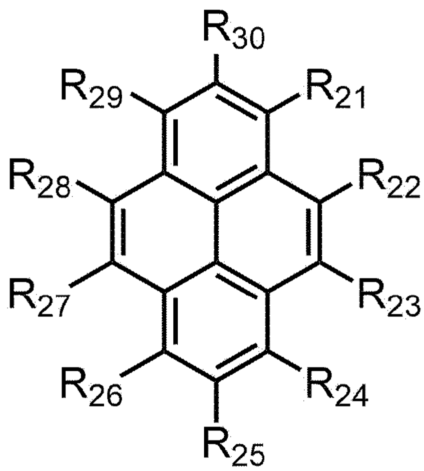

(1D-B)

In the formula (1C-B):

one of $R_4$ to $R_6$, $R_8$, and $R_{10}$ to $R_{12}$ is a group represented by a formula (1D-B) above;
$R_1$ to $R_3$, $R_7$, $R_9$, and $R_4$ to $R_6$, $R_8$, and $R_{10}$ to $R_{12}$ not being the group represented by the formula (1D-B) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $- S-(R_{905})$, a substituted or unsubstituted aralkyl group having 7 to 50 carbon atoms, a group represented by $-C(=O)R_{801}$, a group represented by $-COOR_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;
when $R_{11}$ is a group represented by the formula (1D-B), $R_{12}$ is a hydrogen atom or an unsubstituted phenyl group; and when $R_{12}$ is a group represented by the formula (1D-B), $R_{11}$ is a substituted or unsubstituted phenyl group;
in the formula (1D-B), one of $R_{21}$ to $R_{30}$ represents a bonding position to a benz[a]anthracene ring in the formula (1C-B), and at least one combination of adjacent two or more of $R_{21}$ to $R_{30}$ not being the bonding position in the formula (1D-B) are mutually bonded to form a substituted or unsubstituted monocyclic ring, mutually bonded to form a substituted or unsubstituted fused ring, or not mutually bonded;
$R_{21}$ to $R_{30}$ not being the bonding position to the benz[a]anthracene ring in the formula (1C-B) and forming neither the substituted or unsubstituted monocyclic ring nor the substituted or unsubstituted fused ring in the formula (1D-B) are each independently a hydrogen atom, a substituted or unsubstituted alkyl group having 1 to 50 carbon atoms,

a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and

in the compound represented by the formula (1C-B), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

According to still another aspect of the invention, there is provided a compound represented by a formula (1C-C) below.

[Formula 3]

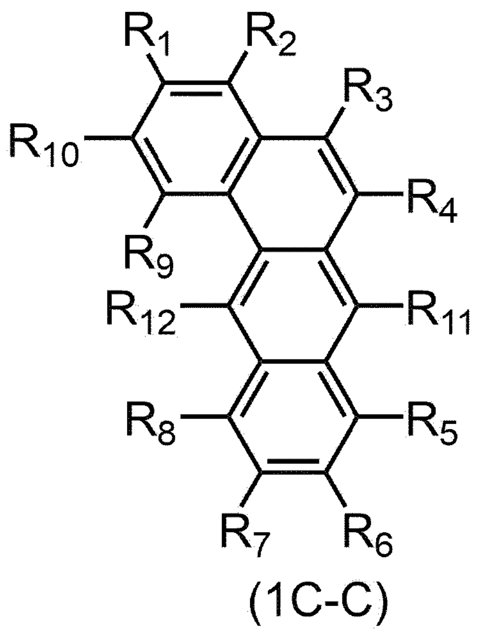

(1C-C)

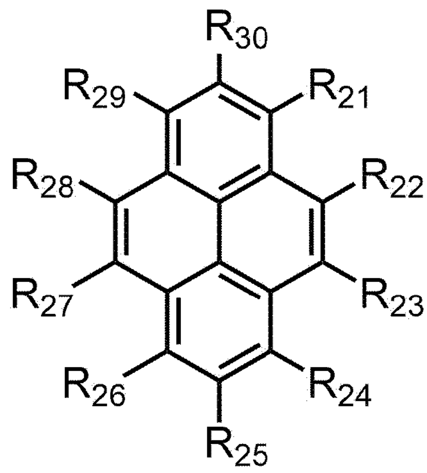

(1D-C)

In the formula (1C-C):

one of $R_4$ to $R_8$ and $R_{10}$ to $R_{12}$ is a group represented by a formula (1D-C) above;

$R_1$ to $R_3$, $R_9$, and $R_4$ to $R_8$ and $R_{10}$ to $R_{12}$ not being the group represented by the formula (1D-C) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a substituted or unsubstituted aralkyl group having 7 to 50 carbon atoms, a group represented by $-C(=O)R_{801}$, a group represented by $-COOR_{802}$, a halogen atom, a cyano group, a nitro group, a substituted or unsubstituted aryl group having 6 to 50 ring carbon atoms, or

a substituted or unsubstituted heterocyclic group having 5 to 50 ring atoms;
when $R_{11}$ is a group represented by the formula (1D-C), $R_{12}$ is a hydrogen atom or a substituted or unsubstituted phenyl group;
when $R_{12}$ is a group represented by the formula (1D-C), $R_{11}$ is a hydrogen atom or a substituted or unsubstituted phenyl group;
in the formula (1D-C), one of $R_{22}$, $R_{23}$, $R_{25}$, $R_{27}$, $R_{28}$, and $R_{30}$ represents a bonding position to a benz[a]anthracene ring in the formula (1C-C), and at least one combination of adjacent two or more of $R_{22}$, $R_{23}$, $R_{25}$, $R_{27}$, $R_{28}$, and $R_{30}$ not being the bonding position, $R_{21}$, $R_{24}$, $R_{26}$, and $R_{29}$ in the formula (1D-C) are mutually bonded to form a substituted or unsubstituted monocyclic ring, mutually bonded to form a substituted or unsubstituted fused ring, or not mutually bonded;
$R_{21}$ to $R_{30}$ not being the bonding position to the benz[a]anthracene ring in the formula (1C-C) and forming neither the substituted or unsubstituted monocyclic ring nor the substituted or unsubstituted fused ring in the formula (1D-C) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and
in the compound represented by the formula (1C-C), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

According to a further aspect of the invention, there is provided an organic electroluminescence device containing the compound according to the above aspect of the invention.

According to the above aspect of the invention, there is provided an organic electroluminescence device including an anode, a cathode, and an organic layer disposed between the anode and the cathode, in which at least one layer of the organic layer contains the compound according to the above aspect of the invention.

According to a still further aspect of the invention, there is provided an organic electroluminescence device including an anode, a cathode, and an organic layer disposed between the anode and the cathode, in which the organic layer includes an emitting region, the emitting region includes a first emitting layer and a second emitting layer, the first emitting layer contains a first compound represented by a formula (1C) below, and the second emitting layer contains a second compound.

[Formula 4]

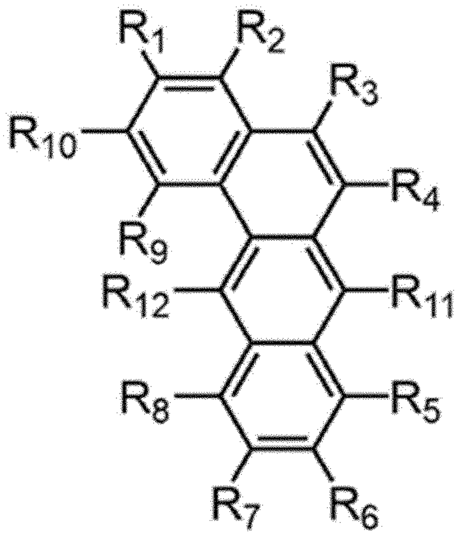

(1C)

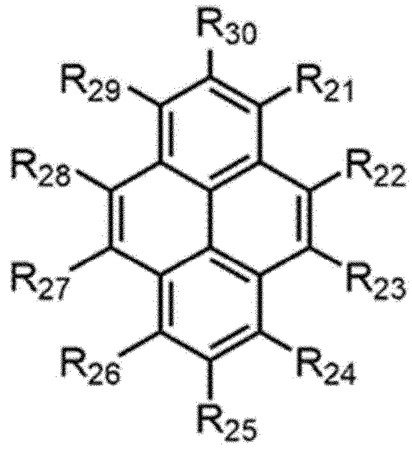

(1D)

In the formula (1C):

one of $R_4$ to $R_8$ and $R_{10}$ to $R_{12}$ is a group represented by a formula (1D) above;

$R_1$ to $R_3$, $R_9$, and $R_4$ to $R_8$ and $R_{10}$ to $R_{12}$ not being the group represented by the formula (1D) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a substituted or unsubstituted aralkyl group having 7 to 50 carbon atoms, a group represented by - $C(=O)R_{801}$, a group represented by $-COOR_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;

when $R_{11}$ is a group represented by the formula (1D), $R_{12}$ is a hydrogen atom or a substituted or unsubstituted phenyl group;

when $R_{12}$ is a group represented by the formula (1D), $R_{11}$ is a hydrogen atom or a substituted or unsubstituted phenyl group;

in the formula (1D), one of $R_{21}$ to $R_{30}$ represents a bonding position to a benz[a]anthracene ring in the formula (1C), and at least one combination of adjacent two or more of $R_{21}$ to $R_{30}$ not being the bonding position in the formula (1D) are mutually bonded to form a substituted or unsubstituted monocyclic ring, mutually bonded to form a substituted or unsubstituted fused ring, or not mutually bonded;

$R_{21}$ to $R_{30}$ not being the bonding position to the benz[a]anthracene ring in the formula (1C) and forming neither the substituted or unsubstituted monocyclic ring nor the substituted or unsubstituted fused ring in the formula (1D) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and

in the compound represented by the formula (1C), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;

when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

According to a still further aspect of the invention, there is provided an electronic device including the organic electroluminescence device according to the above aspect of the invention.

According to the above aspects of the invention, there are provided a compound capable of extending a lifetime of an organic electroluminescence device, an organic electroluminescence device containing the compound, and an electronic device including the organic electroluminescence device.

According to the above aspects of the invention, there are provided an organic electroluminescence device with an improved lifetime and an electronic device including the organic electroluminescence device.

BRIEF EXPLANATION OF DRAWINGS

Fig. 1 schematically depicts an exemplary arrangement of an organic electroluminescence device according to an exemplary embodiment of the invention.

Fig. 2 schematically depicts another exemplary arrangement of an organic electroluminescence device according to an exemplary embodiment of the invention.

DESCRIPTION OF EMBODIMENT(S)

Definitions

Herein, a hydrogen atom includes isotope having different numbers of neutrons, specifically, protium, deuterium and tritium.

In chemical formulae herein, it is assumed that a hydrogen atom (i.e. protium, deuterium and tritium) is bonded to each of bondable positions that are not annexed with signs "R" or the like or "D" representing a deuterium.

Herein, the ring carbon atoms refer to the number of carbon atoms among atoms forming a ring of a compound (e.g., a monocyclic compound, fused-ring compound, cross-linking compound, carbon ring compound, and heterocyclic compound) in which the atoms are bonded to each other to form the ring.

When the ring is substituted by a substituent(s), carbon atom(s) contained in the substituent(s) is not counted in the ring carbon atoms. Unless otherwise specified, the same applies to the "ring carbon atoms" described later. For instance, a benzene ring has 6 ring carbon atoms, a naphthalene ring has 10 ring carbon atoms, a pyridine ring has 5 ring carbon atoms, and a furan ring has 4 ring carbon atoms. Further, for instance, 9,9-diphenylfluorenyl group has 13 ring carbon atoms and 9,9'-spirobifluorenyl group has 25 ring carbon atoms.

When a benzene ring is substituted by a substituent in a form of, for instance, an alkyl group, the number of carbon atoms of the alkyl group is not counted in the number of the ring carbon atoms of the benzene ring. Accordingly, the benzene ring substituted by an alkyl group has 6 ring carbon atoms. When a naphthalene ring is substituted by a substituent in a form of, for instance, an alkyl group, the number of carbon atoms of the alkyl group is not counted in the number of the ring carbon atoms of the naphthalene ring. Accordingly, the naphthalene ring substituted by an alkyl group has 10 ring carbon atoms.

Herein, the ring atoms refer to the number of atoms forming a ring of a compound (e.g., a monocyclic compound, fused-ring compound, cross-linking compound, carbon ring compound, and heterocyclic compound) in which the atoms are bonded to each other to form the ring (e.g., monocyclic ring, fused ring, and ring assembly). Atom(s) not forming the ring (e.g., hydrogen atom(s) for saturating the valence of the atom which forms the ring) and atom(s) in a substituent by which the ring is substituted are not counted as the ring atoms. Unless otherwise specified, the same applies to the "ring atoms" described later. For instance, a pyridine ring has 6 ring atoms, a quinazoline ring has 10 ring atoms, and a furan ring has 5 ring atoms. For instance, the number of hydrogen atom(s) bonded to a pyridine ring or the number of atoms forming a substituent is not counted as the pyridine ring atoms. Accordingly, a pyridine ring bonded to a hydrogen atom(s) or a substituent(s) has 6 ring atoms. For instance, the hydrogen atom(s) bonded to carbon atom(s) of a quinazoline ring or the atoms forming a substituent are not counted as the quinazoline ring atoms. Accordingly, a quinazoline ring bonded

to hydrogen atom(s) or a substituent(s) has 10 ring atoms.

Herein, "XX to YY carbon atoms" in the description of "substituted or unsubstituted ZZ group having XX to YY carbon atoms" represent carbon atoms of an unsubstituted ZZ group and do not include carbon atoms of a substituent(s) of the substituted ZZ group. Herein, "YY" is larger than "XX," "XX" representing an integer of 1 or more and "YY" representing an integer of 2 or more.

Herein, "XX to YY atoms" in the description of "substituted or unsubstituted ZZ group having XX to YY atoms" represent atoms of an unsubstituted ZZ group and does not include atoms of a substituent(s) of the substituted ZZ group. Herein, "YY" is larger than "XX," "XX" representing an integer of 1 or more and "YY" representing an integer of 2 or more.

Herein, an unsubstituted ZZ group refers to an "unsubstituted ZZ group" in a "substituted or unsubstituted ZZ group," and a substituted ZZ group refers to a "substituted ZZ group" in a "substituted or unsubstituted ZZ group."

Herein, the term "unsubstituted" used in a "substituted or unsubstituted ZZ group" means that a hydrogen atom(s) in the ZZ group is not substituted with a substituent(s). The hydrogen atom(s) in the "unsubstituted ZZ group" is protium, deuterium, or tritium.

Herein, the term "substituted" used in a "substituted or unsubstituted ZZ group" means that at least one hydrogen atom in the ZZ group is substituted with a substituent. Similarly, the term "substituted" used in a "BB group substituted by AA group" means that at least one hydrogen atom in the BB group is substituted with the AA group.

Substituent Mentioned Herein

Substituent mentioned herein will be described below.

An "unsubstituted aryl group" mentioned herein has, unless otherwise specified herein, 6 to 50, preferably 6 to 30, more preferably 6 to 18 ring carbon atoms.

An "unsubstituted heterocyclic group" mentioned herein has, unless otherwise specified herein, 5 to 50, preferably 5 to 30, more preferably 5 to 18 ring atoms.

An "unsubstituted alkyl group" mentioned herein has, unless otherwise specified herein, 1 to 50, preferably 1 to 20, more preferably 1 to 6 carbon atoms.

An "unsubstituted alkenyl group" mentioned herein has, unless otherwise specified herein, 2 to 50, preferably 2 to 20, more preferably 2 to 6 carbon atoms.

An "unsubstituted alkynyl group" mentioned herein has, unless otherwise specified herein, 2 to 50, preferably 2 to 20, more preferably 2 to 6 carbon atoms.

An "unsubstituted cycloalkyl group" mentioned herein has, unless otherwise specified herein, 3 to 50, preferably 3 to 20, more preferably 3 to 6 ring carbon atoms.

An "unsubstituted arylene group" mentioned herein has, unless otherwise specified herein, 6 to 50, preferably 6 to 30, more preferably 6 to 18 ring carbon atoms.

An "unsubstituted divalent heterocyclic group" mentioned herein has, unless otherwise specified herein, 5 to 50, preferably 5 to 30, more preferably 5 to 18 ring atoms.

An "unsubstituted alkylene group" mentioned herein has, unless otherwise specified herein, 1 to 50, preferably 1 to 20, more preferably 1 to 6 carbon atoms.

Substituted or Unsubstituted Aryl Group

Specific examples (specific example group G1) of the "substituted or unsubstituted aryl group" mentioned herein include unsubstituted aryl groups (specific example group G1A) below and substituted aryl groups (specific example group G1B). (Herein, an unsubstituted aryl group refers to an "unsubstituted aryl group" in a "substituted or unsubstituted aryl group", and a substituted aryl group refers to a "substituted aryl group" in a "substituted or unsubstituted aryl group.") A simply termed "aryl group" herein includes both of an "unsubstituted aryl group" and a "substituted aryl group".

The "substituted aryl group" refers to a group derived by substituting at least one hydrogen atom in an "unsubstituted aryl group" with a substituent. Examples of the "substituted aryl group" include a group derived by substituting at least one hydrogen atom in the "unsubstituted aryl group" in the specific example group G1A below with a substituent, and examples of the substituted aryl group in the specific example group G1B below. It should be noted that the examples of the "unsubstituted aryl group" and the "substituted aryl group" mentioned herein are merely exemplary, and the "substituted aryl group" mentioned herein includes a group derived by further substituting a hydrogen atom bonded to a carbon atom of a skeleton of a "substituted aryl group" in the specific example group G1B below, and a group derived by further substituting a hydrogen atom of a substituent of the "substituted aryl group" in the specific example group G1B below.

Unsubstituted Aryl Group (Specific Example Group G1A): a phenyl group, p-biphenyl group, m-biphenyl group, o-biphenyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-terphenyl-4-yl group, o-terphenyl-3-yl group, o-terphenyl-2-yl group, 1-naphthyl group, 2-naphthyl group, anthryl group, benzanthryl group, phenanthryl group, benzophenanthryl group, phenalenyl group, pyrenyl group, chrysenyl group, benzochrysenyl group, triphenylenyl group, benzotriphenylenyl group, tetracenyl group, pentacenyl group, fluorenyl group, 9,9'-spirobifluorenyl group, benzofluorenyl group, dibenzofluorenyl group, fluoranthenyl group, benzofluoranthenyl group, perylenyl group, and monovalent aryl group derived by removing one hydrogen atom from cyclic structures represented by formulae (TEMP-1) to (TEMP-15) below.

[Formula 5]

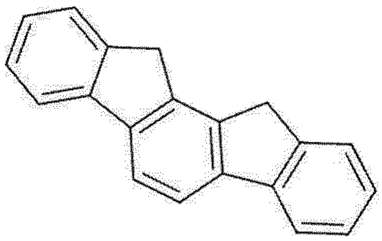

(TEMP-1)

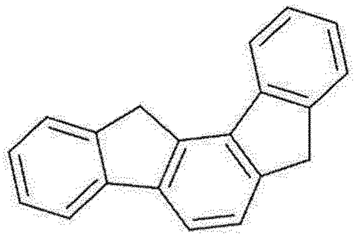

(TEMP-2)

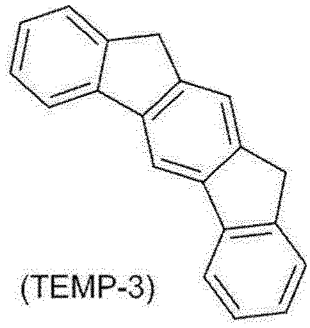

(TEMP-3)

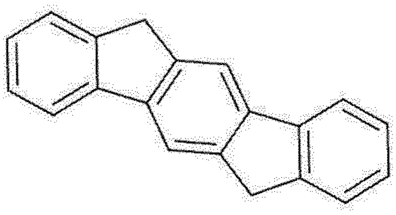

(TEMP-4)

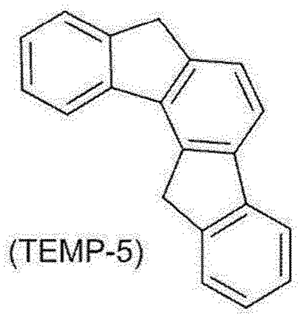

(TEMP-5)

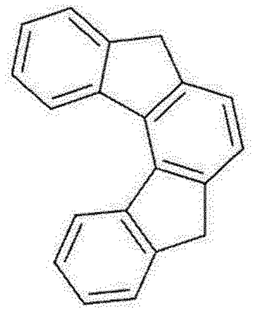

(TEMP-6)

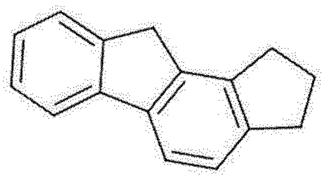

(TEMP-7)

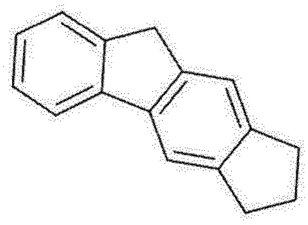

(TEMP-8)

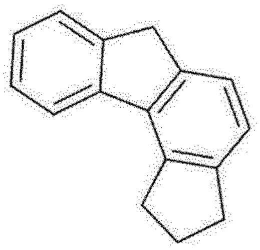

(TEMP-9)

[Formula 6]

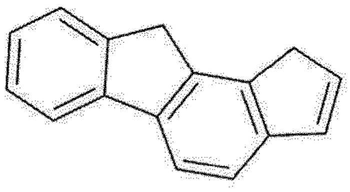

(TEMP-10)

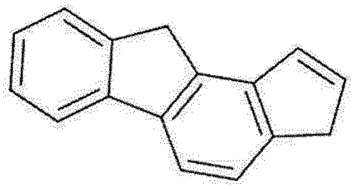

(TEMP-11)

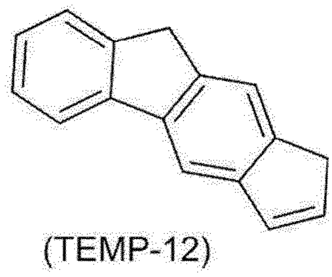

(TEMP-12)

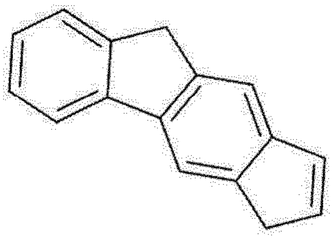

(TEMP-13)

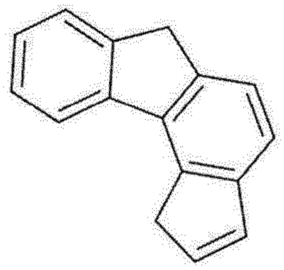

(TEMP-14)

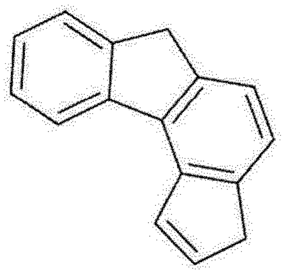

(TEMP-15)

Substituted Aryl Group (Specific Example Group G1B): an o-tolyl group, m-tolyl group, p-tolyl group, para-xylyl group, meta-xylyl group, ortho-xylyl group, paraisopropylphenyl group, meta-isopropylphenyl group, ortho-isopropylphenyl group, para-t-butylphenyl group, meta-t-butylphenyl group, ortho-t-butylphenyl group, 3,4,5-trimethylphenyl group, 9,9-dimethylfluorenyl group, 9,9-diphenylfluorenyl group, 9,9-bis(4-methylphenyl)fluorenyl group, 9,9-bis(4-isopropylphenyl)fluorenyl group, 9,9-bis(4-t-butylphenyl)fluorenyl group, cyanophenyl group, triphenylsilylphenyl group, trimethylsilylphenyl group, phenylnaphthyl group, naphthylphenyl group, and group derived by substituting at least one hydrogen atom of a monovalent group derived from one of the cyclic structures represented by the formulae (TEMP-1) to (TEMP-15) with a substituent.

Substituted or Unsubstituted Heterocyclic Group

The "heterocyclic group" mentioned herein refers to a cyclic group having at least one hetero atom in the ring atoms. Specific examples of the hetero atom include a nitrogen atom, oxygen atom, sulfur atom, silicon atom, phosphorus atom, and boron atom.

The "heterocyclic group" mentioned herein is a monocyclic group or a fused-ring group.

The "heterocyclic group" mentioned herein is an aromatic heterocyclic group or a non-aromatic heterocyclic group.

Specific examples (specific example group G2) of the "substituted or unsubstituted heterocyclic group" mentioned herein include unsubstituted heterocyclic groups (specific example group G2A) and substituted heterocyclic groups (specific example group G2B). (Herein, an unsubstituted heterocyclic group refers to an "unsubstituted heterocyclic group" in a "substituted or unsubstituted heterocyclic group," and a substituted heterocyclic group refers to a "substituted heterocyclic group" in a "substituted or unsubstituted heterocyclic group.") A simply termed "heterocyclic group" herein includes both of an "unsubstituted heterocyclic group" and a "substituted heterocyclic group."

The "substituted heterocyclic group" refers to a group derived by substituting at least one hydrogen atom in an "unsubstituted heterocyclic group" with a substituent. Specific examples of the "substituted heterocyclic group" include a group derived by substituting at least one hydrogen atom in the "unsubstituted heterocyclic group" in the specific example group G2A below with a substituent, and examples of the substituted heterocyclic group in the specific example group G2B below. It should be noted that the examples of the "unsubstituted heterocyclic group" and the "substituted heterocyclic group" mentioned herein are merely exemplary, and the "substituted heterocyclic group" mentioned herein includes a group derived by further substituting a hydrogen atom bonded to a ring atom of a skeleton of a "substituted heterocyclic group" in the specific example group G2B below, and a group derived by further substituting a hydrogen atom of a substituent of the "substituted heterocyclic group" in the specific example group G2B below.

The specific example group G2A includes, for instance, unsubstituted heterocyclic groups including a nitrogen atom (specific example group G2A1) below, unsubstituted heterocyclic groups including an oxygen atom (specific example group G2A2) below, unsubstituted heterocyclic groups including a sulfur atom (specific example group G2A3) below, and monovalent heterocyclic groups (specific example group G2A4) derived by removing a hydrogen atom from cyclic structures represented by formulae (TEMP-16) to (TEMP-33) below.

The specific example group G2B includes, for instance, substituted heterocyclic groups including a nitrogen atom (specific example group G2B1) below, substituted heterocyclic groups including an oxygen atom (specific example group G2B2) below, substituted heterocyclic groups including a sulfur atom (specific example group G2B3) below, and groups derived by substituting at least one hydrogen atom of the monovalent heterocyclic groups (specific example group G2B4) derived from the cyclic structures represented by formulae (TEMP-16) to (TEMP-33) below.

Unsubstituted Heterocyclic Groups Including Nitrogen Atom (Specific Example Group G2A1): a pyrrolyl group, imidazolyl group, pyrazolyl group, triazolyl group, tetrazolyl group, oxazolyl group, isoxazolyl group, oxadiazolyl group, thiazolyl group, isothiazolyl group, thiadiazolyl group, pyridyl group, pyridazynyl group, pyrimidinyl group, pyrazinyl group, triazinyl group, indolyl group, isoindolyl group, indolizinyl group, quinolizinyl group, quinolyl group, isoquinolyl group, cinnolyl group, phthalazinyl group, quinazolinyl group, quinoxalinyl group, benzimidazolyl group, indazolyl group, phenanthrolinyl group, phenanthridinyl group, acridinyl group, phenazinyl group, carbazolyl group, benzocarbazolyl group, morpholino group, phenoxazinyl group, phenothiazinyl group, azacarbazolyl group, and diazacarbazolyl group.

Unsubstituted Heterocyclic Groups Including Oxygen Atom (Specific Example Group G2A2): a furyl group, oxazolyl group, isoxazolyl group, oxadiazolyl group, xanthenyl group, benzofuranyl group, isobenzofuranyl group, dibenzofuranyl group, naphthobenzofuranyl group, benzoxazolyl group, benzisoxazolyl group, phenoxazinyl group, morpholino group, dinaphthofuranyl group, azadibenzofuranyl group, diazadibenzofuranyl group, azanaphthobenzofuranyl group, and diazanaphthobenzofuranyl group.

Unsubstituted Heterocyclic Groups Including Sulfur Atom (Specific Example Group G2A3): a thienyl group, thiazolyl group, isothiazolyl group, thiadiazolyl group, benzothiophenyl group (benzothienyl group), isobenzothiophenyl group (isobenzothienyl group), dibenzothiophenyl group (dibenzothienyl group), naphthobenzothiophenyl group (nahthobenzothienyl group), benzothiazolyl group, benzisothiazolyl group, phenothiazinyl group, dinaphthothiophenyl group (dinaphthothienyl group), azadibenzothiophenyl group (azadibenzothienyl group), diazadibenzothiophenyl group (diazadibenzothienyl group), azanaphthobenzothiophenyl group (azanaphthobenzothienyl group), and diazanaphthobenzothiophenyl group (diazanaphthobenzothienyl group).

Monovalent Heterocyclic Groups Derived by Removing One Hydrogen Atom from Cyclic Structures Represented by Formulae (TEMP-16) to (TEMP-33) (Specific Example Group G2A4):

[Formula 7]

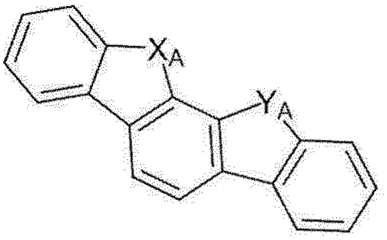

(TEMP-16)

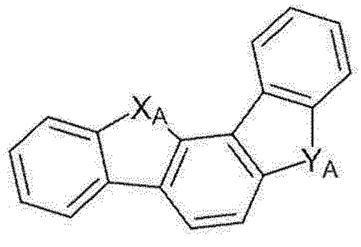

(TEMP-17)

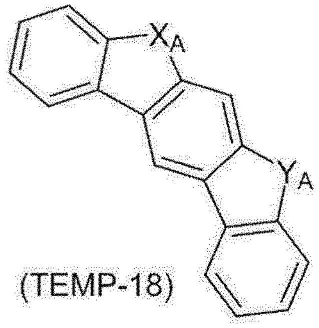

(TEMP-18)

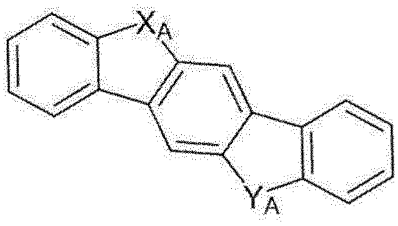

(TEMP-19)

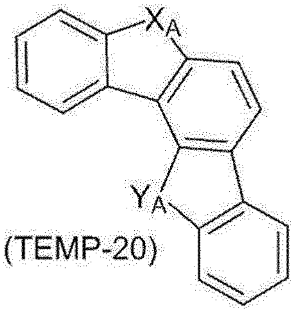

(TEMP-20)

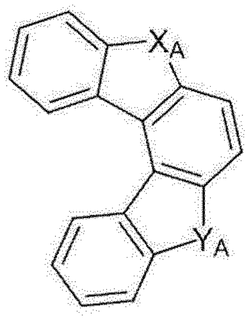

(TEMP-21)

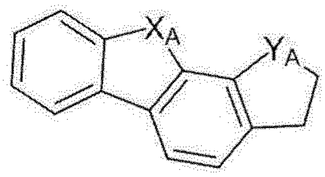

(TEMP-22)

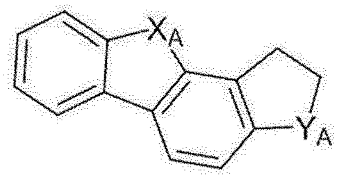

(TEMP-23)

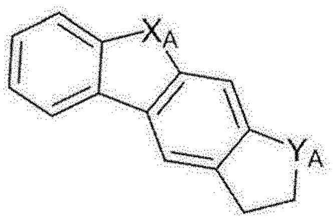

(TEMP-24)

[Formula 8]

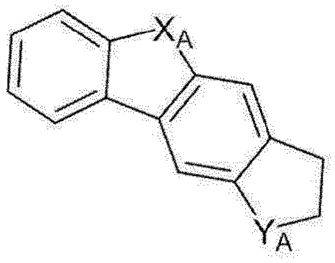

(TEMP-25)

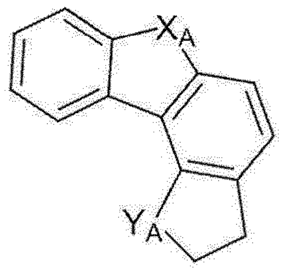

(TEMP-26)

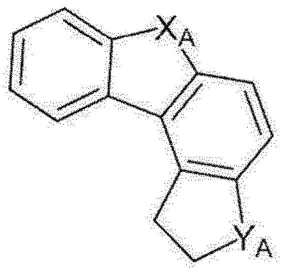

(TEMP-27)

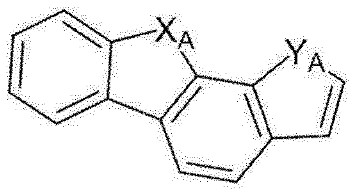

(TEMP-28)

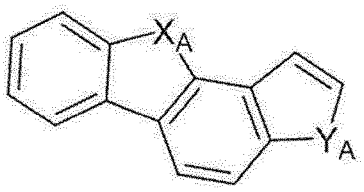

(TEMP-29)

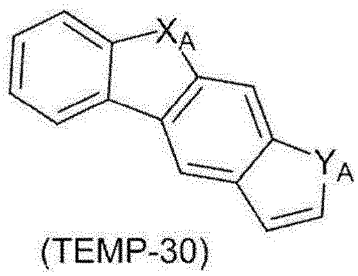

(TEMP-30)

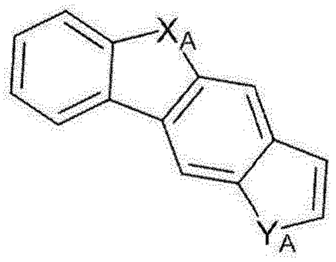

(TEMP-31)

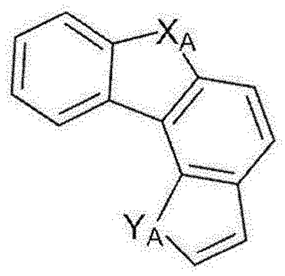

(TEMP-32)

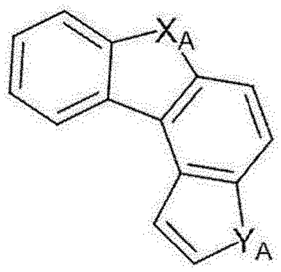

(TEMP-33)

In the formulae (TEMP-16) to (TEMP-33), $X_A$ and $Y_A$ are each independently an oxygen atom, a sulfur atom, NH or $CH_2$, with a proviso that at least one of $X_A$ or $Y_A$ is an oxygen atom, a sulfur atom, or NH.

When at least one of $X_A$ or $Y_A$ in the formulae (TEMP-16) to (TEMP-33) is NH or $CH_2$, the monovalent heterocyclic groups derived from the cyclic structures represented by the formulae (TEMP-16) to (TEMP-33) include a monovalent group derived by removing one hydrogen atom from NH or $CH_2$.

Substituted Heterocyclic Groups Including Nitrogen Atom (Specific Example Group G2B1) a (9-phenyl)carbazolyl group, (9-biphenylyl)carbazolyl group, (9-phenyl)phenylcarbazolyl group, (9-naphthyl)carbazolyl group, diphenylcarbazole-9-yl group, phenylcarbazole-9-yl group, methylbenzimidazolyl group, ethylbenzimidazolyl group, phenyltriazinyl group, biphenylyltriazinyl group, diphenyltriazinyl group, phenylquinazolinyl group, and biphenylquinazolinyl group.

Substituted Heterocyclic Groups Including Oxygen Atom (Specific Example Group G2B2): a phenyldibenzofuranyl group, methyldibenzofuranyl group, t-butyldibenzofuranyl group, and monovalent residue of spiro[9H-xanthene-9,9'-[9H]fluorene].

Substituted Heterocyclic Groups Including Sulfur Atom (Specific Example Group G2B3): a phenyldibenzothiophenyl group, methyldibenzothiophenyl group, t-butyldibenzothiophenyl group, and monovalent residue of spiro[9H-thioxanthene-9,9'-[9H]fluorene].

Groups Obtained by Substituting at Least One Hydrogen Atom of Monovalent Heterocyclic Group Derived from Cyclic Structures Represented by Formulae (TEMP-16) to (TEMP-33) with Substituent (Specific Example Group

G2B4):

The "at least one hydrogen atom of a monovalent heterocyclic group" means at least one hydrogen atom selected from a hydrogen atom bonded to a ring carbon atom of the monovalent heterocyclic group, a hydrogen atom bonded to a nitrogen atom of at least one of $X_A$ or $Y_A$ in a form of NH, and a hydrogen atom of one of $X_A$ and $Y_A$ in a form of a methylene group ($CH_2$).

Substituted or Unsubstituted Alkyl Group

Specific examples (specific example group G3) of the "substituted or unsubstituted alkyl group" mentioned herein include unsubstituted alkyl groups (specific example group G3A) and substituted alkyl groups (specific example group G3B) below. (Herein, an unsubstituted alkyl group refers to an "unsubstituted alkyl group" in a "substituted or unsubstituted alkyl group," and a substituted alkyl group refers to a "substituted alkyl group" in a "substituted or unsubstituted alkyl group.") A simply termed "alkyl group" herein includes both of an "unsubstituted alkyl group" and a "substituted alkyl group".

The "substituted alkyl group" refers to a group derived by substituting at least one hydrogen atom in an "unsubstituted alkyl group" with a substituent. Specific examples of the "substituted alkyl group" include a group derived by substituting at least one hydrogen atom of an "unsubstituted alkyl group" (specific example group G3A) below with a substituent, and examples of the substituted alkyl group (specific example group G3B) below. Herein, the alkyl group for the "unsubstituted alkyl group" refers to a chain alkyl group. Accordingly, the "unsubstituted alkyl group" include linear "unsubstituted alkyl group" and branched "unsubstituted alkyl group." It should be noted that the examples of the "unsubstituted alkyl group" and the "substituted alkyl group" mentioned herein are merely exemplary, and the "substituted alkyl group" mentioned herein includes a group derived by further substituting a hydrogen atom of a skeleton of the "substituted alkyl group" in the specific example group G3B, and a group derived by further substituting a hydrogen atom of a substituent of the "substituted alkyl group" in the specific example group G3B.

Unsubstituted Alkyl Group (Specific Example Group G3A): a methyl group, ethyl group, n-propyl group, isopropyl group, n-butyl group, isobutyl group, s-butyl group, and t-butyl group.

Substituted Alkyl Group (Specific Example Group G3B): a heptafluoropropyl group (including isomer thereof), pentafluoroethyl group, 2,2,2-trifluoroethyl group, and trifluoromethyl group.

Substituted or Unsubstituted Alkenyl Group

Specific examples (specific example group G4) of the "substituted or unsubstituted alkenyl group" mentioned herein include unsubstituted alkenyl groups (specific example group G4A) and substituted alkenyl groups (specific example group G4B). (Herein, an unsubstituted alkenyl group refers to an "unsubstituted alkenyl group" in a "substituted or unsubstituted alkenyl group," and a substituted alkenyl group refers to a "substituted alkenyl group" in a "substituted or unsubstituted alkenyl group.") A simply termed "alkenyl group" herein includes both of an "unsubstituted alkenyl group" and a "substituted alkenyl group".

The "substituted alkenyl group" refers to a group derived by substituting at least one hydrogen atom in an "unsubstituted alkenyl group" with a substituent. Specific examples of the "substituted alkenyl group" include an "unsubstituted alkenyl group" (specific example group G4A) substituted by a substituent, and examples of the substituted alkenyl group (specific example group G4B) below. It should be noted that the examples of the "unsubstituted alkenyl group" and the "substituted alkenyl group" mentioned herein are merely exemplary, and the "substituted alkenyl group" mentioned herein includes a group derived by further substituting a hydrogen atom of a skeleton of the "substituted alkenyl group" in the specific example group G4B with a substituent, and a group derived by further substituting a hydrogen atom of a substituent of the "substituted alkenyl group" in the specific example group G4B with a substituent.

Unsubstituted Alkenyl Group (Specific Example Group G4A): a vinyl group, allyl group, 1-butenyl group, 2-butenyl group, and 3-butenyl group.

Substituted Alkenyl Group (Specific Example Group G4B): a 1,3-butanedienyl group, 1-methylvinyl group, 1-methylallyl group, 1,1-dimethylallyl group, 2-methylallyl group, and 1,2-dimethylallyl group.

Substituted or Unsubstituted Alkynyl Group

Specific examples (specific example group G5) of the "substituted or unsubstituted alkynyl group" mentioned herein include unsubstituted alkynyl groups (specific example group G5A) below. (Herein, an unsubstituted alkynyl group

refers to an "unsubstituted alkynyl group" in a "substituted or unsubstituted alkynyl group.") A simply termed "alkynyl group" herein includes both of "unsubstituted alkynyl group" and "substituted alkynyl group".

The "substituted alkynyl group" refers to a group derived by substituting at least one hydrogen atom in an "unsubstituted alkynyl group" with a substituent. Specific examples of the "substituted alkynyl group" include a group derived by substituting at least one hydrogen atom of the "unsubstituted alkynyl group" (specific example group G5A) below with a substituent.

Unsubstituted Alkynyl Group (Specific Example Group G5A): an ethynyl group.

Substituted or Unsubstituted Cycloalkyl Group

Specific examples (specific example group G6) of the "substituted or unsubstituted cycloalkyl group" mentioned herein include unsubstituted cycloalkyl groups (specific example group G6A) and substituted cycloalkyl groups (specific example group G6B). (Herein, an unsubstituted cycloalkyl group refers to an "unsubstituted cycloalkyl group" in a "substituted or unsubstituted cycloalkyl group," and a substituted cycloalkyl group refers to a "substituted cycloalkyl group" in a "substituted or unsubstituted cycloalkyl group.") A simply termed "cycloalkyl group" herein includes both of "unsubstituted cycloalkyl group" and "substituted cycloalkyl group".

The "substituted cycloalkyl group" refers to a group derived by substituting at least one hydrogen atom of an "unsubstituted cycloalkyl group" with a substituent. Specific examples of the "substituted cycloalkyl group" include a group derived by substituting at least one hydrogen atom of the "unsubstituted cycloalkyl group" (specific example group G6A) below with a substituent, and examples of the substituted cycloalkyl group (specific example group G6B) below. It should be noted that the examples of the "unsubstituted cycloalkyl group" and the "substituted cycloalkyl group" mentioned herein are merely exemplary, and the "substituted cycloalkyl group" mentioned herein includes a group derived by substituting at least one hydrogen atom bonded to a carbon atom of a skeleton of the "substituted cycloalkyl group" in the specific example group G6B with a substituent, and a group derived by further substituting a hydrogen atom of a substituent of the "substituted cycloalkyl group" in the specific example group G6B with a substituent.

Unsubstituted Cycloalkyl Group (Specific Example Group G6A): a cyclopropyl group, cyclobutyl group, cyclopentyl group, cyclohexyl group, 1-adamantyl group, 2-adamantyl group, 1-norbornyl group, and 2-norbornyl group.
Substituted Cycloalkyl Group (Specific Example Group G6B): a 4-methylcyclohexyl group.

Group Represented by -Si($R_{901}$)($R_{902}$)($R_{903}$)

Specific examples (specific example group G7) of the group represented herein by -Si($R_{901}$)($R_{902}$)($R_{903}$) include: -Si(G1)(G1)(G1); -Si(G1)(G2)(G2); - Si(G1)(G1)(G2); -Si(G2)(G2)(G2); -Si(G3)(G3)(G3); and -Si(G6)(G6)(G6);
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3;
G6 represents a "substituted or unsubstituted cycloalkyl group" in the specific example group G6;
a plurality of G1 in -Si(G1)(G1)(G1) are mutually the same or different;
a plurality of G2 in -Si(G1)(G2)(G2) are mutually the same or different;
a plurality of G1 in -Si(G1)(G1)(G2) are mutually the same or different;
a plurality of G2 in -Si(G2)(G2)(G2) are mutually the same or different;
a plurality of G3 in -Si(G3)(G3)(G3) are mutually the same or different; and
a plurality of G6 in -Si(G6)(G6)(G6) are mutually the same or different.

Group Represented by -O-($R_{904}$)

Specific examples (specific example group G8) of a group represented by - O-($R_{904}$) herein include: -O(G1); -O(G2); -O(G3); and -O(G6);
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

Group Represented by -S-($R_{905}$)

Specific examples (specific example group G9) of a group represented herein by -S-($R_{905}$) include: -S(G1); -S(G2); -S(G3); and -S(G6);
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

Group Represented by -N($R_{906}$)($R_{907}$)

Specific examples (specific example group G10) of a group represented herein by -N($R_{906}$)($R_{907}$) include: -N(G1)(G1); -N(G2)(G2); -N(G1)(G2); -N(G3)(G3); and -N(G6)(G6);
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3;
G6 represents a "substituted or unsubstituted cycloalkyl group" in the specific example group G6;
a plurality of G1 in -N(G1)(G1) are mutually the same or different;
a plurality of G2 in -N(G2)(G2) are mutually the same or different;
a plurality of G3 in -N(G3)(G3) are mutually the same or different; and
a plurality of G6 in -N(G6)(G6) are mutually the same or different.

Halogen Atom

Specific examples (specific example group G11) of "halogen atom" mentioned herein include a fluorine atom, chlorine atom, bromine atom, and iodine atom.

Substituted or Unsubstituted Fluoroalkyl Group

The "substituted or unsubstituted fluoroalkyl group" mentioned herein refers to a group derived by substituting at least one hydrogen atom bonded to at least one of carbon atoms forming an alkyl group in the "substituted or unsubstituted alkyl group" with a fluorine atom, and also includes a group (perfluoro group) derived by substituting all of hydrogen atoms bonded to carbon atoms forming the alkyl group in the "substituted or unsubstituted alkyl group" with fluorine atoms. An "unsubstituted fluoroalkyl group" has, unless otherwise specified herein, 1 to 50, preferably 1 to 30, more preferably 1 to 18 carbon atoms. The "substituted fluoroalkyl group" refers to a group derived by substituting at least one hydrogen atom in a "fluoroalkyl group" with a substituent. It should be noted that the examples of the "substituted fluoroalkyl group" mentioned herein include a group derived by further substituting at least one hydrogen atom bonded to a carbon atom of an alkyl chain of a "substituted fluoroalkyl group" with a substituent, and a group derived by further substituting at least one hydrogen atom of a substituent of the "substituted fluoroalkyl group" with a substituent. Specific examples of the "unsubstituted fluoroalkyl group" include a group derived by substituting at least one hydrogen atom of the "alkyl group" (specific example group G3) with a fluorine atom.

Substituted or Unsubstituted Haloalkyl Group

The "substituted or unsubstituted haloalkyl group" mentioned herein refers to a group derived by substituting at least one hydrogen atom bonded to carbon atoms forming the alkyl group in the "substituted or unsubstituted alkyl group" with a halogen atom, and also includes a group derived by substituting all hydrogen atoms bonded to carbon atoms forming the alkyl group in the "substituted or unsubstituted alkyl group" with halogen atoms. An "unsubstituted haloalkyl group" has, unless otherwise specified herein, 1 to 50, preferably 1 to 30, and more preferably 1 to 18 carbon atoms. The "substituted haloalkyl group" refers to a group derived by substituting at least one hydrogen atom in a "haloalkyl group" with a substituent. It should be noted that the examples of the "substituted haloalkyl group" mentioned herein include a group derived by further substituting at least one hydrogen atom bonded to a carbon atom of an alkyl chain of a "substituted haloalkyl group" with a substituent, and a group derived by further substituting at least one hydrogen atom of a substituent of the "substituted haloalkyl group" with a substituent. Specific examples of the "unsubstituted haloalkyl group" include a group derived by substituting at least one hydrogen atom of the "alkyl group" (specific example group G3) with a halogen atom. The haloalkyl group is occasionally referred to as a halogenated alkyl group.

Substituted or Unsubstituted Alkoxy Group

Specific examples of a "substituted or unsubstituted alkoxy group" mentioned herein include a group represented by -O(G3), G3 being the "substituted or unsubstituted alkyl group" in the specific example group G3. An "unsubstituted alkoxy group" has, unless otherwise specified herein, 1 to 50, preferably 1 to 30, more preferably 1 to 18 carbon atoms.

Substituted or Unsubstituted Alkylthio Group

Specific examples of a "substituted or unsubstituted alkylthio group" mentioned herein include a group represented by -S(G3), G3 being the "substituted or unsubstituted alkyl group" in the specific example group G3. An "unsubstituted alkylthio group" has, unless otherwise specified herein, 1 to 50, preferably 1 to 30, more preferably 1 to 18 carbon atoms.

Substituted or Unsubstituted Aryloxy Group

Specific examples of a "substituted or unsubstituted aryloxy group" mentioned herein include a group represented by -O(G1), G1 being the "substituted or unsubstituted aryl group" in the specific example group G1. An "unsubstituted aryloxy group" has, unless otherwise specified herein, 6 to 50, preferably 6 to 30, more preferably 6 to 18 ring carbon atoms.

Substituted or Unsubstituted Arylthio Group

Specific examples of a "substituted or unsubstituted arylthio group" mentioned herein include a group represented by -S(G1), G1 being the "substituted or unsubstituted aryl group" in the specific example group G1. An "unsubstituted arylthio group" has, unless otherwise specified herein, 6 to 50, preferably 6 to 30, more preferably 6 to 18 ring carbon atoms.

Substituted or Unsubstituted Trialkylsilyl Group

Specific examples of a "trialkylsilyl group" mentioned herein include a group represented by -Si(G3)(G3)(G3), G3 being the "substituted or unsubstituted alkyl group" in the specific example group G3. The plurality of G3 in -Si(G3)(G3)(G3) are mutually the same or different. Each of the alkyl groups in the "trialkylsilyl group" has, unless otherwise specified herein, 1 to 50, preferably 1 to 20, more preferably 1 to 6 carbon atoms.

Substituted or Unsubstituted Aralkyl Group

Specific examples of a "substituted or unsubstituted aralkyl group" mentioned herein include a group represented by -(G3)-(G1), G3 being the "substituted or unsubstituted alkyl group" in the specific example group G3, G1 being the "substituted or unsubstituted aryl group" in the specific example group G1. Accordingly, the "aralkyl group" is a group derived by substituting a hydrogen atom of the "alkyl group" with a substituent in a form of the "aryl group," which is an example of the "substituted alkyl group." An "unsubstituted aralkyl group," which is an "unsubstituted alkyl group" substituted by an "unsubstituted aryl group," has, unless otherwise specified herein, 7 to 50 carbon atoms, preferably 7 to 30 carbon atoms, more preferably 7 to 18 carbon atoms.

Specific examples of the "substituted or unsubstituted aralkyl group" include a benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, and 2-β-naphthylisopropyl group.

Preferable examples of the substituted or unsubstituted aryl group mentioned herein include, unless otherwise specified herein, a phenyl group, p-biphenyl group, m-biphenyl group, o-biphenyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-terphenyl-4-yl group, o-terphenyl-3-yl group, o-terphenyl-2-yl group, 1-naphthyl group, 2-naphthyl group, anthryl group, phenanthryl group, pyrenyl group, chrysenyl group, triphenylenyl group, fluorenyl group, 9,9'-spirobifluorenyl group, 9,9-dimethylfluorenyl group, and 9,9-diphenylfluorenyl group.

Preferable examples of the substituted or unsubstituted heterocyclic group mentioned herein include, unless

otherwise specified herein, a pyridyl group, pyrimidinyl group, triazinyl group, quinolyl group, isoquinolyl group, quinazolinyl group, benzimidazolyl group, phenanthrolinyl group, carbazolyl group (1-carbazolyl group, 2-carbazolyl group, 3-carbazolyl group, 4-carbazolyl group, or 9-carbazolyl group), benzocarbazolyl group, azacarbazolyl group, diazacarbazolyl group, dibenzofuranyl group, naphthobenzofuranyl group, azadibenzofuranyl group, diazadibenzofuranyl group, dibenzothiophenyl group, naphthobenzothiophenyl group, azadibenzothiophenyl group, diazadibenzothiophenyl group, (9-phenyl)carbazolyl group ((9-phenyl)carbazole-1-yl group, (9-phenyl)carbazole-2-yl group, (9-phenyl)carbazole-3-yl group, or (9-phenyl)carbazole-4-yl group), (9-biphenylyl)carbazolyl group, (9-phenyl)phenylcarbazolyl group, diphenylcarbazole-9-yl group, phenylcarbazole-9-yl group, phenyltriazinyl group, biphenylyltriazinyl group, diphenyltriazinyl group, phenyldibenzofuranyl group, and phenyldibenzothiophenyl group.

The carbazolyl group mentioned herein is, unless otherwise specified herein, specifically a group represented by one of formulae below.

[Formula 9]

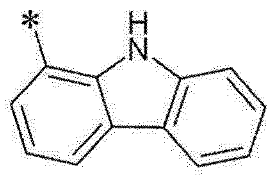
(TEMP-Cz1)

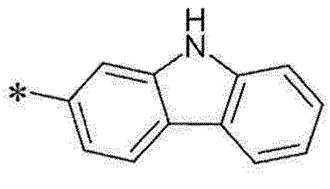
(TEMP-Cz2)

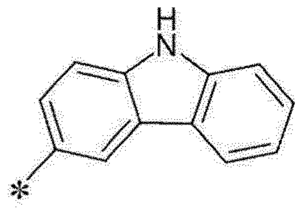
(TEMP-Cz3)

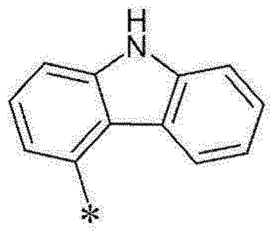
(TEMP-Cz4)

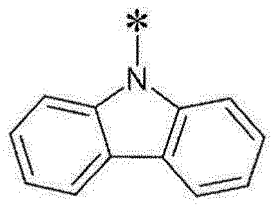
(TEMP-Cz5)

The (9-phenyl)carbazolyl group mentioned herein is, unless otherwise specified herein, specifically a group represented by one of formulae below.

[Formula 10]

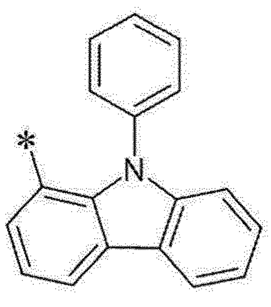
(TEMP-Cz6)

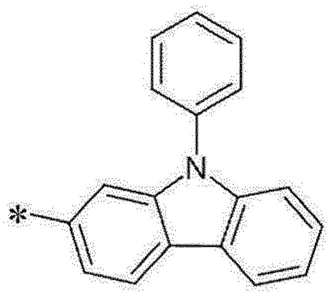
(TEMP-Cz7)

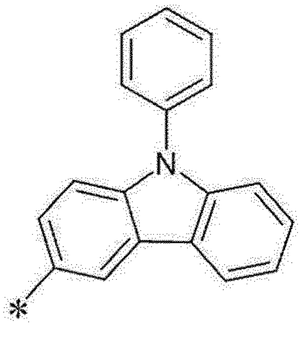
(TEMP-Cz8)

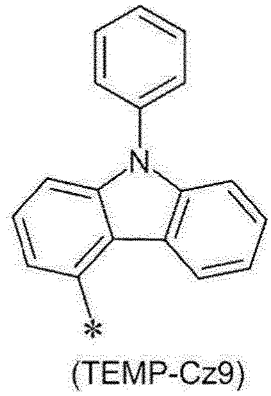
(TEMP-Cz9)

In the formulae (TEMP-Cz1) to (TEMP-Cz9), * represents a bonding position.

The dibenzofuranyl group and dibenzothiophenyl group mentioned herein are, unless otherwise specified herein, each specifically represented by one of formulae below.

[Formula 11]

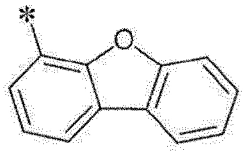

(TEMP-34)

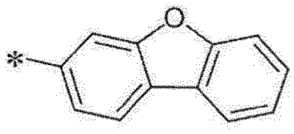

(TEMP-35)

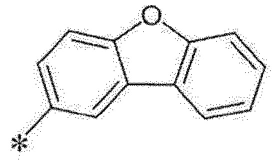

(TEMP-36)

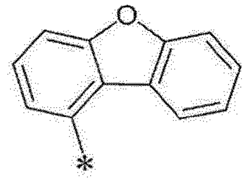

(TEMP-37)

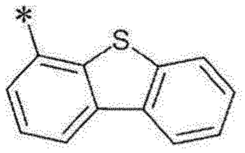

(TEMP-38)

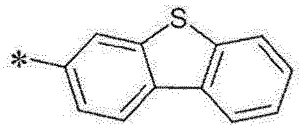

(TEMP-39)

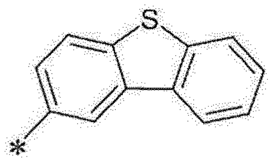

(TEMP-40)

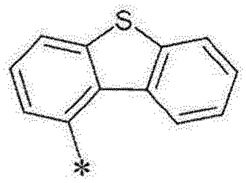

(TEMP-41)

In the formulae (TEMP-34) to (TEMP-41), * represents a bonding position.

Preferable examples of the substituted or unsubstituted alkyl group mentioned herein include, unless otherwise specified herein, a methyl group, ethyl group, propyl group, isopropyl group, n-butyl group, isobutyl group, and t-butyl group.

Substituted or Unsubstituted Arylene Group

The "substituted or unsubstituted arylene group" mentioned herein is, unless otherwise specified herein, a divalent group derived by removing one hydrogen atom on an aryl ring of the "substituted or unsubstituted aryl group." Specific examples of the "substituted or unsubstituted arylene group" (specific example group G12) include a divalent group derived by removing one hydrogen atom on an aryl ring of the "substituted or unsubstituted aryl group" in the specific example group G1.

Substituted or Unsubstituted Divalent Heterocyclic Group

The "substituted or unsubstituted divalent heterocyclic group" mentioned herein is, unless otherwise specified herein, a divalent group derived by removing one hydrogen atom on a heterocycle of the "substituted or unsubstituted heterocyclic group." Specific examples of the "substituted or unsubstituted divalent heterocyclic group" (specific example group G13) include a divalent group derived by removing one hydrogen atom on a heterocyclic ring of the "substituted or unsubstituted heterocyclic group" in the specific example group G2.

Substituted or Unsubstituted Alkylene Group

The "substituted or unsubstituted alkylene group" mentioned herein is, unless otherwise specified herein, a divalent group derived by removing one hydrogen atom on an alkyl chain of the "substituted or unsubstituted alkyl group." Specific examples of the "substituted or unsubstituted alkylene group" (specific example group G14) include a divalent group derived by removing one hydrogen atom on an alkyl chain of the "substituted or unsubstituted alkyl group" in the specific example group G3.

The substituted or unsubstituted arylene group mentioned herein is, unless otherwise specified herein, preferably any one of groups represented by formulae (TEMP-42) to (TEMP-68) below.

[Formula 12]

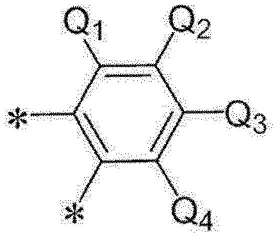

(TEMP-42)

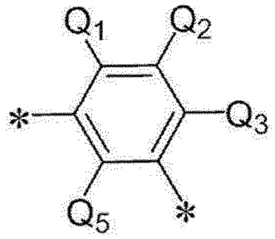

(TEMP-43)

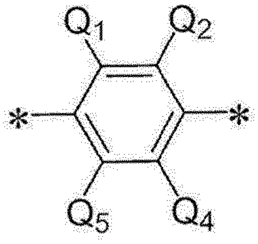

(TEMP-44)

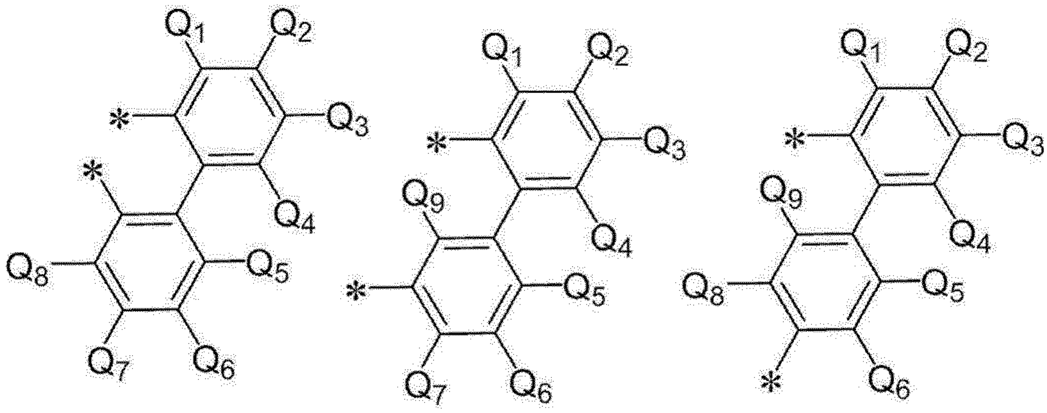

(TEMP-45) (TEMP-46) (TEMP-47)

[Formula 13]

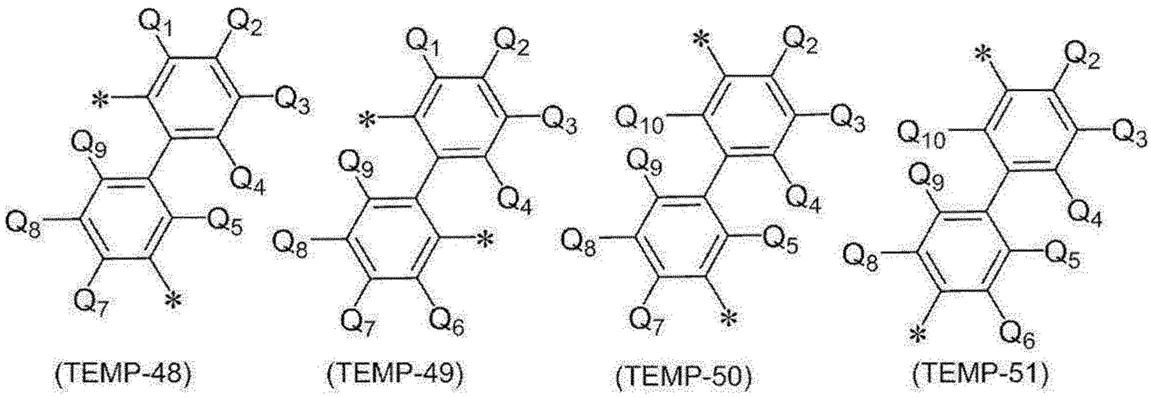

(TEMP-48) (TEMP-49) (TEMP-50) (TEMP-51)

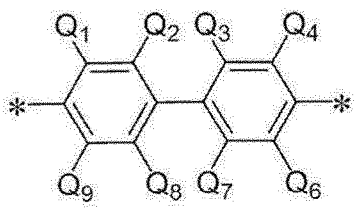

(TEMP-52)

In the formulae (TEMP-42) to (TEMP-52), $Q_1$ to $Q_{10}$ are each independently a hydrogen atom or a substituent.

In the formulae (TEMP-42) to (TEMP-52), * represents a bonding position.

[Formula 14]

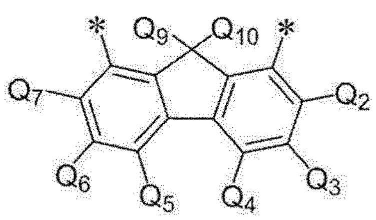
(TEMP-53)

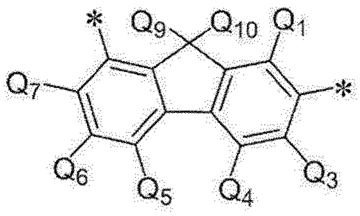
(TEMP-54)

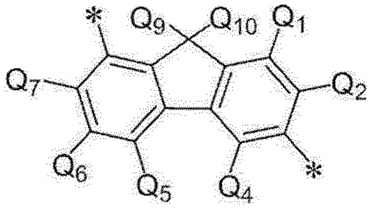
(TEMP-55)

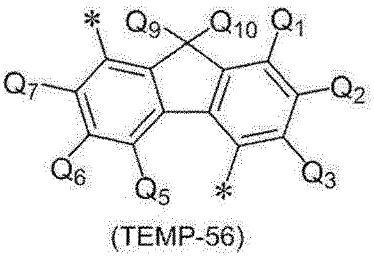
(TEMP-56)

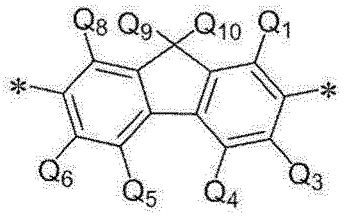
(TEMP-57)

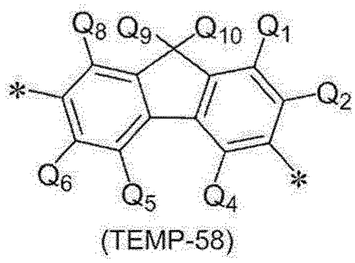
(TEMP-58)

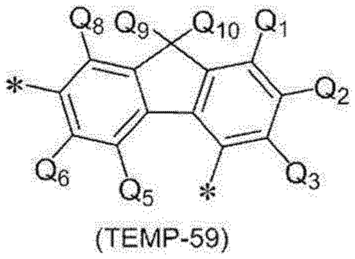
(TEMP-59)

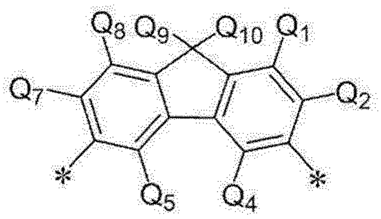
(TEMP-60)

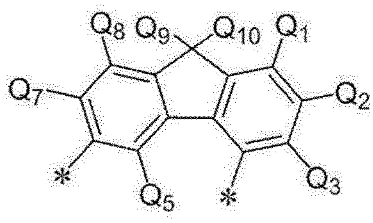
(TEMP-61)

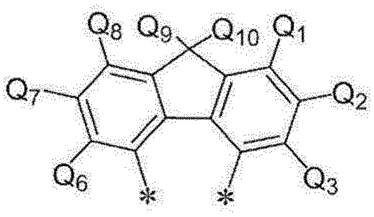
(TEMP-62)

In the formulae (TEMP-53) to (TEMP-62), $Q_1$ to $Q_{10}$ are each independently a hydrogen atom or a substituent.

In the formulae, $Q_9$ and $Q_{10}$ may be mutually bonded through a single bond to form a ring.

In the formulae (TEMP-53) to (TEMP-62), * represents a bonding position.

[Formula 15]

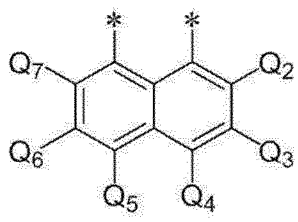
(TEMP-63)

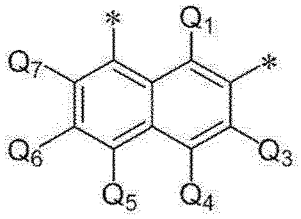
(TEMP-64)

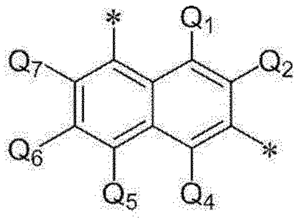
(TEMP-65)

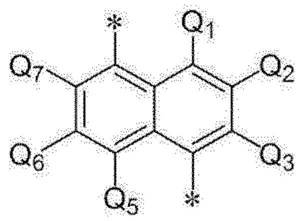
(TEMP-66)

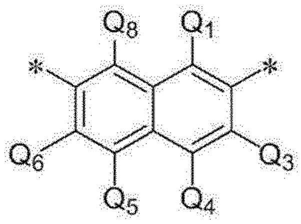
(TEMP-67)

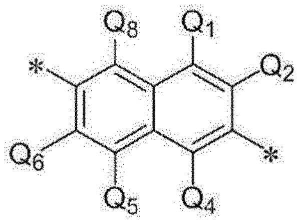
(TEMP-68)

In the formulae (TEMP-63) to (TEMP-68), $Q_1$ to $Q_8$ are each independently a hydrogen atom or a substituent.

In the formulae (TEMP-63) to (TEMP-68), * represents a bonding position.

The substituted or unsubstituted divalent heterocyclic group mentioned herein is, unless otherwise specified herein, preferably a group represented by any one of formulae (TEMP-69) to (TEMP-102) below.

[Formula 16]

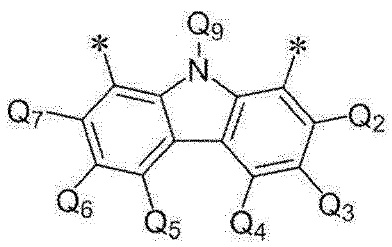
(TEMP-69)

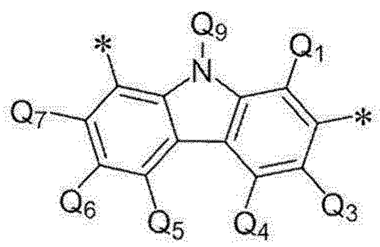
(TEMP-70)

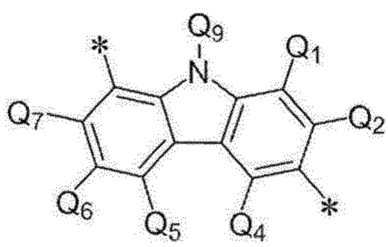
(TEMP-71)

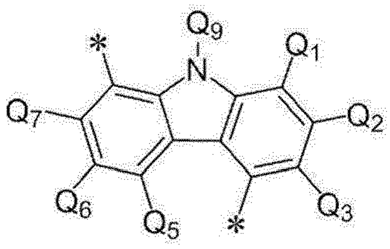
(TEMP-72)

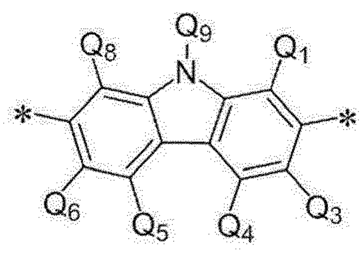
(TEMP-73)

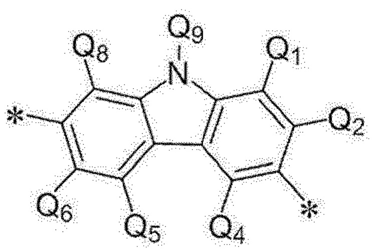
(TEMP-74)

[Formula 17]

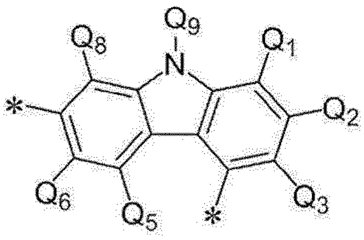

(TEMP-75)

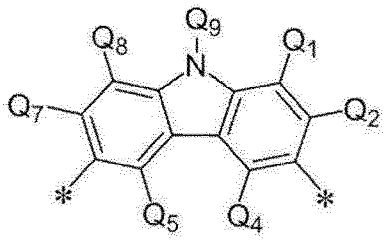

(TEMP-76)

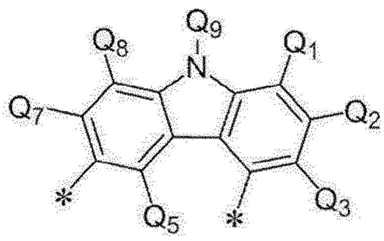

(TEMP-77)

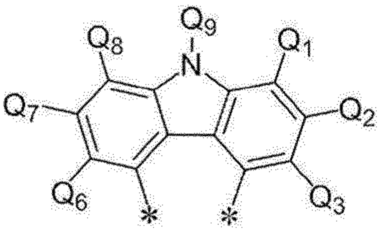

(TEMP-78)

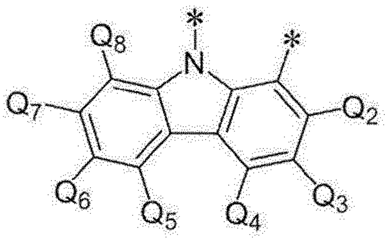

(TEMP-79)

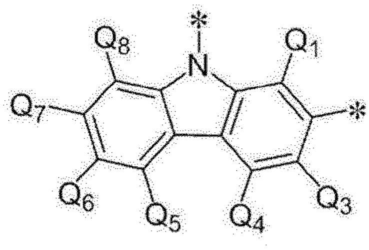

(TEMP-80)

[Formula 18]

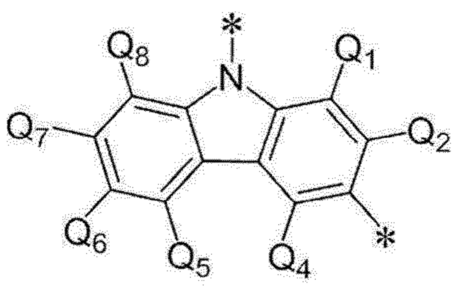

(TEMP-81)

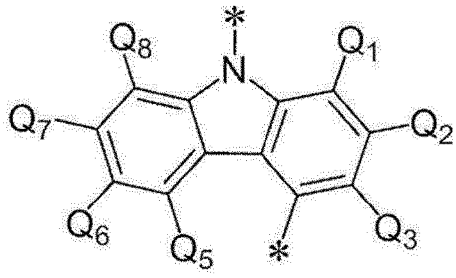

(TEMP-82)

In the formulae (TEMP-69) to (TEMP-82), $Q_1$ to $Q_9$ are each independently a hydrogen atom or a substituent.

[Formula 19]

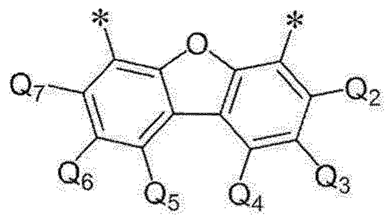
(TEMP-83)

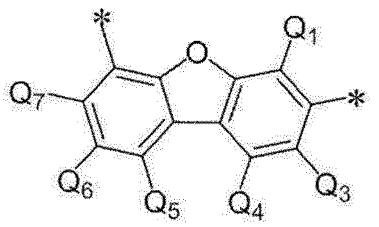
(TEMP-84)

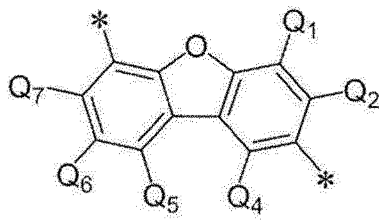
(TEMP-85)

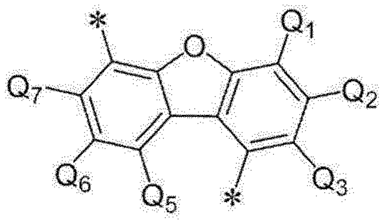
(TEMP-86)

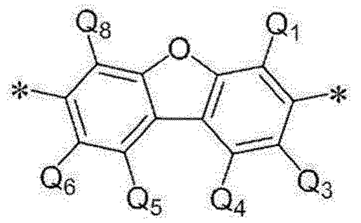
(TEMP-87)

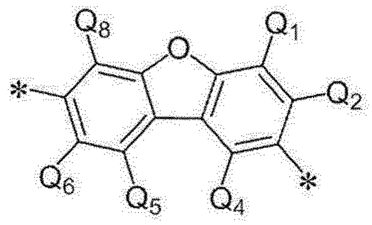
(TEMP-88)

[Formula 20]

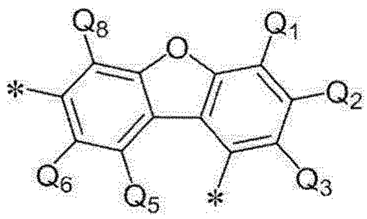
(TEMP-89)

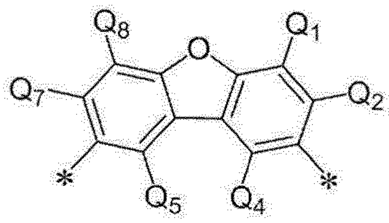
(TEMP-90)

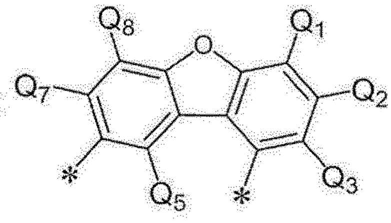
(TEMP-91)

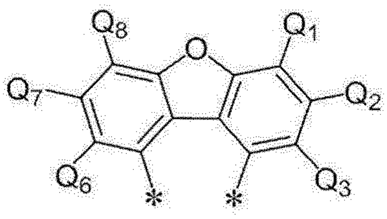
(TEMP-92)

[Formula 21]

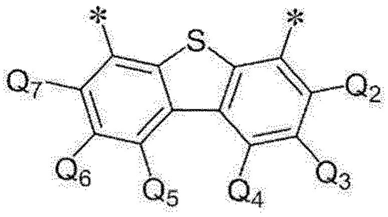

(TEMP-93)

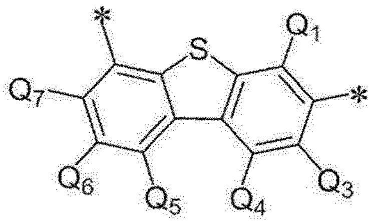

(TEMP-94)

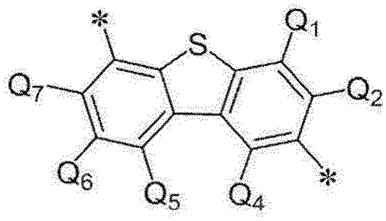

(TEMP-95)

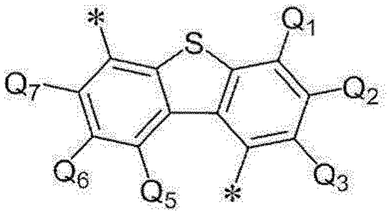

(TEMP-96)

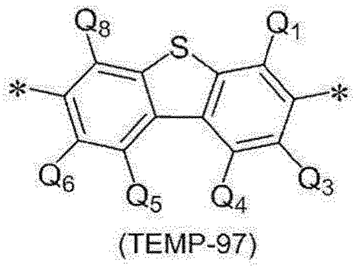

(TEMP-97)

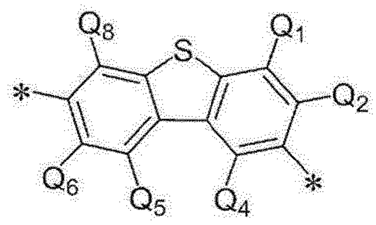

(TEMP-98)

[Formula 22]

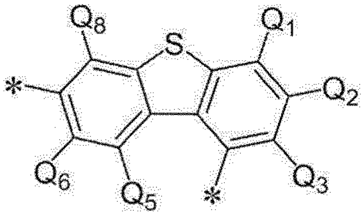

(TEMP-99)

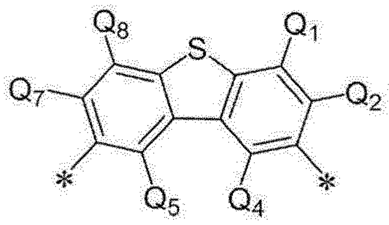

(TEMP-100)

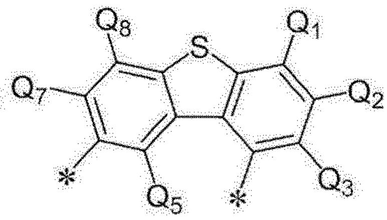

(TEMP-101)

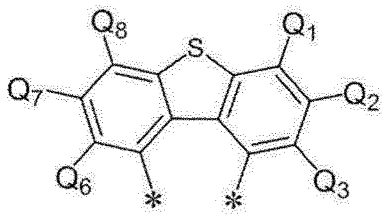

(TEMP-102)

In the formulae (TEMP-83) to (TEMP-102), $Q_1$ to $Q_8$ are each independently a hydrogen atom or a substituent.
The substituent mentioned herein has been described above.

Instance of "Bonded to Form Ring"

Instances where "at least one combination of adjacent two or more (of...) are mutually bonded to form a substituted or unsubstituted monocyclic ring, mutually bonded to form a substituted or unsubstituted fused ring, or not mutually bonded" mentioned herein refer to instances where "at least one combination of adjacent two or more (of...) are mutually bonded to form a substituted or unsubstituted monocyclic ring, "at least one combination of adjacent two or more (of...) are mutually bonded to form a substituted or unsubstituted fused ring," and "at least one combination of adjacent two or more (of...) are not mutually bonded."
Instances where "at least one combination of adjacent two or more (of...) are mutually bonded to form a substituted or unsubstituted monocyclic ring" and "at least one combination of adjacent two or more (of...) are mutually bonded to form a substituted or unsubstituted fused ring" mentioned herein (these instances will be sometimes collectively referred to as an instance of "bonded to form a ring" hereinafter) will be described below. An anthracene compound having a basic skeleton in a form of an anthracene ring and represented by a formula (TEMP-103) below will be used as an example for the description.

[Formula 23]

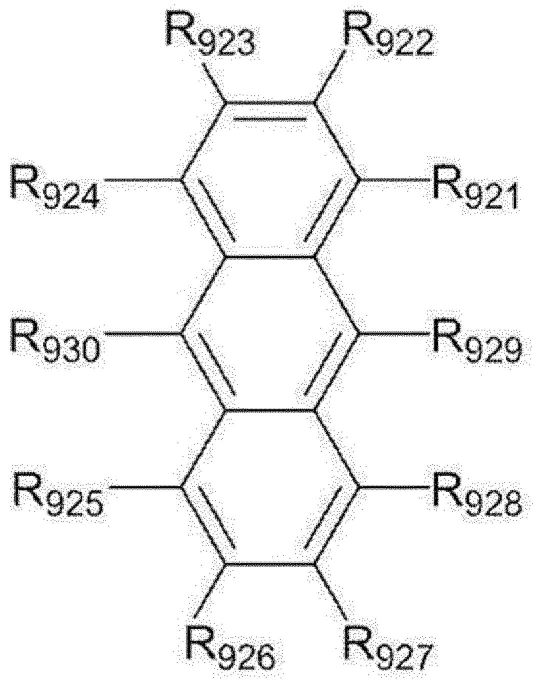

(TEMP-103)

For instance, when "at least one combination of adjacent two or more of $R_{921}$ to $R_{930}$ are mutually bonded to form a ring," the combination of adjacent ones of $R_{921}$ to $R_{930}$ (i.e. the combination at issue) is a combination of $R_{921}$ and $R_{922}$, a combination of $R_{922}$ and $R_{923}$, a combination of $R_{923}$ and $R_{924}$, a combination of $R_{924}$ and $R_{930}$, a combination of $R_{930}$ and $R_{925}$, a combination of $R_{925}$ and $R_{926}$, a combination of $R_{926}$ and $R_{927}$, a combination of $R_{927}$ and $R_{928}$, a combination of $R_{928}$ and $R_{929}$, or a combination of $R_{929}$ and $R_{921}$.

The term "at least one combination" means that two or more of the above combinations of adjacent two or more of $R_{921}$ to $R_{930}$ may simultaneously form rings. For instance, when $R_{921}$ and $R_{922}$ are mutually bonded to form a ring $Q_A$ and $R_{925}$ and $R_{926}$ are simultaneously mutually bonded to form a ring $Q_B$, the anthracene compound represented by the formula (TEMP-103) is represented by a formula (TEMP-104) below.

[Formula 24]

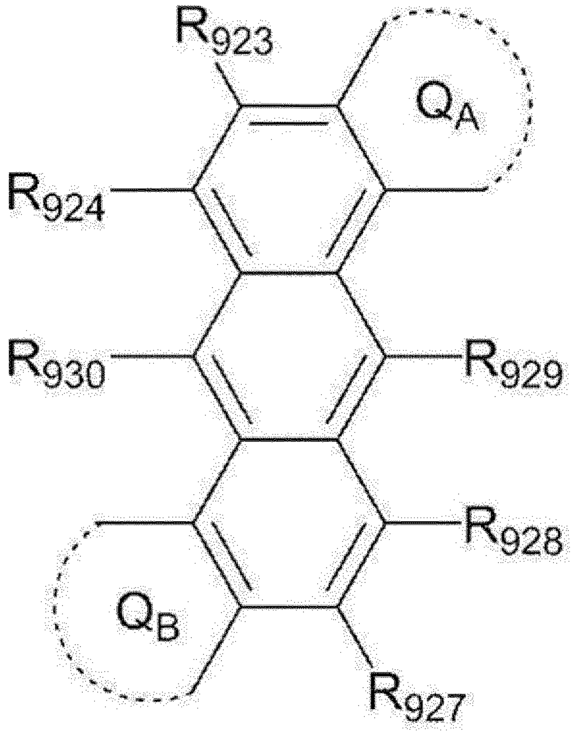

(TEMP-104)

The instance where the "combination of adjacent two or more" form a ring means not only an instance where the "two" adjacent components are bonded but also an instance where adjacent "three or more" are bonded. For instance, $R_{921}$ and $R_{922}$ are mutually bonded to form a ring $Q_A$ and $R_{922}$ and $R_{923}$ are mutually bonded to form a ring Qc, and mutually adjacent three components ($R_{921}$, $R_{922}$ and $R_{923}$) are mutually bonded to form a ring fused to the anthracene basic skeleton. In this case, the anthracene compound represented by the formula (TEMP-103) is represented by a formula (TEMP-105) below. In the formula (TEMP-105) below, the ring $Q_A$ and the ring Qc share $R_{922}$.

[Formula 25]

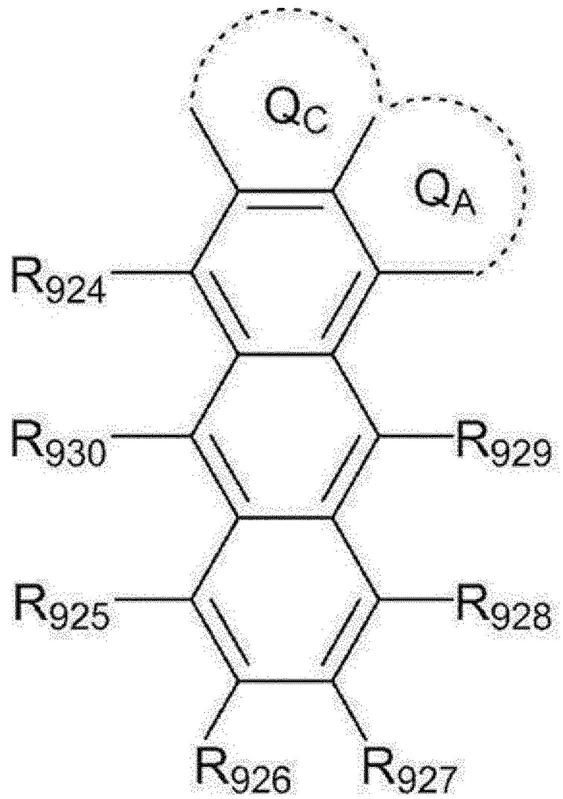

(TEMP-105)

The formed "monocyclic ring" or "fused ring" may be, in terms of the formed ring in itself, a saturated ring or an unsaturated ring. When the "combination of adjacent two" form a "monocyclic ring" or a "fused ring," the "monocyclic ring" or "fused ring" may be a saturated ring or an unsaturated ring. For instance, the ring $Q_A$ and the ring $Q_B$ formed in the formula (TEMP-104) are each independently a "monocyclic ring" or a "fused ring." Further, the ring $Q_A$ and the ring Qc formed in the formula (TEMP-105) are each a "fused ring." The ring $Q_A$ and the ring Qc in the formula (TEMP-105) are fused to form a fused ring. When the ring $Q_A$ in the formula (TEMP-104) is a benzene ring, the ring $Q_A$ is a monocyclic ring. When the ring $Q_A$ in the formula (TEMP-104) is a naphthalene ring, the ring $Q_A$ is a fused ring.

The "unsaturated ring" represents an aromatic hydrocarbon ring or an aromatic heterocycle. The "saturated ring" represents an aliphatic hydrocarbon ring or a non-aromatic heterocycle.

Specific examples of the aromatic hydrocarbon ring include a ring formed by terminating a bond of a group in the specific example of the specific example group G1 with a hydrogen atom.

Specific examples of the aromatic heterocycle include a ring formed by terminating a bond of an aromatic heterocyclic group in the specific example of the specific example group G2 with a hydrogen atom.

Specific examples of the aliphatic hydrocarbon ring include a ring formed by terminating a bond of a group in the specific example of the specific example group G6 with a hydrogen atom.

The phrase "to form a ring" herein means that a ring is formed only by a plurality of atoms of a basic skeleton, or by a combination of a plurality of atoms of the basic skeleton and one or more optional atoms. For instance, the ring $Q_A$ formed by mutually bonding $R_{921}$ and $R_{922}$ shown in the formula (TEMP-104) is a ring formed by a carbon atom of the anthracene skeleton bonded to $R_{921}$, a carbon atom of the anthracene skeleton bonded to $R_{922}$, and one or more optional atoms. Specifically, when the ring $Q_A$ is a monocyclic unsaturated ring formed by $R_{921}$ and $R_{922}$, the ring formed by a carbon atom of the anthracene skeleton bonded to $R_{921}$, a carbon atom of the anthracene skeleton bonded to $R_{922}$, and four carbon atoms is a benzene ring.

The "optional atom" is, unless otherwise specified herein, preferably at least one atom selected from the group consisting of a carbon atom, nitrogen atom, oxygen atom, and sulfur atom. A bond of the optional atom (e.g. a carbon atom and a nitrogen atom) not forming a ring may be terminated by a hydrogen atom or the like or may be substituted by an "optional substituent" described later. When the ring includes any other optional element than the carbon atom, the resultant ring is a heterocycle.

The number of "one or more optional atoms" forming the monocyclic ring or fused ring is, unless otherwise specified herein, preferably in a range from 2 to 15, more preferably in a range from 3 to 12, further preferably in a range

from 3 to 5.

Unless otherwise specified herein, the ring, which may be a "monocyclic ring" or "fused ring," is preferably a "monocyclic ring."

Unless otherwise specified herein, the ring, which may be a "saturated ring" or "unsaturated ring," is preferably an "unsaturated ring."

Unless otherwise specified herein, the "monocyclic ring" is preferably a benzene ring.

Unless otherwise specified herein, the "unsaturated ring" is preferably a benzene ring.

When "at least one combination of adjacent two or more" (of...) are "mutually bonded to form a substituted or unsubstituted monocyclic ring" or "mutually bonded to form a substituted or unsubstituted fused ring," unless otherwise specified herein, at least one combination of adjacent two or more of components are preferably mutually bonded to form a substituted or unsubstituted "unsaturated ring" formed of a plurality of atoms of the basic skeleton, and 1 to 15 atoms of at least one element selected from the group consisting of carbon, nitrogen, oxygen and sulfur.

When the "monocyclic ring" or the "fused ring" has a substituent, the substituent is the substituent described in later-described "optional substituent." When the "monocyclic ring" or the "fused ring" has a substituent, specific examples of the substituent are the substituents described in the above under the subtitle "Substituent Mentioned Herein."

When the "saturated ring" or the "unsaturated ring" has a substituent, the substituent is the substituent described in later-described "optional substituent." When the "monocyclic ring" or the "fused ring" has a substituent, specific examples of the substituent are the substituents described in the above under the subtitle "Substituent Mentioned Herein."

The above is the description for the instances where "at least one combination of adjacent two or more (of...) are mutually bonded to form a substituted or unsubstituted monocyclic ring" and "at least one combination of adjacent two or more (of...) are mutually bonded to form a substituted or unsubstituted fused ring" mentioned herein (sometimes referred to as an instance of "bonded to form a ring").

Substituent for Substituted or Unsubstituted Group

In an exemplary embodiment herein, the substituent for the substituted or unsubstituted group (hereinafter occasionally referred to as an "optional substituent"), is for instance, a group selected from the group consisting of an unsubstituted alkyl group having 1 to 50 carbon atoms, an unsubstituted alkenyl group having 2 to 50 carbon atoms, an unsubstituted alkynyl group having 2 to 50 carbon atoms, an unsubstituted cycloalkyl group having 3 to 50 ring carbon atoms, $-Si(R_{901})(R_{902})(R_{903})$, $-O-(R_{904})$, $-S-(R_{905})$, $-N(R_{906})(R_{907})$, a halogen atom, a cyano group, a nitro group, an unsubstituted aryl group having 6 to 50 ring carbon atoms, and an unsubstituted heterocyclic group having 5 to 50 ring atoms;

$R_{901}$ to $R_{907}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
when two or more $R_{901}$ are present, the two or more $R_{901}$ are mutually the same or different;
when two or more $R_{902}$ are present, the two or more $R_{902}$ are mutually the same or different;
when two or more $R_{903}$ are present, the two or more $R_{903}$ are mutually the same or different;
when two or more $R_{904}$ are present, the two or more $R_{904}$ are mutually the same or different;
when two or more $R_{905}$ are present, the two or more $R_{905}$ are mutually the same or different;
when two or more $R_{906}$ are present, the two or more $R_{906}$ are mutually the same or different; and
when two or more $R_{907}$ are present, the two or more $R_{907}$ are mutually the same or different.

In an exemplary embodiment, the substituent for the substituted or unsubstituted group is a group selected from the group consisting of an alkyl group having 1 to 50 carbon atoms, an aryl group having 6 to 50 ring carbon atoms, and a heterocyclic group having 5 to 50 ring atoms.

In an exemplary embodiment, the substituent for the substituted or unsubstituted group is a group selected from the group consisting of an alkyl group having 1 to 18 carbon atoms, an aryl group having 6 to 18 ring carbon atoms, and a heterocyclic group having 5 to 18 ring atoms.

Specific examples of the above optional substituent are the same as the specific examples of the substituent described in the above under the subtitle "Substituent Mentioned Herein."

Unless otherwise specified herein, adjacent ones of the optional substituents may form a "saturated ring" or an "unsaturated ring," preferably a substituted or unsubstituted saturated five-membered ring, a substituted or unsubstituted saturated six-membered ring, a substituted or unsubstituted unsaturated five-membered ring, or a substituted or unsubstituted unsaturated six-membered ring, more preferably a benzene ring.

Unless otherwise specified herein, the optional substituent may further include a substituent. Examples of the

substituent for the optional substituent are the same as the examples of the optional substituent.

Herein, numerical ranges represented by "AA to BB" represent a range whose lower limit is the value (AA) recited before "to" and whose upper limit is the value (BB) recited after "to."

First Exemplary Embodiment

Compound

A compound according to the exemplary embodiment is represented by a formula (1C-A) below.

[Formula 26]

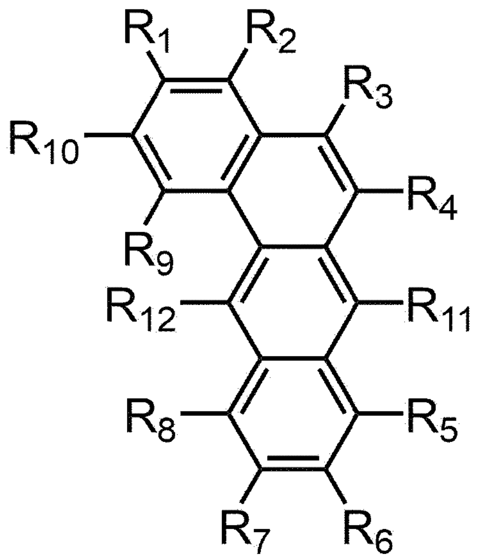

(1C-A)

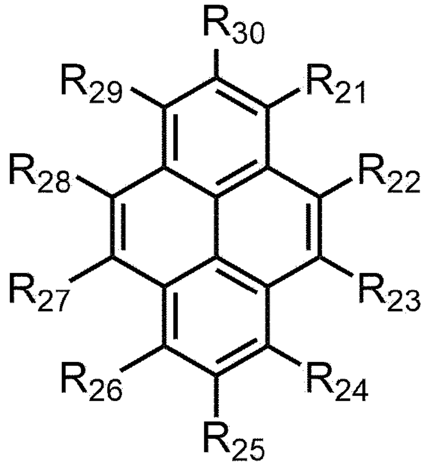

(1D-A)

In the formula (1C-A):

one of $R_4$ to $R_8$ and $R_{10}$ to $R_{12}$ is a group represented by a formula (1D-A) above;

$R_1$ to $R_3$, $R_9$, and $R_4$ to $R_8$ and $R_{10}$ to $R_{12}$ not being the group represented by the formula (1D-A) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a substituted or unsubstituted aralkyl group having 7 to 50 carbon atoms, a group represented by - C(=O)$R_{801}$, a group represented by -COOR$_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;

when $R_{11}$ is a group represented by the formula (1D-A), $R_{12}$ is a hydrogen atom or a substituted or unsubstituted phenyl group;

when $R_{12}$ is a group represented by the formula (1D-A), $R_{11}$ is a hydrogen atom or a substituted or unsubstituted phenyl group;

in the formula (1D-A), one of $R_{21}$ to $R_{30}$ represents a bonding position to a benz[a]anthracene ring in the formula (1C-A), and at least one combination of adjacent two or more of $R_{21}$ to $R_{30}$ not being the bonding position in the formula (1D-A) are mutually bonded to form a substituted or unsubstituted monocyclic ring, mutually bonded to form a substituted or unsubstituted fused ring, or not mutually bonded;

$R_{21}$ to $R_{30}$ not being the bonding position to the benz[a]anthracene ring in the formula (1C-A) and forming neither the substituted or unsubstituted monocyclic ring nor the substituted or unsubstituted fused ring in the formula (1D-A) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl

group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -Q-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and

at least one of $R_{21}$ to $R_{30}$ is other than a hydrogen atom;

in the compound represented by the formula (1C-A), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;

when a plurality of $R_{902}$ are present, the plurality of $R_{902}$ are mutually the same or different;

when a plurality of $R_{903}$ are present, the plurality of $R_{903}$ are mutually the same or different;

when a plurality of $R_{904}$ are present, the plurality of $R_{904}$ are mutually the same or different;

when a plurality of $R_{905}$ are present, the plurality of $R_{905}$ are mutually the same or different;

when a plurality of $R_{906}$ are present, the plurality of $R_{906}$ are mutually the same or different;

when a plurality of $R_{907}$ are present, the plurality of $R_{907}$ are mutually the same or different;

when a plurality of $R_{801}$ are present, the plurality of $R_{801}$ are mutually the same or different; and

when a plurality of $R_{802}$ are present, the plurality of $R_{802}$ are mutually the same or different.

In the group represented by the formula (1D-A) in the compound according to the exemplary embodiment, at least one of $R_{21}$ to $R_{30}$ not being the bonding position to the benz[a]anthracene ring in the formula (1C-A), forming neither the substituted or unsubstituted monocyclic ring nor the substituted or unsubstituted fused ring, and not being the hydrogen atom is also preferably a substituted or unsubstituted aryl group having 6 to 50 ring carbon atoms.

In the group represented by the formula (1D-A) in the compound according to the exemplary embodiment, at least one of $R_{21}$ to $R_{30}$ not being the bonding position to the benz[a]anthracene ring in the formula (1C-A), forming neither the substituted or unsubstituted monocyclic ring nor the substituted or unsubstituted fused ring, and not being the hydrogen atom is also preferably a substituted or unsubstituted phenyl group.

In the compound according to the exemplary embodiment, the group represented by the formula (1D-A) is also preferably a group represented by a formula (1D-A1), (1D-A2), or (1D-A3) below.

[Formula 27]

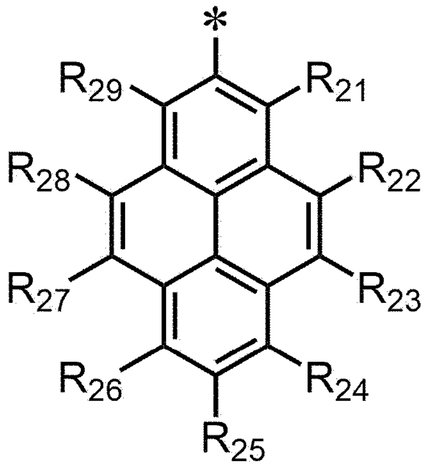

(1D-A1)

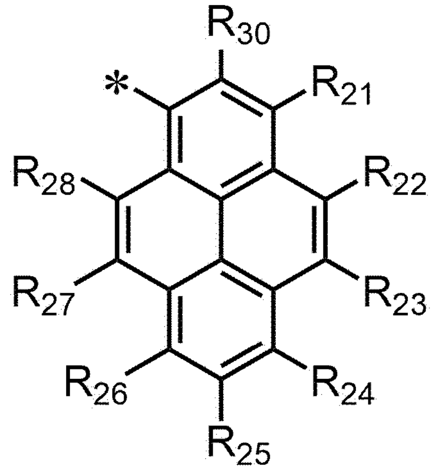

(1D-A2)

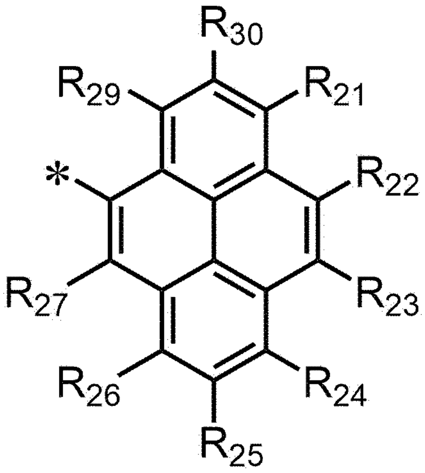

(1D-A3)

In the formula (1D-A1):

at least one combination of adjacent two or more of $R_{21}$ to $R_{29}$ are mutually bonded to form a substituted or unsubstituted monocyclic ring, mutually bonded to form a substituted or unsubstituted fused ring, or not mutually bonded;
$R_{21}$ to $R_{29}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -Q-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms;
at least one of $R_{21}$ to $R_{29}$ is other than a hydrogen atom; and
* represents a bonding position to the benz[a]anthracene ring in the formula (1C-A),
in the formula (1D-A2):

at least one combination of adjacent two or more of $R_{21}$ to $R_{28}$, and $R_{30}$ are mutually bonded to form a substituted or unsubstituted monocyclic ring, mutually bonded to form a substituted or unsubstituted fused ring, or not mutually bonded;
$R_{21}$ to $R_{28}$, and $R_{30}$ forming neither the substituted or unsubstituted monocyclic ring nor the substituted or unsubstituted fused ring each independently represent the same as $R_{21}$ to $R_{29}$ in the formula (1D-A1);

at least one of $R_{21}$ to $R_{28}$, or $R_{30}$ is other than a hydrogen atom; and
* represents a bonding position to the benz[a]anthracene ring in the formula (1C-A),
in the formula (1D-A3):

at least one combination of adjacent two or more of $R_{21}$ to $R_{27}$, $R_{29}$, and $R_{30}$ are mutually bonded to form a substituted or unsubstituted monocyclic ring, mutually bonded to form a substituted or unsubstituted fused ring, or not mutually bonded;
$R_{21}$ to $R_{27}$, $R_{29}$, and $R_{30}$ forming neither the substituted or unsubstituted monocyclic ring nor the substituted or unsubstituted fused ring each independently represent the same as $R_{21}$ to $R_{29}$ in the formula (1D-A1);
at least one of $R_{21}$ to $R_{27}$, $R_{29}$, or $R_{30}$ is other than a hydrogen atom; and
* represents a bonding position to the benz[a]anthracene ring in the formula (1C-A).

In the group represented by the formula (1D-A1), (1D-A2), or (1D-A3) in the compound according to the exemplary embodiment, at least one of $R_{21}$ to $R_{29}$ in the formula (1D-A1), at least one of $R_{21}$ to $R_{28}$, or $R_{30}$ in the formula (1D-A2), or at least one of $R_{21}$ to $R_{27}$, $R_{29}$, or $R_{30}$ in the formula (1D-A3) forming neither the substituted or unsubstituted monocyclic ring nor the substituted or unsubstituted fused ring and not being the hydrogen atom is also preferably a substituted or unsubstituted aryl group having 6 to 50 ring carbon atoms.

In the group represented by the formula (1D-A1), (1D-A2), or (1D-A3) in the compound according to the exemplary embodiment, at least one of $R_{21}$ to $R_{29}$ in the formula (1D-A1), at least one of $R_{21}$ to $R_{28}$, or $R_{30}$ in the formula (1D-A2), or at least one of $R_{21}$ to $R_{27}$, $R_{29}$, or $R_{30}$ in the formula (1D-A3) forming neither the substituted or unsubstituted monocyclic ring nor the substituted or unsubstituted fused ring and not being the hydrogen atom is also preferably a substituted or unsubstituted phenyl group.

In the compound according to the exemplary embodiment, the group represented by the formula (1D-A) is also preferably a group represented by the formula (1D-A1).

In the compound according to the exemplary embodiment, one of $R_5$, $R_6$, $R_7$, $R_8$, $R_{11}$, and $R_{12}$ is also preferably a group represented by the formula (1D-A).

In the compound according to the exemplary embodiment, one of $R_6$, $R_7$, $R_{11}$, and $R_{12}$ is also preferably a group represented by the formula (1D-A).

In the compound according to the exemplary embodiment, $R_{11}$ is also preferably a group represented by the formula (1D-A).

In the compound according to the exemplary embodiment, $R_{12}$ is also preferably a compound represented by the formula (1D-A).

In the compound according to the exemplary embodiment, also preferably, $R_{11}$ is a group represented by the formula (1D-A) and $R_{12}$ is an unsubstituted phenyl group, or $R_{12}$ is a group represented by the formula (1D-A) and $R_{11}$ is an unsubstituted phenyl group.

In the compound according to the exemplary embodiment, also preferably, $R_{11}$ is a group represented by the formula (1D-A) and $R_{12}$ is an unsubstituted phenyl group.

In the compound according to the exemplary embodiment, also preferably, $R_{12}$ is a group represented by the formula (1D-A) and $R_{11}$ is an unsubstituted phenyl group.

The compound according to the exemplary embodiment is also preferably represented by a formula (1C-A-1) below.

The compound according to the exemplary embodiment is also preferably represented by a formula (1C-A-2) below.

[Formula 28]

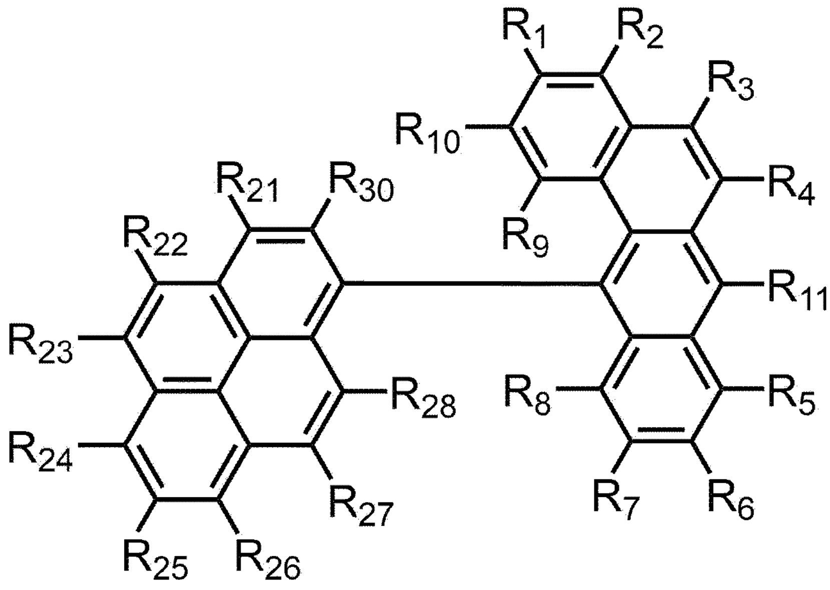

(1C-A-1)

[Formula 29]

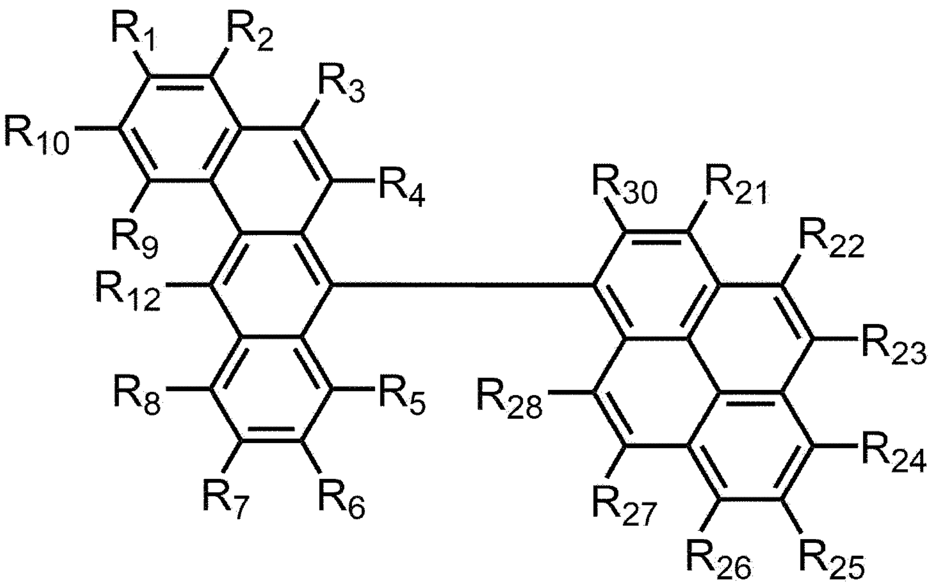

(1C-A-2)

$R_1$ to $R_{11}$ in the formula (1C-A-1) and $R_1$ to $R_{10}$ and $R_{12}$ in the formula (1C-A-2) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a substituted or unsubstituted aralkyl group having 7 to 50 carbon atoms, a group represented by -C(=O)$R_{801}$, a group represented by -COO$R_{802}$, a halogen atom, a cyano group, a nitro group, a substituted or unsubstituted aryl group having 6 to 50 ring carbon atoms, or a substituted or unsubstituted heterocyclic group

having 5 to 50 ring atoms; and

$R_{21}$ to $R_{28}$ and $R_{30}$ in the formulae (1C-A-1) and (1C-A-2) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and

at least one of $R_{21}$ to $R_{28}$ or $R_{30}$ is other than a hydrogen atom.

In the compound according to the exemplary embodiment, $R_{12}$ is also preferably a hydrogen atom.

The compound according to the exemplary embodiment preferably has at least one deuterium atom in a molecule.

In the compound according to the exemplary embodiment, one or more of $R_1$ to $R_3$, and $R_9$ being the hydrogen atom, $R_4$ to $R_8$ and $R_{10}$ to $R_{12}$ not being the group represented by the formula (1D-A) but being the hydrogen atom, and $R_{21}$ to $R_{30}$ being the hydrogen atom are each also preferably a deuterium atom.

In the compound according to the exemplary embodiment, all of $R_1$ to $R_3$, and $R_9$ being the hydrogen atom, $R_4$ to $R_8$ and $R_{10}$ to $R_{12}$ not being the group represented by the formula (1D-A) but being the hydrogen atom, and $R_{21}$ to $R_{30}$ being the hydrogen atom are also preferably deuterium atoms.

In the compound according to the exemplary embodiment, when $R_1$ to $R_3$, and $R_9$ not being the hydrogen atom, $R_4$ to $R_8$ and $R_{10}$ to $R_{12}$ being neither the group represented by the formula (1D-A) nor the hydrogen atom, and $R_{21}$ to $R_{30}$ not being the hydrogen atom each have a hydrogen atom, one or more of those hydrogen atoms are each also preferably a deuterium atom.

In the compound according to the exemplary embodiment, when $R_1$ to $R_3$, and $R_9$ not being the hydrogen atom, $R_4$ to $R_8$ and $R_{10}$ to $R_{12}$ being neither the group represented by the formula (1D-A) nor the hydrogen atom, and $R_{21}$ to $R_{30}$ not being the hydrogen atom each have a hydrogen atom, all of those hydrogen atoms are also preferably deuterium atoms.

In the compound according to the exemplary embodiment, when one of $R_5$, $R_6$, $R_7$, and $R_8$ is a group represented by the formula (1D-A), $R_2$ and $R_3$ are also preferably hydrogen atoms.

The compound according to the exemplary embodiment is also preferably a compound containing no hetero atom in a molecule.

In the compound according to the exemplary embodiment, also preferably, the groups specified to be "substituted or unsubstituted" are each an unsubstituted group, and the rings specified to be "substituted or unsubstituted" are each an unsubstituted ring.

## Method of Producing Compound according to the Exemplary Embodiment

The compound according to the exemplary embodiment can be produced in accordance with a synthesis method described later in Examples. Further, the compound according to the exemplary embodiment can be produced by application of known substitution reactions and materials tailored for the target compound, in accordance with the synthesis method described later in Examples.

## Specific Examples of Compound according to the Exemplary Embodiment

Specific examples of the compound according to the exemplary embodiment are compounds that are represented by the formula (1C) as a first compound used in an organic electroluminescence device according to a fourth exemplary embodiment as described later and that fall within a definition range of the compound according to the exemplary embodiment.

## Second Exemplary Embodiment

### Compound

A compound according to the exemplary embodiment is represented by a formula (1C-B) below.

[Formula 30]

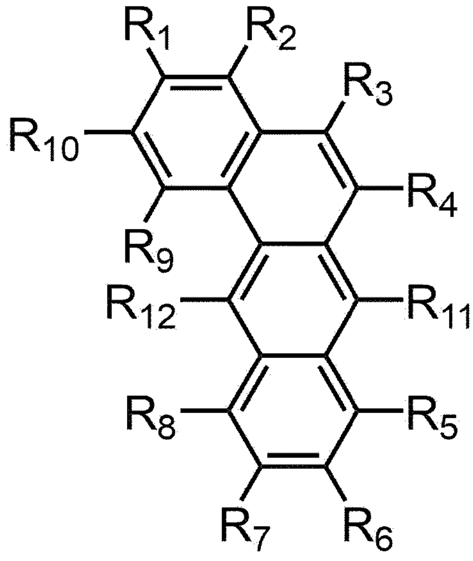

(1C-B)

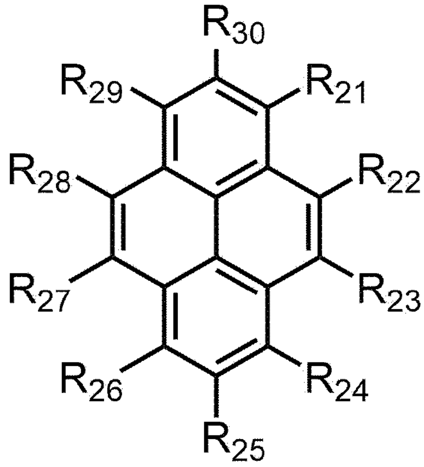

(1D-B)

In the formula (1C-B):

one of $R_4$ to $R_6$, $R_8$, and $R_{10}$ to $R_{12}$ is a group represented by a formula (1D-B) above;

$R_1$ to $R_3$, $R_7$, $R_9$, and $R_4$ to $R_6$, $R_8$, and $R_{10}$ to $R_{12}$ not being the group represented by the formula (1D-B) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by - S-($R_{905}$), a substituted or unsubstituted aralkyl group having 7 to 50 carbon atoms, a group represented by -C(=O)$R_{801}$, a group represented by -COOR$_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;

when $R_{11}$ is a group represented by the formula (1D-B), $R_{12}$ is a hydrogen atom or an unsubstituted phenyl group; and when $R_{12}$ is a group represented by the formula (1D-B), $R_{11}$ is a substituted or unsubstituted phenyl group;

in the formula (1D-B), one of $R_{21}$ to $R_{30}$ represents a bonding position to a benz[a]anthracene ring in the formula (1C-B), and at least one combination of adjacent two or more of $R_{21}$ to $R_{30}$ not being the bonding position in the formula (1D-B) are mutually bonded to form a substituted or unsubstituted monocyclic ring, mutually bonded to form a substituted or unsubstituted fused ring, or not mutually bonded;

$R_{21}$ to $R_{30}$ not being the bonding position to the benz[a]anthracene ring in the formula (1C-B) and forming neither the substituted or unsubstituted monocyclic ring nor the substituted or unsubstituted fused ring in the formula (1D-B) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and

in the compound represented by the formula (1C-B), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;

when a plurality of $R_{902}$ are present, the plurality of $R_{902}$ are mutually the same or different;

when a plurality of $R_{903}$ are present, the plurality of $R_{903}$ are mutually the same or different;

when a plurality of $R_{904}$ are present, the plurality of $R_{904}$ are mutually the same or different;

when a plurality of $R_{905}$ are present, the plurality of $R_{905}$ are mutually the same or different;

when a plurality of $R_{906}$ are present, the plurality of $R_{906}$ are mutually the same or different;

when a plurality of $R_{907}$ are present, the plurality of $R_{907}$ are mutually the same or different; when a plurality of $R_{801}$ are present, the plurality of $R_{801}$ are mutually the same or different; and when a plurality of $R_{802}$ are present, the plurality of $R_{802}$ are mutually the same or different.

In the group represented by the formula (1D-B) in the compound according to the exemplary embodiment, at least one of $R_{21}$ to $R_{30}$ in the formula (1D-B) not being the bonding position to the benz[a]anthracene ring in the formula (1C-B) and forming neither the substituted or unsubstituted monocyclic ring nor the substituted or unsubstituted fused ring is also preferably other than a hydrogen atom.

In the group represented by the formula (1D-B) in the compound according to the exemplary embodiment, at least one of $R_{21}$ to $R_{30}$ in the formula (1D-B) not being the bonding position to the benz[a]anthracene ring in the formula (1C-B) and forming neither the substituted or unsubstituted monocyclic ring nor the substituted or unsubstituted fused ring is also preferably a substituted or unsubstituted aryl group having 6 to 50 ring carbon atoms.

In the group represented by the formula (1D-B) in the compound according to the exemplary embodiment, at least one of $R_{21}$ to $R_{30}$ in the formula (1D-B) not being the bonding position to the benz[a]anthracene ring in the formula (1C-B) and forming neither the substituted or unsubstituted monocyclic ring nor the substituted or unsubstituted fused ring is also preferably a substituted or unsubstituted phenyl group.

In the compound according to the exemplary embodiment, the group represented by the formula (1D-B) is also preferably a group represented by a formula (1D-B1), (1D-B2), or (1D-B3) below.

[Formula 31]

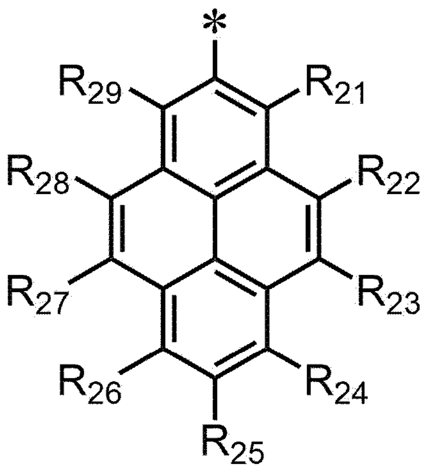

(1D-B1)

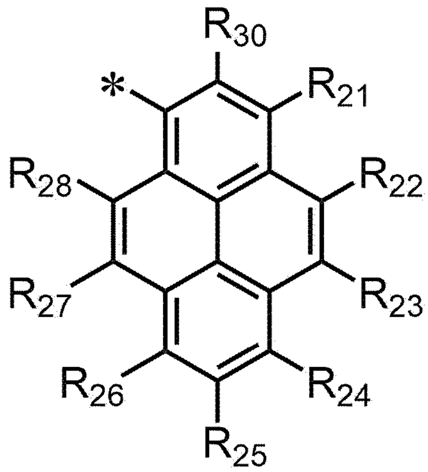

(1D-B2)

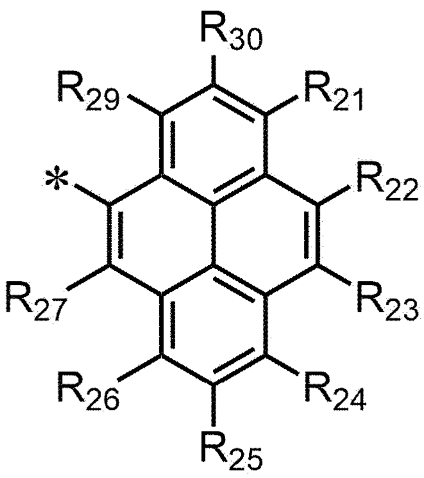

(1D-B3)

In the formula (1D-B1):

at least one combination of adjacent two or more of $R_{21}$ to $R_{29}$ are mutually bonded to form a substituted or unsubstituted monocyclic ring, mutually bonded to form a substituted or unsubstituted fused ring, or not mutually bonded; $R_{21}$ to $R_{29}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and

* represents a bonding position to the benz[a]anthracene ring in the formula (1C-B),

in the formula (1D-B2):

at least one combination of adjacent two or more of $R_{21}$ to $R_{28}$, and $R_{30}$ are mutually bonded to form a substituted or unsubstituted monocyclic ring, mutually bonded to form a substituted or unsubstituted fused ring, or not mutually bonded;

$R_{21}$ to $R_{28}$ and $R_{30}$ forming neither the substituted or unsubstituted monocyclic ring nor the substituted or unsubstituted fused ring each independently represent the same as $R_{21}$ to $R_{29}$ in the formula (1D-B1); and

* represents a bonding position to the benz[a]anthracene ring in the formula (1C-B),

in the formula (1D-B3):

at least one combination of adjacent two or more of $R_{21}$ to $R_{27}$, $R_{29}$, and $R_{30}$ are mutually bonded to form a substituted or unsubstituted monocyclic ring, mutually bonded to form a substituted or unsubstituted fused ring, or not mutually bonded;

$R_{21}$ to $R_{27}$, $R_{29}$, and $R_{30}$ forming neither the substituted or unsubstituted monocyclic ring nor the substituted or unsubstituted fused ring each independently represent the same as $R_{21}$ to $R_{29}$ in the formula (1D-B1); and

* represents a bonding position to the benz[a]anthracene ring in the formula (1C-B).

In the group represented by the formula (1D-B1), (1D-B2), or (1D-B3) in the compound according to the exemplary embodiment, at least one of $R_{21}$ to $R_{29}$ in the formula (1D-B1), at least one of $R_{21}$ to $R_{28}$, or $R_{30}$ in the formula (1D-B2), or at least one of $R_{21}$ to $R_{27}$, $R_{29}$, or $R_{30}$ in the formula (1D-B3) forming neither the substituted or unsubstituted monocyclic ring nor the substituted or unsubstituted fused ring is also preferably other than a hydrogen atom.

In the group represented by the formula (1D-B1), (1D-B2), or (1D-B3) in the compound according to the exemplary embodiment, at least one of $R_{21}$ to $R_{29}$ in the formula (1D-B1), at least one of $R_{21}$ to $R_{28}$, or $R_{30}$ in the formula (1D-B2), or at least one of $R_{21}$ to $R_{27}$, $R_{29}$, or $R_{30}$ in the formula (1D-B3) forming neither the substituted or unsubstituted monocyclic ring nor the substituted or unsubstituted fused ring is also preferably a substituted or unsubstituted aryl group having 6 to 50 ring carbon atoms.

In the group represented by the formula (1D-B1), (1D-B2), or (1D-B3) in the compound according to the exemplary embodiment, at least one of $R_{21}$ to $R_{29}$ in the formula (1D-B1), at least one of $R_{21}$ to $R_{28}$, or $R_{30}$ in the formula (1D-B2), or at least one of $R_{21}$ to $R_{27}$, $R_{29}$, or $R_{30}$ in the formula (1D-B3) forming neither the substituted or unsubstituted monocyclic ring nor the substituted or unsubstituted fused ring is also preferably a substituted or unsubstituted phenyl group.

In the compound according to the exemplary embodiment, the group represented by the formula (1D-B) is also preferably a group represented by the formula (1D-B1).

In the compound according to the exemplary embodiment, one of $R_5$, $R_6$, $R_8$, $R_{11}$, and $R_{12}$ is also preferably a group represented by the formula (1D-B).

In the compound according to the exemplary embodiment, one of $R_6$, $R_{11}$, and $R_{12}$ is also preferably a group represented by the formula (1D-B).

In the compound according to the exemplary embodiment, $R_{11}$ is also preferably a group represented by the formula (1D-B).

In the compound according to the exemplary embodiment, $R_{12}$ is also preferably a group represented by the formula (1D-B).

In the compound according to the exemplary embodiment, also preferably, $R_{11}$ is a group represented by the formula (1D-B) and $R_{12}$ is an unsubstituted phenyl group, or $R_{12}$ is a group represented by the formula (1D-B) and $R_{11}$ is an unsubstituted phenyl group.

In the compound according to the exemplary embodiment, also preferably, $R_{11}$ is a group represented by the

formula (1D-B) and $R_{12}$ is an unsubstituted phenyl group.

In the compound according to the exemplary embodiment, also preferably, $R_{12}$ is a group represented by the formula (1D-B) and $R_{11}$ is an unsubstituted phenyl group.

The compound according to the exemplary embodiment is also preferably represented by a formula (1C-B-1) below.

The compound according to the exemplary embodiment is also preferably represented by a formula (1C-B-2) below.

[Formula 32]

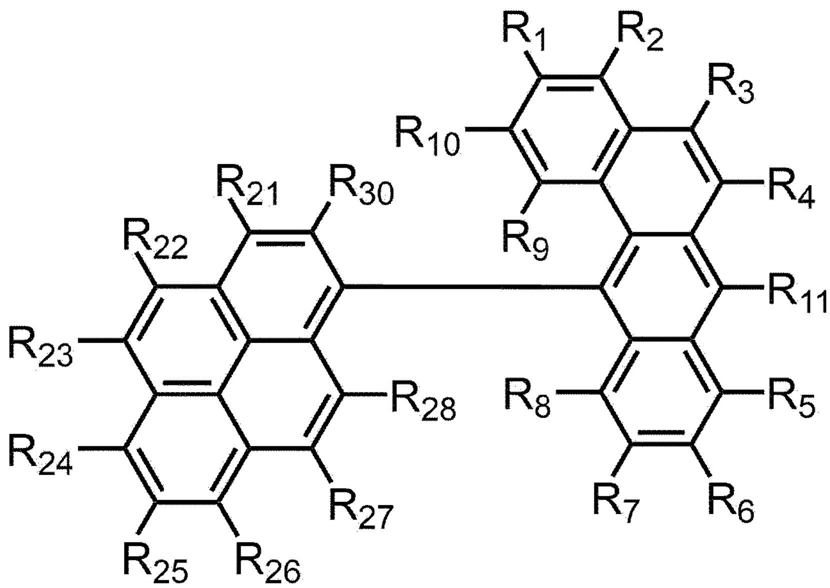

(1C-B-1)

[Formula 33]

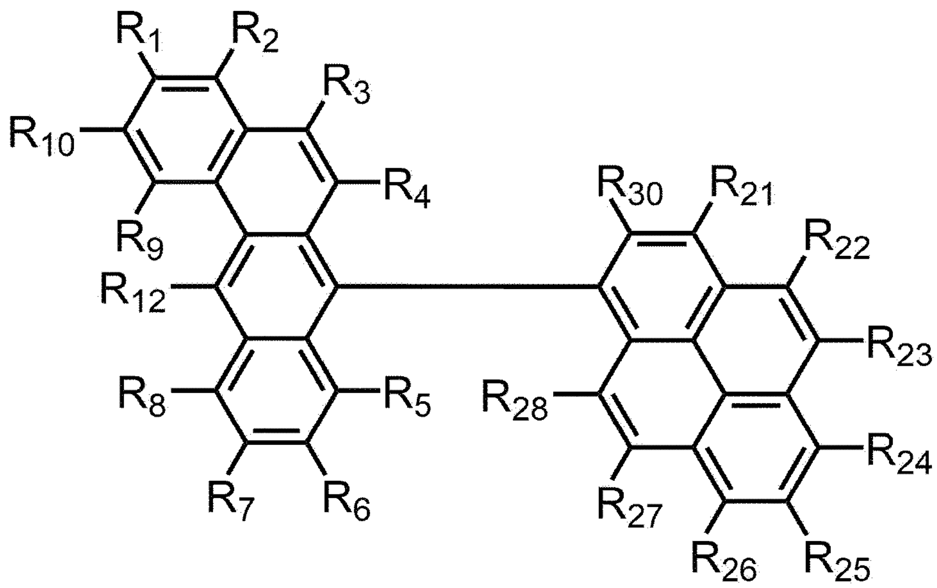

(1C-B-2)

$R_1$ to $R_{10}$ in the formula (1C-B-1) and $R_1$ to $R_{10}$ in the formula (1C-B-2) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a substituted or unsubstituted aralkyl group having 7 to 50 carbon atoms, a group represented by -C(=O)$R_{801}$, a group represented by - COO$R_{802}$, a halogen atom, a cyano group, a nitro group, a substituted or unsubstituted aryl group having 6 to 50 ring carbon atoms, or a substituted or unsubstituted heterocyclic group having 5 to 50 ring atoms; and

$R_{21}$ to $R_{28}$ and $R_{30}$ in the formulae (1C-B-1) and (1C-B-2) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms;

$R_{11}$ in the formula (1C-B-1) is a substituted or unsubstituted phenyl group; and

$R_{12}$ in the formula (1C-B-2) is a hydrogen atom or an unsubstituted phenyl group.

In the compound according to the exemplary embodiment, also preferably, $R_7$ and $R_{12}$ are each a hydrogen atom.

The compound according to the exemplary embodiment also preferably has at least one deuterium atom in a molecule.

In the compound according to the exemplary embodiment, one or more of $R_1$ to $R_3$, $R_7$, and $R_9$ being the hydrogen atom, $R_4$ to $R_6$, $R_8$, and $R_{10}$ to $R_{12}$ not being the group represented by the formula (1D-B) but being the hydrogen atom, and $R_{21}$ to $R_{30}$ being the hydrogen atom are each also preferably a deuterium atom.

In the compound according to the exemplary embodiment, all of $R_1$ to $R_3$, $R_7$, and $R_9$ being the hydrogen atom, $R_4$ to $R_6$, $R_8$, and $R_{10}$ to $R_{12}$ not being the group represented by the formula (1D-B) but being the hydrogen atom, and $R_{21}$ to $R_{30}$ being the hydrogen atom are also preferably deuterium atoms.

In the compound according to the exemplary embodiment, when $R_1$ to $R_3$, $R_7$, and $R_9$ not being the hydrogen atom, $R_4$ to $R_8$ and $R_{10}$ to $R_{12}$ being neither the group represented by the formula (1D-B) nor the hydrogen atom, and $R_{21}$ to $R_{30}$ not being the hydrogen atom each have a hydrogen atom, one or more of those hydrogen atoms are each also preferably a deuterium atom.

In the compound according to the exemplary embodiment, when $R_1$ to $R_3$, $R_7$, and $R_9$ not being the hydrogen atom, $R_4$ to $R_8$ and $R_{10}$ to $R_{12}$ being neither the group represented by the formula (1D-B) nor the hydrogen atom, and $R_{21}$ to $R_{30}$ not being the hydrogen atom each have a hydrogen atom, all of those hydrogen atoms are also preferably deuterium atoms.

The compound according to the exemplary embodiment is also preferably a compound containing no hetero atom in a molecule.

In the compound according to the exemplary embodiment, also preferably, the groups specified to be "substituted or unsubstituted" are each an unsubstituted group, and the rings specified to be "substituted or unsubstituted" are each an unsubstituted ring.

Method of Producing Compound according to the Exemplary Embodiment

The compound according to the exemplary embodiment can be produced in accordance with a synthesis method described later in Examples. Further, the compound according to the exemplary embodiment can be produced by application of known substitution reactions and materials tailored for the target compound, in accordance with the synthesis method described later in Examples.

Specific Examples of Compound according to the Exemplary Embodiment

Specific examples of the compound according to the exemplary embodiment are compounds that are represented by the formula (1C) as the first compound used in the organic electroluminescence device according to the fourth exemplary embodiment as described later and that fall within a definition range of the compound according to the exemplary embodiment.

Third Exemplary Embodiment

Compound

A compound according to the exemplary embodiment is represented by a formula (1C-C) below.

[Formula 34]

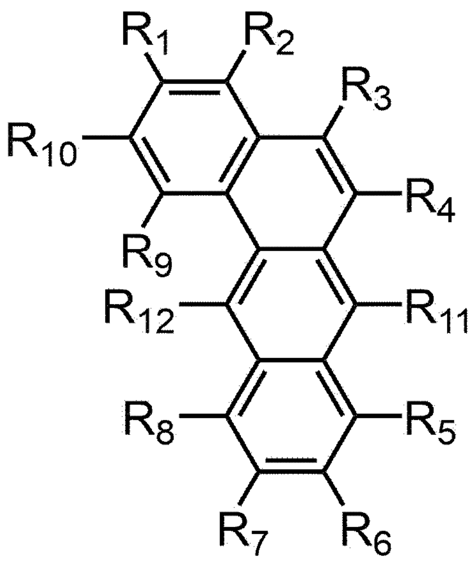

(1C-C)

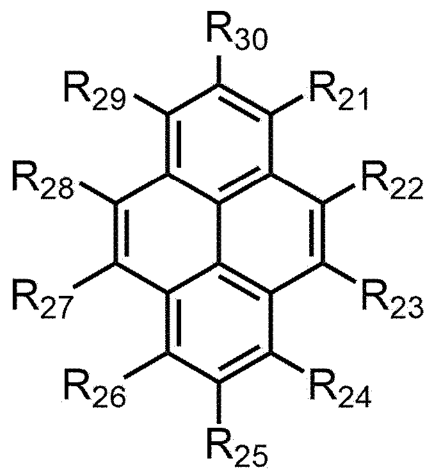

(1D-C)

In the formula (1C-C):

one of $R_4$ to $R_8$ and $R_{10}$ to $R_{12}$ is a group represented by a formula (1D-C) above;

$R_1$ to $R_3$, $R_9$, and $R_4$ to $R_8$ and $R_{10}$ to $R_{12}$ not being the group represented by the formula (1D-C) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a substituted or unsubstituted aralkyl group having 7 to 50 carbon atoms, a group represented by - C(=O)$R_{801}$, a group represented by -COO$R_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;

when $R_{11}$ is a group represented by the formula (1D-C), $R_{12}$ is a hydrogen atom or a substituted or unsubstituted phenyl group; and

when $R_{12}$ is a group represented by the formula (1D-C), $R_{11}$ is a hydrogen atom or a substituted or unsubstituted phenyl group;

in the formula (1D-C), one of $R_{22}$, $R_{23}$, $R_{25}$, $R_{27}$, $R_{28}$, and $R_{30}$ represents a bonding position to a benz[a]anthracene ring in the formula (1C-C), and at least one combination of adjacent two or more of $R_{22}$, $R_{23}$, $R_{25}$, $R_{27}$, $R_{28}$, and $R_{30}$ not being the bonding position, $R_{21}$, $R_{24}$, $R_{26}$, and $R_{29}$ in the formula (1D-C) are mutually bonded to form a substituted or unsubstituted monocyclic ring, mutually bonded to form a substituted or unsubstituted fused ring, or not mutually bonded;

$R_{21}$ to $R_{30}$ not being the bonding position to the benz[a]anthracene ring in the formula (1C-C) and forming neither the substituted or unsubstituted monocyclic ring nor the substituted or unsubstituted fused ring in the formula (1D-C) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and

in the compound represented by the formula (1C-C), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

In the group represented by the formula (1D-C) in the compound according to the exemplary embodiment, at least one of $R_{21}$ to $R_{30}$ in the formula (1D-C) not being the bonding position to the benz[a]anthracene ring in the formula (1C-C) and forming neither the substituted or unsubstituted monocyclic ring nor the substituted or unsubstituted fused ring is also preferably other than a hydrogen atom.

In the group represented by the formula (1D-C) in the compound according to the exemplary embodiment, at least one of $R_{21}$ to $R_{30}$ in the formula (1D-C) not being the bonding position to the benz[a]anthracene ring in the formula (1C-C) and forming neither the substituted or unsubstituted monocyclic ring nor the substituted or unsubstituted fused ring is also preferably a substituted or unsubstituted aryl group having 6 to 50 ring carbon atoms.

In the group represented by the formula (1D-C) in the compound according to the exemplary embodiment, at least one of $R_{21}$ to $R_{30}$ in the formula (1D-C) not being the bonding position to the benz[a]anthracene ring in the formula (1C-C) and forming neither the substituted or unsubstituted monocyclic ring nor the substituted or unsubstituted fused ring is also preferably a substituted or unsubstituted phenyl group.

In the compound according to the exemplary embodiment, the group represented by the formula (1D-C) is also preferably a group represented by a formula (1D-C1) or (1D-C3) below.

[Formula 35]

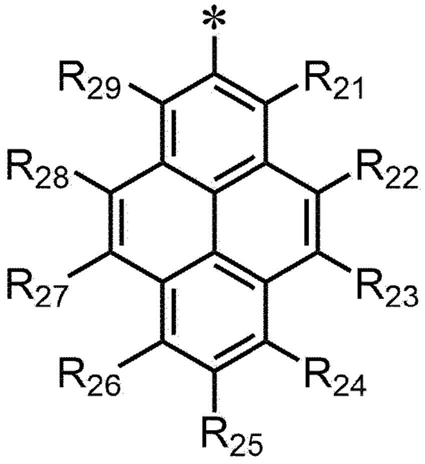

(1D-C1)

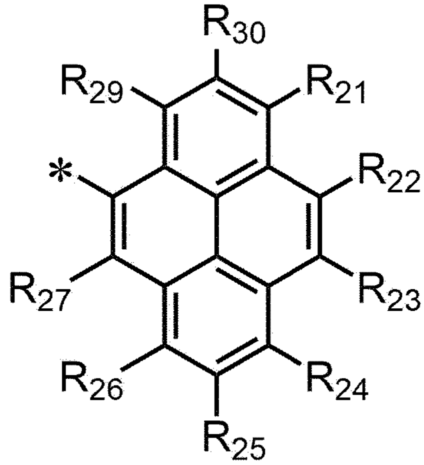

(1D-C3)

In the formula (1D-C1):

at least one combination of adjacent two or more of $R_{21}$ to $R_{29}$ are mutually bonded to form a substituted or unsubstituted monocyclic ring, mutually bonded to form a substituted or unsubstituted fused ring, or not mutually bonded;
$R_{21}$ to $R_{29}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and
* represents a bonding position to the benz[a]anthracene ring in the formula (1C-C), and
in the formula (1D-C3):

at least one combination of adjacent two or more of $R_{21}$ to $R_{27}$, $R_{29}$, and $R_{30}$ are mutually bonded to form a substituted or unsubstituted monocyclic ring, mutually bonded to form a substituted or unsubstituted fused ring, or not mutually bonded;
$R_{21}$ to $R_{27}$, $R_{29}$, and $R_{30}$ forming neither the substituted or unsubstituted monocyclic ring nor the substituted or unsubstituted fused ring each independently represent the same as $R_{21}$ to $R_{29}$ in the formula (1D-C1); and
* represents a bonding position to the benz[a]anthracene ring in the formula (1C-C).

In the group represented by the formula (1D-C1) or (1D-C3) in the compound according to the exemplary embodiment, at least one of $R_{21}$ to $R_{29}$ in the formula (1D-C1) or at least one of $R_{21}$ to $R_{27}$, $R_{29}$, or $R_{30}$ in the formula (1D-C3) forming neither the substituted or unsubstituted monocyclic ring nor the substituted or unsubstituted fused ring is also preferably other than a hydrogen atom.
In the group represented by the formula (1D-C1) or (1D-C3) in the compound according to the exemplary embodiment, at least one of $R_{21}$ to $R_{29}$ in the formula (1D-C1) or at least one of $R_{21}$ to $R_{27}$, $R_{29}$, or $R_{30}$ in the formula (1D-C3) forming neither the substituted or unsubstituted monocyclic ring nor the substituted or unsubstituted fused ring is also preferably a substituted or unsubstituted aryl group having 6 to 50 ring carbon atoms.
In the group represented by the formula (1D-C1) or (1D-C3) in the compound according to the exemplary embodiment, at least one of $R_{21}$ to $R_{29}$ in the formula (1D-C1) or at least one of $R_{21}$ to $R_{27}$, $R_{29}$, or $R_{30}$ in the formula (1D-C3) forming neither the substituted or unsubstituted monocyclic ring nor the substituted or unsubstituted fused ring is also preferably a substituted or unsubstituted phenyl group.
In the compound according to the exemplary embodiment, the group represented by the formula (1D-C) is also preferably a group represented by the formula (1D-C1).
In the compound according to the exemplary embodiment, one of $R_5$, $R_6$, $R_7$, $R_8$, $R_{11}$, and $R_{12}$ is also preferably

a group represented by the formula (1D-C).

In the compound according to the exemplary embodiment, one of $R_6$, $R_7$, $R_{11}$, and $R_{12}$ is also preferably a group represented by the formula (1D-C).

In the compound according to the exemplary embodiment, $R_{11}$ is also preferably a group represented by the formula (1D-C).

In the compound according to the exemplary embodiment, $R_{12}$ is also preferably a group represented by the formula (1D-C).

In the compound according to the exemplary embodiment, also preferably, $R_{11}$ is a group represented by the formula (1D-C) and $R_{12}$ is an unsubstituted phenyl group, or $R_{12}$ is a group represented by the formula (1D-C) and $R_{11}$ is an unsubstituted phenyl group.

In the compound according to the exemplary embodiment, also preferably, $R_{11}$ is a group represented by the formula (1D-C) and $R_{12}$ is an unsubstituted phenyl group.

In the compound according to the exemplary embodiment, also preferably, $R_{12}$ is a group represented by the formula (1D-C) and $R_{11}$ is an unsubstituted phenyl group.

The compound according to the exemplary embodiment is also preferably represented by a formula (1C-C-1) below.

The compound according to the exemplary embodiment is also preferably represented by a formula (1C-C-2) below.

[Formula 36]

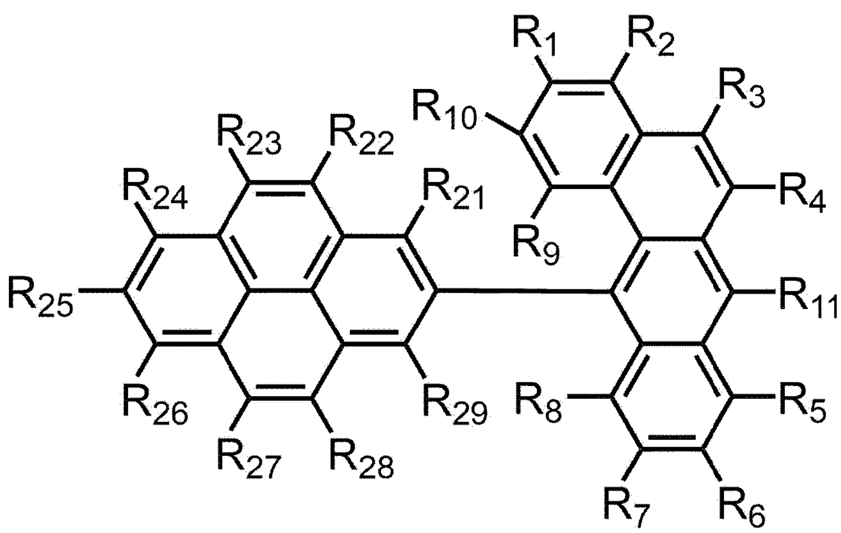

(1C-C-1)

[Formula 37]

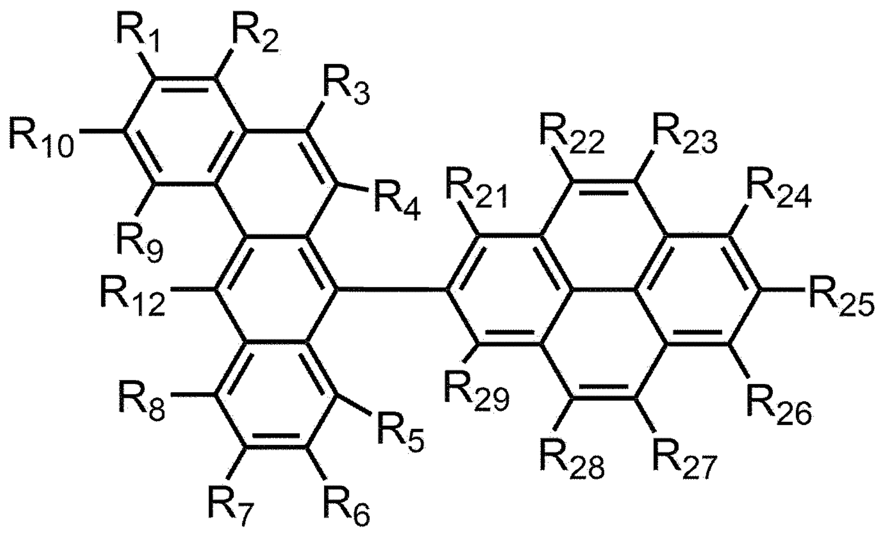

(1C-C-2)

$R_1$ to $R_{11}$ in the formula (1C-C-1) and $R_1$ to $R_{10}$ and $R_{12}$ in the formula (1C-C-2) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a substituted or unsubstituted aralkyl group having 7 to 50 carbon atoms, a group represented by $-C(=O)R_{801}$, a group represented by $-COOR_{802}$, a halogen atom, a cyano group, a nitro group, a substituted or unsubstituted aryl group having 6 to 50 ring carbon atoms, or a substituted or unsubstituted heterocyclic group having 5 to 50 ring atoms; and

$R_{21}$ to $R_{29}$ in the formulae (1C-C-1) and (1C-C-2) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

In the compound according to the exemplary embodiment, $R_{12}$ is also preferably a hydrogen atom.

The compound according to the exemplary embodiment also preferably has at least one deuterium atom in a molecule.

In the compound according to the exemplary embodiment, one or more of $R_1$ to $R_3$, and $R_9$ being the hydrogen atom, $R_4$ to $R_8$ and $R_{10}$ to $R_{12}$ not being the group represented by the formula (1D-C) but being the hydrogen atom, and $R_{21}$ to $R_{30}$ being the hydrogen atom are each also preferably a deuterium atom.

In the compound according to the exemplary embodiment, all of $R_1$ to $R_3$, and $R_9$ being the hydrogen atom, $R_4$ to $R_8$ and $R_{10}$ to $R_{12}$ not being the group represented by the formula (1D-C) but being the hydrogen atom, and $R_{21}$ to $R_{30}$ being the hydrogen atom are also preferably deuterium atoms.

In the compound according to the exemplary embodiment, when $R_1$ to $R_3$, and $R_9$ not being the hydrogen atom, $R_4$ to $R_8$ and $R_{10}$ to $R_{12}$ being neither the group represented by the formula (1D-C) nor the hydrogen atom, and $R_{21}$ to $R_{30}$ not being the hydrogen atom each have a hydrogen atom, one or more of those hydrogen atoms are each also preferably a deuterium atom.

In the compound according to the exemplary embodiment, when $R_1$ to $R_3$, and $R_9$ not being the hydrogen atom, $R_4$ to $R_8$ and $R_{10}$ to $R_{12}$ being neither the group represented by the formula (1D-C) nor the hydrogen atom, and $R_{21}$ to $R_{30}$ not being the hydrogen atom each have a hydrogen atom, all of those hydrogen atoms are also preferably deuterium atoms.

The compound according to the exemplary embodiment is also preferably a compound containing no hetero atom in a molecule.

In the compound according to the exemplary embodiment, also preferably, the groups specified to be "substituted or unsubstituted" are each an unsubstituted group, and the rings specified to be "substituted or unsubstituted" are each an unsubstituted ring.

Method of Producing Compound according to the Exemplary Embodiment

The compound according to the exemplary embodiment can be produced in accordance with synthesis method described later in Examples. Further, the compound according to the exemplary embodiment can be produced by application of known substitution reactions and materials tailored for the target compound, in accordance with the synthesis method described later in Examples.

Specific Examples of Compound according to the Exemplary Embodiment

Specific examples of the compound according to the exemplary embodiment are compounds that are represented by the formula (1C) as the first compound used in the organic electroluminescence device according to the fourth exemplary embodiment as described later and that fall within a definition range of the compound according to the exemplary embodiment.

Fourth Exemplary Embodiment

Organic Electroluminescence Device

An organic EL device according to a fourth exemplary embodiment of the invention will be described below.

In an exemplary arrangement, the organic EL device according to the exemplary embodiment contains a compound according to the first exemplary embodiment, the second exemplary embodiment, or the third exemplary embodiment.

In an exemplary arrangement, the organic EL device according to the exemplary embodiment includes an anode, a cathode, and an organic layer disposed between the anode and the cathode. The organic layer includes at least one layer formed from an organic compound(s). Alternatively, the organic layer includes a plurality of layers formed from an organic compound(s). The organic layer may further contain an inorganic compound(s).

In an exemplary arrangement of the organic EL device according to the exemplary embodiment, at least one layer of the organic layer contains a compound according to the first exemplary embodiment.

In an exemplary arrangement of the organic EL device according to the exemplary embodiment, at least one layer of the organic layer contains a compound according to the second exemplary embodiment.

In an exemplary arrangement of the organic EL device according to the exemplary embodiment, at least one layer of the organic layer contains a compound according to the third exemplary embodiment.

In the organic EL device of the exemplary embodiment, at least one layer of the organic layer preferably includes an emitting region. In the organic EL device of the exemplary embodiment, the emitting region preferably includes at least one emitting layer. In an exemplary embodiment, the emitting layer contains a compound represented by the formula (1C-A). In an exemplary embodiment, the emitting layer contains a compound represented by the formula (1C-B). In an exemplary embodiment, the emitting layer contains a compound represented by the formula (1C-C).

Also preferably, an organic EL device according to the exemplary embodiment includes an anode, a cathode, and an emitting region disposed between the anode and the cathode, in which the emitting region includes a first emitting layer and a second emitting layer, the first emitting layer contains, as a first compound, a compound represented by the formula (1C-A), and the second emitting layer contains a second compound.

In the organic EL device of the exemplary embodiment, the compound represented by the formula (1C-A) and the second compound are mutually different compounds.

Also preferably, an organic EL device according to the exemplary embodiment includes an anode, a cathode, and an emitting region disposed between the anode and the cathode, in which the emitting region includes a first emitting layer and a second emitting layer, the first emitting layer contains, as a first compound, a compound represented by the formula (1C-B), and the second emitting layer contains a second compound.

In the organic EL device of the exemplary embodiment, the compound represented by the formula (1C-B) and the second compound are mutually different compounds.

Also preferably, an organic EL device according to the exemplary embodiment includes an anode, a cathode, and an emitting region disposed between the anode and the cathode, in which the emitting region includes a first emitting layer and a second emitting layer, the first emitting layer contains, as a first compound, a compound represented by the formula (1C-C), and the second emitting layer contains a second compound.

In the organic EL device of the exemplary embodiment, the compound represented by the formula (1C-C) and

the second compound are mutually different compounds.

In an exemplary arrangement, the organic EL device according to the exemplary embodiment includes an anode, a cathode, and an organic layer disposed between the anode and the cathode, in which the organic layer includes an emitting region that includes a first emitting layer and a second emitting layer, the first emitting layer contains a first compound represented by a formula (1C) below, and the second emitting layer contains a second compound.

[Formula 38]

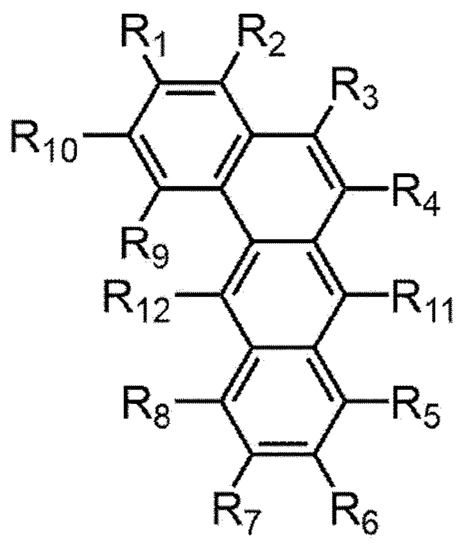

(1C)

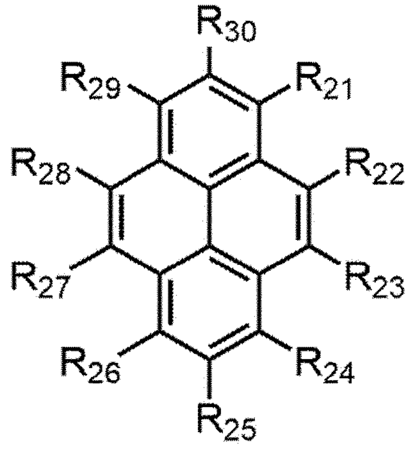

(1D)

In the formula (1C):

one of $R_4$ to $R_8$ and $R_{10}$ to $R_{12}$ is a group represented by a formula (1D) above;

$R_1$ to $R_3$, $R_9$, and $R_4$ to $R_8$ and $R_{10}$ to $R_{12}$ not being the group represented by the formula (1D) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a substituted or unsubstituted aralkyl group having 7 to 50 carbon atoms, a group represented by - $C(=O)R_{801}$, a group represented by $-COOR_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;

when $R_{11}$ is a group represented by the formula (1D), $R_{12}$ is a hydrogen atom or a substituted or unsubstituted phenyl group; and

when $R_{12}$ is a group represented by the formula (1D), $R_{11}$ is a hydrogen atom or a substituted or unsubstituted phenyl group;

in the formula (1D), one of $R_{21}$ to $R_{30}$ represents a bonding position to a benz[a]anthracene ring in the formula (1C), and at least one combination of adjacent two or more of $R_{21}$ to $R_{30}$ not being the bonding position in the formula (1D) are mutually bonded to form a substituted or unsubstituted monocyclic ring, mutually bonded to form a substituted or unsubstituted fused ring, or not mutually bonded;

$R_{21}$ to $R_{30}$ not being the bonding position to the benz[a]anthracene ring in the formula (1C) and forming neither the substituted or unsubstituted monocyclic ring nor the substituted or unsubstituted fused ring in the formula (1D) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and

in the compound represented by the formula (1C), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

In an exemplary arrangement of the organic electroluminescence according to the exemplary embodiment, at least one of $R_{21}$ to $R_{30}$ in the formula (1D) not being the bonding position to the benz[a]anthracene ring in the formula (1C) and forming neither the substituted or unsubstituted monocyclic ring nor the substituted or unsubstituted fused ring is also preferably other than a hydrogen atom.

In an exemplary arrangement of the organic electroluminescence device according to the exemplary embodiment, at least one of $R_{21}$ to $R_{30}$ in the formula (1D) not being the bonding position to the benz[a]anthracene ring in the formula (1C) and forming neither the substituted or unsubstituted monocyclic ring nor the substituted or unsubstituted fused ring is a substituted or unsubstituted aryl group having 6 to 50 ring carbon atoms.

In an exemplary arrangement of the organic electroluminescence device according to the exemplary embodiment, at least one of $R_{21}$ to $R_{30}$ in the formula (1D) not being the bonding position to the benz[a]anthracene ring in the formula (1C) and forming neither the substituted or unsubstituted monocyclic ring nor the substituted or unsubstituted fused ring is a substituted or unsubstituted phenyl group.

In an exemplary arrangement of the organic electroluminescence device according to the exemplary embodiment, the group represented by the formula (1D) is also preferably a group represented by a formula (1D-1), (1D-2), or (1D-3) below.

[Formula 39]

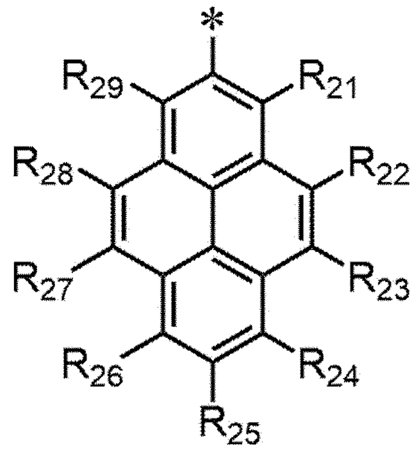

(1D-1)

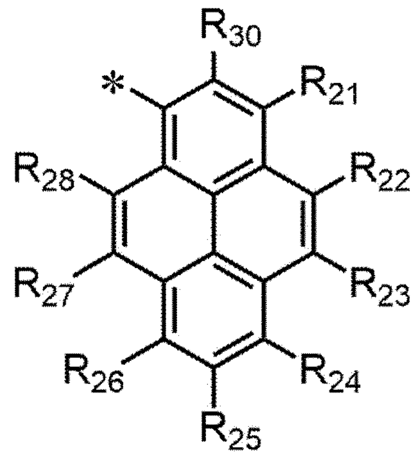

(1D-2)

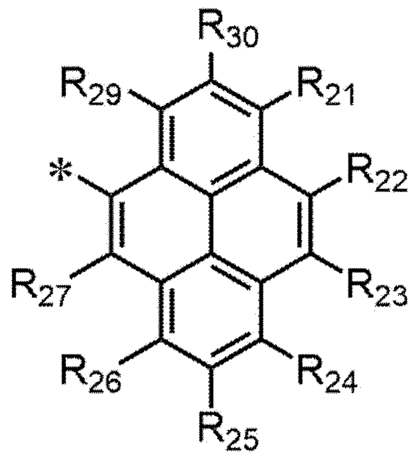

(1D-3)

In the formula (1D-1):

at least one combination of adjacent two or more of $R_{21}$ to $R_{29}$ are mutually bonded to form a substituted or unsubstituted monocyclic ring, mutually bonded to form a substituted or unsubstituted fused ring, or not mutually bonded; $R_{21}$ to $R_{29}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and

* represents a bonding position to the benz[a]anthracene ring in the formula (1C),

in the formula (1D-2):

at least one combination of adjacent two or more of $R_{21}$ to $R_{28}$, and $R_{30}$ are mutually bonded to form a substituted or unsubstituted monocyclic ring, mutually bonded to form a substituted or unsubstituted fused ring, or not mutually bonded;

$R_{21}$ to $R_{28}$ and $R_{30}$ forming neither the substituted or unsubstituted monocyclic ring nor the substituted or unsubstituted fused ring each independently represent the same as $R_{21}$ to $R_{29}$ in the formula (1D-1); and

* represents a bonding position to the benz[a]anthracene ring in the formula (1C),
in the formula (1D-3):

at least one combination of adjacent two or more of $R_{21}$ to $R_{27}$, $R_{29}$, and $R_{30}$ are mutually bonded to form a substituted or unsubstituted monocyclic ring, mutually bonded to form a substituted or unsubstituted fused ring, or not mutually bonded;
$R_{21}$ to $R_{27}$, $R_{29}$, and $R_{30}$ forming neither the substituted or unsubstituted monocyclic ring nor the substituted or unsubstituted fused ring each independently represent the same as $R_{21}$ to $R_{29}$ in the formula (1D-1); and
* represents a bonding position to the benz[a]anthracene ring in the formula (1C).

In an exemplary arrangement of the organic electroluminescence device according to the exemplary embodiment, at least one of $R_{21}$ to $R_{29}$ in the formula (1D-1), at least one of $R_{21}$ to $R_{28}$, or $R_{30}$ in the formula (1D-2), or at least one of $R_{21}$ to $R_{27}$, $R_{29}$, or $R_{30}$ in the formula (1D-3) forming neither the substituted or unsubstituted monocyclic ring nor the substituted or unsubstituted fused ring is also preferably other than a hydrogen atom.

In an exemplary arrangement of the organic electroluminescence device according to the exemplary embodiment, at least one of $R_{21}$ to $R_{29}$ in the formula (1D-1), at least one of $R_{21}$ to $R_{28}$, or $R_{30}$ in the formula (1D-2), or at least one of $R_{21}$ to $R_{27}$, $R_{29}$, or $R_{30}$ in the formula (1D-3) forming neither the substituted or unsubstituted monocyclic ring nor the substituted or unsubstituted fused ring is also preferably a substituted or unsubstituted aryl group having 6 to 50 ring carbon atoms.

In an exemplary arrangement of the organic electroluminescence device according to the exemplary embodiment, at least one of $R_{21}$ to $R_{29}$ in the formula (1D-1), at least one of $R_{21}$ to $R_{28}$, or $R_{30}$ in the formula (1D-2), or at least one of $R_{21}$ to $R_{27}$, $R_{29}$, or $R_{30}$ in the formula (1D-3) forming neither the substituted or unsubstituted monocyclic ring nor the substituted or unsubstituted fused ring is also preferably a substituted or unsubstituted phenyl group.

In an exemplary arrangement of the organic electroluminescence device according to the exemplary embodiment, the group represented by the formula (1D) is also preferably a group represented by the formula (1D-1).

In an exemplary arrangement of the organic electroluminescence device according to the exemplary embodiment, one of $R_5$, $R_6$, $R_7$, $R_8$, $R_{11}$, and $R_{12}$ in the compound represented by the formula (1C) is also preferably a group represented by the formula (1D).

In an exemplary arrangement of the organic electroluminescence device according to the exemplary embodiment, one of $R_6$, $R_7$, $R_{11}$, and $R_{12}$ in the compound represented by the formula (1C) is also preferably a group represented by the formula (1D).

In an exemplary arrangement of the organic electroluminescence device according to the exemplary embodiment, $R_{11}$ in the compound represented by the formula (1C) is also preferably a group represented by the formula (1D).

In an exemplary arrangement of the organic electroluminescence device according to the exemplary embodiment, $R_{12}$ in the compound represented by the formula (1C) is also preferably a group represented by the formula (1D).

In an exemplary arrangement of the organic electroluminescence device according to the exemplary embodiment, also preferably, in the compound represented by the formula (1C), $R_{11}$ is a group represented by the formula (1D) and $R_{12}$ is an unsubstituted phenyl group, or $R_{12}$ is a group represented by the formula (1D) and $R_{11}$ is an unsubstituted phenyl group.

In an exemplary arrangement of the organic electroluminescence device according to the exemplary embodiment, also preferably, $R_{11}$ is a group represented by the formula (1D) and $R_{12}$ is an unsubstituted phenyl group in the compound represented by the formula (1C).

In an exemplary arrangement of the organic electroluminescence device according to the exemplary embodiment, also preferably, $R_{12}$ is a group represented by the formula (1D) and $R_{11}$ is an unsubstituted phenyl group in the compound represented by the formula (1C).

In an exemplary arrangement of the organic electroluminescence device according to the exemplary embodiment, the compound represented by the formula (1C) is also preferably a compound represented by the formula (1C-1) below.

In an exemplary arrangement of the organic electroluminescence device according to the exemplary embodiment, the compound represented by the formula (1C) is also preferably a compound represented by the formula (1C-2) below.

[Formula 40]

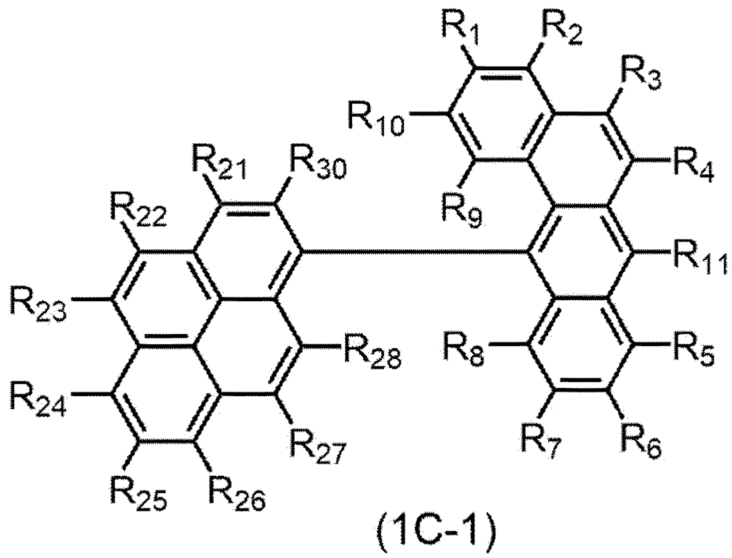

(1C-1)

[Formula 41]

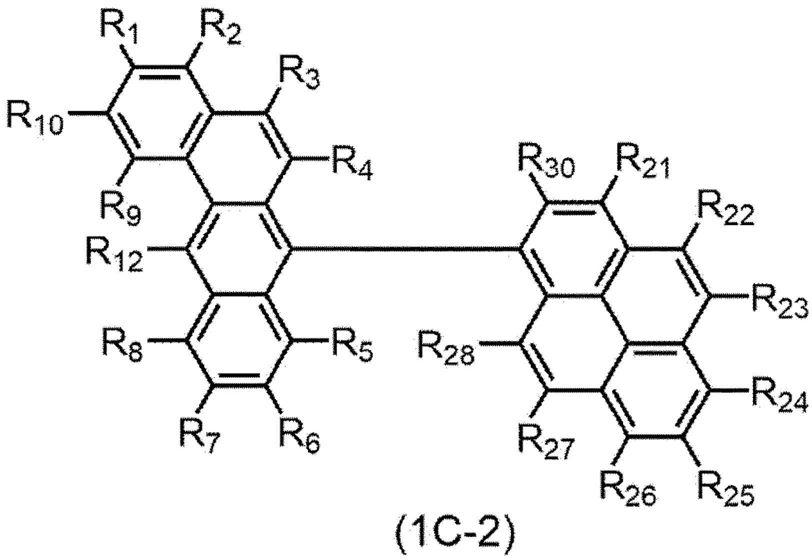

(1C-2)

$R_1$ to $R_{11}$ in the formula (1C-1) and $R_1$ to $R_{10}$ and $R_{12}$ in the formula (1C-2) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a substituted or unsubstituted aralkyl group having 7 to 50 carbon atoms, a group represented by -C(=O)$R_{801}$, a group represented by - COO$R_{802}$, a halogen atom, a cyano group, a nitro group, a substituted or unsubstituted aryl group having 6 to 50 ring carbon atoms, or a substituted or unsubstituted heterocyclic group having 5 to 50 ring atoms; and

$R_{21}$ to $R_{28}$ and $R_{30}$ in the formulae (1C-1) and (1C-2) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms.

In an exemplary arrangement of the organic electroluminescence device according to the exemplary embodiment, also preferably, $R_{12}$ is a group represented by the formula (1D) and $R_{11}$ is a hydrogen atom in the compound represented by the formula (1C).

In an exemplary arrangement of the organic electroluminescence device according to the exemplary embodiment, also preferably, $R_{12}$ is a group represented by the formula (1D) and $R_{11}$ is a substituted or unsubstituted phenyl group.

In an exemplary arrangement of the organic electroluminescence device according to the exemplary embodiment, also preferably, $R_1$ to $R_3$, $R_9$, and $R_4$ to $R_8$ and $R_{10}$ to $R_{12}$ not being the group represented by the formula (1D) in the compound represented by the formula (1C) are each independently a hydrogen atom, or a substituted or unsubstituted aryl group having 6 to 50 ring carbon atoms.

In an exemplary arrangement of the organic electroluminescence device according to the exemplary embodiment, also preferably, $R_{21}$ to $R_{30}$ in the formula (1D) not being the bonding position to the benz[a]anthracene ring in the formula (1C) and forming neither the substituted or unsubstituted monocyclic ring nor the substituted or unsubstituted fused ring are each independently a hydrogen atom, or a substituted or unsubstituted aryl group having 6 to 50 ring carbon atoms.

In an exemplary arrangement, the organic electroluminescence device according to the exemplary embodiment is also preferably a compound containing no hetero atom in a molecule.

In an exemplary arrangement of the organic electroluminescence device according to the exemplary embodiment, also preferably, the groups specified to be "substituted or unsubstituted" are each an unsubstituted group, and the rings specified to be "substituted or unsubstituted" are each an unsubstituted ring.

In an exemplary arrangement of the organic electroluminescence device according to the exemplary embodiment, $R_{12}$ in the compound represented by the formula (1C) is also preferably a hydrogen atom.

In an exemplary arrangement of the organic electroluminescence device according to the exemplary embodiment, also preferably, the compound represented by the formula (1C) has at least one deuterium atom in a molecule.

In an exemplary arrangement of the organic electroluminescence device according to the exemplary embodiment, also preferably, in the compound represented by the formula (1C), one or more of $R_1$ to $R_3$, and $R_9$ being the hydrogen atom, $R_4$ to $R_8$ and $R_{10}$ to $R_{12}$ not being the group represented by the formula (1D) but being the hydrogen atom, and $R_{21}$ to $R_{30}$ being the hydrogen atom are each also preferably a deuterium atom.

In an exemplary arrangement of the organic electroluminescence device according to the exemplary embodiment, also preferably, in the compound represented by the formula (1C), all of $R_1$ to $R_3$, and $R_9$ being the hydrogen atom, $R_4$ to $R_8$ and $R_{10}$ to $R_{12}$ not being the group represented by the formula (1D) but being the hydrogen atom, and $R_{21}$ to $R_{30}$ being the hydrogen atom are deuterium atoms.

In an exemplary arrangement of the organic electroluminescence device according to the exemplary embodiment, when $R_1$ to $R_3$, and $R_9$ not being the hydrogen atom, $R_4$ to $R_8$ and $R_{10}$ to $R_{12}$ being neither the group represented by the formula (1D) nor the hydrogen atom, and $R_{21}$ to $R_{30}$ not being the hydrogen atom, in the compound represented by the formula (1C), each have a hydrogen atom, one or more of those hydrogen atoms are each also preferably a deuterium atom.

In an exemplary arrangement of the organic electroluminescence device according to the exemplary embodiment, when $R_1$ to $R_3$, and $R_9$ not being the hydrogen atom, $R_4$ to $R_8$ and $R_{10}$ to $R_{12}$ being neither the group represented by the formula (1D) nor the hydrogen atom, and $R_{21}$ to $R_{30}$ not being the hydrogen atom, in the compound represented by the formula (1C), each have a hydrogen atom, all of those hydrogen atoms are also preferably deuterium atoms.

In an exemplary arrangement of the organic electroluminescence device according to the exemplary embodiment, $R_4$, $R_5$, $R_6$, $R_7$, $R_8$, $R_{10}$, $R_{11}$, or $R_{12}$ in the compound represented by the formula (1C) is a group represented by the formula (1D).

In an exemplary arrangement of the organic electroluminescence device according to the exemplary embodiment, $R_4$, $R_5$, $R_6$, $R_7$, $R_8$, $R_{10}$, $R_{11}$, or $R_{12}$ in the compound represented by the formula (1C-A) is a group represented by the formula (1D-A).

In an exemplary arrangement of the organic electroluminescence device according to the exemplary embodiment, $R_4$, $R_5$, $R_6$, $R_8$, $R_{10}$, $R_{11}$, or $R_{12}$ in the compound represented by the formula (1C-B) is a group represented by the formula (1D-B).

In an exemplary arrangement of the organic electroluminescence device according to the exemplary embodiment, $R_4$, $R_5$, $R_6$, $R_7$, $R_8$, $R_{10}$, $R_{11}$, or $R_{12}$ in the compound represented by the formula (1C-C) is a group represented by the formula (1D-C).

When $R_4$, $R_5$, $R_6$, $R_7$, $R_8$, $R_{10}$, $R_{11}$, or $R_{12}$ at a position of the HOMO and LUMO with a larger electron density or at a site with a smaller energy $S_1$ is a group selected from the group consisting of a group represented by the formula (1D), a group represented by the formula (1D-A), and a group represented by the formula (1D-C), excitation resistance is easily improvable and the lifetime of the organic EL device is easily extendable.

When $R_4$, $R_5$, $R_6$, $R_8$, $R_{10}$, $R_{11}$, or $R_{12}$ at a position of the HOMO and LUMO with a larger electron density or

at a site with a smaller energy $S_1$ is a group represented by the formula (1D-B), excitation resistance is easily improvable and the lifetime of the organic EL device is easily extendable.

The reason why the lifetime of the organic EL device is extendable is assumed as follows: when the first compound used for the first emitting layer has a structure in which a benz[a]anthracene ring is directly bonded to a group represented by the formula (1D), a group represented by the formula (1D-A), a group represented by the formula (1D-B), or a group represented by the formula (1D-C), a triplet energy $T_1$ is distributed both to the benz[a]anthracene ring and to a pyrene skeleton of the group represented by the formula (1D), the group represented by the formula (1D-A), the group represented by the formula (1D-B), or the group represented by the formula (1D-C).

Method of Producing Compound Represented by Formula (1C)

The compound represented by the formula (1C) can be produced in accordance with a synthesis method described later in Examples. Further, the compound represented by the formula (1C) can be produced by application of known substitution reactions and materials tailored for the target compound, in accordance with the synthesis method described later in Examples.

Specific Examples of Compound Represented by Formula (1C)

Specific examples of the compound represented by the formula (1C) include compounds below. However, the invention is by no means limited to the specific examples. In the chemical formulae herein, a deuterium atom is denoted by D, and a protium atom is denoted by H or description thereof is omitted.

[Formula 42]

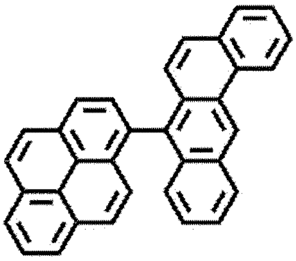

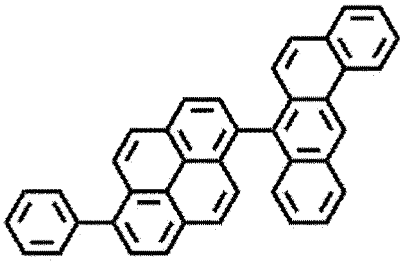

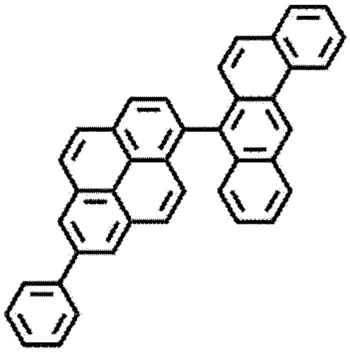

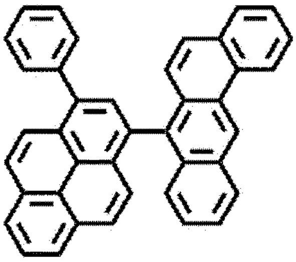

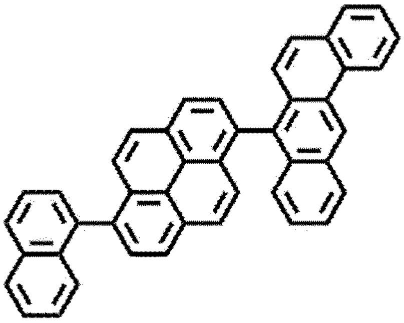

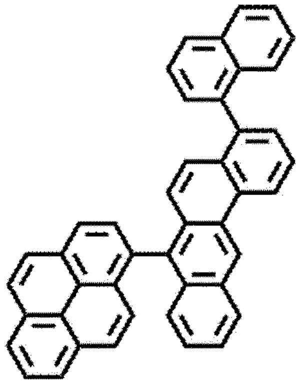

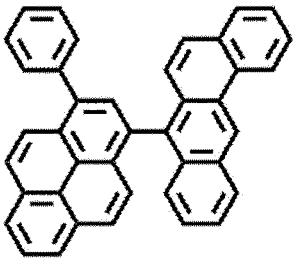

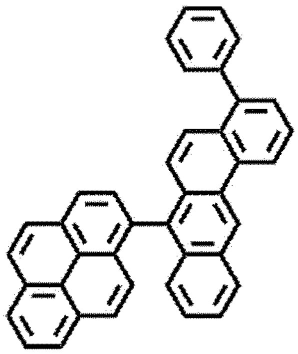

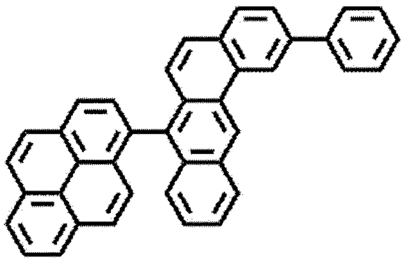

[Formula 43]

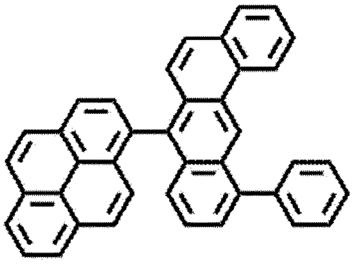
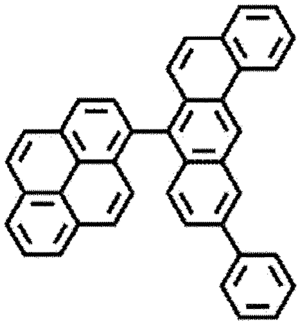
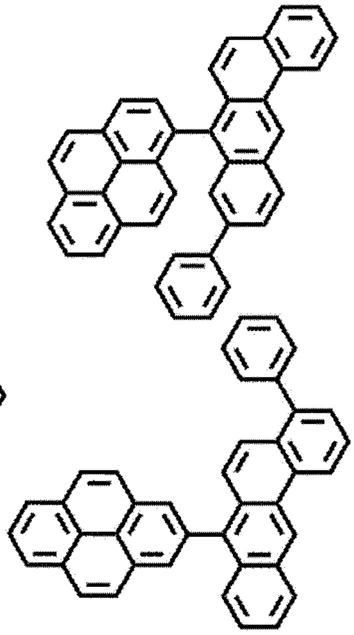
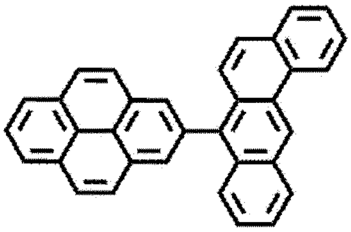
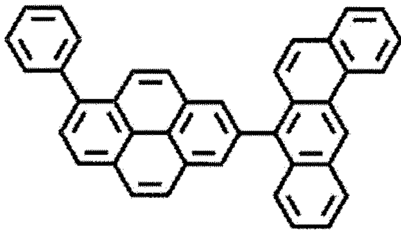

[Formula 44]

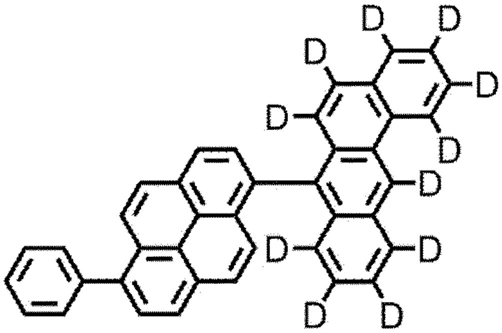

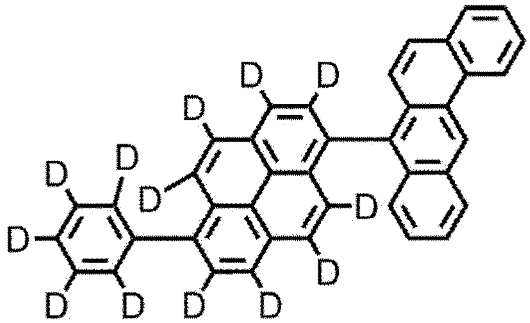

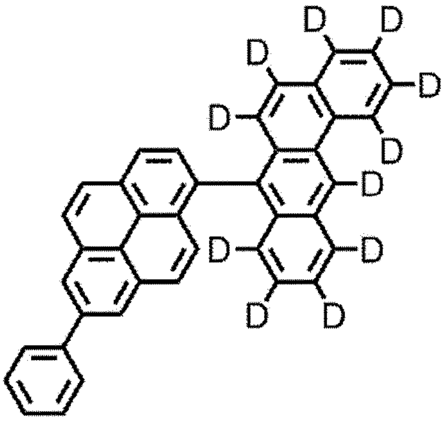

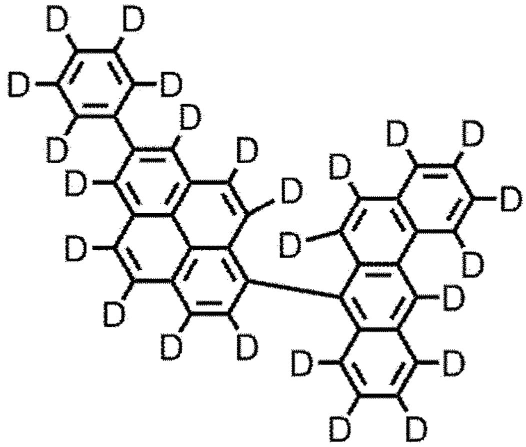

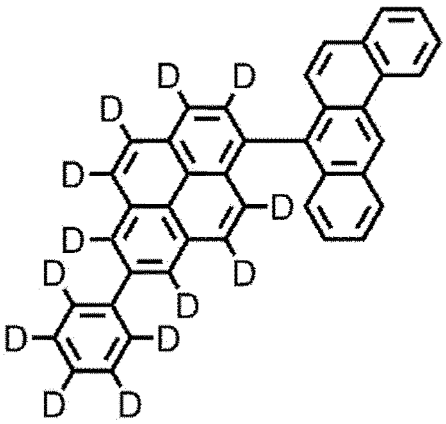

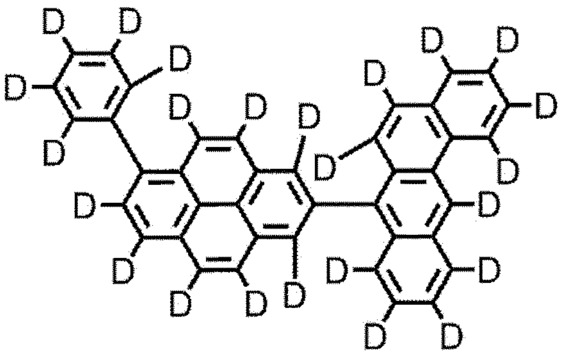

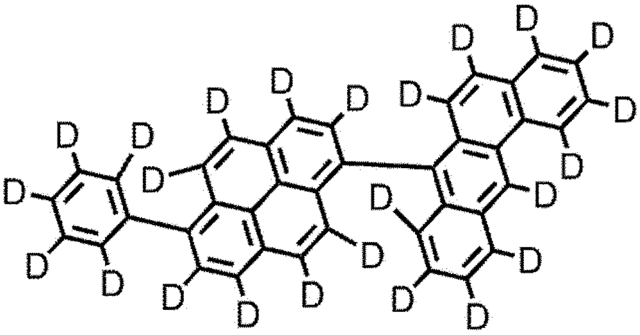

[Formula 45]

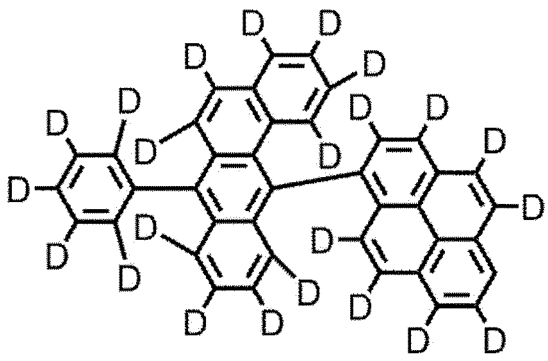

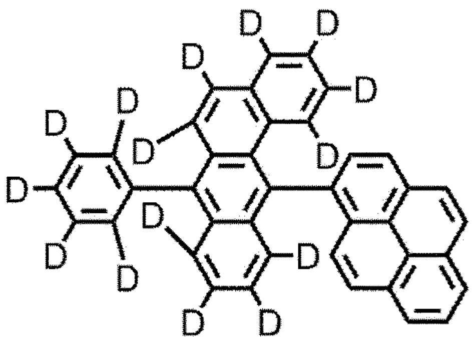

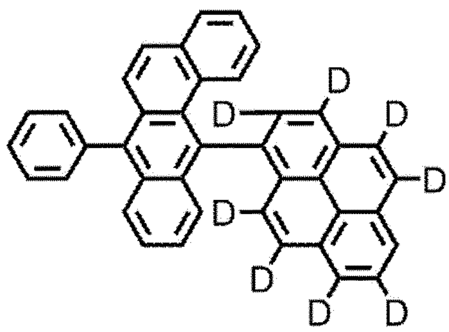

[Formula 46]

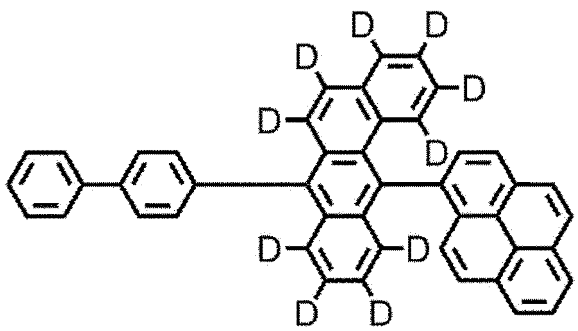

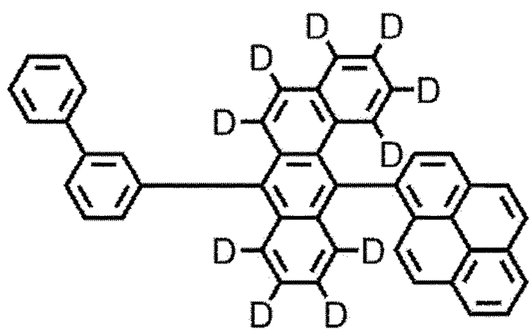

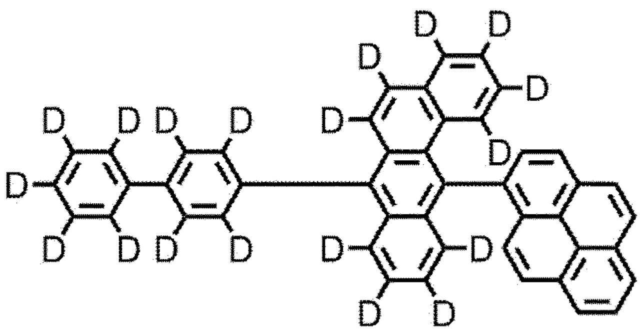

[Formula 47]

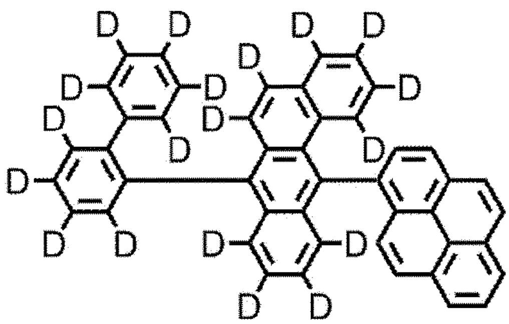

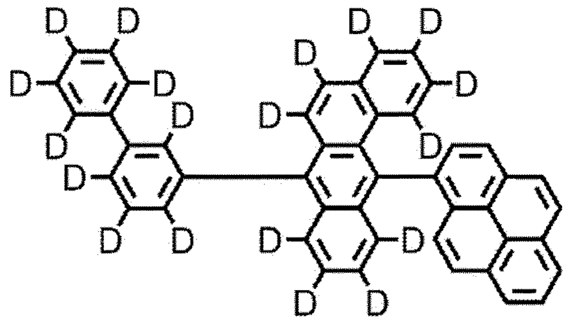

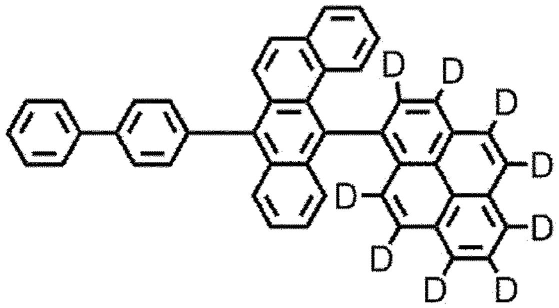

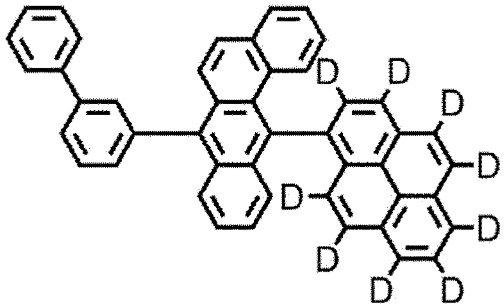

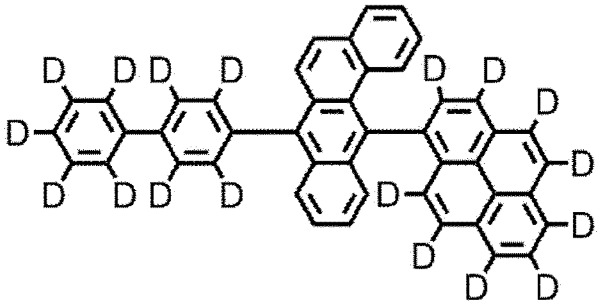

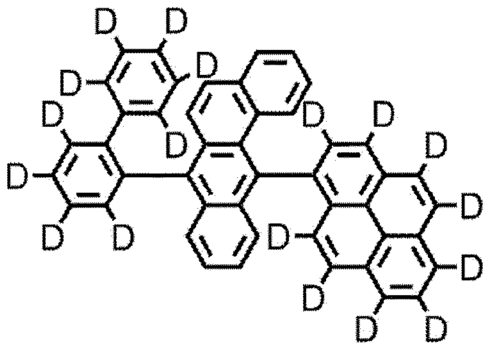

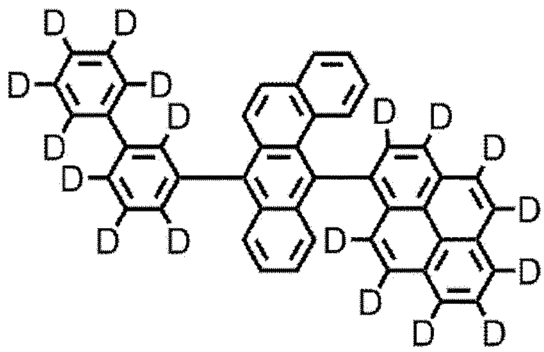

[Formula 48]

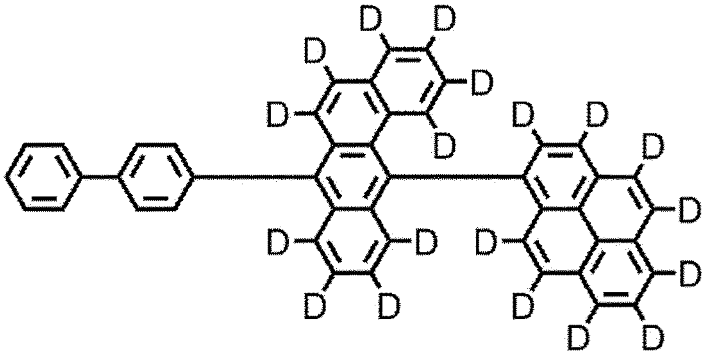

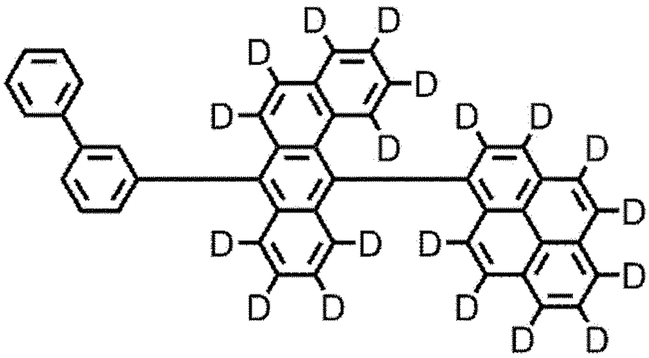

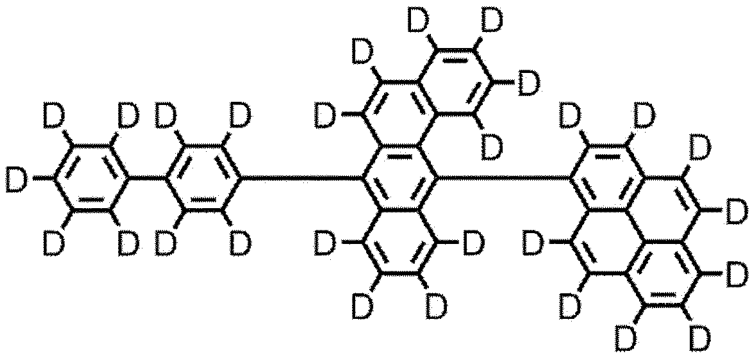

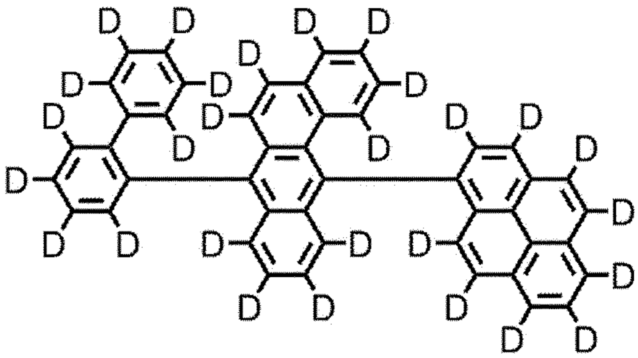

[Formula 49]

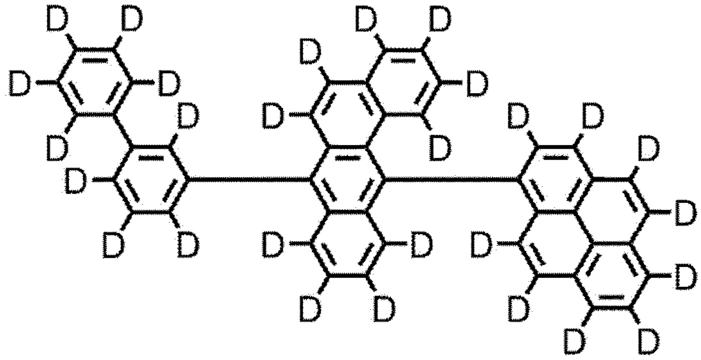

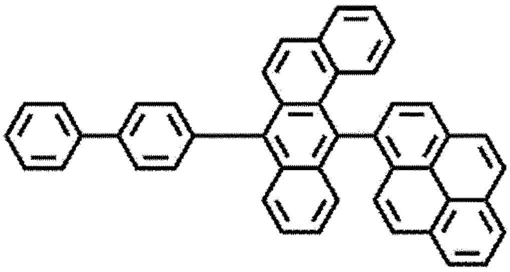

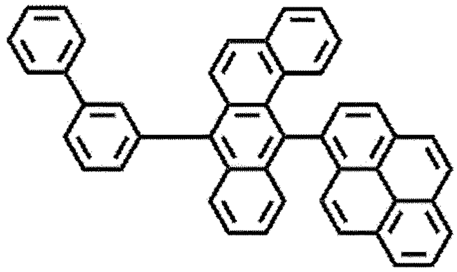

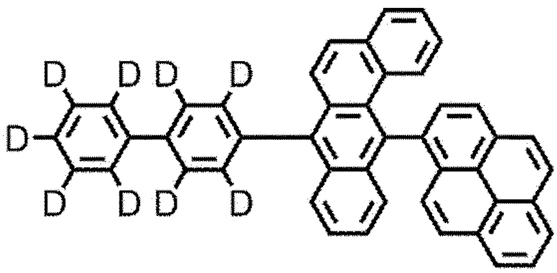

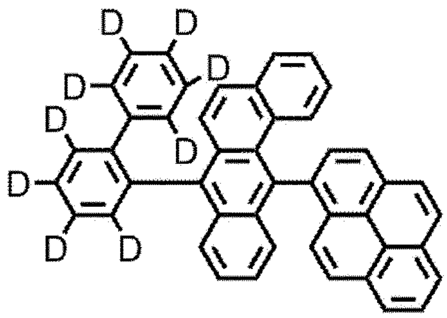

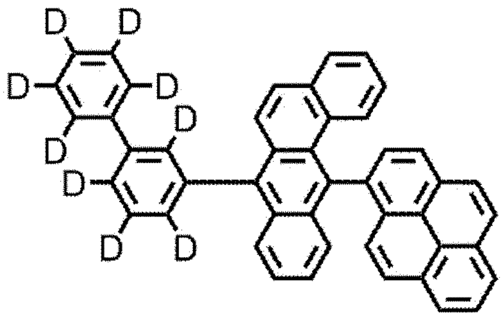

[Formula 50]

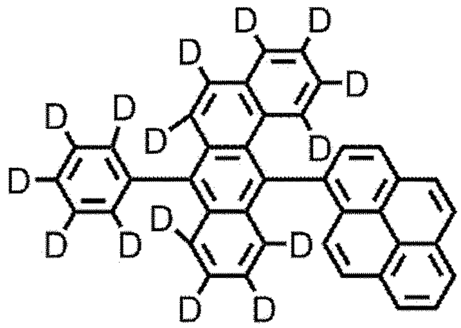

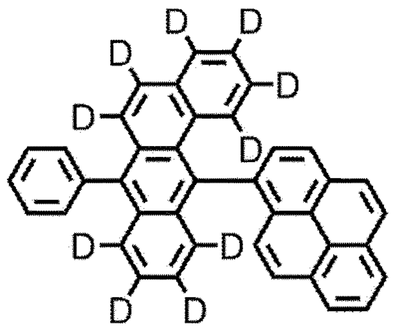

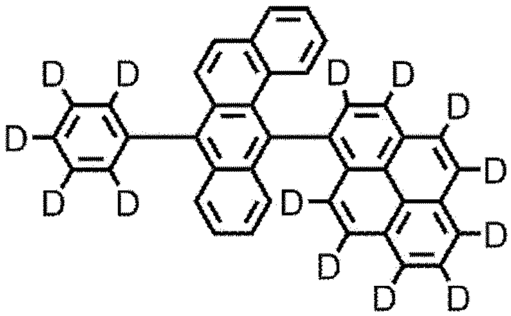

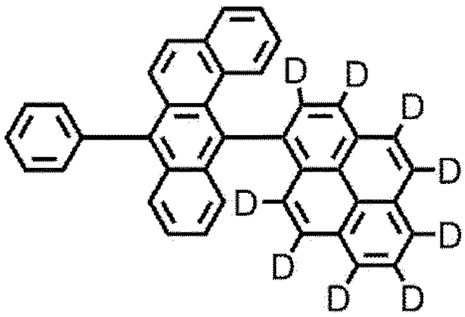

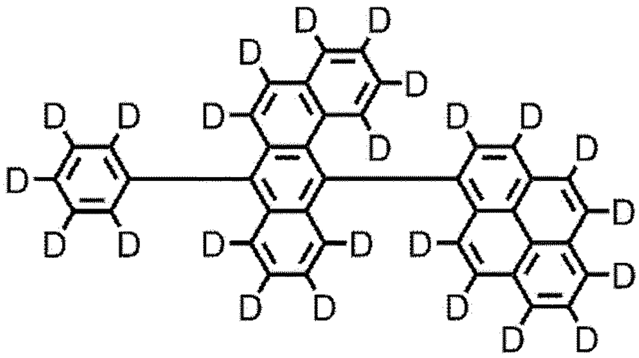

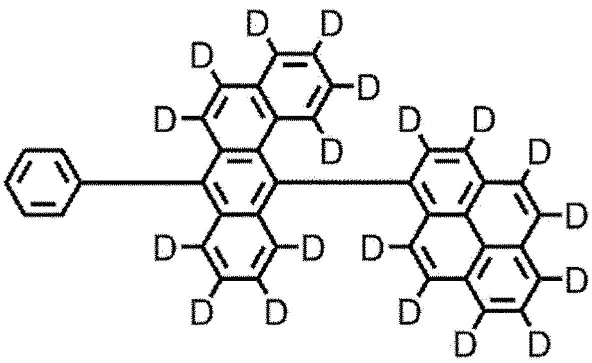

[Formula 51]

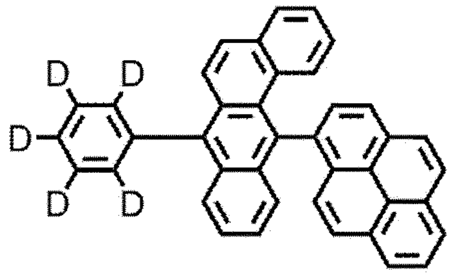
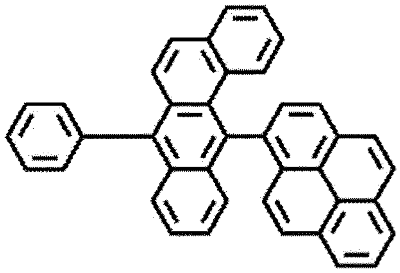

[Formula 52]

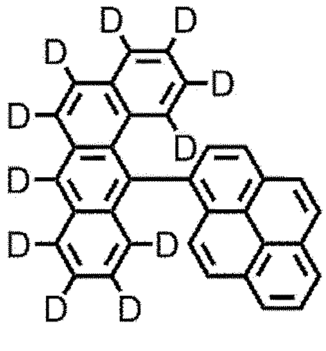
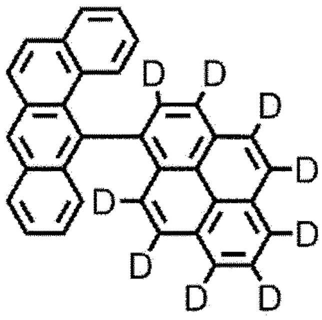
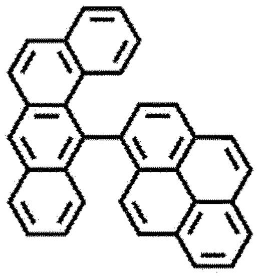

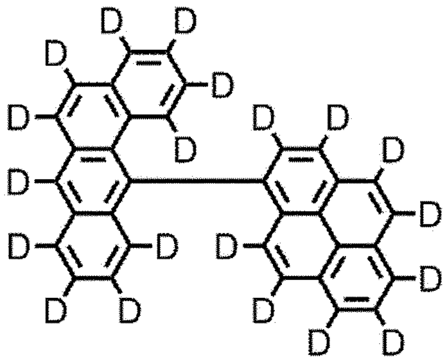
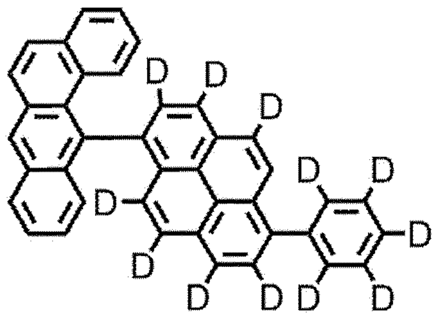

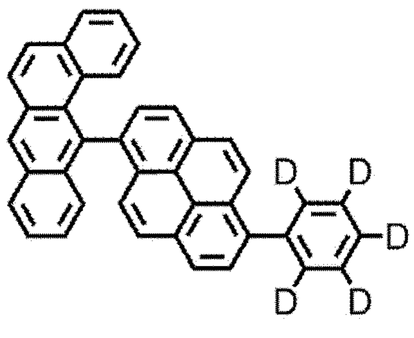
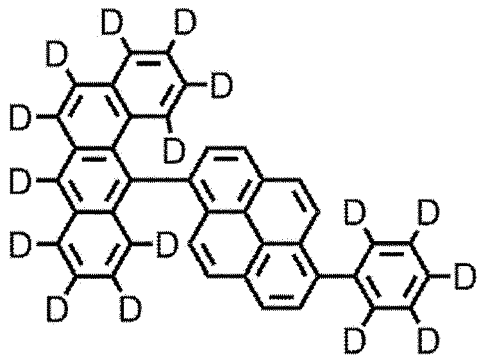

[Formula 53]

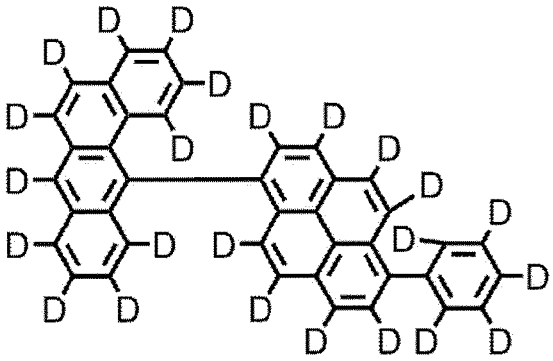

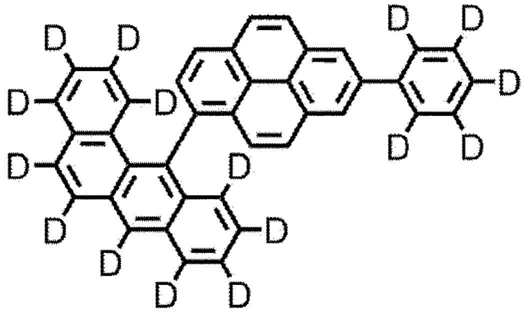

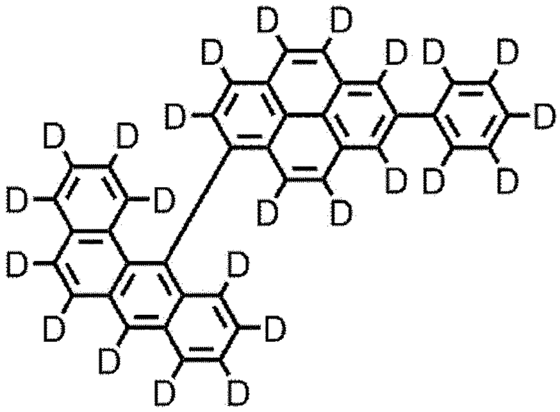

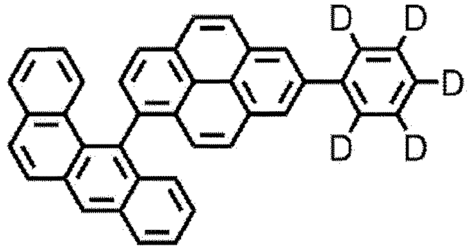

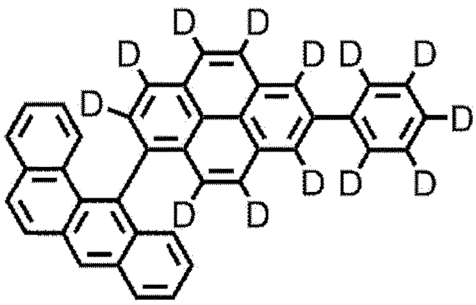

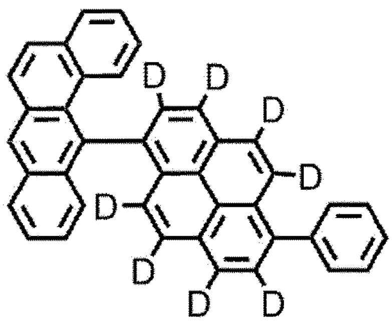

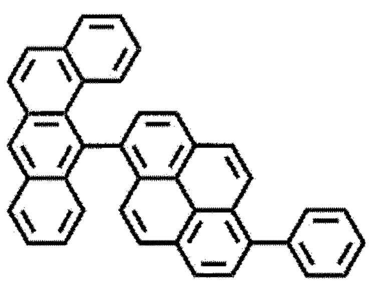

[Formula 54]

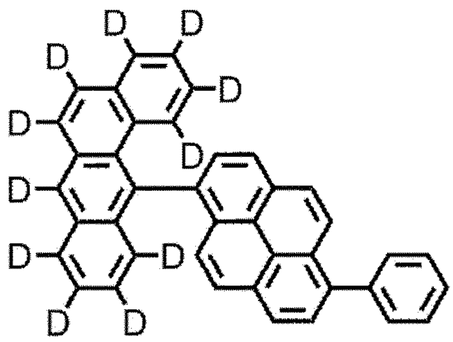

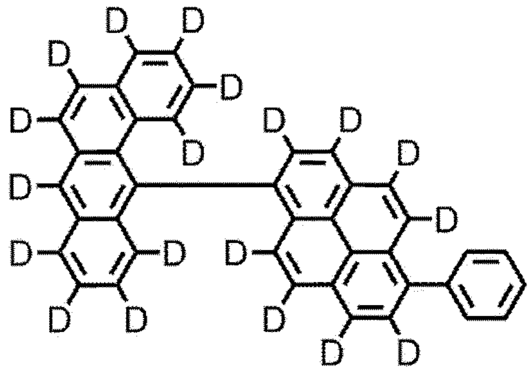

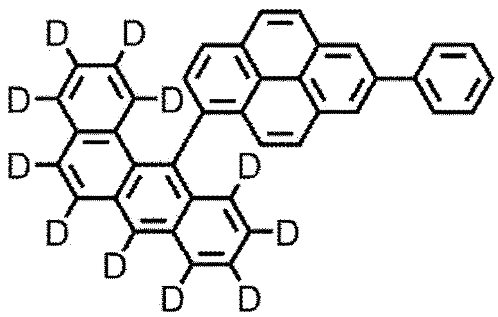

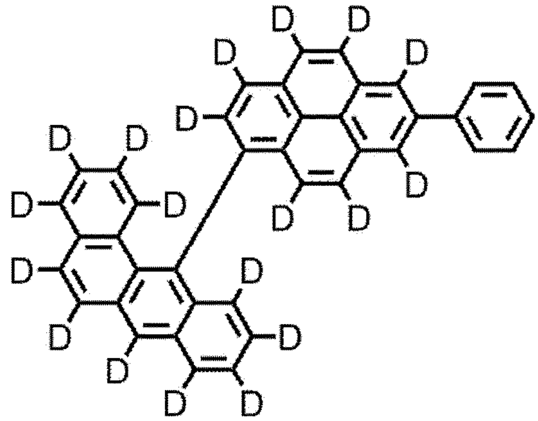

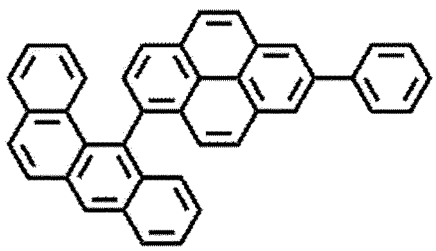

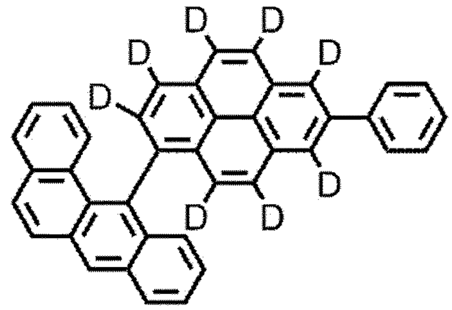

[Formula 55]

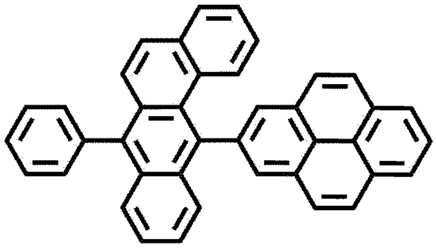

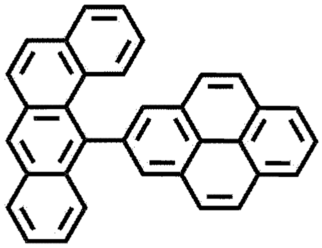

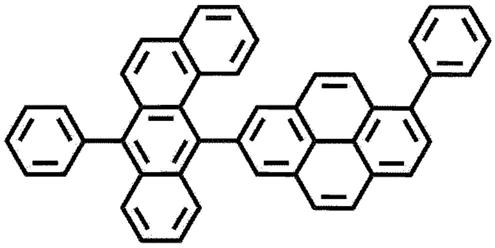

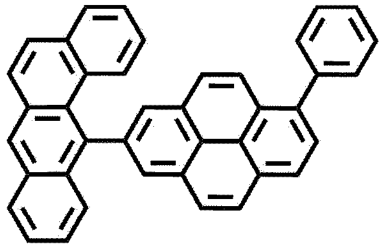

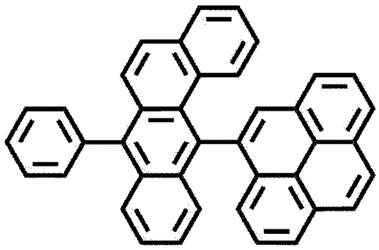

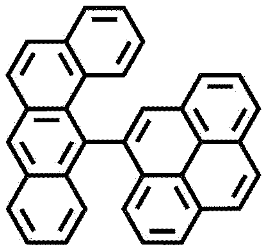

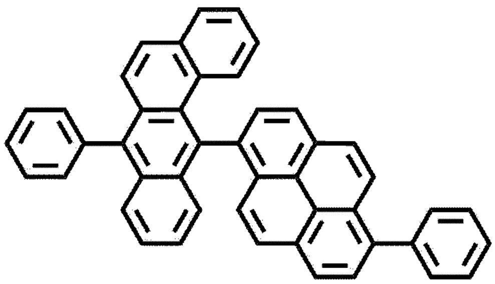

[Formula 56]

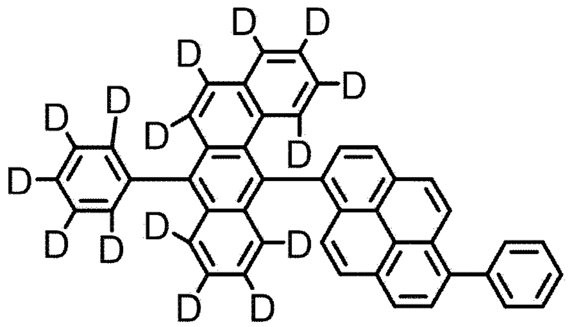

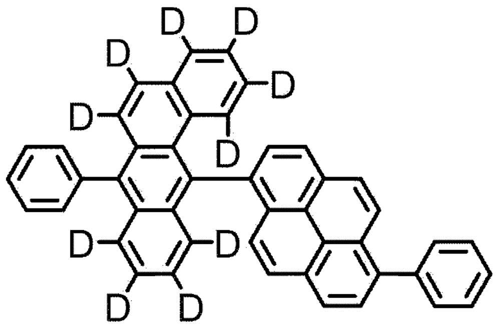

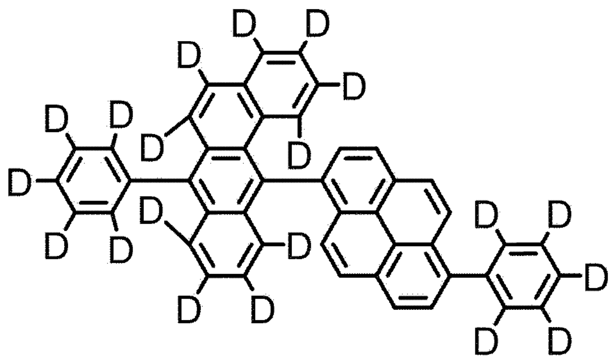

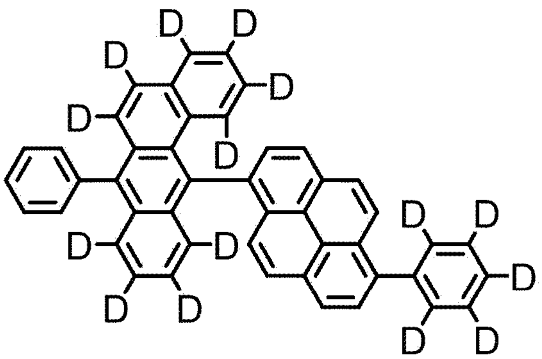

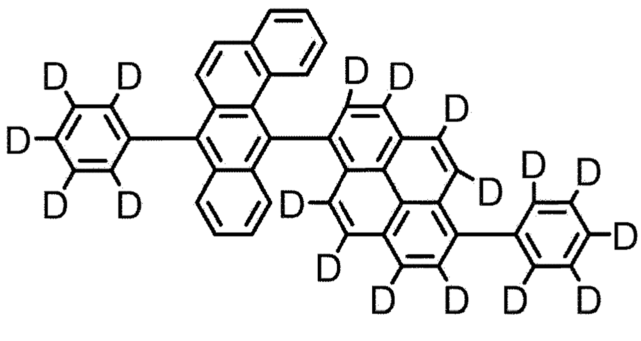

[Formula 57]

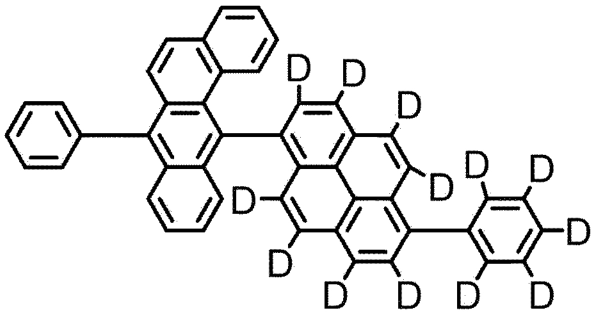

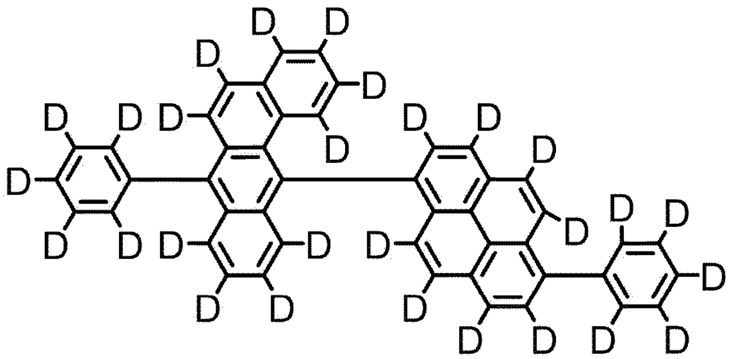

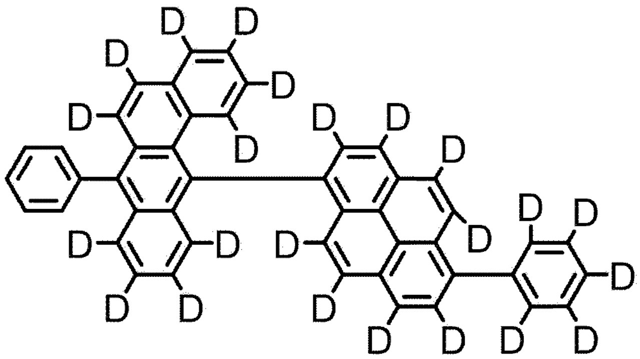

[Formula 58]

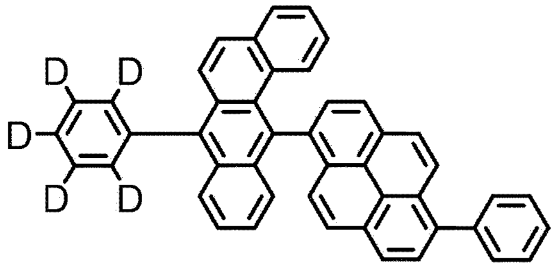

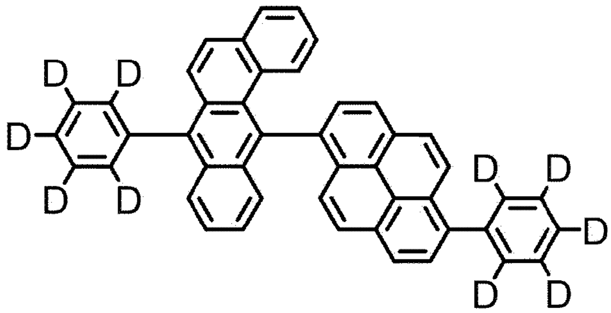

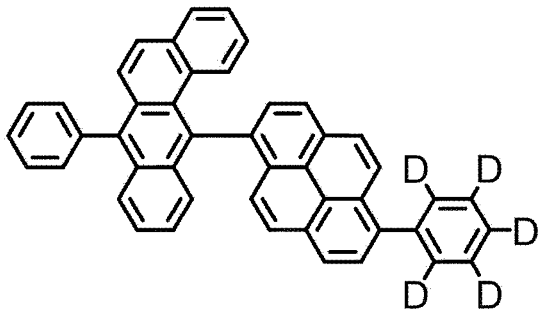

[Formula 59]

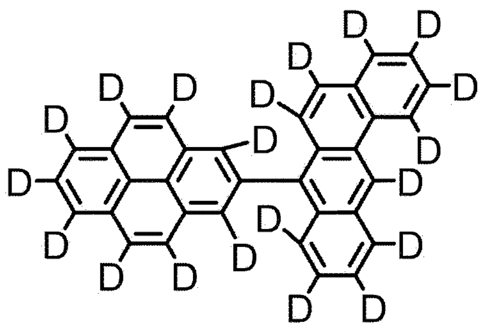

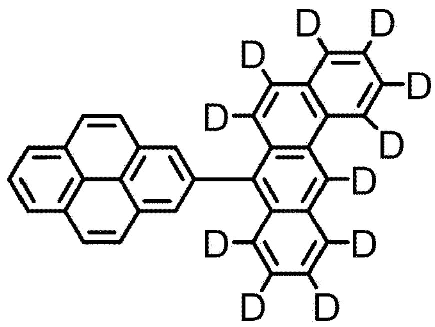

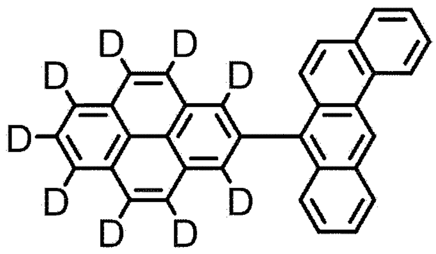

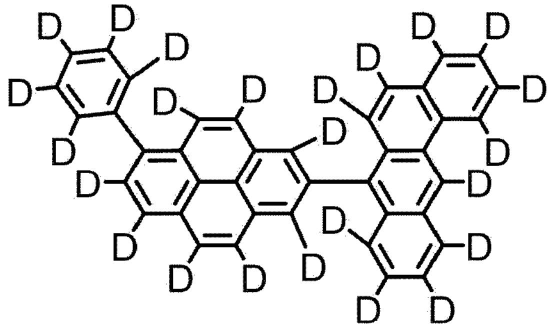

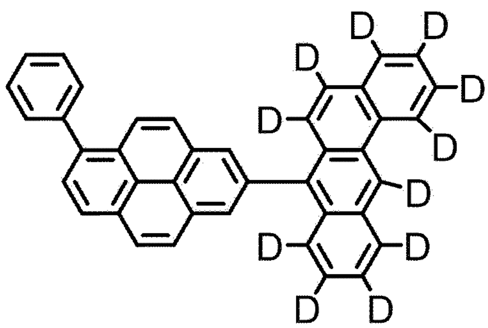

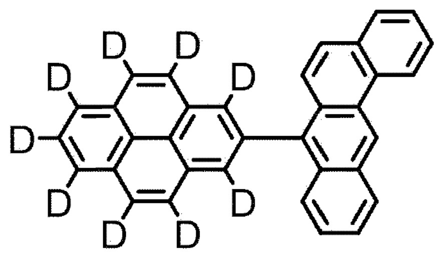

[Formula 60]

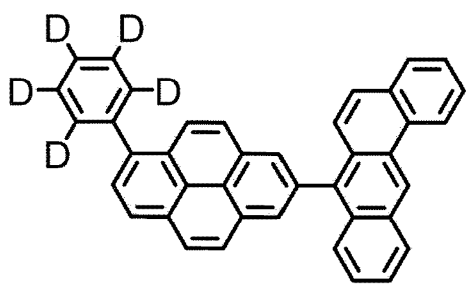

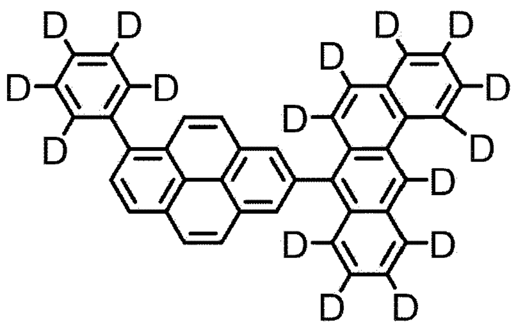

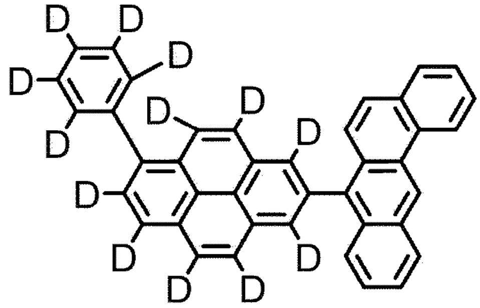

[Formula 61]

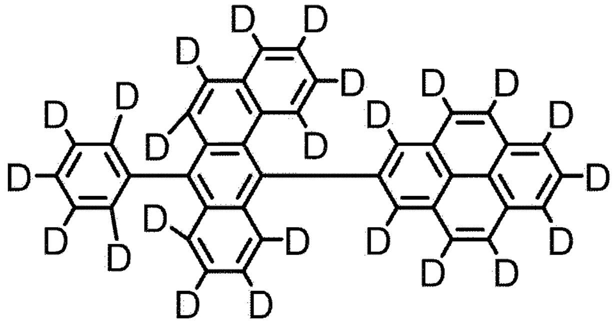

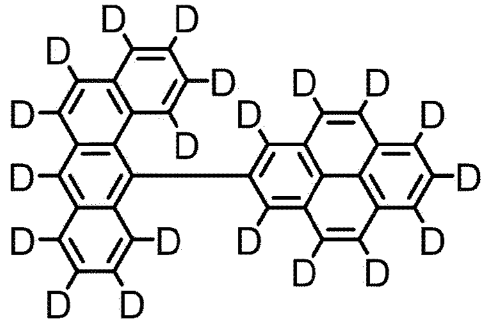

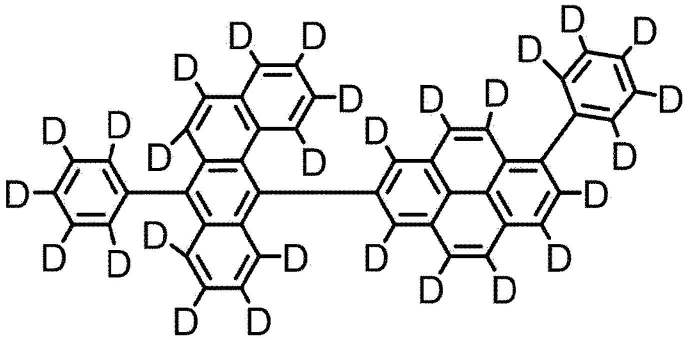

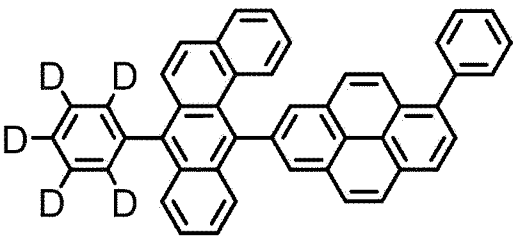

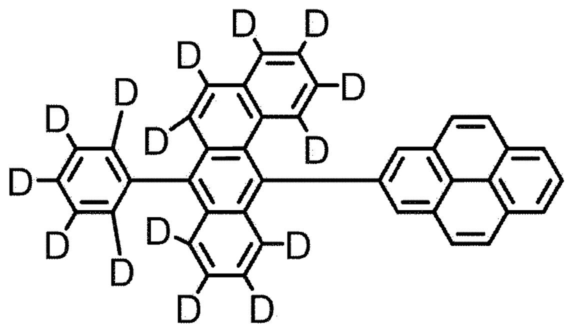

[Formula 62]

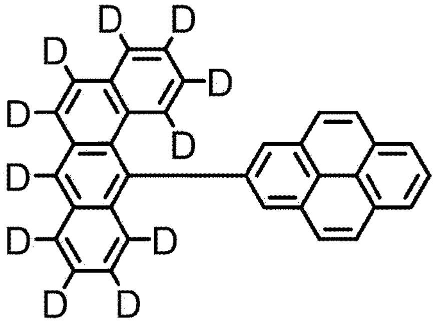

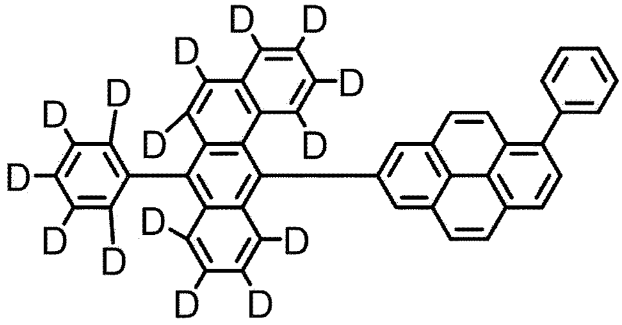

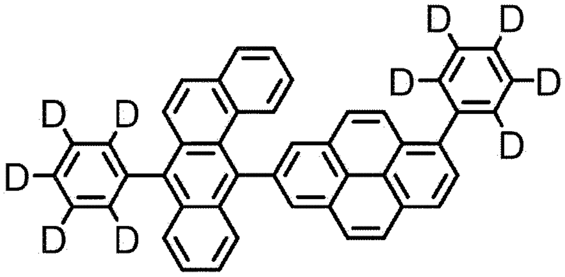

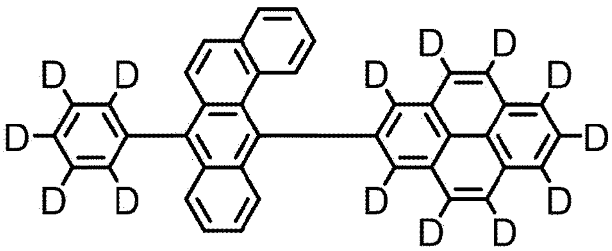

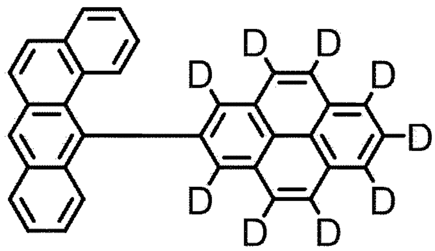

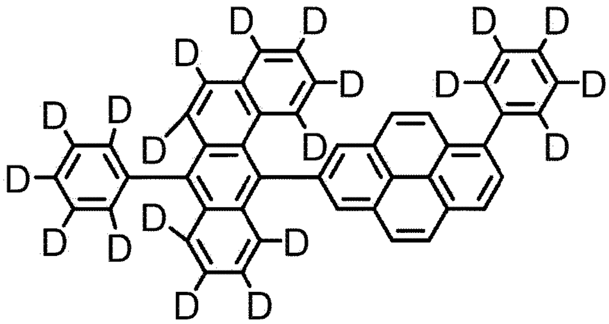

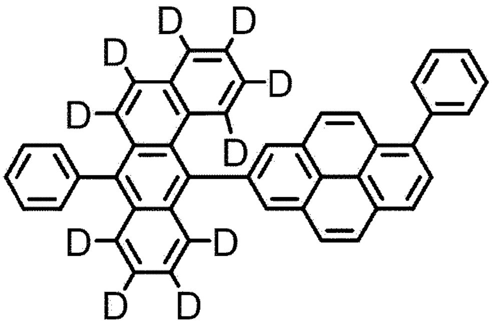

[Formula 63]

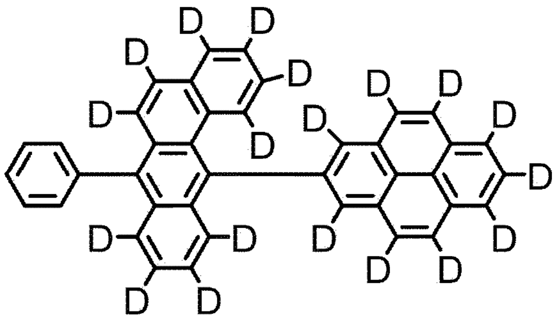

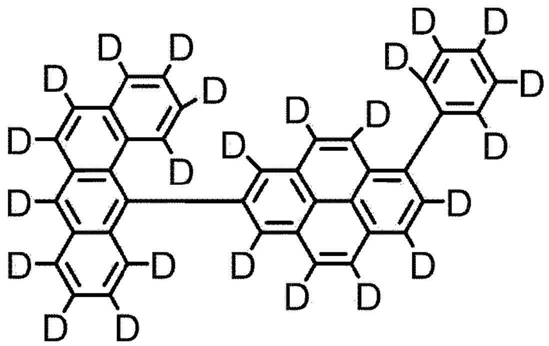

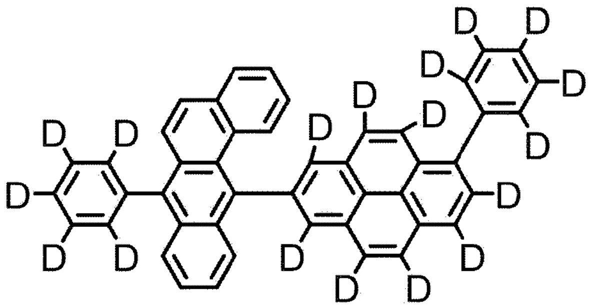

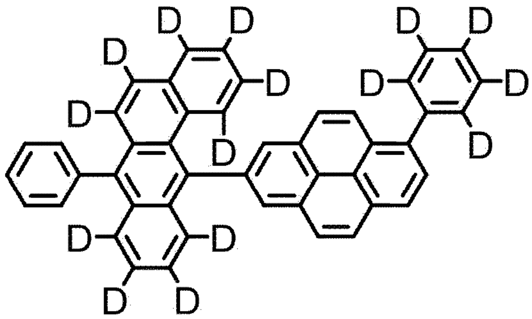

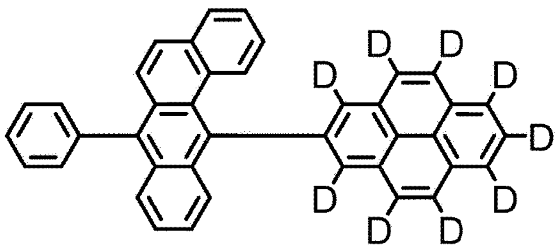

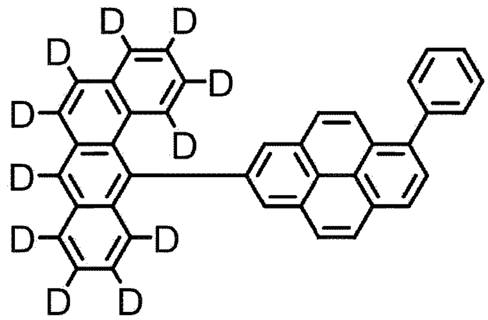

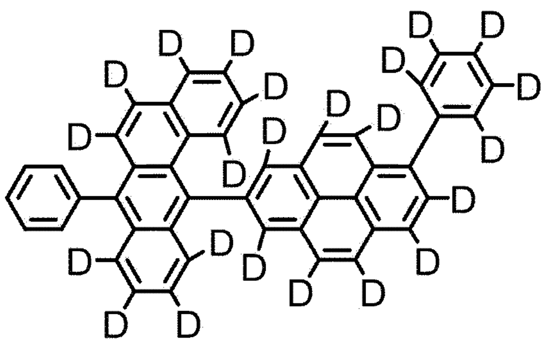

[Formula 64]

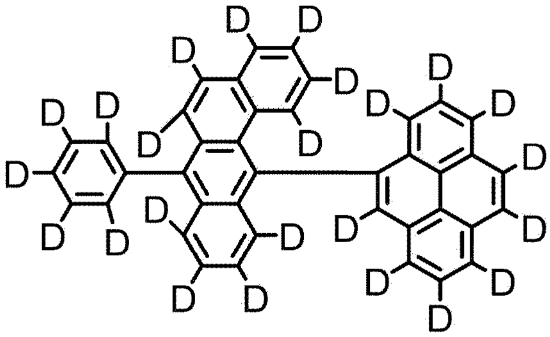

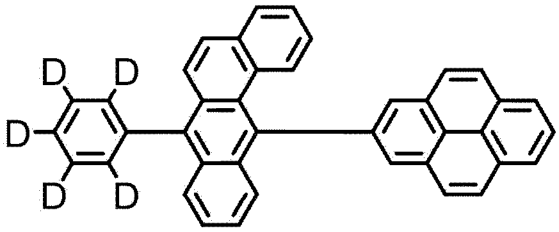

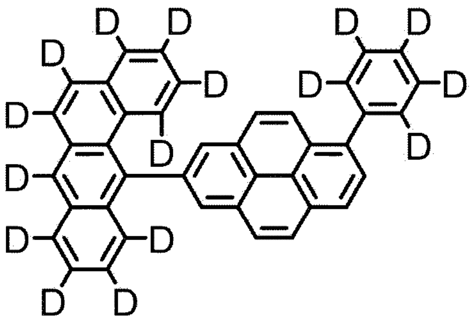

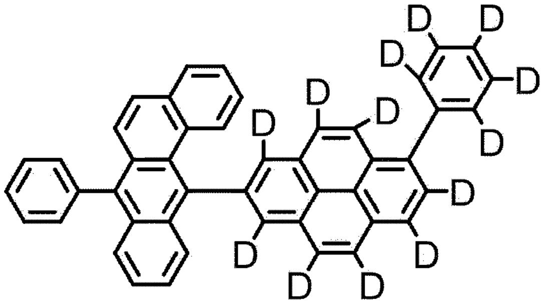

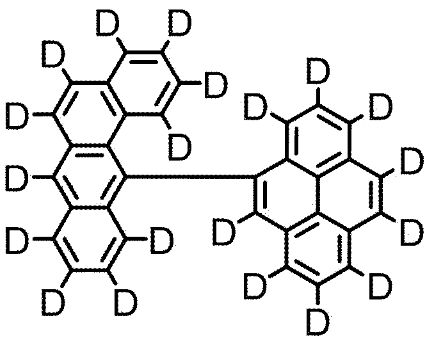

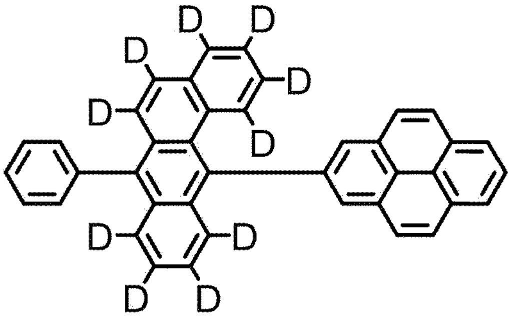

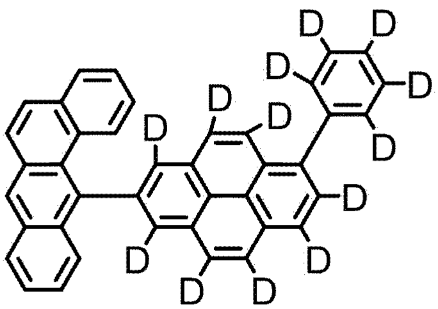

When the emitting region includes the first emitting layer and the second emitting layer, for instance, the anode, the first emitting layer, the second emitting layer, and the cathode may be provided in this order in the organic EL device according to the exemplary embodiment. Alternatively, the order of laying the emitting layers may be reversed, and the anode, the second emitting layer, the first emitting layer, and the cathode may be provided in this order.

When the emitting region includes the first emitting layer and the second emitting layer, also preferably, the second emitting layer is provided between the anode and the cathode and the first emitting layer is provided between the anode and the second emitting layer in the organic EL device according to the exemplary embodiment.

When the emitting region includes the first emitting layer and the second emitting layer, also preferably, the first emitting layer is provided between the anode and the cathode and the second emitting layer is provided between the anode and the first emitting layer in the organic EL device according to the exemplary embodiment.

Emission Wavelength of Organic EL Device

The organic EL device according to the exemplary embodiment preferably emits light having a maximum peak wavelength of 500 nm or less when being driven, more preferably emits light having a maximum peak wavelength in a range from 430 nm to 480 nm.

The maximum peak wavelength of the light emitted from the organic EL device when being driven is measured as follows. Voltage is applied to the organic EL device such that a current density is 10 $mA/cm^2$, where spectral radiance spectrum is measured by a spectroradiometer CS-2000 (produced by Konica Minolta, Inc.). A peak wavelength of an emission spectrum, a luminous intensity of which is the maximum in the obtained spectral radiance spectrum, is measured and defined as the maximum peak wavelength (unit: nm).

A method of measuring the maximum peak wavelength of the compound herein is as follows. A toluene solution of a measurement target compound at a concentration ranging from $10^{-6}$ mol/L to $10^{-5}$ mol/L is prepared and put in a quartz cell. An emission spectrum (ordinate axis: luminous intensity, abscissa axis: wavelength) of the thus-obtained sample is measured at a normal temperature (300K). The emission spectrum can be measured using a spectrophotometer (apparatus name: F-7000) produced by Hitachi High-Tech Science Corporation. It should be noted that the apparatus for measuring the emission spectrum is not limited to the apparatus used herein.

A peak wavelength of the emission spectrum exhibiting the maximum luminous intensity is defined as the maximum peak wavelength. Herein, the maximum peak wavelength is occasionally referred to as a maximum fluorescence peak wavelength (FL-peak).

In the organic EL device according to the exemplary embodiment, the organic layer may consist of the emitting layer. Alternatively, the organic layer may further include, for instance, at least one layer selected from the group consisting of a hole injecting layer, a hole transporting layer, an electron injecting layer, an electron transporting layer, a hole blocking layer, and an electron blocking layer.

In the organic EL device according to the exemplary embodiment, the hole transporting layer is preferably provided between the anode and the emitting region.

In the organic EL device according to the exemplary embodiment, when the emitting region includes the first emitting layer and the second emitting layer that are layered in this order from a side close to the anode, the hole transporting layer is preferably provided between the anode and the first emitting layer. When the second emitting layer and the first emitting layer are layered in this order from the side close to the anode, the hole transporting layer is preferably provided between the anode and the second emitting layer.

In the organic EL device according to the exemplary embodiment, the electron transporting layer is preferably provided between the cathode and the emitting region.

In the organic EL device according to the exemplary embodiment, when the emitting region includes the first emitting layer and the second emitting layer that are layered in this order from a side close to the anode, the electron transporting layer is preferably provided between the cathode and the second emitting layer. When the second emitting layer and the first emitting layer are layered in this order from the side close to the anode, the electron transporting layer is preferably provided between the cathode and the first emitting layer.

Fig. 1 schematically depicts an exemplary arrangement of the organic EL device of the exemplary embodiment.

An organic EL device 1A depicted in Fig. 1 includes a substrate 2, an anode 3, a cathode 4, and an organic layer 10A disposed between the anode 3 and the cathode 4. The organic layer 10A includes a hole transporting zone 6, an emitting region 5A, and an electron transporting zone 7 that are layered on the anode 3 in this order. The hole transporting zone 6 includes a hole injecting layer 61 and a hole transporting layer 62 in this order from a side close to the anode 3. The emitting region 5A includes a single emitting layer 5. The electron transporting zone 7 includes an electron transporting layer 71 and an electron injecting layer 72 in this order from a side close to the emitting region 5A.

Emitting Layer

The emitting layer 5 contains a compound according to the first exemplary embodiment, the second exemplary embodiment, or the third exemplary embodiment.

In the organic EL device 1A, the compound contained in the emitting layer 5 is preferably a compound represented by the formula (1C-A).

In the organic EL device 1A, the compound contained in the emitting layer 5 is also preferably a compound represented by the formula (1C-B).

In the organic EL device 1A, the compound contained in the emitting layer 5 is also preferably a compound represented by the formula (1C-C).

Luminescent Compound

In the organic EL device 1A, also preferably, the emitting layer 5 further contains a luminescent compound (preferably a fluorescent compound).

The luminescent compound contained in the emitting layer 5 may be at least one compound selected from the group consisting of a compound represented by a formula (3), a compound represented by a formula (4), a compound represented by a formula (5), a compound represented by a formula (6), a compound represented by a formula (7), a compound represented by a formula (8), a compound represented by a formula (9), and a compound represented by a formula (10) below.

Compound Represented by Formula (3)

The compound represented by the formula (3) will be described below.

[Formula 65]

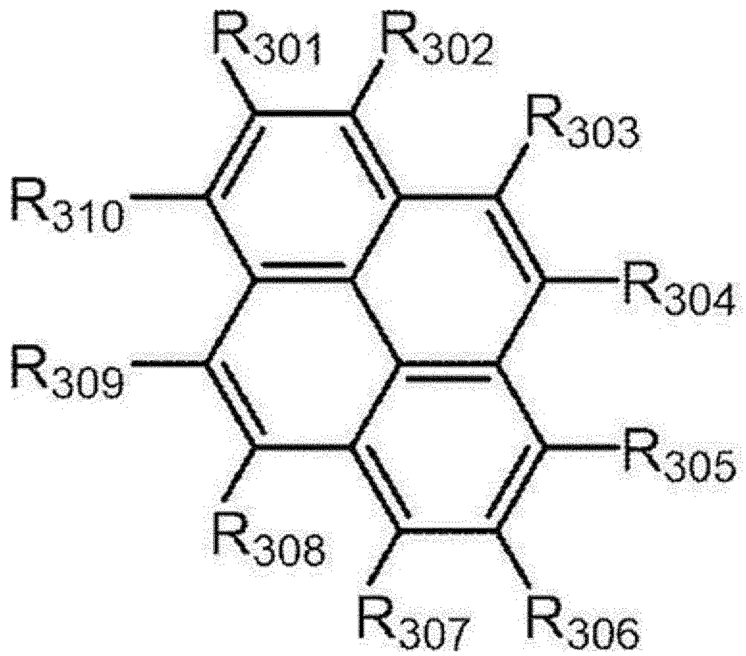

(3)

In the formula (3):

at least one combination of adjacent two or more of $R_{301}$ to $R_{310}$ are mutually bonded to form a substituted or unsubstituted monocyclic ring, mutually bonded to form a substituted or unsubstituted fused ring, or not mutually bonded;

at least one of $R_{301}$ to $R_{310}$ is a monovalent group represented by a formula (31) below; and

$R_{301}$ to $R_{310}$ forming neither the monocyclic ring nor the fused ring and not being the monovalent group represented by the formula (31) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

[Formula 66]

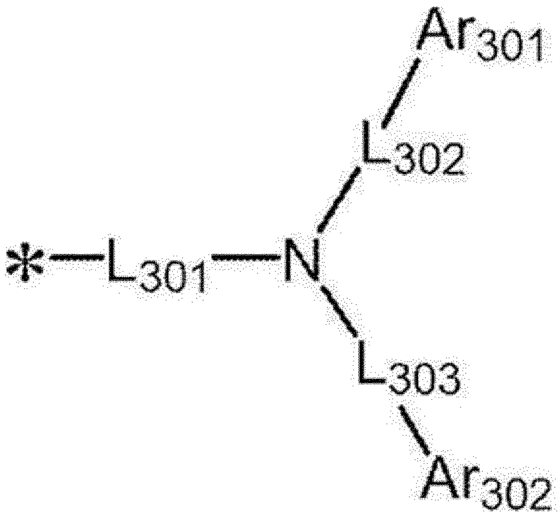

(31)

In the formula (31):

$Ar_{301}$ and $Ar_{302}$ are each independently a substituted or unsubstituted aryl group having 6 to 50 ring carbon atoms, or a substituted or unsubstituted heterocyclic group having 5 to 50 ring atoms;
$L_{301}$ to $L_{303}$ are each independently a single bond, a substituted or unsubstituted arylene group having 6 to 30 ring carbon atoms, or a substituted or unsubstituted divalent heterocyclic group having 5 to 30 ring atoms; and
* represents a bonding position to a pyrene ring in the formula (3).

In the luminescent compound, $R_{901}$, $R_{902}$, $R_{903}$, $R_{904}$, $R_{905}$, $R_{906}$ and $R_{907}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms, preferably, a substituted or unsubstituted alkyl group having 1 to 50 carbon atoms or a substituted or unsubstituted aryl group having 6 to 50 ring carbon atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different; and
when a plurality of $R_{907}$ are present, the plurality of $R_{907}$ are mutually the same or different.

In the formula (3), two of $R_{301}$ to $R_{310}$ are each preferably a group represented by the formula (31).
In an exemplary embodiment, the compound represented by the formula (3) is a compound represented by a formula (33) below.

[Formula 67]

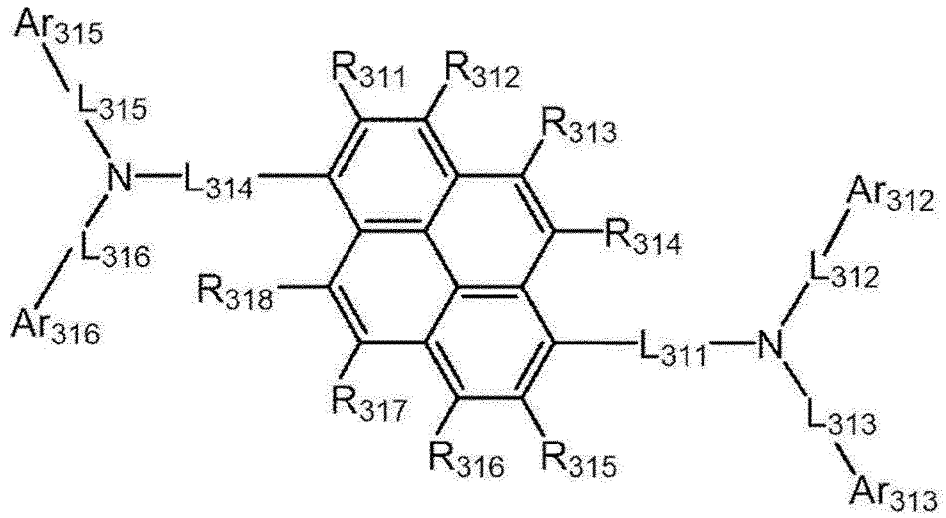

(33)

In the formula (33):

$R_{311}$ to $R_{318}$ each independently represent the same as $R_{301}$ to $R_{310}$ in the formula (3) not being the monovalent group represented by the formula (31);
$L_{311}$ to $L_{316}$ are each independently a single bond, a substituted or unsubstituted arylene group having 6 to 30 ring carbon atoms, or a substituted or unsubstituted divalent heterocyclic group having 5 to 30 ring atoms; and
$Ar_{312}$, $Ar_{313}$, $Ar_{315}$, and $Ar_{316}$ are each independently a substituted or unsubstituted aryl group having 6 to 50 ring carbon atoms, or a substituted or unsubstituted heterocyclic group having 5 to 50 ring atoms.

In the formula (31), $L_{301}$ is preferably a single bond, and $L_{302}$ and $L_{303}$ are each preferably a single bond.
In an exemplary embodiment, the compound represented by the formula (3) is represented by a formula (34) or a formula (35) below.

[Formula 68]

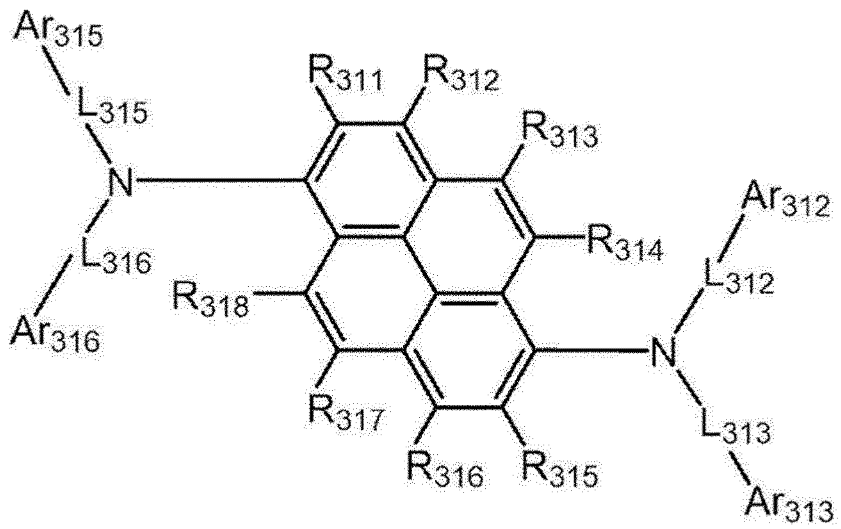

(34)

In the formula (34):

$R_{311}$ to $R_{318}$ each independently represent the same as $R_{301}$ to $R_{310}$ in the formula (3) not being the monovalent group represented by the formula (31);
$L_{312}$, $L_{313}$, $L_{315}$ and $L_{316}$ each independently represent the same as $L_{312}$, $L_{313}$, $L_{315}$ and $L_{316}$ in the formula (33); and
$Ar_{312}$, $Ar_{313}$, $Ar_{315}$ and $Ar_{316}$ each independently represent the same as $Ar_{312}$, $Ar_{313}$, $Ar_{315}$ and $Ar_{316}$ in the formula

(33).

[Formula 69]

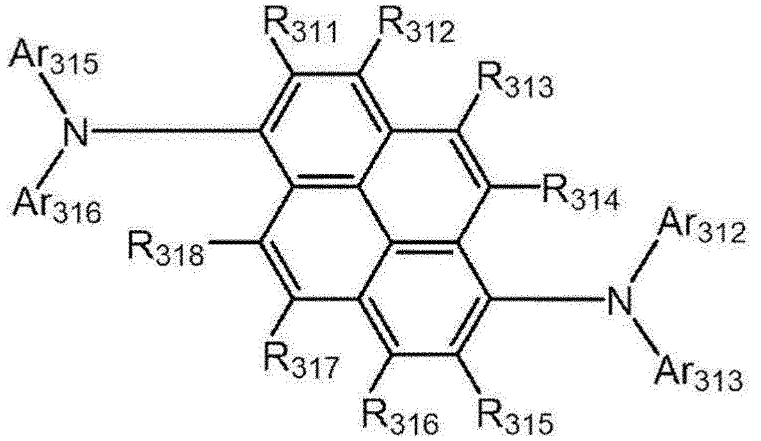

(35)

In the formula (35):

$R_{311}$ to $R_{318}$ each independently represent the same as $R_{301}$ to $R_{310}$ in the formula (3) not being the monovalent group represented by the formula (31); and

$Ar_{312}$, $Ar_{313}$, $Ar_{315}$ and $Ar_{316}$ each independently represent the same as $Ar_{312}$, $Ar_{313}$, $Ar_{315}$ and $Ar_{316}$ in the formula (33).

In the formula (31), at least one of $Ar_{301}$ or $Ar_{302}$ is preferably a group represented by a formula (36) below.

In the formulae (33) to (35), at least one of $Ar_{312}$ or $Ar_{313}$ is preferably a group represented by the formula (36).

In the formulae (33) to (35), at least one of $Ar_{315}$ or $Ar_{316}$ is preferably a group represented by the formula (36).

[Formula 70]

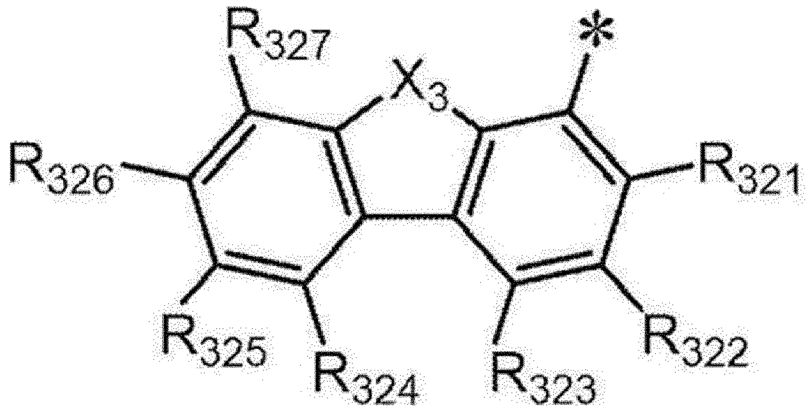

(36)

In the formula (36):

$X_3$ represents an oxygen atom or a sulfur atom;

at least one combination of adjacent two or more of $R_{321}$ to $R_{327}$ are mutually bonded to form a substituted or unsubstituted monocyclic ring, mutually bonded to form a substituted or unsubstituted fused ring, or not mutually bonded;

$R_{321}$ to $R_{327}$ forming neither the monocyclic ring nor the fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon

atoms, or a substituted or unsubstituted heterocyclic group having 5 to 50 ring atoms; and
* represents a bonding position to $L_{302}$, $L_{303}$, $L_{312}$, $L_{313}$, $L_{315}$ or $L_{316}$.

$X_3$ is preferably an oxygen atom.

At least one of $R_{321}$ to $R_{327}$ is preferably a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

In the formula (31), preferably, $Ar_{301}$ is a group represented by the formula (36) and $Ar_{302}$ is a substituted or unsubstituted aryl group having 6 to 50 ring carbon atoms.

In the formulae (33) to (35), preferably, $Ar_{312}$ is a group represented by the formula (36) and $Ar_{313}$ is a substituted or unsubstituted aryl group having 6 to 50 ring carbon atoms.

In the formulae (33) to (35), preferably, $Ar_{315}$ is a group represented by the formula (36) and $Ar_{316}$ is a substituted or unsubstituted aryl group having 6 to 50 ring carbon atoms.

In an exemplary embodiment, the compound represented by the formula (3) is represented by a formula (37) below.

[Formula 71]

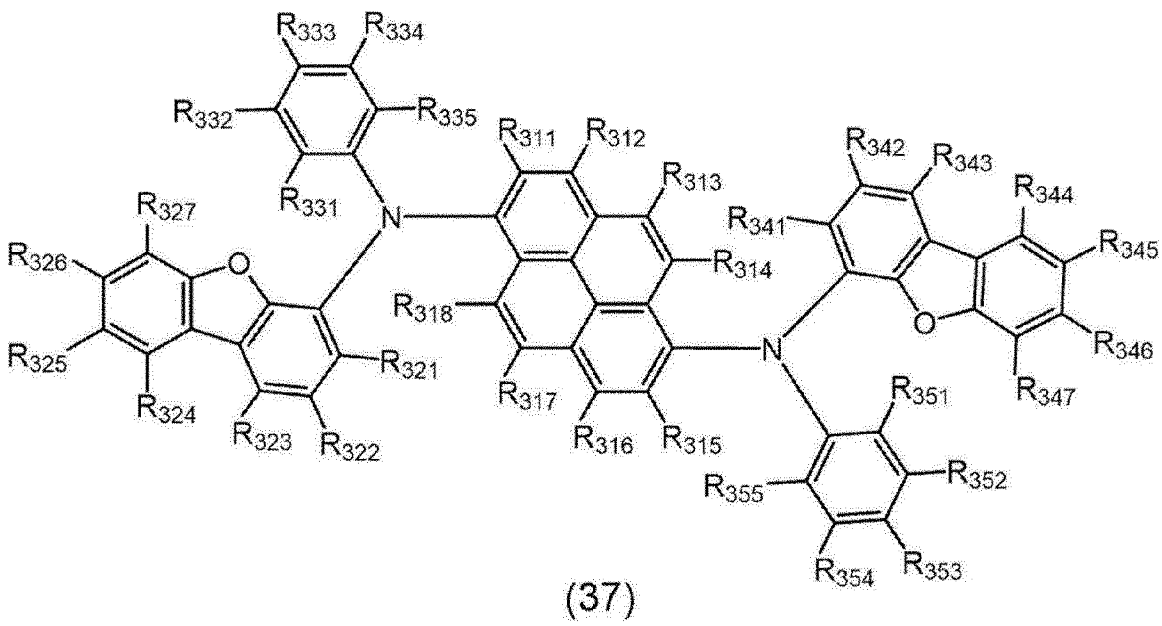

(37)

In the formula (37):

$R_{311}$ to $R_{318}$ each independently represent the same as $R_{301}$ to $R_{310}$ in the formula (3) not being the monovalent group represented by the formula (31);

at least one combination of adjacent two or more of $R_{321}$ to $R_{327}$ are mutually bonded to form a substituted or unsubstituted monocyclic ring, mutually bonded to form a substituted or unsubstituted fused ring, or not mutually bonded;

at least one combination of adjacent two or more of $R_{341}$ to $R_{347}$ are mutually bonded to form a substituted or unsubstituted monocyclic ring, mutually bonded to form a substituted or unsubstituted fused ring, or not mutually bonded;

$R_{321}$ to $R_{327}$ and $R_{341}$ to $R_{347}$ forming neither the monocyclic ring nor the fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to

50 ring carbon atoms, or a substituted or unsubstituted heterocyclic group having 5 to 50 ring atoms; and

$R_{331}$ to $R_{335}$ and $R_{351}$ to $R_{355}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by - S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms.

Specific examples of the compound represented by the formula (3) include compounds given below.

[Formula 72]

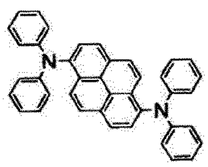
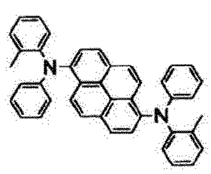
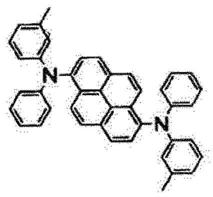
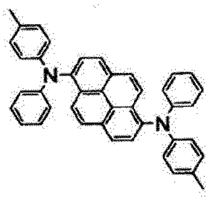
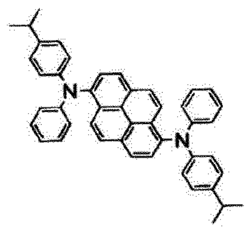
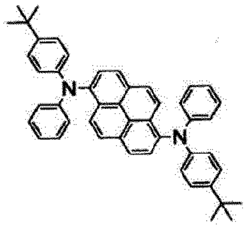
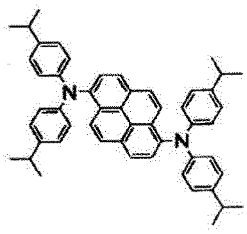
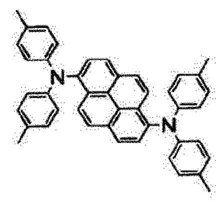
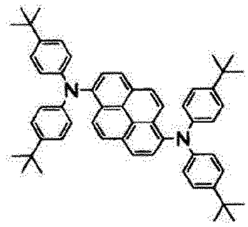
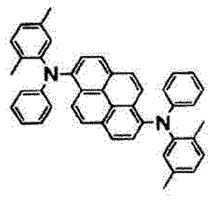
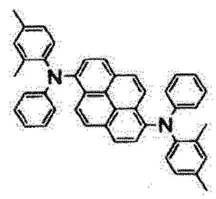
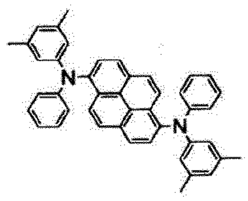
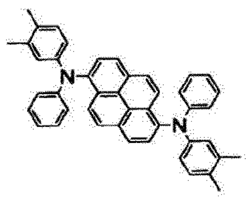
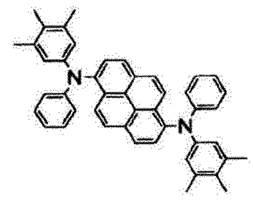
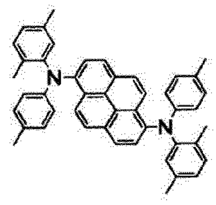
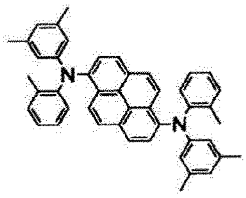
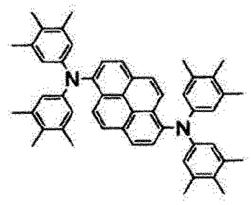
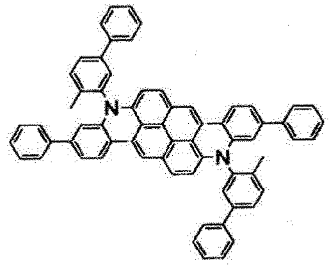
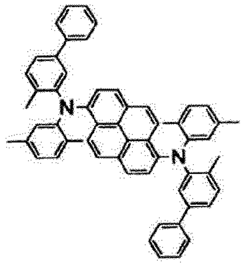
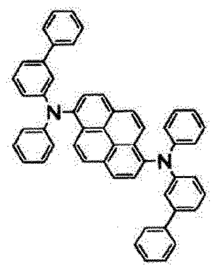

[Formula 73]

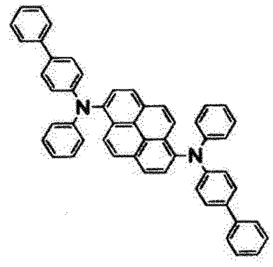
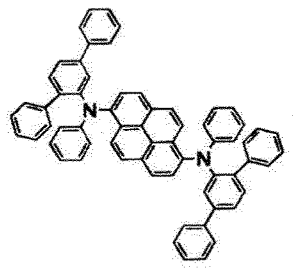
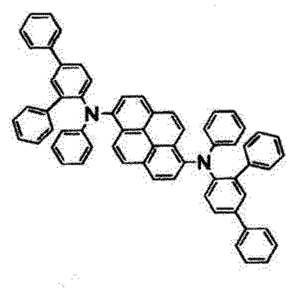
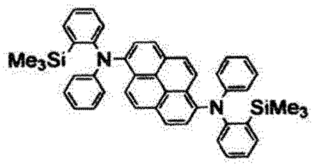
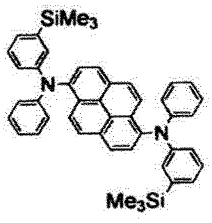
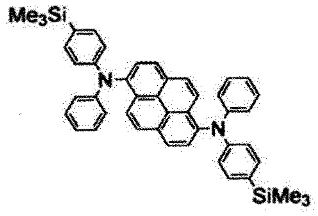
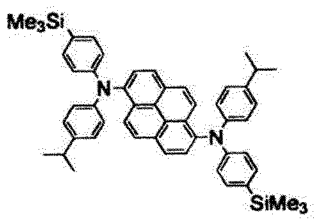
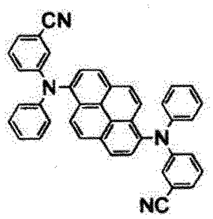
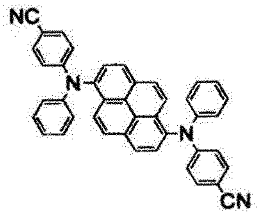
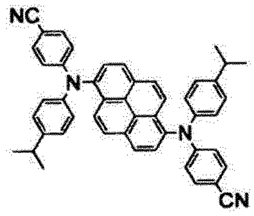
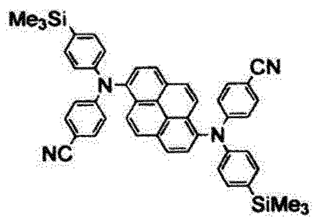
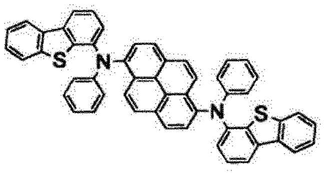
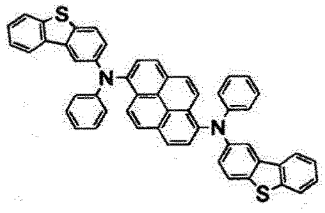
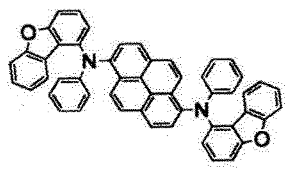
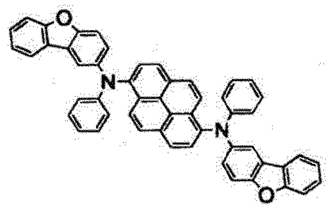
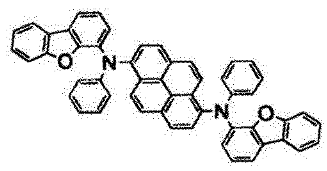

[Formula 74]

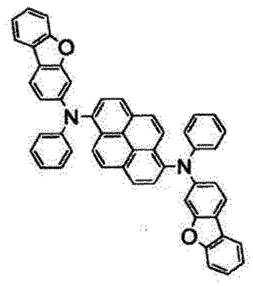
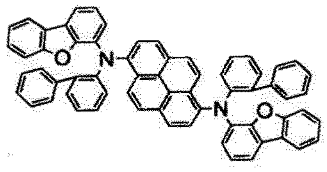
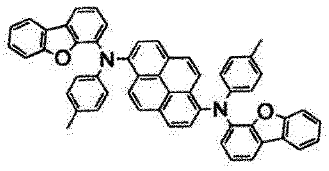
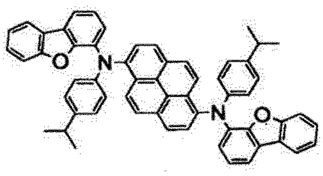
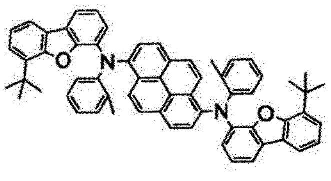
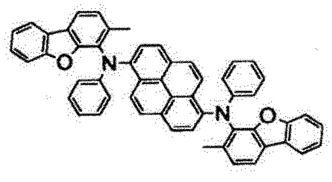
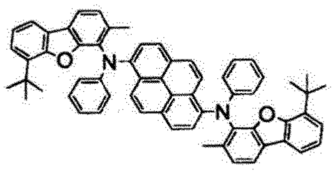
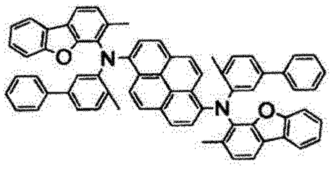
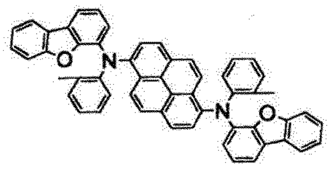
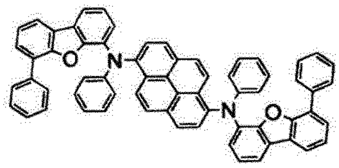
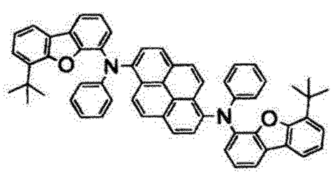
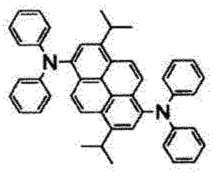
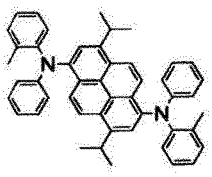
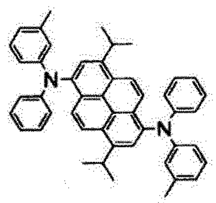
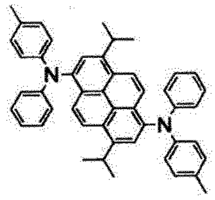
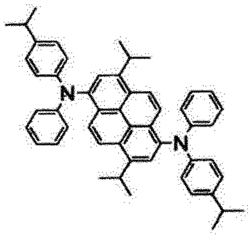
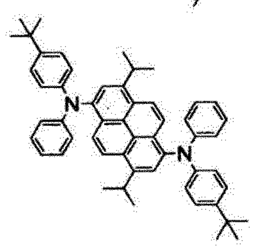
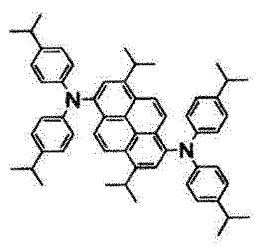
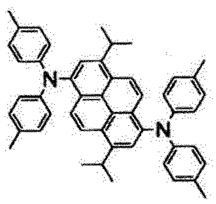

[Formula 75]

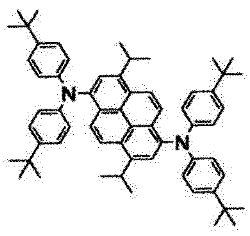
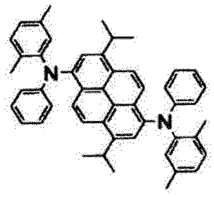
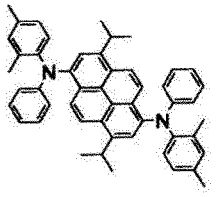
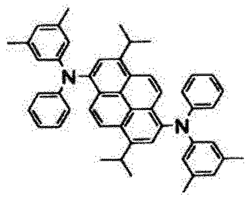
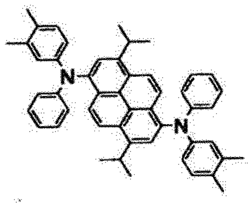
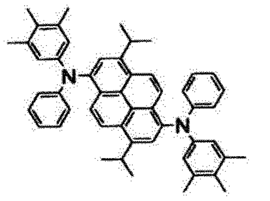
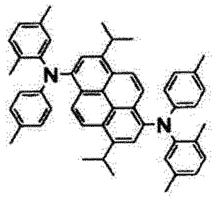
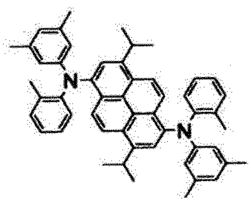
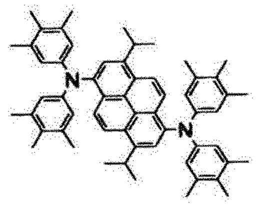
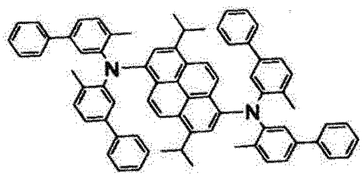
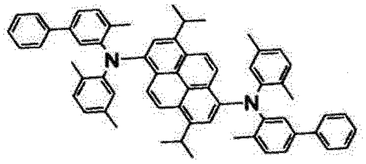
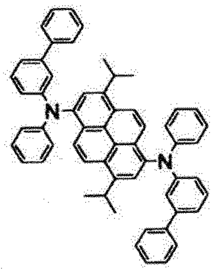
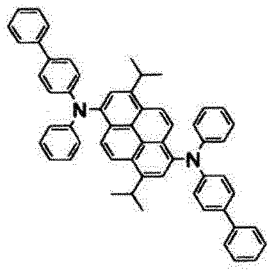
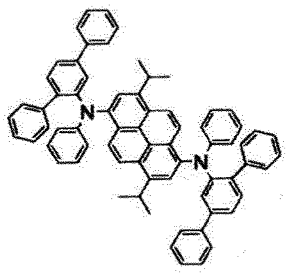
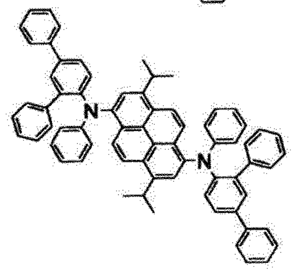
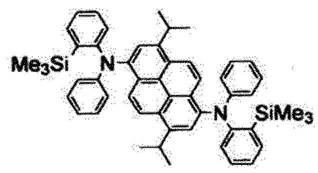
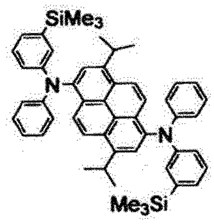
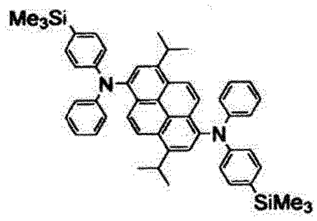
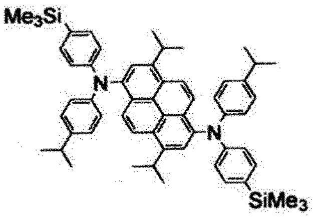
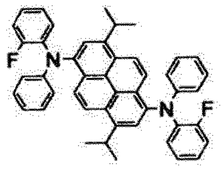
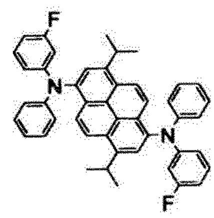

[Formula 76]

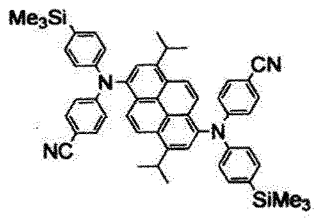
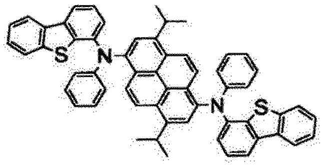
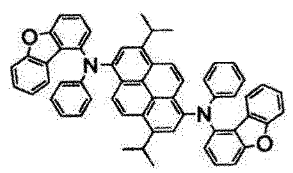
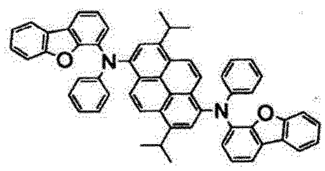
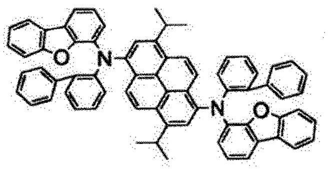
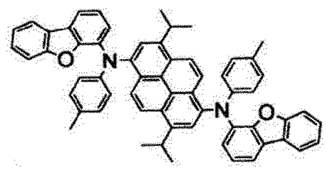
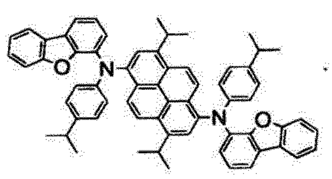
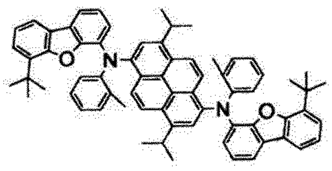
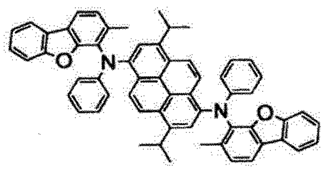
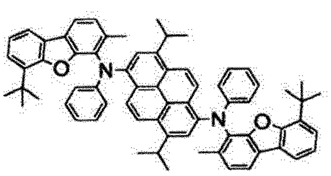
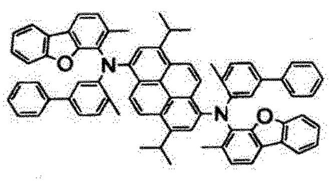
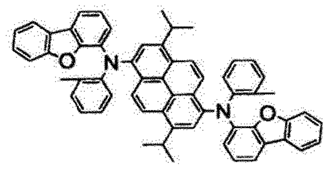
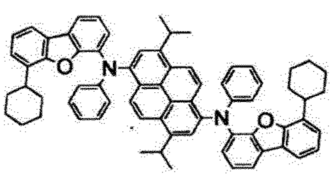
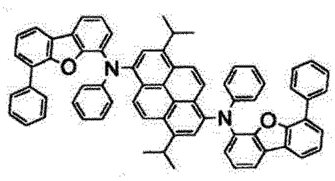
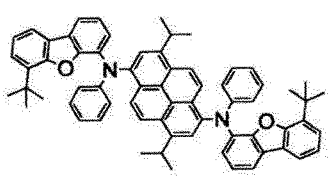

Compound Represented by Formula (4)

The compound represented by the formula (4) will be described below.

[Formula 77]

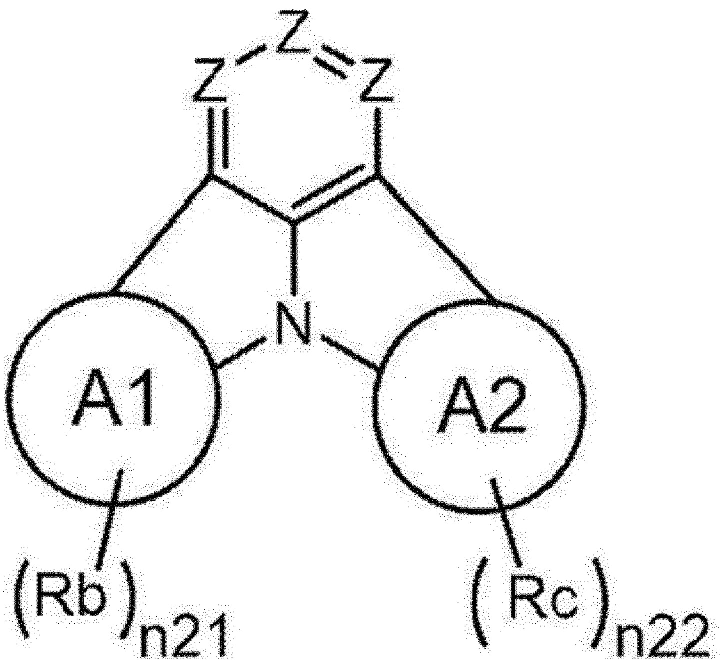

(4)

In the formula (4):

each Z is independently CRa or a nitrogen atom;

a ring A1 and a ring A2 are each independently a substituted or unsubstituted aromatic hydrocarbon ring having 6 to 50 ring carbon atoms, or a substituted or unsubstituted heterocycle having 5 to 50 ring atoms;

when a plurality of Ra are present, at least one combination of adjacent two or more of the plurality of Ra are mutually bonded to form a substituted or unsubstituted monocyclic ring, mutually bonded to form a substituted or unsubstituted fused ring, or not mutually bonded;

n21 and n22 are each independently 0, 1, 2, 3 or 4;

when a plurality of Rb are present, at least one combination of adjacent two or more of the plurality of Rb are mutually bonded to form a substituted or unsubstituted monocyclic ring, mutually bonded to form a substituted or unsubstituted fused ring, or not mutually bonded;

when a plurality of Rc are present, at least one combination of adjacent two or more of the plurality of Rc are mutually bonded to form a substituted or unsubstituted monocyclic ring, mutually bonded to form a substituted or unsubstituted fused ring, or not mutually bonded; and

Ra, Rb, and Rc forming neither the monocyclic ring nor the fused ring are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

The "aromatic hydrocarbon ring" for the ring A1 and the ring A2 has the same structure as a compound formed by introducing a hydrogen atom to the "aryl group" described above.

Ring atoms of the "aromatic hydrocarbon ring" for the ring A1 and the ring A2 include two carbon atoms on a fused bicyclic structure at the center of the formula (4).

Specific examples of the "substituted or unsubstituted aromatic hydrocarbon ring having 6 to 50 ring carbon atoms" include a compound formed by introducing a hydrogen atom to the "aryl group" described in the specific example group G1.

The "heterocycle" for the ring A1 and the ring A2 has the same structure as a compound formed by introducing a hydrogen atom to the "heterocyclic group" described above.

Ring atoms of the "heterocycle" for the ring A1 and the ring A2 include two carbon atoms on a fused bicyclic structure at the center of the formula (4).

Specific examples of the "substituted or unsubstituted heterocycle having 5 to 50 ring atoms" include a compound

formed by introducing a hydrogen atom to the "heterocyclic group" described in the specific example group G2.

Rb is bonded to any one of carbon atoms forming the aromatic hydrocarbon ring as the ring A1 or any one of atoms forming the heterocycle as the ring A1.

Rc is bonded to any one of carbon atoms forming the aromatic hydrocarbon ring as the ring A2 or any one of atoms forming the heterocycle as the ring A2.

At least one of Ra, Rb, or Rc is preferably a group represented by a formula (4a) below. More preferably, at least two of Ra, Rb, and Rc are each a group represented by the formula (4a).

[Formula 78]

$$*-L_{401}-Ar_{401} \quad (4a)$$

In the formula (4a):

$L_{401}$ is a single bond, a substituted or unsubstituted arylene group having 6 to 30 ring carbon atoms, or a substituted or unsubstituted divalent heterocyclic group having 5 to 30 ring atoms; and

$Ar_{401}$ is a substituted or unsubstituted aryl group having 6 to 50 ring carbon atoms, a substituted or unsubstituted heterocyclic group having 5 to 50 ring atoms, or a group represented by a formula (4b) below.

[Formula 79]

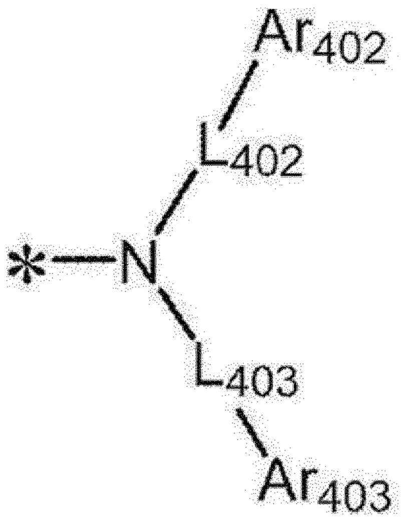

(4b)

In the formula (4b):

$L_{402}$ and $L_{403}$ are each independently a single bond, a substituted or unsubstituted arylene group having 6 to 30 ring carbon atoms, or a substituted or unsubstituted divalent heterocyclic group having 5 to 30 ring atoms;

a combination of $Ar_{402}$ and $Ar_{403}$ are mutually bonded to form a substituted or unsubstituted monocyclic ring, mutually bonded to form a substituted or unsubstituted fused ring, or not mutually bonded; and

$Ar_{402}$ and $Ar_{403}$ forming neither the monocyclic ring nor the fused ring are each independently a substituted or unsubstituted aryl group having 6 to 50 ring carbon atoms, or a substituted or unsubstituted heterocyclic group having 5 to 50 ring atoms.

In an exemplary embodiment, the compound represented by the formula (4) is represented by a formula (42) below.

[Formula 80]

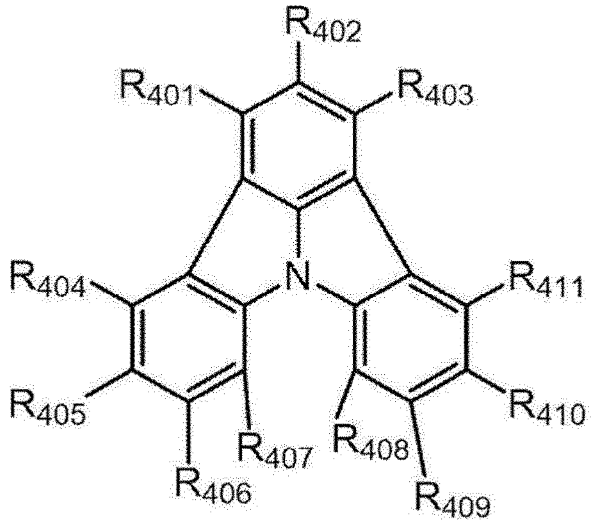

(42)

In the formula (42):

at least one combination of adjacent two or more of $R_{401}$ to $R_{411}$ are mutually bonded to form a substituted or unsubstituted monocyclic ring, mutually bonded to form a substituted or unsubstituted fused ring, or not mutually bonded; and

$R_{401}$ to $R_{411}$ forming neither the monocyclic ring nor the fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

At least one of $R_{401}$ to $R_{411}$ is preferably a group represented by the formula (4a). More preferably, at least two of $R_{401}$ to $R_{411}$ are each a group represented by the formula (4a).

$R_{404}$ and $R_{411}$ are each preferably a group represented by the formula (4a).

In an exemplary embodiment, the compound represented by the formula (4) is a compound formed by bonding a structure represented by a formula (4-1) or a formula (4-2) below to the ring A1.

Further, in an exemplary embodiment, the compound represented by the formula (42) is a compound formed by bonding a structure represented by the formula (4-1) or the formula (4-2) to a ring bonded to $R_{404}$ to $R_{407}$.

[Formula 81]

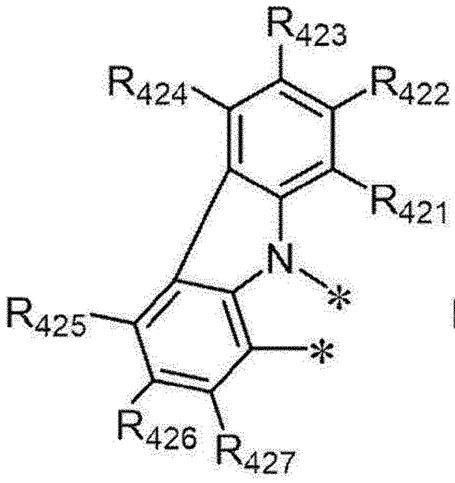

(4-1)

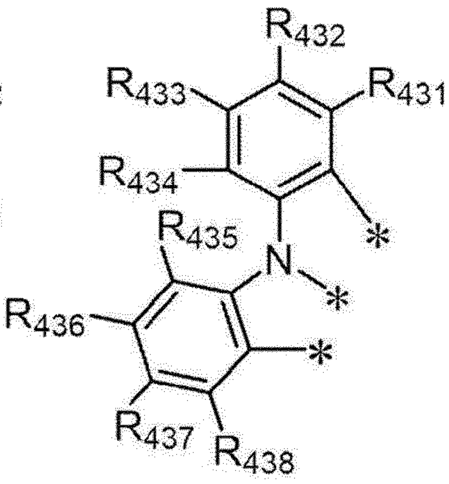

(4-2)

In the formula (4-1), two bonds * are each independently bonded to a ring carbon atom of the aromatic hydrocarbon ring or a ring atom of the heterocycle as the ring A1 in the formula (4) or bonded to one of $R_{404}$ to $R_{407}$ in the formula (42);

in the formula (4-2), three bonds * are each independently bonded to a ring carbon atom of the aromatic hydrocarbon ring or a ring atom of the heterocycle as the ring A1 in the formula (4) or bonded to one of $R_{404}$ to $R_{407}$ in the formula (42);
at least one combination of adjacent two or more of $R_{421}$ to $R_{427}$ are mutually bonded to form a substituted or unsubstituted monocyclic ring, mutually bonded to form a substituted or unsubstituted fused ring, or not mutually bonded;
at least one combination of adjacent two or more of $R_{431}$ to $R_{438}$ are mutually bonded to form a substituted or unsubstituted monocyclic ring, mutually bonded to form a substituted or unsubstituted fused ring, or not mutually bonded; and
$R_{421}$ to $R_{427}$ and $R_{431}$ to $R_{438}$ forming neither the monocyclic ring nor the fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

In an exemplary embodiment, the compound represented by the formula (4) is a compound represented by a formula (41-3), a formula (41-4) or a formula (41-5) below.

[Formula 82]

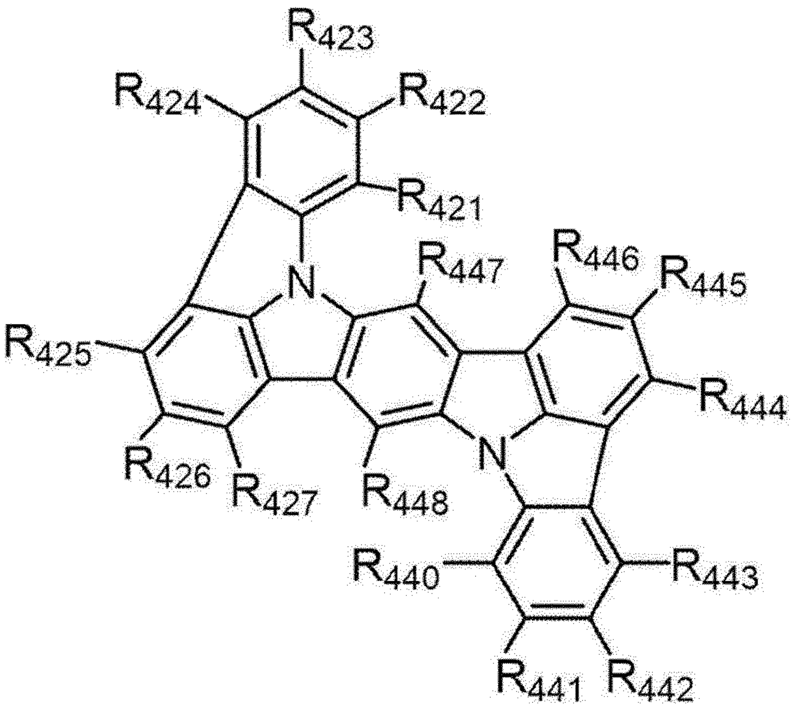

(41-3)

[Formula 83]

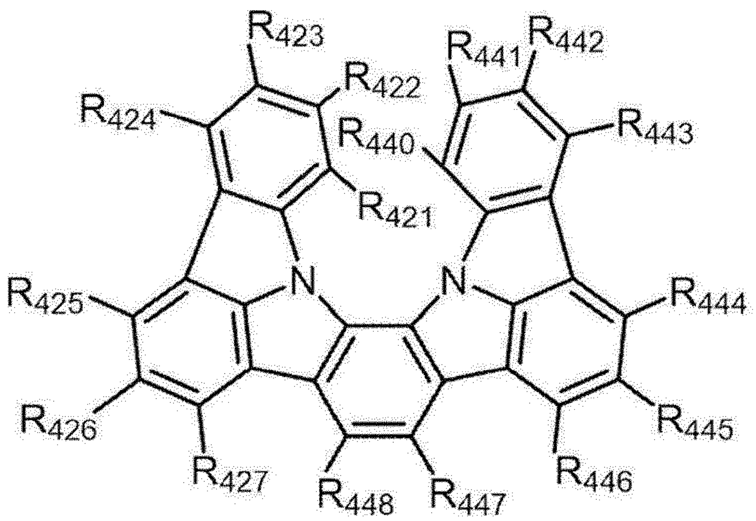

(41-4)

[Formula 84]

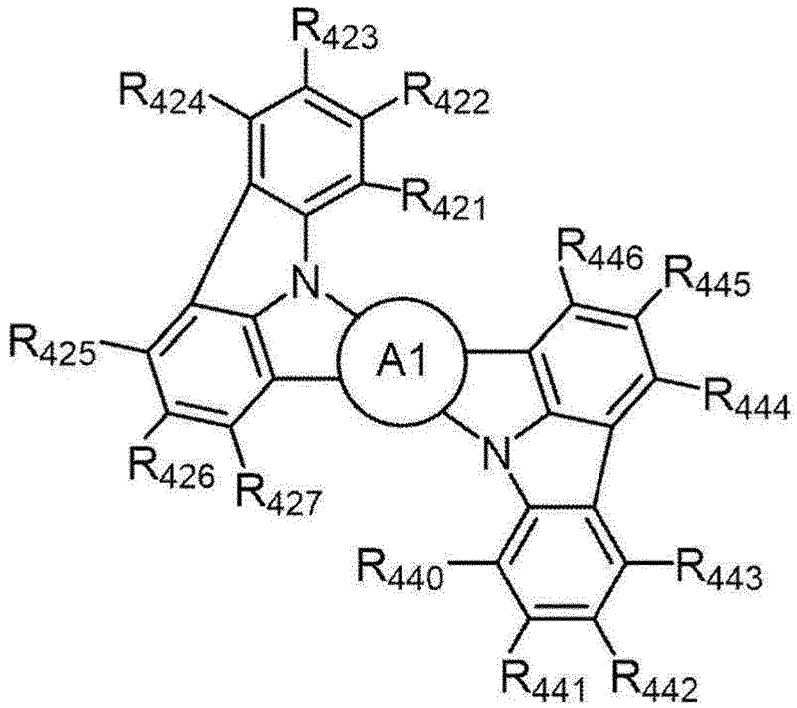

(41-5)

In the formulae (41-3), (41-4), and (41-5):

a ring A1 is as defined in the formula (4);
$R_{421}$ to $R_{427}$ each independently represent the same as $R_{421}$ to $R_{427}$ in the formula (4-1); and
$R_{440}$ to $R_{448}$ each independently represent the same as $R_{401}$ to $R_{411}$ in the formula (42).

In an exemplary embodiment, a substituted or unsubstituted aromatic hydrocarbon ring having 6 to 50 ring carbon atoms as the ring A1 in the formula (41-5) is a substituted or unsubstituted naphthalene ring, or a substituted or unsubstituted fluorene ring.

In an exemplary embodiment, a substituted or unsubstituted heterocycle having 5 to 50 ring atoms as the ring A1 in the formula (41-5) is a substituted or unsubstituted dibenzofuran ring, a substituted or unsubstituted carbazole ring, or a substituted or unsubstituted dibenzothiophene ring.

In an exemplary embodiment, the compound represented by the formula (4) or the formula (42) is selected from the group consisting of compounds represented by formulae (461) to (467) below.

[Formula 85]

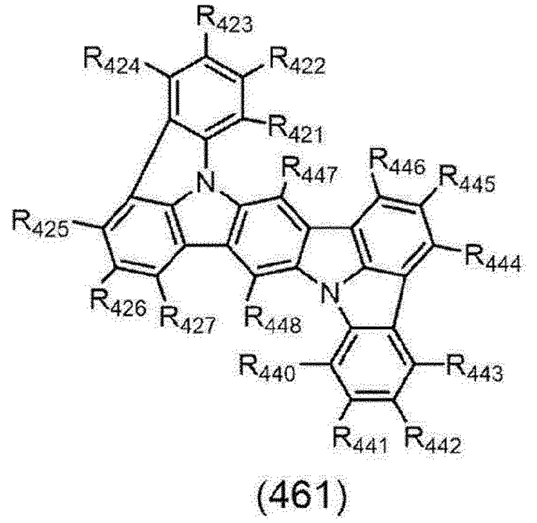

(461)

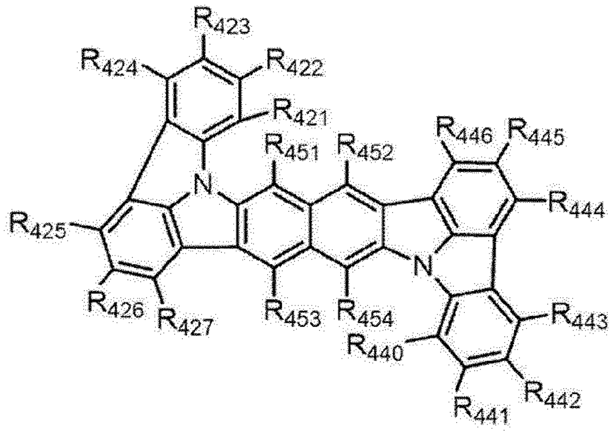

(462)

[Formula 86]

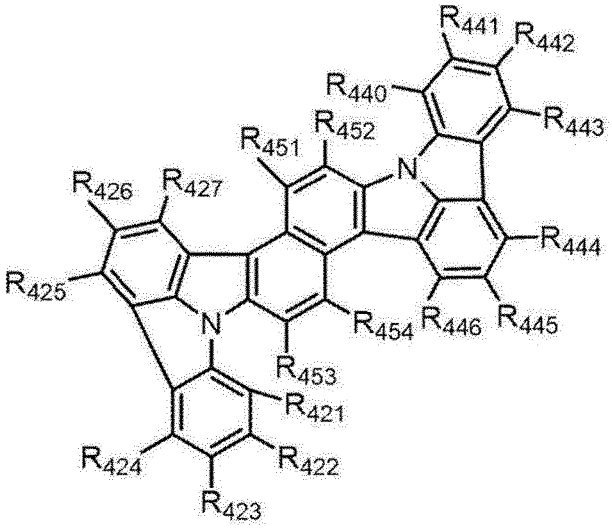

(463)

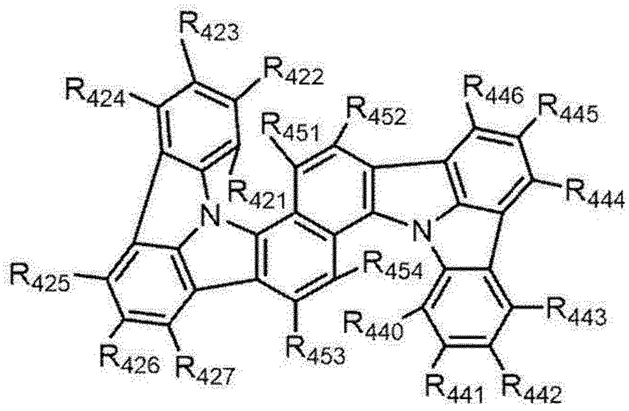

(464)

[Formula 87]

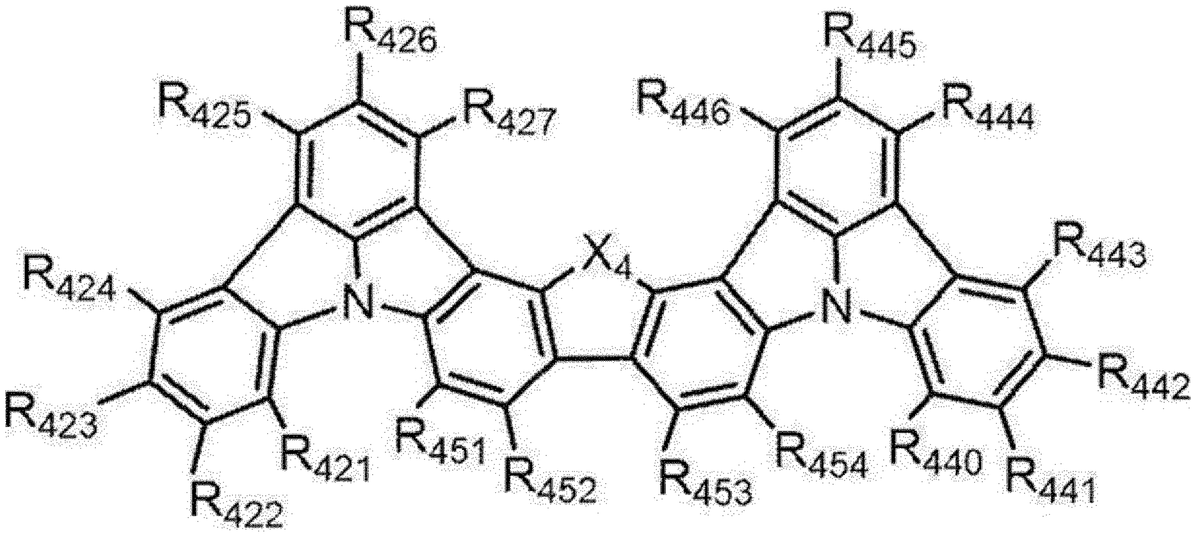

(465)

[Formula 88]

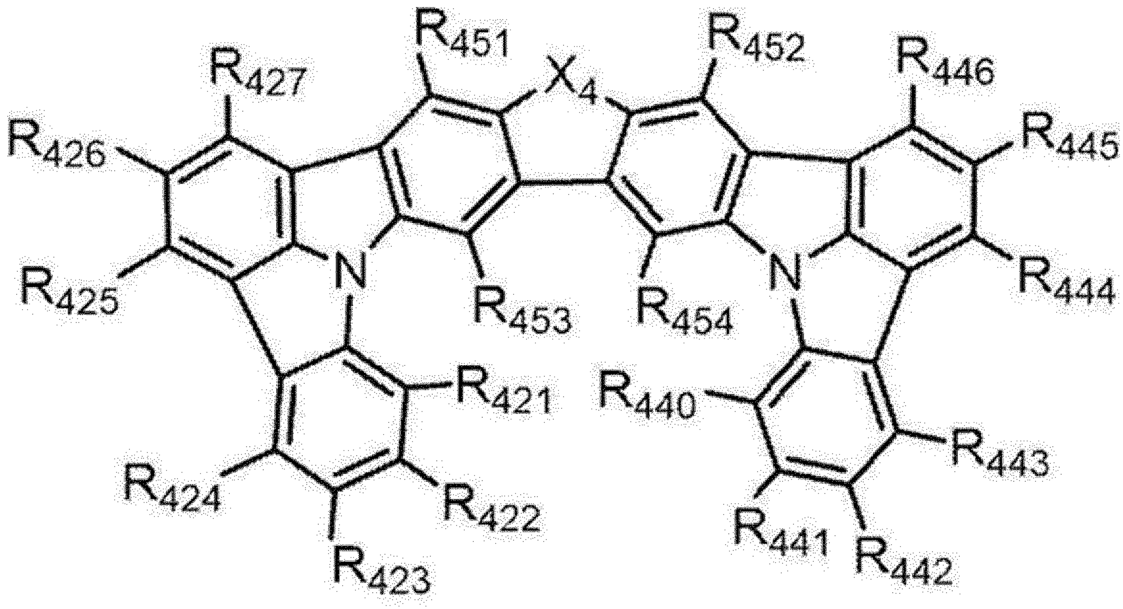

(466)

[Formula 89]

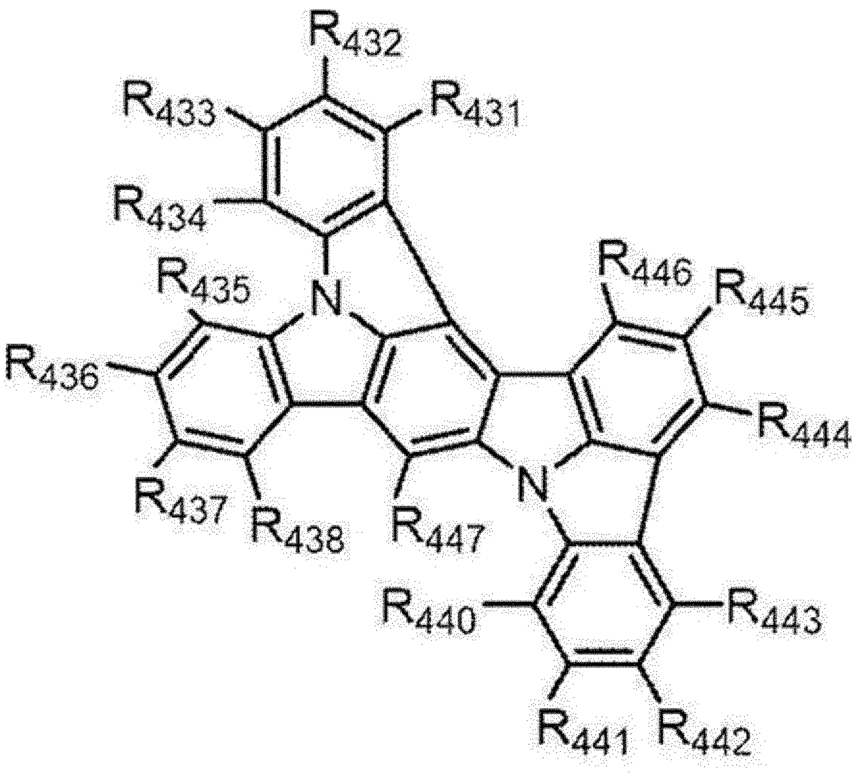

(467)

In the formulae (461), (462), (463), (464), (465), (466), and (467):

$R_{421}$ to $R_{427}$ each independently represent the same as $R_{421}$ to $R_{427}$ in the formula (4-1);
$R_{431}$ to $R_{438}$ each independently represent the same as $R_{431}$ to $R_{438}$ in the formula (4-2);
$R_{440}$ to $R_{448}$ and $R_{451}$ to $R_{454}$ each independently represent the same as $R_{401}$ to $R_{411}$ in the formula (42);
$X_4$ is an oxygen atom, $NR_{301}$, or $C(R_{802})(R_{803})$;
$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms, preferably, a substituted or unsubstituted alkyl group having 1 to 50 carbon atoms or a substituted or unsubstituted aryl group having 6 to 50 ring carbon atoms;
when a plurality of $R_{801}$ are present, the plurality of $R_{301}$ are mutually the same or different;
when a plurality of $R_{802}$ are present, the plurality of $R_{802}$ are mutually the same or different; and
when a plurality of $R_{803}$ are present, the plurality of $R_{803}$ are mutually the same or different.

In an exemplary embodiment, in the compound represented by the formula (42), at least one combination of adjacent two or more of $R_{401}$ to $R_{411}$ are mutually bonded to form a substituted or unsubstituted monocyclic ring or are mutually bonded to form a substituted or unsubstituted fused ring. The compound represented by the formula (42) in the exemplary embodiment is described in detail as a compound represented by a formula (45) below.

Compound Represented by Formula (45)

The compound represented by the formula (45) will be described below.

[Formula 90]

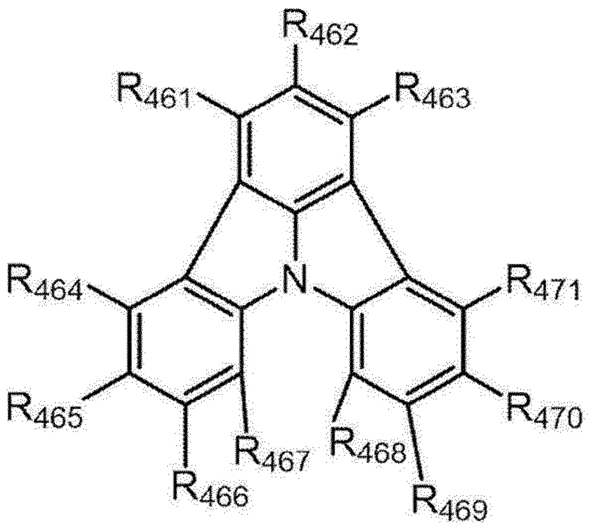

(45)

In the formula (45):

two or more of combinations selected from the group consisting of a combination of $R_{461}$ and $R_{462}$, a combination of $R_{462}$ and $R_{463}$, a combination of $R_{464}$ and $R_{465}$, a combination of $R_{465}$ and $R_{466}$, a combination of $R_{466}$ and $R_{467}$, a combination of $R_{468}$ and $R_{469}$, a combination of $R_{469}$ and $R_{470}$, and a combination of $R_{470}$ and $R_{471}$ are mutually bonded to form a substituted or unsubstituted monocyclic ring or mutually bonded to form a substituted or unsubstituted fused ring;
the combination of $R_{461}$ and $R_{462}$ and the combination of $R_{462}$ and $R_{463}$; the combination of $R_{464}$ and $R_{465}$ and the combination of $R_{465}$ and $R_{466}$; the combination of $R_{465}$ and $R_{466}$ and the combination of $R_{466}$ and $R_{467}$; the combination of $R_{468}$ and $R_{469}$ and the combination of $R_{469}$ and $R_{470}$; and the combination of $R_{469}$ and $R_{470}$ and the combination of $R_{470}$ and $R_{471}$ do not simultaneously form a ring;
the two or more rings formed by $R_{461}$ to $R_{471}$ are mutually the same or different; and
$R_{461}$ to $R_{471}$ forming neither the monocyclic ring nor the fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$,

a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms.

In the formula (45), $R_n$ and $R_{n+1}$ (n being an integer selected from 461, 462, 464 to 466, and 468 to 470) are mutually bonded to form a substituted or unsubstituted monocyclic ring or fused ring together with two ring carbon atoms bonded to $R_n$ and $R_{n+1}$. The ring is preferably formed of atoms selected from the group consisting of a carbon atom, an oxygen atom, a sulfur atom, and a nitrogen atom, and is made of preferably 3 to 7 atoms, more preferably 5 or 6 atoms.

The number of the above cyclic structures in the compound represented by the formula (45) is, for instance, 2, 3, or 4. The two or more of the cyclic structures may be present on the same benzene ring on the basic skeleton represented by the formula (45) or may be present on different benzene rings. For instance, when three cyclic structures are present, each of the cyclic structures may be present on the corresponding one of the three benzene rings of the formula (45).

Examples of the above cyclic structures in the compound represented by the formula (45) include structures represented by formulae (451) to (460) below.

[Formula 91]

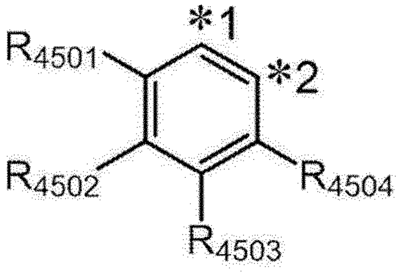

(451)

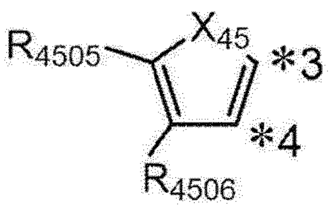

(452)

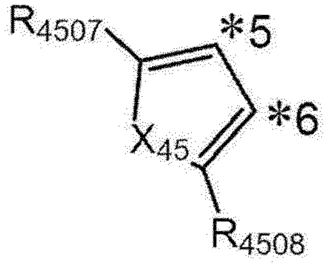

(453)

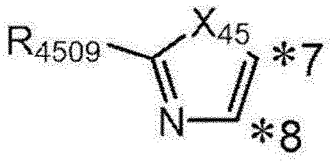

(454)

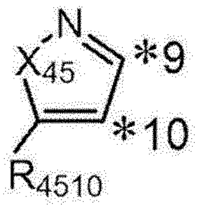

(455)

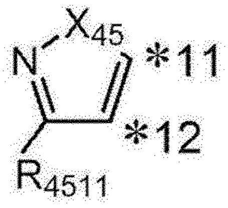

(456)

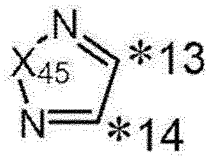

(457)

In the formulae (451) to (457):

each combination of *1 and *2, *3 and *4, *5 and *6, *7 and *8, *9 and *10, *11 and *12, and *13 and *14 represents the two ring carbon atoms bonded to $R_n$ and $R_{n+1}$;
the ring carbon atom bonded to $R_n$ may be any one of the two ring carbon atoms represented by *1 and *2, *3 and *4, *5 and *6, *7 and *8, *9 and *10, *11 and *12, and *13 and *14;
$X_{45}$ is C($R_{4512}$)($R_{4513}$), N$R_{4514}$, an oxygen atom, or a sulfur atom;
at least one combination of adjacent two or more of $R_{4501}$ to $R_{4506}$ and $R_{4512}$ to $R_{4513}$ are mutually bonded to form a substituted or unsubstituted monocyclic ring, mutually bonded to form a substituted or unsubstituted fused ring, or not mutually bonded; and
$R_{4501}$ to $R_{4514}$ forming neither the monocyclic ring nor the fused ring each independently represent the same as $R_{461}$ to $R_{471}$ in the formula (45).

[Formula 92]

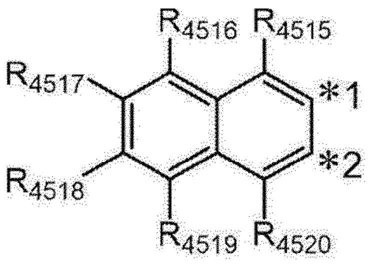

(458)

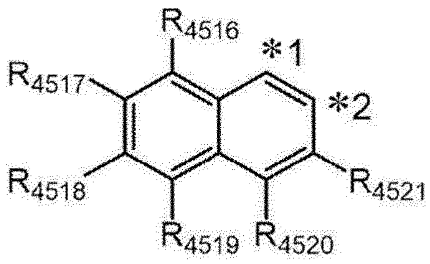

(459)

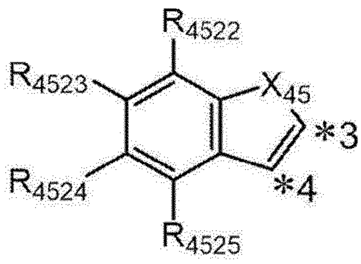

(460)

In the formulae (458) to (460):

*1 and *2, and *3 and *4 respectively represent the two ring carbon atoms bonded to $R_n$ and $R_{n+1}$;
the ring carbon atom bonded to $R_n$ may be any one of the two ring carbon atoms represented by *1 and *2, or *3 and *4;
$X_{45}$ is $C(R_{4512})(R_{4513})$, $NR_{4514}$, an oxygen atom, or a sulfur atom;
at least one combination of adjacent two or more of $R_{4512}$ to $R_{4513}$ and $R_{4515}$ to $R_{4525}$ are mutually bonded to form a substituted or unsubstituted monocyclic ring, mutually bonded to form a substituted or unsubstituted fused ring, or not mutually bonded; and
$R_{4512}$ to $R_{4513}$, $R_{4515}$ to $R_{4521}$ and $R_{4522}$ to $R_{4525}$ forming neither the monocyclic ring nor the fused ring, and $R_{4514}$ each independently represent the same as $R_{461}$ to $R_{471}$ in the formula (45).

In the formula (45), preferably, at least one of $R_{462}$, $R_{464}$, $R_{465}$, $R_{470}$ or $R_{471}$ (preferably, at least one of $R_{462}$, $R_{465}$ or $R_{470}$, more preferably $R_{462}$) is a group forming no cyclic structure.

(i) In the formula (45), a substituent, if present, for a cyclic structure formed by $R_n$ and $R_{n+1}$, (ii) in the formula (45), $R_{461}$ to $R_{471}$ forming no cyclic structure, and (iii) $R_{4501}$ to $R_{4514}$, $R_{4515}$ to $R_{4525}$ in the formulae (451) to (460) are preferably each independently a group selected from the group consisting of a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-N(R_{906})(R_{907})$, a substituted or unsubstituted aryl group having 6 to 50 ring carbon atoms, a substituted or unsubstituted heterocyclic group having 5 to 50 ring atoms, or groups represented by formulae (461) to (464) below.

[Formula 93]

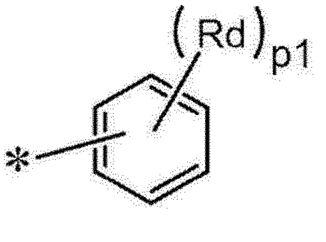

(461)

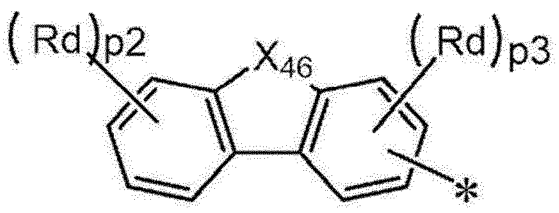

(462)

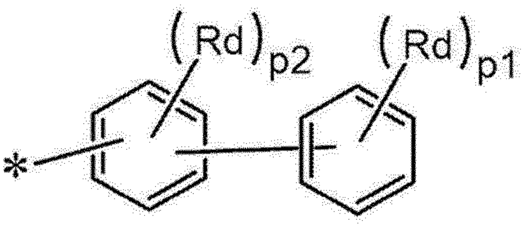

(463)

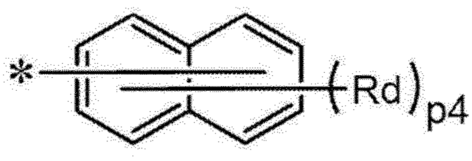

(464)

In the formulae (461) to (464):

each $R_d$ is independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and

$X_{46}$ is $C(R_{801})(R_{802})$, $NR_{803}$, an oxygen atom, or a sulfur atom;

$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms, preferably, a substituted or unsubstituted alkyl group having 1 to 50 carbon atoms or a substituted or unsubstituted aryl group having 6 to 50 ring carbon atoms;

when a plurality of $R_{801}$ are present, the plurality of $R_{801}$ are mutually the same or different;

when a plurality of $R_{802}$ are present, the plurality of $R_{802}$ are mutually the same or different;

when a plurality of $R_{803}$ are present, the plurality of $R_{803}$ are mutually the same or different;

p1 is 5;

p2 is 4;

p3 is 3;

p4 is 7; and

* in the formulae (461) to (464) each independently represent a bonding position to a cyclic structure.

In the luminescent compound, $R_{901}$ to $R_{907}$ are as defined above.

In an exemplary embodiment, the compound represented by the formula (45) is represented by one of formulae (45-1) to (45-6) below.

[Formula 94]

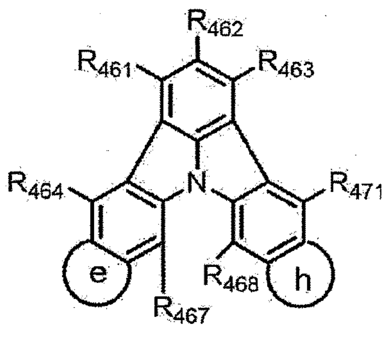

(45-1)

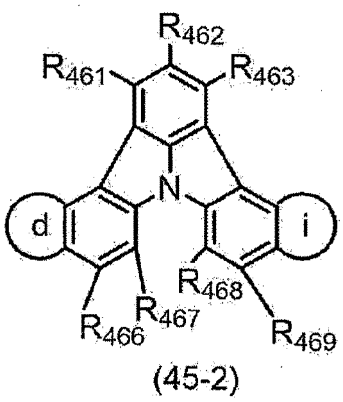

(45-2)

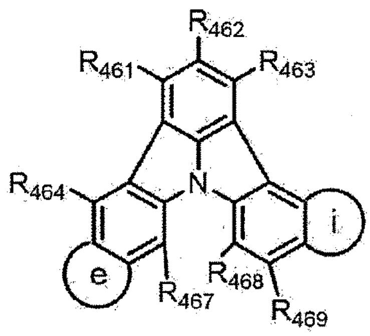

(45-3)

[Formula 95]

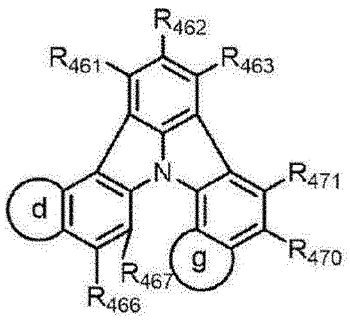

(45-4)

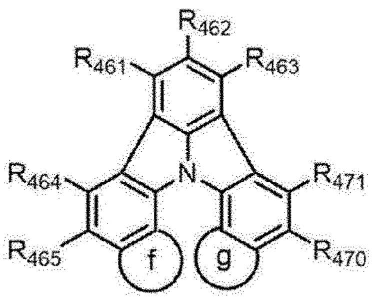

(45-5)

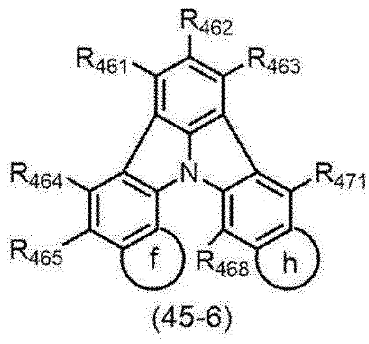

(45-6)

In the formulae (45-1) to (45-6):

rings d to i are each independently a substituted or unsubstituted monocyclic ring or a substituted or unsubstituted fused ring; and

$R_{461}$ to $R_{471}$ each independently represent the same as $R_{461}$ to $R_{471}$ in the formula (45).

In an exemplary embodiment, the compound represented by the formula (45) is represented by one of formulae (45-7) to (45-12) below.

[Formula 96]

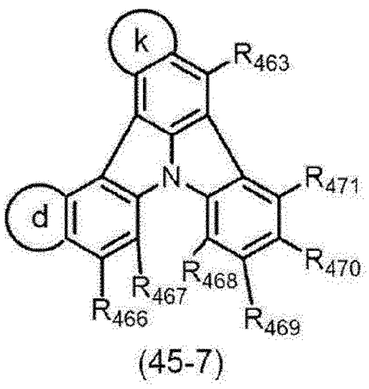

(45-7)

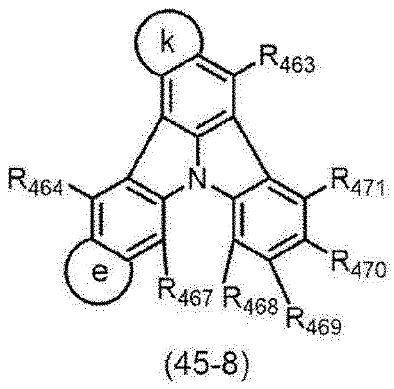

(45-8)

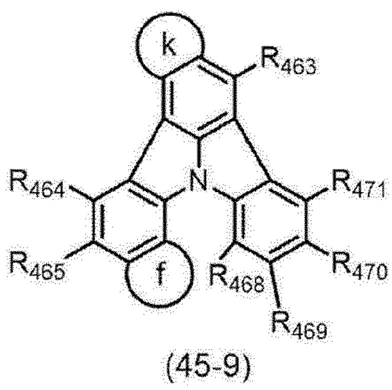

(45-9)

[Formula 97]

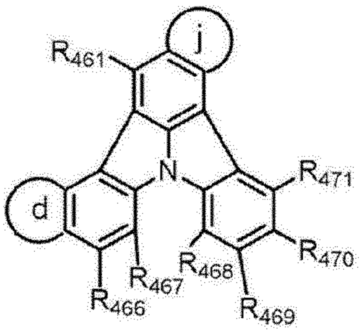

(45-10)

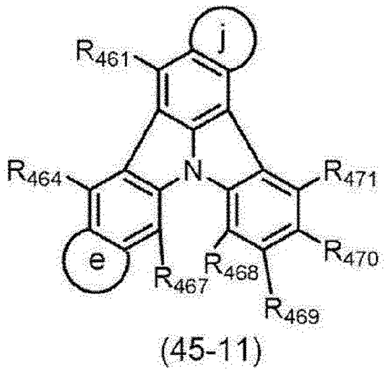

(45-11)

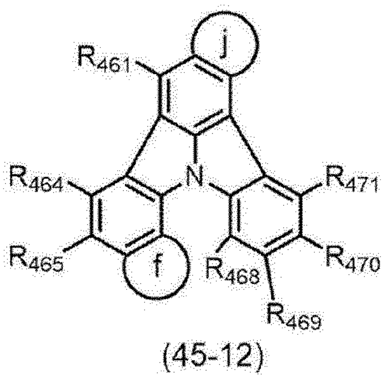

(45-12)

In the formulae (45-7) to (45-12):

rings d to f, k and j are each independently a substituted or unsubstituted monocyclic ring or a substituted or unsubstituted fused ring; and

$R_{461}$ to $R_{471}$ each independently represent the same as $R_{461}$ to $R_{471}$ in the formula (45).

In an exemplary embodiment, the compound represented by the formula (45) is represented by one of formulae (45-13) to (45-21) below.

[Formula 98]

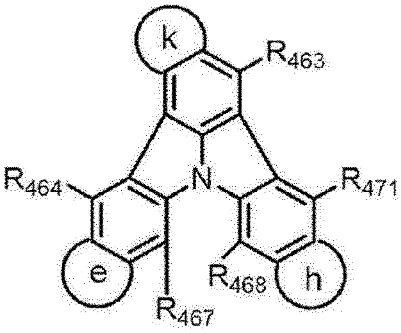

(45-13)

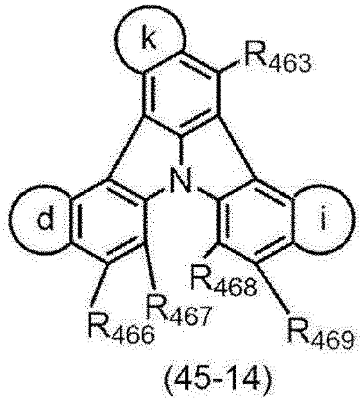

(45-14)

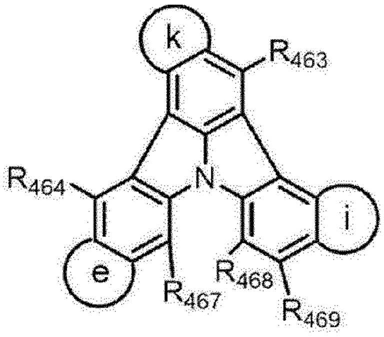

(45-15)

[Formula 99]

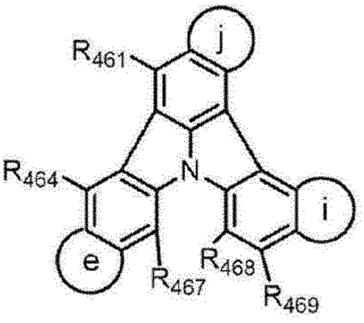

(45-16)

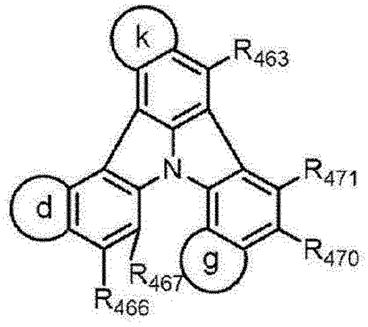

(45-17)

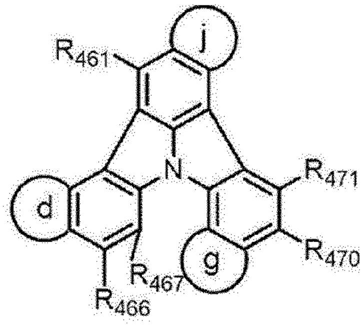

(45-18)

[Formula 100]

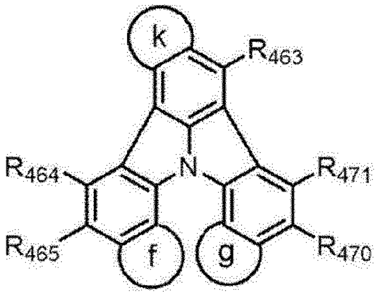

(45-19)

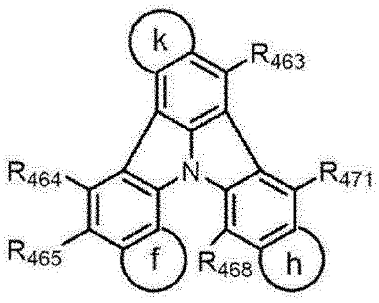

(45-20)

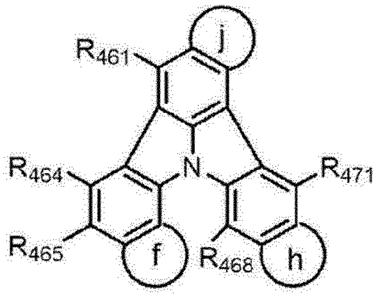

(45-21)

In the formulae (45-13) to (45-21):

rings d to k are each independently a substituted or unsubstituted monocyclic ring or a substituted or unsubstituted fused ring; and

$R_{461}$ to $R_{471}$ each independently represent the same as $R_{461}$ to $R_{471}$ in the formula (45).

When the ring g or the ring h further has a substituent, examples of the substituent include a substituted or unsubstituted alkyl group having 1 to 50 carbon atoms, a substituted or unsubstituted aryl group having 6 to 50 ring carbon atoms, a group represented by the formula (461), a group represented by the formula (463), and a group represented by the formula (464).

In an exemplary embodiment, the compound represented by the formula (45) is represented by one of formulae (45-22) to (45-25) below.

[Formula 101]

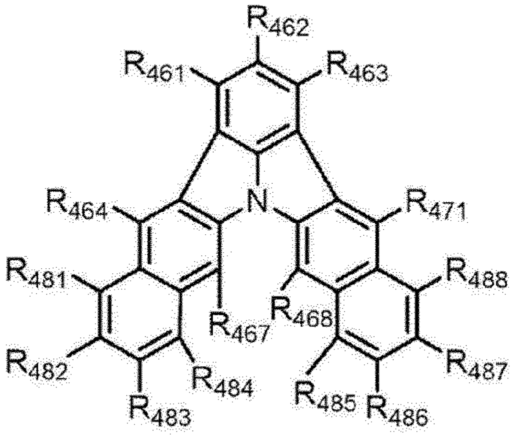

(45-22)

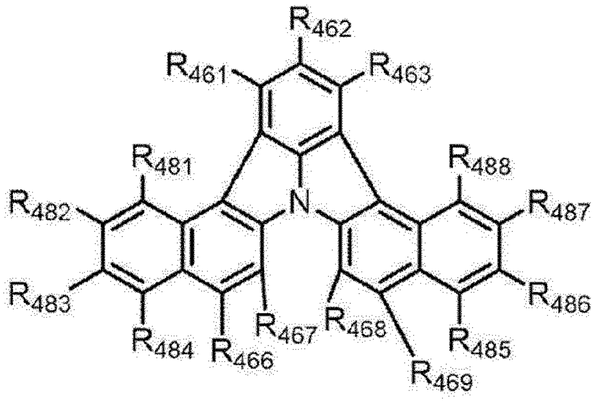

(45-23)

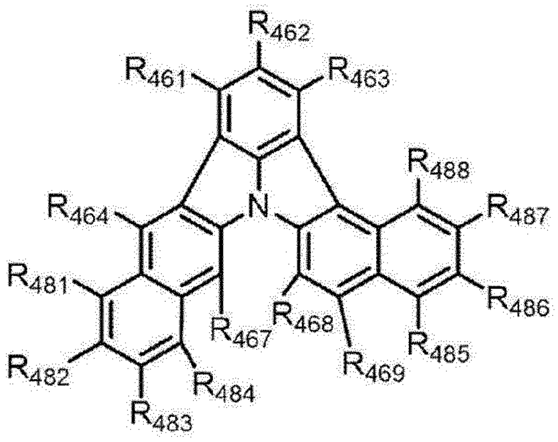

(45-24)

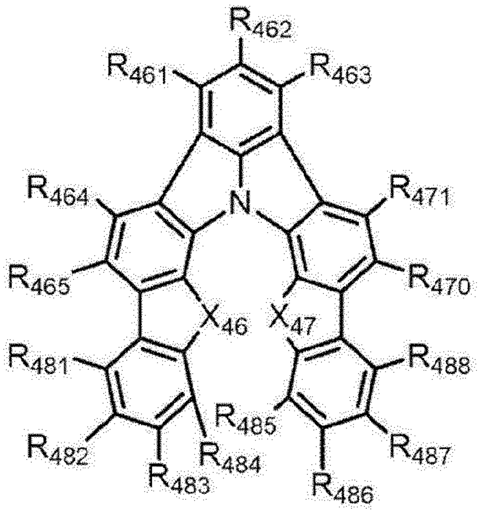

(45-25)

In the formulae (45-22) to (45-25):

$X_{46}$ and $X_{47}$ are each independently $C(R_{801})(R_{802})$, $NR_{803}$, an oxygen atom, or a sulfur atom;
$R_{461}$ to $R_{471}$ and $R_{481}$ to $R_{488}$ each independently represent the same as $R_{461}$ to $R_{471}$ in the formula (45);
$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms, preferably, a substituted or unsubstituted alkyl group having 1 to 50 carbon atoms or a substituted or unsubstituted aryl group having 6 to 50 ring carbon atoms;
when a plurality of $R_{801}$ are present, the plurality of $R_{801}$ are mutually the same or different;
when a plurality of $R_{802}$ are present, the plurality of $R_{802}$ are mutually the same or different; and
when a plurality of $R_{803}$ are present, the plurality of $R_{803}$ are mutually the same or different.

In an exemplary embodiment, the compound represented by the formula (45) is represented by a formula (45-26) below.

[Formula 102]

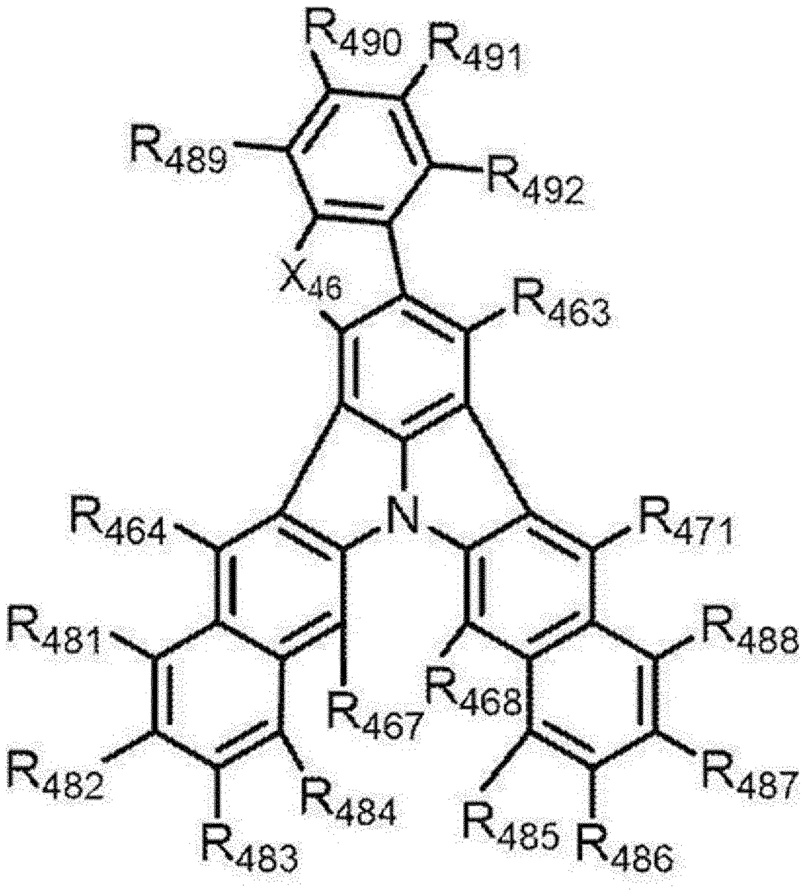

(45-26)

In the formula (45-26):

$X_{46}$ is $C(R_{801})(R_{802})$, $NR_{803}$, an oxygen atom, or a sulfur atom;
$R_{463}$, $R_{464}$, $R_{467}$, $R_{468}$, $R_{471}$, and $R_{481}$ to $R_{492}$ each independently represent the same as $R_{461}$ to $R_{471}$ in the formula (45);
$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms, preferably, a substituted or unsubstituted alkyl group having 1 to 50 carbon atoms or a substituted or unsubstituted aryl group having 6 to 50 ring carbon atoms;
when a plurality of $R_{801}$ are present, the plurality of $R_{801}$ are mutually the same or different;
when a plurality of $R_{802}$ are present, the plurality of $R_{802}$ are mutually the same or different; and
when a plurality of $R_{803}$ are present, the plurality of $R_{803}$ are mutually the same or different.

Specific examples of the compound represented by the formula (4) include compounds given below. In the specific examples below, Ph represents a phenyl group, and D represents a deuterium atom.

[Formula 103]

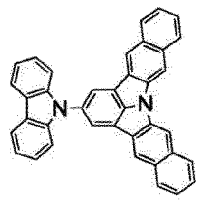
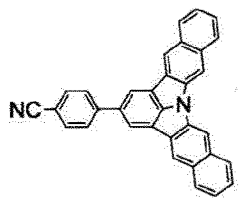
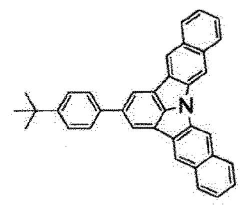
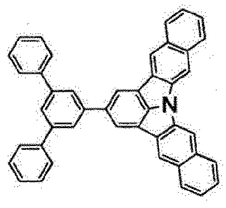
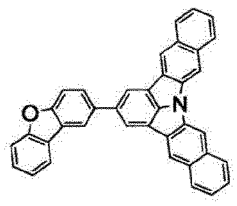
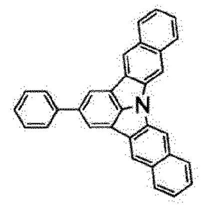
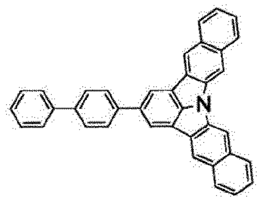
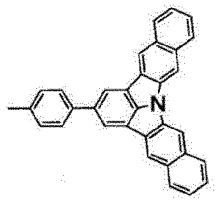
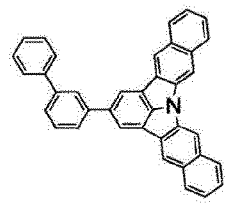
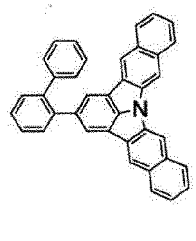
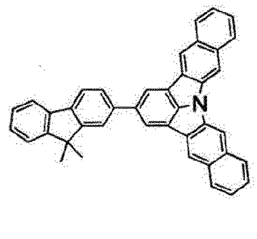
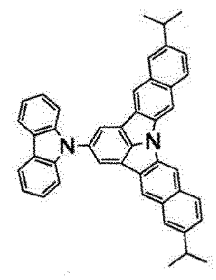
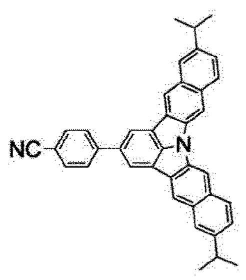
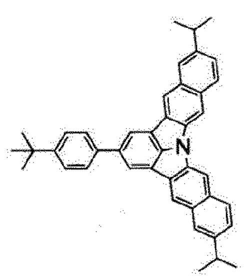
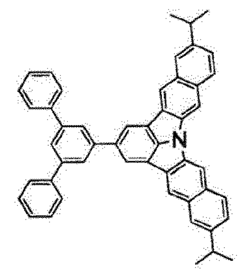
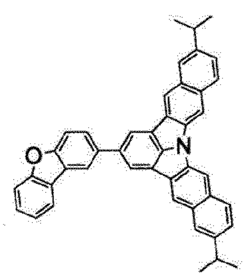
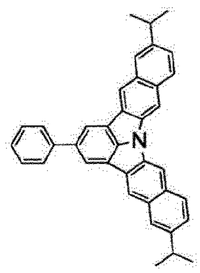
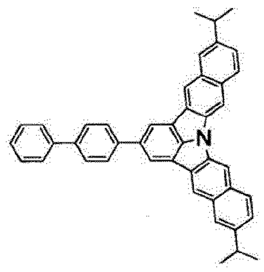

[Formula 104]

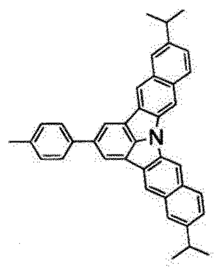
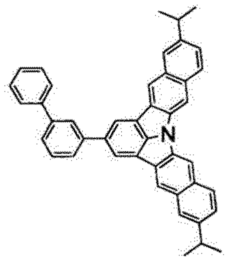
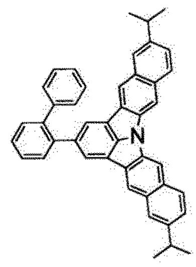

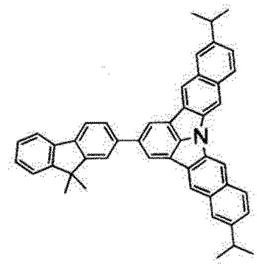
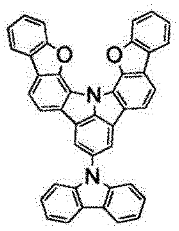
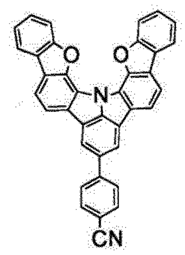

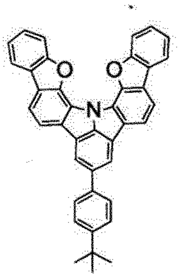
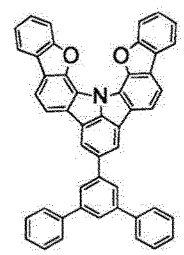
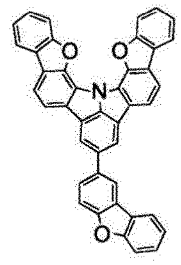

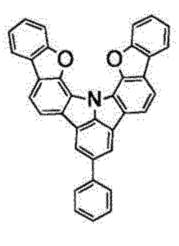
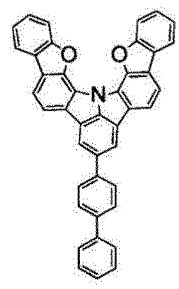
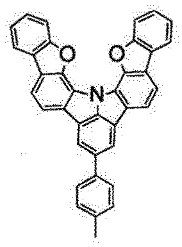

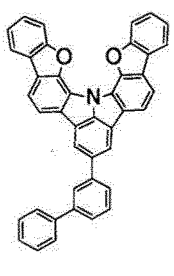
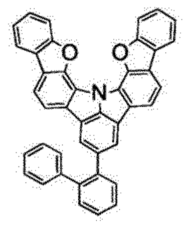
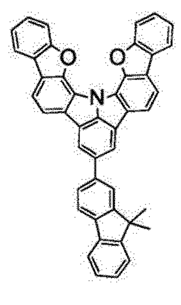

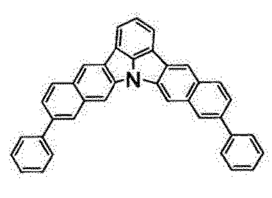
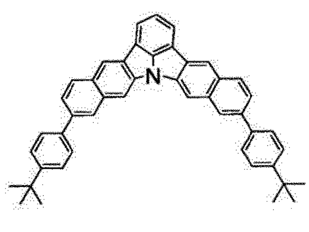
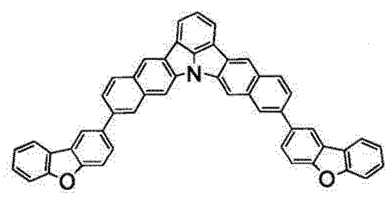

[Formula 105]

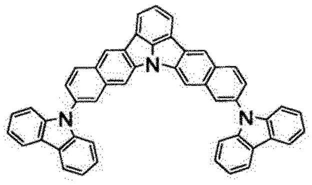
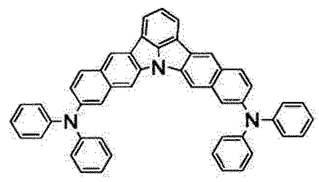
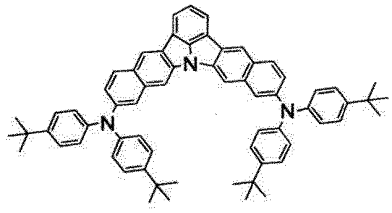
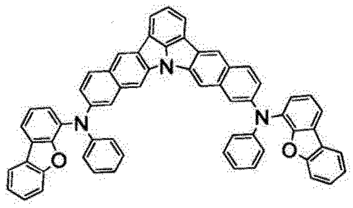
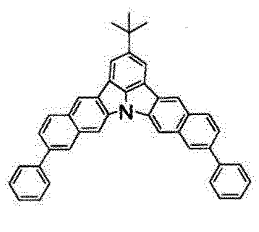
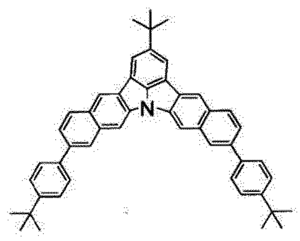
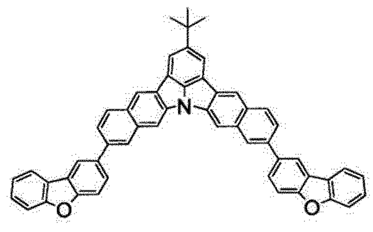
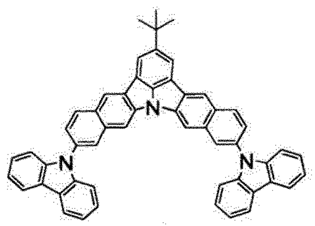
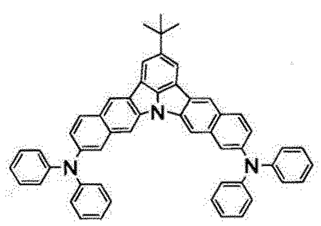
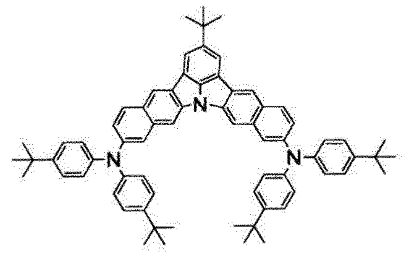
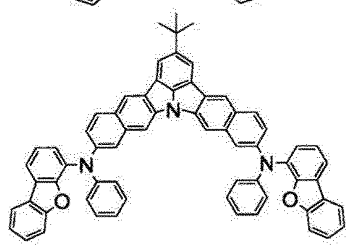
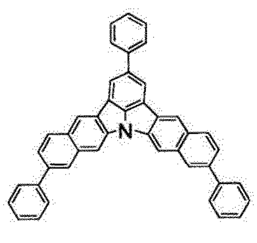
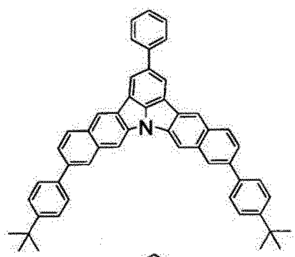
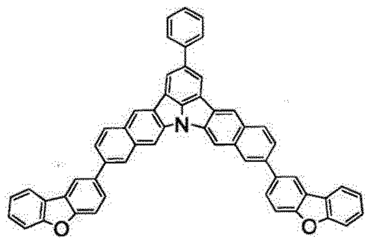
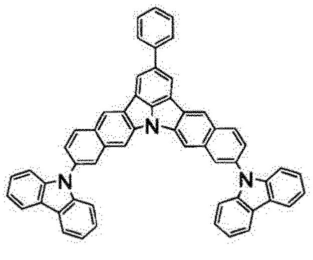
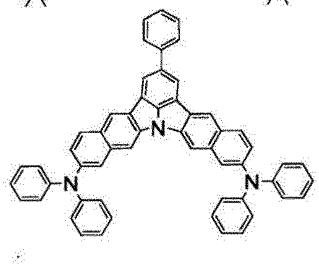
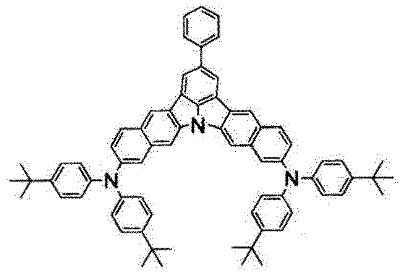

[Formula 106]

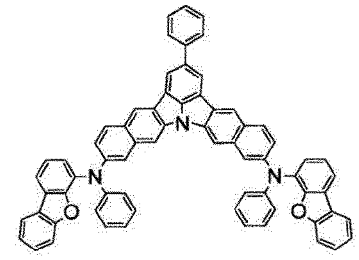

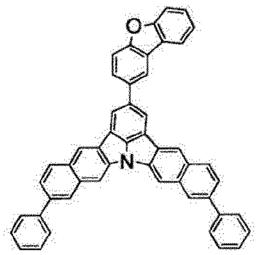

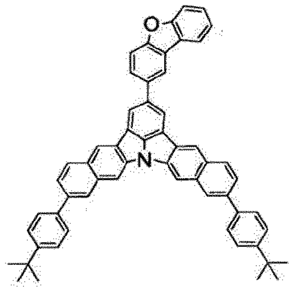

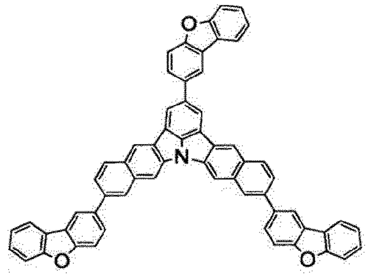

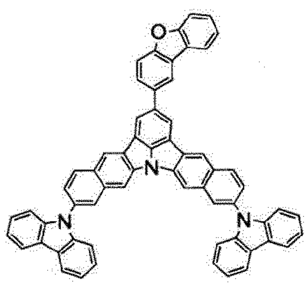

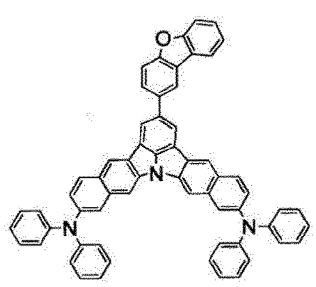

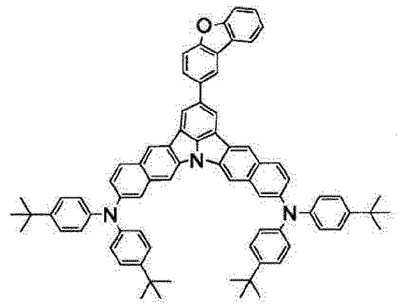

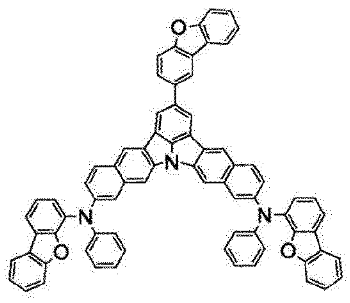

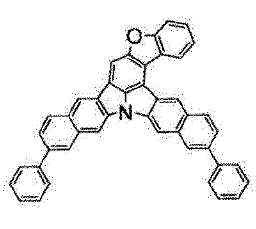

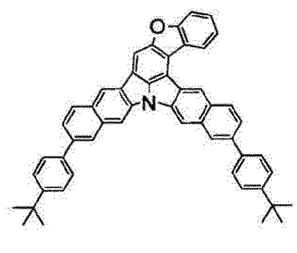

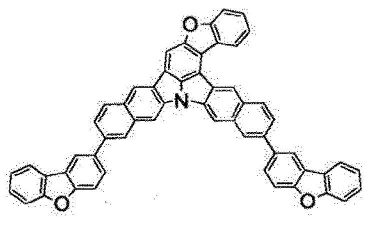

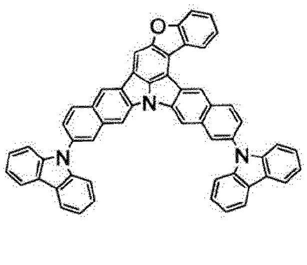

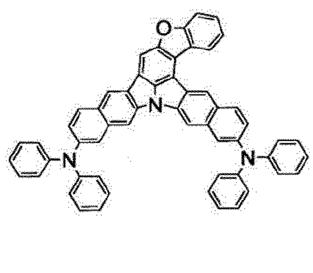

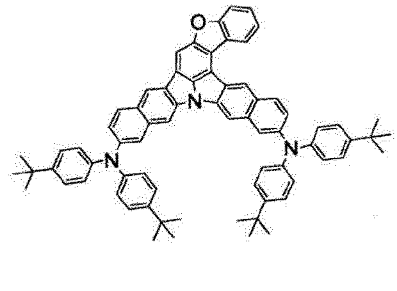

[Formula 107]

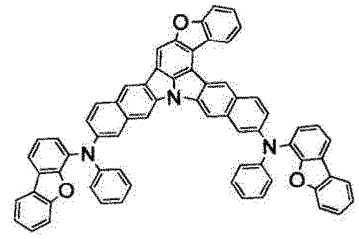

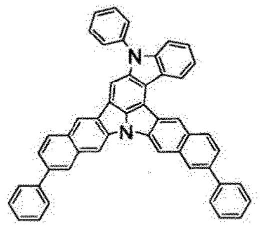

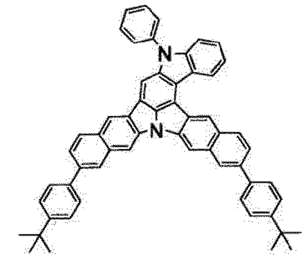

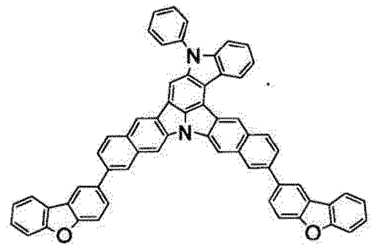

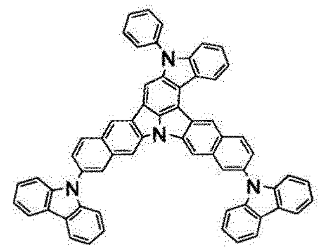

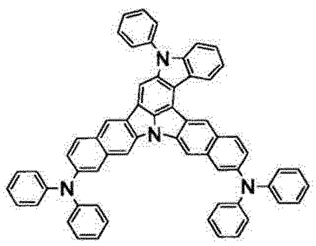

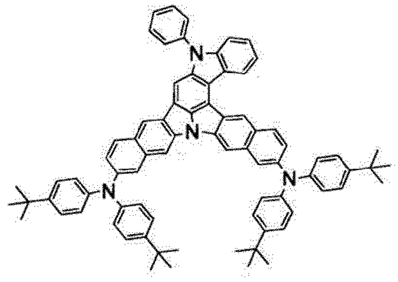

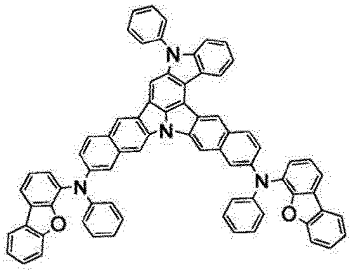

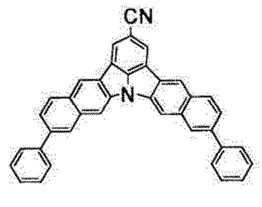

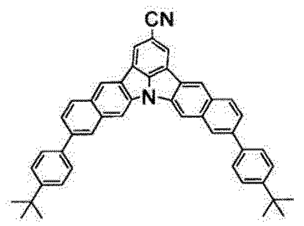

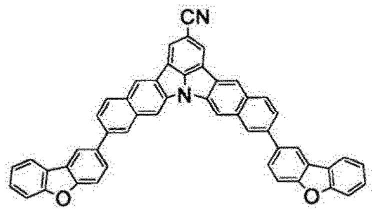

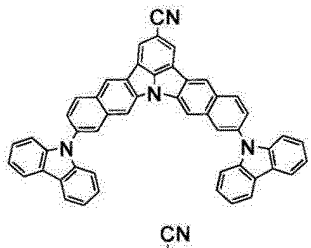

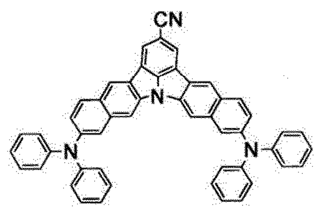

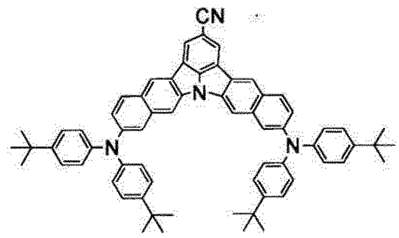

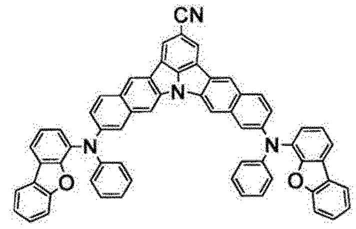

[Formula 108]

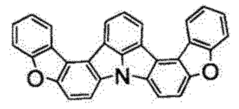
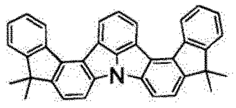
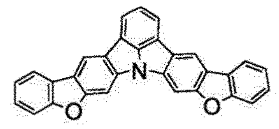
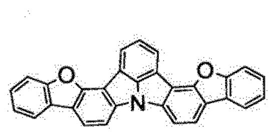
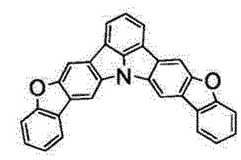
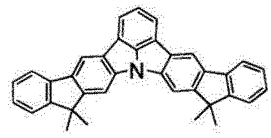
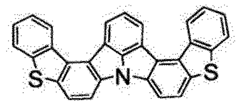
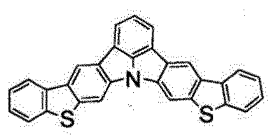
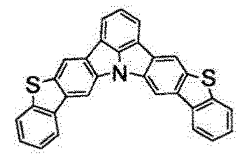
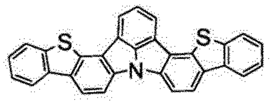
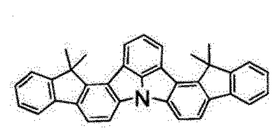
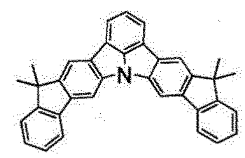
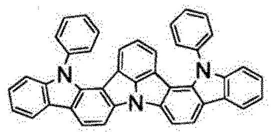
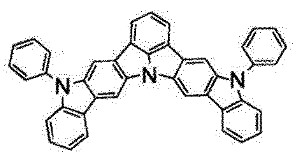
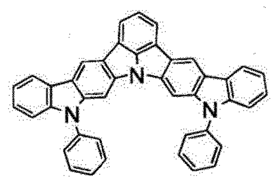
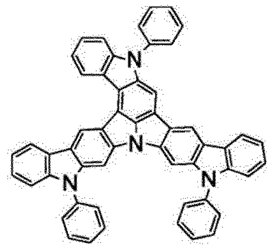
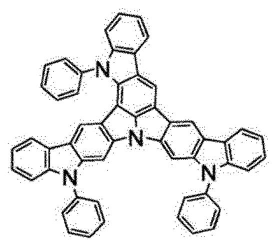
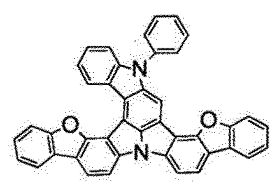
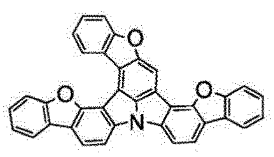
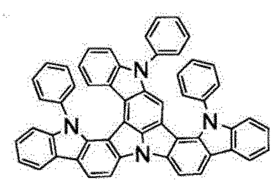
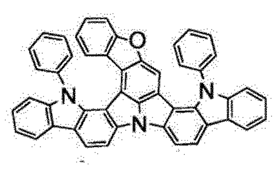
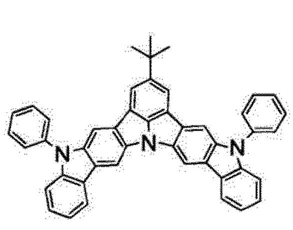
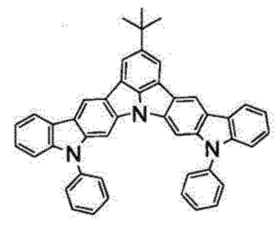

[Formula 109]

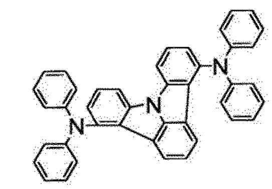
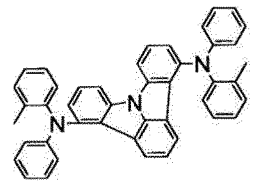
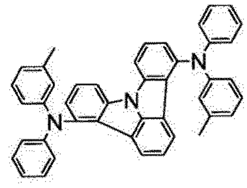

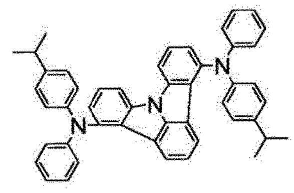
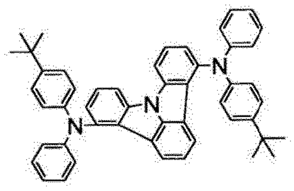
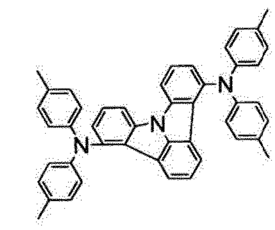

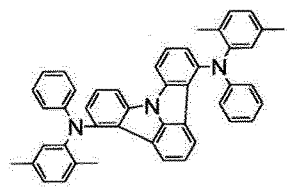
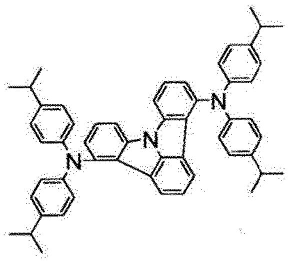
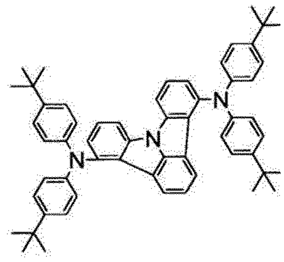

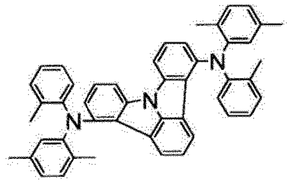
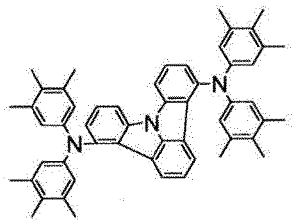
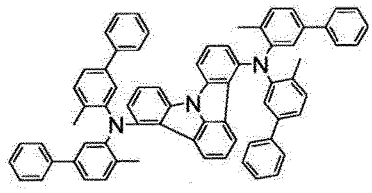

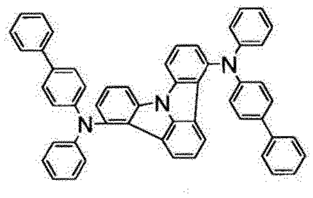
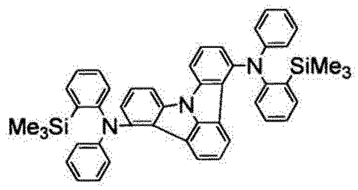
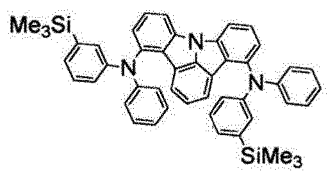

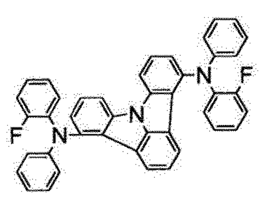
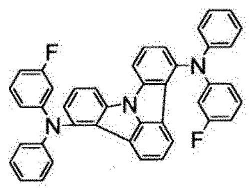
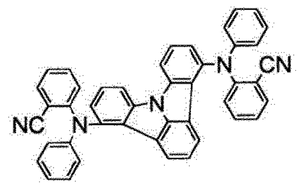

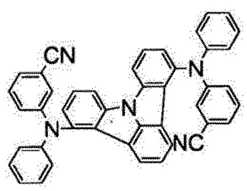
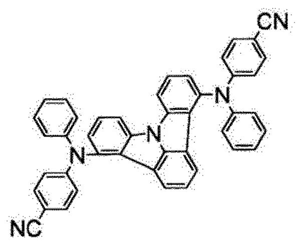
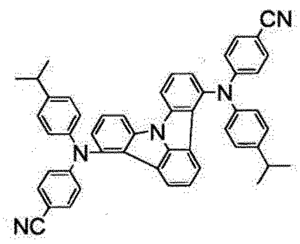

[Formula 110]

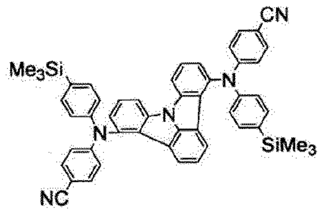
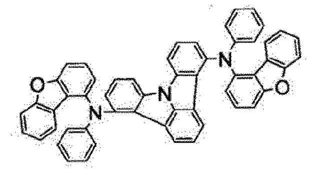
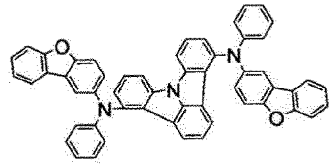
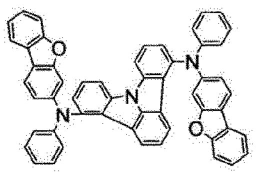
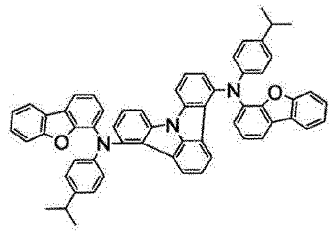
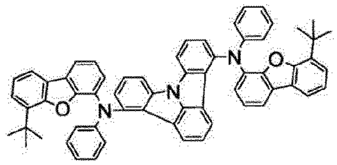
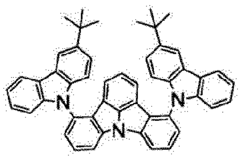
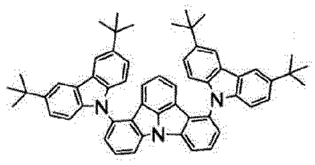
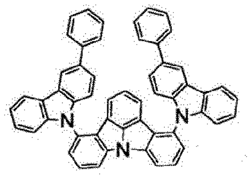
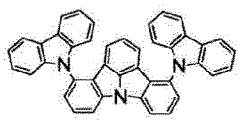
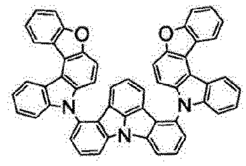
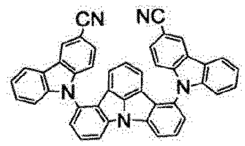
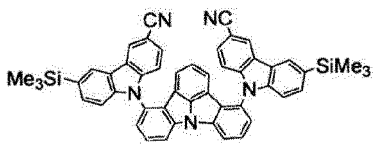
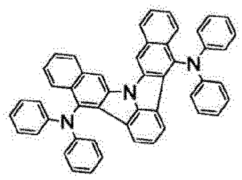
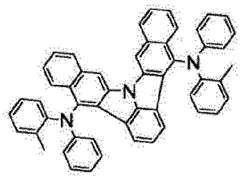
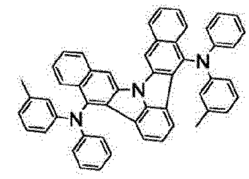
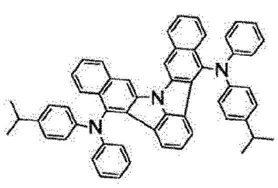
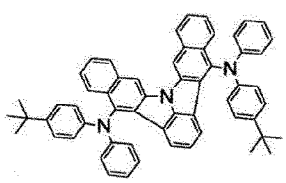
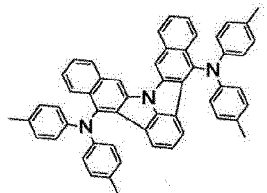
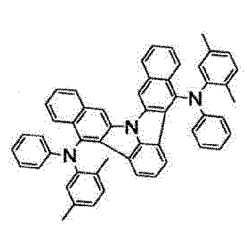
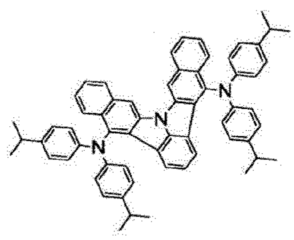
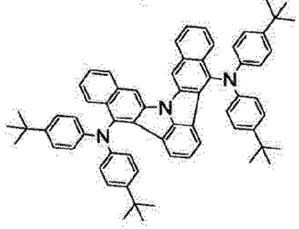

[Formula 111]

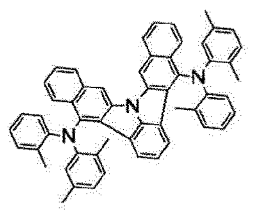
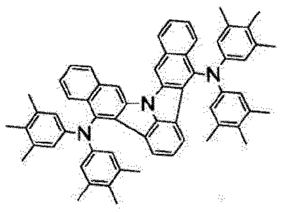
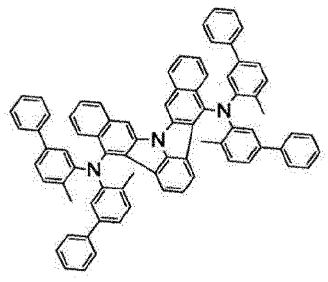
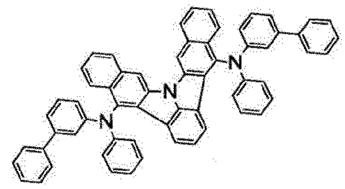
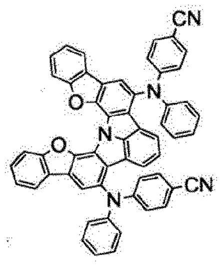
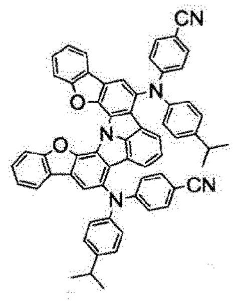
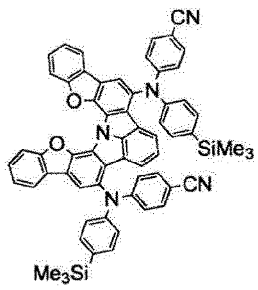
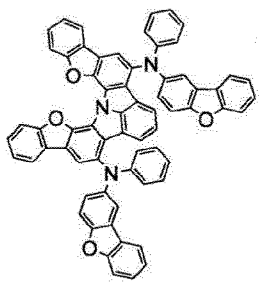
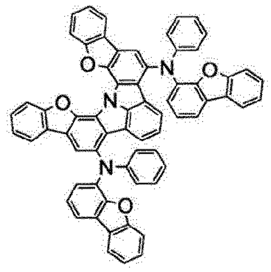
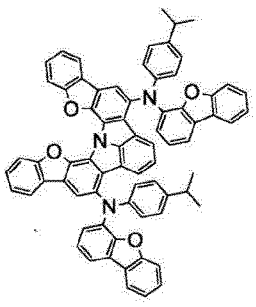
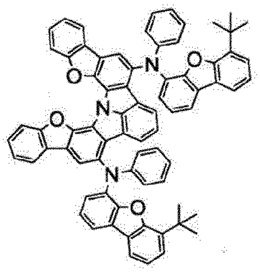
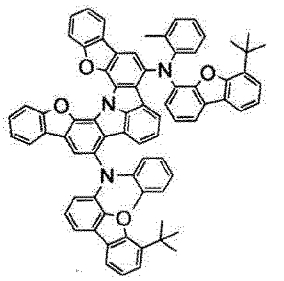
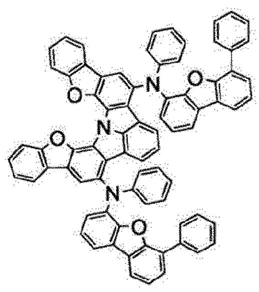
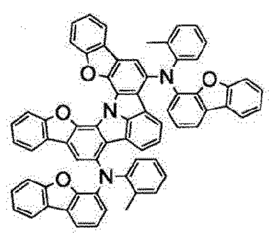

[Formula 112]

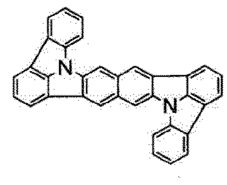
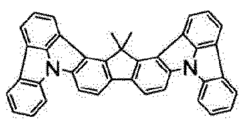
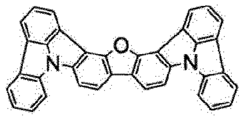
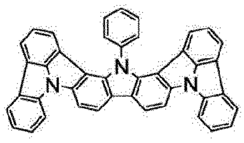
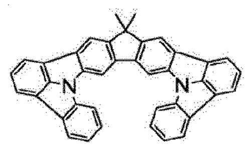
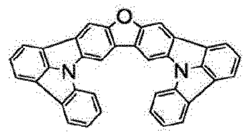
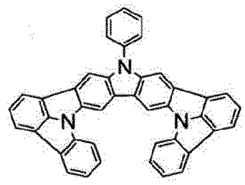
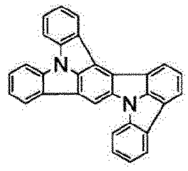
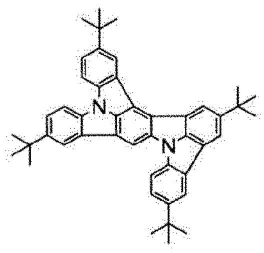
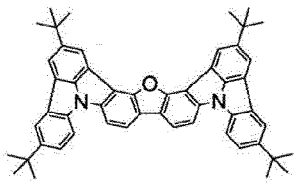

Compound Represented by Formula (5)

The compound represented by the formula (5) will be described below. The compound represented by the formula (5) corresponds to a compound represented by the formula (41-3).

[Formula 113]

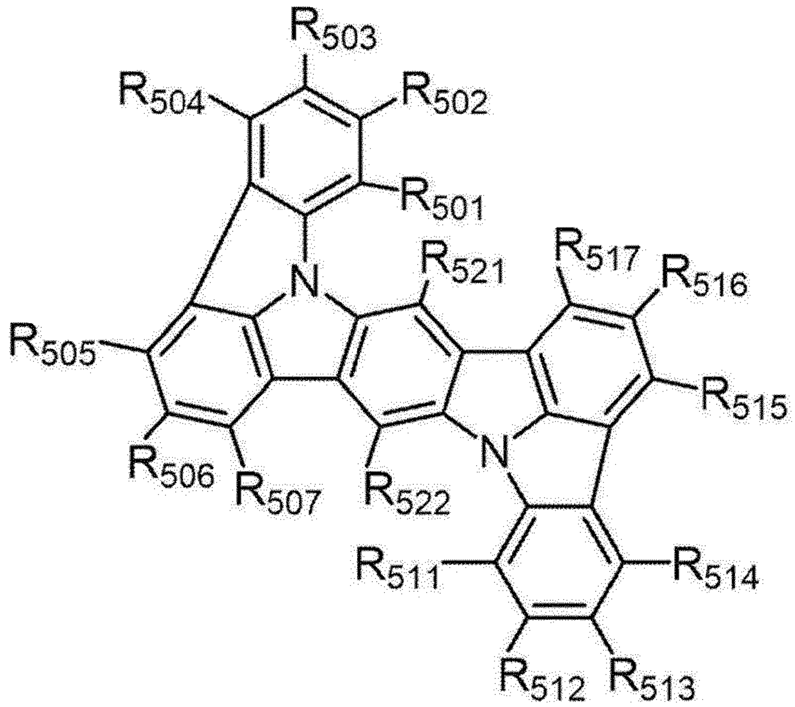

(5)

In the formula (5):

at least one combination of adjacent two or more of $R_{501}$ to $R_{507}$ and $R_{511}$ to $R_{517}$ are mutually bonded to form a substituted or unsubstituted monocyclic ring, mutually bonded to form a substituted or unsubstituted fused ring, or not mutually bonded;

$R_{501}$ to $R_{507}$ and $R_{511}$ to $R_{517}$ forming neither the monocyclic ring nor the fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and

$R_{521}$ and $R_{522}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms.

"A combination of adjacent two or more of $R_{501}$ to $R_{507}$ and $R_{511}$ to $R_{517}$" refers to, for instance, a combination of $R_{501}$ and $R_{502}$, a combination of $R_{502}$ and $R_{503}$, a combination of $R_{503}$ and $R_{504}$, a combination of $R_{505}$ and $R_{506}$, a combination of $R_{506}$ and $R_{507}$, and a combination of $R_{501}$, $R_{502}$, and $R_{503}$.

In an exemplary embodiment, at least one, preferably two, selected from the group consisting of $R_{501}$ to $R_{507}$ and $R_{511}$ to $R_{517}$ are each a group represented by - N($R_{906}$)($R_{907}$).

In an exemplary embodiment, $R_{501}$ to $R_{507}$ and $R_{511}$ to $R_{517}$ are each independently a hydrogen atom, a substituted or unsubstituted aryl group having 6 to 50 ring carbon atoms, or a substituted or unsubstituted heterocyclic group having 5 to 50 ring atoms.

In an exemplary embodiment, the compound represented by the formula (5) is a compound represented by a formula (52) below.

[Formula 114]

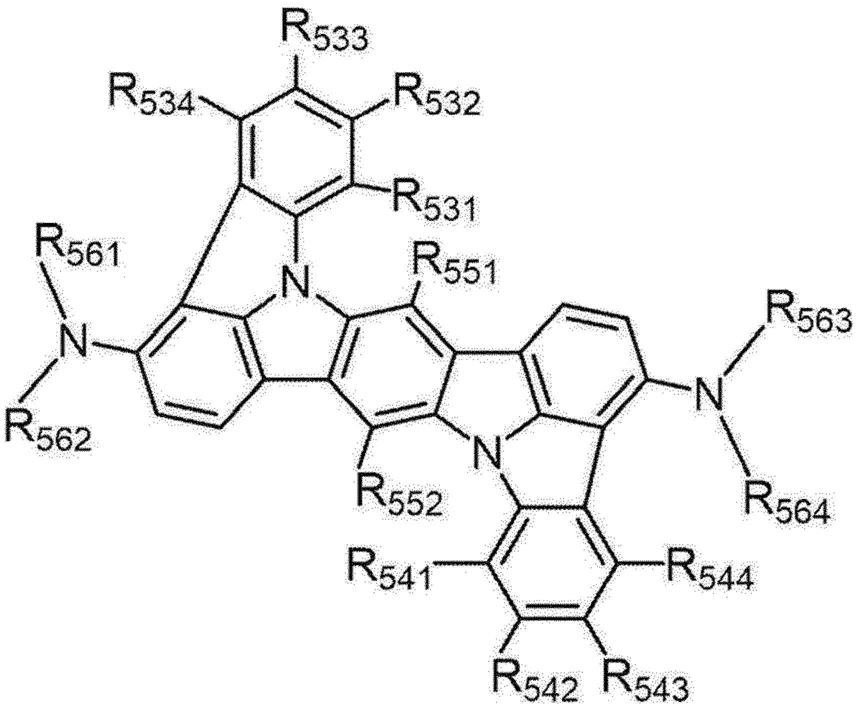

(52)

In the formula (52):

at least one combination of adjacent two or more of $R_{531}$ to $R_{534}$ and $R_{541}$ to $R_{544}$ are mutually bonded to form a substituted or unsubstituted monocyclic ring, mutually bonded to form a substituted or unsubstituted fused ring, or not mutually bonded;

$R_{531}$ to $R_{534}$ and $R_{541}$ to $R_{544}$ forming neither the monocyclic ring nor the fused ring, $R_{551}$, and $R_{552}$ are each independently a hydrogen atom, a substituted or unsubstituted aryl group having 6 to 50 ring carbon atoms, or a substituted or unsubstituted heterocyclic group having 5 to 50 ring atoms; and

$R_{561}$ to $R_{564}$ are each independently a substituted or unsubstituted aryl group having 6 to 50 ring carbon atoms, or a substituted or unsubstituted heterocyclic group having 5 to 50 ring atoms.

In an exemplary embodiment, the compound represented by the formula (5) is a compound represented by a formula (53) below.

[Formula 115]

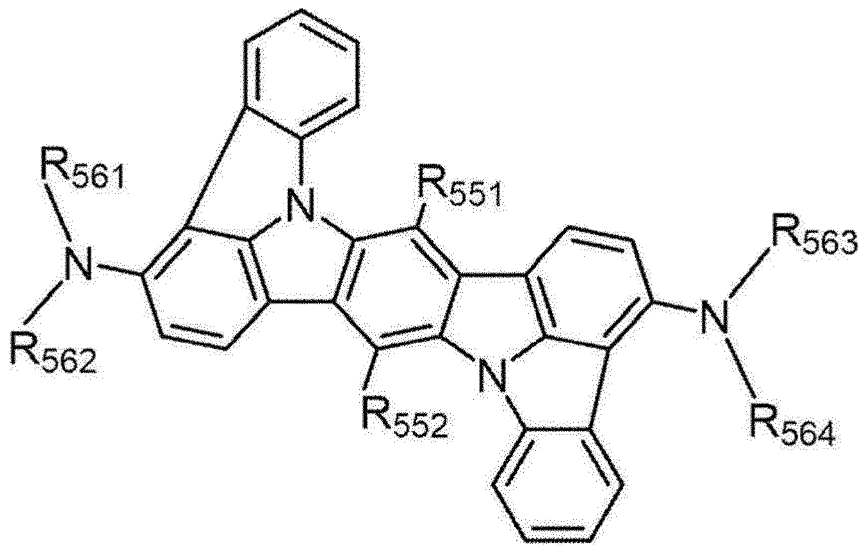

(53)

In the formula (53), $R_{551}$, $R_{552}$ and $R_{561}$ to $R_{564}$ each independently represent the same as $R_{551}$, $R_{552}$ and $R_{561}$ to $R_{564}$ in the formula (52).

In an exemplary embodiment, $R_{561}$ to $R_{564}$ in the formulae (52) and (53) are each independently a substituted or unsubstituted aryl group having 6 to 50 ring carbon atoms (preferably a phenyl group).

In an exemplary embodiment, $R_{521}$ and $R_{522}$ in the formula (5) and $R_{551}$ and $R_{552}$ in the formulae (52) and (53) are each a hydrogen atom.

In an exemplary embodiment, the substituent for the "substituted or unsubstituted" group in the formulae (5), (52) and (53) is a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

Specific examples of the compound represented by the formula (5) include compounds given below.

[Formula 116]

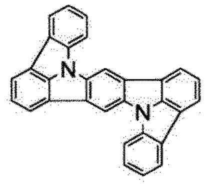
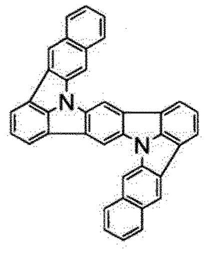
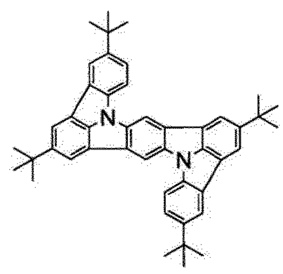
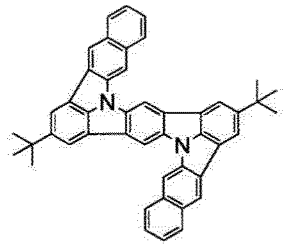
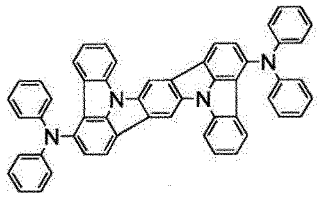
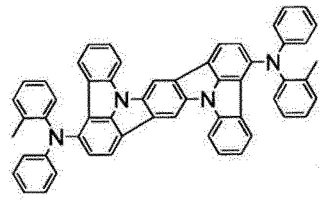
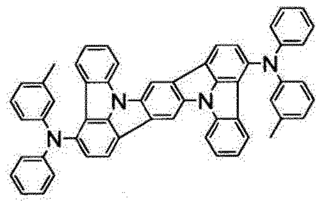
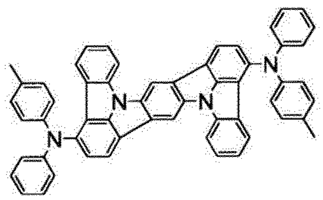
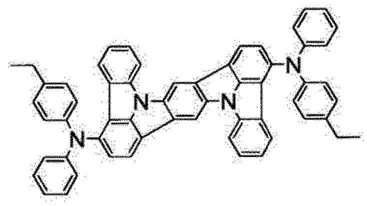
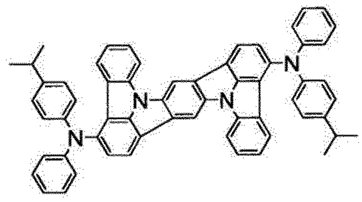

[Formula 117]

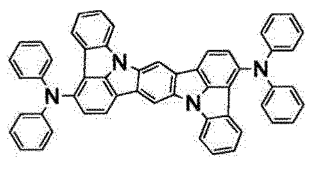
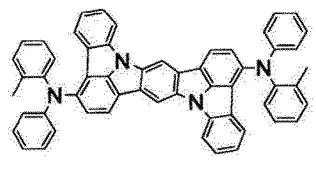
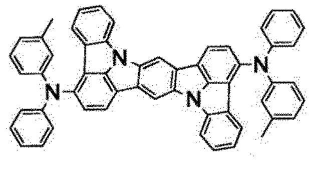
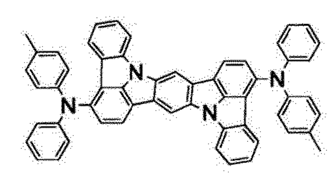
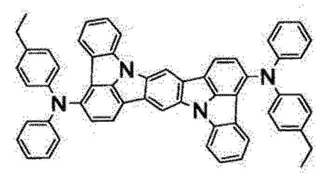
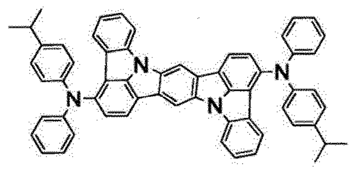
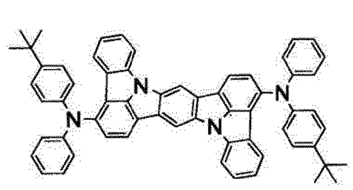
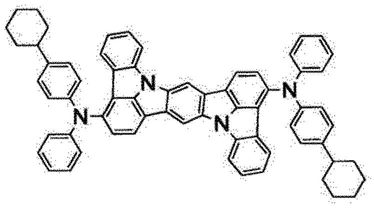
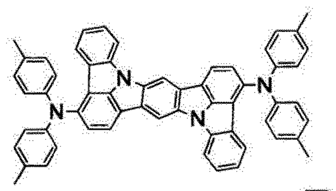
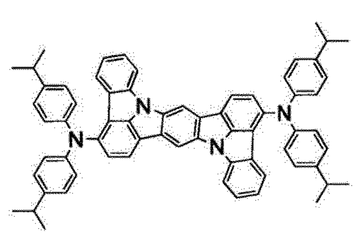
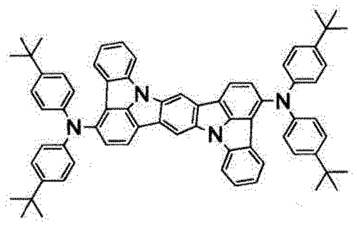
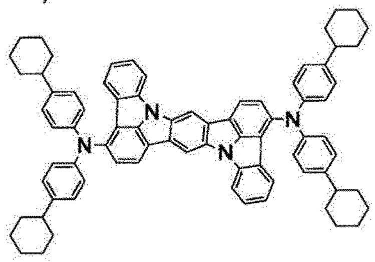
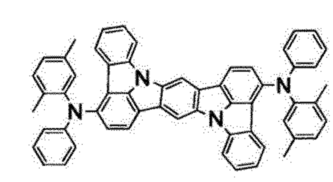
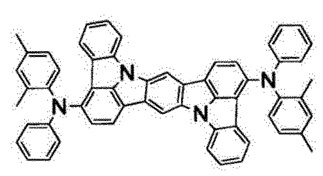
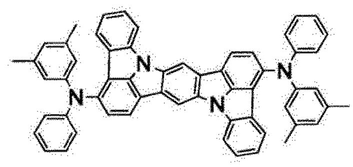
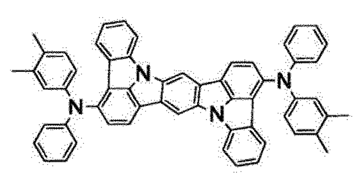
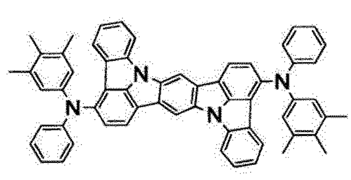
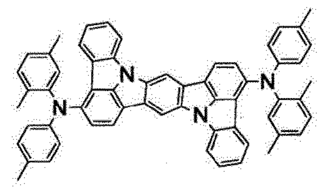
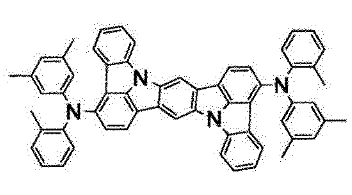
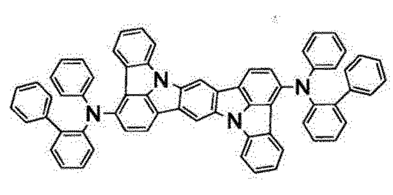
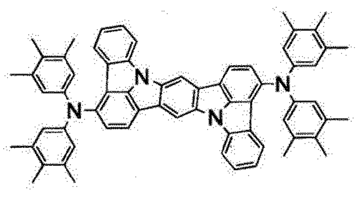
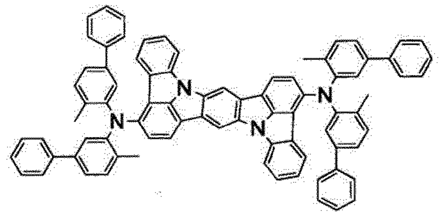
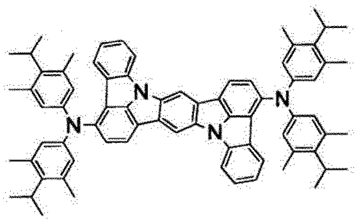
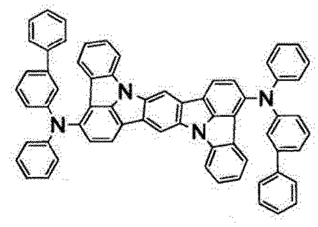

[Formula 118]

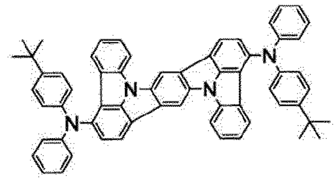
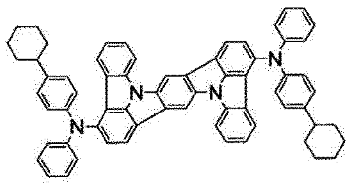
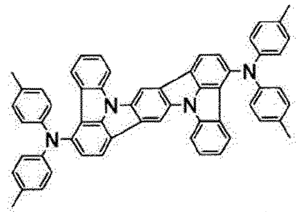
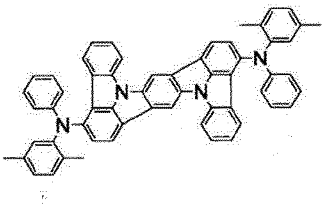
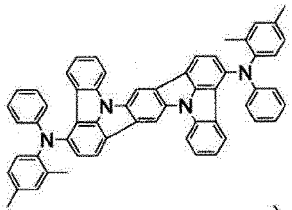
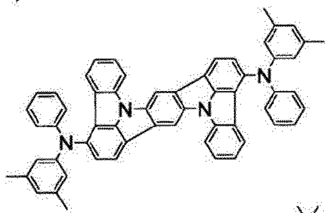
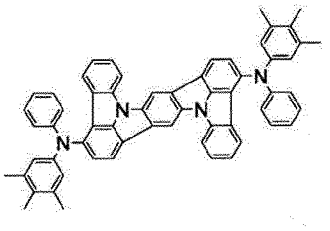
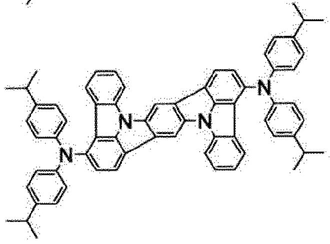
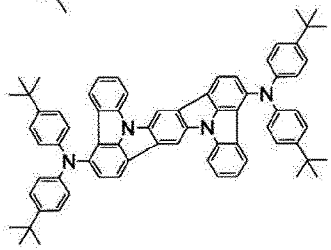
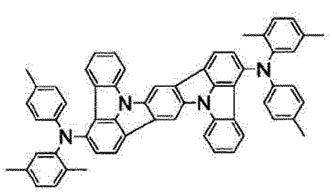
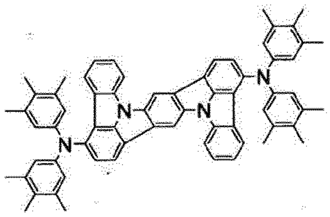
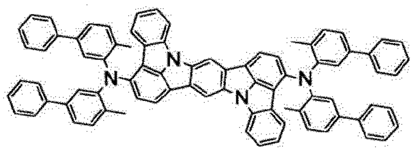
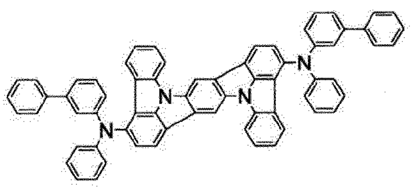
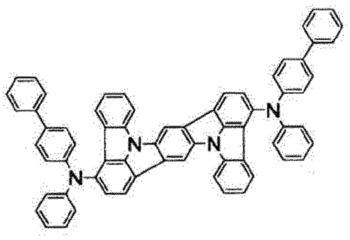
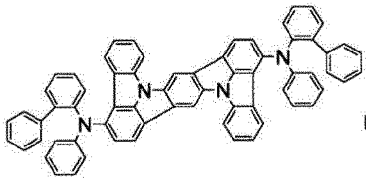
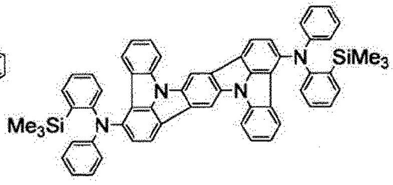
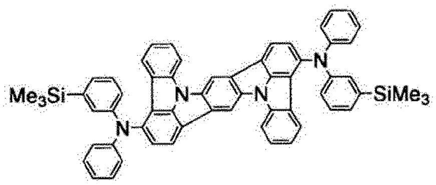
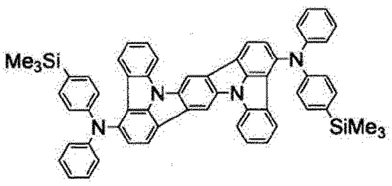
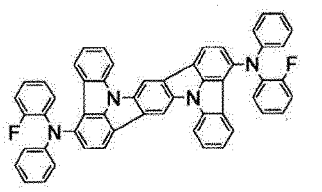
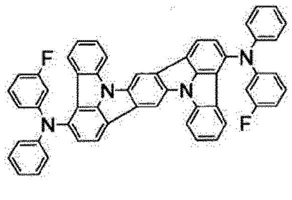

[Formula 119]

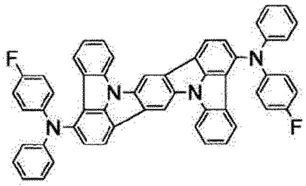
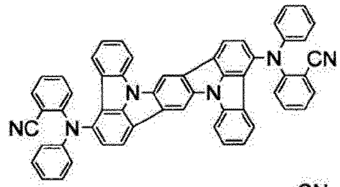
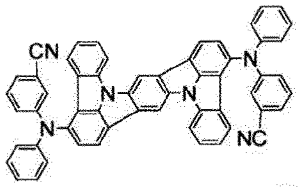
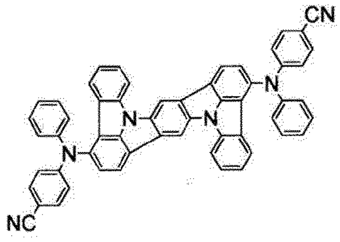
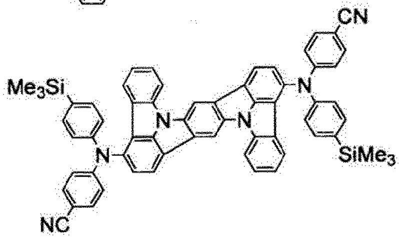
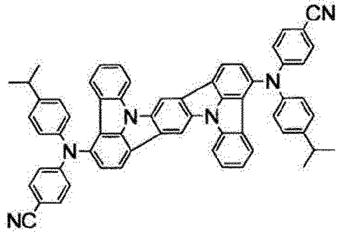
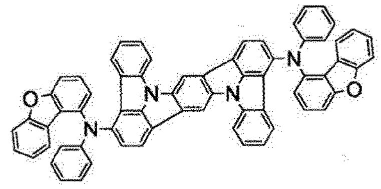
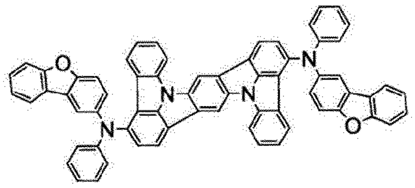
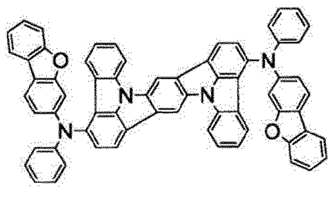
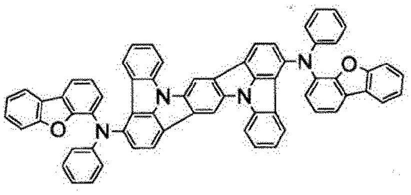
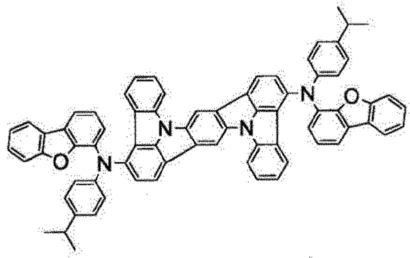
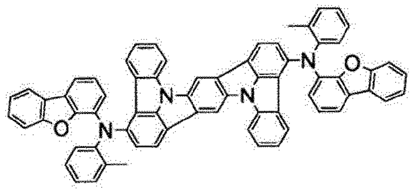
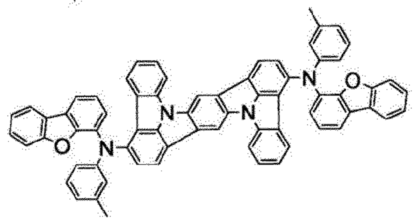
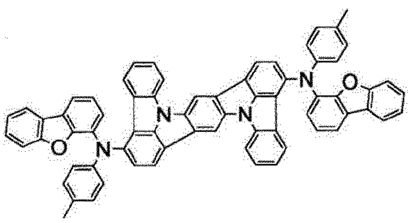
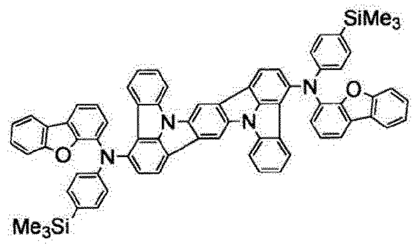
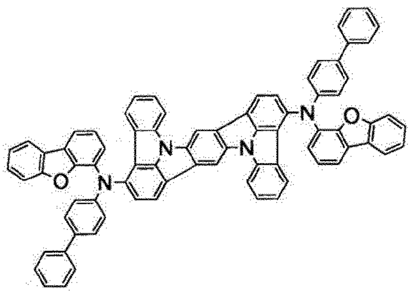
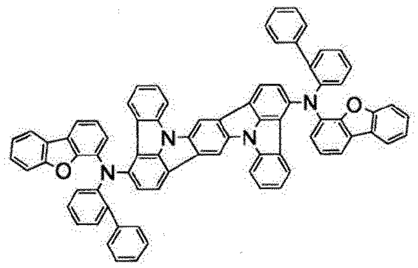

[Formula 120]

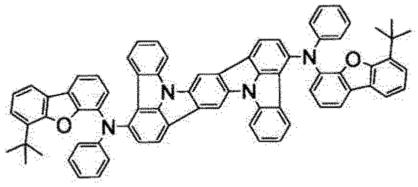
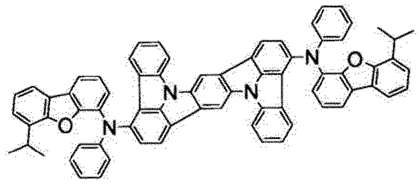
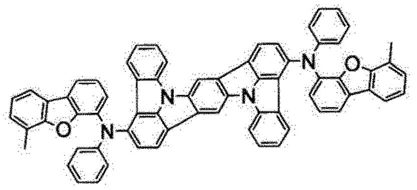
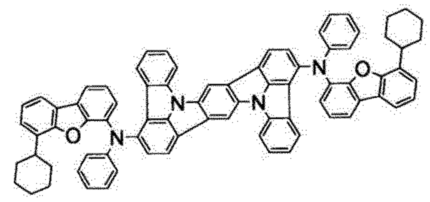
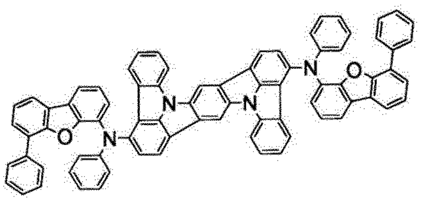
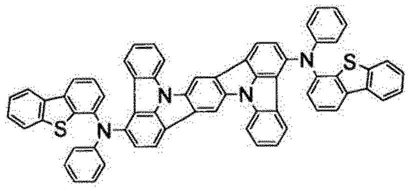
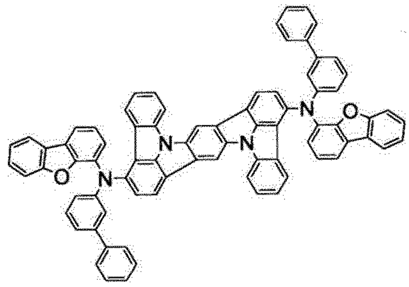
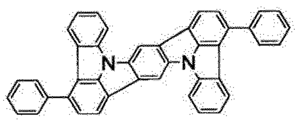
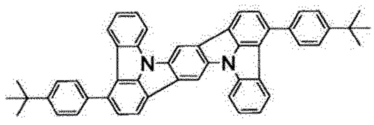
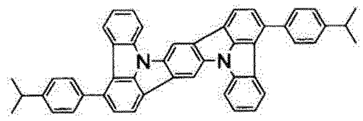
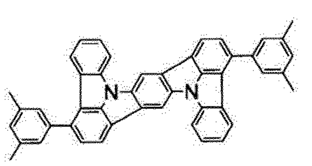
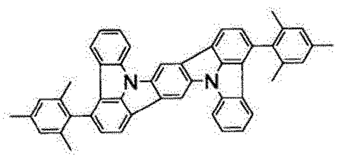
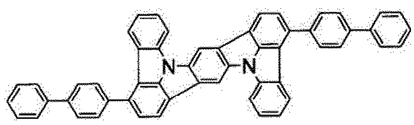
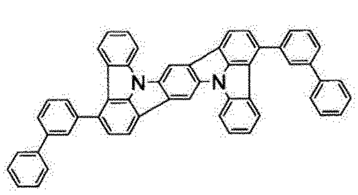
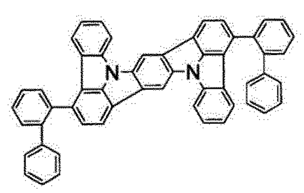
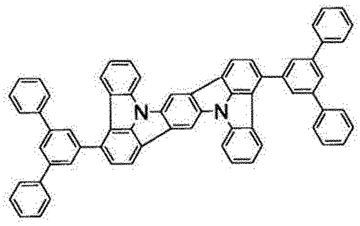
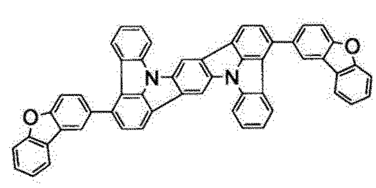
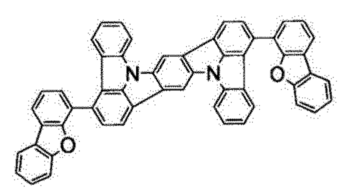
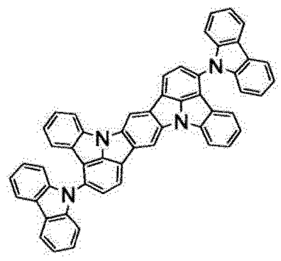

[Formula 121]

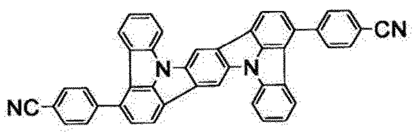

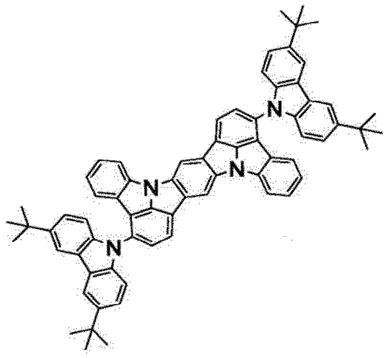

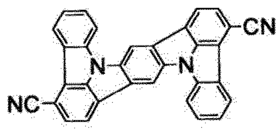

[Formula 122]

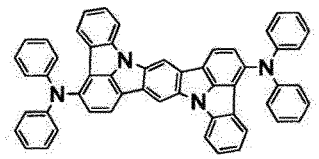
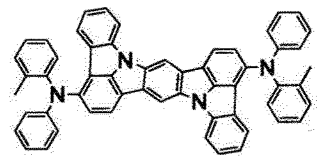
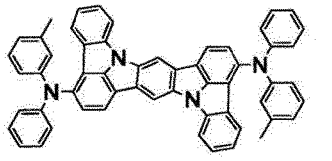

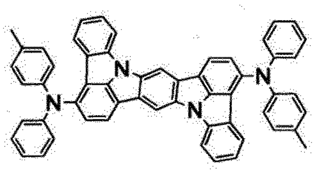
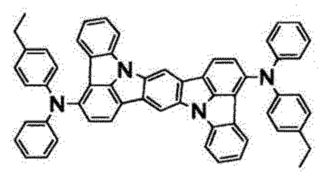
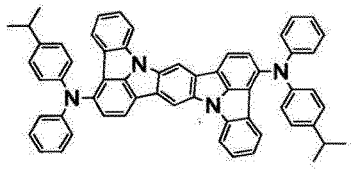

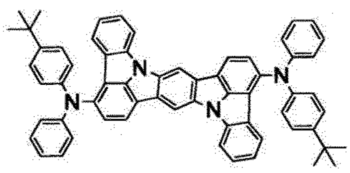
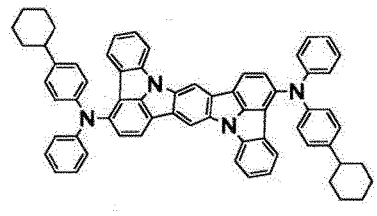
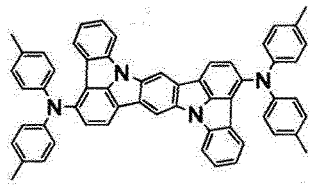

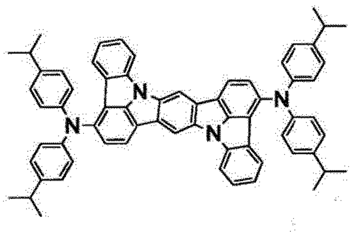
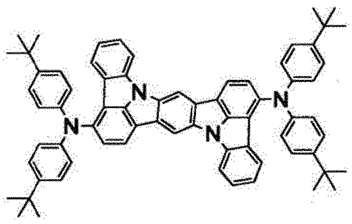
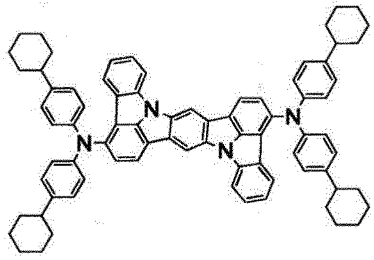

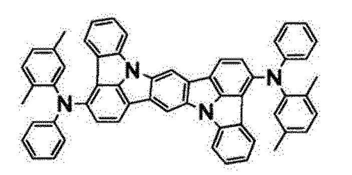
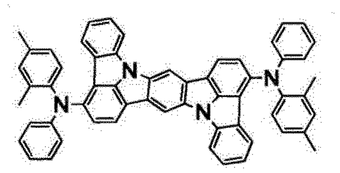
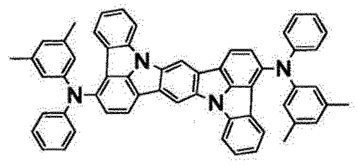

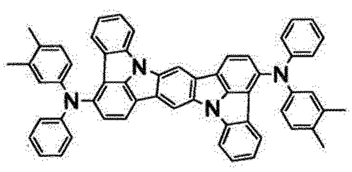
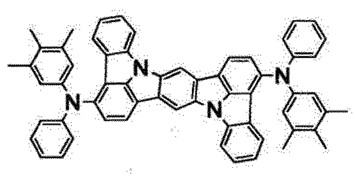
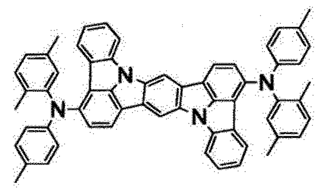

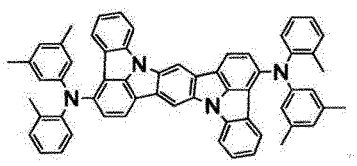
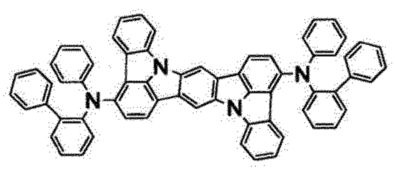
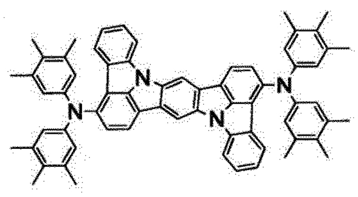

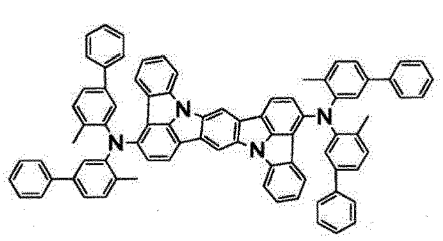
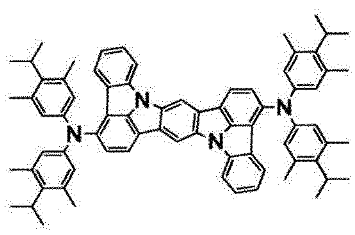
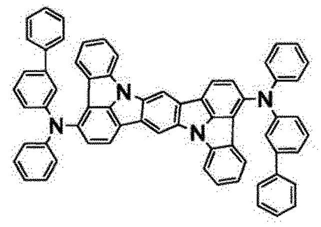

[Formula 123]

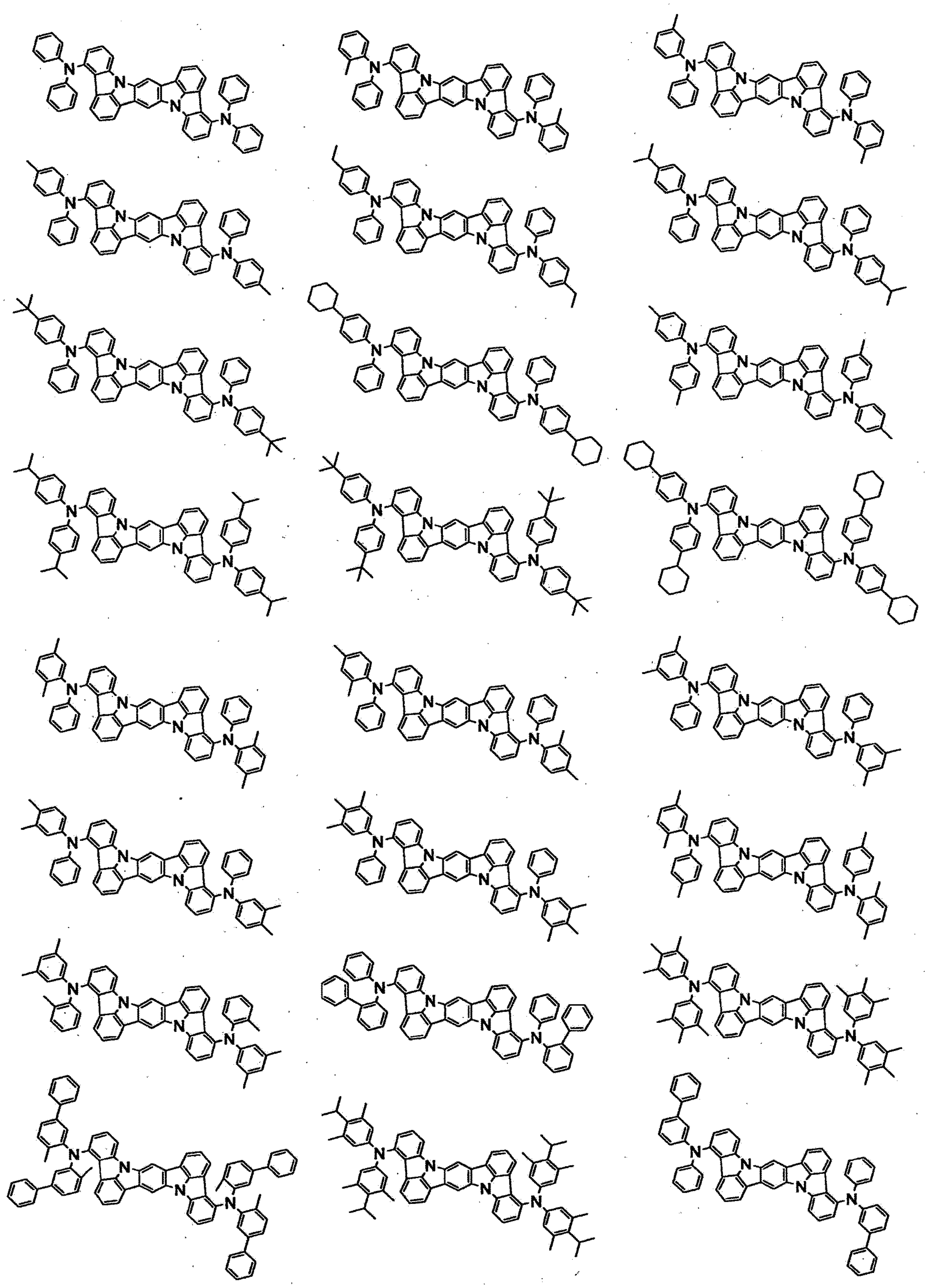

[Formula 124]

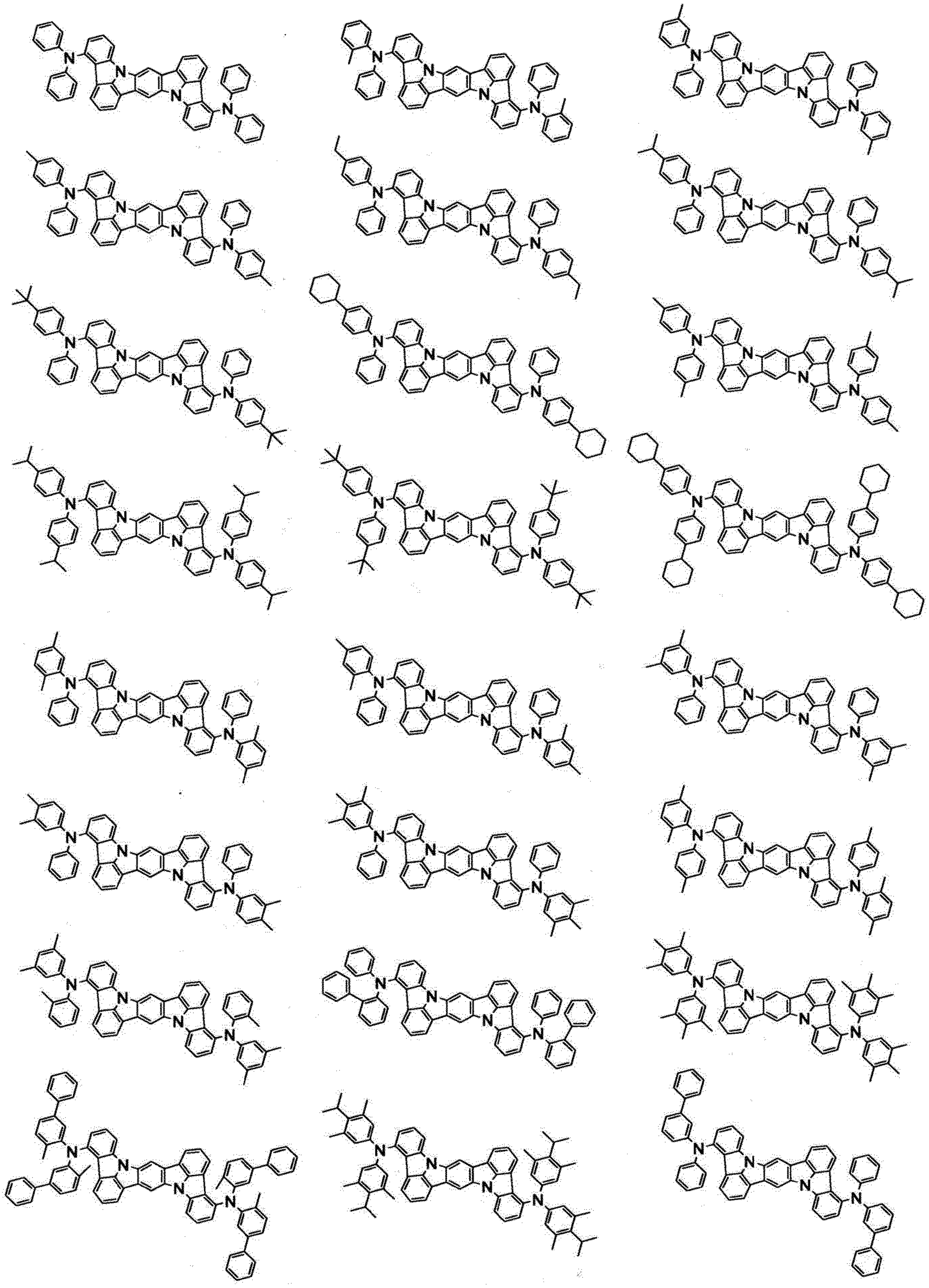

[Formula 125]

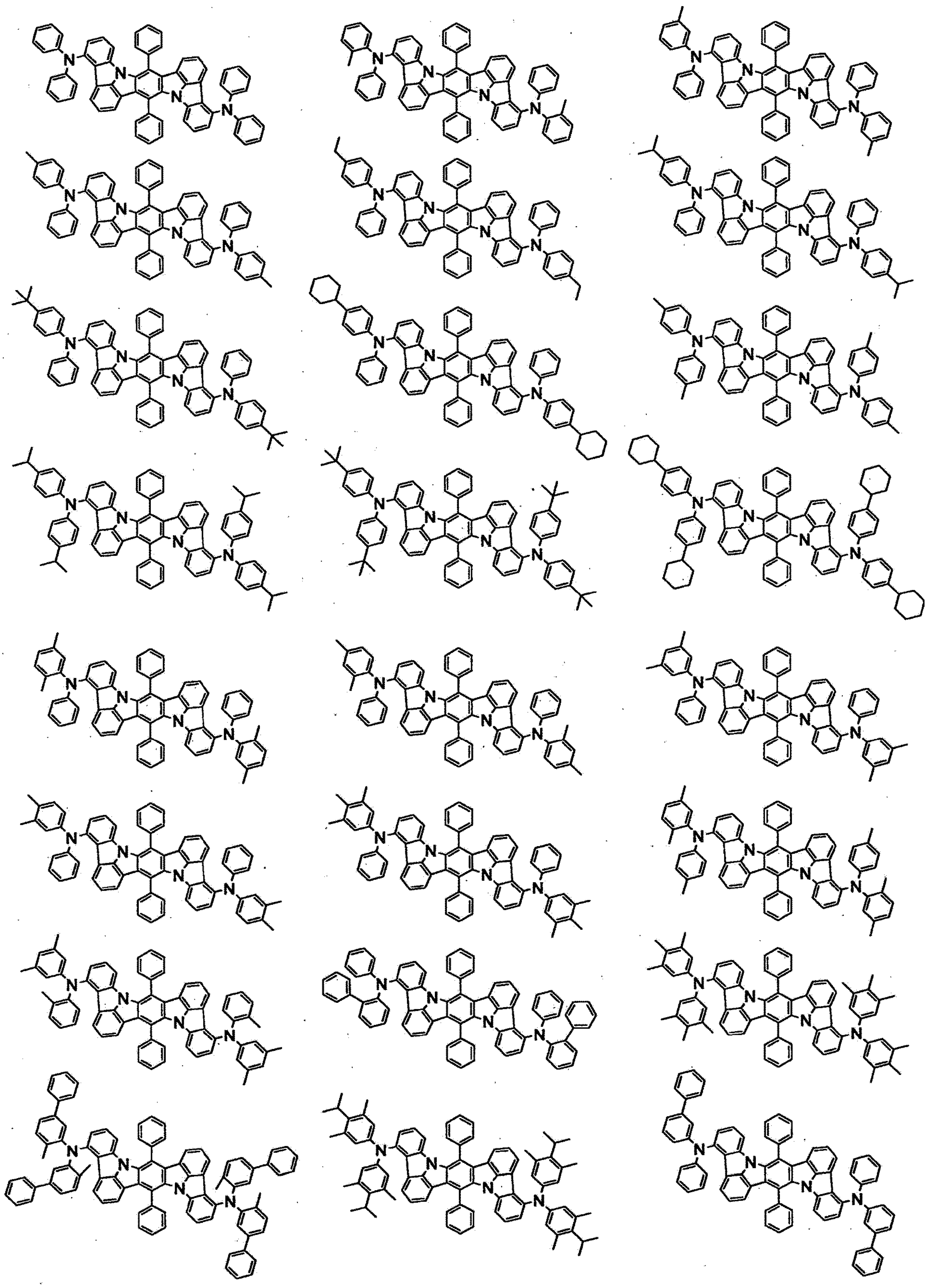

[Formula 126]

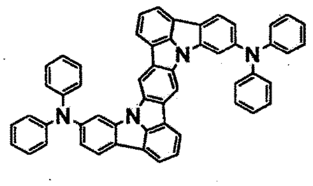
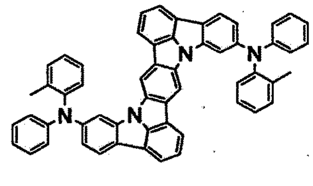
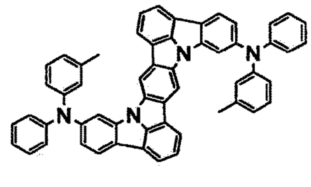
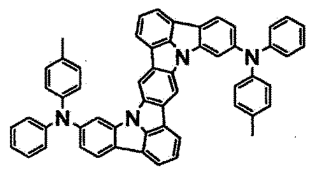
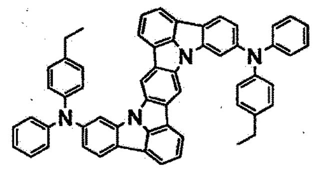
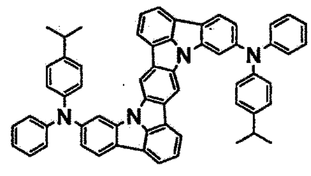
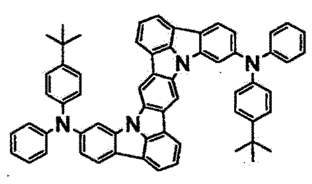
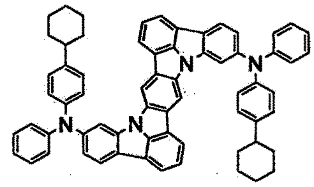
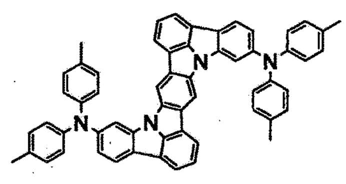
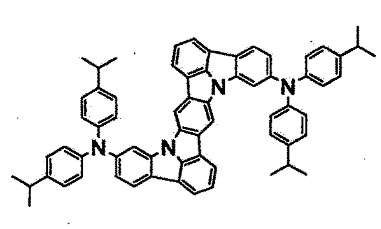
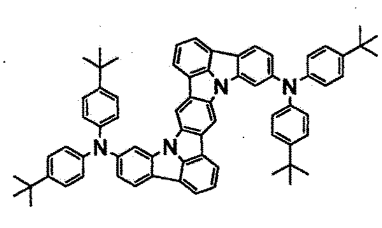
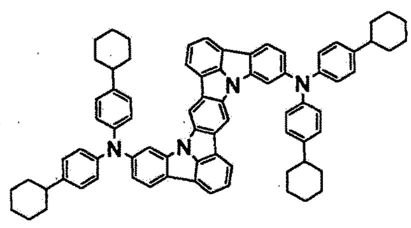
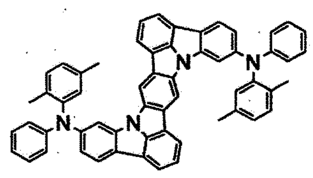
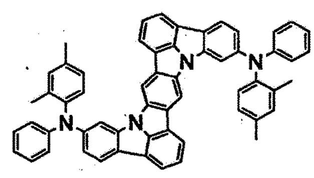
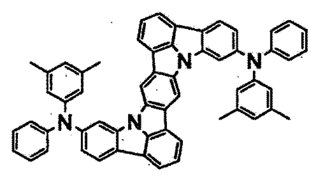
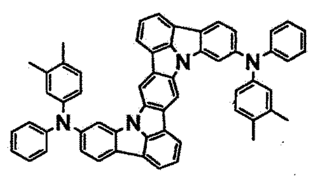
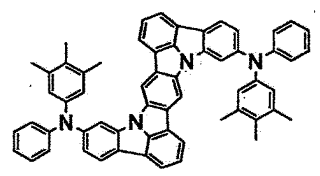
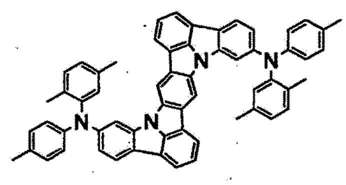
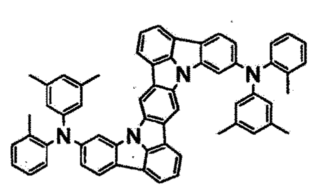
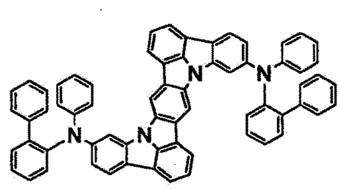
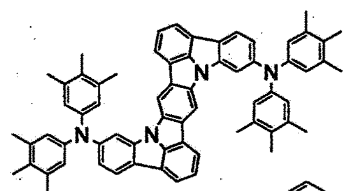
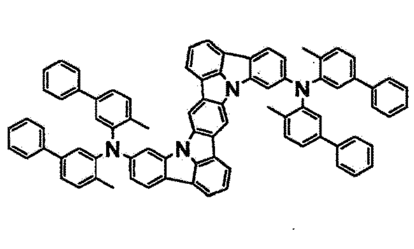
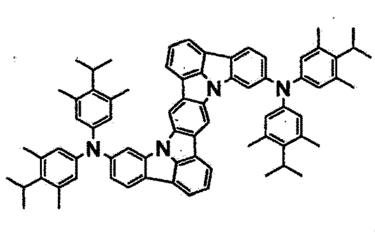
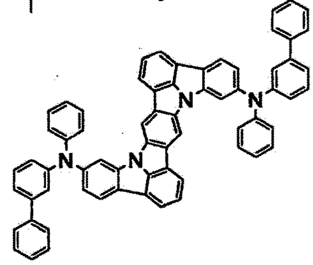

[Formula 127]

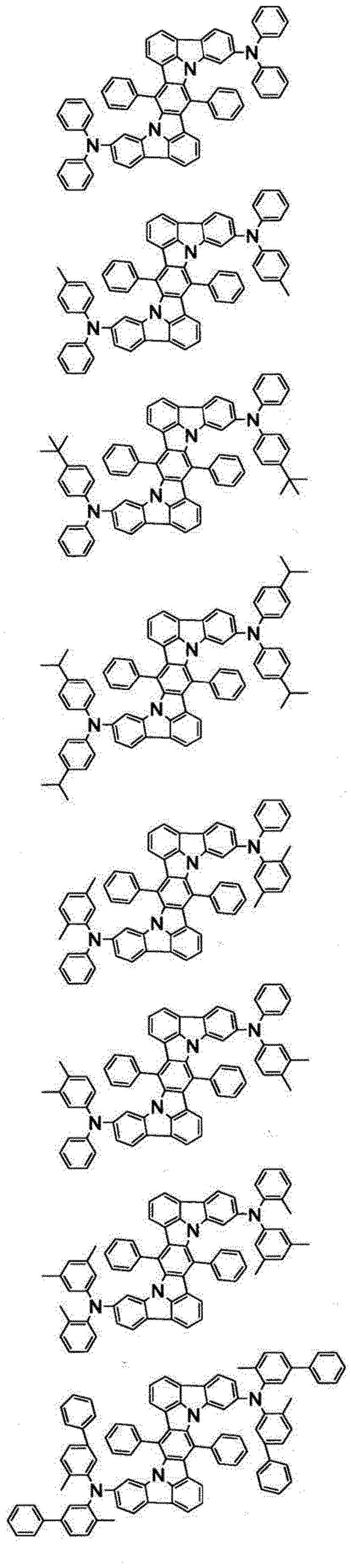

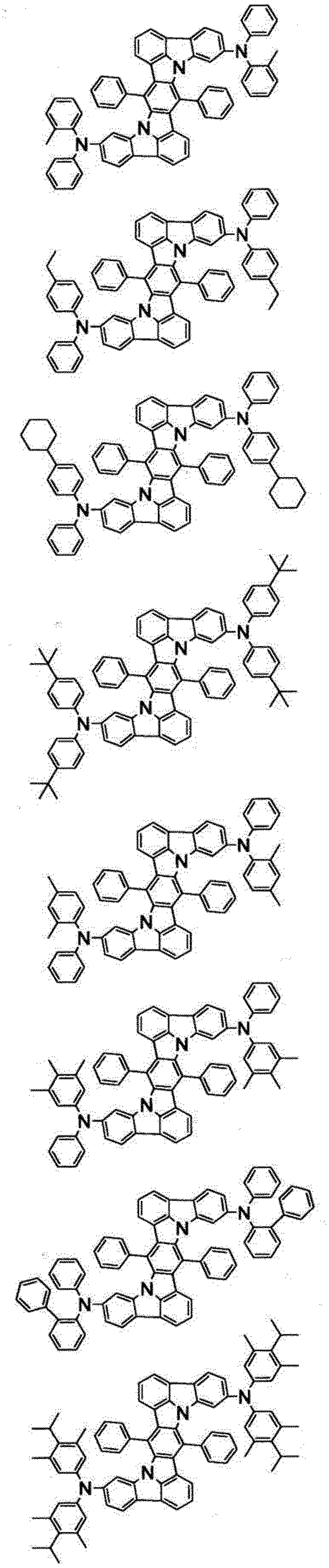

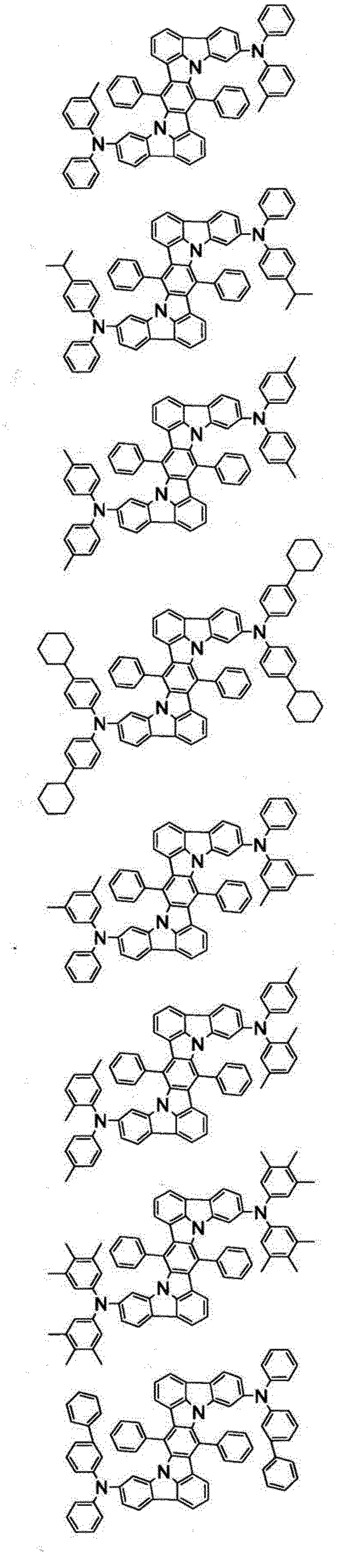

[Formula 128]

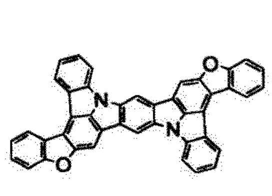
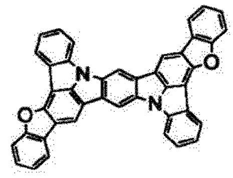
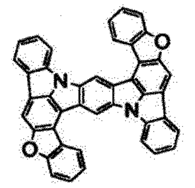
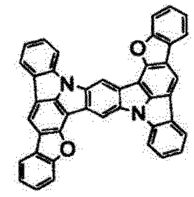
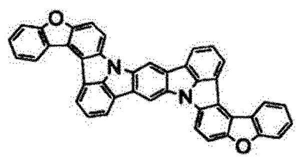
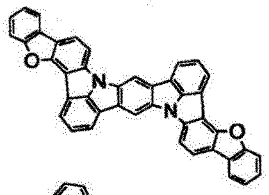
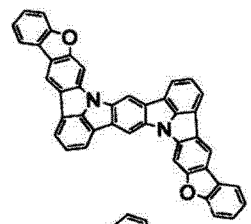
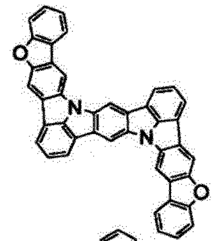
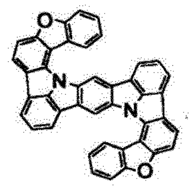
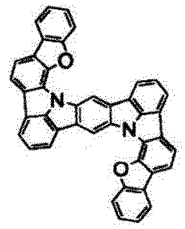
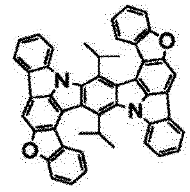
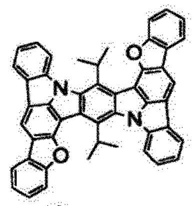
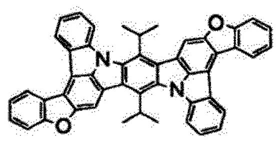
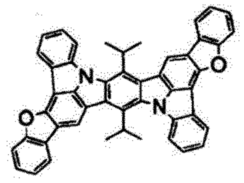
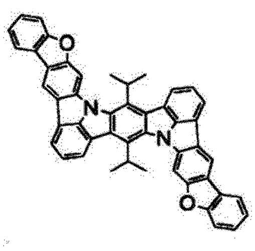
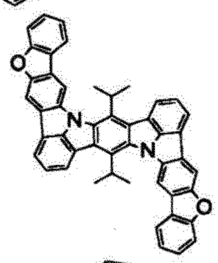
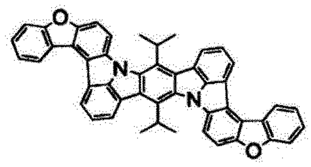
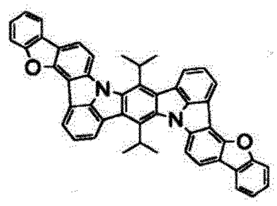
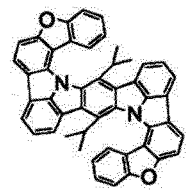
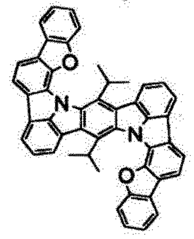
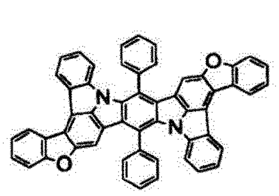
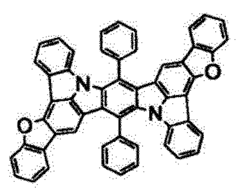
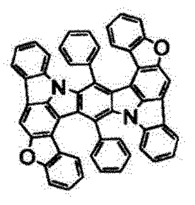
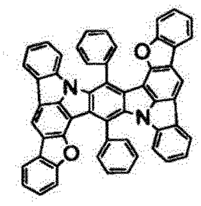
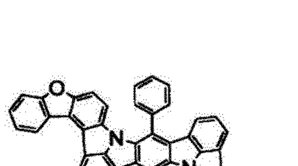
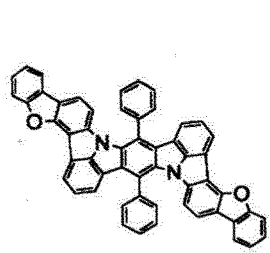
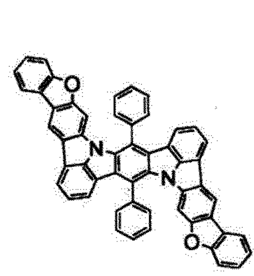
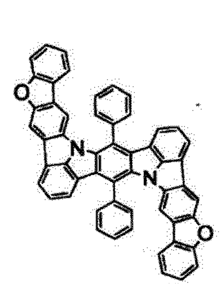

[Formula 129]

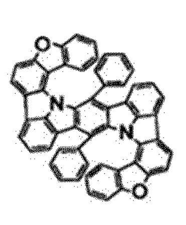 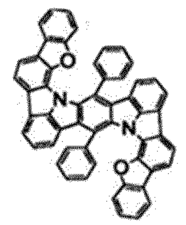 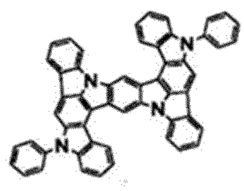 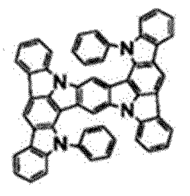

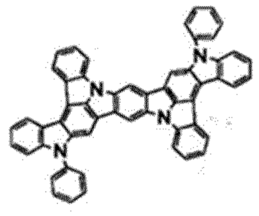 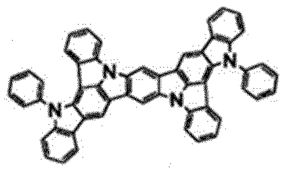 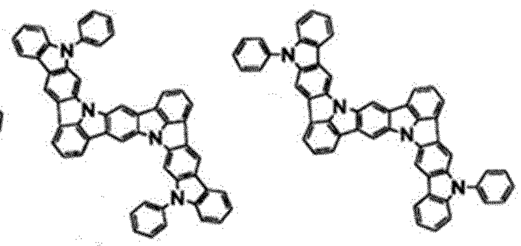

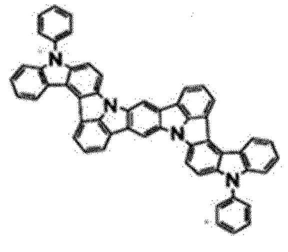 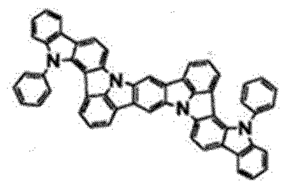 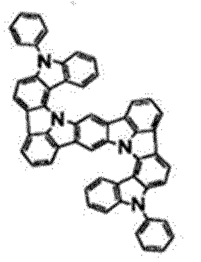 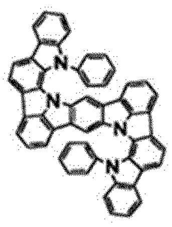

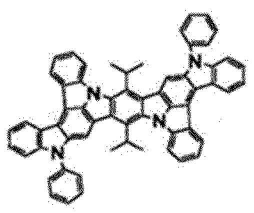 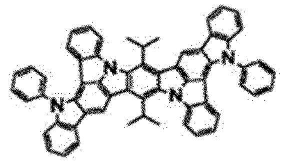 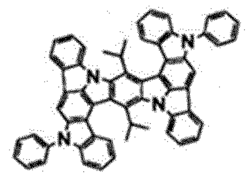 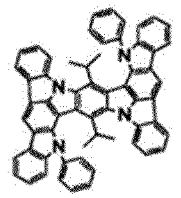

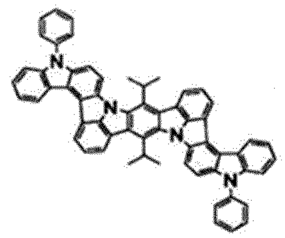 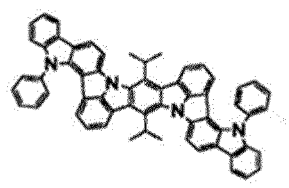 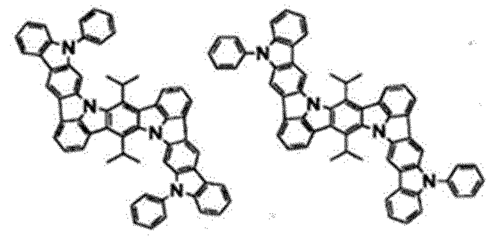

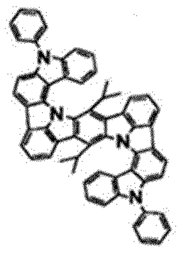 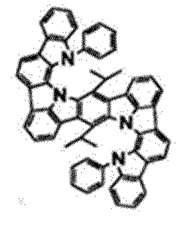 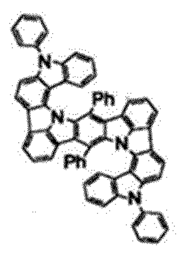 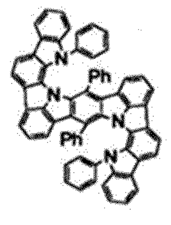

In the formula, Ph is a phenyl group.

[Formula 130]

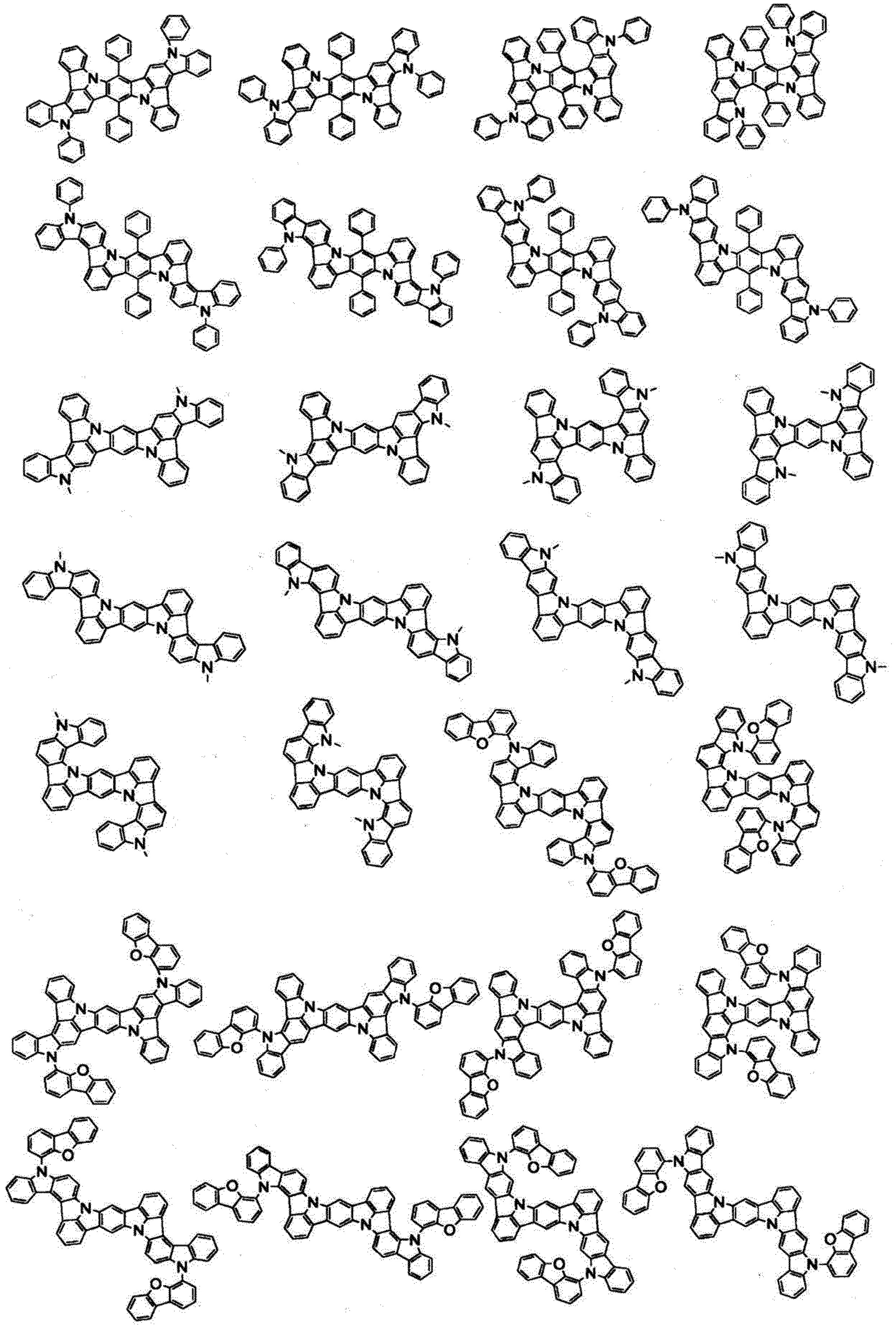

[Formula 131]

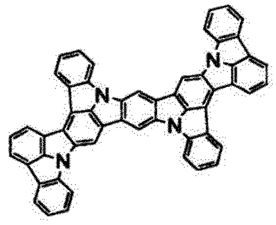
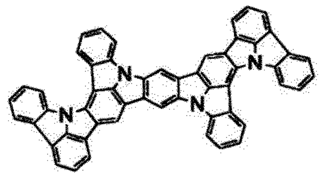
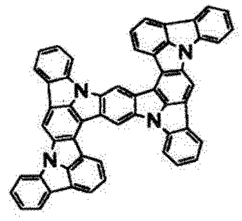
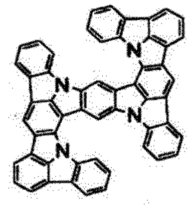
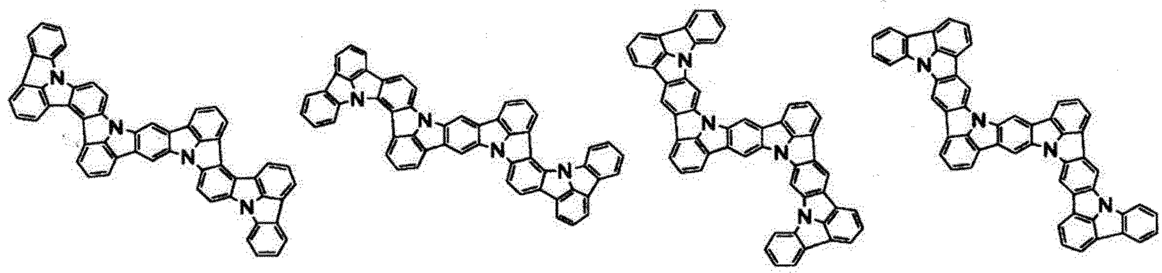
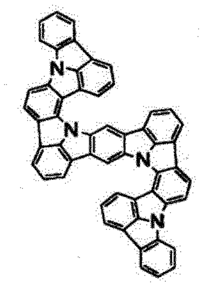
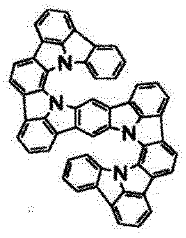
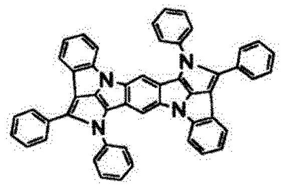
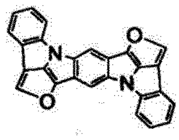
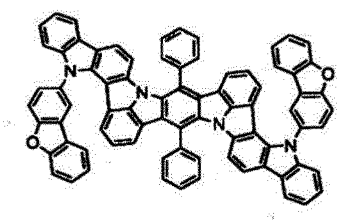
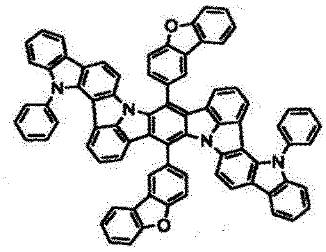
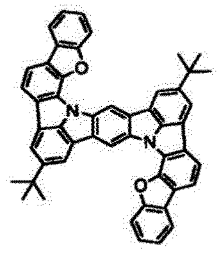
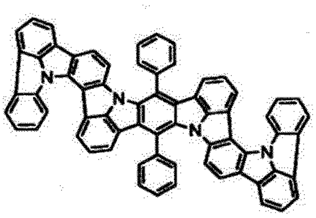
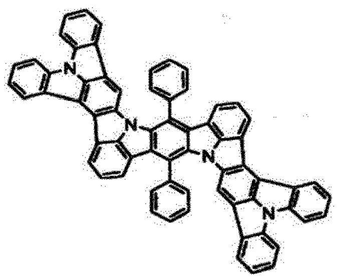

[Formula 132]

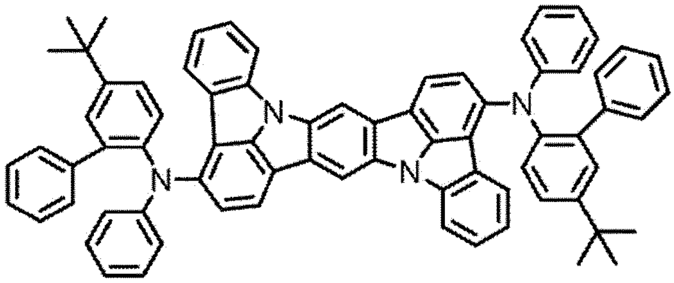

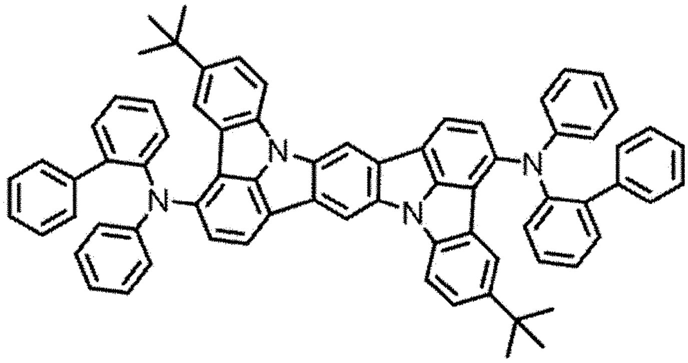

Compound Represented by Formula (6)

The compound represented by the formula (6) will be described below.

[Formula 133]

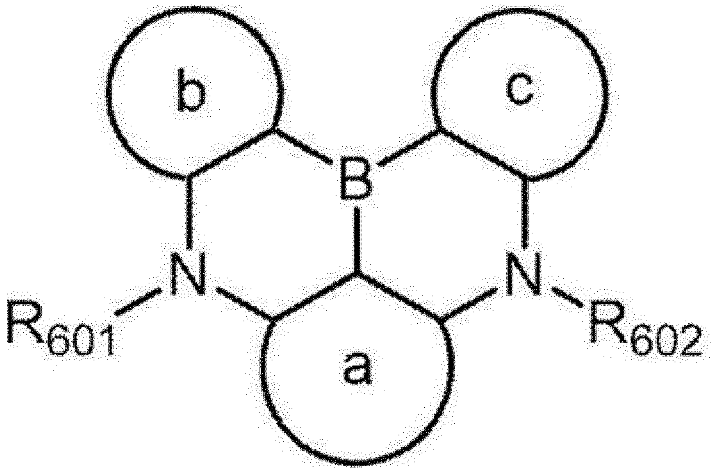

(6)

In the formula (6):

a ring a, a ring b and a ring c are each independently a substituted or unsubstituted aromatic hydrocarbon ring having 6 to 50 ring carbon atoms, or a substituted or unsubstituted heterocycle having 5 to 50 ring atoms;
$R_{601}$ and $R_{602}$ are each independently bonded to the ring a, ring b or ring c to form a substituted or unsubstituted heterocycle, or not bonded thereto to form no substituted or unsubstituted heterocycle; and
$R_{601}$ and $R_{602}$ not forming the substituted or unsubstituted heterocycle are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

The ring a, ring b and ring c are each a ring (a substituted or unsubstituted aromatic hydrocarbon ring having

6 to 50 ring carbon atoms, or a substituted or unsubstituted heterocycle having 5 to 50 ring atoms) fused with a fused bicyclic structure formed of a boron atom and two nitrogen atoms at the center of the formula (6).

The "aromatic hydrocarbon ring" for the rings a, b, and c has the same structure as a compound formed by introducing a hydrogen atom to the "aryl group" described above.

Ring atoms of the "aromatic hydrocarbon ring" for the ring a include three carbon atoms on the fused bicyclic structure at the center of the formula (6).

Ring atoms of the "aromatic hydrocarbon ring" for the rings b and c include two carbon atoms on the fused bicyclic structure at the center of the formula (6).

Specific examples of the "substituted or unsubstituted aromatic hydrocarbon ring having 6 to 50 ring carbon atoms" include a compound formed by introducing a hydrogen atom to the "aryl group" described in the specific example group G1.

The "heterocycle" for the rings a, b, and c has the same structure as a compound formed by introducing a hydrogen atom to the "heterocyclic group" described above.

Ring atoms of the "heterocycle" for the ring a include three carbon atoms on the fused bicyclic structure at the center of the formula (6). Ring atoms of the "heterocycle" for the rings b and c include two carbon atoms on the fused bicyclic structure at the center of the formula (6). Specific examples of the "substituted or unsubstituted heterocycle having 5 to 50 ring atoms" include a compound formed by introducing a hydrogen atom to the "heterocyclic group" described in the specific example group G2.

$R_{601}$ and $R_{602}$ may be each independently bonded with the ring a, ring b, or ring c to form a substituted or unsubstituted heterocycle. The "heterocycle" in this arrangement includes a nitrogen atom on the fused bicyclic structure at the center of the formula (6). The heterocycle in the above arrangement optionally includes a hetero atom other than the nitrogen atom. $R_{601}$ and $R_{602}$ being bonded with the ring a, ring b, or ring c specifically means that atoms forming $R_{601}$ and $R_{602}$ are bonded with atoms forming the ring a, ring b, or ring c. For instance, $R_{601}$ may be bonded with the ring a to form a bicyclic (or tri-or-more cyclic) fused nitrogen-containing heterocycle, in which the ring including $R_{601}$ and the ring a are fused. Specific examples of the nitrogen-containing heterocycle include a compound corresponding to the nitrogen-containing bi(or-more)cyclic fused heterocyclic group in the specific example group G2.

The same applies to $R_{601}$ bonded with the ring b, $R_{602}$ bonded with the ring a, and $R_{602}$ bonded with the ring c.

In an exemplary embodiment, the ring a, ring b and ring c in the formula (6) are each independently a substituted or unsubstituted aromatic hydrocarbon ring having 6 to 50 ring carbon atoms.

In an exemplary embodiment, the ring a, ring b and ring c in the formula (6) are each independently a substituted or unsubstituted benzene ring or a substituted or unsubstituted naphthalene ring.

In an exemplary embodiment, $R_{601}$ and $R_{602}$ in the formula (6) are each independently a substituted or unsubstituted aryl group having 6 to 50 ring carbon atoms, or a substituted or unsubstituted heterocyclic group having 5 to 50 ring atoms, preferably, a substituted or unsubstituted aryl group having 6 to 50 ring carbon atoms.

In an exemplary embodiment, the compound represented by the formula (6) is a compound represented by a formula (62) below.

[Formula 134]

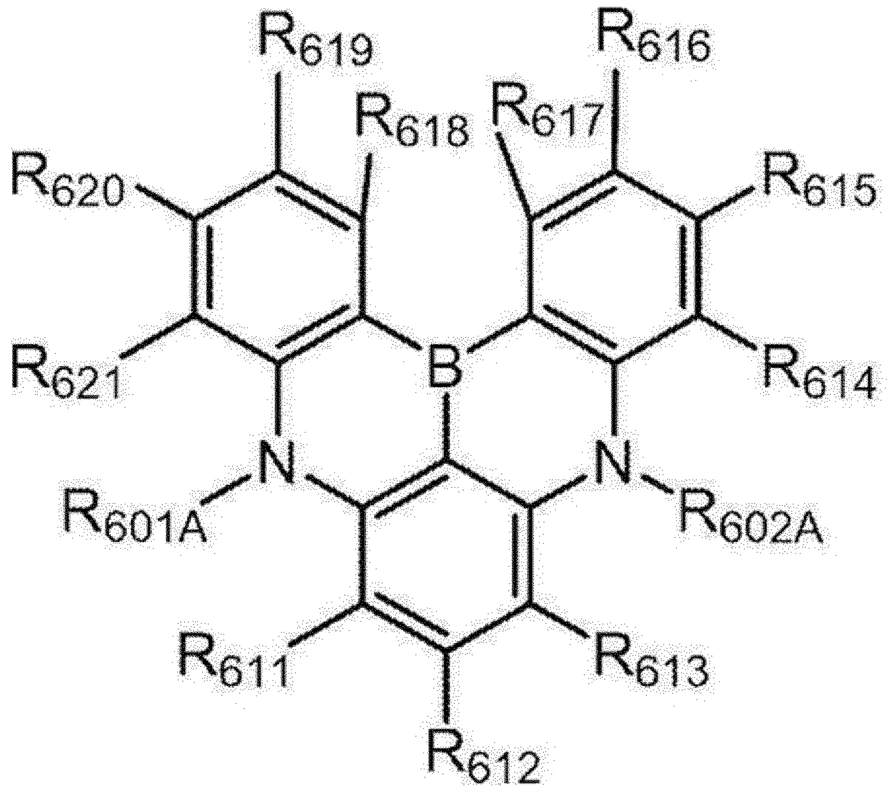

(62)

In the formula (62):

$R_{601A}$ is bonded with at least one of $R_{611}$ or $R_{621}$ to form a substituted or unsubstituted heterocycle, or not bonded therewith to form no substituted or unsubstituted heterocycle;
$R_{602A}$ is bonded with at least one of $R_{613}$ or $R_{614}$ to form a substituted or unsubstituted heterocycle, or not bonded therewith to form no substituted or unsubstituted heterocycle;
$R_{601A}$ and $R_{602A}$ not forming the substituted or unsubstituted heterocycle are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
at least one combination of adjacent two or more of $R_{611}$ to $R_{621}$ are mutually bonded to form a substituted or unsubstituted monocyclic ring, mutually bonded to form a substituted or unsubstituted fused ring, or not mutually bonded; and
$R_{611}$ to $R_{621}$ not forming the substituted or unsubstituted heterocycle, not forming the monocyclic ring, and not forming the fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

$R_{601A}$ and $R_{602A}$ in the formula (62) are groups corresponding to $R_{601}$ and $R_{602}$ in the formula (6), respectively.
For instance, $R_{601A}$ and $R_{611}$ are optionally bonded with each other to form a bicyclic (or tri-or-more cyclic) fused nitrogen-containing heterocycle, in which the ring including $R_{601A}$ and $R_{611}$ and a benzene ring corresponding to the ring a are fused. Specific examples of the nitrogen-containing heterocycle include a compound corresponding to the nitrogen-containing bi(or-more)cyclic fused heterocyclic group in the specific example group G2. The same applies to $R_{601A}$ bonded with $R_{621}$, $R_{602A}$ bonded with $R_{613}$, and $R_{602A}$ bonded with $R_{614}$.

At least one combination of adjacent two or more of $R_{611}$ to $R_{621}$ may be mutually bonded to form a substituted or unsubstituted monocyclic ring, or mutually bonded to form a substituted or unsubstituted fused ring.

For instance, $R_{611}$ and $R_{612}$ are optionally mutually bonded to form a structure in which a benzene ring, indole ring, pyrrole ring, benzofuran ring, benzothiophene ring or the like is fused to the six-membered ring bonded with $R_{611}$ and $R_{612}$, the resultant fused ring forming a naphthalene ring, carbazole ring, indole ring, dibenzofuran ring, or dibenzothiophene ring.

In an exemplary embodiment, $R_{611}$ to $R_{621}$ not contributing to ring formation are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

In an exemplary embodiment, $R_{611}$ to $R_{621}$ not contributing to ring formation are each independently a hydrogen atom, a substituted or unsubstituted aryl group having 6 to 50 ring carbon atoms, or a substituted or unsubstituted heterocyclic group having 5 to 50 ring atoms.

In an exemplary embodiment, $R_{611}$ to $R_{621}$ not contributing to ring formation are each independently a hydrogen atom, or a substituted or unsubstituted alkyl group having 1 to 50 carbon atoms.

In an exemplary embodiment, $R_{611}$ to $R_{621}$ not contributing to ring formation are each independently a hydrogen atom, or a substituted or unsubstituted alkyl group having 1 to 50 carbon atoms; and

at least one of $R_{611}$ to $R_{621}$ is a substituted or unsubstituted alkyl group having 1 to 50 carbon atoms.

In an exemplary embodiment, the compound represented by the formula (62) is a compound represented by a formula (63) below.

[Formula 135]

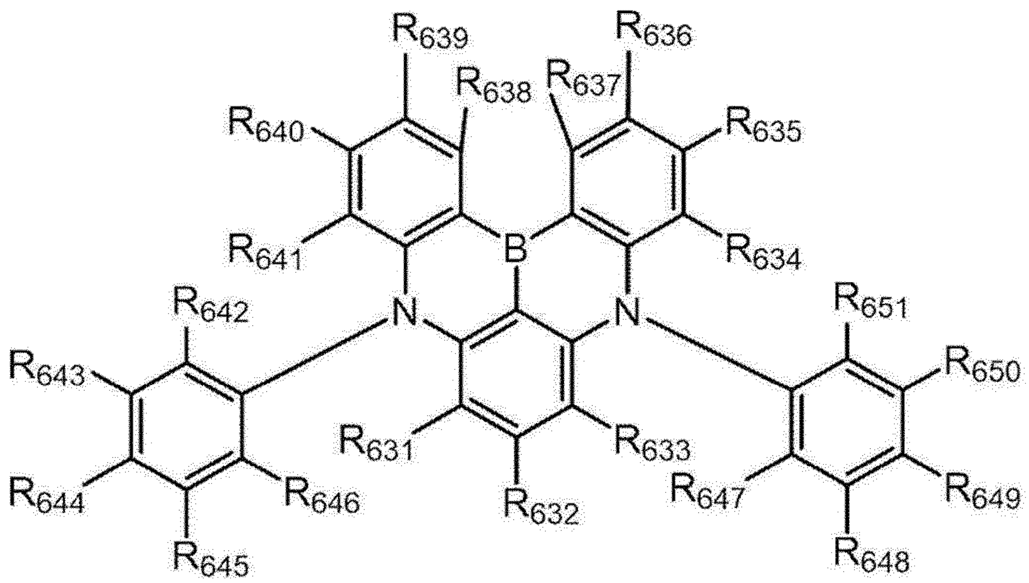

(63)

In the formula (63):

$R_{631}$ is bonded with $R_{646}$ to form a substituted or unsubstituted heterocycle, or not bonded therewith to form no substituted or unsubstituted heterocycle;

$R_{633}$ is bonded with $R_{647}$ to form a substituted or unsubstituted heterocycle, or not bonded therewith to form no substituted or unsubstituted heterocycle;

$R_{634}$ is bonded with $R_{651}$ to form a substituted or unsubstituted heterocycle, or not bonded therewith to form no substituted or unsubstituted heterocycle;

$R_{641}$ is bonded with $R_{642}$ to form a substituted or unsubstituted heterocycle, or not bonded therewith to form no substituted or unsubstituted heterocycle;

at least one combination of adjacent two or more of $R_{631}$ to $R_{651}$ are mutually bonded to form a substituted or unsubstituted monocyclic ring, mutually bonded to form a substituted or unsubstituted fused ring, or not mutually bonded; and

$R_{631}$ to $R_{651}$ not forming the substituted or unsubstituted heterocycle, not forming the monocyclic ring, and not forming the fused ring are each independently a hydrogen atom, a substituted or unsubstituted alkyl group having

1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms.

$R_{631}$ is optionally bonded with $R_{646}$ to form a substituted or unsubstituted heterocycle. For instance, $R_{631}$ and $R_{646}$ are optionally bonded with each other to form a tri-or-more cyclic fused nitrogen-containing heterocycle, in which a benzene ring bonded with $R_{646}$, a ring including a nitrogen atom, and a benzene ring corresponding to the ring a are fused. Specific examples of the nitrogen-containing heterocycle include a compound corresponding to a nitrogen-containing tri(-or-more)cyclic fused heterocyclic group in the specific example group G2. The same applies to $R_{633}$ bonded with $R_{647}$, $R_{634}$ bonded with $R_{651}$, and $R_{641}$ bonded with $R_{642}$.

In an exemplary embodiment, $R_{631}$ to $R_{651}$ not contributing to ring formation are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

In an exemplary embodiment, $R_{631}$ to $R_{651}$ not contributing to ring formation are each independently a hydrogen atom, a substituted or unsubstituted aryl group having 6 to 50 ring carbon atoms, or a substituted or unsubstituted heterocyclic group having 5 to 50 ring atoms.

In an exemplary embodiment, $R_{631}$ to $R_{651}$ not contributing to ring formation are each independently a hydrogen atom, or a substituted or unsubstituted alkyl group having 1 to 50 carbon atoms.

In an exemplary embodiment, $R_{631}$ to $R_{651}$ not contributing to ring formation are each independently a hydrogen atom, or a substituted or unsubstituted alkyl group having 1 to 50 carbon atoms; and

at least one of $R_{631}$ to $R_{651}$ is a substituted or unsubstituted alkyl group having 1 to 50 carbon atoms.

In an exemplary embodiment, the compound represented by the formula (63) is a compound represented by a formula (63A) below.

[Formula 136]

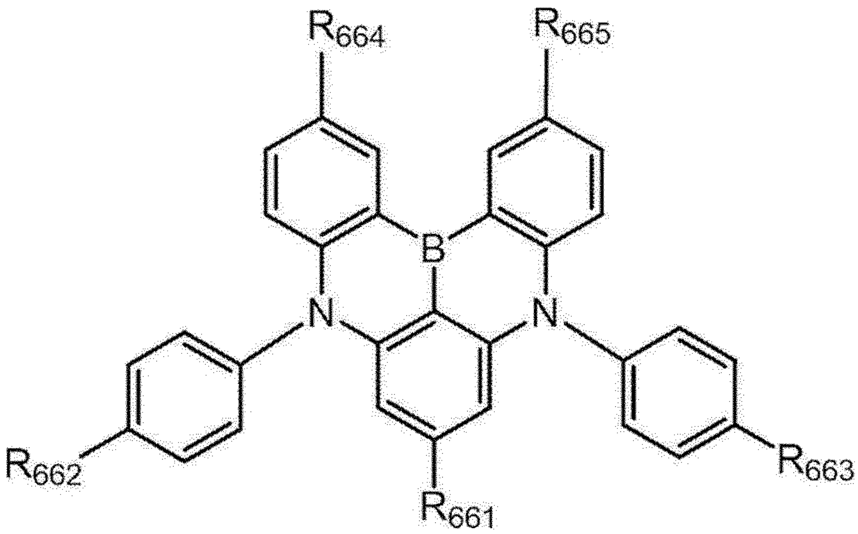

(63A)

In the formula (63A):

$R_{661}$ is a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, or a substituted or unsubstituted aryl group having 6 to 50 ring carbon atoms; and

$R_{662}$ to $R_{665}$ are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, or a substituted or unsubstituted aryl group having 6 to 50 ring carbon atoms.

In an exemplary embodiment, $R_{661}$ to $R_{665}$ are each independently a substituted or unsubstituted alkyl group having 1 to 50 carbon atoms, or a substituted or unsubstituted aryl group having 6 to 50 ring carbon atoms.
In an exemplary embodiment, $R_{661}$ to $R_{665}$ are each independently a substituted or unsubstituted alkyl group having 1 to 50 carbon atoms.
In an exemplary embodiment, the compound represented by the formula (63) is a compound represented by a formula (63B) below.

[Formula 137]

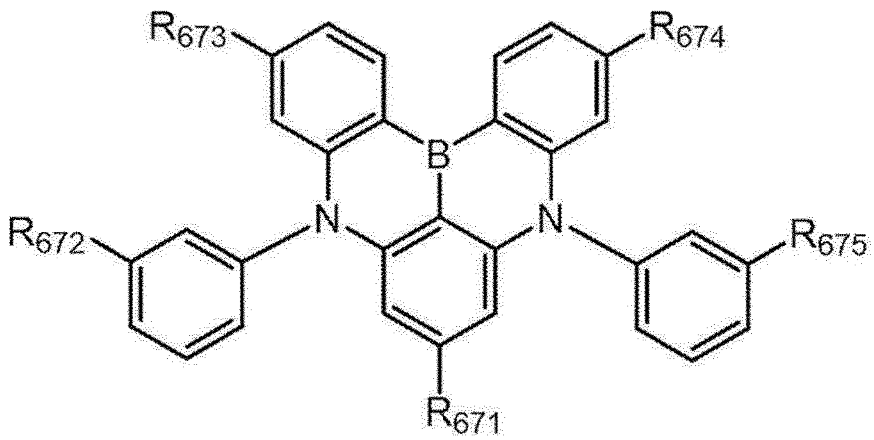

(63B)

In the formula (63B):

$R_{671}$ and $R_{672}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-N(R_{906})(R_{907})$, or a substituted or unsubstituted aryl group having 6 to 50 ring carbon atoms; and
$R_{673}$ to $R_{675}$ are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-N(R_{906})(R_{907})$, or a substituted or unsubstituted aryl group having 6 to 50 ring carbon atoms.

In an exemplary embodiment, the compound represented by the formula (63) is a compound represented by a formula (63B') below.

[Formula 138]

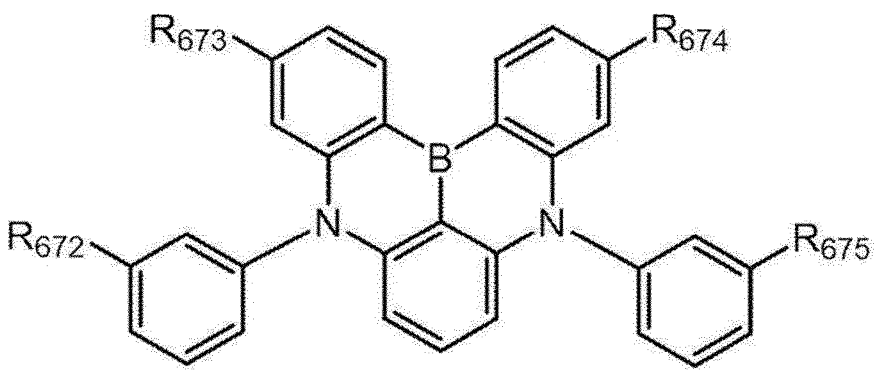

(63B')

In the formula (63B'), $R_{672}$ to $R_{675}$ each independently represent the same as $R_{672}$ to $R_{675}$ in the formula (63B).
In an exemplary embodiment, at least one of $R_{671}$ to $R_{675}$ is a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50

ring carbon atoms, a group represented by -N($R_{906}$)($R_{907}$), or a substituted or unsubstituted aryl group having 6 to 50 ring carbon atoms.

In an exemplary embodiment: $R_{672}$ is a hydrogen atom, a substituted or unsubstituted alkyl group having 1 to 50 carbon atoms, a group represented by - N($R_{906}$)($R_{907}$), or a substituted or unsubstituted aryl group having 6 to 50 ring carbon atoms; and

$R_{671}$ and $R_{673}$ to $R_{675}$ are each independently a substituted or unsubstituted alkyl group having 1 to 50 carbon atoms, a group represented by -N($R_{906}$)($R_{907}$), or a substituted or unsubstituted aryl group having 6 to 50 ring carbon atoms.

In an exemplary embodiment, the compound represented by the formula (63) is a compound represented by a formula (63C) below.

[Formula 139]

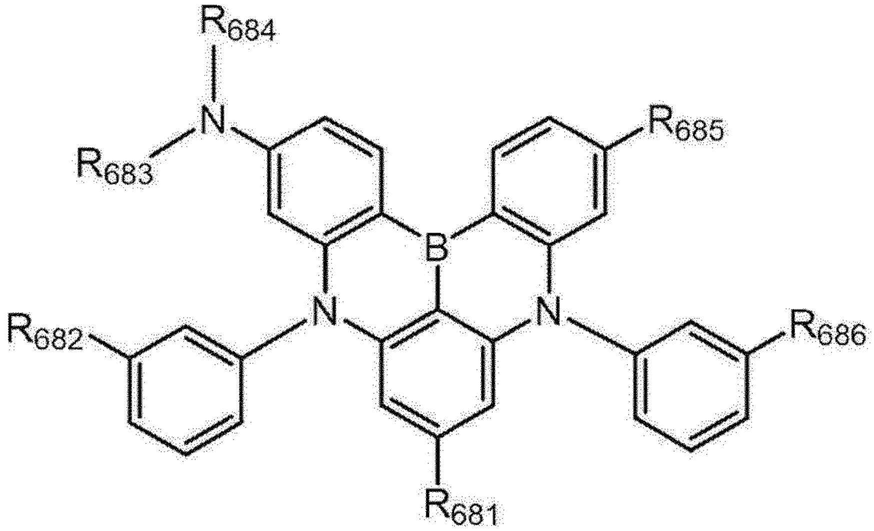

(63C)

In the formula (63C):

$R_{681}$ and $R_{682}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, or a substituted or unsubstituted aryl group having 6 to 50 ring carbon atoms; and

$R_{683}$ to $R_{686}$ are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, or a substituted or unsubstituted aryl group having 6 to 50 ring carbon atoms.

In an exemplary embodiment, the compound represented by the formula (63) is a compound represented by a formula (63C') below.

[Formula 140]

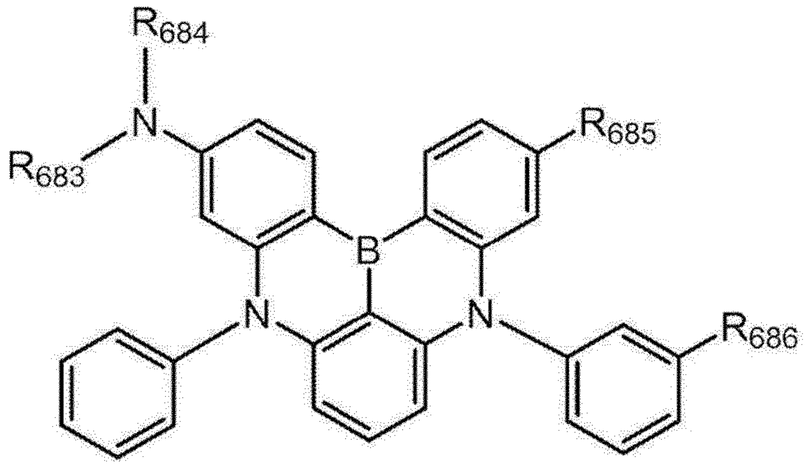

(63C')

In the formula (63C'), $R_{683}$ to $R_{686}$ each independently represent the same as $R_{683}$ to $R_{686}$ in the formula (63C).

In an exemplary embodiment, $R_{681}$ to $R_{686}$ are each independently a substituted or unsubstituted alkyl group having 1 to 50 carbon atoms, or a substituted or unsubstituted aryl group having 6 to 50 ring carbon atoms.

In an exemplary embodiment, $R_{681}$ to $R_{686}$ are each independently a substituted or unsubstituted aryl group having 6 to 50 carbon atoms.

The compound represented by the formula (6) is producible by initially bonding the ring a, ring b and ring c with linking groups (a group including N-$R_{601}$ and a group including N-$R_{602}$) to form an intermediate (first reaction), and bonding the ring a, ring b and ring c with a linking group (a group including a boron atom) to form a final product (second reaction). In the first reaction, an amination reaction (e.g. Buchwald-Hartwig reaction) is applicable. In the second reaction, Tandem Hetero-Friedel-Crafts Reactions or the like is applicable.

Specific examples of the compound represented by the formula (6) are given below. It should however be noted that these specific examples are merely exemplary and do not limit the compound represented by the formula (6).

[Formula 141]

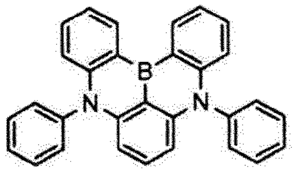
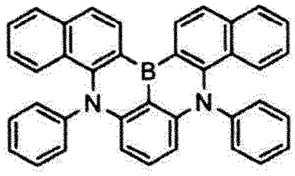
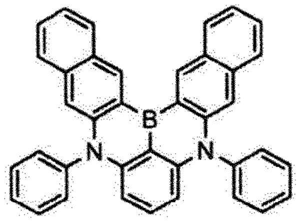
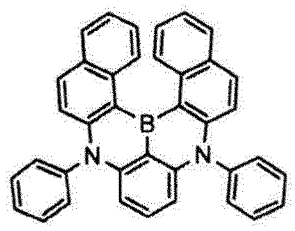
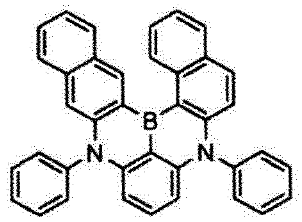
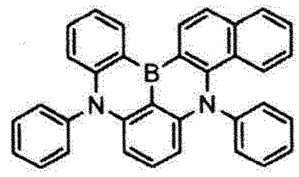
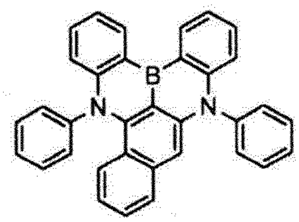
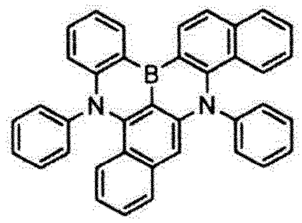
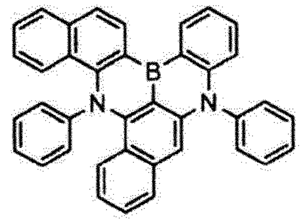
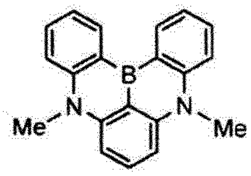
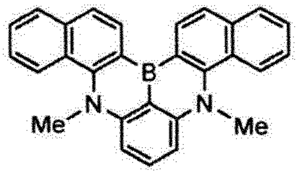
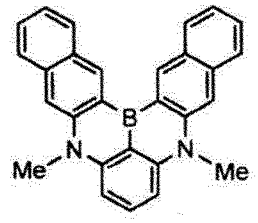
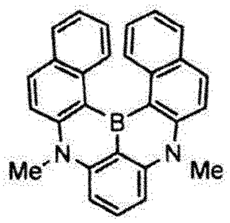
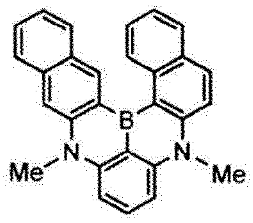
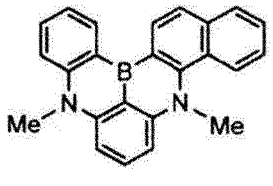
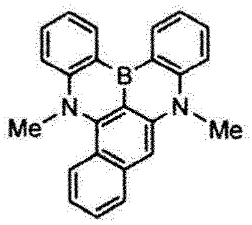
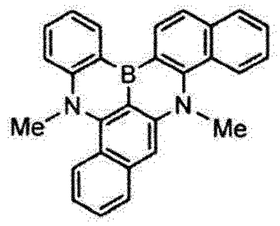
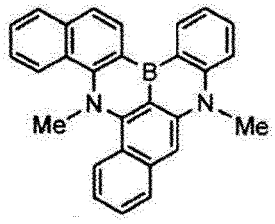

[Formula 142]

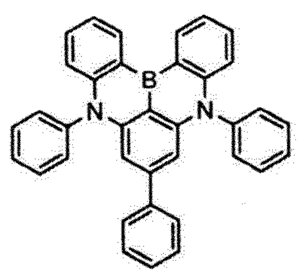

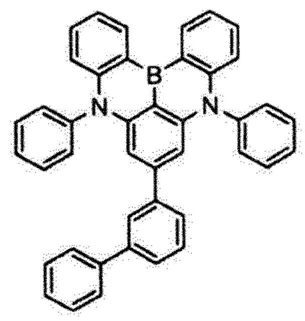

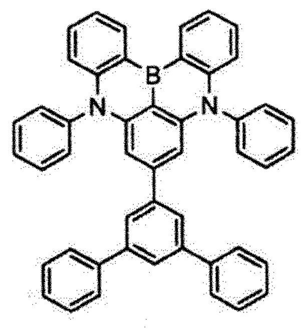

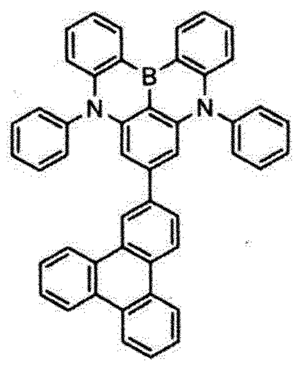

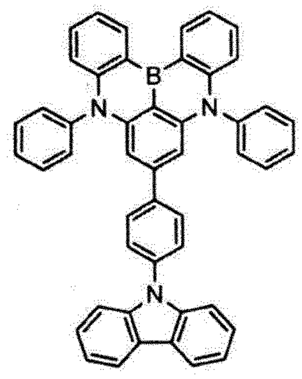

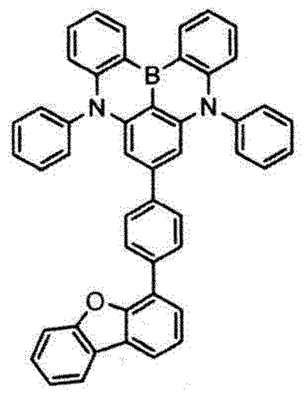

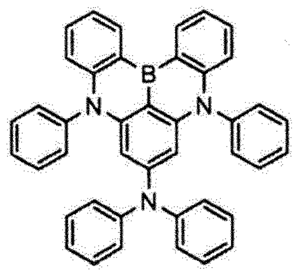

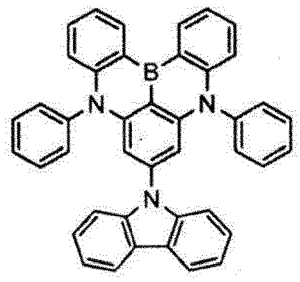

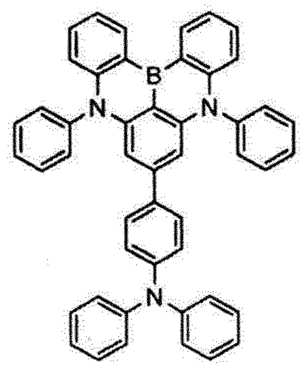

[Formula 143]

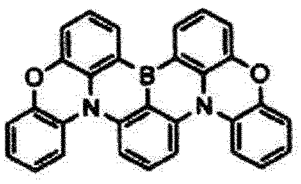
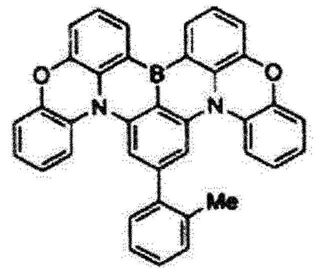
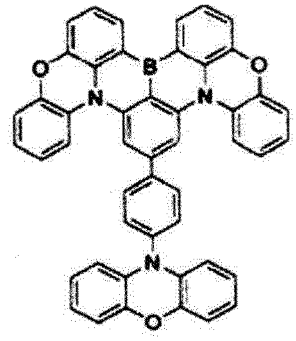
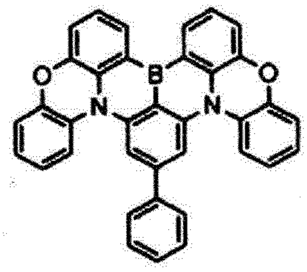
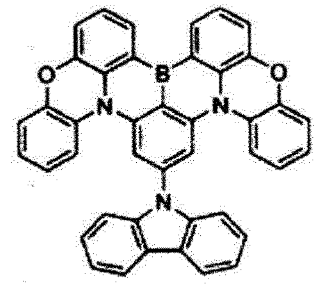
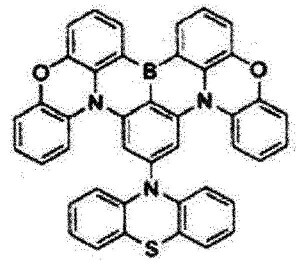
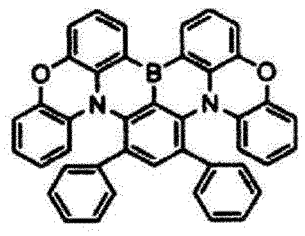
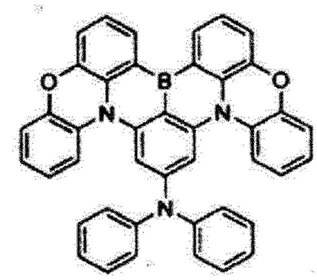
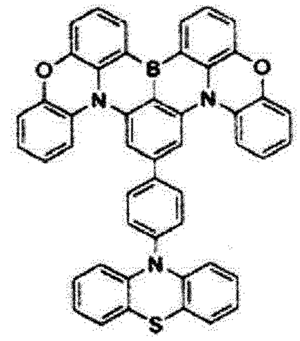
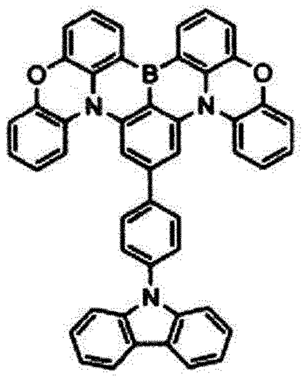
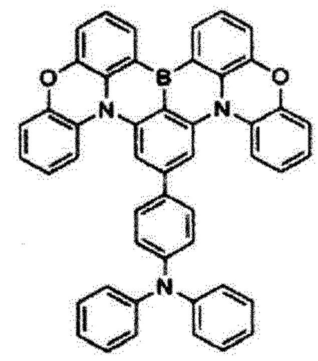
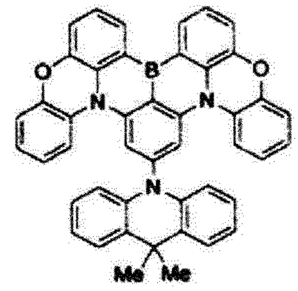
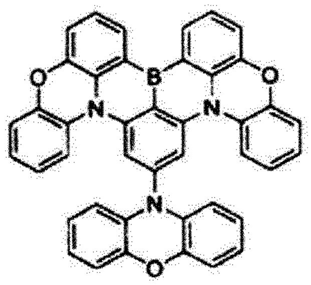
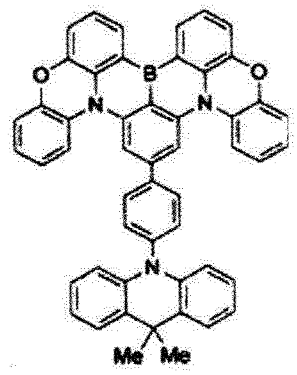

[Formula 144]

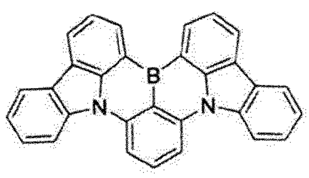
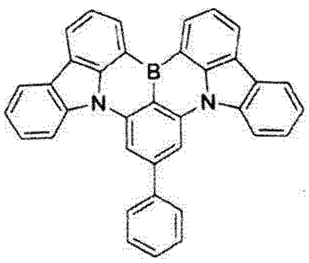
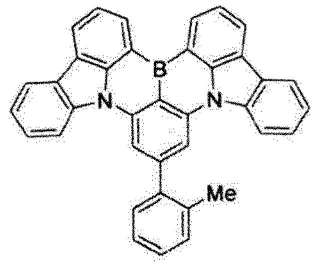
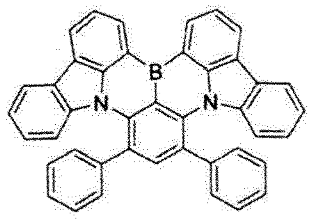
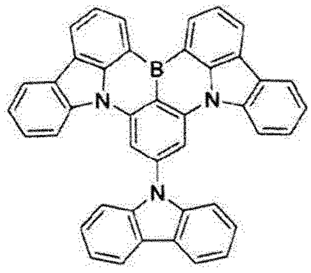
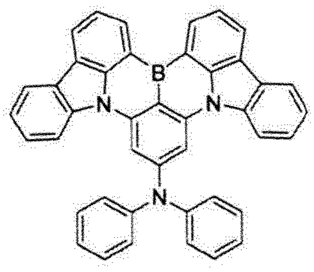
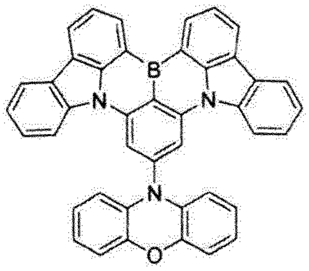
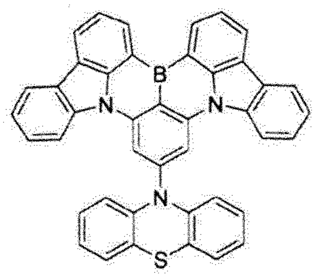
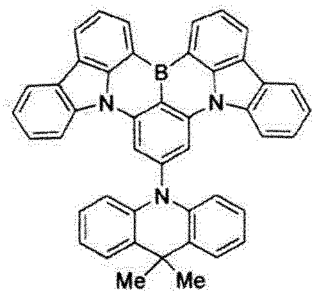
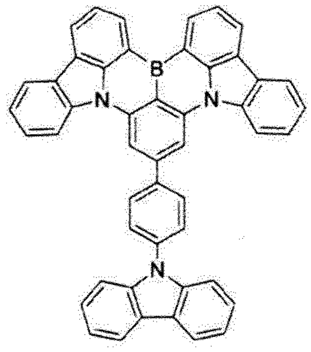
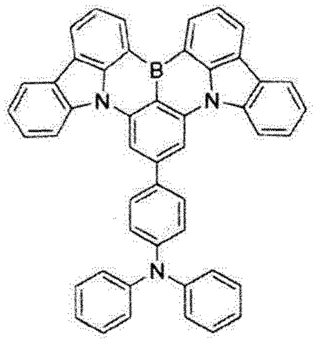
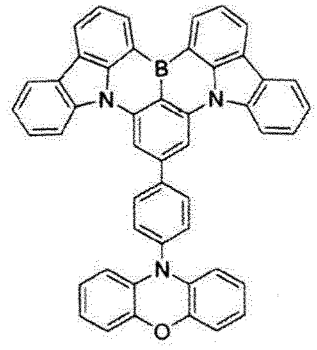
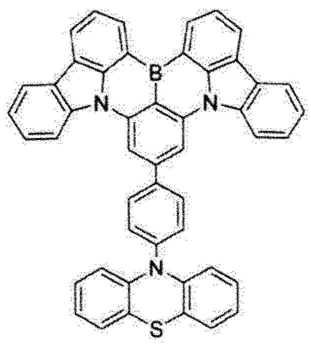
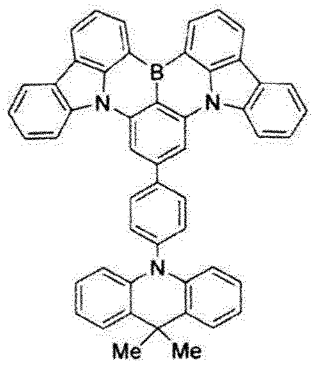

[Formula 145]

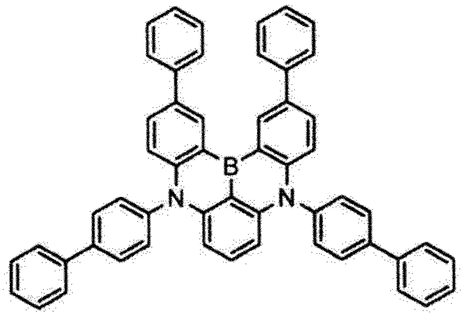

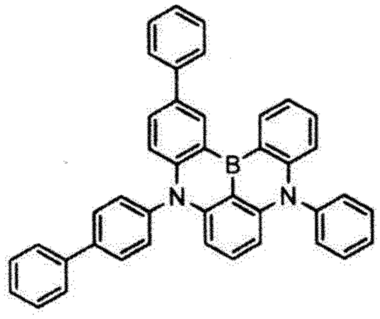

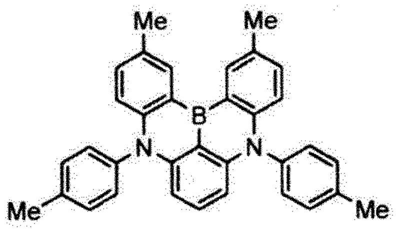

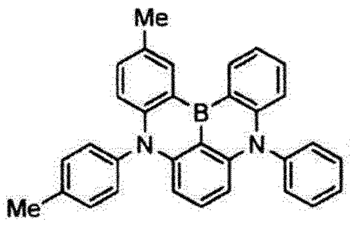

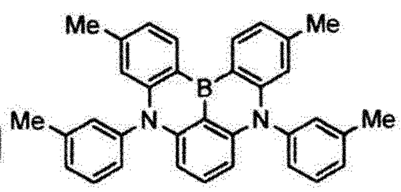

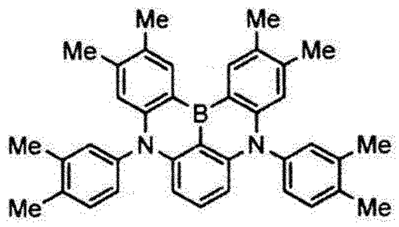

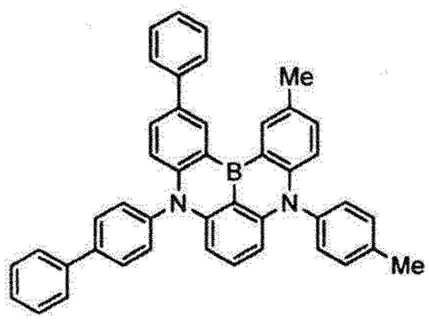

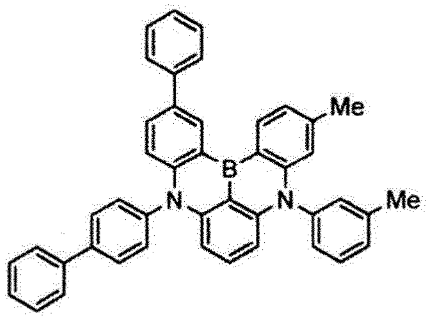

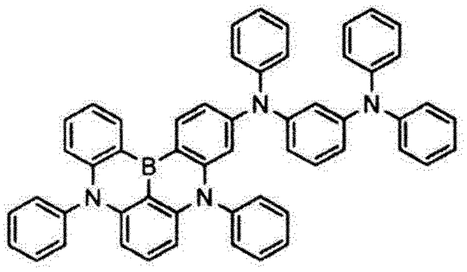

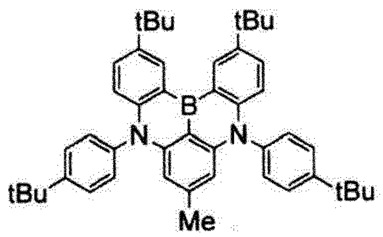

[Formula 146]

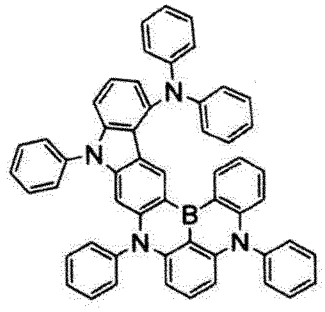
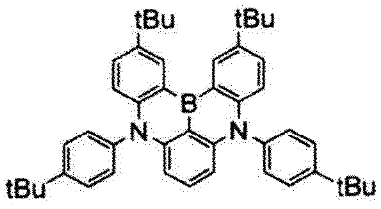
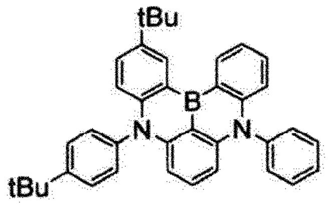
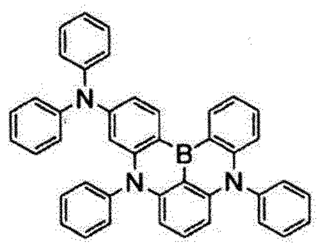
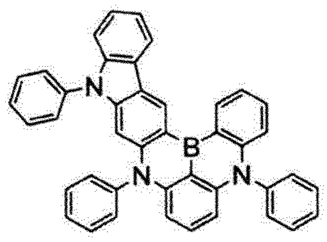
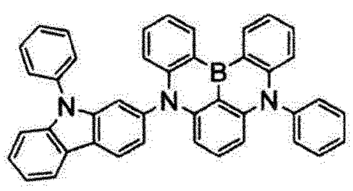
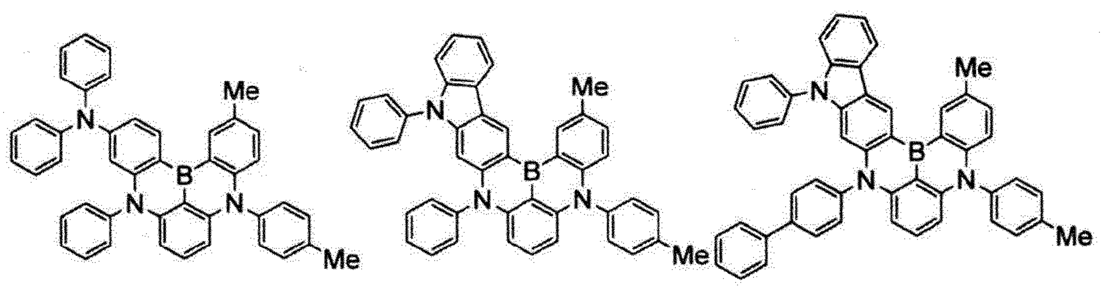
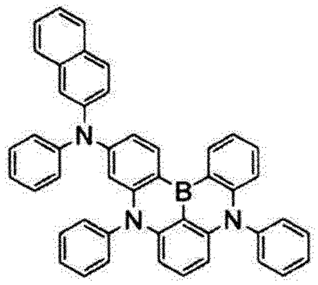
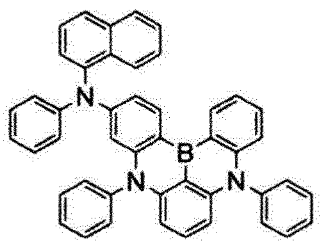
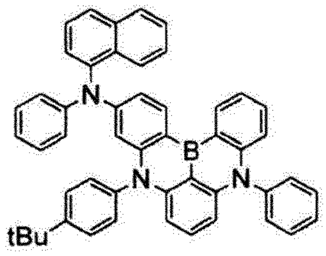
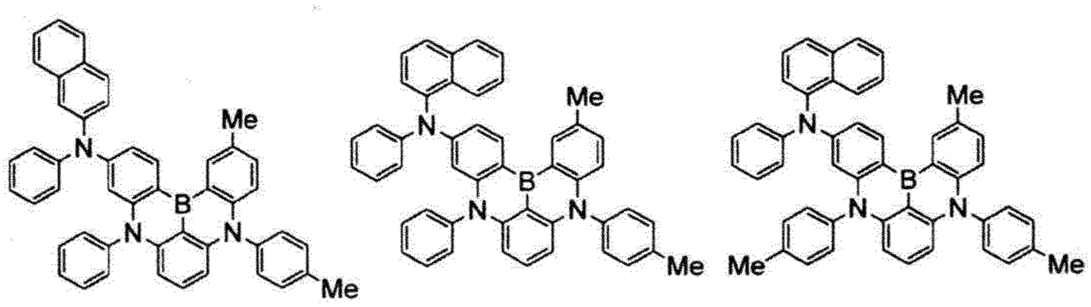

[Formula 147]

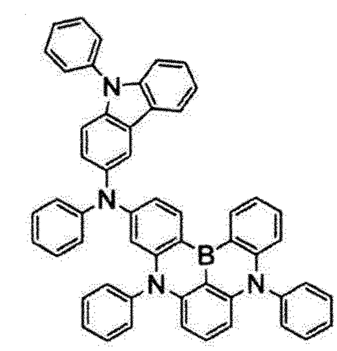
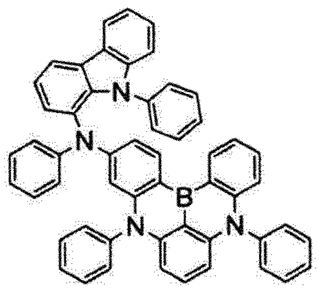
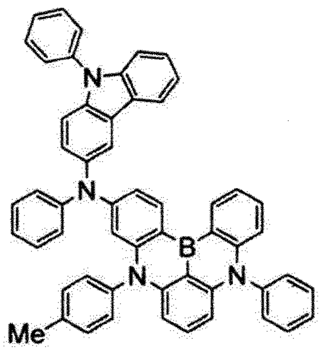

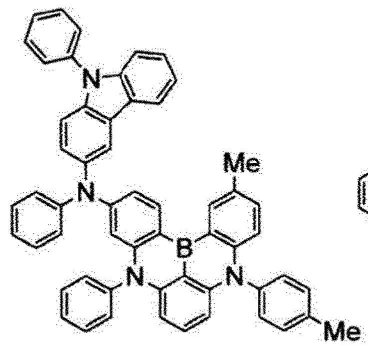
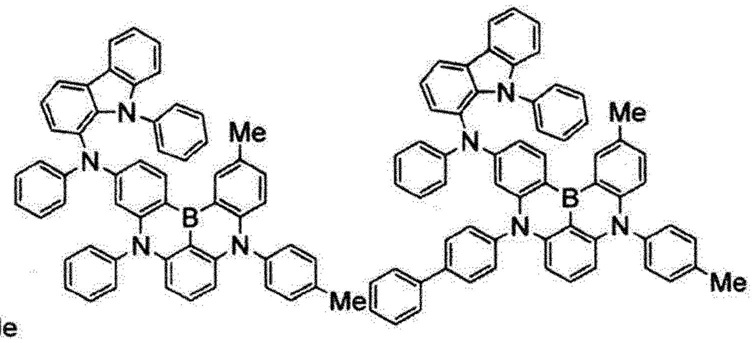

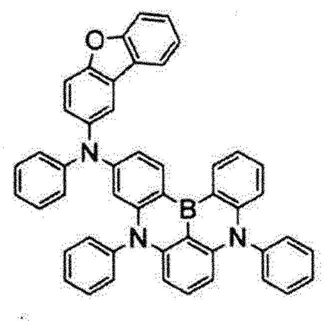
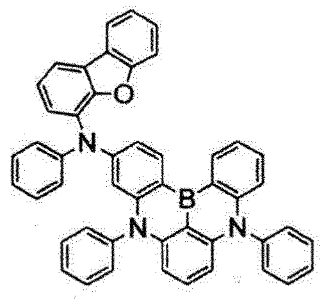
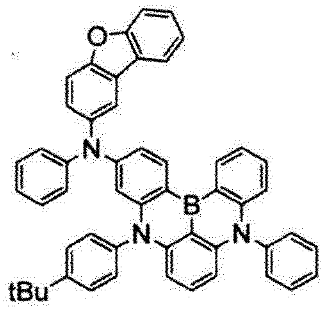

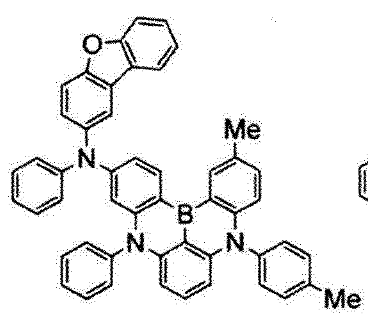
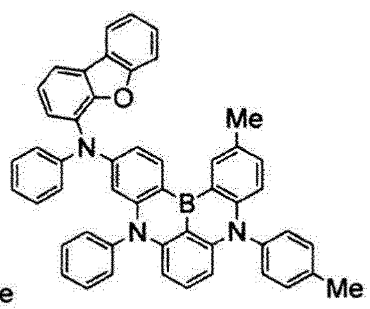
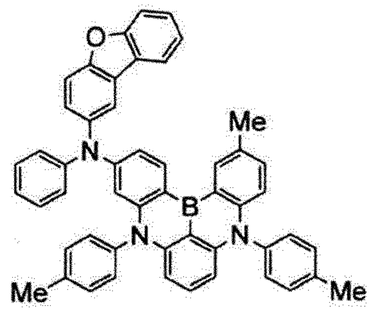

[Formula 148]

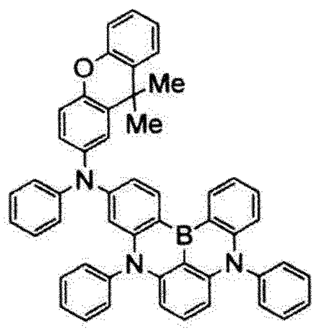

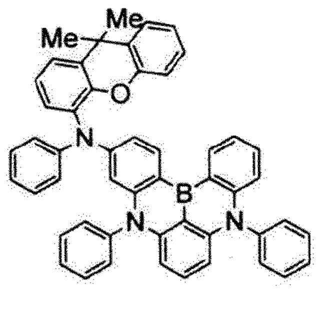

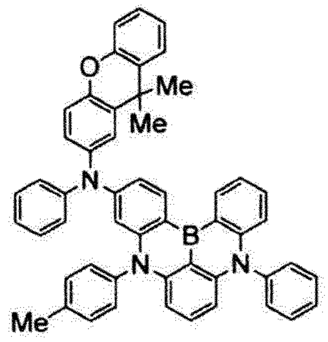

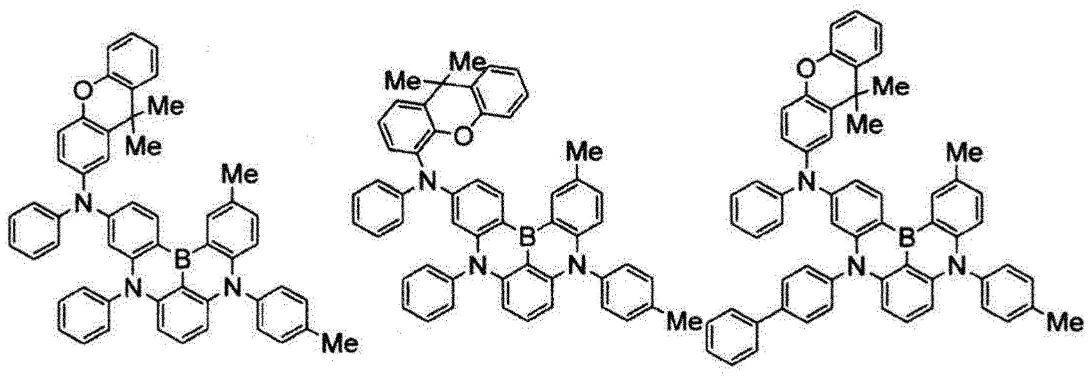

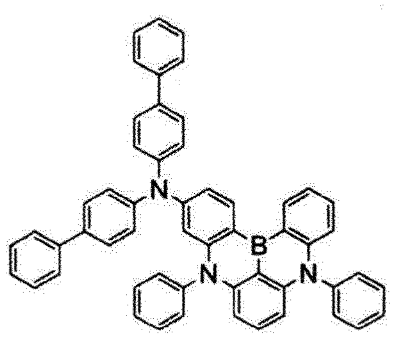

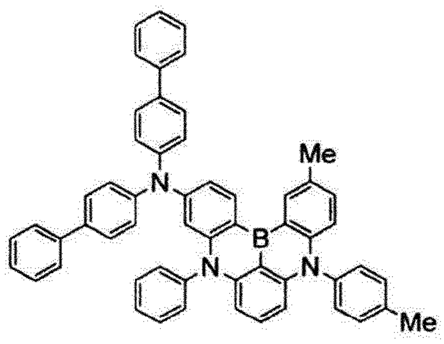

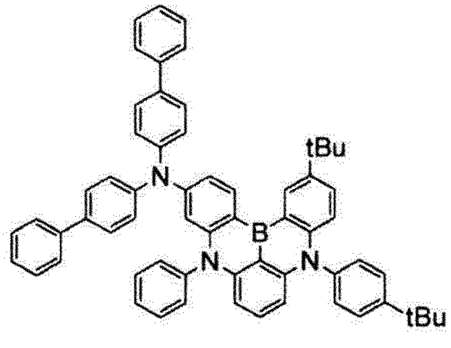

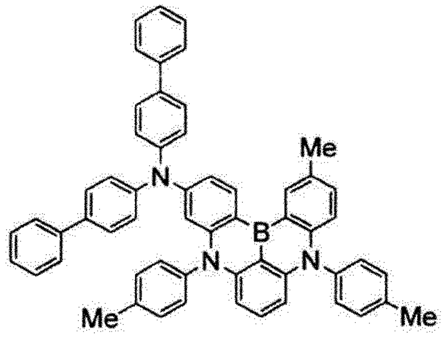

[Formula 149]

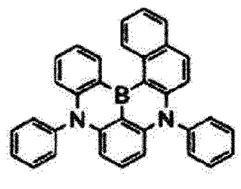
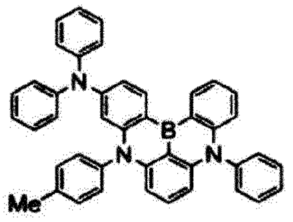
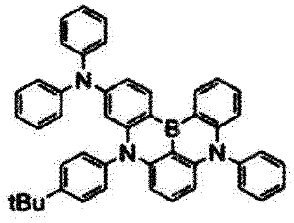
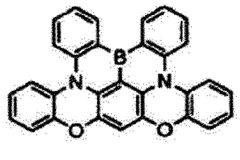

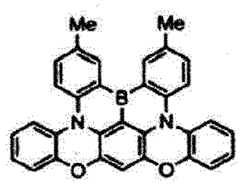

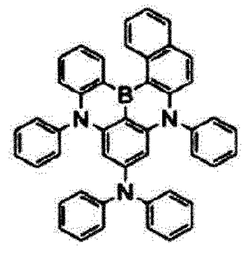
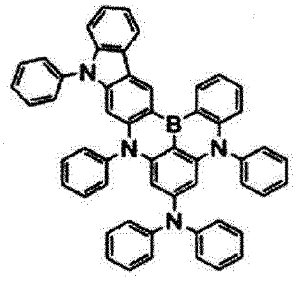
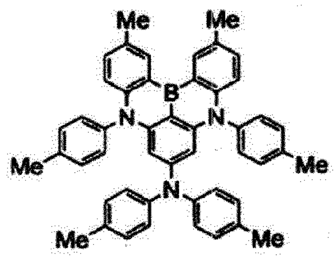

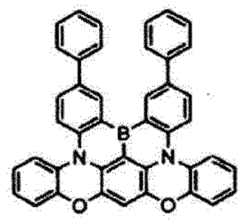

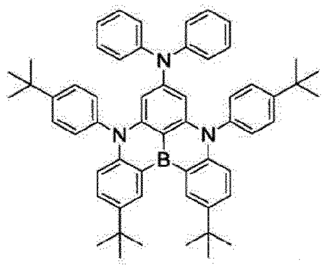

[Formula 150]

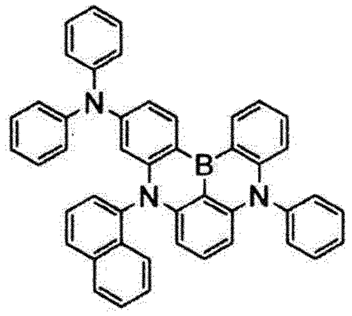

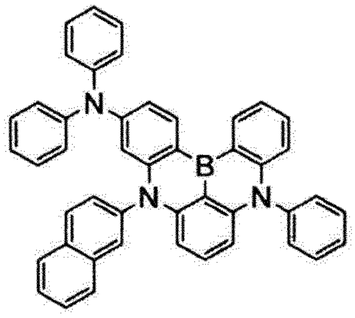

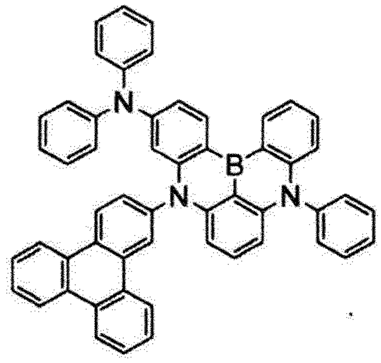

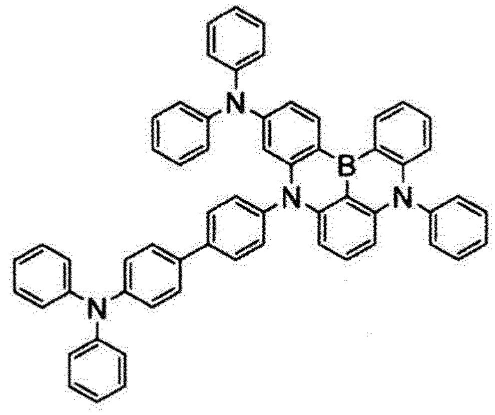

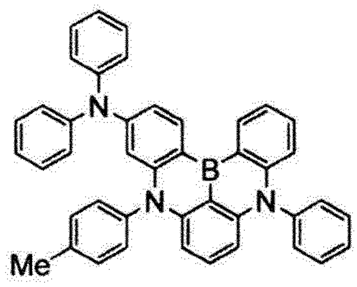

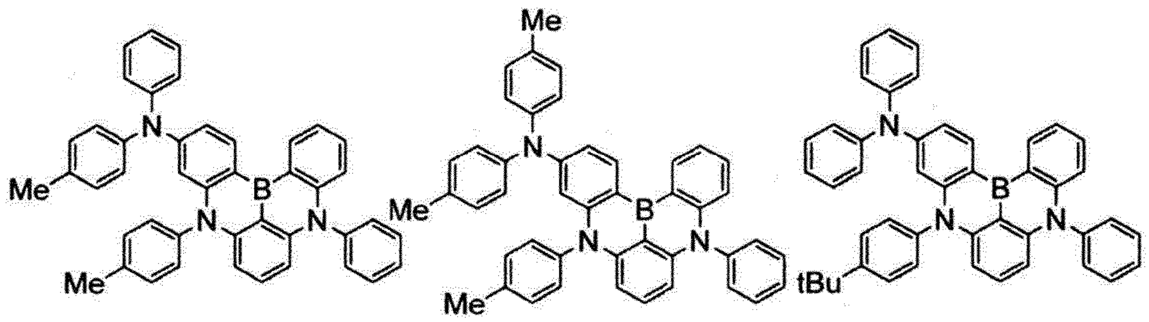

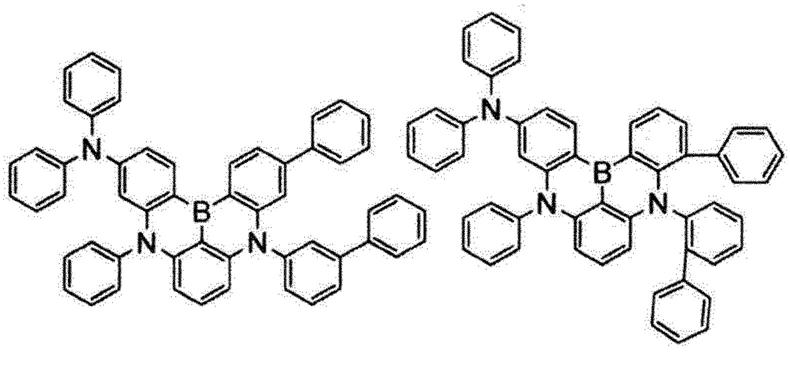

[Formula 151]

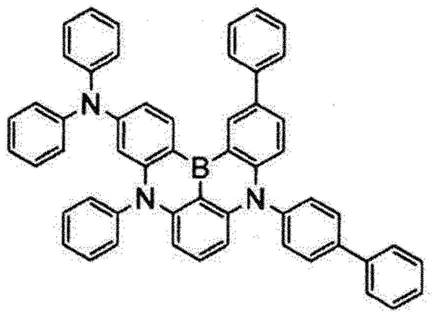

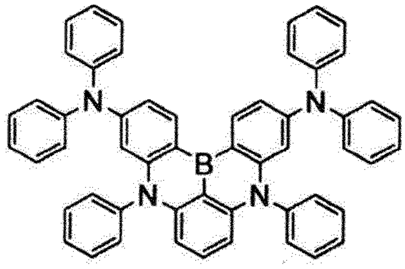

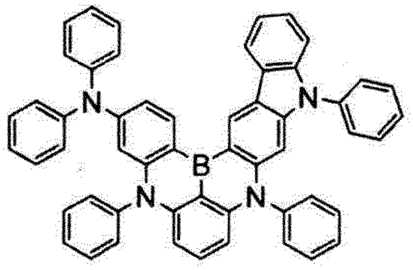

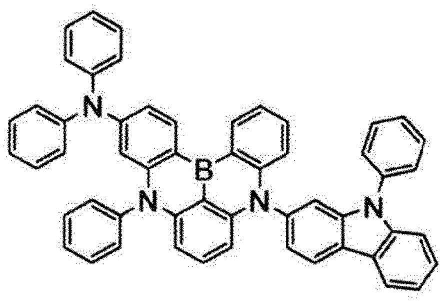

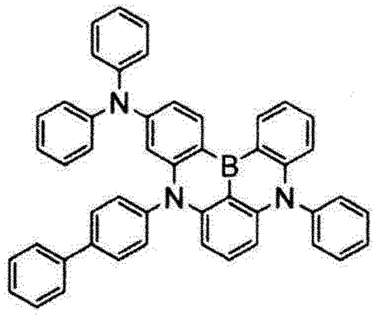

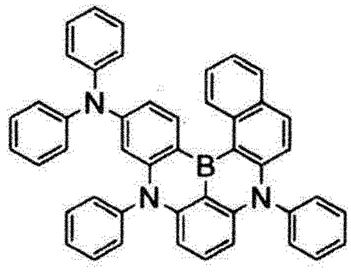

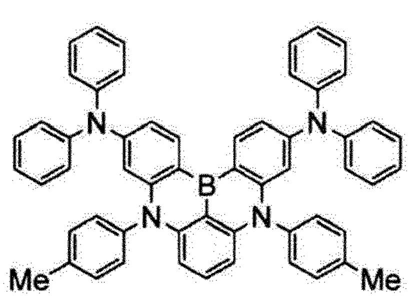

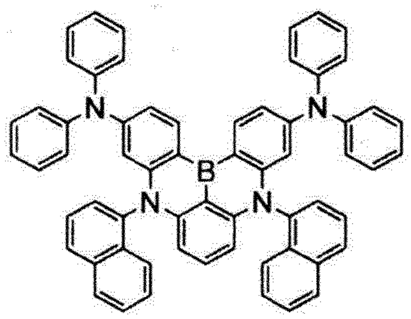

[Formula 152]

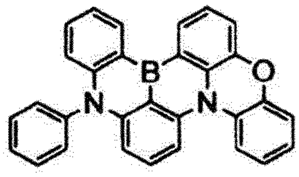

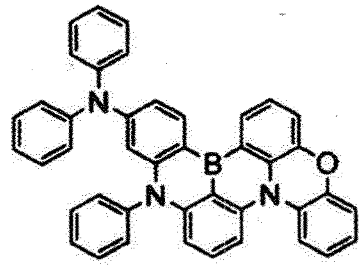

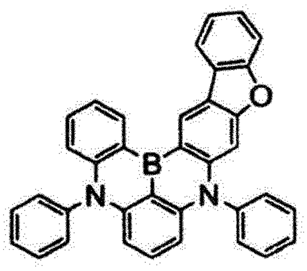

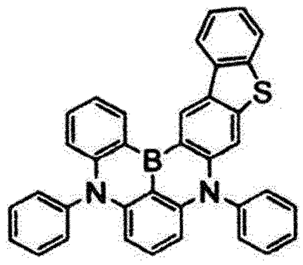

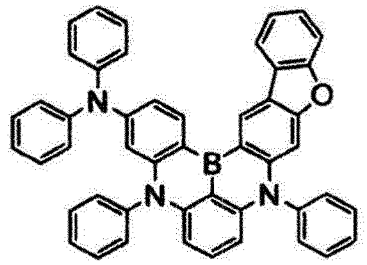

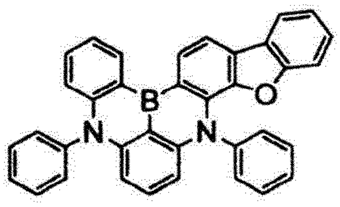

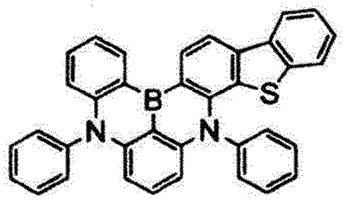

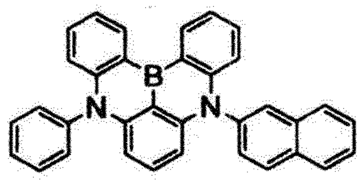

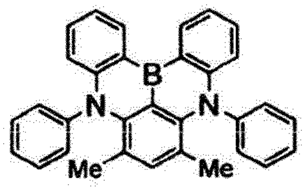

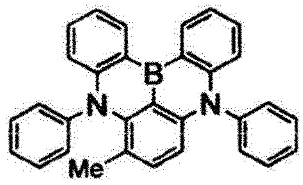

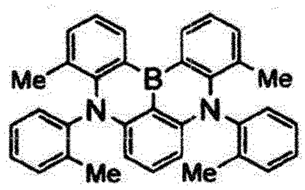

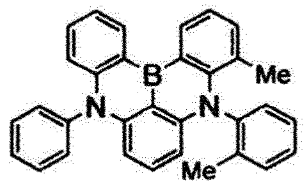

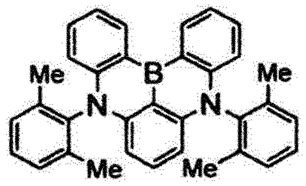

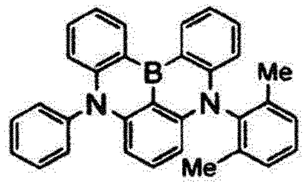

[Formula 153]

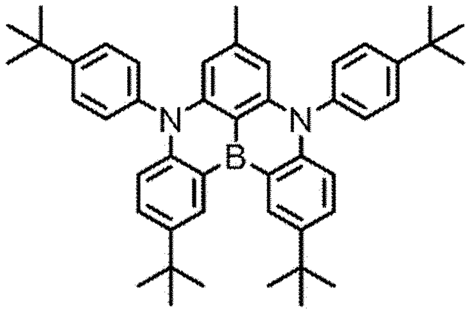

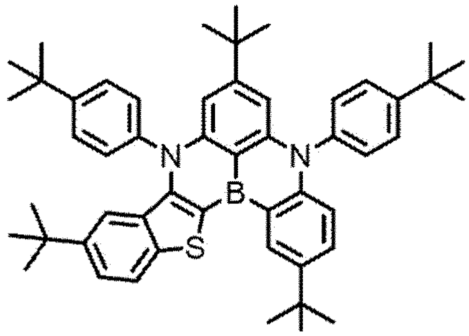

[Formula 154]

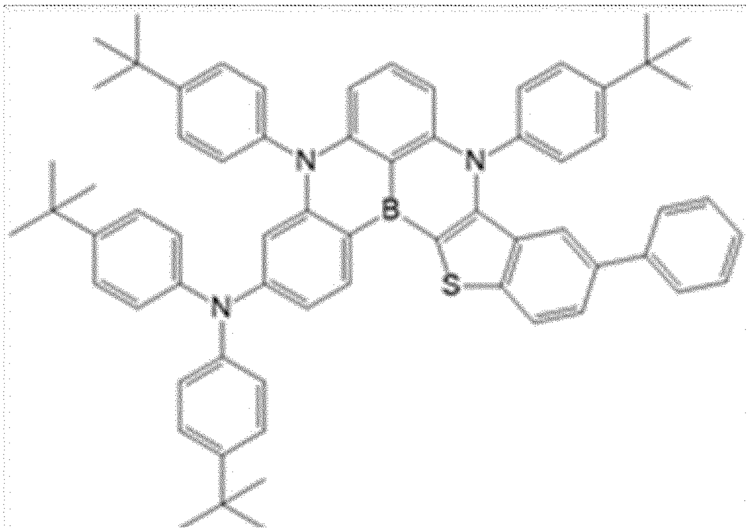

[Formula 155]

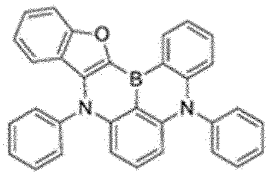
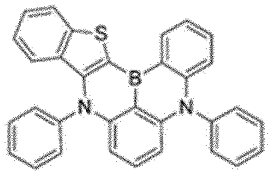
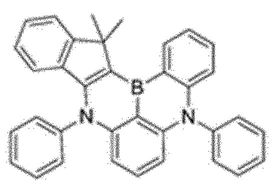
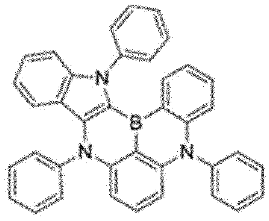

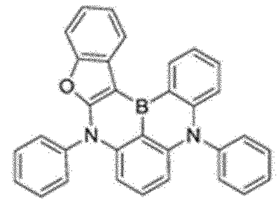
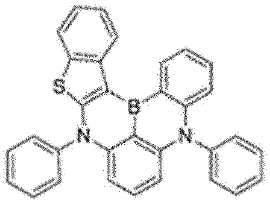
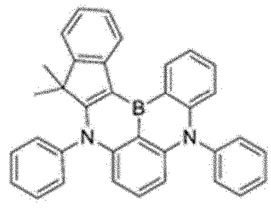
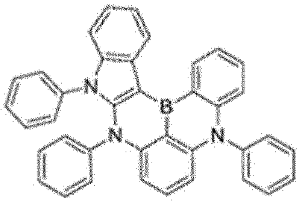

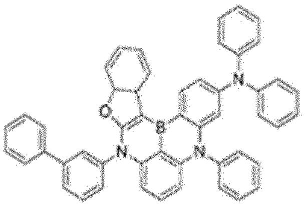
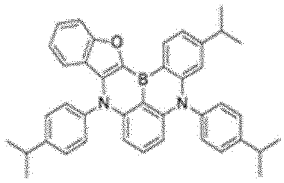
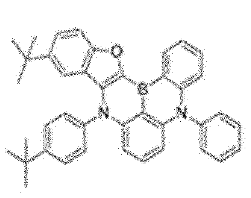
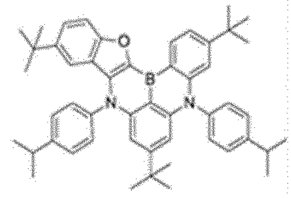

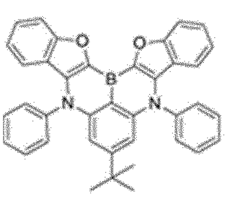
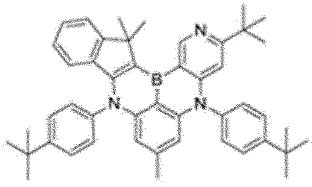
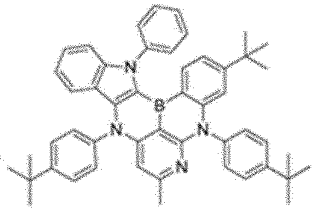
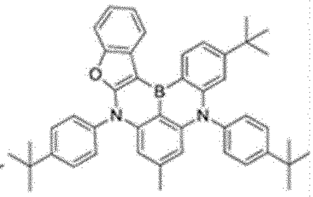

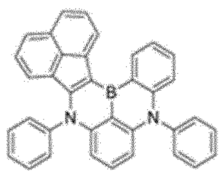
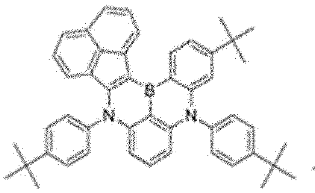
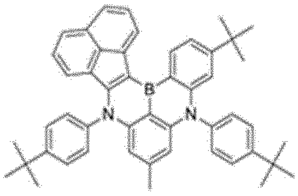
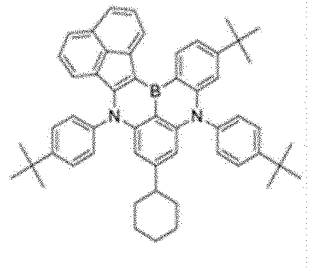

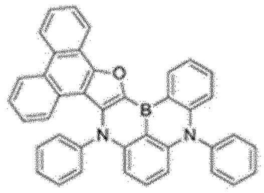
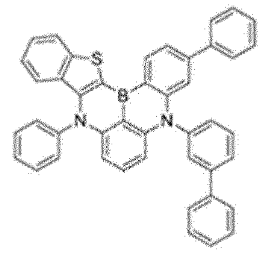
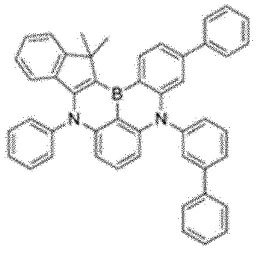
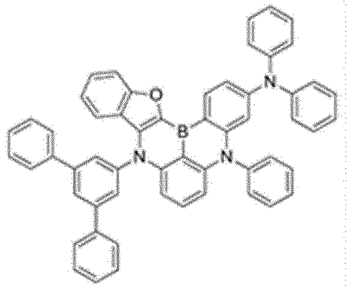

[Formula 156]

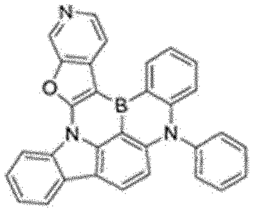
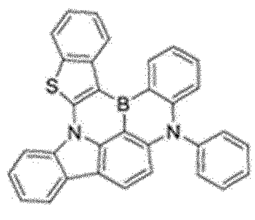
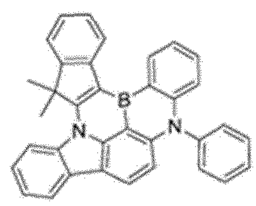
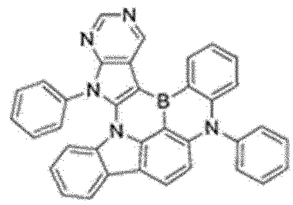

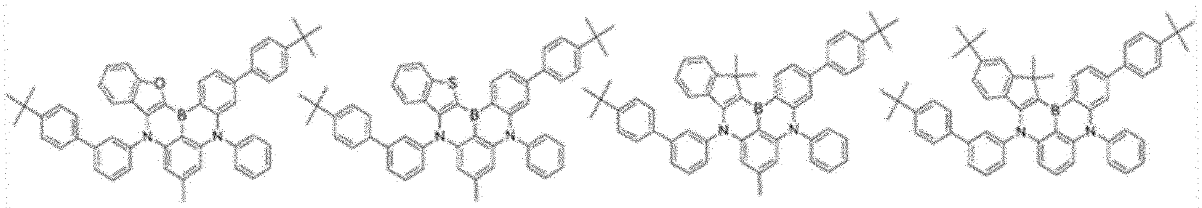

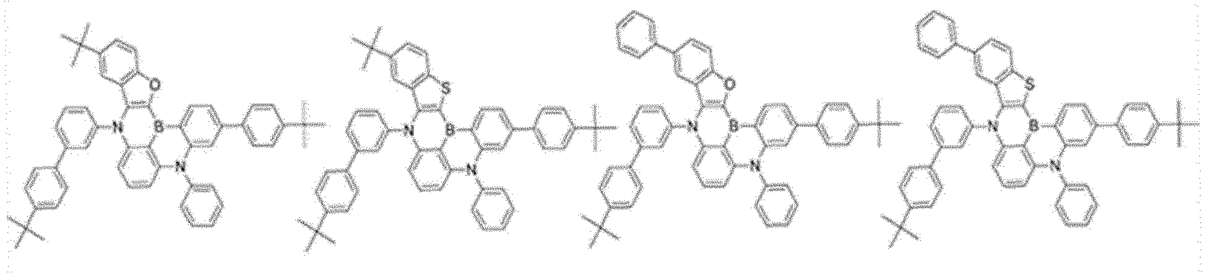

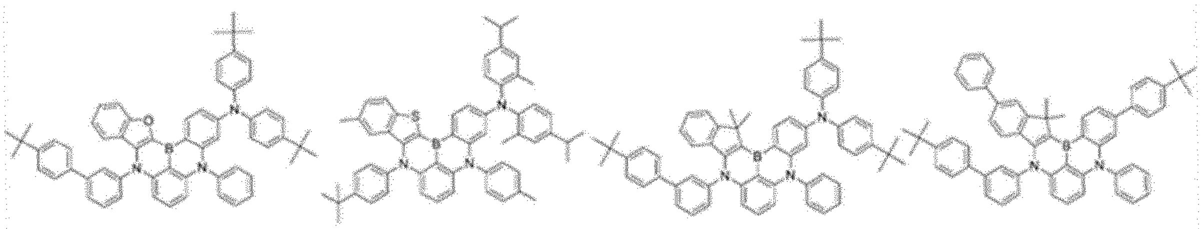

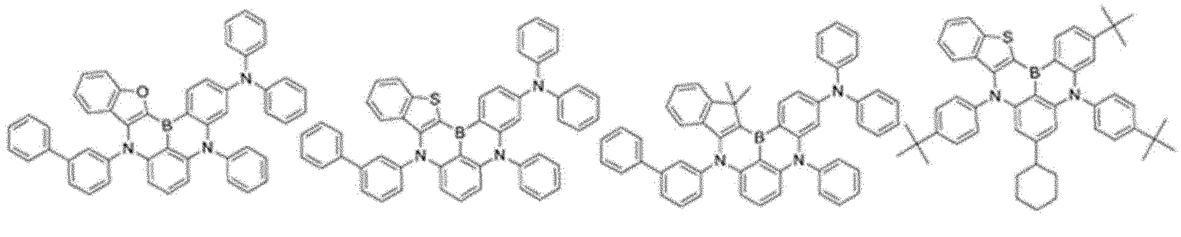

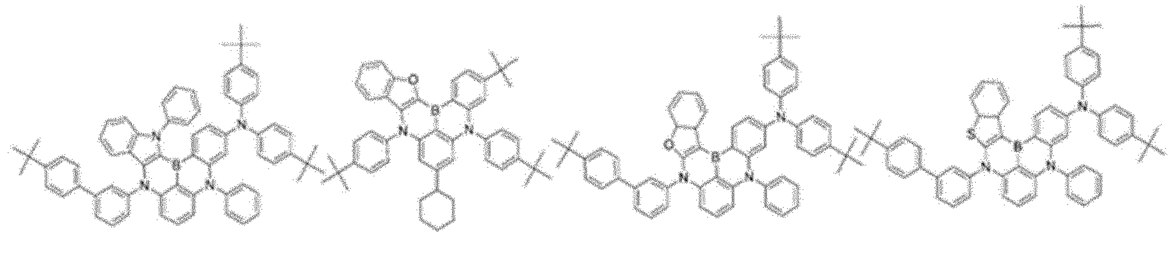

[Formula 157]

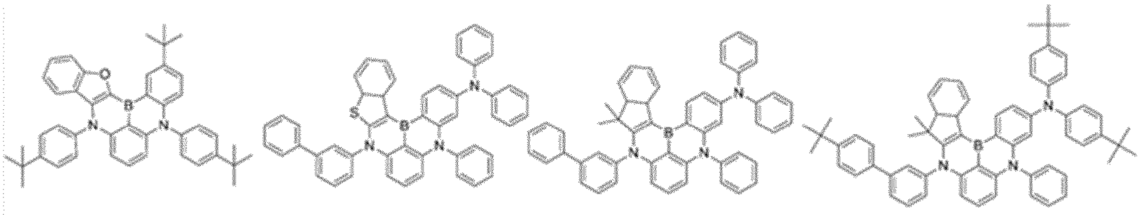

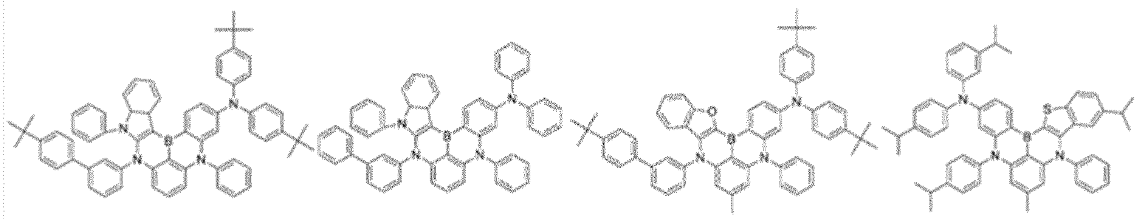

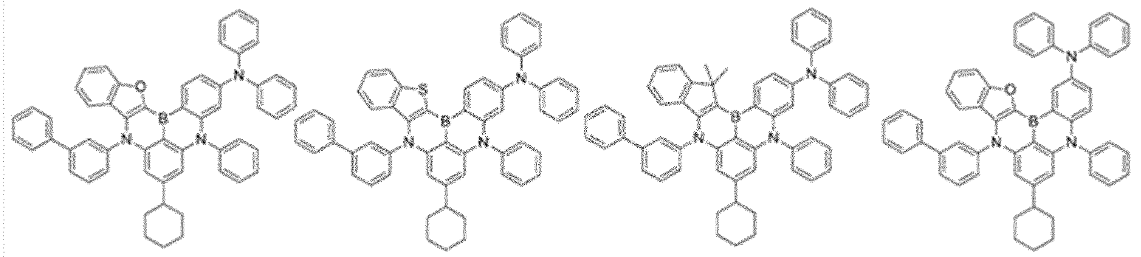

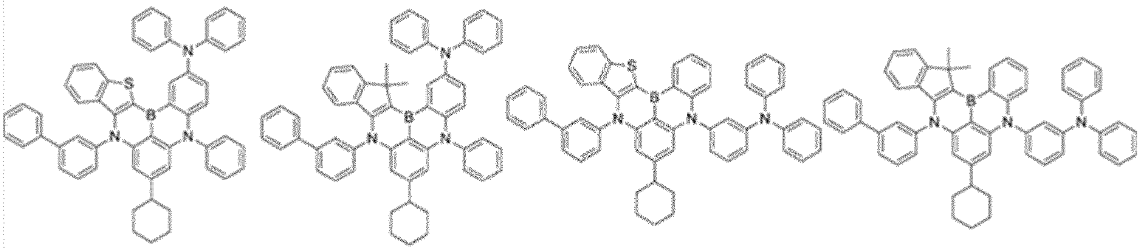

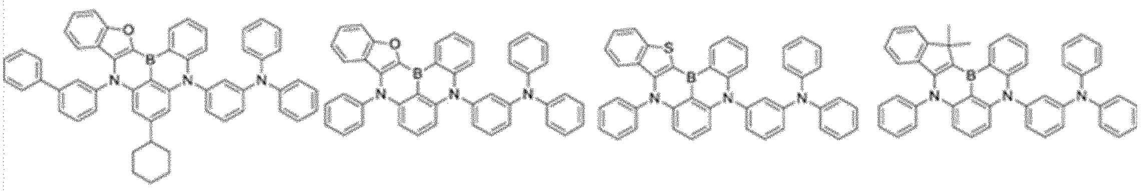

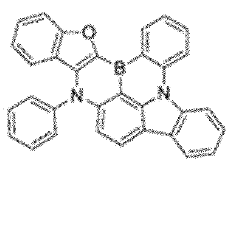

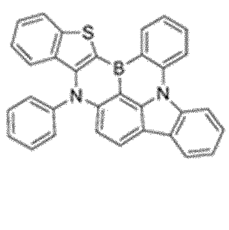

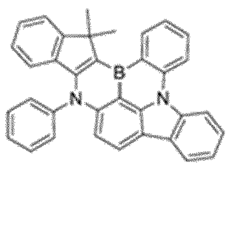

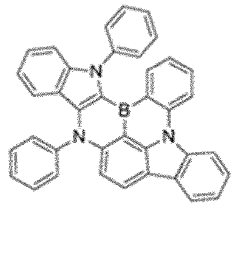

[Formula 158]

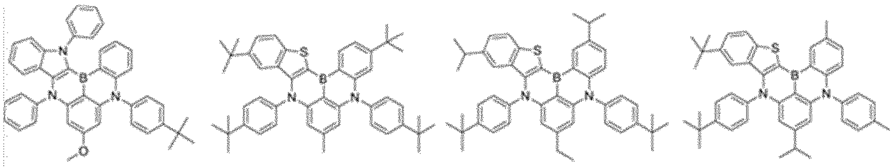

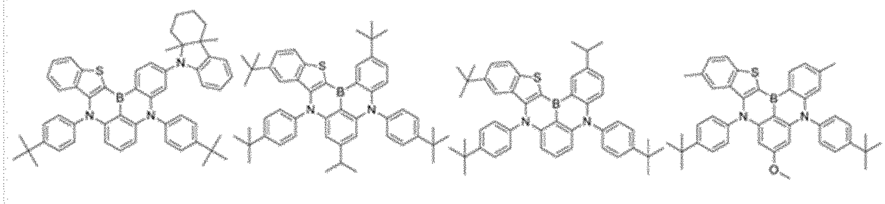

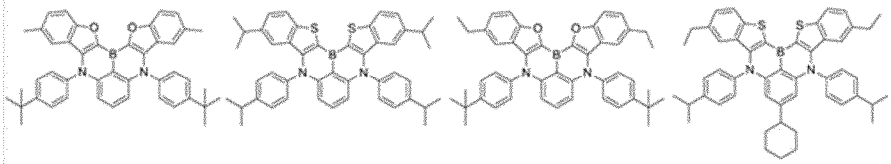

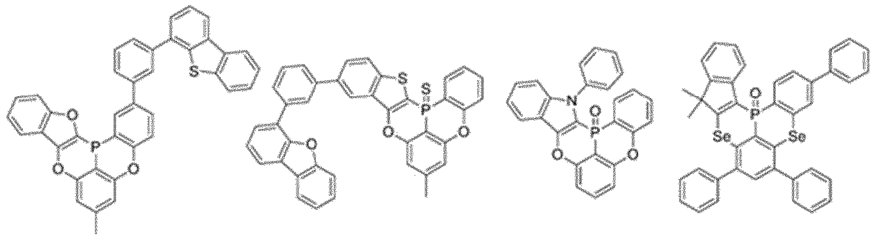

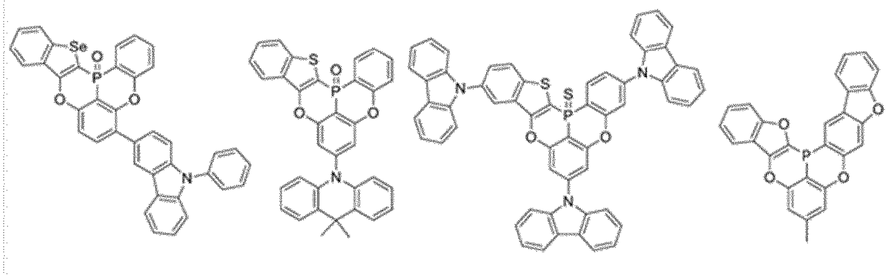

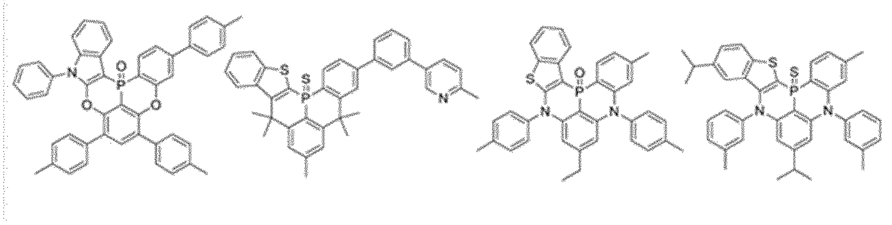

Compound Represented by Formula (7)

The compound represented by the formula (7) will be described below.

[Formula 159]

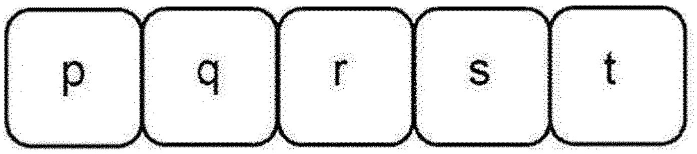

(7)

[Formula 160]

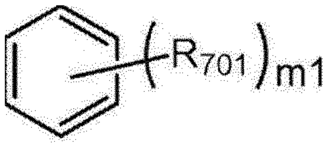

(72)

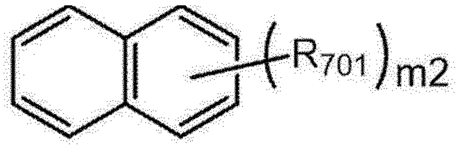

(73)

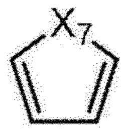

(74)

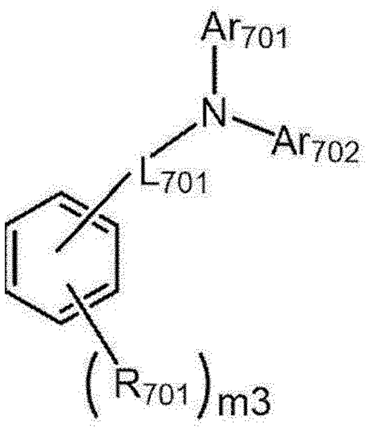

(75)

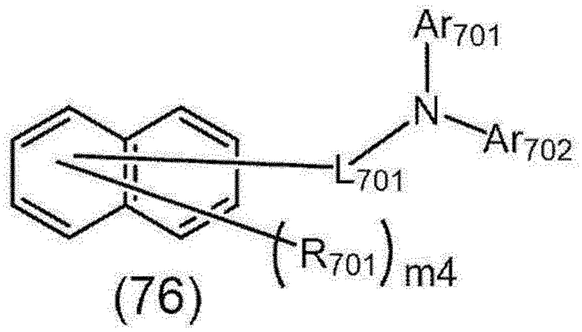

(76)

In the formula (7):

a ring r is a ring represented by the formula (72) or the formula (73), the ring r being fused with adjacent ring(s) at any position(s);

a ring q and a ring s are each independently a ring represented by the formula (74) and fused with adjacent ring(s) at any position(s);

a ring p and a ring t are each independently a structure represented by the formula (75) or the formula (76) and fused with adjacent ring(s) at any position(s);

$X_7$ is an oxygen atom, a sulfur atom, or $NR_{702}$;

when a plurality of $R_{701}$ are present, adjacent ones of the plurality of $R_{701}$ are mutually bonded to form a substituted or unsubstituted monocyclic ring, mutually bonded to form a substituted or unsubstituted fused ring, or not mutually bonded;

$R_{701}$ and $R_{702}$ forming neither the monocyclic ring nor the fused ring are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

$Ar_{701}$ and $Ar_{702}$ are each independently a substituted or unsubstituted alkyl group having 1 to 50 carbon atoms, a substituted or unsubstituted alkenyl group having 2 to 50 carbon atoms, a substituted or unsubstituted alkynyl group

having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

$L_{701}$ is a substituted or unsubstituted alkylene group having 1 to 50 carbon atoms, a substituted or unsubstituted alkenylene group having 2 to 50 carbon atoms, a substituted or unsubstituted alkynylene group having 2 to 50 carbon atoms, a substituted or unsubstituted cycloalkylene group having 3 to 50 ring carbon atoms, a substituted or unsubstituted arylene group having 6 to 50 ring carbon atoms, or a substituted or unsubstituted divalent heterocyclic group having 5 to 50 ring atoms;

m1 is 0, 1, or 2;

m2 is 0, 1, 2, 3, or 4;

m3 is each independently 0, 1, 2, or 3;

m4 is each independently 0, 1, 2, 3, 4, or 5;

when a plurality of $R_{701}$ are present, the plurality of $R_{701}$ are mutually the same or different;

when a plurality of $X_7$ are present, the plurality of $X_7$ are mutually the same or different;

when a plurality of $R_{702}$ are present, the plurality of $R_{702}$ are mutually the same or different;

when a plurality of $Ar_{701}$ are present, the plurality of $Ar_{701}$ are mutually the same or different;

when a plurality of $Ar_{702}$ are present, the plurality of $Ar_{702}$ are mutually the same or different; and

when a plurality of $L_{701}$ are present, the plurality of $L_{701}$ are mutually the same or different.

In the formula (7), each of the ring p, ring q, ring r, ring s, and ring t is fused with an adjacent ring(s) sharing two carbon atoms. The fused position and orientation are not limited but may be defined as required.

In an exemplary embodiment, in the formula (72) or the formula (73) representing the ring r, m1 = 0 or m2 = 0 is satisfied.

In an exemplary embodiment, the compound represented by the formula (7) is represented by any one of formulae (71-1) to (71-6) below.

[Formula 161]

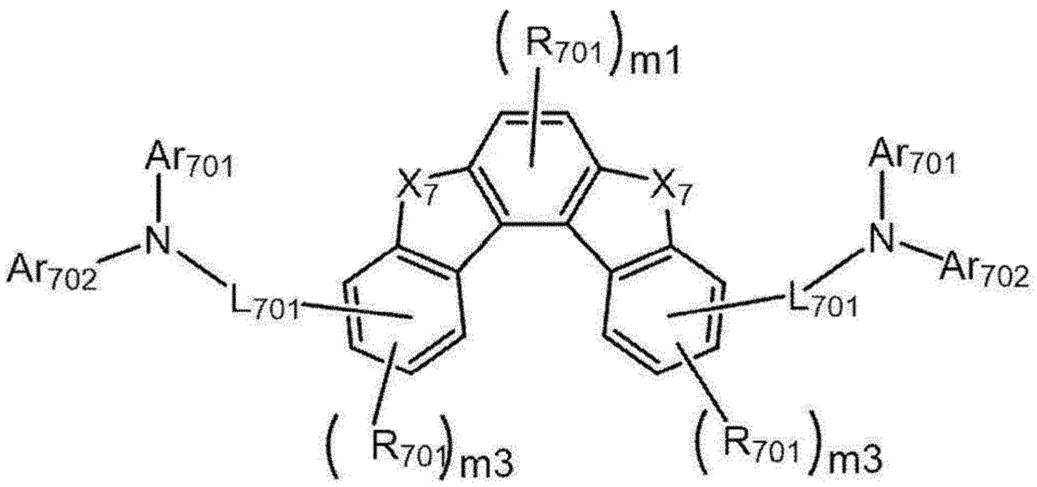

(71-1)

[Formula 162]

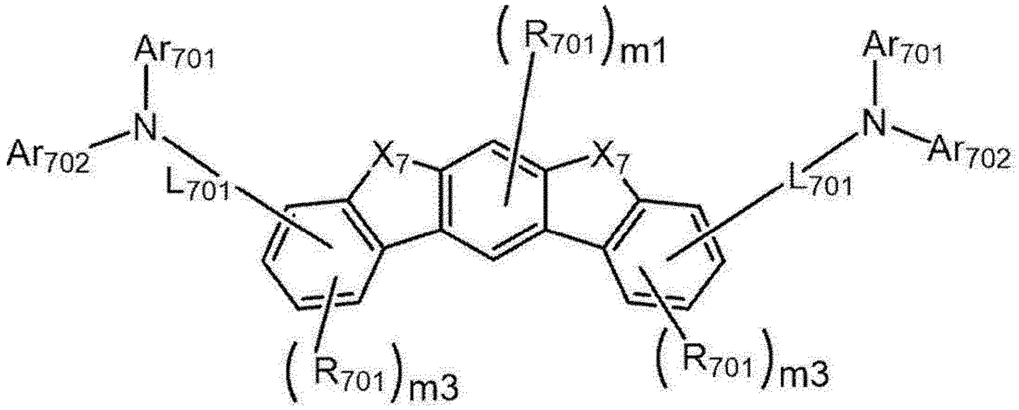

(71-2)

[Formula 163]

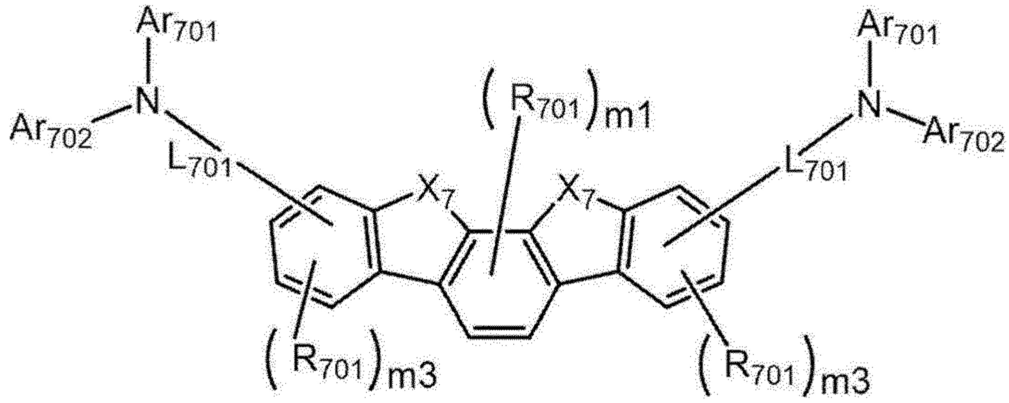

(71-3)

[Formula 164]

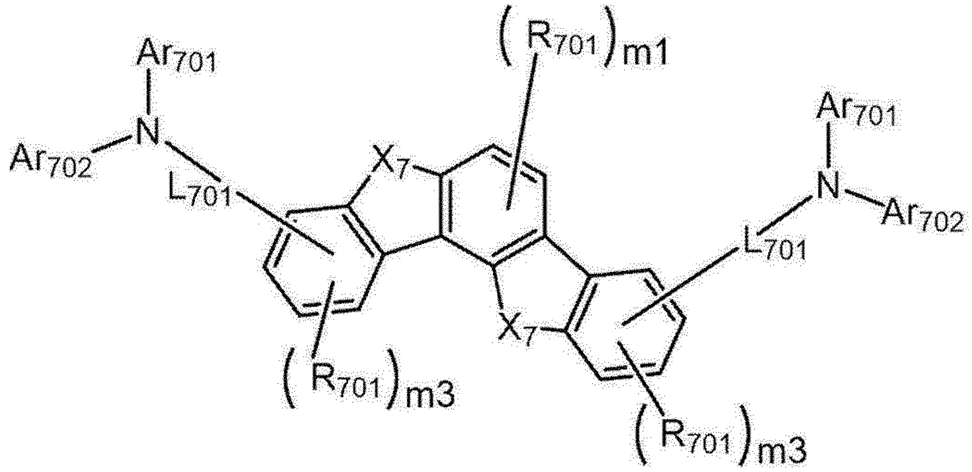

(71-4)

[Formula 165]

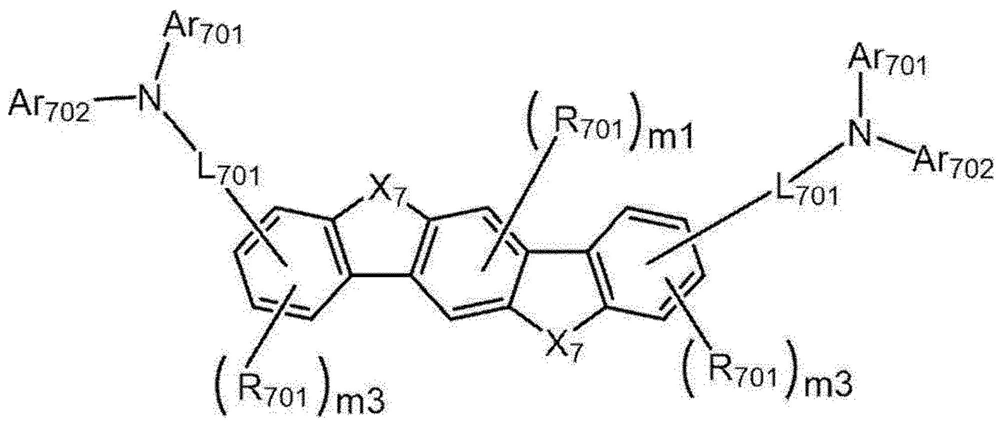

(71-5)

[Formula 166]

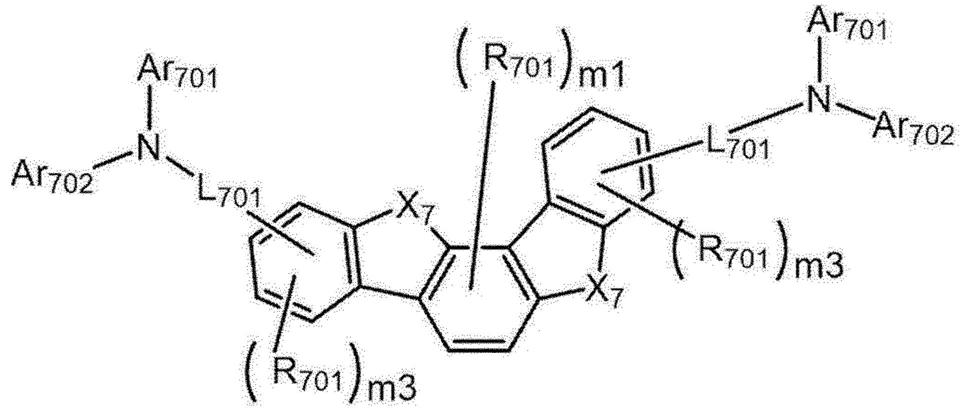

(71-6)

In the formulae (71-1) to (71-6), $R_{701}$, $X_7$, $Ar_{701}$, $Ar_{702}$, $L_{701}$, m1 and m3 respectively represent the same as $R_{701}$, $X_7$, $Ar_{701}$, $Ar_{702}$, $L_{701}$, m1 and m3 in the formula (7).

In an exemplary embodiment, the compound represented by the formula (7) is represented by any one of formulae (71-11) to (71-13) below.

[Formula 167]

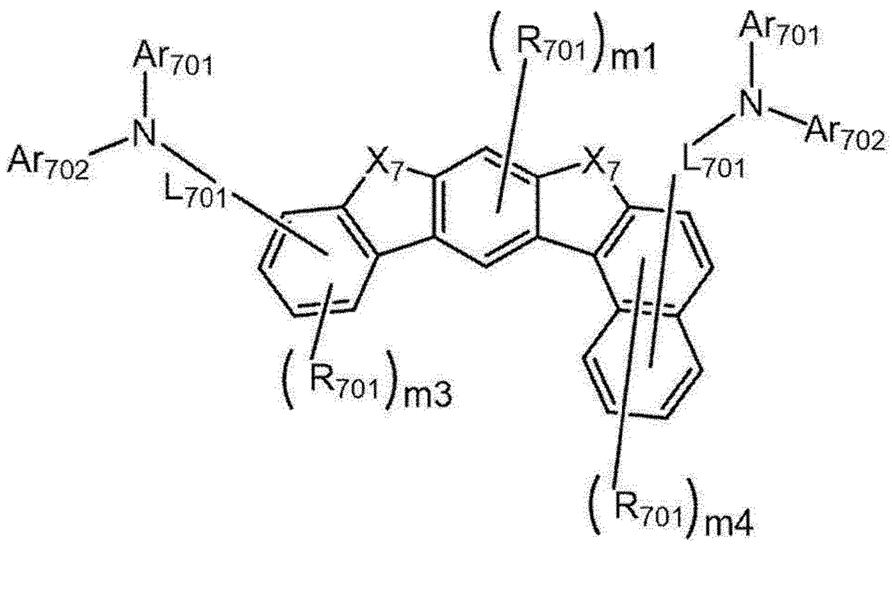

(71-11)

[Formula 168]

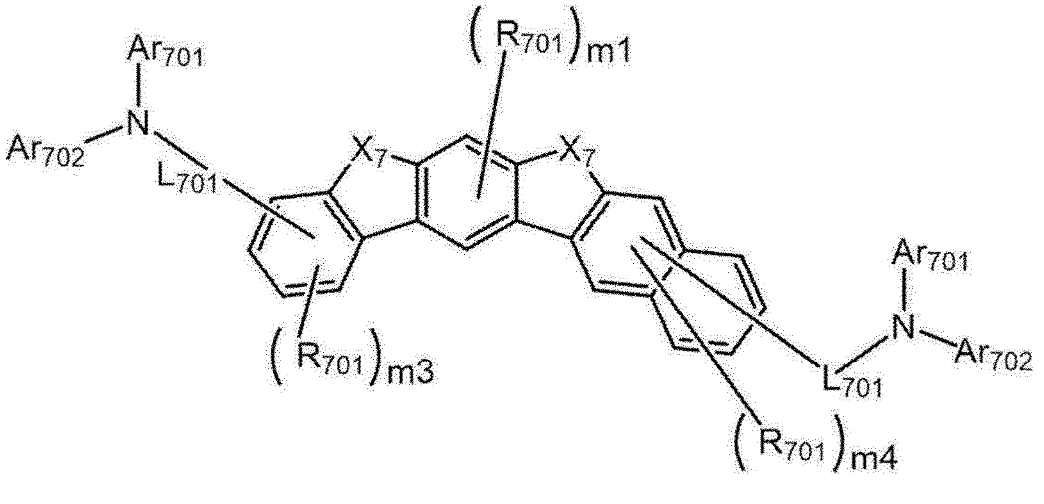

(71-12)

[Formula 169]

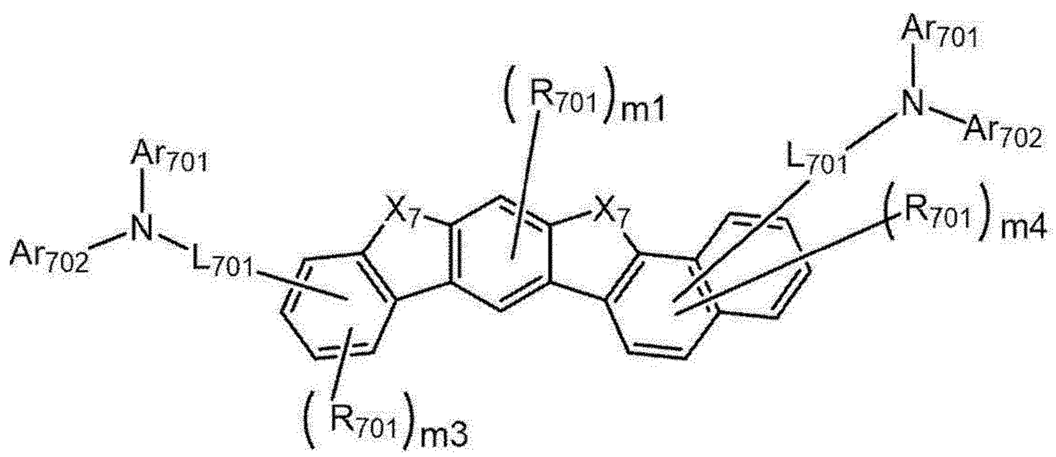

(71-13)

In the formulae (71-11) to (71-13), $R_{701}$, $X_7$, $Ar_{701}$, $Ar_{702}$, $L_{701}$, m1, m3 and m4 respectively represent the same as $R_{701}$, $X_7$, $Ar_{701}$, $Ar_{702}$, $L_{701}$, m1, m3 and m4 in the formula (7).

In an exemplary embodiment, the compound represented by the formula (7) is represented by any one of formulae (71-21) to (71-25) below.

[Formula 170]

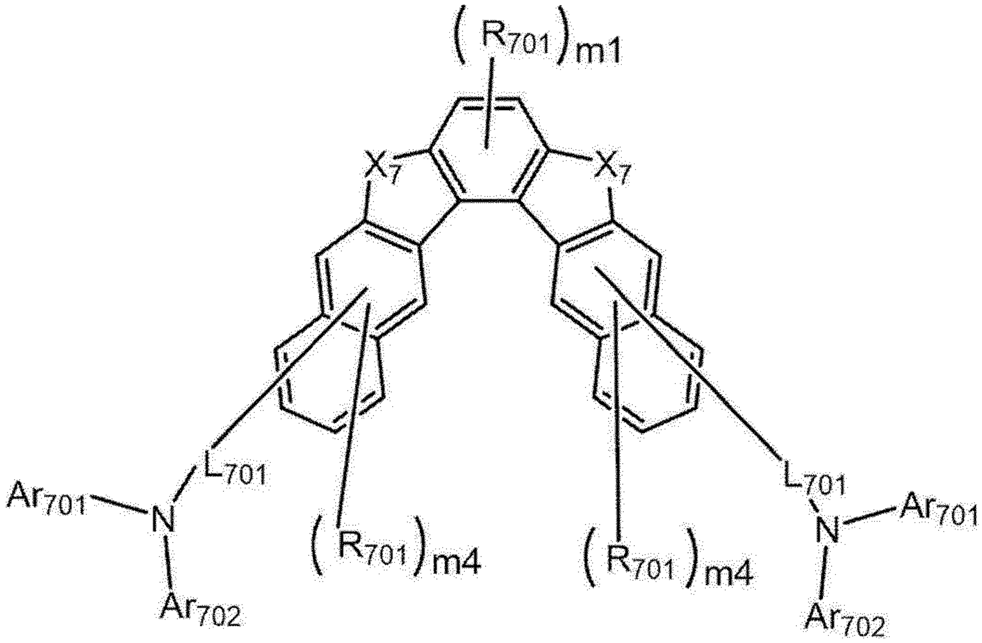

(71-21)

[Formula 171]

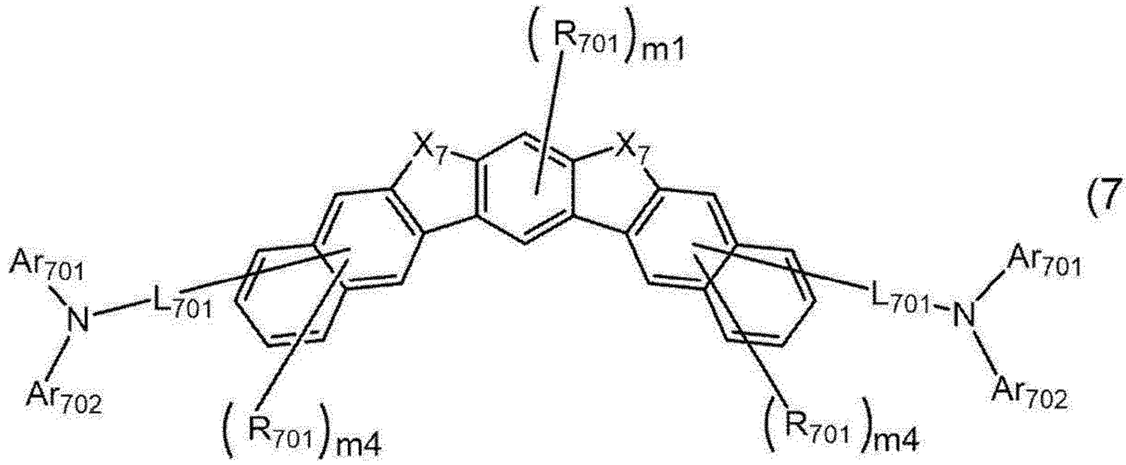

(71-22)

[Formula 172]

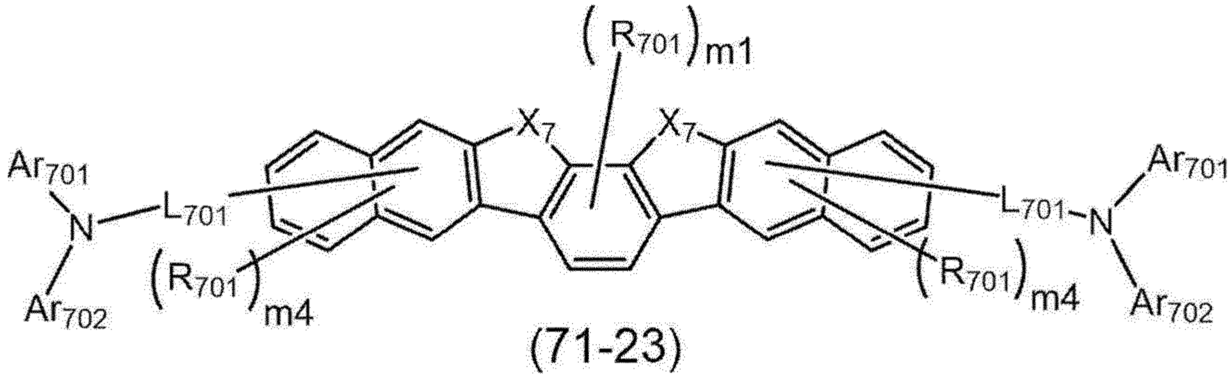

(71-23)

[Formula 173]

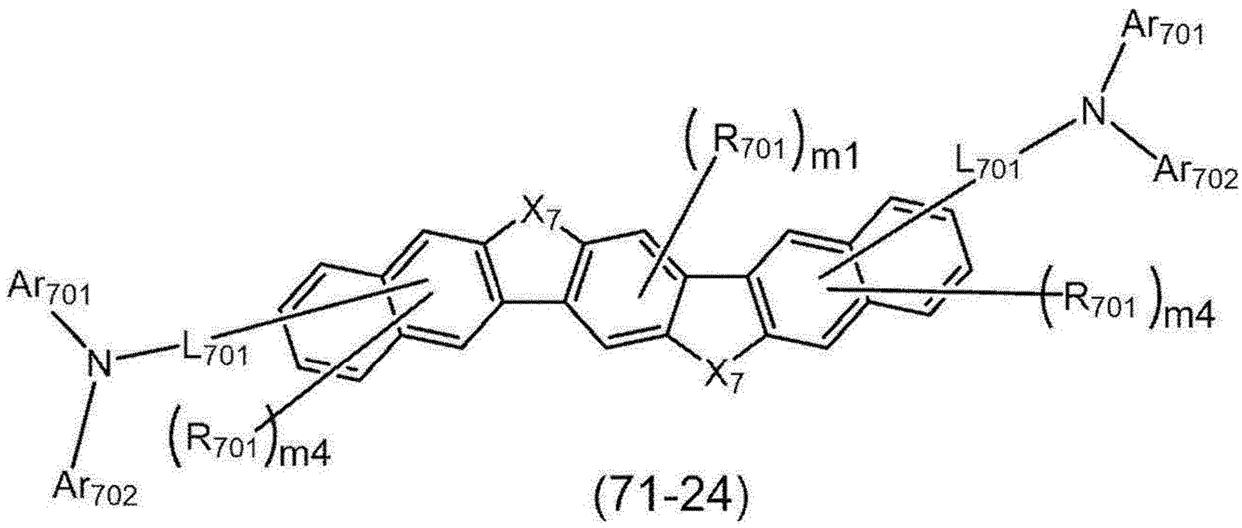

(71-24)

[Formula 174]

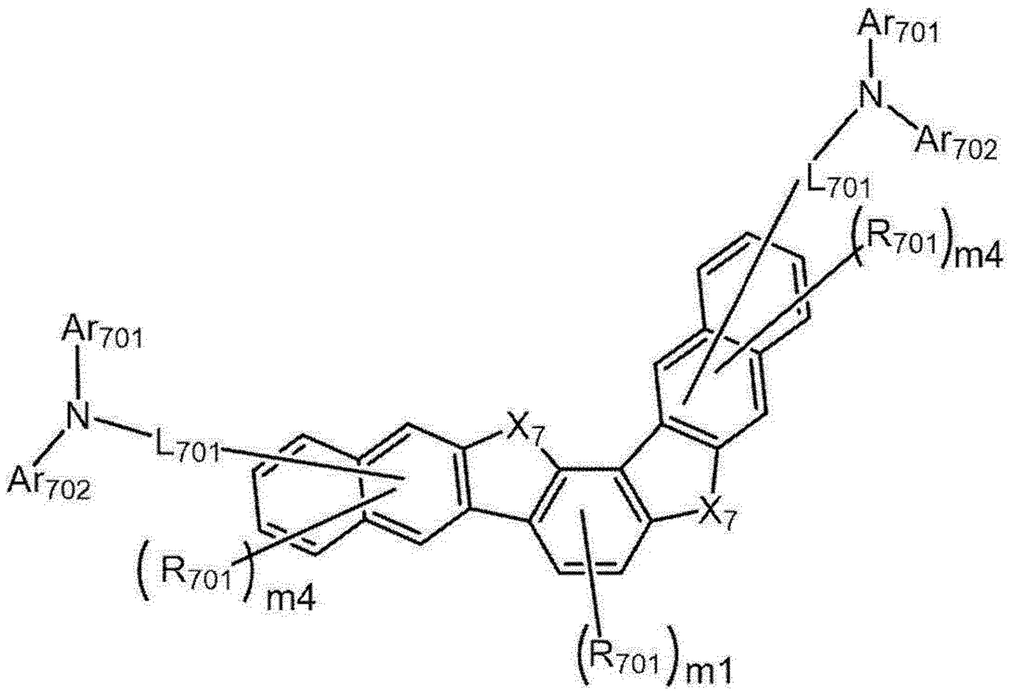

(71-25)

In the formulae (71-21) to (71-25), $R_{701}$, $X_7$, $Ar_{701}$, $Ar_{702}$, $L_{701}$, m1 and m4 respectively represent the same as $R_{701}$, $X_7$, $Ar_{701}$, $Ar_{702}$, $L_{701}$, m1 and m4 in the formula (7).

In an exemplary embodiment, the compound represented by the formula (7) is represented by any one of formulae (71-31) to (71-33) below.

[Formula 175]

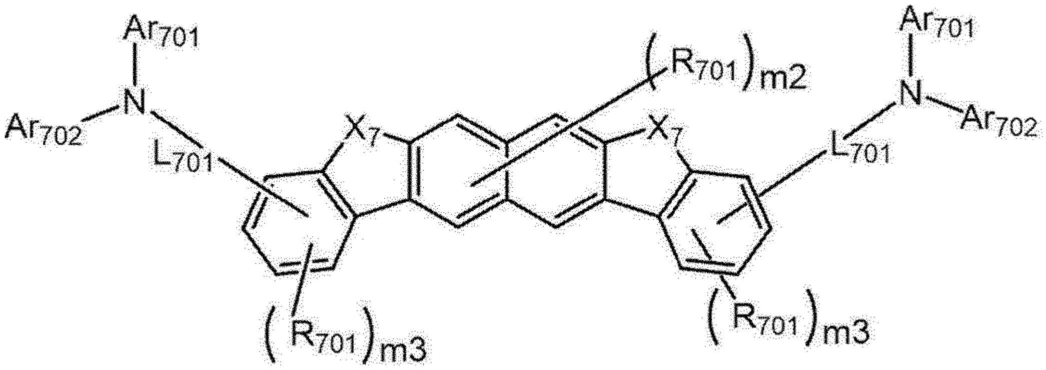

(71-31)

[Formula 176]

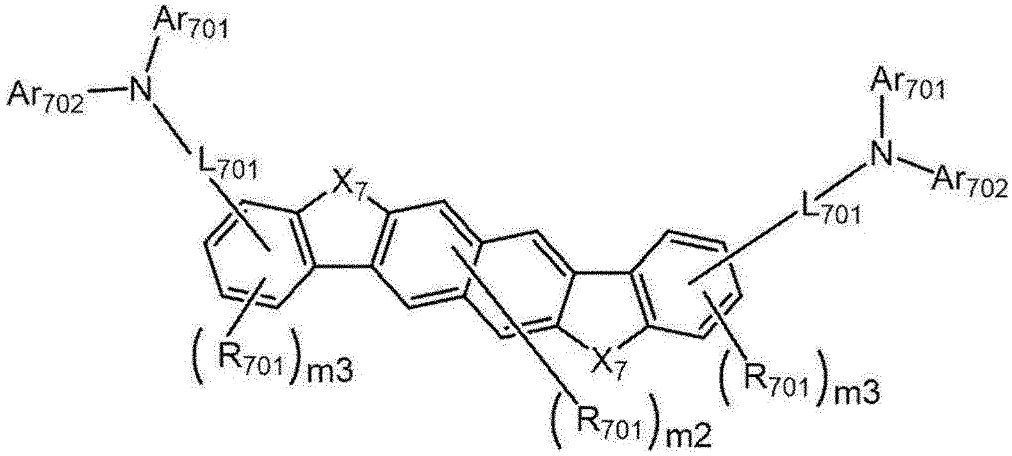

(71-32)

[Formula 177]

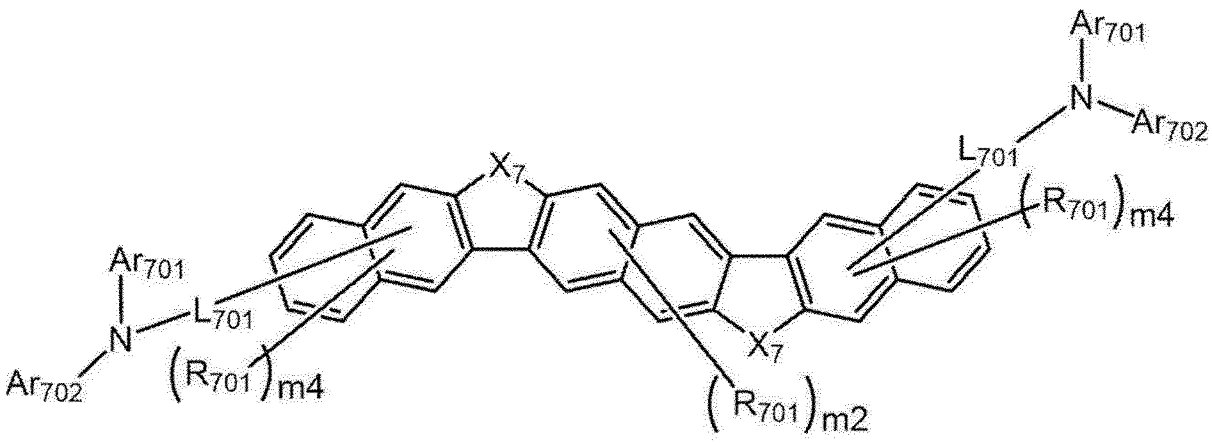

(71-33)

In the formulae (71-31) to (71-33), $R_{701}$, $X_7$, $Ar_{701}$, $Ar_{702}$, $L_{701}$, and m2 to m4 respectively represent the same as $R_{701}$, $X_7$, $Ar_{701}$, $Ar_{702}$, $L_{701}$, and m2 to m4 in the formula (7).

In an exemplary embodiment, $Ar_{701}$ and $Ar_{702}$ are each independently a substituted or unsubstituted aryl group having 6 to 50 ring carbon atoms.

In an exemplary embodiment, one of $Ar_{701}$ and $Ar_{702}$ is a substituted or unsubstituted aryl group having 6 to 50 ring carbon atoms, and the other of $Ar_{701}$ and $Ar_{702}$ is a substituted or unsubstituted heterocyclic group having 5 to 50 ring atoms.

Specific examples of the compound represented by the formula (7) include compounds given below.

[Formula 178]

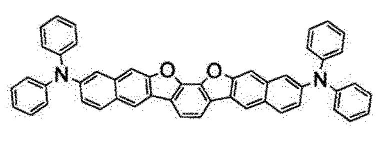
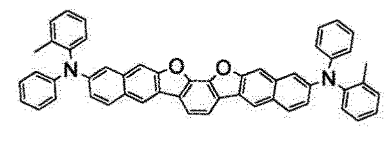
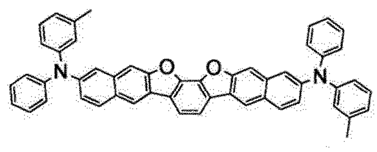

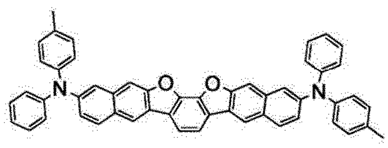
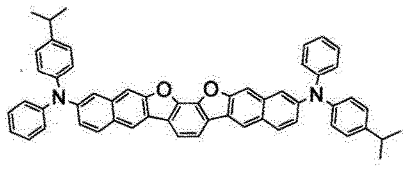
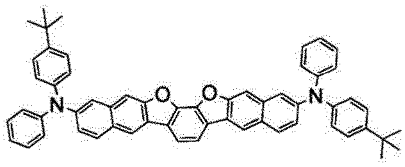

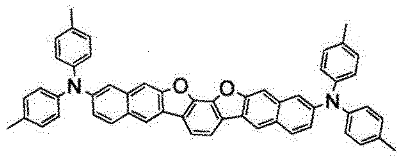
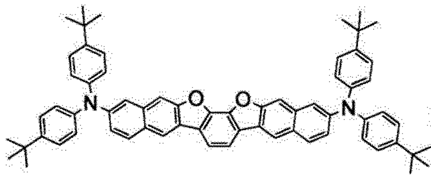
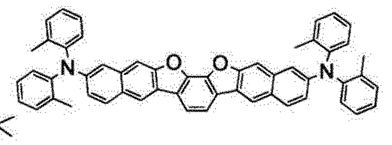

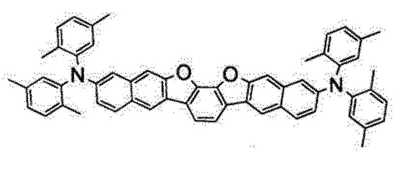
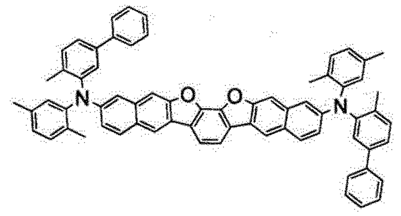

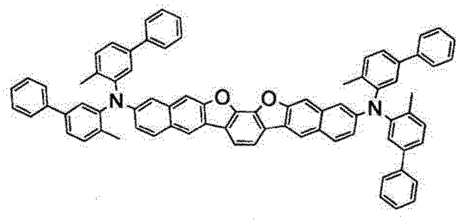
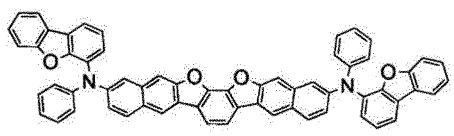

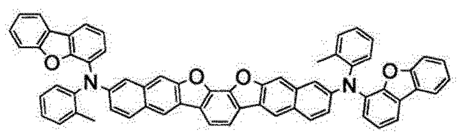
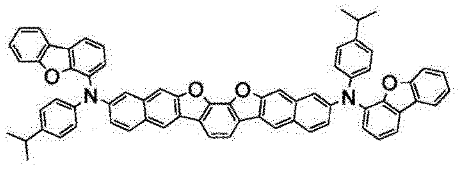

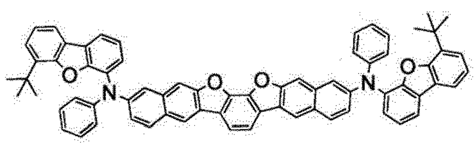
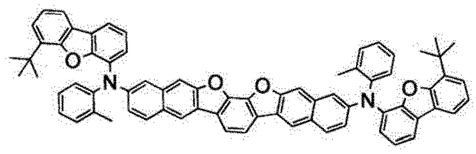

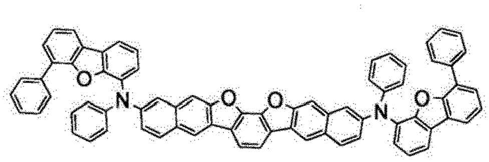
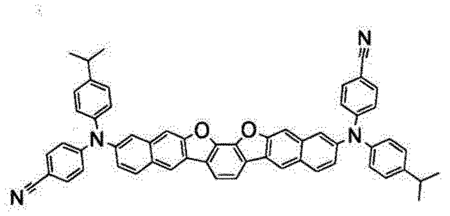

[Formula 179]

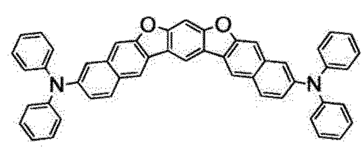
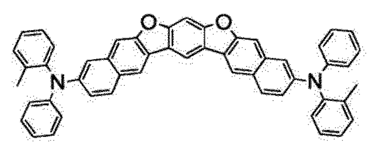
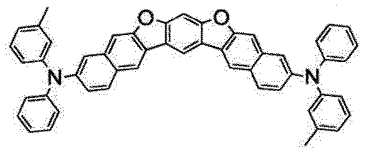
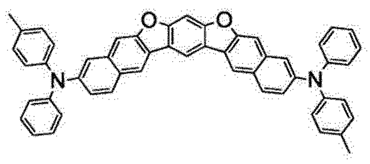
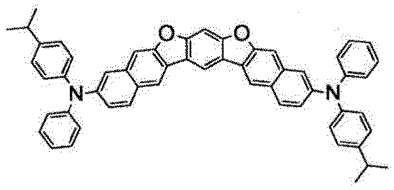
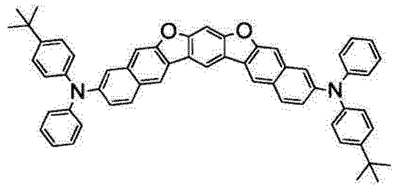
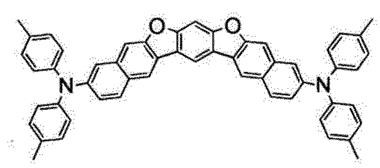
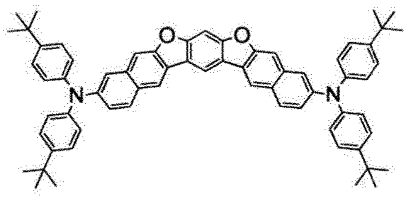
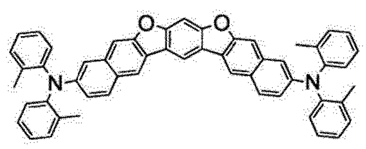
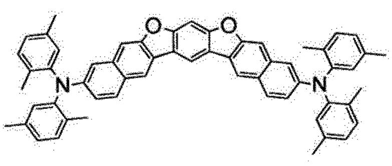
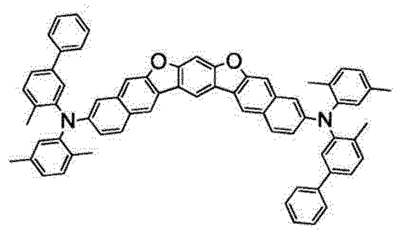
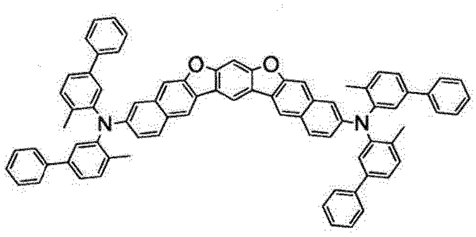
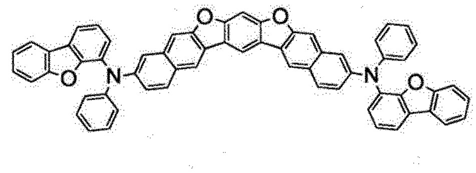
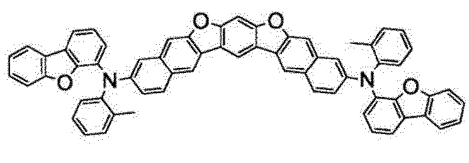
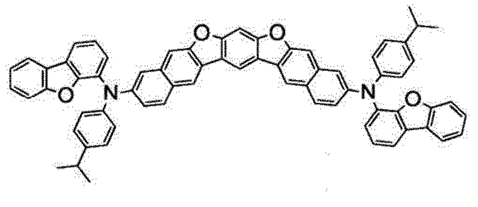
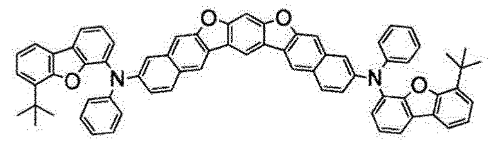
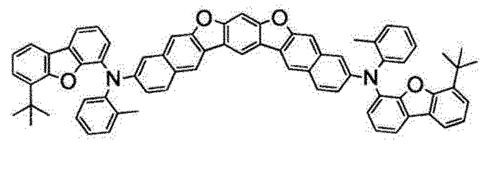
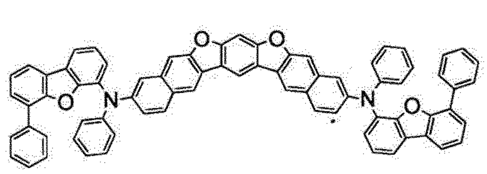
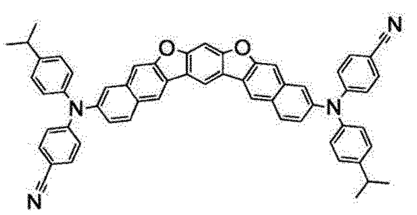

[Formula 180]

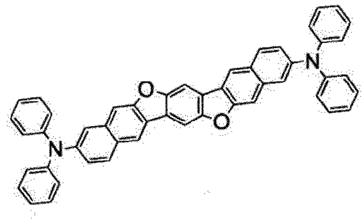
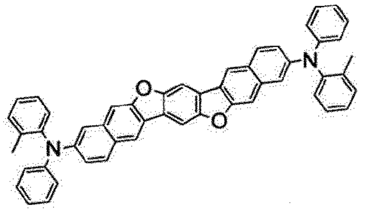
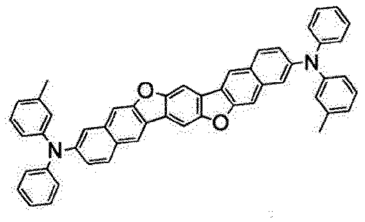
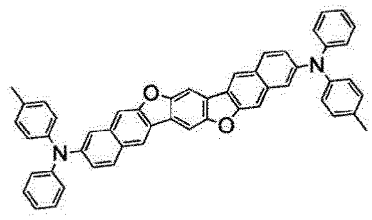
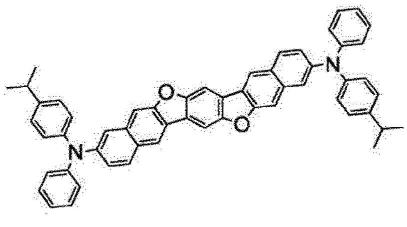
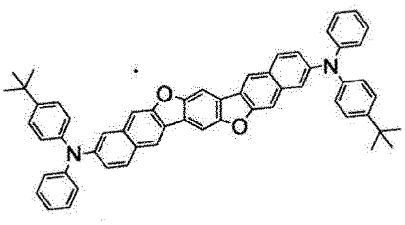
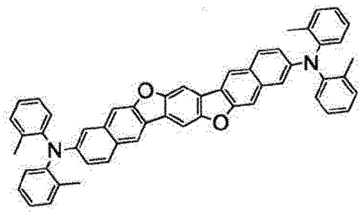
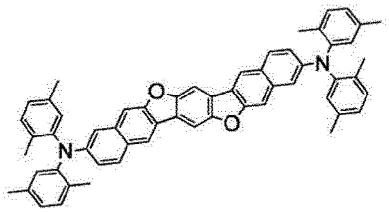
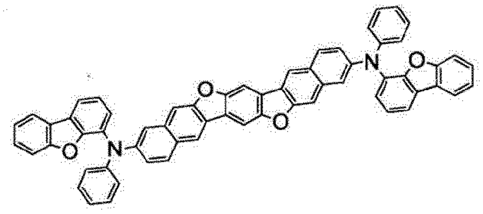
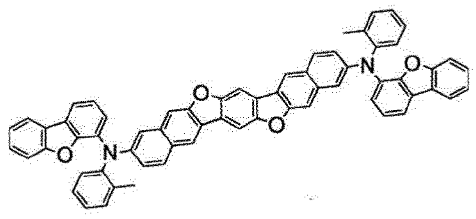
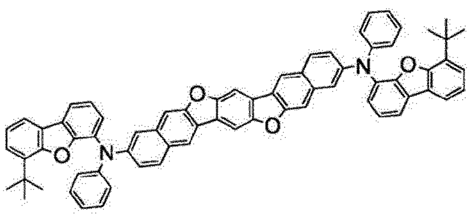
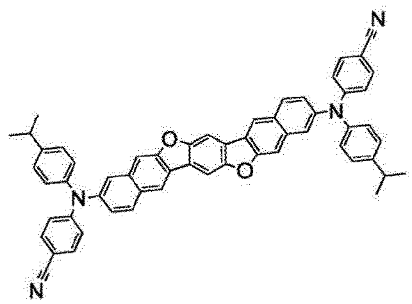
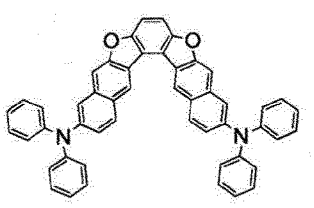
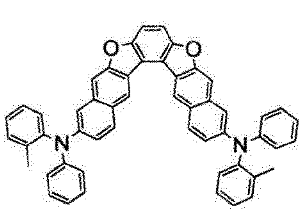
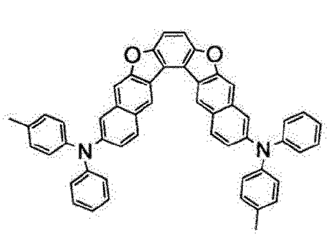

[Formula 181]

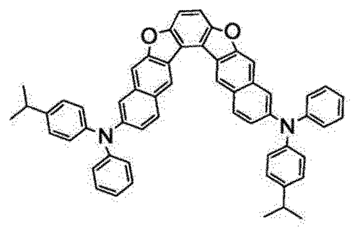
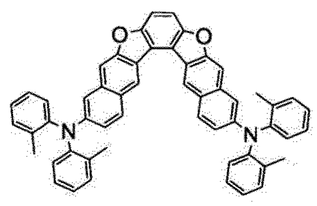
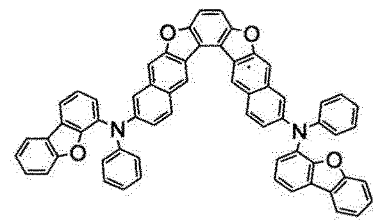
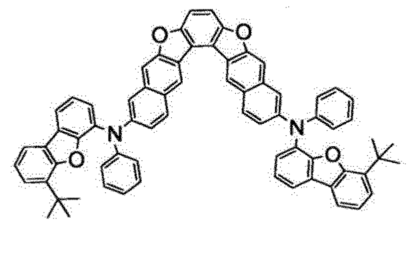
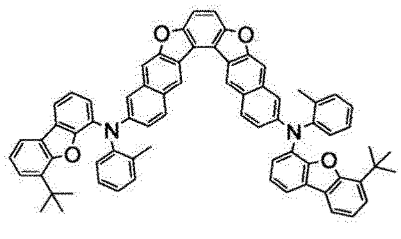
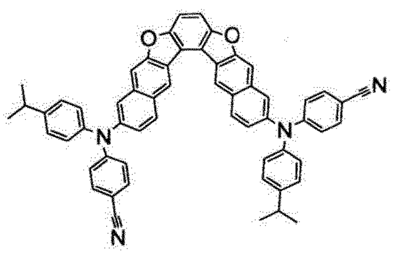
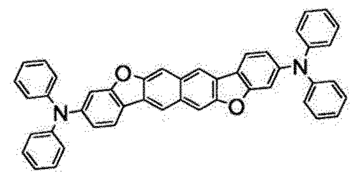
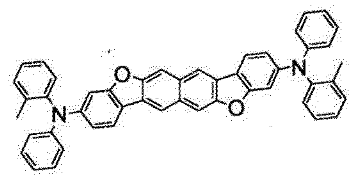
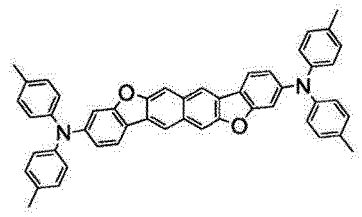
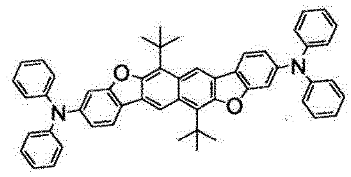
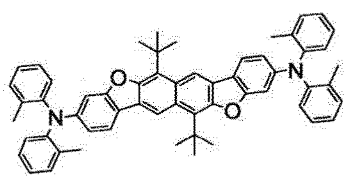
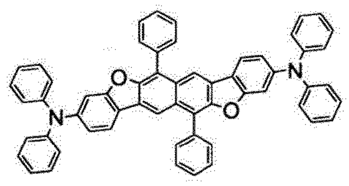
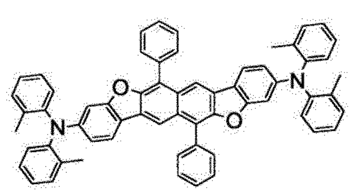
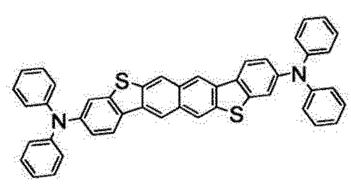
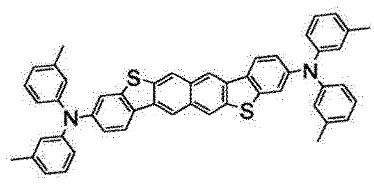
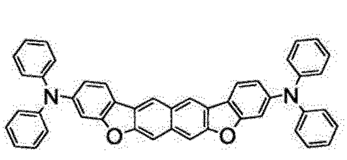
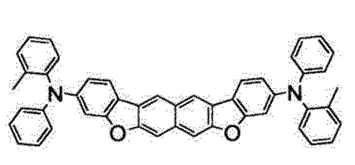
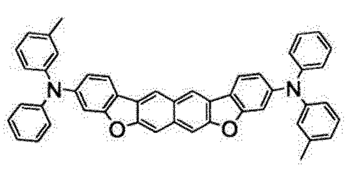
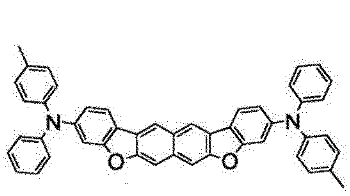
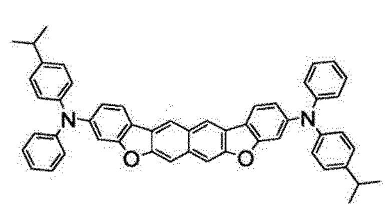
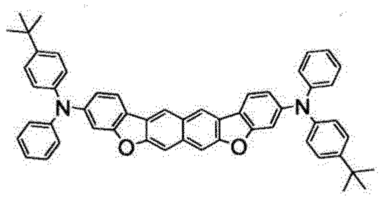

[Formula 182]

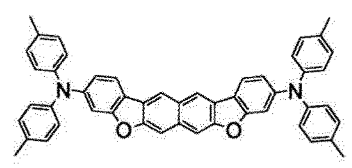
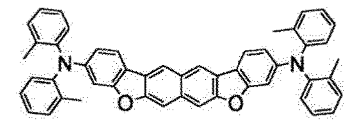
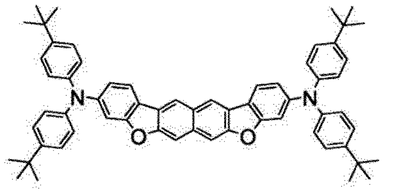
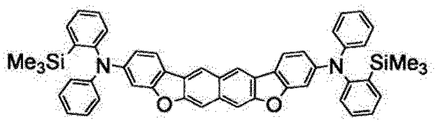
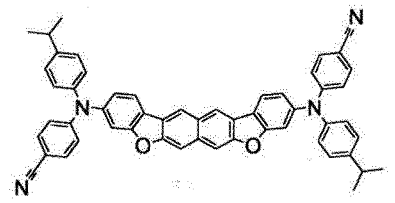
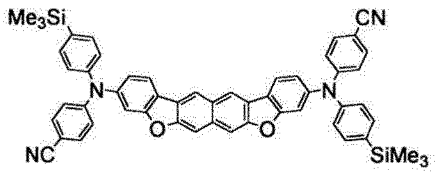
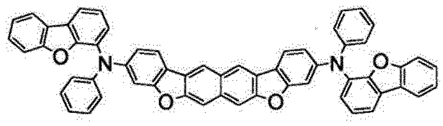
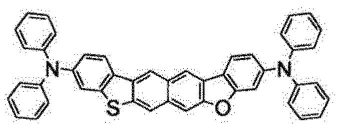
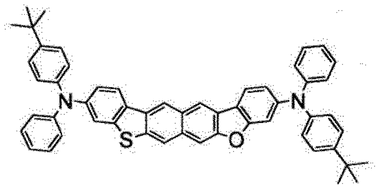
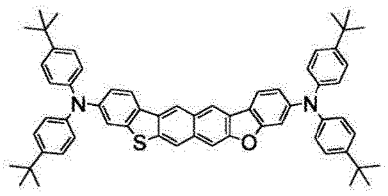
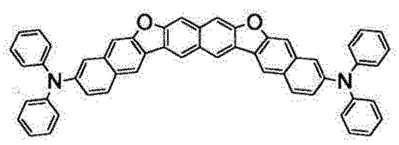
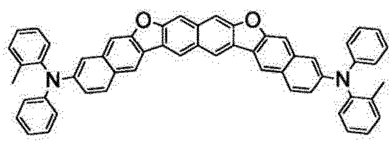
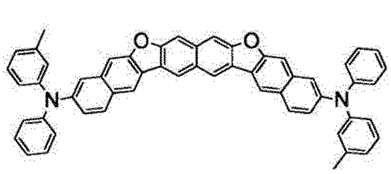
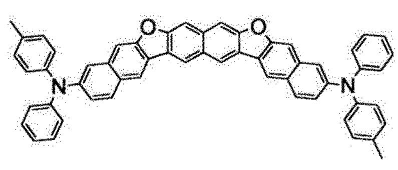
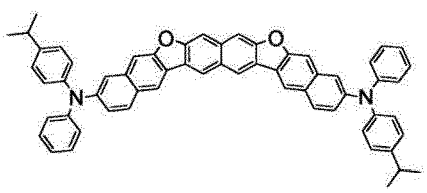
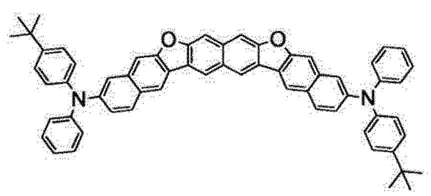

[Formula 183]

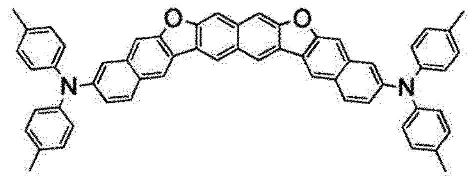

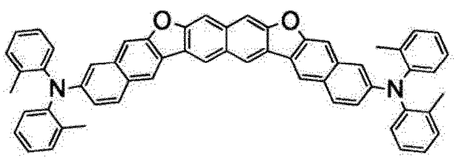

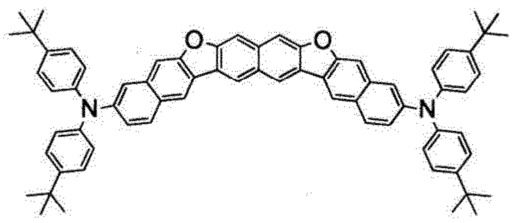

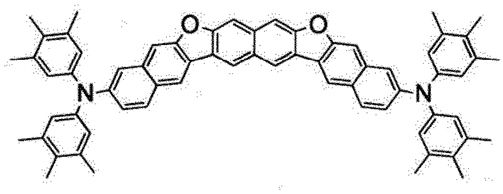

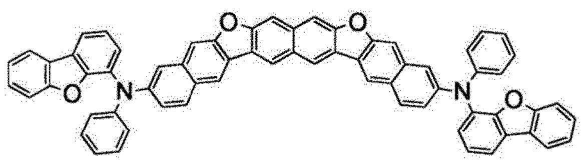

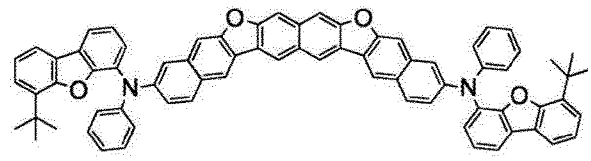

Compound Represented by Formula (8)

The compound represented by the formula (8) will be described below.

[Formula 184]

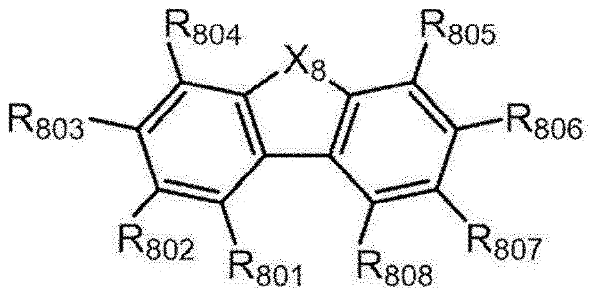

(8)

In the formula (8):

at least one combination of $R_{801}$ and $R_{802}$, $R_{802}$ and $R_{803}$, and $R_{803}$ and $R_{804}$ are mutually bonded to form a divalent group represented by a formula (82) below; and

at least one combination of $R_{805}$ and $R_{806}$, $R_{806}$ and $R_{807}$, and $R_{807}$ and $R_{808}$ are mutually bonded to form a divalent group represented by a formula (83) below.

[Formula 185]

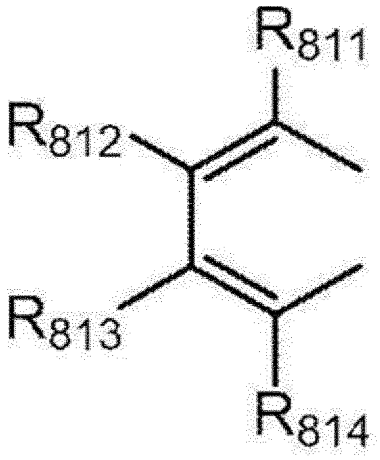

(82)

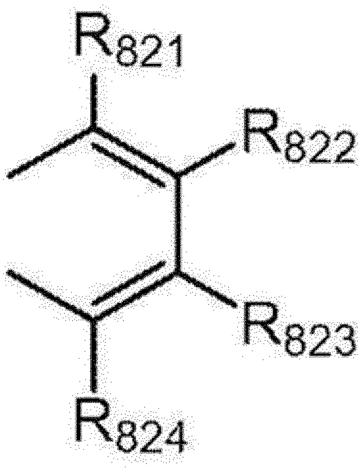

(83)

At least one of $R_{801}$ to $R_{804}$ not forming the divalent group represented by the formula (82) or $R_{811}$ to $R_{814}$ is a monovalent group represented by a formula (84) below;

at least one of $R_{805}$ to $R_{808}$ not forming the divalent group represented by the formula (83) or $R_{821}$ to $R_{824}$ is a monovalent group represented by a formula (84) below;
$X_8$ is an oxygen atom, a sulfur atom, or $NR_{809}$; and
$R_{801}$ to $R_{808}$ not forming the divalent group represented by the formula (82) or (83) and not being the monovalent group represented by the formula (84), $R_{811}$ to $R_{814}$ and $R_{821}$ to $R_{824}$ not being the monovalent group represented by the formula (84), and $R_{809}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

[Formula 186]

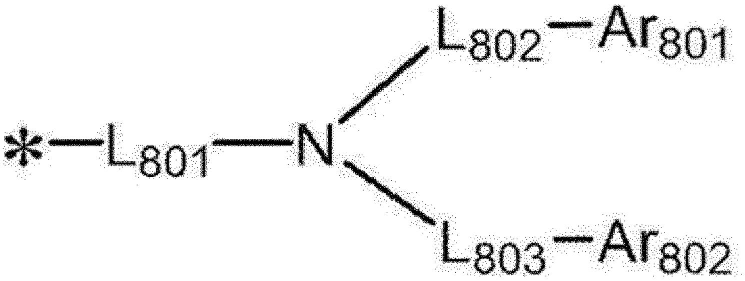

(84)

In the formula (84):

$Ar_{801}$ and $Ar_{802}$ are each independently a substituted or unsubstituted aryl group having 6 to 50 ring carbon atoms, or a substituted or unsubstituted heterocyclic group having 5 to 50 ring atoms;
$L_{801}$ to $L_{803}$ are each independently a single bond, a substituted or unsubstituted arylene group having 6 to 30 ring carbon atoms, a substituted or unsubstituted divalent heterocyclic group having 5 to 30 ring atoms, or a divalent linking group formed by bonding two, three or four groups selected from the group consisting of a substituted or unsubstituted arylene group having 6 to 30 ring carbon atoms and a substituted or unsubstituted divalent heterocyclic group having 5 to 30 ring atoms; and
* in the formula (84) represents a bonding position to a cyclic structure represented by the formula (8) or a bonding

position to a group represented by the formula (82) or (83).

In the formula (8), the positions for the divalent group represented by the formula (82) and the divalent group represented by the formula (83) to be formed are not specifically limited but the divalent groups may be formed at any possible positions on $R_{801}$ to $R_{808}$.

In an exemplary embodiment, the compound represented by the formula (8) is represented by any one of formulae (81-1) to (81-6) below.

[Formula 187]

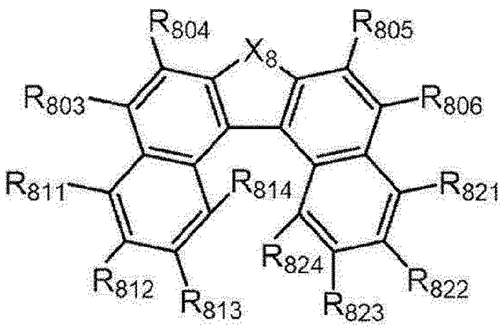

(81-1)

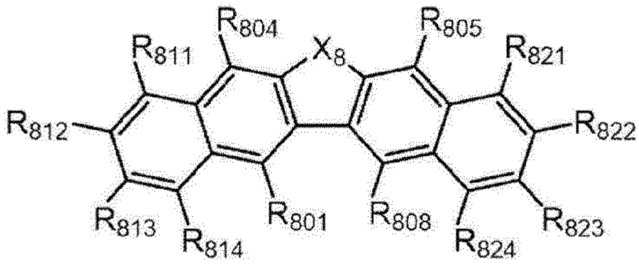

(81-2)

[Formula 188]

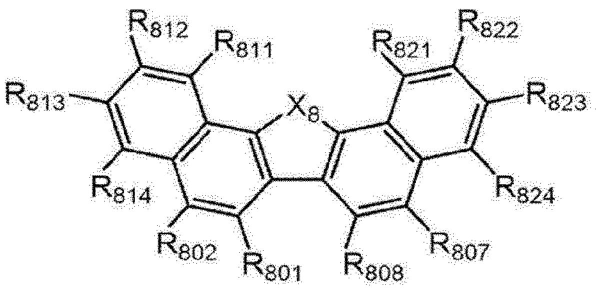

(81-3)

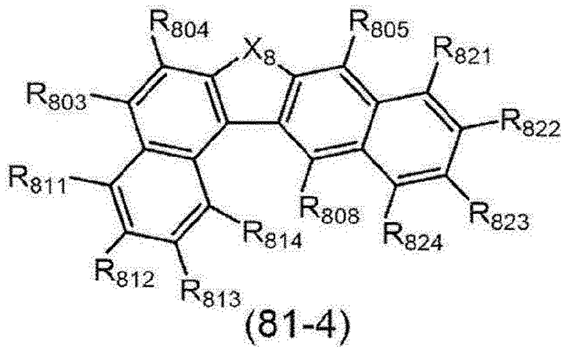

(81-4)

[Formula 189]

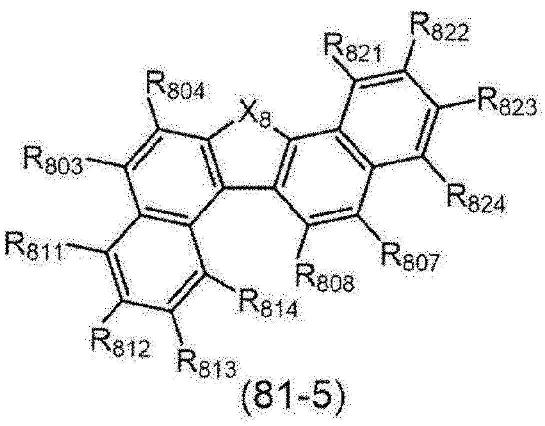

(81-5)

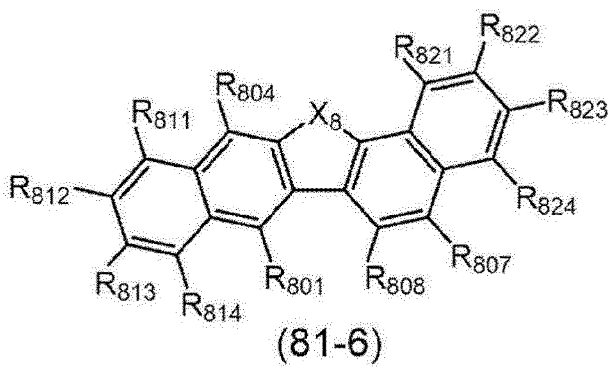

(81-6)

In the formulae (81-1) to (81-6):

$X_8$ represents the same as $X_8$ in the formula (8);

at least two of $R_{801}$ to $R_{824}$ are each a monovalent group represented by the formula (84); and

$R_{801}$ to $R_{824}$ not being the monovalent group represented by the formula (84) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

In an exemplary embodiment, the compound represented by the formula (8) is represented by any one of formulae (81-7) to (81-18) below.

[Formula 190]

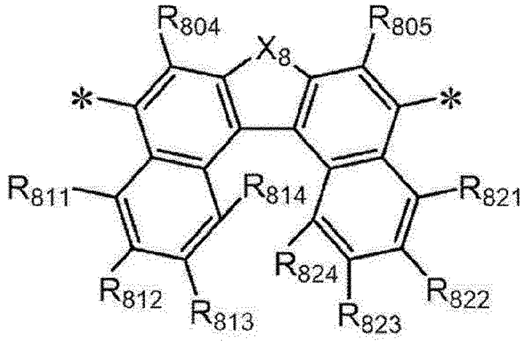

(81-7)

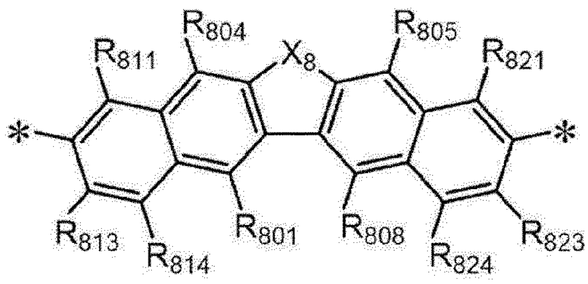

(81-8)

[0561]

[Formula 191]

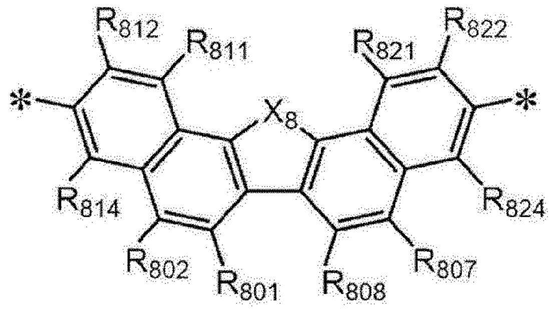

(81-9)

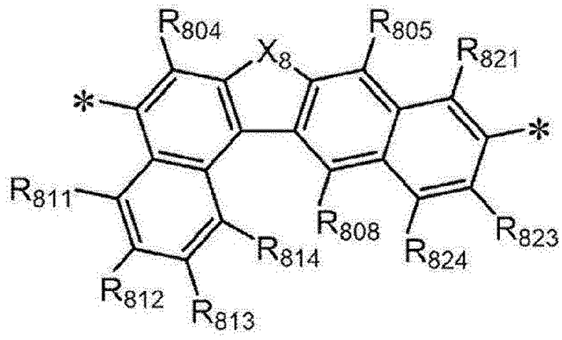

(81-10)

[Formula 192]

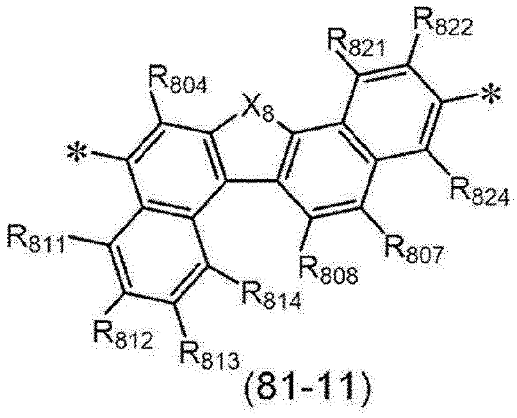

(81-11)

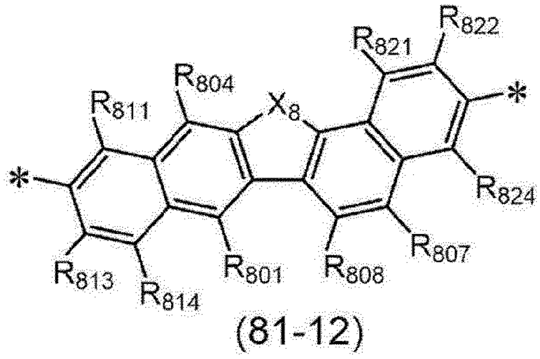

(81-12)

[Formula 193]

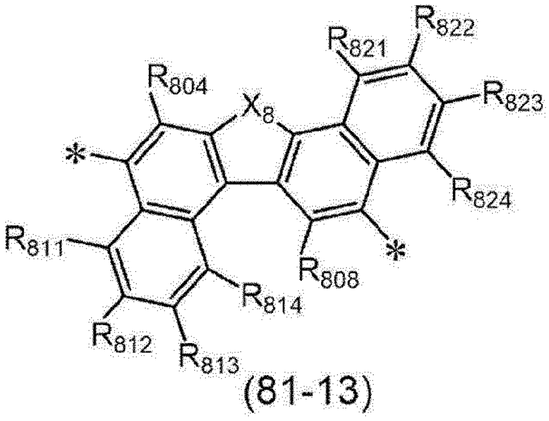

(81-13)

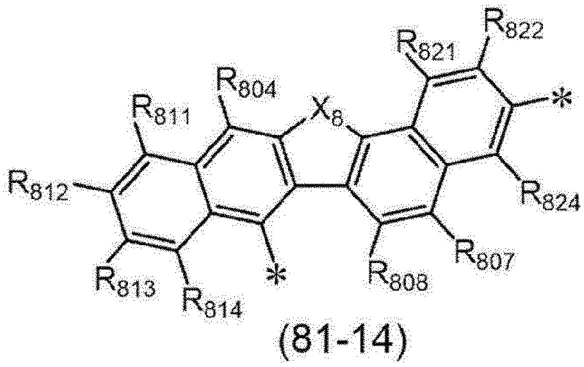

(81-14)

[Formula 194]

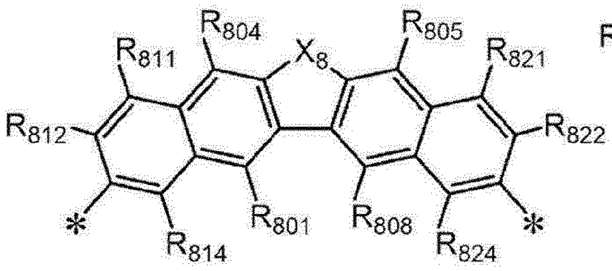

(81-15)

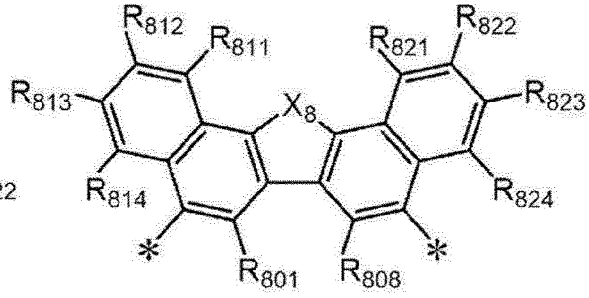

(81-16)

[Formula 195]

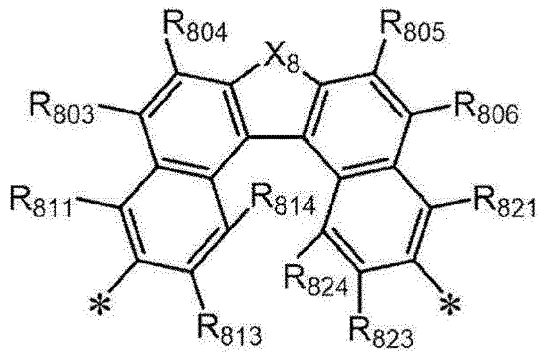

(81-17)

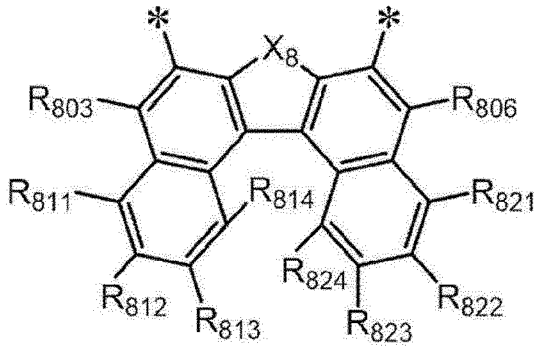

(81-18)

In the formulae (81-7) to (81-18):

$X_8$ represents the same as $X_8$ in the formula (8);
* is a single bond bonded to a monovalent group represented by the formula (84); and
$R_{801}$ to $R_{824}$ each independently represent the same as $R_{801}$ to $R_{824}$ in the formulae (81-1) to (81-6) not being the monovalent group represented by the formula (84).

$R_{801}$ to $R_{808}$ not forming the divalent group represented by the formula (82) or (83) and not being the monovalent group represented by the formula (84), and $R_{811}$ to $R_{814}$ and $R_{821}$ to $R_{824}$ not being the monovalent group represented by the formula (84) are preferably each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

The monovalent group represented by the formula (84) is preferably represented by a formula (85) or (86) below.

[Formula 196]

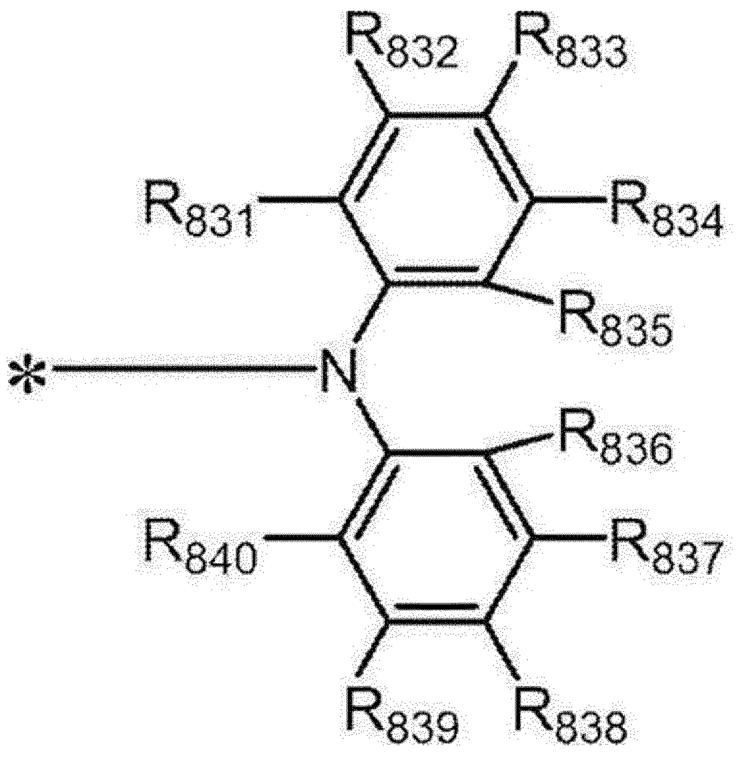

(85)

In the formula (85):

$R_{831}$ to $R_{840}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and

* in the formula (85) represents the same as * in the formula (84).

[Formula 197]

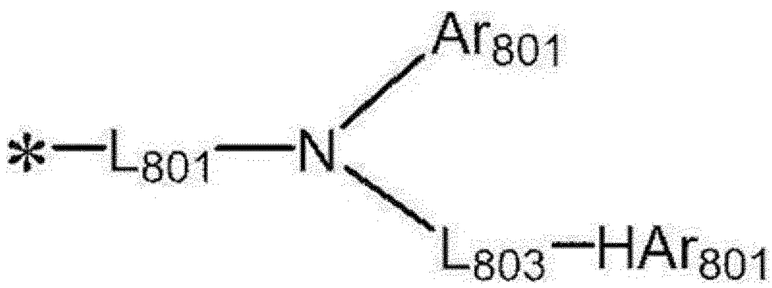

(86)

In the formula (86):

$Ar_{801}$, $L_{801}$, and $L_{803}$ represent the same as $Ar_{801}$, $L_{801}$, and $L_{803}$ in the formula (84); and

$HAr_{801}$ is a structure represented by a formula (87) below.

[Formula 198]

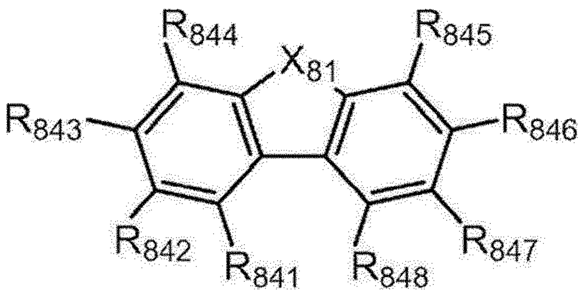

(87)

In the formula (87):

$X_{81}$ is an oxygen atom or a sulfur atom;

one of $R_{841}$ to $R_{848}$ is a single bond with $L_{803}$; and

$R_{841}$ to $R_{848}$ not being the single bond are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

Specific examples of the compound represented by the formula (8) include compounds given below as well as the compounds disclosed in WO 2014/104144.

[Formula 199]

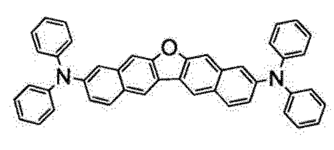
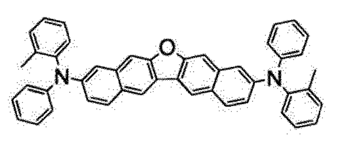
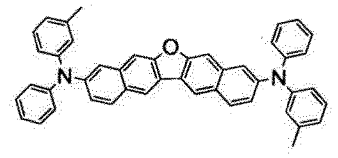
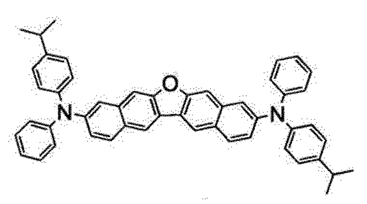
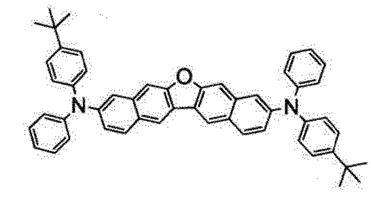
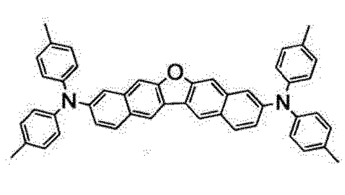
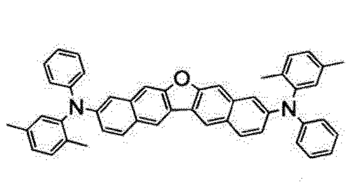
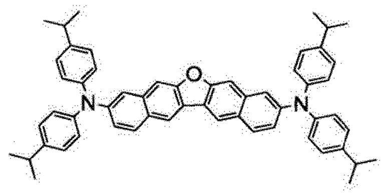
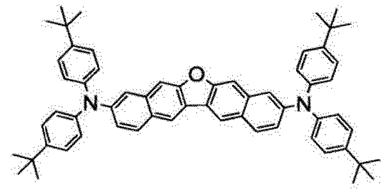
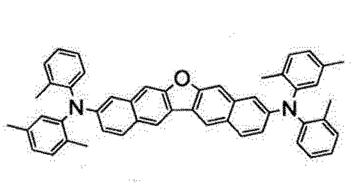
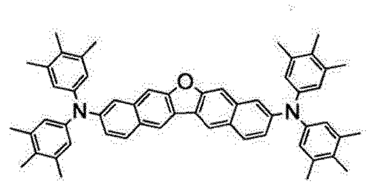
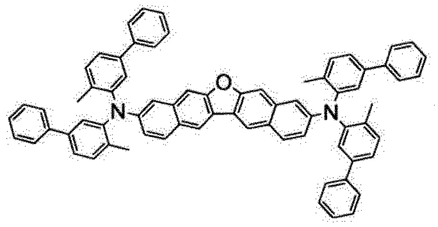
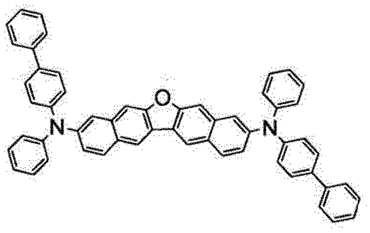
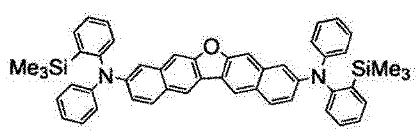
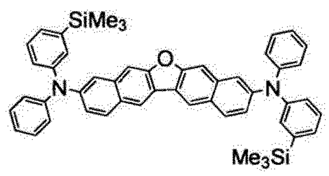
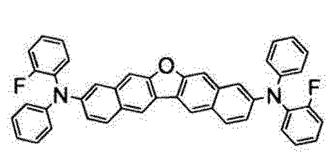
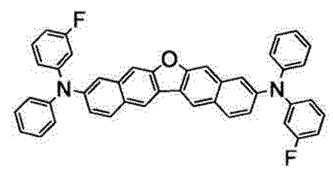
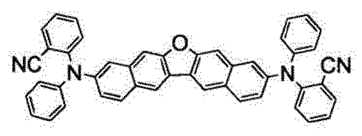
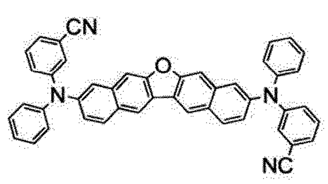
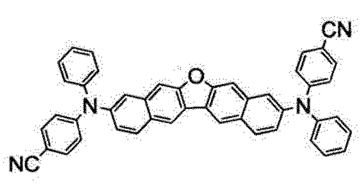
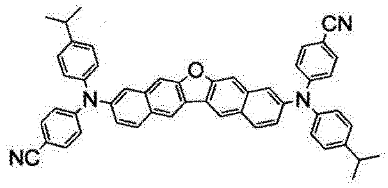

[Formula 200]

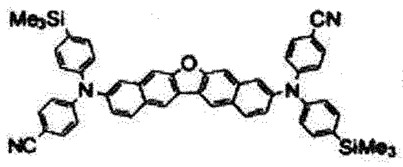
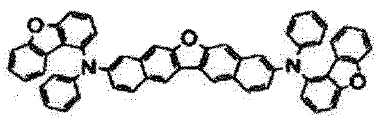
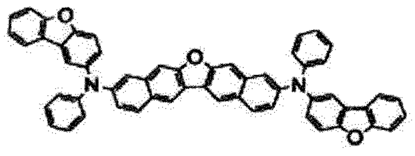

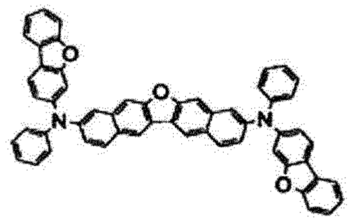
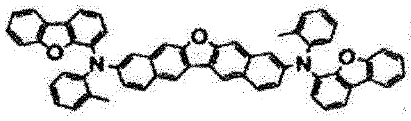
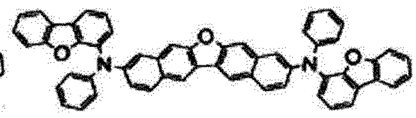

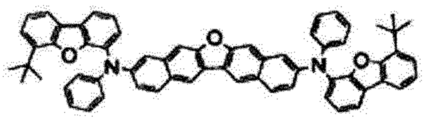
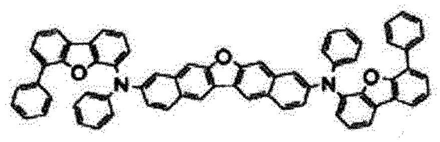
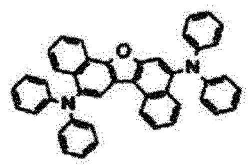

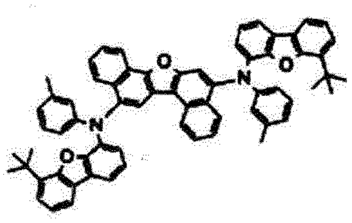
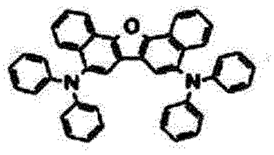
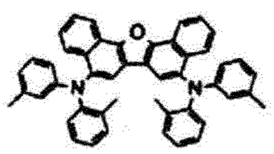

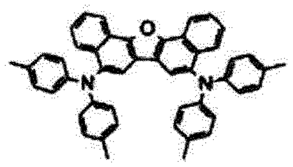

[Formula 201]

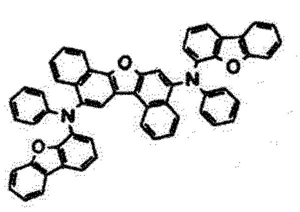
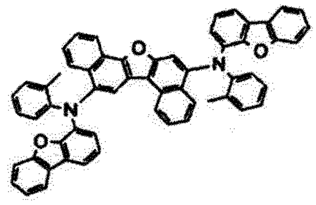
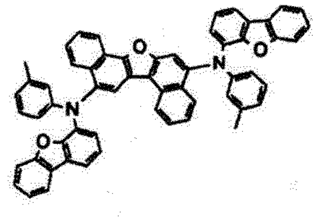
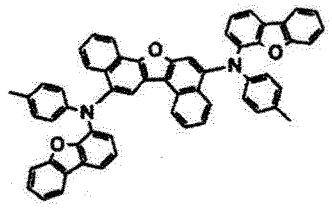
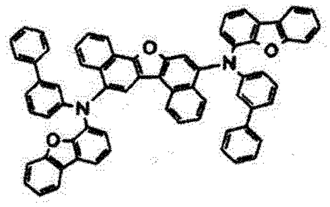
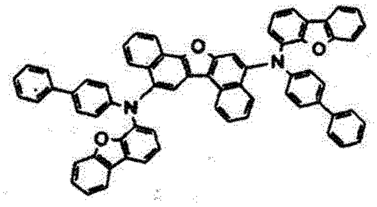
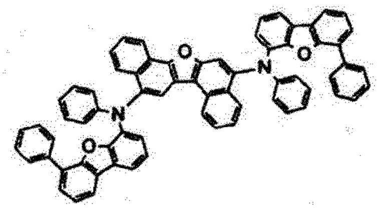
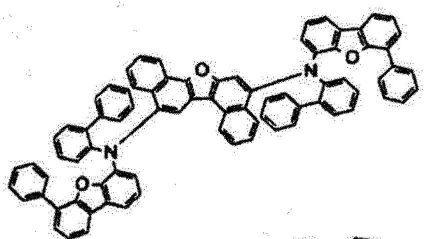
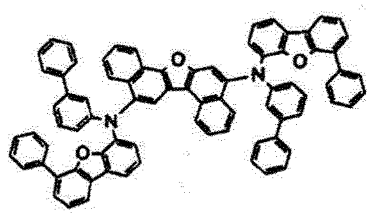
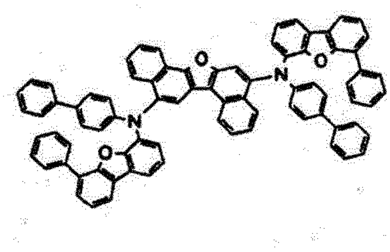
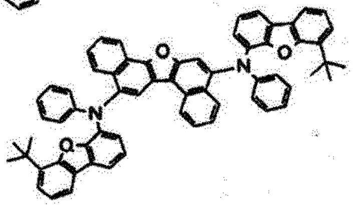
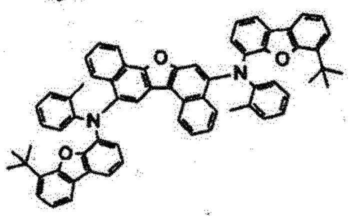

[Formula 202]

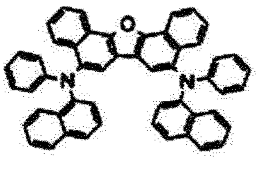
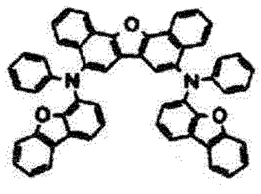
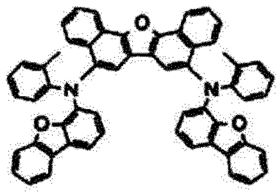
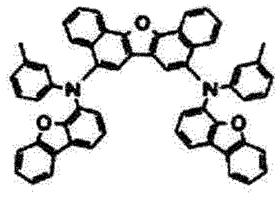

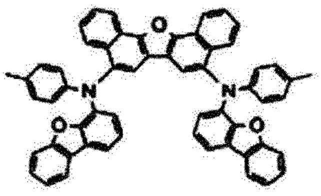
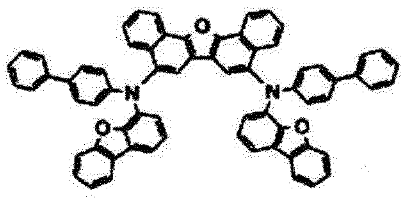

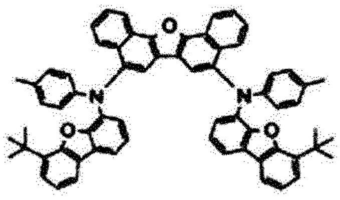
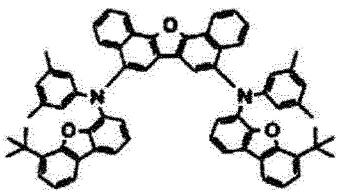

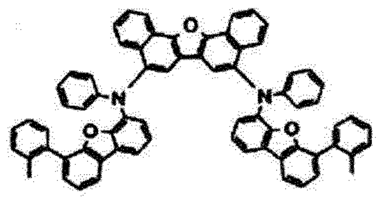
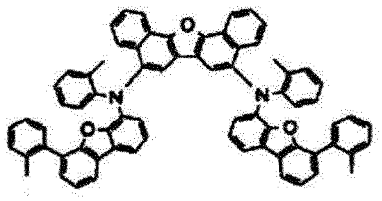

[Formula 203]

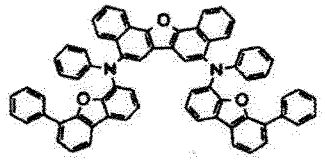

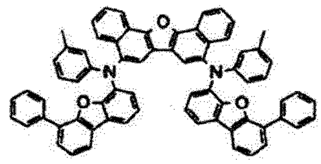

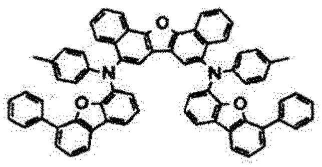

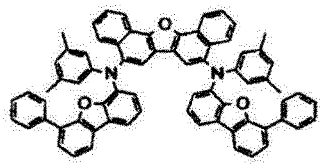

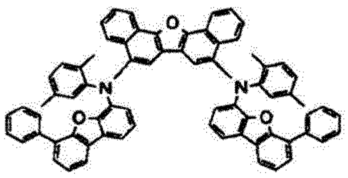

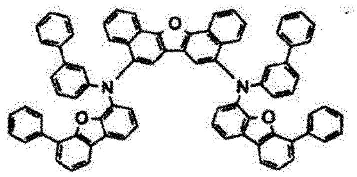

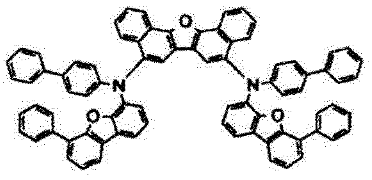

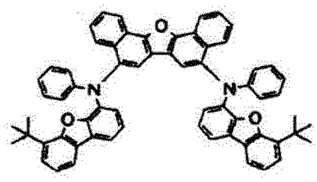

[Formula 204]

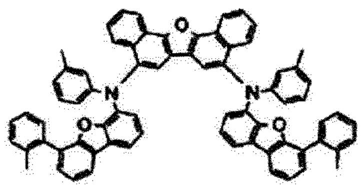 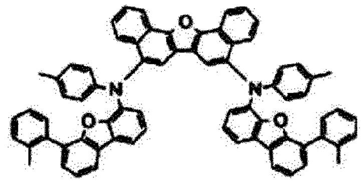 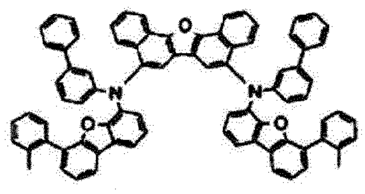

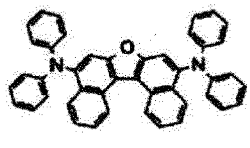 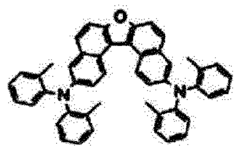 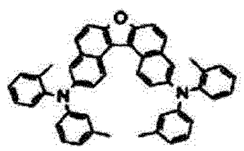

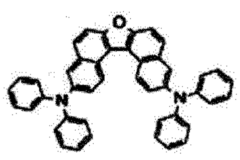 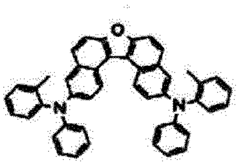 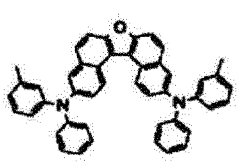 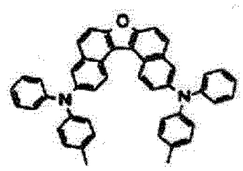

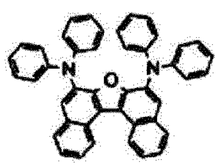 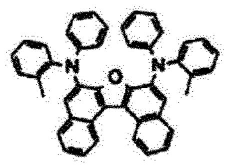 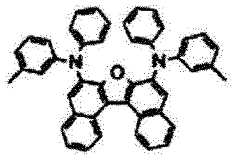 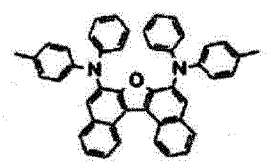

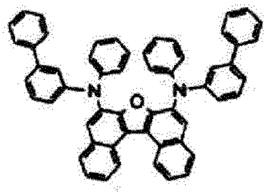 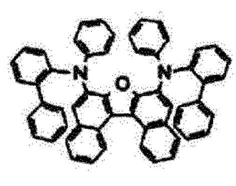

Compound Represented by Formula (9)

The compound represented by the formula (9) will be described below.

[Formula 205]

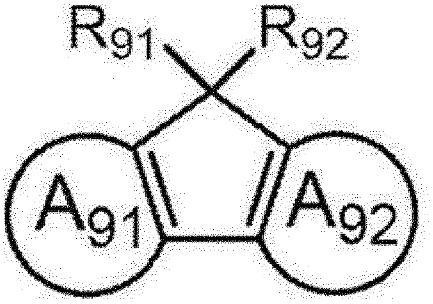

(9)

In the formula (9):

a ring $A_{91}$ and a ring $A_{92}$ are each independently a substituted or unsubstituted aromatic hydrocarbon ring having 6 to 50 ring carbon atoms, or a substituted or unsubstituted heterocycle having 5 to 50 ring atoms; and
at least one of the ring $A_{91}$ or the ring $A_{92}$ is bonded with * in a structure represented by a formula (92) below.

[Formula 206]

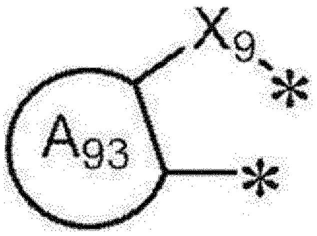

(92)

In the formula (92):

a ring $A_{93}$ is a substituted or unsubstituted aromatic hydrocarbon ring having 6 to 50 ring carbon atoms, or a substituted or unsubstituted heterocycle having 5 to 50 ring atoms;

$X_9$ is $NR_{93}$, $C(R_{94})(R_{95})$, $Si(R_{96})(R_{97})$, $Ge(R_{98})(R_{99})$, an oxygen atom, a sulfur atom, or a selenium atom;

$R_{91}$ and $R_{92}$ are mutually bonded to form a substituted or unsubstituted monocyclic ring, mutually bonded to form a substituted or unsubstituted fused ring, or not mutually bonded; and

$R_{91}$ and $R_{92}$ forming neither the monocyclic ring nor the fused ring, and $R_{93}$ to $R_{99}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

At least one of the ring $A_{91}$ or the ring $A_{92}$ is bonded to * in a structure represented by the formula (92). In other words, the ring carbon atoms of the aromatic hydrocarbon ring or the ring atoms of the heterocycle of the ring $A_{91}$ in an exemplary embodiment are bonded to * in a structure represented by the formula (92). Further, the ring carbon atoms of the aromatic hydrocarbon ring or the ring atoms of the heterocycle of the ring $A_{92}$ in an exemplary embodiment are bonded to * in a structure represented by the formula (92).

In an exemplary embodiment, a group represented by a formula (93) below is bonded to one or both of the ring $A_{91}$ and the ring $A_{92}$.

[Formula 207]

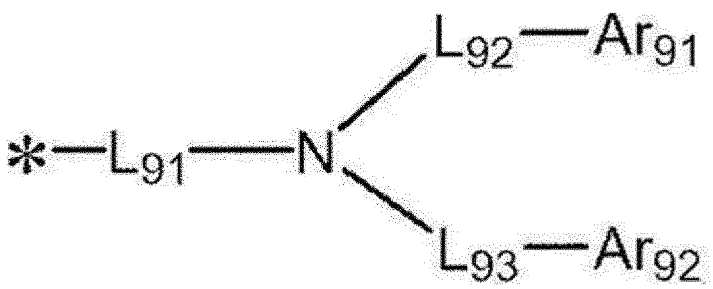

(93)

In the formula (93):

$Ar_{91}$ and $Ar_{92}$ are each independently a substituted or unsubstituted aryl group having 6 to 50 ring carbon atoms, or a substituted or unsubstituted heterocyclic group having 5 to 50 ring atoms;

$L_{91}$ to $L_{93}$ are each independently a single bond, a substituted or unsubstituted arylene group having 6 to 30 ring carbon atoms, a substituted or unsubstituted divalent heterocyclic group having 5 to 30 ring atoms, or a divalent linking group formed by bonding two, three or four groups selected from the group consisting of a substituted or unsubstituted arylene group having 6 to 30 ring carbon atoms and a substituted or unsubstituted divalent heterocyclic group having 5 to 30 ring atoms; and

* in the formula (93) represents a bonding position to one of the ring $A_{91}$ and the ring $A_{92}$.

In an exemplary embodiment, in addition to the ring $A_{91}$, the ring carbon atoms of the aromatic hydrocarbon ring or the ring atoms of the heterocycle of the ring $A_{92}$ are bonded to * in a structure represented by the formula (92).

In this case, the structures represented by the formula (92) may be mutually the same or different.

In an exemplary embodiment, $R_{91}$ and $R_{92}$ are each independently a substituted or unsubstituted aryl group having 6 to 50 ring carbon atoms.

In an exemplary embodiment, $R_{91}$ and $R_{92}$ are mutually bonded to form a fluorene structure.

In an exemplary embodiment, the rings $A_{91}$ and $A_{92}$ are each independently a substituted or unsubstituted aromatic hydrocarbon ring having 6 to 50 ring carbon atoms, example of which is a substituted or unsubstituted benzene ring.

In an exemplary embodiment, the ring $A_{93}$ is a substituted or unsubstituted aromatic hydrocarbon ring having 6 to 50 ring carbon atoms, example of which is a substituted or unsubstituted benzene ring.

In an exemplary embodiment, $X_9$ is an oxygen atom or a sulfur atom.

Specific examples of the compound represented by the formula (9) include compounds given below.

[Formula 208]

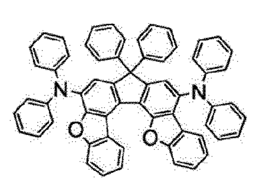
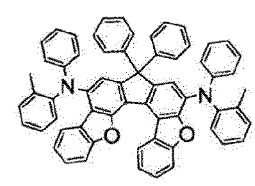
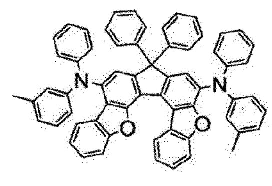
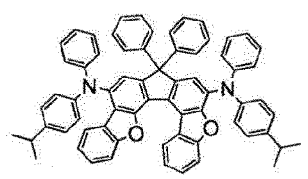
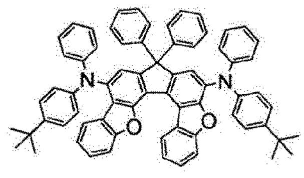
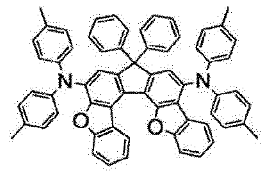
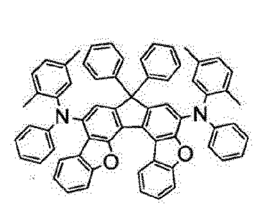
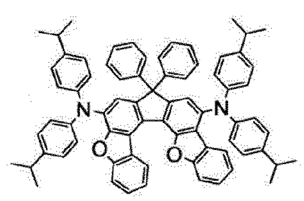
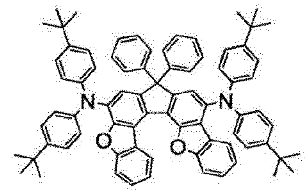
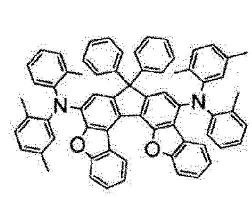
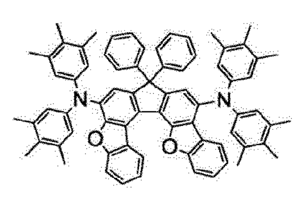
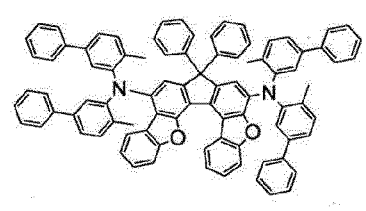
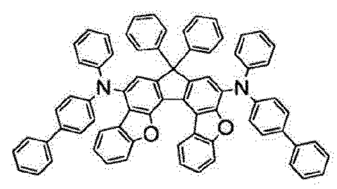
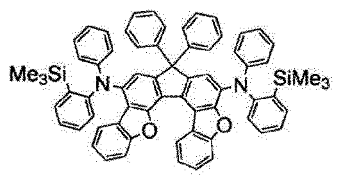
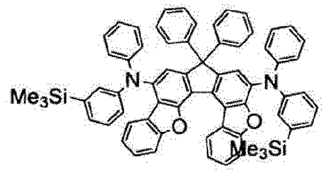
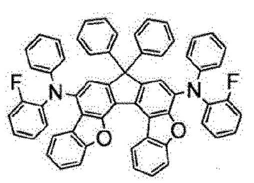
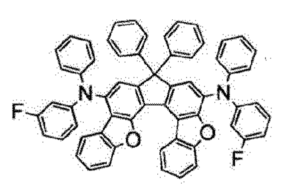
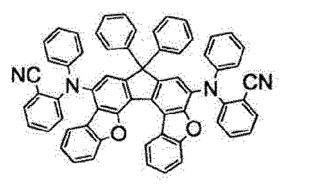
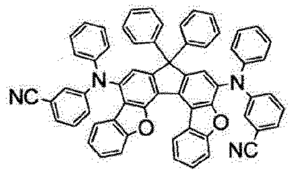
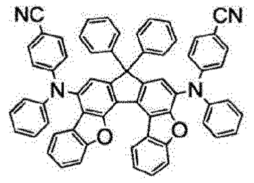
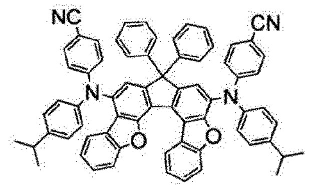

[Formula 209]

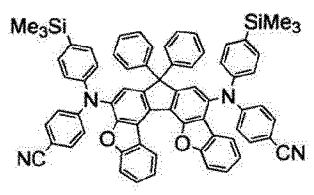
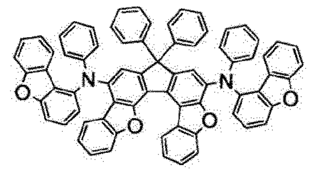
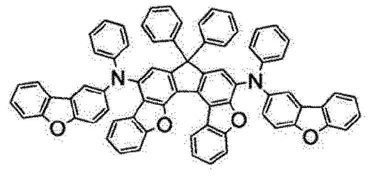
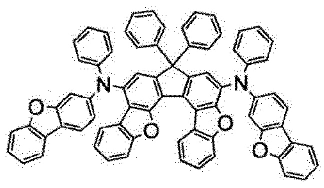
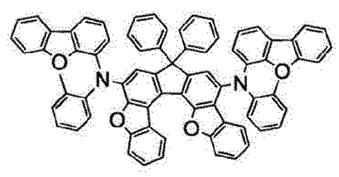
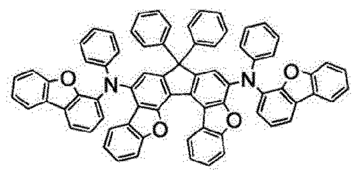
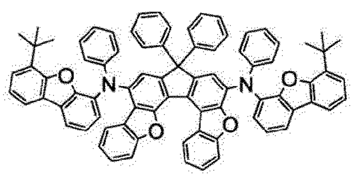
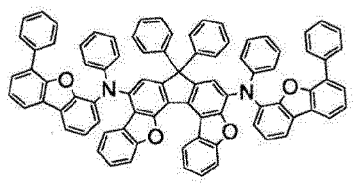
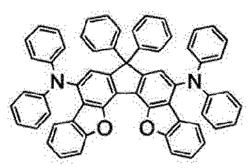
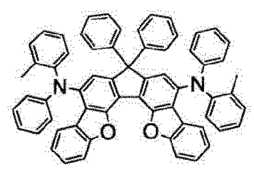
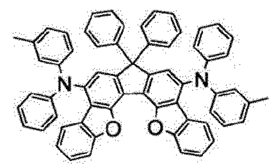
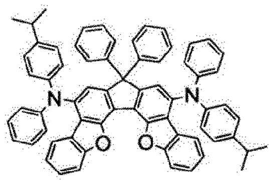
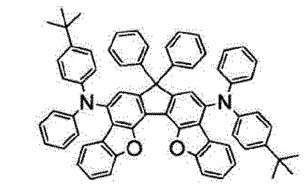
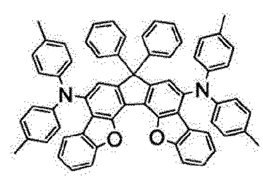
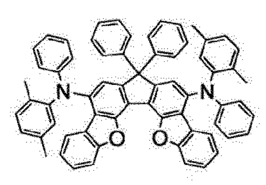
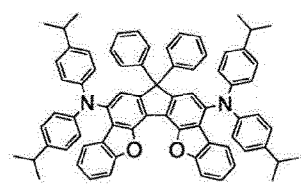
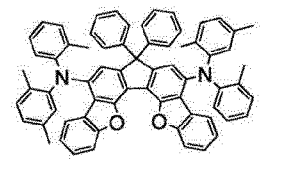
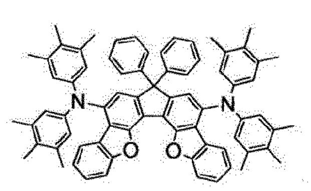
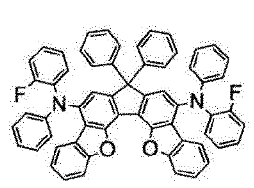
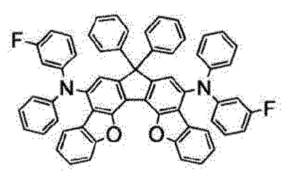

[Formula 210]

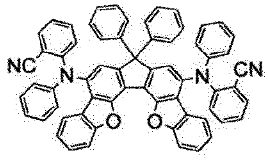 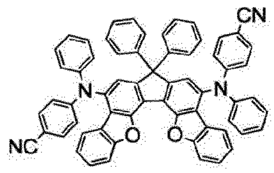 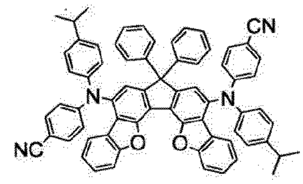

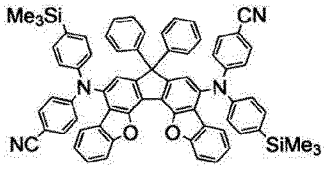 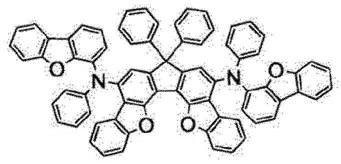

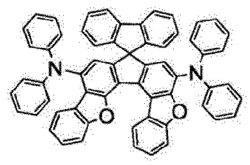 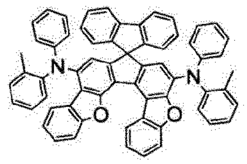 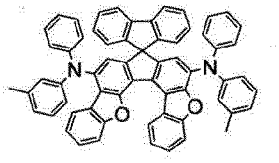

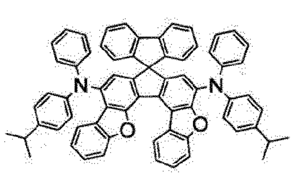 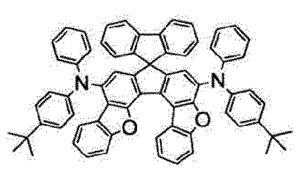 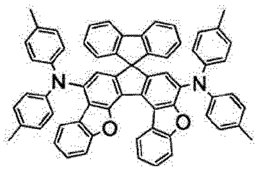

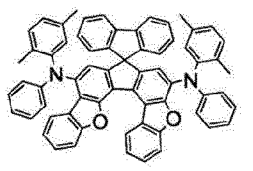 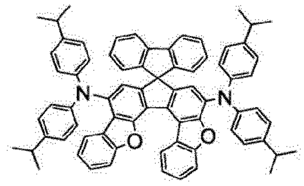 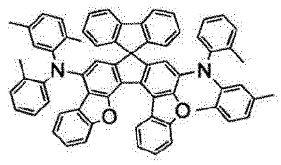

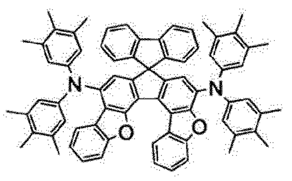 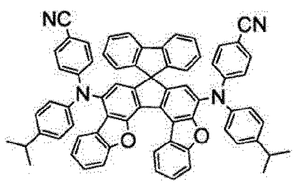 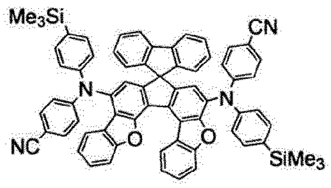

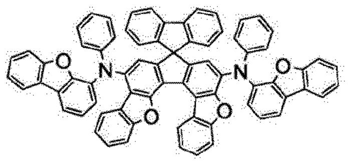

[Formula 211]

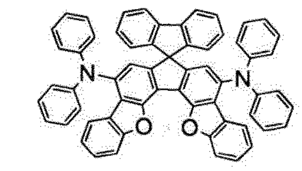

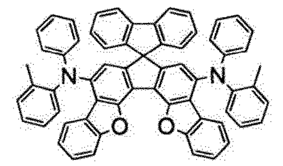

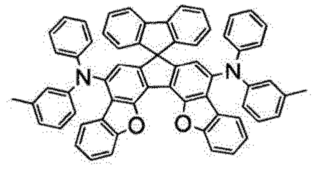

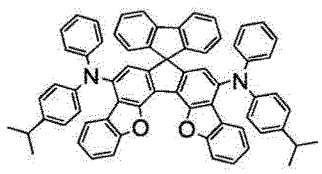

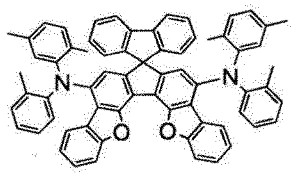

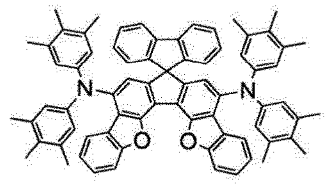

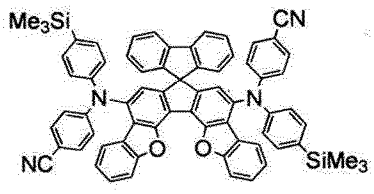

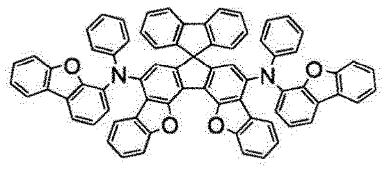

Compound Represented by Formula (10)

The compound represented by the formula (10) will be described below.

[Formula 212]

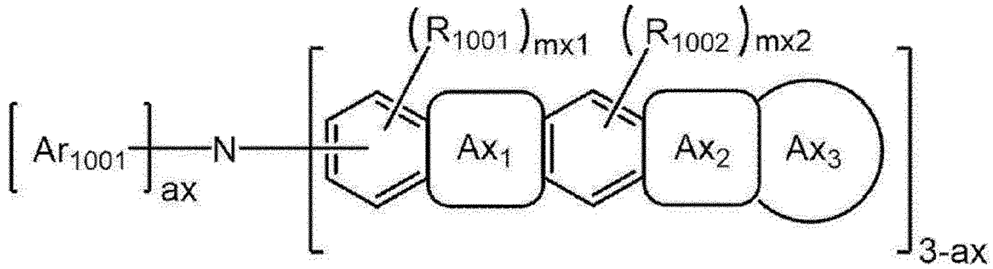

(10)

[Formula 213]

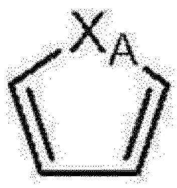

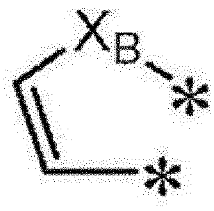

(10a) (10b)

In the formula (10):

a ring $Ax_1$ is a ring represented by the formula (10a) that is fused with adjacent ring(s) at any position(s);
a ring $Ax_2$ is a ring represented by the formula (10b) that is fused with adjacent ring(s) at any position(s);
two * in the formula (10b) are bonded to a ring Axs at any position(s);

$X_A$ and $X_B$ are each independently $C(R_{1003})(R_{1004})$, $Si(R_{1005})(R_{1006})$, an oxygen atom or a sulfur atom;
the ring Axs is a substituted or unsubstituted aromatic hydrocarbon ring having 6 to 50 ring carbon atoms, or a substituted or unsubstituted heterocycle having 5 to 50 ring atoms;
$Ar_{1001}$ is a substituted or unsubstituted aryl group having 6 to 50 ring carbon atoms, or a substituted or unsubstituted heterocyclic group having 5 to 50 ring atoms;
$R_{1001}$ to $R_{1006}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;
mx1 is 3, and mx2 is 2;
a plurality of $R_{1001}$ are mutually the same or different;
a plurality of $R_{1002}$ are mutually the same or different;
ax is 0, 1, or 2;
when ax is 0 or 1, the structures enclosed by brackets indicated by "3-ax" are mutually the same or different; and
when ax is 2, a plurality of $Ar_{1001}$ are mutually the same or different.

In an exemplary embodiment, $Ar_{1001}$ is a substituted or unsubstituted aryl group having 6 to 50 ring carbon atoms.

In an exemplary embodiment, the ring Axs is a substituted or unsubstituted aromatic hydrocarbon ring having 6 to 50 ring carbon atoms, example of which is a substituted or unsubstituted benzene ring, a substituted or unsubstituted naphthalene ring, or a substituted or unsubstituted anthracene ring.

In an exemplary embodiment, $R_{1003}$ and $R_{1004}$ are each independently a substituted or unsubstituted alkyl group having 1 to 50 carbon atoms.

In an exemplary embodiment, ax is 1.

Specific examples of the compound represented by the formula (10) include compounds given below.

[Formula 214]

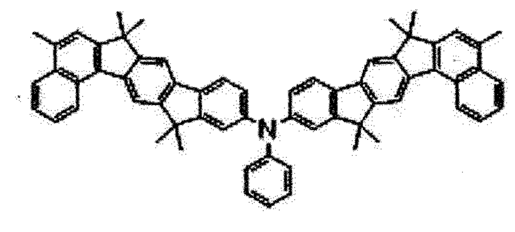

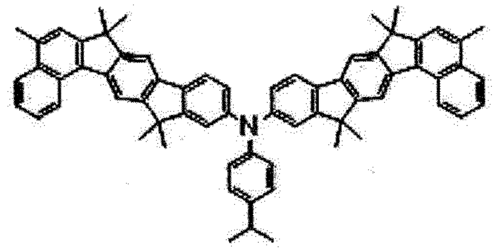

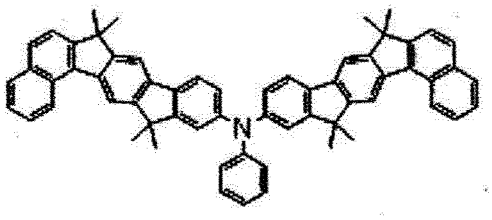

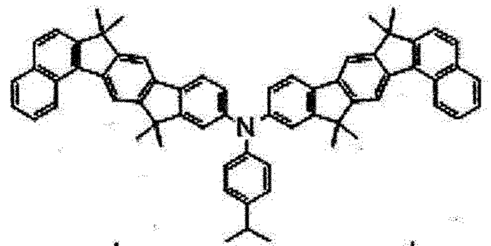

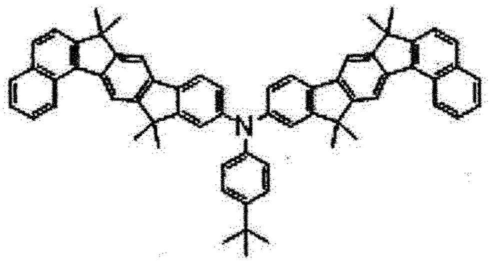

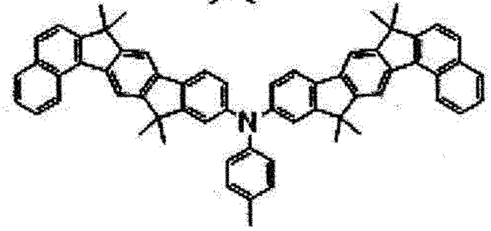

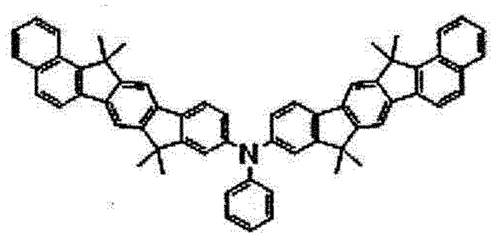

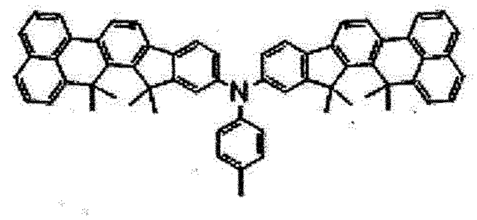

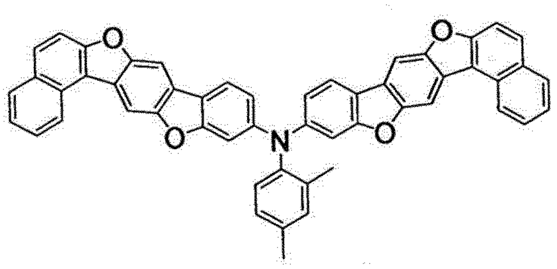

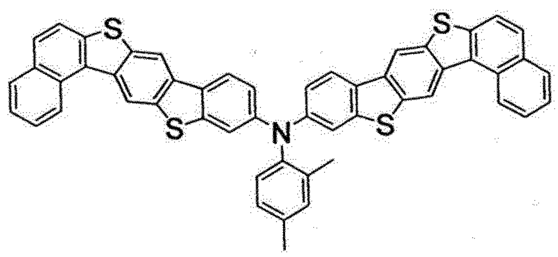

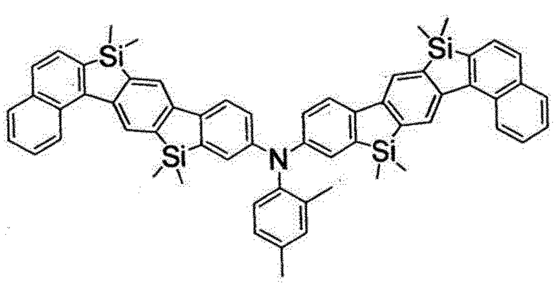

In an exemplary embodiment, the emitting layer 5 contains, as the luminescent compound, at least one compound selected from the group consisting of a compound represented by the formula (4), a compound represented by the formula (5), a compound represented by the formula (6), and a compound represented by a formula (63a) below.

[Formula 215]

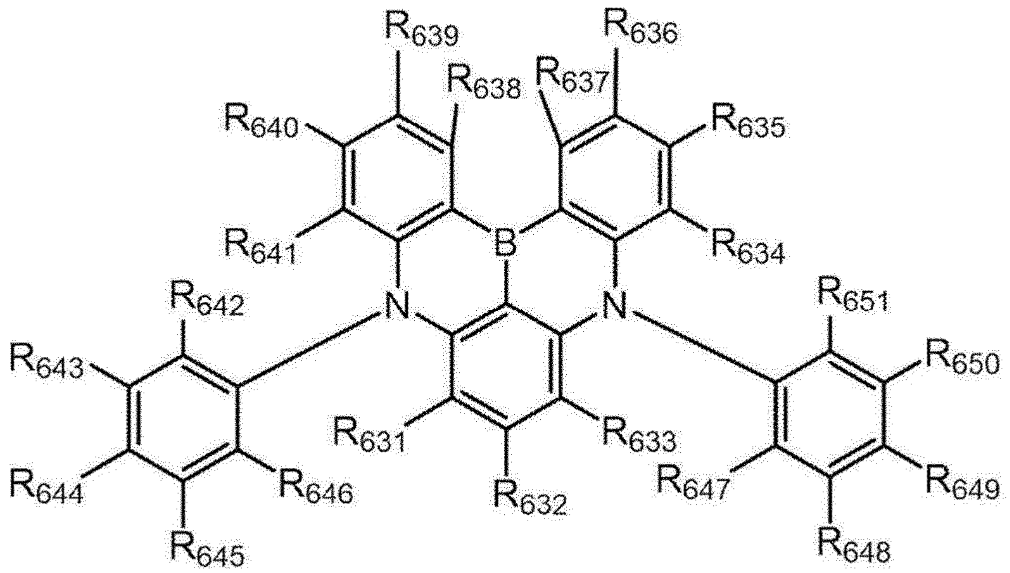

(63a)

In the formula (63a):

$R_{631}$ is bonded with $R_{646}$ to form a substituted or unsubstituted heterocycle, or not bonded therewith to form no substituted or unsubstituted heterocycle;
$R_{633}$ is bonded with $R_{647}$ to form a substituted or unsubstituted heterocycle, or not bonded therewith to form no substituted or unsubstituted heterocycle;
$R_{634}$ is bonded with $R_{651}$ to form a substituted or unsubstituted heterocycle, or not bonded therewith to form no substituted or unsubstituted heterocycle;
$R_{641}$ is bonded with $R_{642}$ to form a substituted or unsubstituted heterocycle, or not bonded therewith to form no substituted or unsubstituted heterocycle;
at least one combination of adjacent two or more of $R_{631}$ to $R_{651}$ are mutually bonded to form a substituted or unsubstituted monocyclic ring, mutually bonded to form a substituted or unsubstituted fused ring, or not mutually bonded;
$R_{631}$ to $R_{651}$ not forming the substituted or unsubstituted heterocycle, not forming the monocyclic ring, and not forming the fused ring are each independently a hydrogen atom, a halogen atom, a cyano group, a nitro group, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aryl group having 6 to 50 ring carbon atoms, or a substituted or unsubstituted heterocyclic group having 5 to 50 ring atoms; and
at least one of $R_{631}$ to $R_{651}$ not forming the substituted or unsubstituted heterocycle, not forming the monocyclic ring, and not forming the fused ring is a halogen atom, a cyano group, a nitro group, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

In an exemplary embodiment, the compound represented by the formula (4) is a compound represented by the formula (41-3), the formula (41-4), or the formula (41-5), the ring A1 in the formula (41-5) being a substituted or unsubstituted fused aromatic hydrocarbon ring having 10 to 50 ring carbon atoms or a substituted or unsubstituted fused heterocycle having 8 to 50 ring atoms.

In an exemplary embodiment, the substituted or unsubstituted fused aromatic hydrocarbon ring having 10 to 50 ring carbon atoms in the formulae (41-3), (41-4) and (41-5) is a substituted or unsubstituted naphthalene ring, a substituted or unsubstituted anthracene ring, or a substituted or unsubstituted fluorene ring; and

the substituted or unsubstituted fused heterocycle having 8 to 50 ring atoms is a substituted or unsubstituted dibenzofuran ring, a substituted or unsubstituted carbazole ring, or a substituted or unsubstituted dibenzothiophene ring.

In an exemplary embodiment, the substituted or unsubstituted fused aromatic hydrocarbon ring having 10 to 50 ring carbon atoms in the formula (41-3), (41-4) or (41-5) is a substituted or unsubstituted naphthalene ring, or a substituted or unsubstituted fluorene ring; and

the substituted or unsubstituted fused heterocycle having 8 to 50 ring atoms is a substituted or unsubstituted dibenzofuran ring, a substituted or unsubstituted carbazole ring, or a substituted or unsubstituted dibenzothiophene ring.

In an exemplary embodiment, the compound represented by the formula (4) is selected from the group consisting of a compound represented by a formula (461), a compound represented by a formula (462), a compound represented by a formula (463), a compound represented by a formula (464), a compound represented by a formula (465), a compound represented by a formula (466), and a compound represented by a formula (467) below.

[Formula 216]

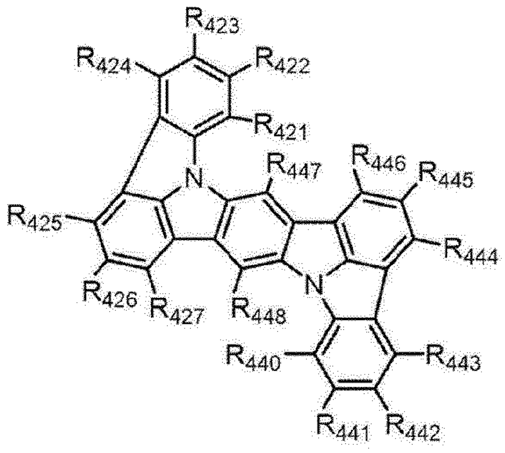

(461)

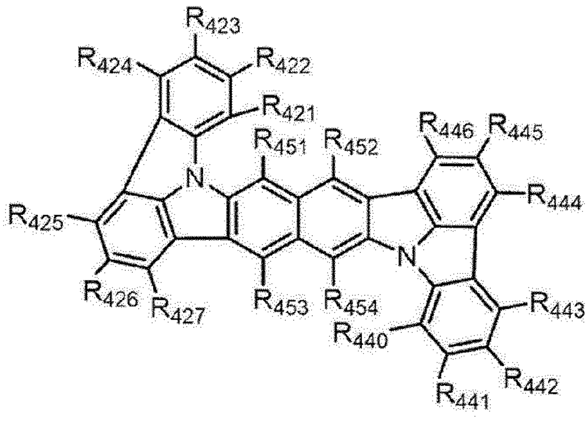

(462)

[Formula 217]

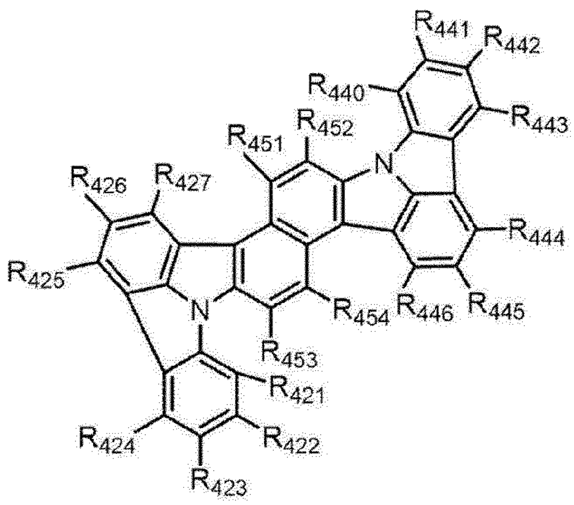

(463)

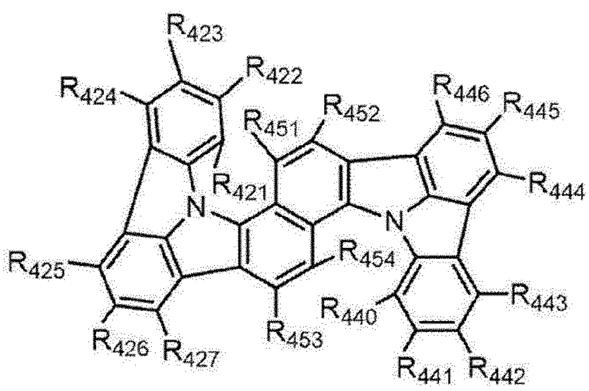

(464)

[Formula 218]

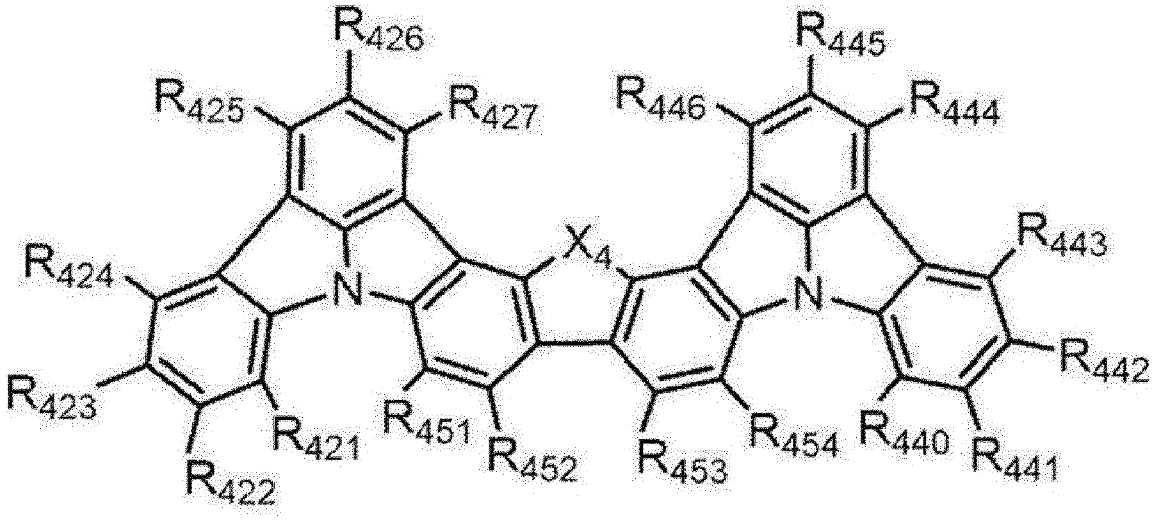

(465)

[Formula 219]

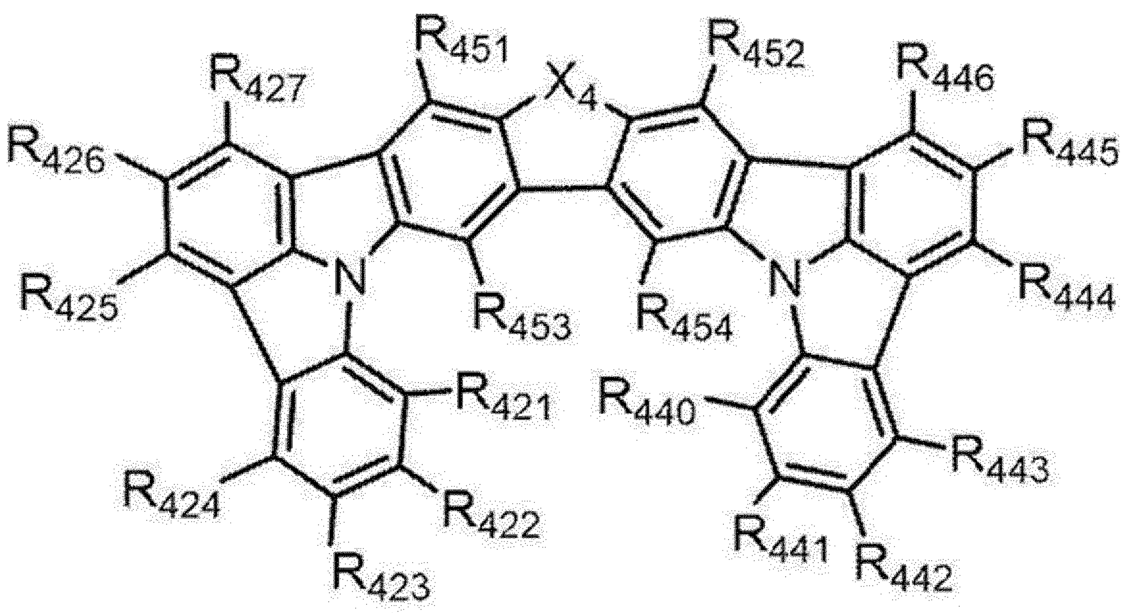

(466)

[Formula 220]

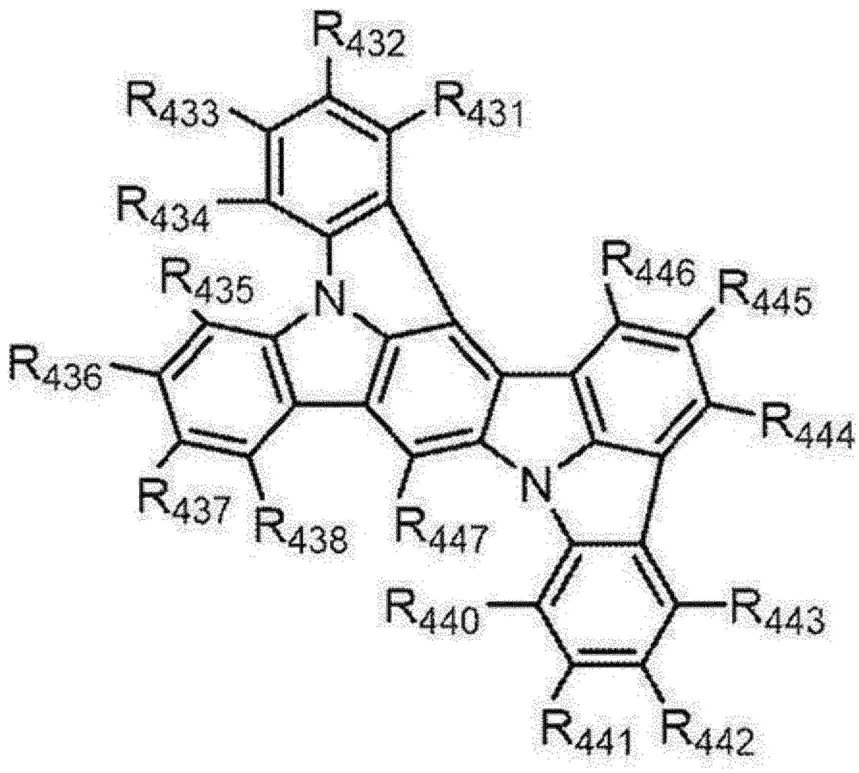

(467)

In the formulae (461) to (467):

at least one combination of adjacent two or more of $R_{421}$ to $R_{427}$, $R_{431}$ to $R_{436}$, $R_{440}$ to $R_{448}$, and $R_{451}$ to $R_{454}$ are mutually bonded to form a substituted or unsubstituted monocyclic ring, mutually bonded to form a substituted or unsubstituted fused ring, or not mutually bonded;

$R_{421}$ to $R_{427}$, $R_{431}$ to $R_{436}$, $R_{440}$ to $R_{448}$, and $R_{451}$ to $R_{454}$ forming neither the monocyclic ring nor the fused ring, $R_{437}$, and $R_{438}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

$X_4$ is an oxygen atom, $NR_{801}$, or $C(R_{802})(R_{803})$;

$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms; preferably, a substituted or unsubstituted alkyl group having 1 to 50 carbon atoms or a substituted or unsubstituted aryl group having 6 to 50 ring carbon atoms;

when a plurality of $R_{801}$ are present, the plurality of $R_{801}$ are mutually the same or different;

when a plurality of $R_{802}$ are present, the plurality of $R_{802}$ are mutually the same or different; and

when a plurality of $R_{803}$ are present, the plurality of $R_{803}$ are mutually the same or different.

In an exemplary embodiment, $R_{421}$ to $R_{427}$ and $R_{440}$ to $R_{448}$ are each independently a hydrogen atom, a substituted or unsubstituted aryl group having 6 to 50 ring carbon atoms, or a substituted or unsubstituted heterocyclic group having 5 to 50 ring atoms.

In an exemplary embodiment, $R_{421}$ to $R_{427}$ and $R_{440}$ to $R_{447}$ are each independently selected from the group

consisting of a hydrogen atom, a substituted or unsubstituted aryl group having 6 to 18 ring carbon atoms, and a substituted or unsubstituted heterocyclic group having 5 to 18 ring atoms.

In an exemplary embodiment, the compound represented by the formula (41-3) is a compound represented by a formula (41-3-1) below.

[Formula 221]

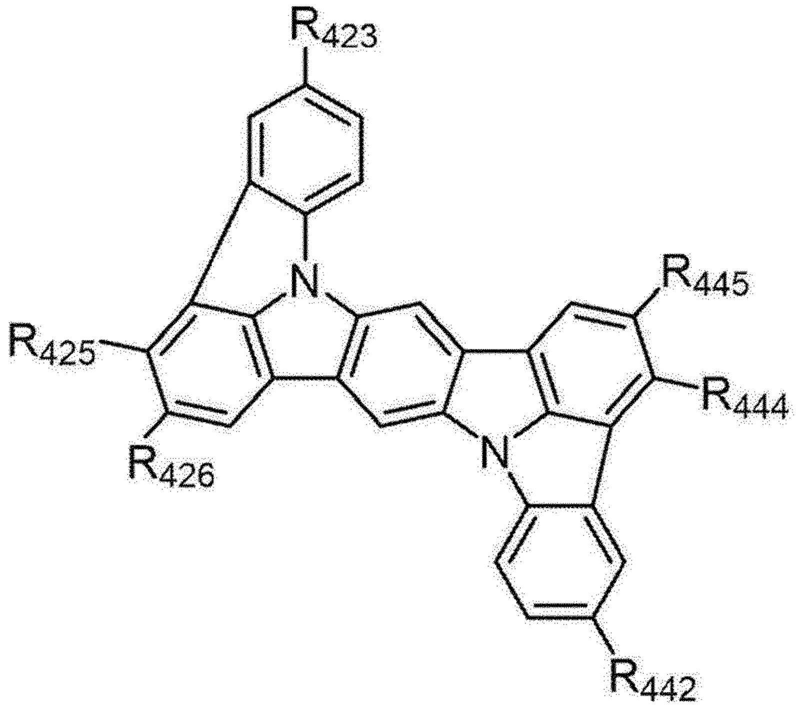

(41-3-1)

In the formula (41-3-1), $R_{423}$, $R_{425}$, $R_{426}$, $R_{442}$, $R_{444}$ and $R_{445}$ each independently represent the same as $R_{423}$, $R_{425}$, $R_{426}$, $R_{442}$, $R_{444}$ and $R_{445}$ in the formula (41-3).

In an exemplary embodiment, the compound represented by the formula (41-3) is a compound represented by a formula (41-3-2) below.

[Formula 222]

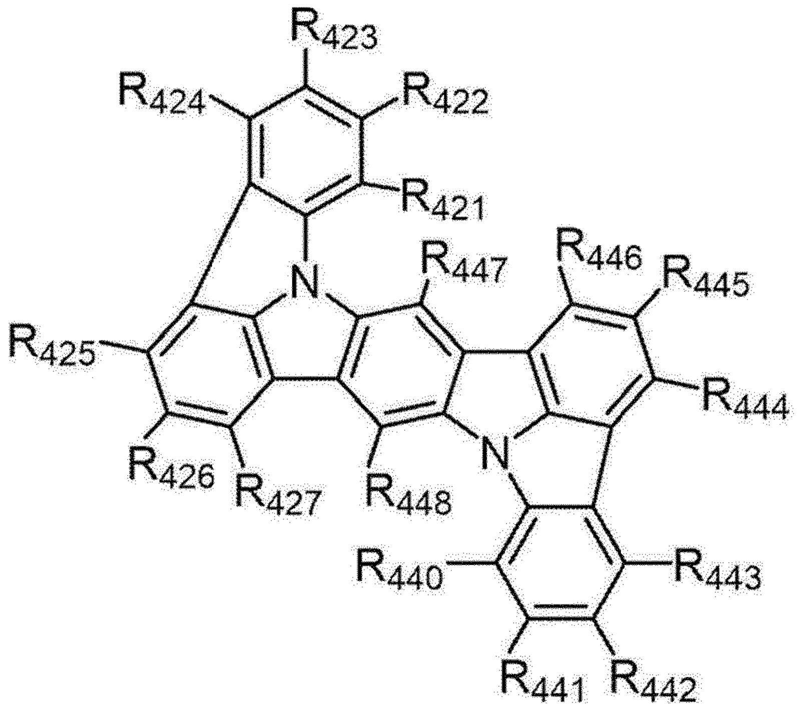

(41-3-2)

In the formula (41-3-2), $R_{421}$ to $R_{427}$ and $R_{440}$ to $R_{448}$ each independently represent the same as $R_{421}$ to $R_{427}$ and $R_{440}$ to $R_{448}$ in the formula (41-3); and

at least one of $R_{421}$ to $R_{427}$ or $R_{440}$ to $R_{446}$ is a group represented by - $N(R_{906})(R_{907})$.

In an exemplary embodiment, two of $R_{421}$ to $R_{427}$ and $R_{440}$ to $R_{446}$ in the formula (41-3-2) are each a group represented by $-N(R_{906})(R_{907})$.

In an exemplary embodiment, the compound represented by the formula (41-3-2) is a compound represented by a formula (41-3-3) below.

[Formula 223]

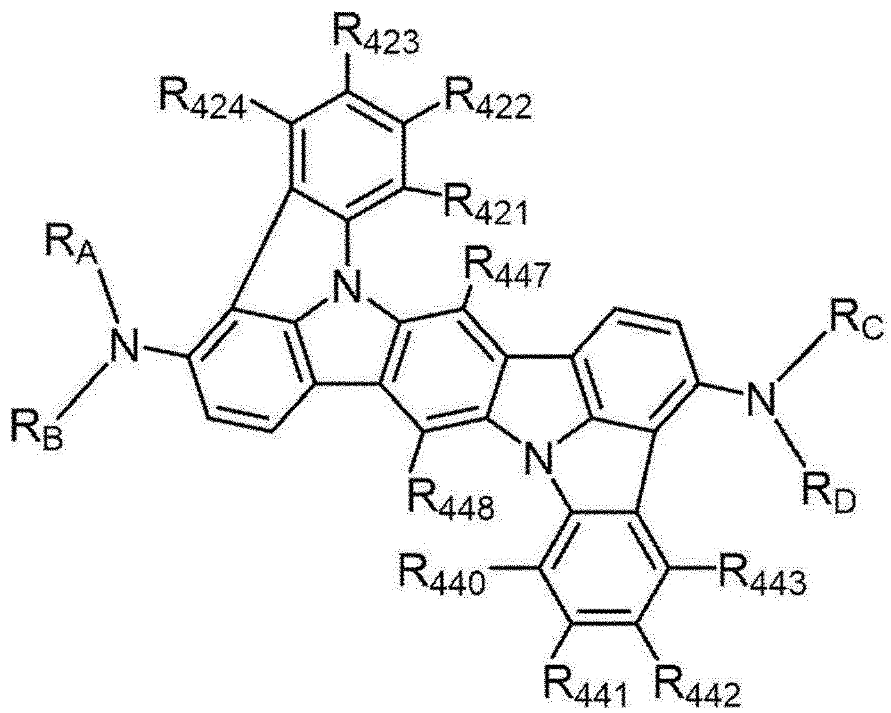

(41-3-3)

In the formula (41-3-3), $R_{421}$ to $R_{424}$, $R_{440}$ to $R_{443}$, $R_{447}$ and $R_{448}$ each independently represent the same as $R_{421}$ to $R_{424}$, $R_{440}$ to $R_{443}$, $R_{447}$ and $R_{448}$ in the formula (41-3); and

$R_A$, $R_B$, Rc, and $R_D$ are each independently a substituted or unsubstituted aryl group having 6 to 18 ring carbon atoms, or a substituted or unsubstituted heterocyclic group having 5 to 18 ring atoms.

In an exemplary embodiment, the compound represented by the formula (41-3-3) is a compound represented by a formula (41-3-4) below.

[Formula 224]

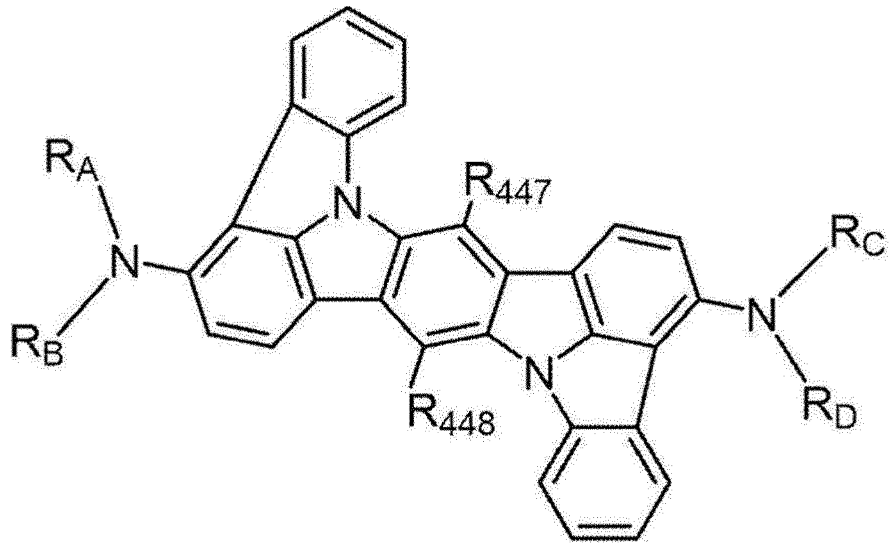

(41-3-4)

In the formula (41-3-4), $R_{447}$, $R_{448}$, $R_A$, $R_B$, Rc and $R_D$ each independently represent the same as $R_{447}$, $R_{448}$, $R_A$, $R_B$, Rc and $R_D$ in the formula (41-3-3).

In an exemplary embodiment, $R_A$, $R_B$, $R_C$, and $R_D$ are each independently a substituted or unsubstituted aryl group having 6 to 18 ring carbon atoms.

In an exemplary embodiment, $R_A$, $R_B$, $R_C$, and $R_D$ are each independently a substituted or unsubstituted phenyl group.

In an exemplary embodiment, $R_{447}$ and $R_{448}$ are each a hydrogen atom.

In the organic EL device 1A, the luminescent compound contained in the emitting layer 5 is preferably a compound that emits light having a maximum peak wavelength of 500 nm or less, more preferably a compound that emits light having a maximum peak wavelength in a range from 430 nm to 480 nm.

In the organic EL device 1A, the luminescent compound contained in the emitting layer 5 is preferably a compound that emits fluorescence having a maximum peak wavelength of 500 nm or less, more preferably a compound that emits fluorescence having a maximum peak wavelength in a range from 430 nm to 480 nm.

When the emitting layer 5 of the organic EL device 1A contains a compound according to the first exemplary embodiment and a luminescent compound, the compound according to the first exemplary embodiment is preferably a host material (occasionally referred to as a matrix material) and the luminescent compound is preferably a dopant material (occasionally referred to as a guest material, emitter, or luminescent material).

When the emitting layer 5 of the organic EL device 1A contains a compound according to the second exemplary embodiment and a luminescent compound, the compound according to the second exemplary embodiment is preferably a host material (occasionally referred to as a matrix material) and the luminescent compound is preferably a dopant material (occasionally referred to as a guest material, emitter, or luminescent material).

When the emitting layer 5 of the organic EL device 1A contains a compound according to the third exemplary embodiment and a luminescent compound, the compound according to the third exemplary embodiment is preferably a host material (occasionally referred to as a matrix material) and the luminescent compound is preferably a dopant material (occasionally referred to as a guest material, emitter, or luminescent material).

Herein, the "host material" refers to, for instance, a material that accounts for "50 mass% or more of the layer".

That is, for instance, the emitting layer 5 contains 50 mass% or more of a compound represented by the formula (1A) with respect to the total mass of the emitting layer in the organic EL device 1A.

That is, for instance, the emitting layer 5 contains 50 mass% or more of a compound represented by the formula

(1C) with respect to the total mass of the emitting layer in the organic EL device 1A.

Film Thickness of Emitting Layer

A film thickness of the emitting layer 5 is preferably in a range from 5 nm to 50 nm, more preferably in a range from 7 nm to 50 nm, and still more preferably in a range from 10 nm to 50 nm. A film thickness of the emitting layer of 5 nm or more facilitates the formation of the emitting layer and the adjustment of chromaticity. A film thickness of the emitting layer of 50 nm or less easily inhibits an increase in drive voltage.

Content Ratios of Compounds in Emitting Layer

When the emitting layer 5 contains a compound according to the first exemplary embodiment and a luminescent compound, content ratios of the compound according to the first exemplary embodiment and the luminescent compound in the emitting layer 5 preferably fall, for instance, within ranges below.

The content ratio of the compound according to the first exemplary embodiment falls within, preferably, in a range from 80 mass% to 99 mass%, more preferably in a range from 90 mass% to 99 mass%, and still more preferably in a range from 95 mass% to 99 mass%.

The content ratio of the luminescent compound falls within, preferably, in a range from 1 mass% to 10 mass%, more preferably in a range from 1 mass% to 7 mass%, and still more preferably in a range from 1 mass% to 5 mass%.

The upper limit of the total of the content ratios of the compound according to the first exemplary embodiment and the luminescent compound in the emitting layer 5 is 100 mass%.

When the emitting layer 5 contains a compound according to the second exemplary embodiment and a luminescent compound, content ratios of the compound according to the second exemplary embodiment and the luminescent compound in the emitting layer 5 preferably fall, for instance, within ranges below.

The content ratio of the compound according to the second exemplary embodiment falls within, preferably, in a range from 80 mass% to 99 mass%, more preferably in a range from 90 mass% to 99 mass%, and still more preferably in a range from 95 mass% to 99 mass%.

The content ratio of the luminescent compound falls within, preferably, in a range from 1 mass% to 10 mass%, more preferably in a range from 1 mass% to 7 mass%, and still more preferably in a range from 1 mass% to 5 mass%.

The upper limit of the total of the content ratios of the compound according to the second exemplary embodiment and the luminescent compound in the emitting layer 5 is 100 mass%.

When the emitting layer 5 contains a compound according to the third exemplary embodiment and a luminescent compound, content ratios of the compound according to the third exemplary embodiment and the luminescent compound in the emitting layer 5 preferably fall, for instance, within ranges below.

The content ratio of the compound according to the third exemplary embodiment falls within, preferably, in a range from 80 mass% to 99 mass%, more preferably in a range from 90 mass% to 99 mass%, and still more preferably in a range from 95 mass% to 99 mass%.

The content ratio of the luminescent compound falls within, preferably, in a range from 1 mass% to 10 mass%, more preferably in a range from 1 mass% to 7 mass%, and still more preferably in a range from 1 mass% to 5 mass%.

The upper limit of the total of the content ratios of the compound according to the third exemplary embodiment and the luminescent compound in the emitting layer 5 is 100 mass%.

In the exemplary embodiment, the emitting layer 5 may further contain any other material than a compound according to the first exemplary embodiment and a luminescent compound.

The emitting layer 5 may contain a single type of compound according to the first exemplary embodiment or two or more types of compounds according to the first exemplary embodiment. The emitting layer 5 may contain a single type of luminescent compound or two or more types of luminescent compounds.

In the exemplary embodiment, the emitting layer 5 may further contain any other material than a compound according to the second exemplary embodiment and a luminescent compound.

The emitting layer 5 may contain a single type of compound according to the second exemplary embodiment or two or more types of compounds according to the second exemplary embodiment. The emitting layer 5 may contain a single type of luminescent compound or two or more types of luminescent compounds.

In the exemplary embodiment, the emitting layer 5 may further contain any other material than a compound according to the third exemplary embodiment and a luminescent compound.

The emitting layer 5 may contain a single type of compound according to the third exemplary embodiment or two or more types of compounds according to the third exemplary embodiment. The emitting layer 5 may contain a single type of luminescent compound or two or more types of luminescent compounds.

Fig. 2 schematically depicts another exemplary arrangement of the organic EL device according to the exemplary embodiment.

An organic EL device 1B depicted in Fig. 2 is different from the organic EL device 1A in that an organic layer 10B includes a first emitting region 5B, and the rest of components and arrangements of the organic EL device 1B are the same as those of the organic EL device 1A. The first emitting region 5B includes a first emitting layer 51 and a second emitting layer 52 in this order from a side close to the anode 3.

The first emitting layer 51 contains a first compound and the second emitting layer 52 contains a second compound. The first compound and the second compound are mutually different compounds.

First Compound

In an exemplary arrangement of the organic EL device 1B, the first compound is a compound according to the first exemplary embodiment.

In an exemplary arrangement of the organic EL device according to the exemplary embodiment, the first compound is optionally a compound represented by the formula (1C-A).

In an exemplary arrangement of the organic EL device 1B, the first compound is a compound according to the second exemplary embodiment.

In an exemplary arrangement of the organic EL device according to the exemplary embodiment, the first compound is optionally a compound represented by the formula (1C-B).

In an exemplary arrangement of the organic EL device 1B, the first compound is a compound according to the third exemplary embodiment.

In an exemplary arrangement of the organic EL device according to the exemplary embodiment, the first compound is optionally a compound represented by the formula (1C-C).

In an exemplary arrangement of the organic EL device 1B, the first compound is a compound represented by the formula (1C).

Second Compound

In the organic EL device 1B, the second compound is preferably a compound represented by a formula (2) below.

[Formula 225]

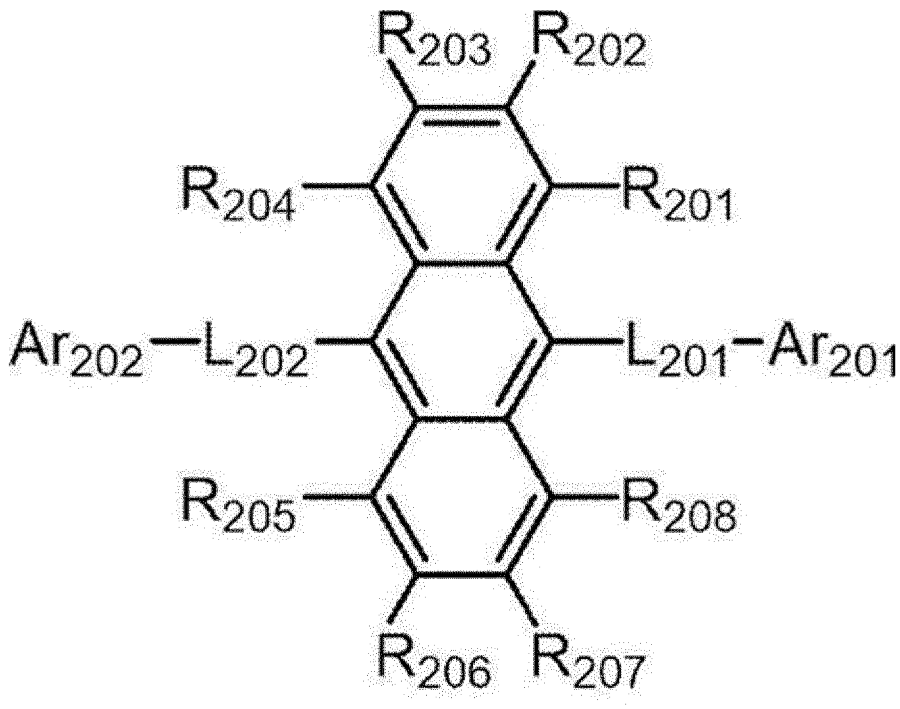

(2)

In the formula (2):

$R_{201}$ to $R_{208}$ are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a substituted or unsubstituted aralkyl group having 7 to 50 carbon atoms, a group represented by -C(=O)$R_{801}$, a group represented by -COOR$_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;

$L_{201}$ and $L_{202}$ are each independently a single bond, a substituted or unsubstituted arylene group having 6 to 50 ring carbon atoms, or a substituted or unsubstituted divalent heterocyclic group having 5 to 50 ring atoms; and

$Ar_{201}$ and $Ar_{202}$ are each independently a substituted or unsubstituted aryl group having 6 to 50 ring carbon atoms, or a substituted or unsubstituted heterocyclic group having 5 to 50 ring atoms.

In the second compound according to the exemplary embodiment, $R_{901}$, $R_{902}$, $R_{903}$, $R_{904}$, $R_{905}$, $R_{906}$, $R_{907}$, $R_{801}$ and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

In the organic EL device according to the exemplary embodiment, it is preferable that $R_{201}$ to $R_{208}$ are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by - S-($R_{905}$), a group represented by -N($R_{906}$)($R_{907}$), a substituted or unsubstituted aralkyl group having 7 to 50 carbon atoms, a group represented by -C(=O)$R_{801}$, a group represented by -COOR$_{802}$, a halogen atom, a cyano group, or a nitro group; $L_{201}$ and $L_{202}$ are each independently a single bond, a substituted or unsubstituted arylene group having 6 to 50 ring carbon atoms, or a substituted or unsubstituted divalent heterocyclic group having 5 to 50 ring atoms; and
$Ar_{201}$ and $Ar_{202}$ are each independently a substituted or unsubstituted aryl group having 6 to 50 ring carbon atoms, or a substituted or unsubstituted heterocyclic group having 5 to 50 ring atoms.

In the organic EL device according to the exemplary embodiment, it is preferable that $L_{201}$ and $L_{202}$ are each independently a single bond, or a substituted or unsubstituted arylene group having 6 to 50 ring carbon atoms; and $Ar_{201}$ and $Ar_{202}$ are each independently a substituted or unsubstituted aryl group having 6 to 50 ring carbon atoms.

In the organic EL device according to the exemplary embodiment, it is preferable that $Ar_{201}$ and $Ar_{202}$ are each independently a phenyl group, naphthyl group, phenanthryl group, biphenyl group, terphenyl group, diphenylfluorenyl group, dimethylfluorenyl group, benzodiphenylfluorenyl group, benzodimethylfluorenyl group, dibenzofuranyl group, dibenzothienyl group, naphthobenzofuranyl group, or naphthobenzothienyl group.

In the organic EL device according to the exemplary embodiment, the second compound represented by the formula (2) is preferably a compound represented by a formula (201), a formula (202), a formula (203), a formula (204), a formula (205), a formula (206), a formula (207), a formula (208), or a formula (209) below.

[Formula 226]

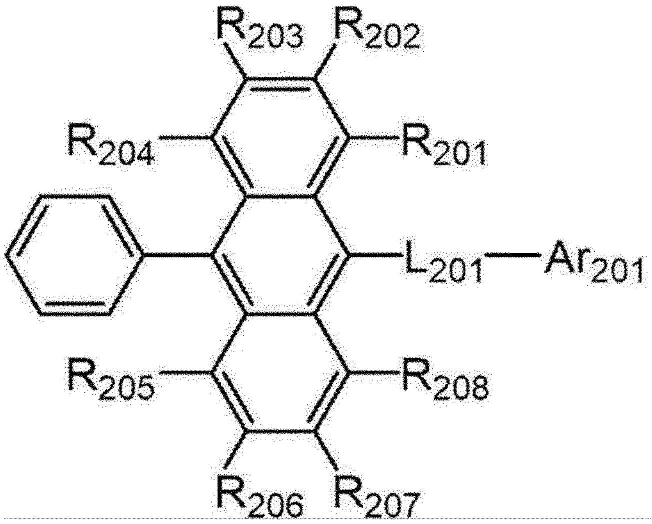

(201)

[Formula 227]

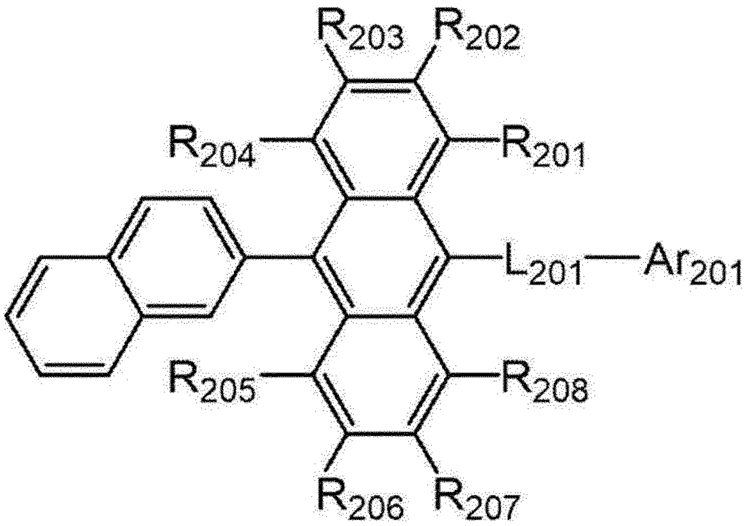

(202)

[Formula 228]

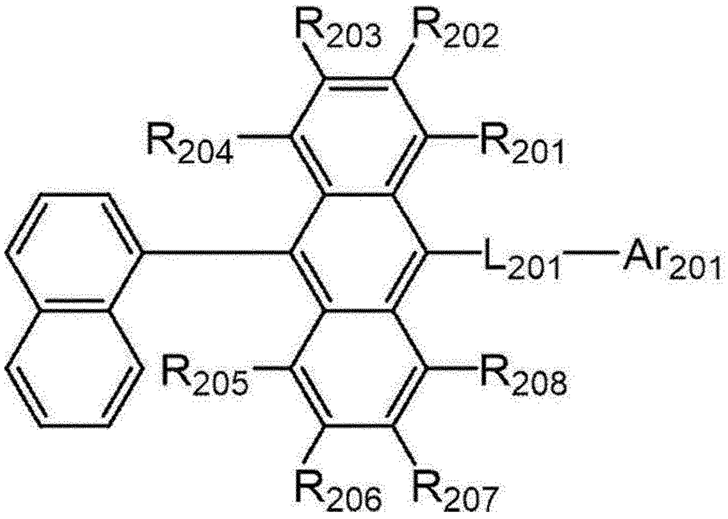

(203)

[Formula 229]

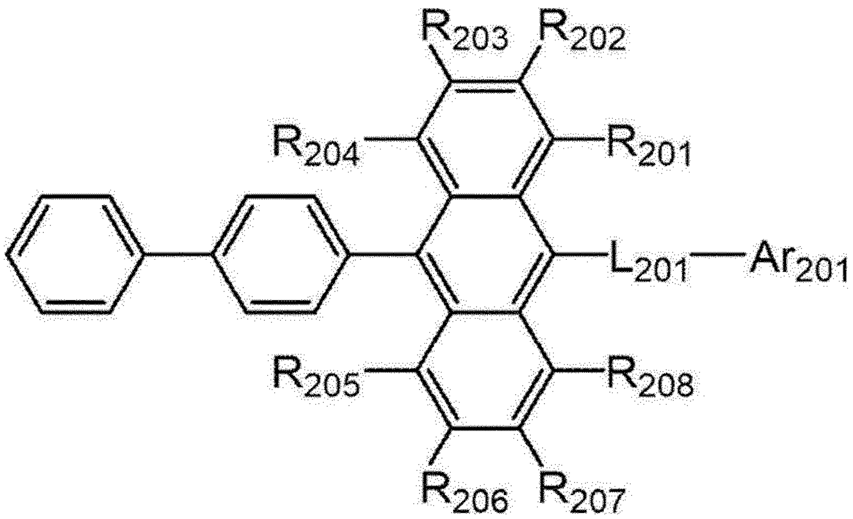

(204)

[Formula 230]

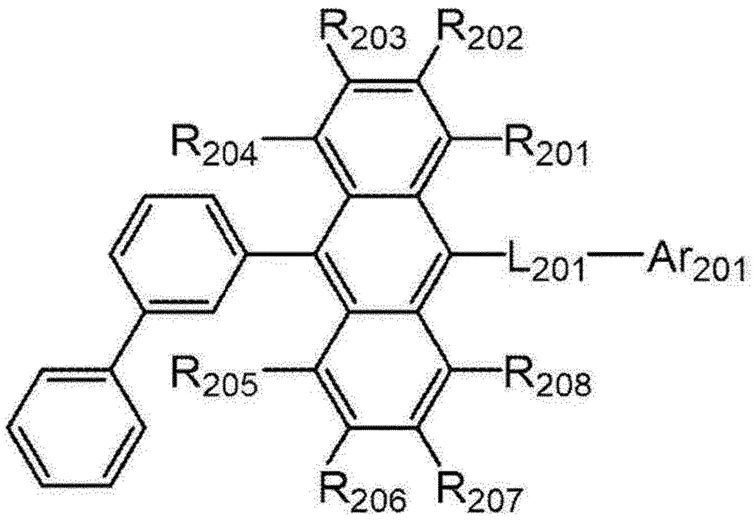

(205)

[Formula 231]

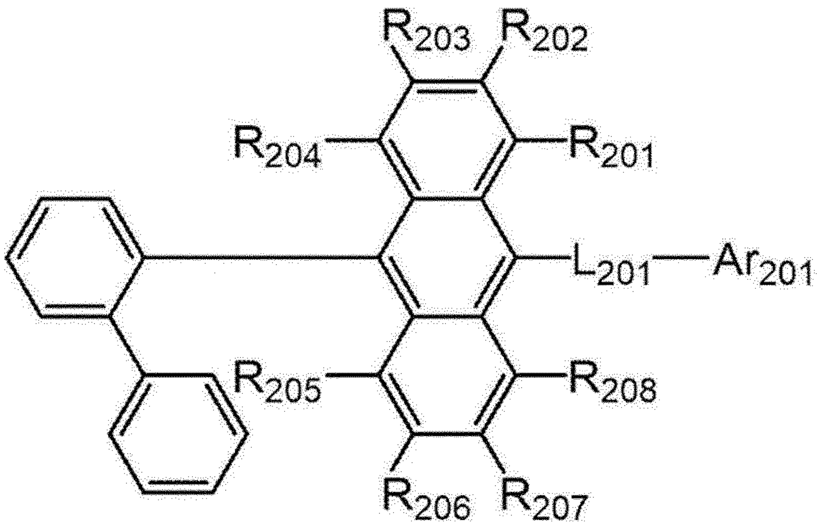

(206)

[Formula 232]

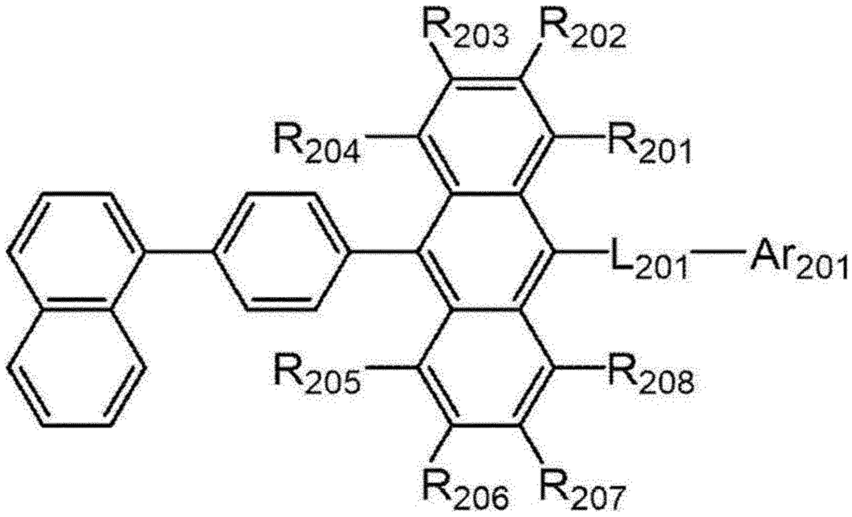

(207)

[Formula 233]

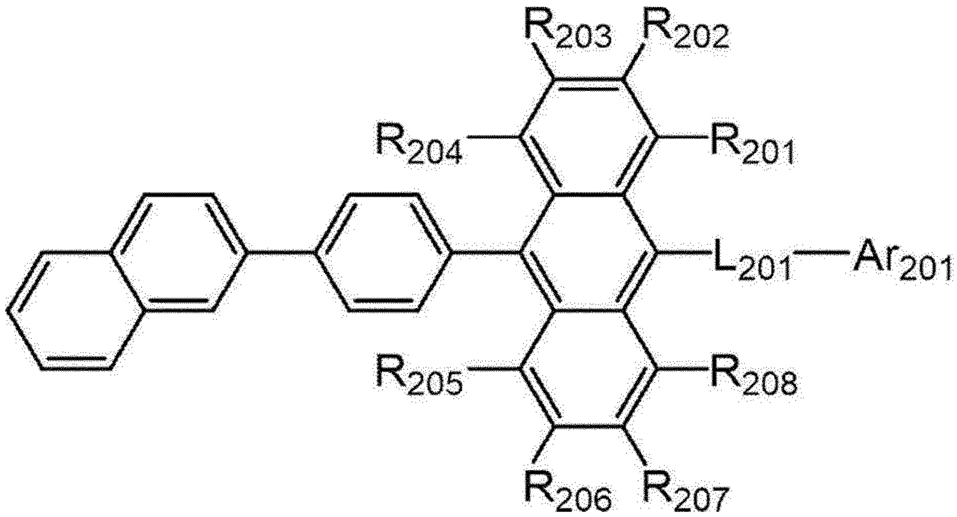

(208)

[Formula 234]

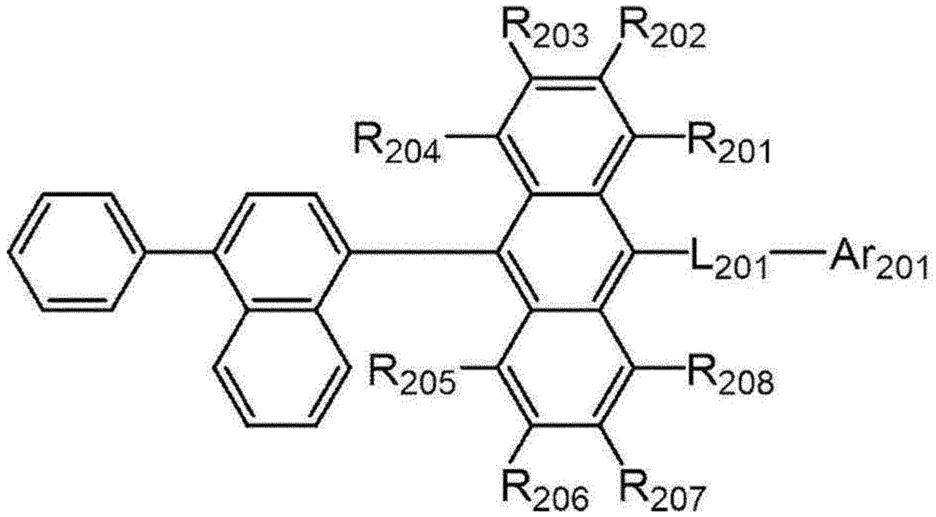

(209)

In the formulae (201) to (209):

$L_{201}$ and $Ar_{201}$ respectively represent the same as $L_{201}$ and $Ar_{201}$ in the formula (2); and
$R_{201}$ to $R_{208}$ each independently represent the same as $R_{201}$ to $R_{208}$ in the formula (2).

The second compound represented by the formula (2) is also preferably a compound represented by a formula (221), a formula (222), a formula (223), a formula (224), a formula (225), a formula (226), a formula (227), a formula (228), or a formula (229) below.

[Formula 235]

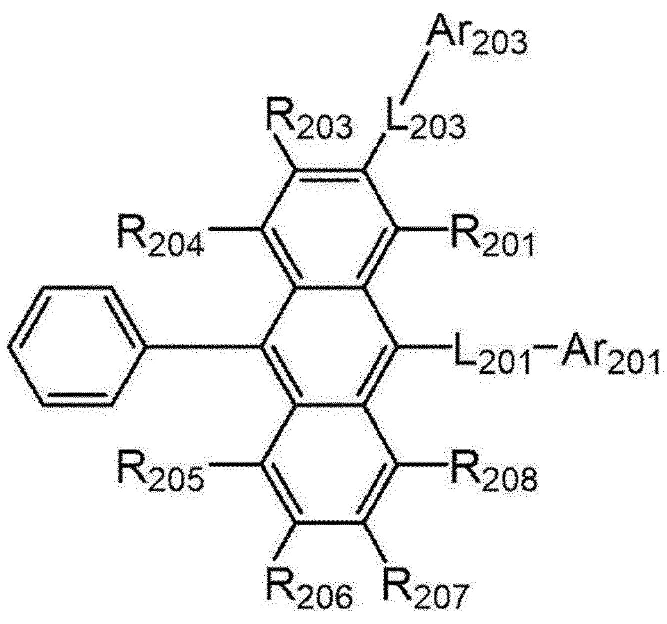

(221)

[Formula 236]

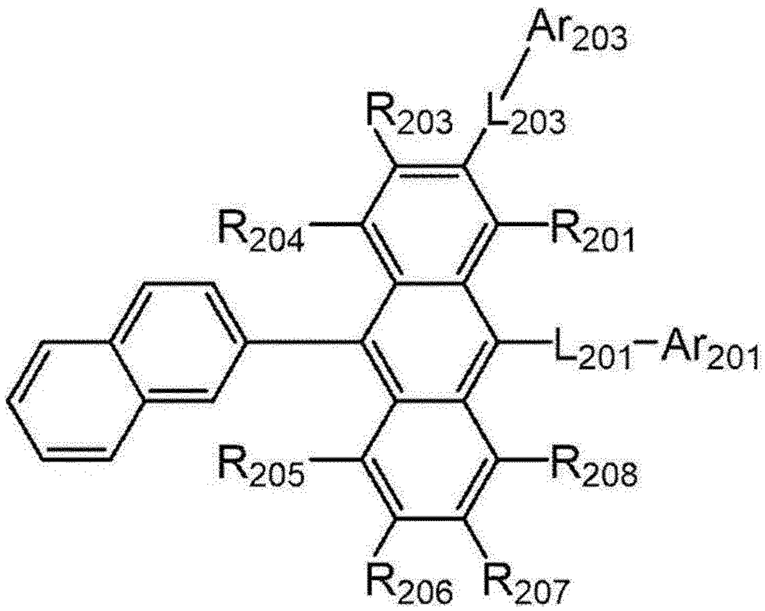

(222)

[Formula 237]

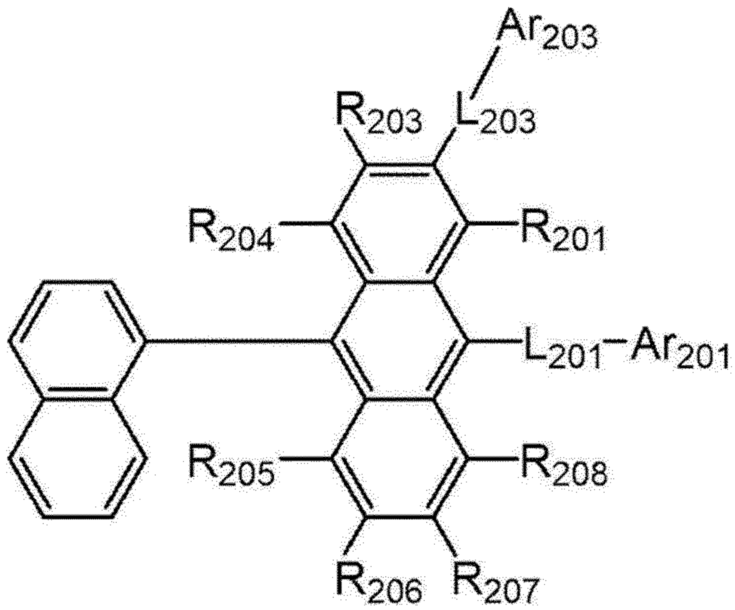

(223)

[Formula 238]

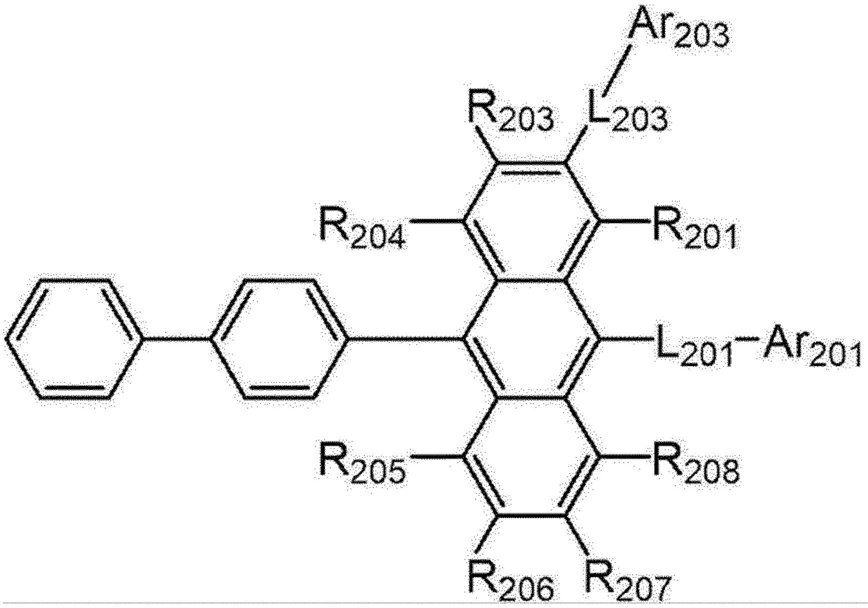

(224)

[Formula 239]

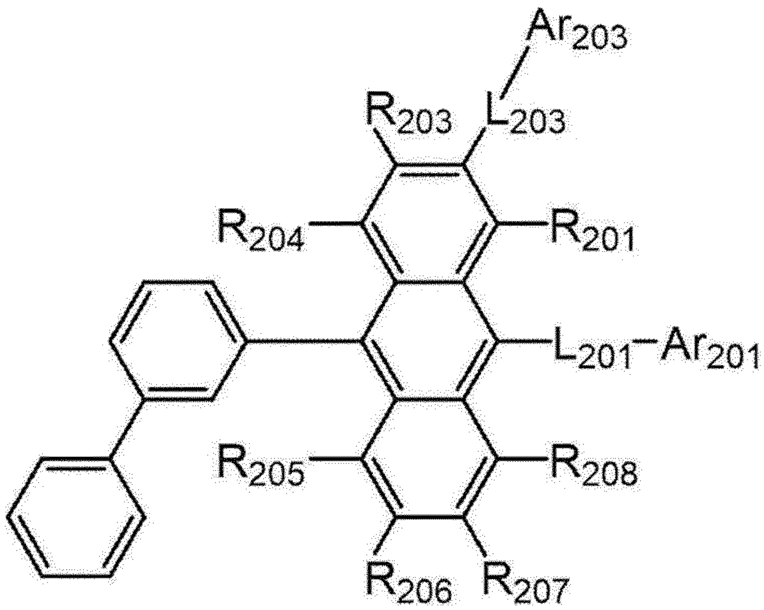

(225)

[Formula 240]

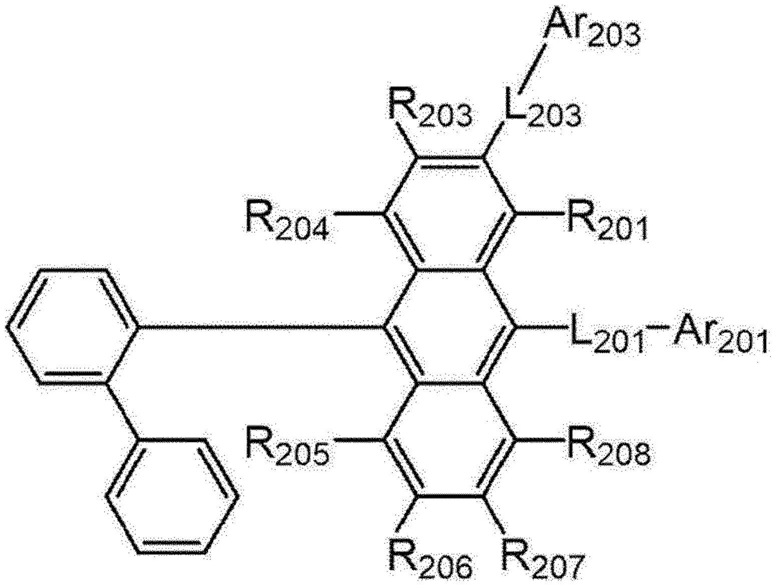

(226)

[Formula 241]

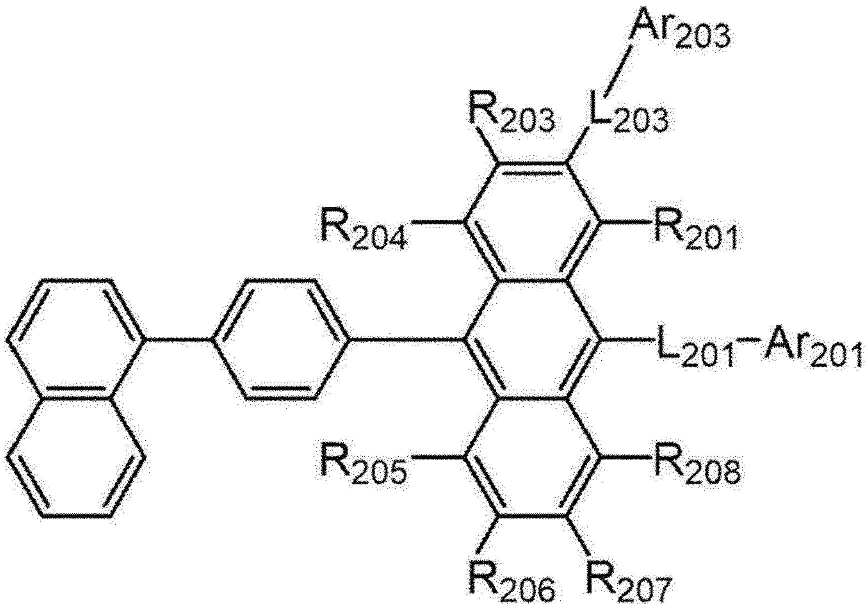

(227)

[Formula 242]

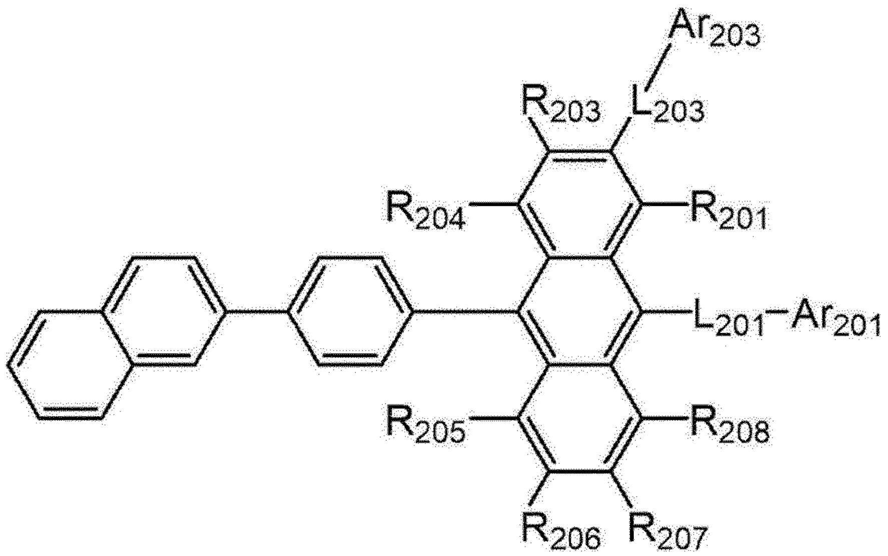

(228)

[Formula 243]

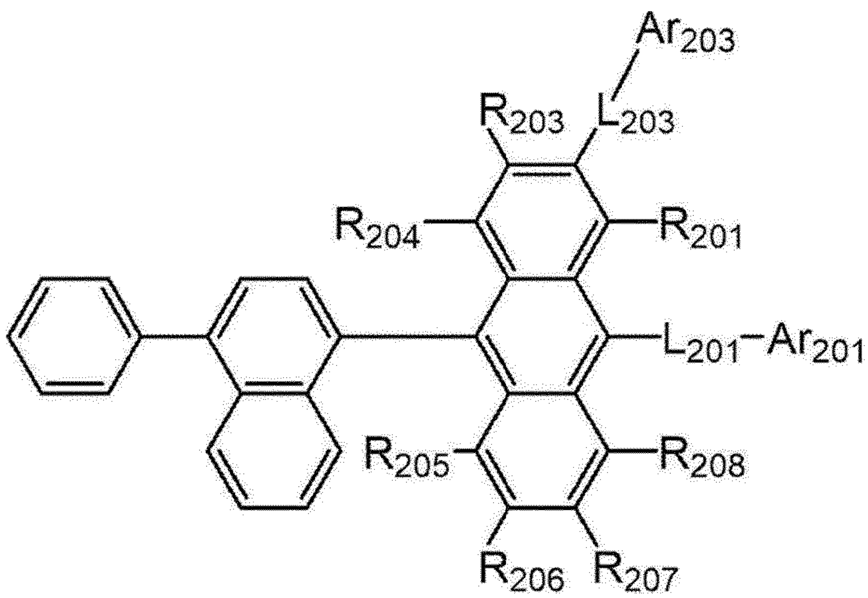

(229)

In the formulae (221), (222), (223), (224), (225), (226), (227), (228), and (229):

$R_{201}$ and $R_{203}$ to $R_{208}$ each independently represent the same as $R_{201}$ and $R_{203}$ to $R_{208}$ in the formula (2);
$L_{201}$ and $Ar_{201}$ respectively represent the same as $L_{201}$ and $Ar_{201}$ in the formula (2);
$L_{203}$ represents the same as $L_{201}$ in the formula (2);
$L_{203}$ and $L_{201}$ are mutually the same or different;
$Ar_{203}$ represents the same as $Ar_{201}$ in the formula (2); and
$Ar_{203}$ and $Ar_{201}$ are mutually the same or different.

The second compound represented by the formula (2) is also preferably a compound represented by a formula (241), a formula (242), a formula (243), a formula (244), a formula (245), a formula (246), a formula (247), a formula (248), or a formula (249) below.

[Formula 244]

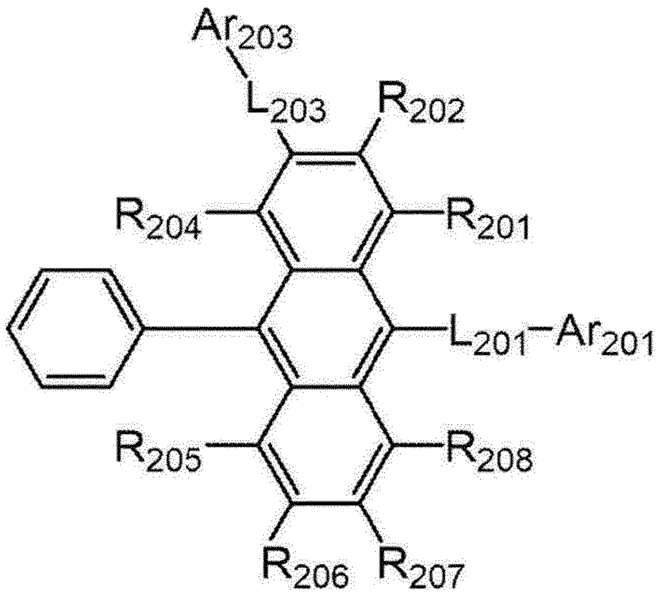

(241)

[Formula 245]

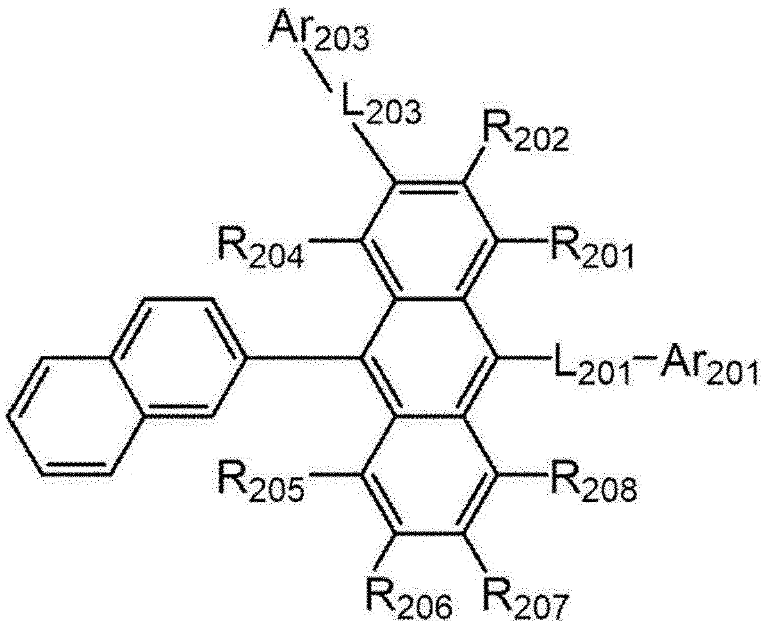

(242)

[Formula 246]

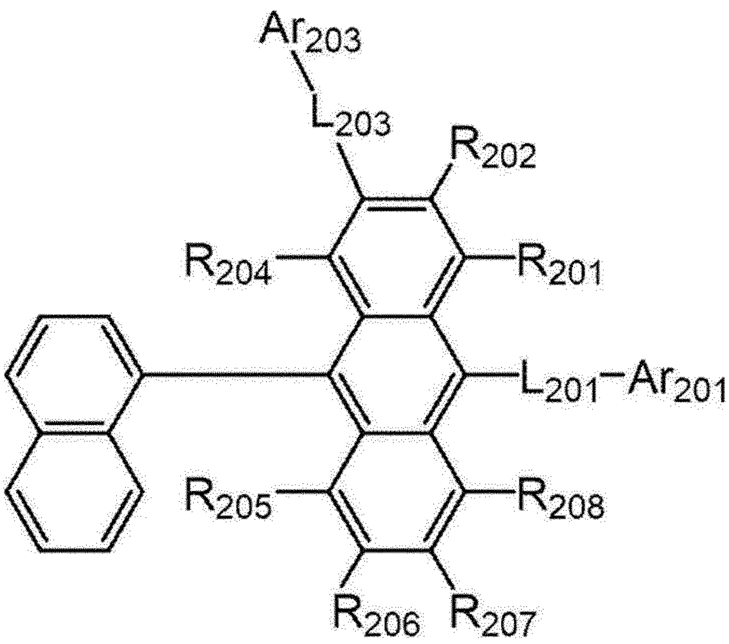

(243)

[Formula 247]

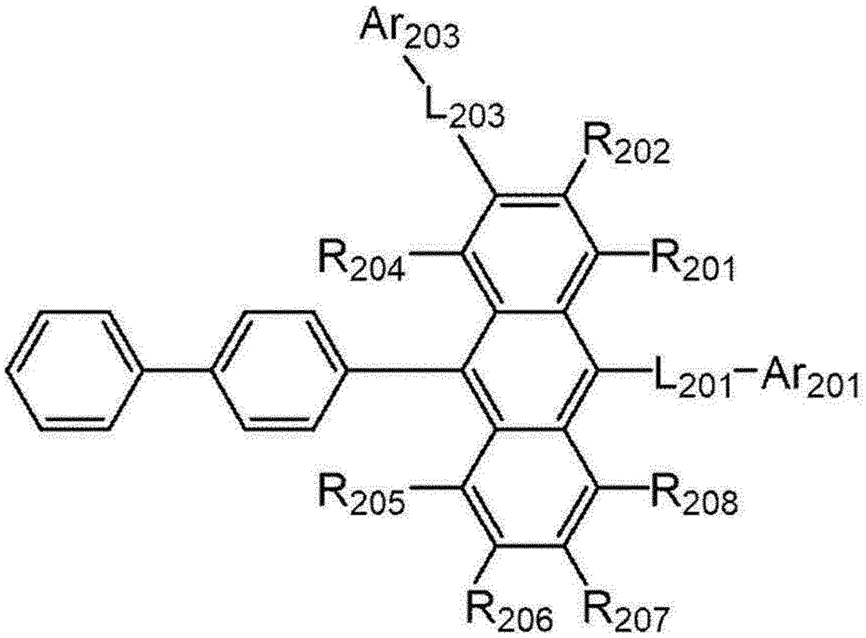

(244)

[Formula 248]

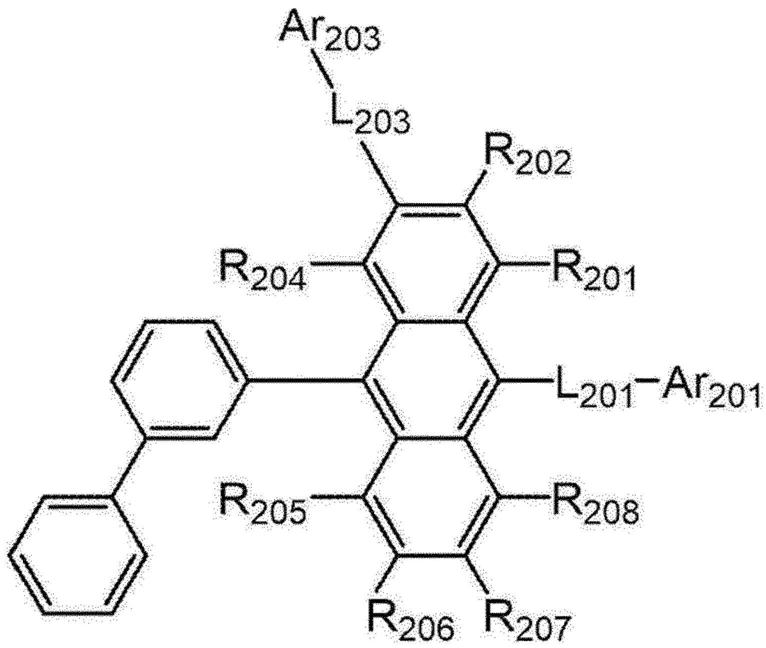

(245)

[Formula 249]

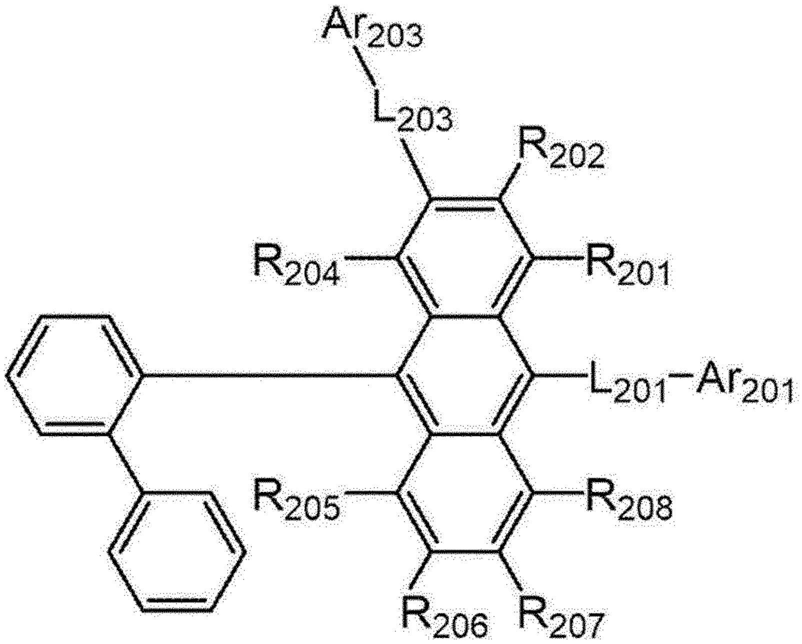

(246)

[Formula 250]

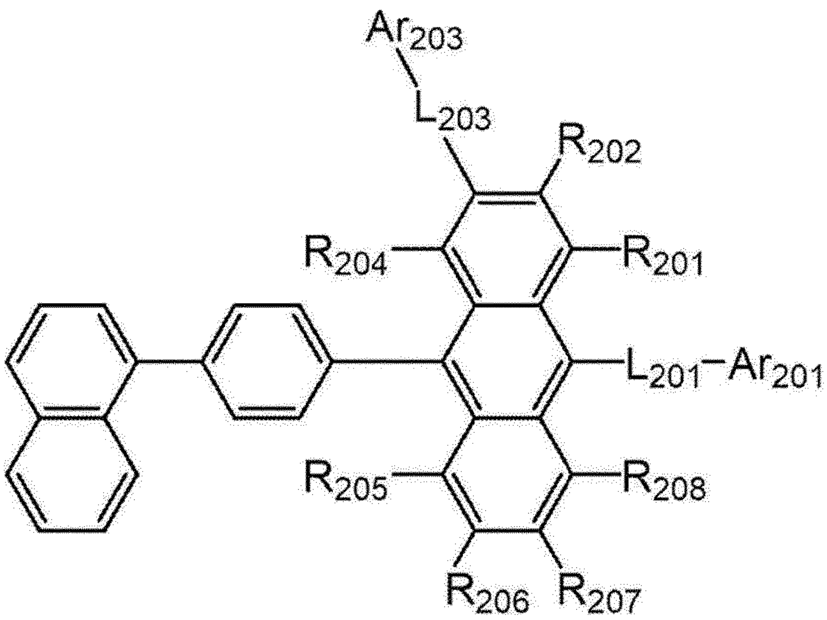

(247)

[Formula 251]

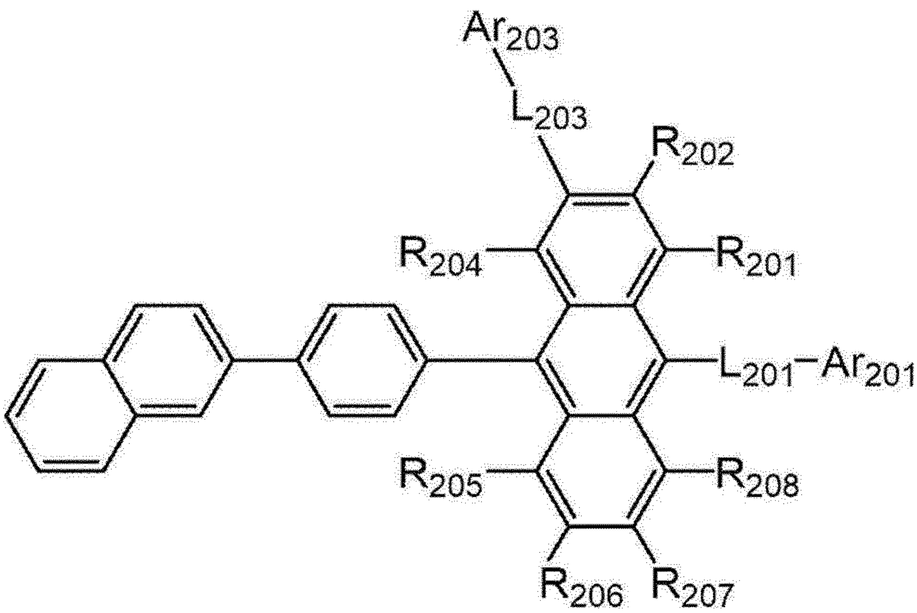

(248)

[Formula 252]

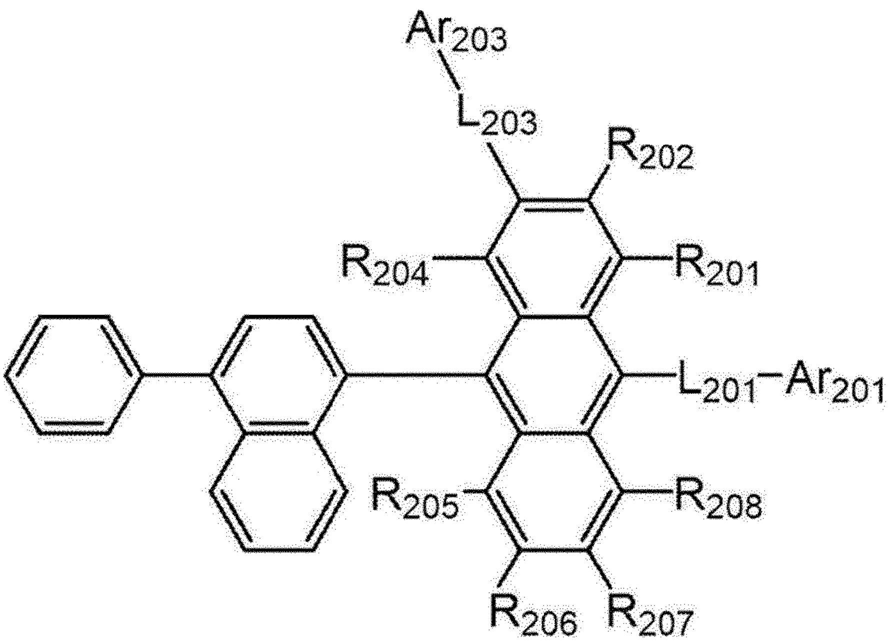

(249)

In the formulae (241), (242), (243), (244), (245), (246), (247), (248), and (249):

$R_{201}$, $R_{202}$ and $R_{204}$ to $R_{208}$ each independently represent the same as $R_{201}$, $R_{202}$ and $R_{204}$ to $R_{208}$ in the formula (2);
$L_{201}$ and $Ar_{201}$ respectively represent the same as $L_{201}$ and $Ar_{201}$ in the formula (2);
$L_{203}$ represents the same as $L_{201}$ in the formula (2);
$L_{203}$ and $L_{201}$ are mutually the same or different;
$Ar_{203}$ represents the same as $Ar_{201}$ in the formula (2); and
$Ar_{203}$ and $Ar_{201}$ are mutually the same or different.

$R_{201}$ to $R_{208}$ in the second compound represented by the formula (2) are preferably each independently a hydrogen atom, a substituted or unsubstituted alkyl group having 1 to 50 carbon atoms, a substituted or unsubstituted cycloalkyl group having 3 to 50 ring carbon atoms, or a group represented by -Si($R_{901}$)($R_{902}$)($R_{903}$).

$L_{201}$ is preferably a single bond, or an unsubstituted arylene group having 6 to 22 ring carbon atoms; and
$Ar_{201}$ is preferably a substituted or unsubstituted aryl group having 6 to 22 ring carbon atoms.

In the organic EL device according to the exemplary embodiment, $R_{201}$ to $R_{208}$ in the second compound represented by the formula (2) are preferably each independently a hydrogen atom, a substituted or unsubstituted alkyl group having 1 to 50 carbon atoms, a substituted or unsubstituted cycloalkyl group having 3 to 50 ring carbon atoms, or a group represented by -Si($R_{901}$)($R_{902}$)($R_{903}$).

In the organic EL device according to the exemplary embodiment, $R_{201}$ to $R_{208}$ in the second compound represented by the formula (2) are each preferably a hydrogen atom.

In the second compound, the groups specified to be "substituted or unsubstituted" are each preferably an unsubstituted group.

In the organic EL device according to the exemplary embodiment, for instance, $Ar_{201}$ in the second compound represented by the formula (2) is a substituted or unsubstituted dibenzofuranyl group.

In the organic EL device according to the exemplary embodiment, for instance, $Ar_{201}$ in the second compound represented by the formula (2) is an unsubstituted dibenzofuranyl group.

In the organic EL device according to the exemplary embodiment, for instance, the second compound represented by the formula (2) contains one or more hydrogen atoms, and the one or more hydrogen atoms include at least one deuterium atom.

In the organic EL device according to the exemplary embodiment, for instance, $L_{201}$ in the second compound represented by the formula (2) is TEMP-63 or TEMP-68.

[Formula 253]

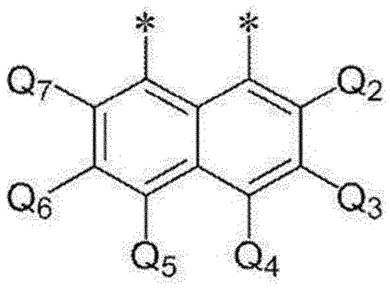

(TEMP-63)

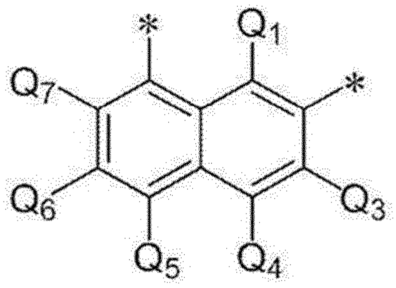

(TEMP-64)

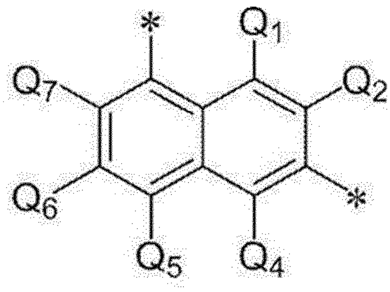

(TEMP-65)

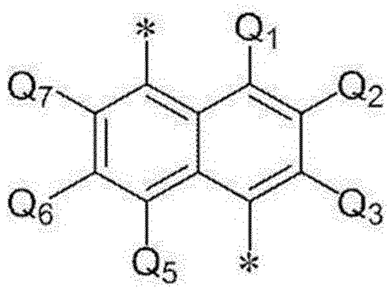

(TEMP-66)

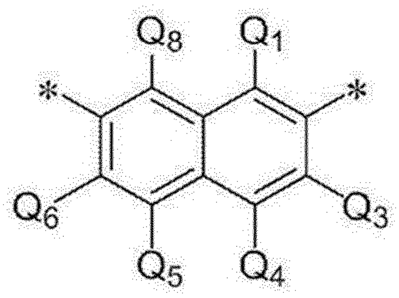

(TEMP-67)

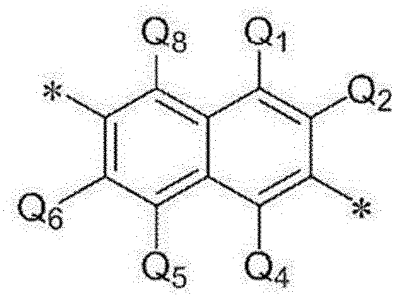

(TEMP-68)

In the organic EL device according to the exemplary embodiment, for instance, $Ar_{201}$ in the second compound represented by the formula (2) is at least one group selected from the group consisting of a substituted or unsubstituted anthryl group, benzanthryl group, phenanthryl group, benzophenanthryl group, phenalenyl group, pyrenyl group, chrysenyl group, benzochrysenyl group, triphenylenyl group, benzotriphenylenyl group, tetracenyl group, pentacenyl group, fluoranthenyl group, benzofluoranthenyl group, and perylenyl group.

In the organic EL device according to the exemplary embodiment, for instance, $Ar_{201}$ in the second compound represented by the formula (2) is a substituted or unsubstituted fluorenyl group.

In the organic EL device according to the exemplary embodiment, for instance, $Ar_{201}$ in the second compound represented by the formula (2) is a substituted or unsubstituted xanthenyl group.

In the organic EL device according to the exemplary embodiment, for instance, $Ar_{201}$ in the second compound represented by the formula (2) is a benzoxanthenyl group.

Method of Producing Second Compound

The second compound can be produced by a known method. Further, the second compound can be produced based on a known method through a known alternative reaction using a known material(s) tailored for the target compound.

Specific Examples of Second Compound

Specific examples of the second compound include the following compounds. However, the invention is not limited to the specific examples of the second compound.

[Formula 254]

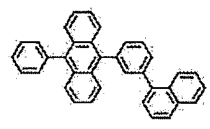
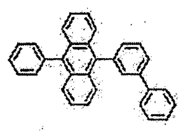
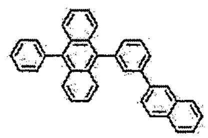
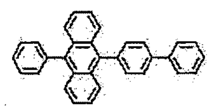
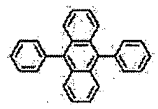
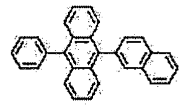
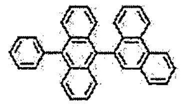
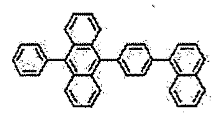
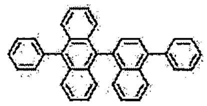
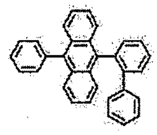
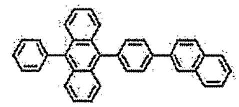
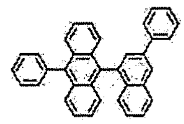
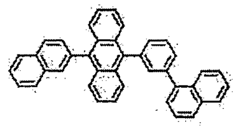
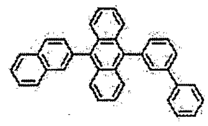
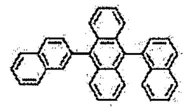
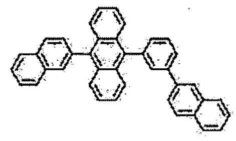
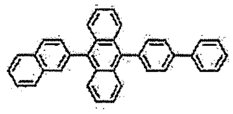
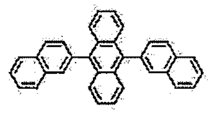
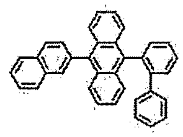
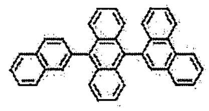
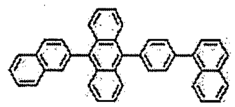
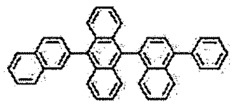
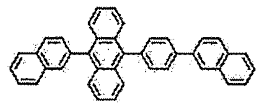
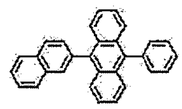
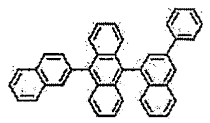
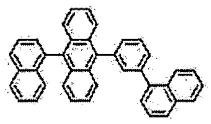
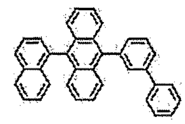
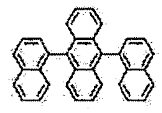
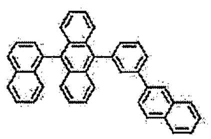
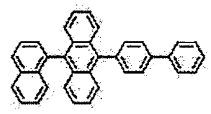
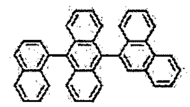
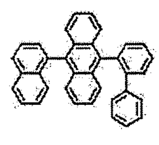
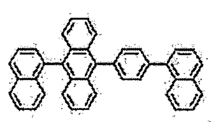
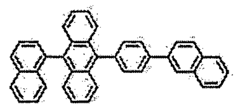
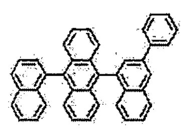
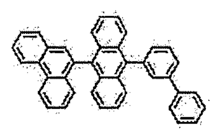
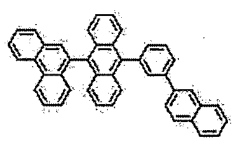
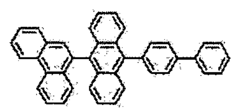
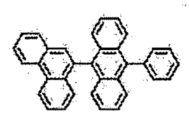
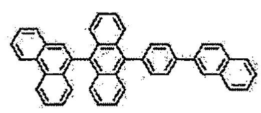

[Formula 255]

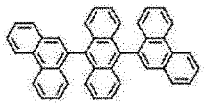
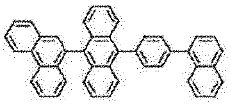
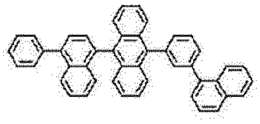
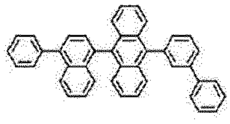

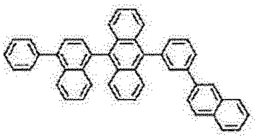
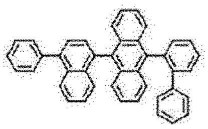
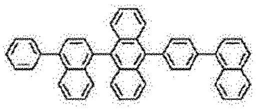
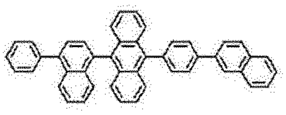

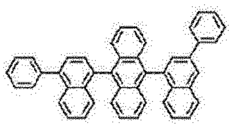
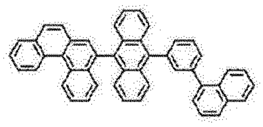
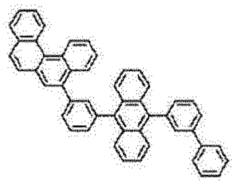
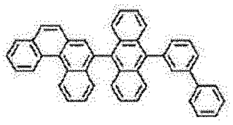

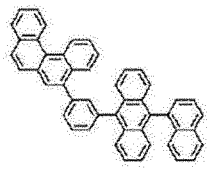
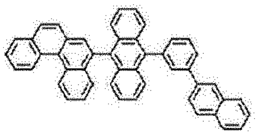
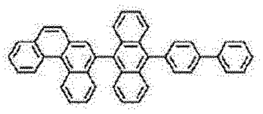
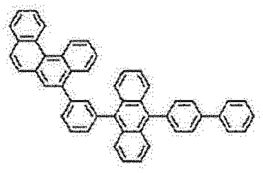

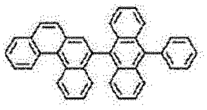
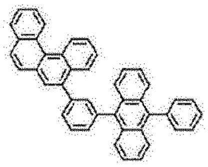
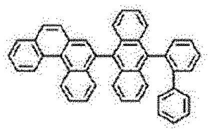
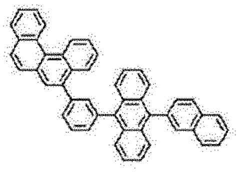

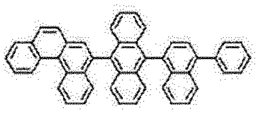
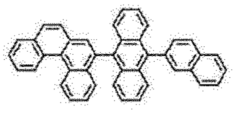
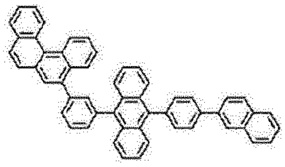
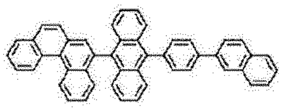

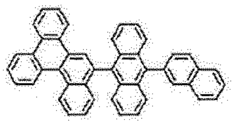
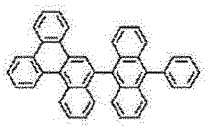
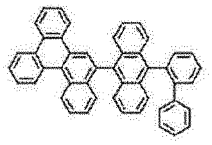
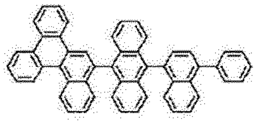

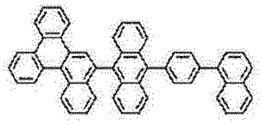
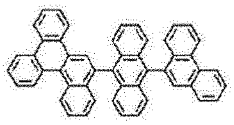
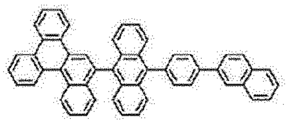
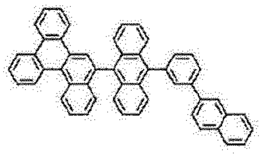

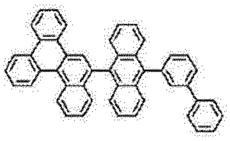
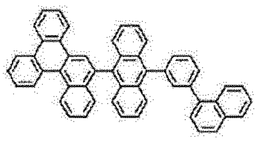
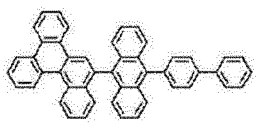
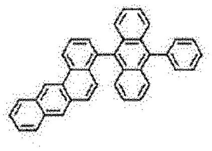

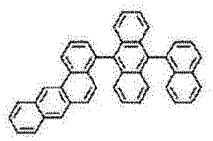
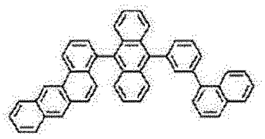
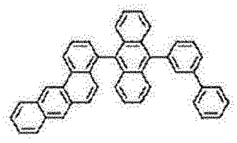
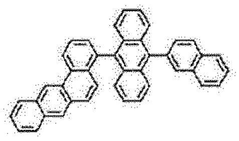

[Formula 256]

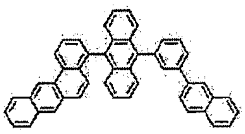
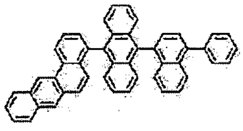
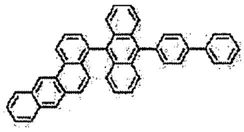
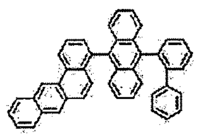

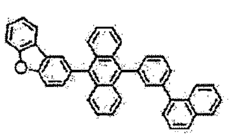
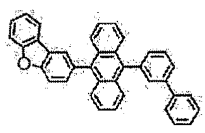
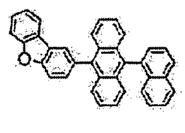
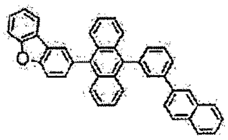

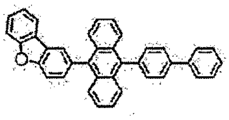
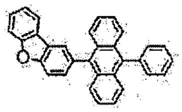
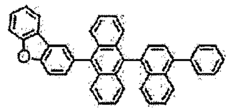
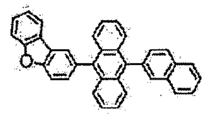

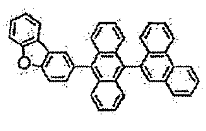
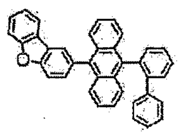
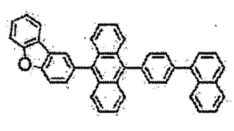
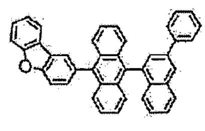

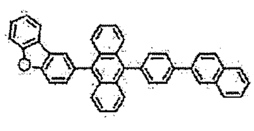
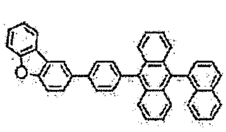
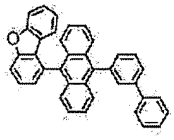
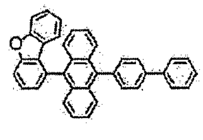

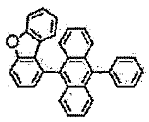
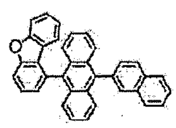
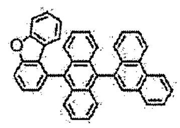
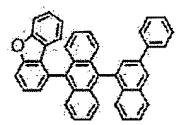

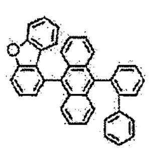
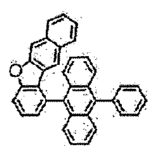
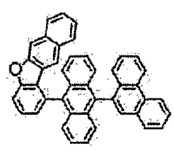
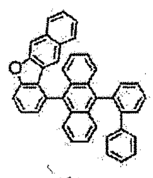

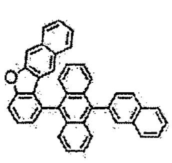
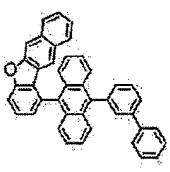
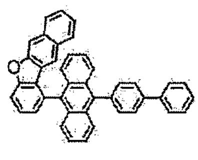
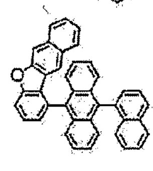

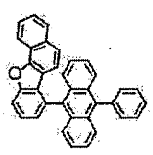
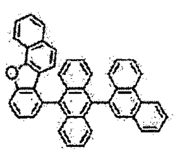
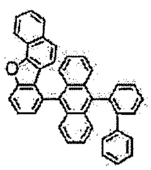
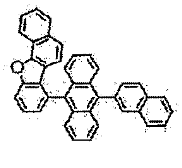

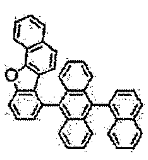
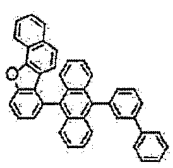
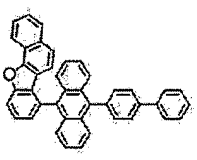
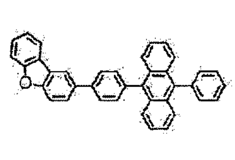

[Formula 257]

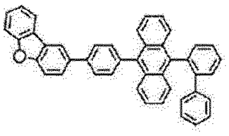
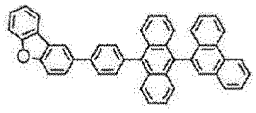
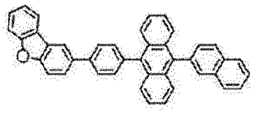
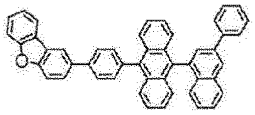
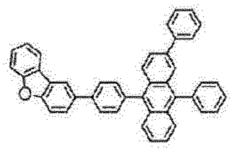
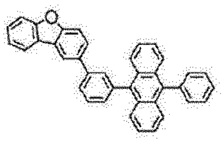
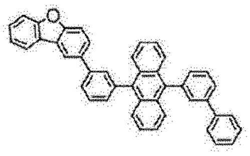
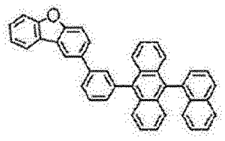
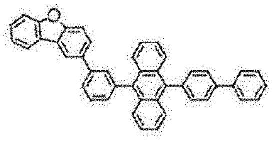
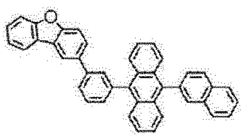
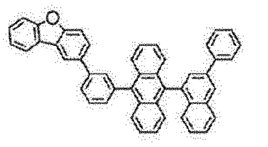
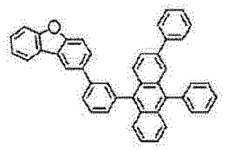
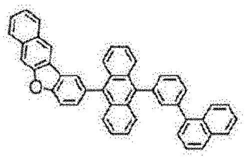
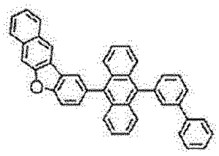
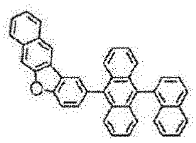
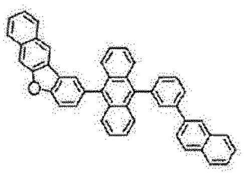
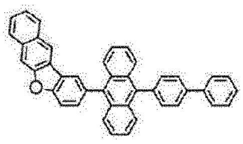
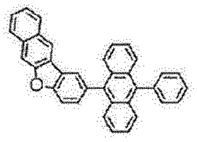
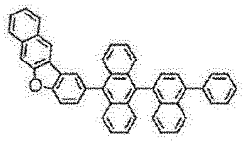
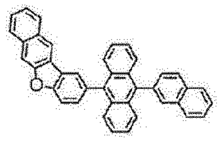
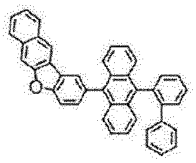
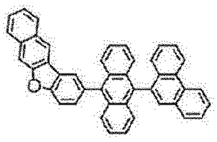
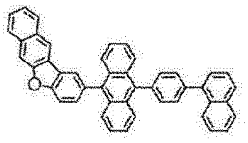
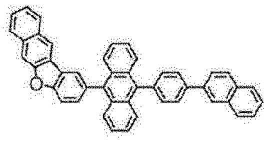
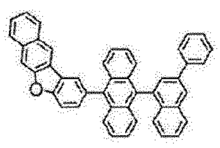
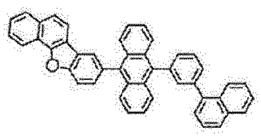
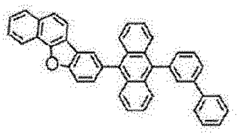
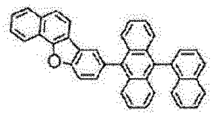
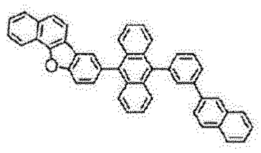
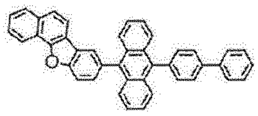
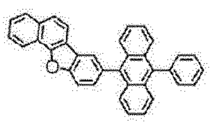
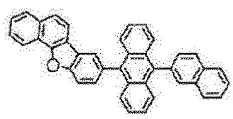
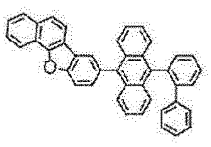
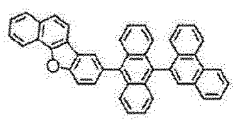
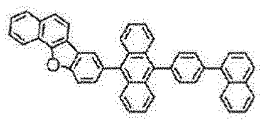
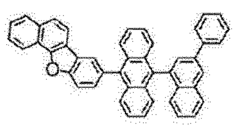
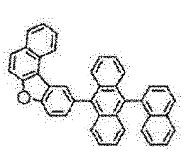
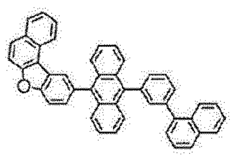
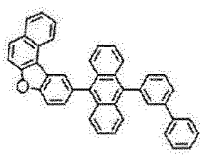
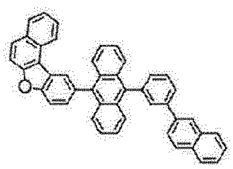

[Formula 258]

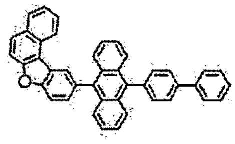 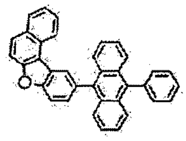 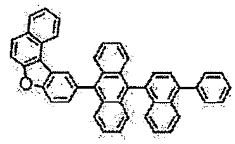 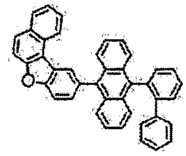

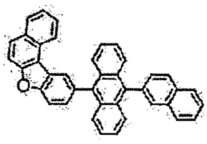 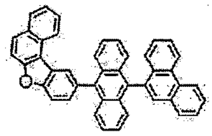 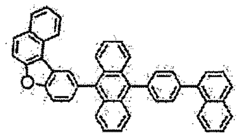 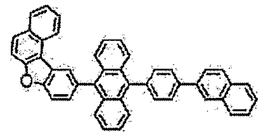

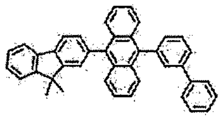 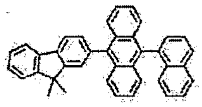 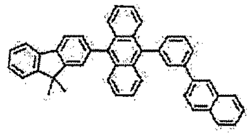 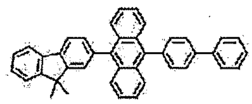

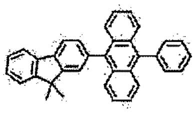 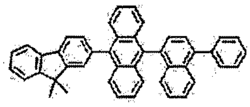 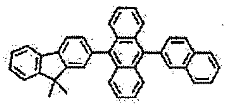 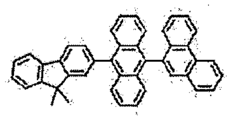

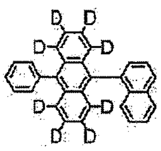 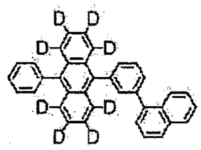 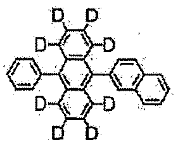 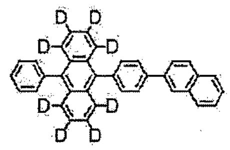

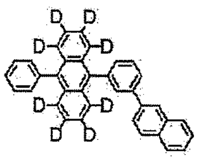 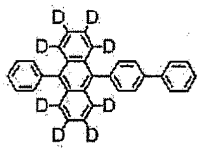 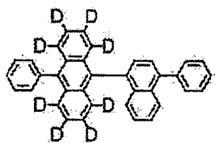 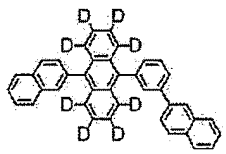

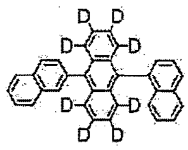 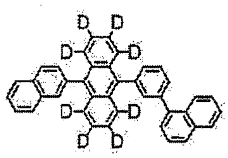 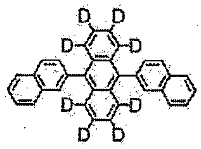 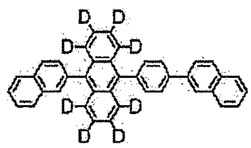

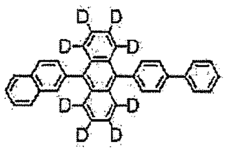 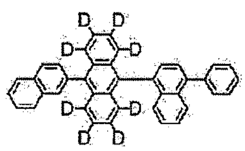 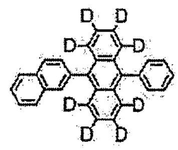 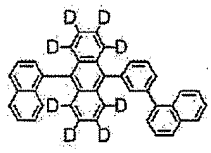

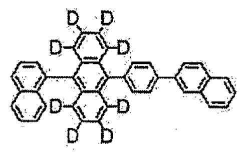 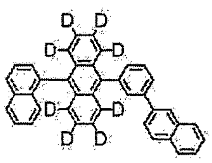 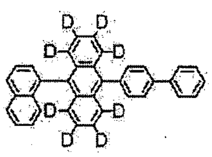 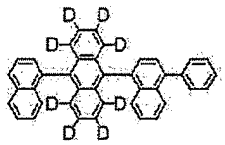

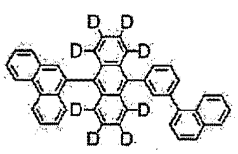 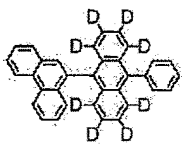 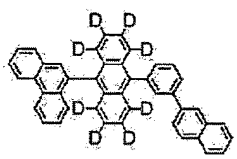 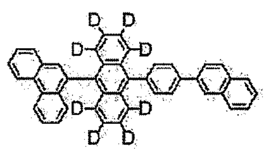

[Formula 259]

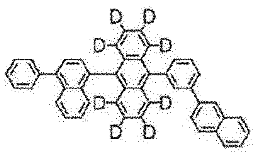 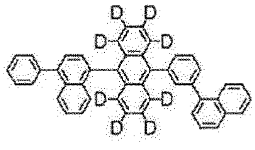 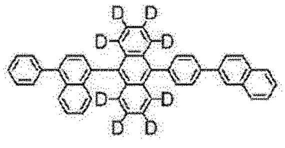 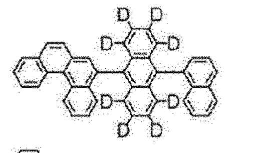
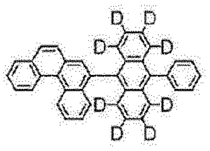 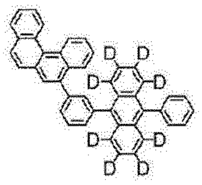 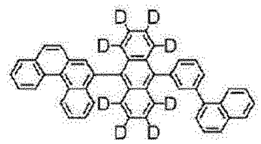 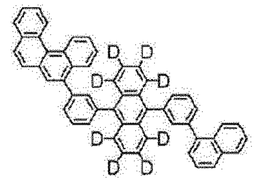
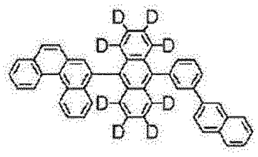 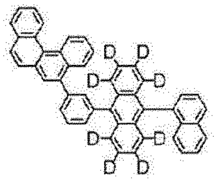 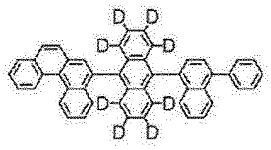 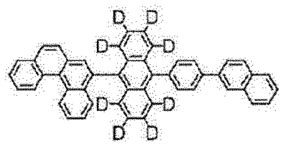
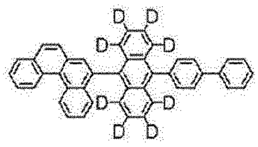 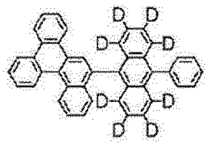 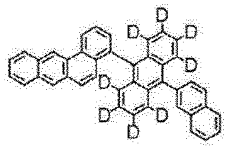 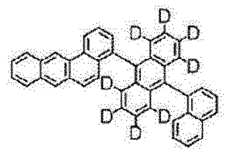
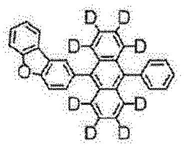 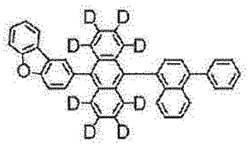 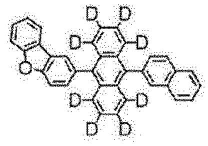 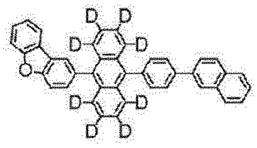
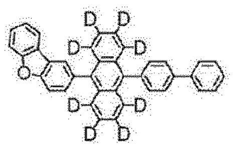 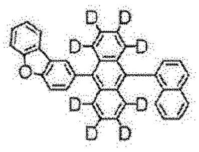 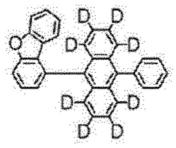 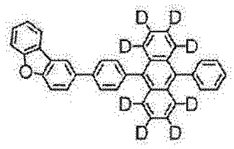
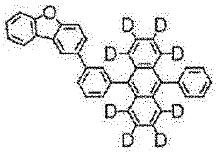 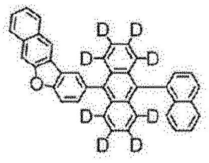 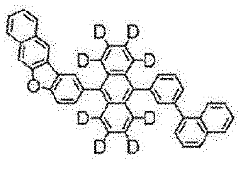 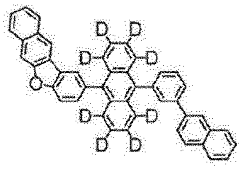
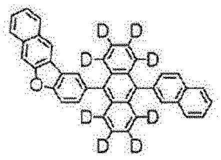 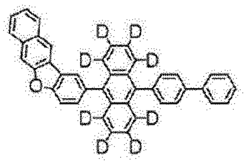 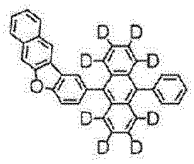 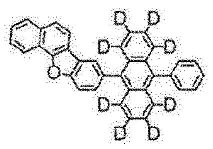
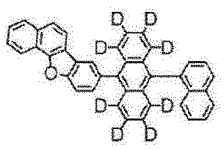 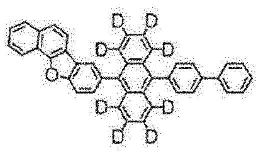 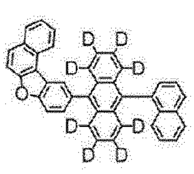 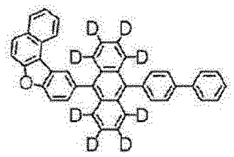
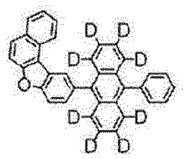 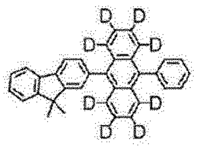 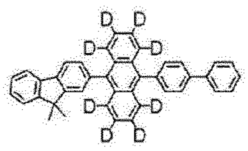 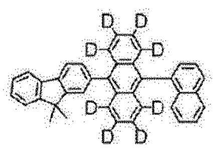

[Formula 260]

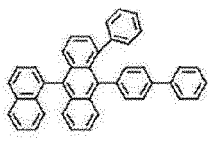
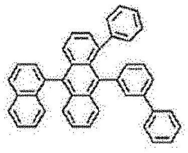
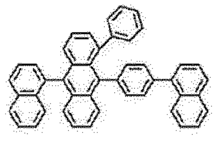
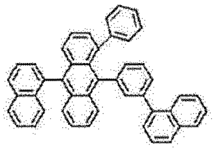
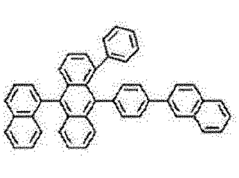
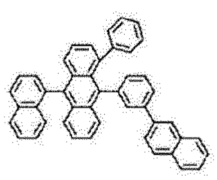
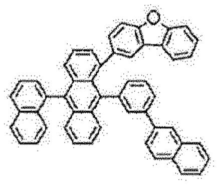
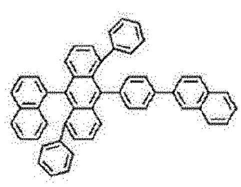
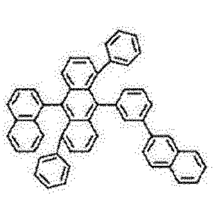
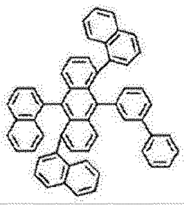
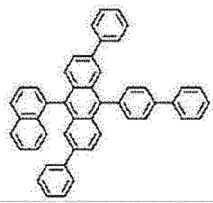
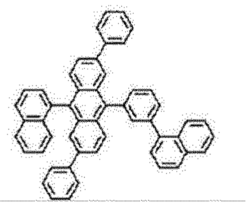

[Formula 261]

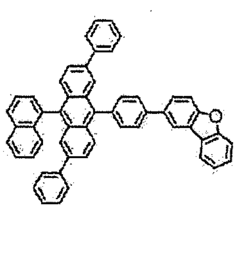
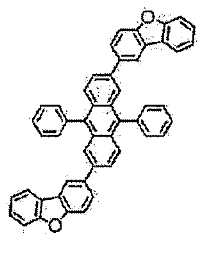
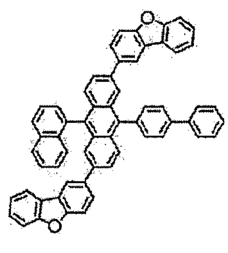
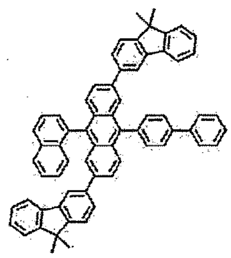
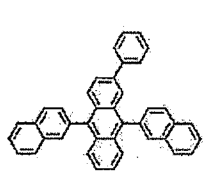
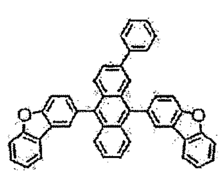
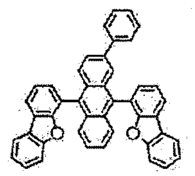
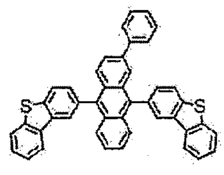

[Formula 262]

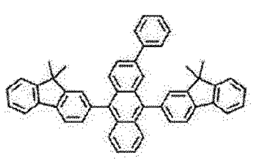 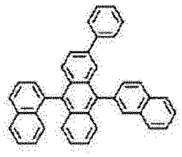 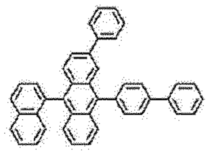 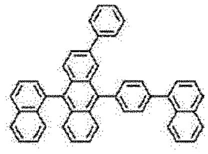

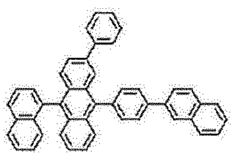 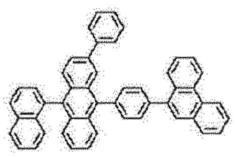 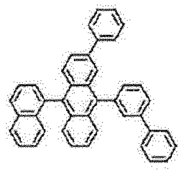 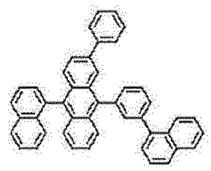

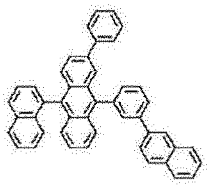 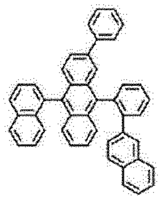 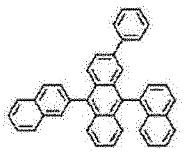 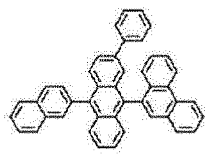

[Formula 263]

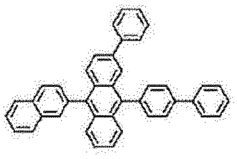 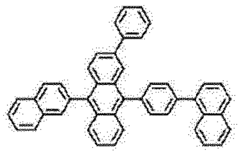 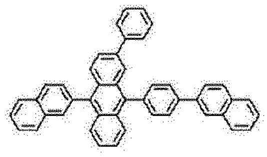 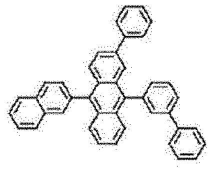

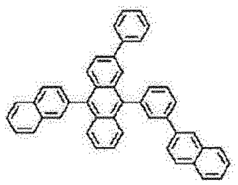 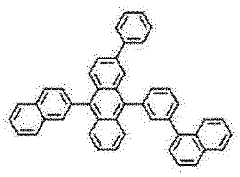 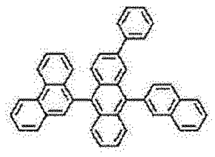 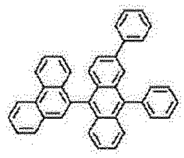

[Formula 264]

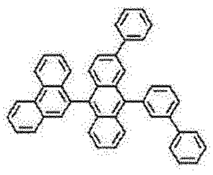 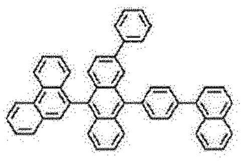 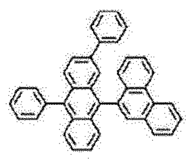 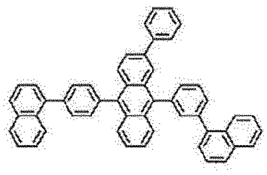

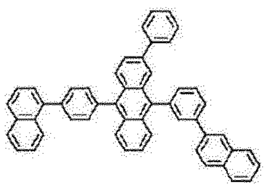 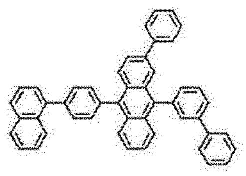 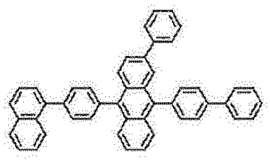 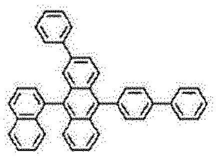

[Formula 265]

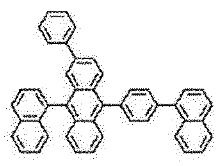
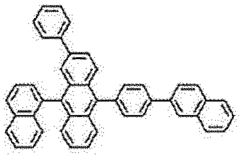
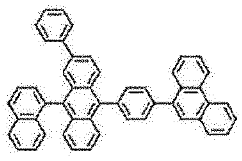
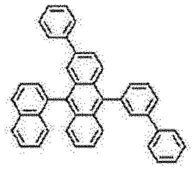
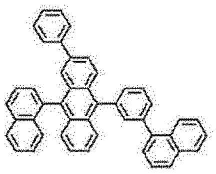
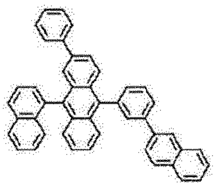
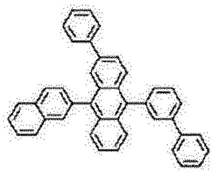
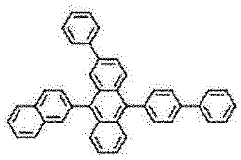

[Formula 266]

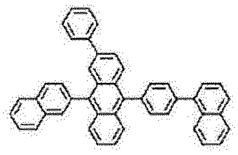
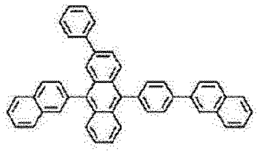
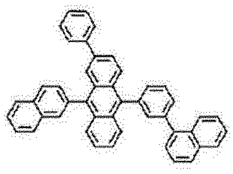
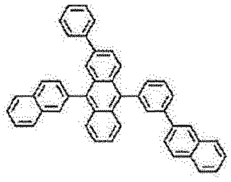
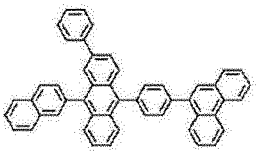
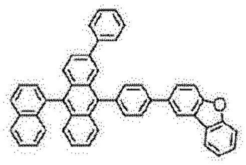
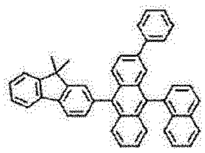
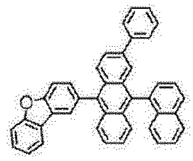

[Formula 267]

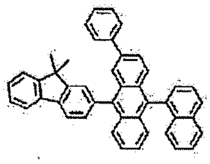
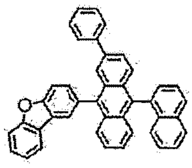
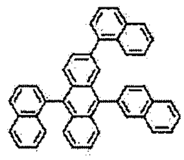
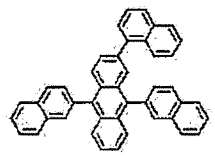
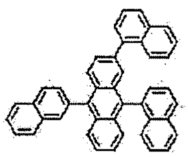
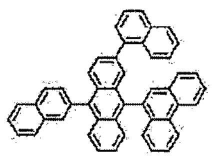
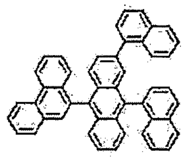
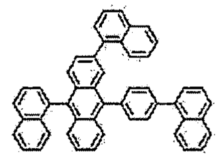

[Formula 268]

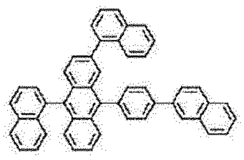
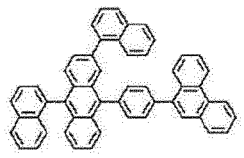
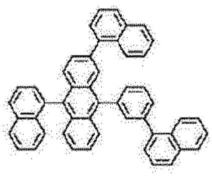
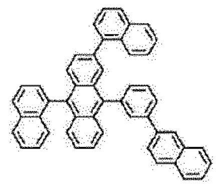
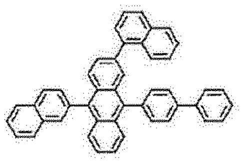
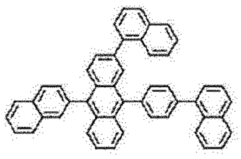
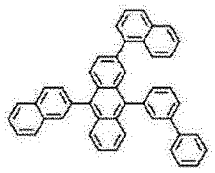
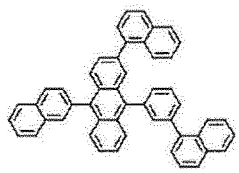

[Formula 269]

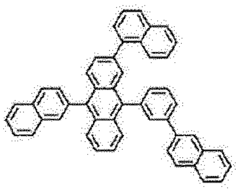
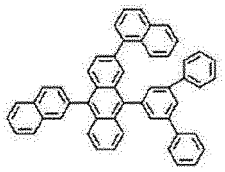
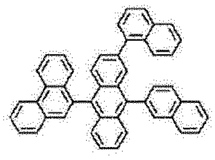
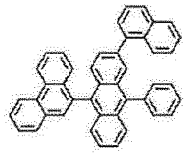
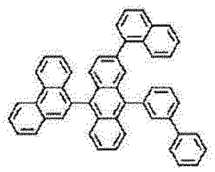
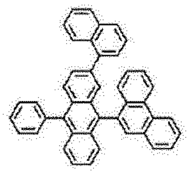
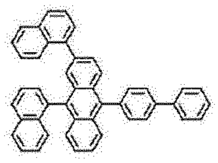
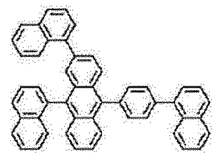

[Formula 270]

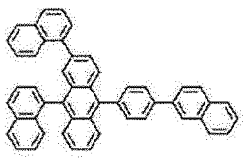
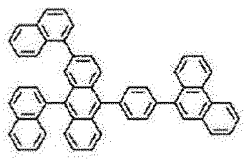
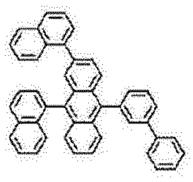
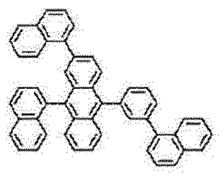
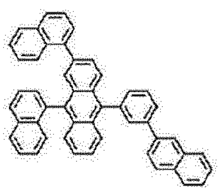
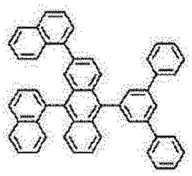
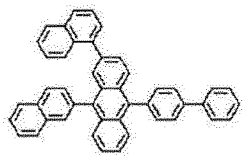
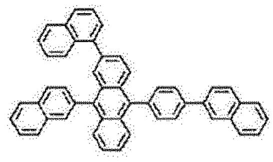

[Formula 271]

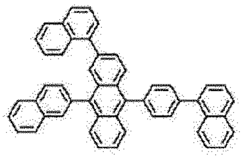
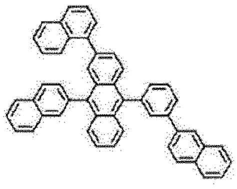
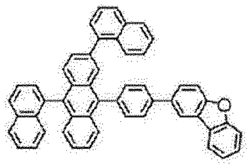
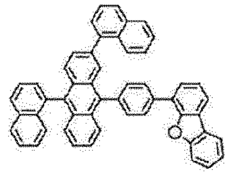
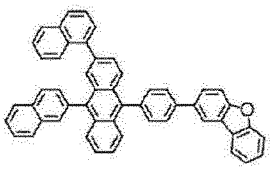
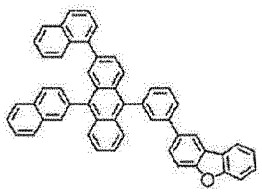
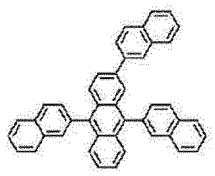
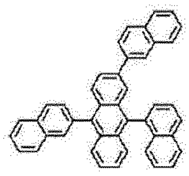

[Formula 272]

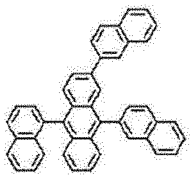
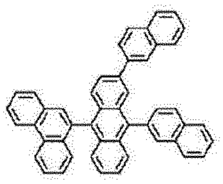
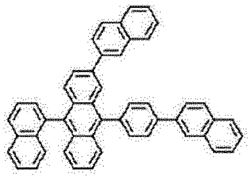
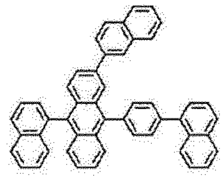
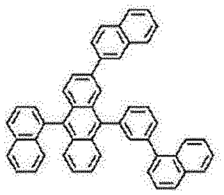
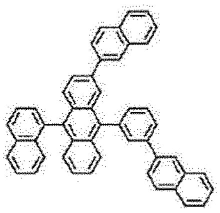
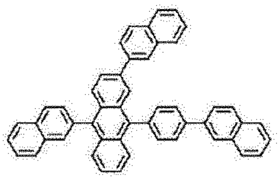
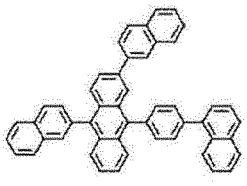

[Formula 273]

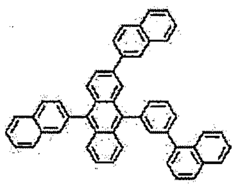
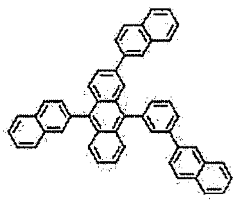
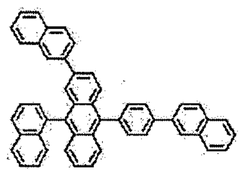
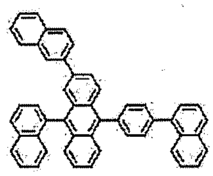
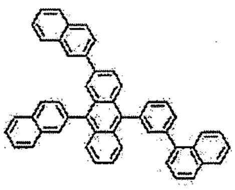
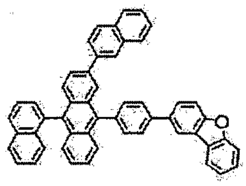
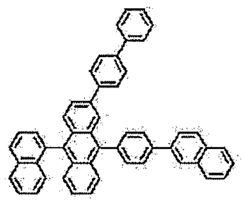
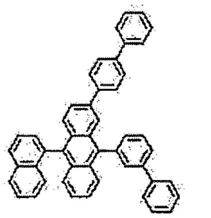

[Formula 274]

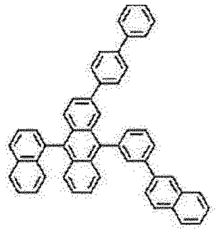
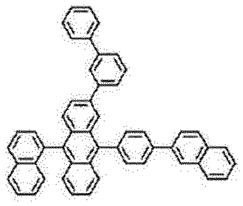
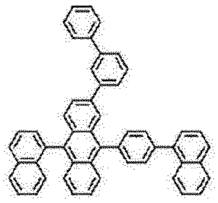
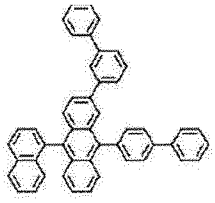
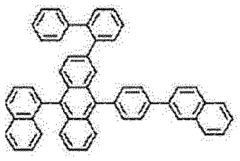
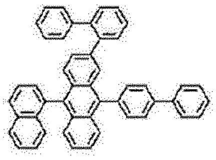
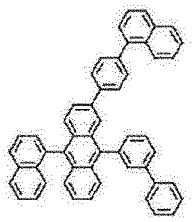
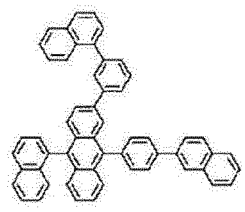

[Formula 275]

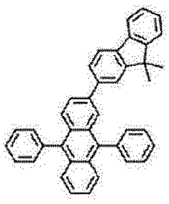
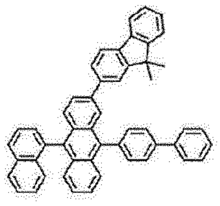
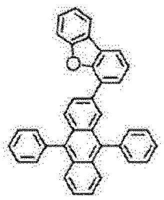
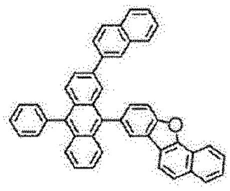
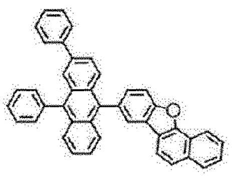
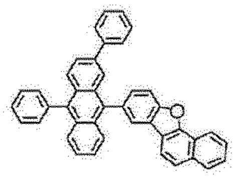
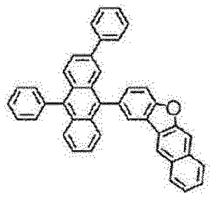
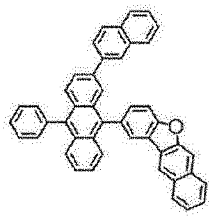

[Formula 276]

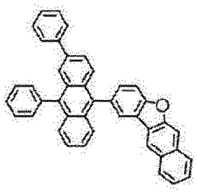
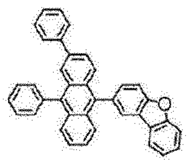
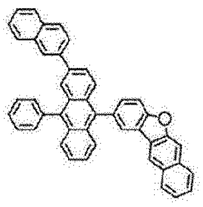
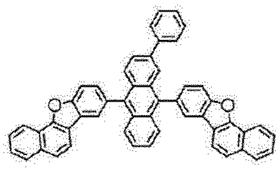
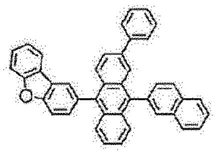
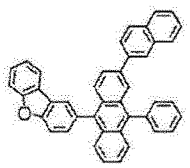
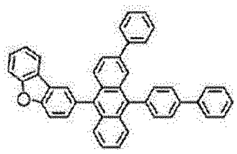
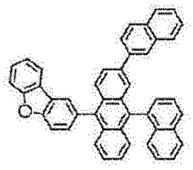

[Formula 277]

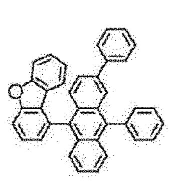 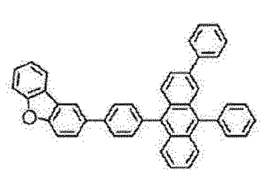 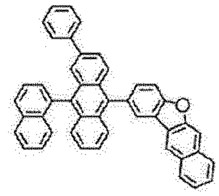 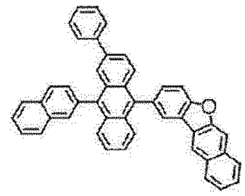

[Formula 278]

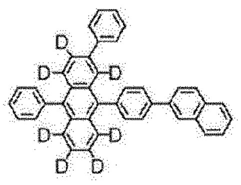 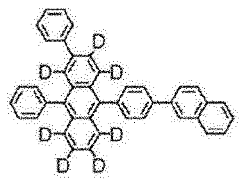 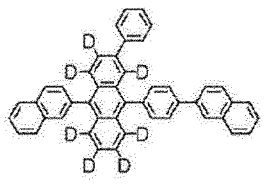 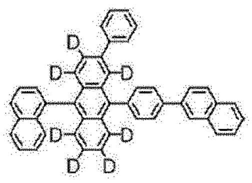

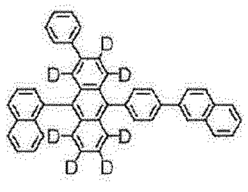 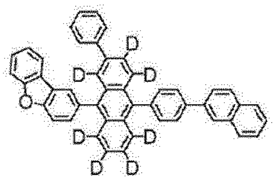 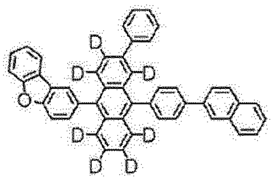 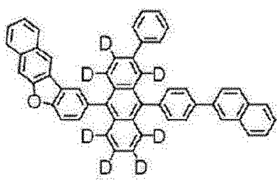

[Formula 279]

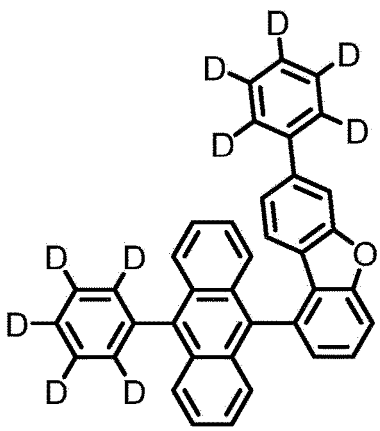

First Luminescent Compound and Second Luminescent Compound

In the organic EL device 1B, also preferably, the first emitting layer 51 further contains a first luminescent compound (preferably a fluorescent compound).

In the organic EL device 1B, also preferably, the second emitting layer 52 further contains a second luminescent compound (preferably a fluorescent compound).

The first luminescent compound contained in the first emitting layer 51 and the second luminescent compound contained in the second emitting layer 52 are mutually the same or different.

Examples of the first luminescent compound and the second luminescent compound are similar to those exemplified in the luminescent compound in the organic EL device 1A.

The organic EL device 1B in an exemplary embodiment contains, as at least one of the first luminescent compound in the first emitting layer 51 or the second luminescent compound in the second emitting layer 52, at least one compound selected from the group consisting of a compound represented by the formula (4), a compound represented by the formula (5), a compound represented by the formula (6), and a compound represented by the formula (63a).

In an exemplary embodiment, the substituent for "the substituted or unsubstituted" group in each of the formulae is an unsubstituted alkyl group having 1 to 50 carbon atoms, an unsubstituted alkenyl group having 2 to 50 carbon atoms, an unsubstituted alkynyl group having 2 to 50 carbon atoms, an unsubstituted cycloalkyl group having 3 to 50 ring carbon

atoms, a group represented by - $Si(R_{901a})(R_{902a})(R_{903a})$, a group represented by $-O-(R_{904a})$, a group represented by - $S-(R_{905a})$, a group represented by $-N(R_{906a})(R_{907a})$, a halogen atom, a cyano group, a nitro group, an unsubstituted aryl group having 6 to 50 ring carbon atoms, or an unsubstituted heterocyclic group having 5 to 50 ring atoms;

$R_{901a}$ to $R_{907a}$ are each independently a hydrogen atom, an unsubstituted alkyl group having 1 to 50 carbon atoms, an unsubstituted aryl group having 6 to 50 ring carbon atoms, or an unsubstituted heterocyclic group having 5 to 50 ring atoms;
when two or more $R_{901a}$ are present, the two or more $R_{901a}$ are mutually the same or different;
when two or more $R_{902a}$ are present, the two or more $R_{902a}$ are mutually the same or different;
when two or more $R_{903a}$ are present, the two or more $R_{903a}$ are mutually the same or different;
when two or more $R_{904a}$ are present, the two or more $R_{904a}$ are mutually the same or different;
when two or more $R_{905a}$ are present, the two or more $R_{905a}$ are mutually the same or different;
when two or more $R_{906a}$ are present, the two or more $R_{906a}$ are mutually the same or different; and
when two or more $R_{907a}$ are present, the two or more $R_{907a}$ are mutually the same or different.

In an exemplary embodiment, the substituent for "the substituted or unsubstituted" group in each of the formulae is an unsubstituted alkyl group having 1 to 50 carbon atoms, an unsubstituted aryl group having 6 to 50 ring carbon atoms, or an unsubstituted heterocyclic group having 5 to 50 ring atoms.

In an exemplary embodiment, the substituent for "the substituted or unsubstituted" group in each of the formulae is an unsubstituted alkyl group having 1 to 18 carbon atoms, an unsubstituted aryl group having 6 to 18 ring carbon atoms, or an unsubstituted heterocyclic group having 5 to 18 ring atoms.

In the organic EL device 1B, the first luminescent compound contained in the first emitting layer 51 is preferably a compound that emits light having a maximum peak wavelength of 500 nm or less, more preferably a compound that emits light having a maximum peak wavelength in a range from 430 nm to 480 nm.

In the organic EL device 1B, the first luminescent compound contained in the first emitting layer 51 is preferably a compound that emits fluorescence having a maximum peak wavelength of 500 nm or less, more preferably a compound that emits fluorescence having a maximum peak wavelength in a range from 430 nm to 480 nm.

In the organic EL device 1B, the second luminescent compound contained in the second emitting layer 52 is preferably a compound that emits light having a maximum peak wavelength of 500 nm or less, more preferably a compound that emits light having a maximum peak wavelength in a range from 430 nm to 480 nm.

In the organic EL device 1B, the second luminescent compound contained in the second emitting layer 52 is preferably a compound that emits fluorescence having a maximum peak wavelength of 500 nm or less, more preferably a compound that emits fluorescence having a maximum peak wavelength in a range from 430 nm to 480 nm.

When the first emitting layer 51 of the organic EL device 1B contains the first compound and the first luminescent compound, the first compound is preferably a host material and the first luminescent compound is preferably a dopant material.

In the organic EL device 1B, a triplet energy of the first compound $T_1(H1)$ and a triplet energy of the second compound $T_1(H2)$ preferably satisfy a relationship of a numerical formula (Numerical Formula 1) below.

$$T_1(H1) > T_1(H2) \ldots \text{(Numerical Formula 1)}$$

Conventionally, triplet-triplet annihilation (occasionally referred to as TTA) is known as a technique for enhancing the luminous efficiency of the organic EL device. TTA is a mechanism in which triplet excitons collide with one another to generate singlet excitons. The TTA mechanism is also referred to as a TTF mechanism as described in WO2010/134350.

The TTF phenomenon will be described. Holes injected from an anode and electrons injected from a cathode are recombined in an emitting layer to generate excitons. As for the spin state, as is conventionally known, singlet excitons account for 25% and triplet excitons account for 75%. In a conventionally known fluorescent device, light is emitted when singlet excitons of 25% are relaxed to the ground state. The remaining triplet excitons of 75% are returned to the ground state without emitting light through a thermal deactivation process. Accordingly, the theoretical limit value of the internal quantum efficiency of the conventional fluorescent device is believed to be 25%.

The behavior of triplet excitons generated within an organic substance has been theoretically examined. According to S. M. Bachilo et al. (J. Phys. Chem. A, 104, 7711 (2000)), assuming that high-order excitons such as quintet excitons are quickly returned to triplet excitons, triplet excitons (hereinafter abbreviated as $^3A^*$) collide with one another with an increase in density thereof, whereby a reaction shown by the following formula occurs. In the formula, 'A represents the ground state and $^1A^*$ represents the lowest singlet excitons.

$$^3A^* + {}^3A^* \rightarrow (4/9)^1A + (1/9)^1A^* + (13/9)^3A^*$$

In other words, $5^3A^* \rightarrow 4^1A + 1A^*$ is satisfied, and it is expected that, among triplet excitons initially generated, which account for 75%, one fifth thereof (i.e., 20%) is changed to singlet excitons. Accordingly, the amount of singlet excitons which contribute to emission is 40%, which is a value obtained by adding 15% (75% × (1/5) = 15%) to 25%, which is the amount ratio of initially generated singlet excitons. At this time, a ratio of luminous intensity derived from TTF (TTF ratio) relative to the total luminous intensity is 15/40, i.e., 37.5%. Assuming that singlet excitons are generated by collision of initially generated triplet excitons accounting for 75% (i.e., one singlet exciton is generated from two triplet excitons), a significantly high internal quantum efficiency of 62.5% is obtained, which is a value obtained by adding 37.5% (75% × (1/2) = 37.5%) to 25% (the amount ratio of initially generated singlet excitons). At this time, the TTF ratio is 37.5/62.5 = 60%.

In the organic EL device according to the exemplary embodiment, it is considered that triplet excitons generated by recombination of holes and electrons in the first emitting layer and present on an interface between the first emitting layer and the organic layer in direct contact therewith are not likely to be quenched even under the presence of excessive carriers on the interface between the first emitting layer and the organic layer. For instance, the presence of a recombination region locally on an interface between the first emitting layer and a hole transporting layer or an electron blocking layer is considered to cause quenching by excessive electrons. Meanwhile, the presence of a recombination region locally on an interface between the first emitting layer and an electron transporting layer or a hole blocking layer is considered to cause quenching by excessive holes.

When the organic EL device 1B includes at least two emitting layers (i.e., the first emitting layer 51 and the second emitting layer 52) satisfying a predetermined relationship, specifically, includes the first emitting layer 51 and the second emitting layer 52 so that the triplet energy of the first compound $T_1(H1)$ in the first emitting layer 51 and the triplet energy of the second compound $T_1(H2)$ in the second emitting layer 52 satisfy the relationship of the numerical formula (Numerical Formula 1), triplet excitons generated in the first emitting layer 51 can transfer to the second emitting layer 52 without being quenched by excessive carriers and be inhibited from back-transferring from the second emitting layer 52 to the first emitting layer 51. Consequently, the second emitting layer 52 exhibits the TTF mechanism to effectively generate singlet excitons, thereby improving the luminous efficiency.

Accordingly, the organic EL device 1B includes, as different regions, the first emitting layer 51 mainly generating triplet excitons and the second emitting layer 52 mainly exhibiting the TTF mechanism with triplet excitons having transferred from the first emitting layer 51, and a difference in triplet energy is provided by using a compound having a smaller triplet energy than that of the first compound in the first emitting layer as the second compound in the second emitting layer 52, thereby improving the luminous efficiency.

Triplet Energy $T_1$

A method of measuring a triplet energy $T_1$ is exemplified by a method below.

A measurement target compound is dissolved in EPA (diethylether : isopentane : ethanol = 5:5:2 in volume ratio) so as to fall within a range from $10^{-5}$ mol/L to $10^{-4}$ mol/L to prepare a solution, and the obtained solution is encapsulated in a quartz cell to provide a measurement sample. A phosphorescence spectrum (ordinate axis: phosphorescent luminous intensity, abscissa axis: wavelength) of the measurement sample is measured at a low temperature (77K). A tangent is drawn to the rise of the phosphorescence spectrum close to the short-wavelength region. An energy amount is calculated by a conversion equation (F1) below on a basis of a wavelength value $\lambda_{edge}$ [nm] at an intersection of the tangent and the abscissa axis. The calculated energy amount is defined as triplet energy $T_1$.

$$\text{Conversion Equation (F1): } T_1 \text{ [eV]} = 1239.85/\lambda_{edge}$$

The tangent to the rise of the phosphorescence spectrum close to the short-wavelength region is drawn as follows. While moving on a curve of the phosphorescence spectrum from the short-wavelength region to the local maximum value closest to the short-wavelength region among the local maximum values of the phosphorescence spectrum, a tangent is checked at each point on the curve toward the long-wavelength of the phosphorescence spectrum. An inclination of the tangent is increased along the rise of the curve (i.e., a value of the ordinate axis is increased). A tangent drawn at a point of the local maximum inclination (i.e., a tangent at an inflection point) is defined as the tangent to the rise of the phosphorescence spectrum close to the short-wavelength region.

A local maximum point where a peak intensity is 15% or less of the maximum peak intensity of the spectrum is not counted as the above-mentioned local maximum peak intensity closest to the short-wavelength region. The tangent drawn at a point that is closest to the local maximum peak intensity closest to the short-wavelength region and where the inclination of the curve is the local maximum is defined as a tangent to the rise of the phosphorescence spectrum

close to the short-wavelength region.

For phosphorescence measurement, a spectrophotofluorometer body F-4500 (produced by Hitachi High-Technologies Corporation) is usable. The measurement apparatus is not limited thereto. A combination of a cooling unit, a low temperature container, an excitation light source and a light-receiving unit may be used for measurement.

When the first emitting layer 51 contains the first compound and the first luminescent compound in the organic EL device 1B, a singlet energy of the first compound $S_1(H1)$ and a singlet energy of the first luminescent compound $S_1(D3)$ preferably satisfy a relationship of a numerical formula (Numerical Formula 2) below.

$$S_1(H1) > S_1(D3) \ldots \text{(Numerical Formula 2)}$$

Singlet Energy $S_1$

A method of measuring a singlet energy $S_1$ with use of a solution (occasionally referred to as a solution method) is exemplified by a method below.

A toluene solution of a measurement target compound at a concentration ranging from $10^{-5}$ mol/L to $10^{-4}$ mol/L is prepared and put in a quartz cell. An absorption spectrum (ordinate axis: absorption intensity, abscissa axis: wavelength) of the thus-obtained sample is measured at a normal temperature (300K). A tangent is drawn to the fall of the absorption spectrum close to the long-wavelength region, and a wavelength value $\lambda_{edge}$ (nm) at an intersection of the tangent and the abscissa axis is assigned to a conversion equation (F2) below to calculate singlet energy $S_1$.

$$\text{Conversion Equation (F2): } S_1\ [\text{eV}] = 1239.85/\lambda\text{edge}$$

Any apparatus for measuring the absorption spectrum is usable. For instance, a spectrophotometer (U3310 produced by Hitachi, Ltd.) is usable.

The tangent to the fall of the absorption spectrum close to the long-wavelength region is drawn as follows. While moving on a curve of the absorption spectrum from the local maximum value closest to the long-wavelength region, among the local maximum values of the absorption spectrum, in a long-wavelength direction, a tangent at each point on the curve is checked. An inclination of the tangent is decreased and increased in a repeated manner as the curve falls (i.e., a value of the ordinate axis is decreased). A tangent drawn at a point where the inclination of the curve is the local minimum closest to the long-wavelength region (except when absorbance is 0.1 or less) is defined as the tangent to the fall of the absorption spectrum close to the long-wavelength region.

The local maximum absorbance of 0.2 or less is not counted as the above-mentioned local maximum absorbance closest to the long-wavelength region.

When the second emitting layer 52 of the organic EL device 1B contains the second compound and the second luminescent compound, the second compound is preferably a host material and the second luminescent compound is preferably a dopant material.

When the second emitting layer 52 of the organic EL device 1B contains the second compound and the second luminescent compound, a singlet energy of the second compound $S_1(H2)$ and a singlet energy of the second luminescent compound $S_1(D4)$ preferably satisfy a relationship of a numerical formula (Numerical Formula 3) below.

$$S_1(H2) > S_1(D4) \ldots \text{(Numerical Formula 3)}$$

The first emitting layer 51 and the second emitting layer 52 preferably contain no phosphorescent material (no dopant material).

Further, the first emitting layer 51 and the second emitting layer 52 preferably contain no heavy-metal complex and no phosphorescent rare earth metal complex. Here, examples of the heavy-metal complex include an iridium complex, osmium complex, and platinum complex.

Furthermore, the first emitting layer 51 and the second emitting layer 52 also preferably contain no metal complex.

Film Thickness of Emitting Layer

A film thickness of each of the first emitting layer 51 and the second emitting layer 52 in the organic EL device 1B is preferably in a range from 5 nm to 50 nm, more preferably in a range from 7 nm to 50 nm, and still more preferably in a range from 10 nm to 50 nm. A film thickness of the emitting layer of 5 nm or more facilitates the formation of the

emitting layer and the adjustment of chromaticity. A film thickness of the emitting layer of 50 nm or less easily inhibits an increase in drive voltage.

Content Ratios of Compounds in Emitting Layer

When the first emitting layer 51 contains the first compound and the first luminescent compound, content ratios of the first compound and the first luminescent compound in the first emitting layer 51 preferably fall, for instance, within ranges below.

The content ratio of the first compound falls within, preferably, in a range from 80 mass% to 99 mass%, more preferably in a range from 90 mass% to 99 mass%, and still more preferably in a range from 95 mass% to 99 mass%.

The content ratio of the first luminescent compound falls within, preferably, in a range from 1 mass% to 10 mass%, more preferably in a range from 1 mass% to 7 mass%, and still more preferably in a range from 1 mass% to 5 mass%.

The upper limit of the total of the content ratios of the first compound and the first luminescent compound in the first emitting layer 51 is 100 mass%.

In the exemplary embodiment, the first emitting layer 51 may further contain any other material than the first compound and the first luminescent compound.

The first emitting layer 51 may contain a single type of the first compound or two or more types of the first compound. The first emitting layer 51 may contain a single type of the first luminescent compound or two or more types of the first luminescent compound.

When the second emitting layer 52 contains the second compound and the second luminescent compound, content ratios of the second compound and the second luminescent compound in the second emitting layer 52 preferably fall, for instance, within ranges below.

The content ratio of the second compound falls within, preferably, in a range from 80 mass% to 99 mass%, more preferably in a range from 90 mass% to 99 mass%, and still more preferably in a range from 95 mass% to 99 mass%.

The content ratio of the second luminescent compound falls within, preferably, in a range from 1 mass% to 10 mass%, more preferably in a range from 1 mass% to 7 mass%, and still more preferably in a range from 1 mass% to 5 mass%.

The upper limit of the total of the content ratios of the second compound and the second luminescent compound in the second emitting layer 52 is 100 mass%.

In the exemplary embodiment, the second emitting layer 52 may further contain any other material than the second compound and the second luminescent compound.

The second emitting layer 52 may contain a single type of the second compound or two or more types of the second compound. The second emitting layer 52 may contain a single type of the second luminescent compound or two or more types of the second luminescent compound.

In the organic EL device 1B, the first emitting layer 51 and the second emitting layer 52 are also preferably in direct contact with each other.

Herein, a layer arrangement in which the first emitting layer 51 and the second emitting layer 52 are in direct contact with each other in the organic EL device 1B may include one of arrangements (LS1), (LS2) and (LS3) below.

(LS1) An arrangement in which a region containing both the first compound as a host material (hereinafter occasionally referred to as a first host material) and the second compound as a host material (hereinafter occasionally referred to as a second host material) is generated in a process of vapor-depositing the compound of the first emitting layer 51 and vapor-depositing the compound of the second emitting layer 52, and is present on the interface between the first emitting layer 51 and the second emitting layer 52.

(LS2) An arrangement in which in a case of containing a luminescent compound in the first emitting layer 51 and the second emitting layer 52, a region containing the first host material, the second host material and the luminescent compound is generated in a process of vapor-depositing the compound of the first emitting layer 51 and vapor-depositing the compound of the second emitting layer 52, and is present on the interface between the first emitting layer 51 and the second emitting layer 52.

(LS3) An arrangement in which in a case of containing a luminescent compound in the first emitting layer 51 and the second emitting layer 52, a region containing the luminescent compound, a region containing the first host material, or a region containing the second host material is generated in a process of vapor-depositing the compound of the first emitting layer 51 and vapor-depositing the compound of the second emitting layer 52, and is present on the interface between the first emitting layer 51 and the second emitting layer 52.

When the organic EL device 1B includes a third emitting layer, the first emitting layer 51 and the second emitting layer 52 are preferably in direct contact with each other and the second emitting layer 52 and the third emitting layer are preferably in direct contact with each other.

A layer arrangement in which the second emitting layer 52 and the third emitting layer are in direct contact with

each other in the organic EL device 1B may include one of arrangements (LS4), (LS5) and (LS6) below.

(LS4) An arrangement in which a region containing both the second host material and a third host material (host material contained in the third emitting layer) is generated in a process of vapor-depositing the compound of the second emitting layer 52 and vapor-depositing the compound of the third emitting layer, and is present on the interface between the second emitting layer 52 and the third emitting layer.

(LS5) An arrangement in which in a case of containing a luminescent compound in the second emitting layer 52 and the third emitting layer, a region containing the second host material, the third host material and the luminescent compound is generated in a process of vapor-depositing the compound of the second emitting layer 52 and vapor-depositing the compound of the third emitting layer, and is present on the interface between the second emitting layer 52 and the third emitting layer.

(LS6) An arrangement in which in a case of containing a luminescent compound in the second emitting layer 52 and the third emitting layer, a region containing the luminescent compound, a region containing the second host material, or a region containing the third host material is generated in a process of vapor-depositing the compound of the second emitting layer 52 and vapor-depositing the compound of the third emitting layer, and is present on the interface between the second emitting layer 52 and the third emitting layer.

Also preferably, the organic EL device 1B further includes an interposed layer.

When the organic EL device 1B includes an interposed layer, the interposed layer is preferably disposed between the first emitting layer 51 and the second emitting layer 52.

## Interposed Layer

The interposed layer is preferably a non-doped layer. The interposed layer preferably contains no metal atom.

The interposed layer contains an interposed layer material. The interposed layer material is preferably not a luminescent compound.

The interposed layer material, which is not particularly limited, is preferably any other material than the luminescent compound.

Examples of the interposed layer material include: 1) a heterocyclic compound such as an oxadiazole derivative, benzimidazole derivative, or phenanthroline derivative; 2) a fused aromatic compound such as a carbazole derivative, anthracene derivative, phenanthrene derivative, pyrene derivative or chrysene derivative; and 3) an aromatic amine compound such as a triarylamine derivative or a fused polycyclic aromatic amine derivative.

The interposed layer material may be one or both of the first compound contained in the first emitting layer 51 and the second compound contained in the second emitting layer 52.

When the interposed layer contains a plurality of interposed layer materials, the content of each interposed layer material is preferably 10 mass% or more with respect to the total mass of the interposed layer.

The interposed layer contains the interposed layer material preferably at 60 mass% or more, more preferably at 70 mass% or more, still more preferably at 80 mass% or more, still further more preferably at 90 mass% or more, and yet still further more preferably at 95 mass% or more, with respect to the total mass of the interposed layer.

The interposed layer may contain a single type of interposed layer material or two or more types of interposed layer materials.

When the interposed layer contains two or more types of interposed layer materials, the upper limit of the total of the content ratios of the two or more types of interposed layer materials is 100 mass%.

It should be noted that the interposed layer of the exemplary embodiment may further contain any other material than the interposed layer material.

The interposed layer may be provided in the form of a single layer or a laminate of two or more layers.

The film thickness of the interposed layer, which is not particularly limited, is preferably in a range from 3 nm to 15 nm, more preferably in a range from 5 nm to 10 nm per layer.

The arrangements of respective layers common to the organic EL devices 1A and 1B will be further described below. It should be noted that the reference numerals are occasionally omitted below.

## Substrate

The substrate 2 is used as a support for the organic EL device. For instance, glass, quartz, plastics and the like are usable for the substrate 2. A flexible substrate is also usable. The flexible substrate, which is a bendable substrate, is exemplified by a plastic substrate. Examples of a material for the plastic substrate include polycarbonate, polyarylate, polyethersulfone, polypropylene, polyester, polyvinyl fluoride, polyvinyl chloride, polyimide, and polyethylene naphthalate. Further, an inorganic vapor deposition film is also usable.

Anode

Metal, an alloy, an electrically conductive compound, a mixture thereof, or the like having a large work function (specifically, 4.0 eV or more) is preferably used as the anode 3 formed on the substrate. Specific examples of the material include indium tin oxide (ITO), indium oxide-tin oxide containing silicon or silicon oxide, indium oxide-zinc oxide, indium oxide containing tungsten oxide and zinc oxide, and graphene. In addition, gold (Au), platinum (Pt), nickel (Ni), tungsten (W), chrome (Cr), molybdenum (Mo), iron (Fe), cobalt (Co), copper (Cu), palladium (Pd), titanium (Ti), and nitrides of a metal material (e.g., titanium nitride) are usable.

The material is typically formed into a film by a sputtering method. For instance, the indium oxide-zinc oxide can be formed into a film by the sputtering method using a target in which zinc oxide in a range from 1 mass% to 10 mass% is added to indium oxide. Moreover, for instance, the indium oxide containing tungsten oxide and zinc oxide can be formed by the sputtering method using a target in which tungsten oxide in a range from 0.5 mass% to 5 mass% and zinc oxide in a range from 0.1 mass% to 1 mass% are added to indium oxide. In addition, the anode may be formed by a vacuum deposition method, a coating method, an inkjet method, a spin coating method or the like.

Among the EL layers formed on the anode, since the hole injecting layer adjacent to the anode is formed of a composite material into which holes are easily injectable irrespective of the work function of the anode, a material usable as an electrode material (e.g., metal, an alloy, an electroconductive compound, a mixture thereof, and the elements belonging to the group 1 or 2 of the periodic table) is also usable for the anode.

A material having a small work function such as elements belonging to Groups 1 and 2 in the periodic table of the elements, specifically, an alkali metal such as lithium (Li) and cesium (Cs), an alkaline earth metal such as magnesium (Mg), calcium (Ca) and strontium (Sr), alloys (e.g., MgAg and AlLi) including the alkali metal or the alkaline earth metal, a rare earth metal such as europium (Eu) and ytterbium (Yb), alloys including the rare earth metal are also usable for the anode. It should be noted that the vacuum deposition method and the sputtering method are usable for forming the anode using the alkali metal, alkaline earth metal and the alloy thereof. Further, when a silver paste is used for the anode, the coating method and the inkjet method are usable.

Cathode

It is preferable to use metal, an alloy, an electroconductive compound, a mixture thereof, or the like having a small work function (specifically, 3.8 eV or less) for the cathode 4. Examples of the material for the cathode include elements belonging to Groups 1 and 2 in the periodic table of the elements, specifically, an alkali metal such as lithium (Li) and cesium (Cs), an alkaline earth metal such as magnesium (Mg), calcium (Ca) and strontium (Sr), alloys (e.g., MgAg and AlLi) including the alkali metal or the alkaline earth metal, a rare earth metal such as europium (Eu) and ytterbium (Yb), and alloys including the rare earth metal.

It should be noted that the vacuum deposition method and the sputtering method are usable for forming the cathode using the alkali metal, alkaline earth metal and the alloy thereof. Further, when a silver paste is used for the cathode, the coating method and the inkjet method are usable.

By providing the electron injecting layer, various conductive materials such as Al, Ag, ITO, graphene, and indium oxide-tin oxide containing silicon or silicon oxide may be used for forming the cathode regardless of the work function. The conductive materials can be formed into a film using the sputtering method, inkjet method, spin coating method, and the like.

Hole Injecting Layer

The hole injecting layer 61 is a layer containing a substance exhibiting a high hole injectability. Examples of the substance exhibiting a high hole injectability include molybdenum oxide, titanium oxide, vanadium oxide, rhenium oxide, ruthenium oxide, chrome oxide, zirconium oxide, hafnium oxide, tantalum oxide, silver oxide, tungsten oxide, and manganese oxide.

In addition, the examples of the substance exhibiting a high hole injectability include: aromatic amine compounds such as 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl (abbreviation: DPAB), 4,4'-bis(N-{4-[N'-(3-methylphenyl)-N'-phenylamino]phenyl}-N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1); and dipyrazino[2,3-f:20,30-h]quinoxaline-2,3,6,7,10,11-hexacarbonitrile (HAT-CN), those of which are low-molecule organic compounds.

In addition, a high polymer compound (e.g., oligomer, dendrimer and polymer) is usable as the substance

exhibiting a high hole injectability. Examples of the high-molecule compound include poly(N-vinylcarbazole) (abbreviation: PVK), poly(4-vinyltriphenylamine) (abbreviation: PVTPA), poly[N-(4-{N'-[4-(4-diphenylam ino)phenyl]phenyl-N'-phenylam ino}phenyl)methacylamide] (abbreviation: PTPDMA), and poly[N,N'-bis(4-butylphenyl)-N,N'-bis(phenyl)benzidine] (abbreviation: Poly-TPD). Moreover, an acid-added high polymer compound such as poly(3,4-ethylenedioxythiophene)/poly(styrene sulfonic acid) (PEDOT/PSS) and polyaniline/poly(styrene sulfonic acid) (PAni/PSS) are also usable.

Hole Transporting Layer

The hole transporting layer 62 is a layer containing a substance exhibiting a high hole transportability. An aromatic amine compound, carbazole derivative, anthracene derivative and the like are usable for the hole transporting layer 62. Specific examples of a material for the hole transporting layer include 4,4'-bis[N-(1-naphthyl)-N-phenylamino]biphenyl (abbreviation: NPB), N,N'-bis(3-methylphenyl)-N,N'-diphenyl-[1,1'-biphenyl]-4,4'-diamine (abbreviation: TPD), 4-phenyl-4'-(9-phenylfluorene-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4''-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB). The above-described substances mostly have a hole mobility of $10^{-6}cm^2/(V·s)$ or more.

For the hole transporting layer 62, a carbazole derivative such as CBP, 9-[4-(N-carbazolyl)]phenyl-10-phenylanthracene (CzPA), and 9-phenyl-3-[4-(10-phenyl-9-anthryl)phenyl]-9H-carbazole (PCzPA) and an anthracene derivative such as t-BuDNA, DNA, and DPAnth may be used. A high polymer compound such as poly(N-vinylcarbazole) (abbreviation: PVK) and poly(4-vinyltriphenylamine) (abbreviation: PVTPA) is also usable.

However, in addition to the above substances, any substance exhibiting a higher hole transportability than an electron transportability may be used. It should be noted that the layer containing the substance exhibiting a high hole transportability may be not only a single layer but also a laminate of two or more layers formed of the above substance(s).

Electron Transporting Layer

The electron transporting layer 71 is a layer containing a substance exhibiting a high electron transportability. For the electron transporting layer 71, 1) a metal complex such as an aluminum complex, beryllium complex, and zinc complex, 2) a hetero aromatic compound such as imidazole derivative, benzimidazole derivative, azine derivative, carbazole derivative, and phenanthroline derivative, and 3) a high polymer compound are usable. Specifically, as a low-molecule organic compound, a metal complex such as Alq, tris(4-methyl-8-quinolinato)aluminum (abbreviation: $Almq_3$), bis(10-hydroxybenzo[h]quinolinato)beryllium (abbreviation: $BeBq_2$), BAlq, Znq, ZnPBO and ZnBTZ is usable. In addition to the metal complex, a heteroaromatic compound such as 2-(4-biphenylyl)-5-(4-tert-butylphenyl)-1,3,4-oxadiazole (abbreviation: PBD), 1,3-bis[5-(ptert-butylphenyl)-1,3,4-oxadiazole-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzoxazole-2-yl)stilbene (abbreviation: BzOs) is usable. In the exemplary embodiment, a benzimidazole compound is suitably usable. The above-described substances mostly have an electron mobility of $10^{-6}$ $cm^2/Vs$ or more. It should be noted that any other substance than the above substances may be used for the electron transporting layer as long as the substance exhibits a higher electron transportability than the hole transportability. The electron transporting layer may be a single layer or a laminate of two or more layers formed of the above substance.

Further, a high polymer compound is usable for the electron transporting layer 71. For instance, poly[(9,9-dihexylfluorene-2,7-diyl)-co-(pyridine-3,5-diyl)] (abbreviation: PF-Py), and poly[(9,9-dioctylfluorene-2,7-diyl)-co-(2,2'-bipyridine-6,6'-diyl)] (abbreviation: PF-BPy) are usable.

Electron Injecting Layer

The electron injecting layer 72 is a layer containing a substance exhibiting a high electron injectability. Examples of a material for the electron injecting layer 72 include an alkali metal, alkaline earth metal and a compound thereof, examples of which include lithium (Li), cesium (Cs), calcium (Ca), lithium fluoride (LiF), cesium fluoride (CsF), calcium fluoride ($CaF_2$), and lithium oxide (LiOx). In addition, the alkali metal, alkaline earth metal or the compound thereof may be added to the substance exhibiting the electron transportability in use. Specifically, for instance, magnesium (Mg) added to Alq may be used. In this case, the electrons can be more efficiently injected from the cathode.

Alternatively, the electron injecting layer 72 may be provided by a composite material in a form of a mixture of the organic compound and the electron donor. Such a composite material exhibits excellent electron injectability and electron transportability since electrons are generated in the organic compound by the electron donor. In this case, the

organic compound is preferably a material excellent in transporting the generated electrons. Specifically, the above examples (e.g., the metal complex and the hetero aromatic compound) of the substance forming the electron transporting layer are usable. As the electron donor, any substance exhibiting electron donating property to the organic compound is usable. Specifically, the electron donor is preferably alkali metal, alkaline earth metal and rare earth metal such as lithium, cesium, magnesium, calcium, erbium and ytterbium. The electron donor is also preferably alkali metal oxide and alkaline earth metal oxide such as lithium oxide, calcium oxide, and barium oxide. Moreover, a Lewis base such as magnesium oxide is usable. Further, the organic compound such as tetrathiafulvalene (abbreviation: TTF) is usable.

Layer Formation Method(s)

A method of forming each layer of the organic EL device in the exemplary embodiment is subject to no limitation except for the above particular description. However, known methods of dry film-forming such as vacuum deposition, sputtering, plasma or ion plating and wet film-forming such as spin coating, dipping, flow coating or ink-jet are applicable.

Film Thickness

The film thickness of each layer of the organic layer of the organic EL device in the exemplary embodiment is not limited unless otherwise specified in the above. In general, the thickness preferably ranges from several nanometers to 1 $\mu$m because an excessively small film thickness is likely to cause defects (e.g. pin holes) and an excessively large thickness leads to the necessity of applying high voltage and consequent reduction in efficiency.

According to the exemplary embodiment, an organic electroluminescence device with an improved lifetime can be provided.

Fifth Exemplary Embodiment

Electronic Device

An electronic device according to a fifth exemplary embodiment is installed with the organic EL device according to any one of the above exemplary embodiments. Examples of the electronic device include a display device and a light-emitting unit. Examples of the display device include a display component (e.g., an organic EL panel module), TV, mobile phone, tablet and personal computer. Examples of the light-emitting unit include an illuminator and a vehicle light.

Modification of Embodiments

The scope of the invention is not limited by the above exemplary embodiments but includes any modification and improvement as long as such modification and improvement are compatible with the invention.

For instance, the number of emitting layers is not limited to one or two, and more than two emitting layers may be layered. When the organic EL device includes more than two emitting layers, for instance, any other emitting layer(s) than the emitting layers described in the above exemplary embodiments may be a fluorescent emitting layer or a phosphorescent emitting layer with use of emission caused by electron transfer from the triplet excited state directly to the ground state.

When the organic EL device includes a plurality of emitting layers, these emitting layers may be mutually adjacently provided, or may form a so-called tandem organic EL device, in which a plurality of emitting units are layered via an interposed layer.

For instance, a blocking layer may be provided adjacent to at least one of a side of the emitting layer close to the anode or a side of the emitting layer close to the cathode. The blocking layer is preferably provided in contact with the emitting layer to block at least one of holes, electrons, or excitons.

For instance, when the blocking layer is provided in contact with the side of the emitting layer close to the cathode, the blocking layer permits transport of electrons, and blocks holes from reaching a layer provided closer to the cathode (e.g., the electron transporting layer) beyond the blocking layer. When the organic EL device includes the electron transporting layer, the blocking layer is preferably interposed between the emitting layer and the electron transporting layer.

When the blocking layer is provided in contact with the side of the emitting layer close to the anode, the blocking layer permits transport of holes and blocks electrons from reaching a layer provided closer to the anode (e.g., the hole transporting layer) beyond the blocking layer. When the organic EL device includes the hole transporting layer, the blocking layer is preferably interposed between the emitting layer and the hole transporting layer.

Alternatively, the blocking layer may be provided adjacent to the emitting layer so that the excitation energy does not leak out from the emitting layer toward neighboring layer(s). The blocking layer blocks excitons generated in

the emitting layer from being transferred to a layer(s) (e.g., the electron transporting layer and the hole transporting layer) closer to the electrode(s) beyond the blocking layer.

The emitting layer is preferably bonded with the blocking layer.

The specific structure, shape, and the like of the components in the invention may be designed in any manner as long as an object of the invention can be achieved. Examples

The invention will be described in further detail with reference to Examples. The scope of the invention is by no means limited to Examples.

Compounds

Structures of the compound represented by the formula (1C) and the compound represented by the formula (1C-B) used for producing organic EL devices in Examples are given below.

[Formula 280]

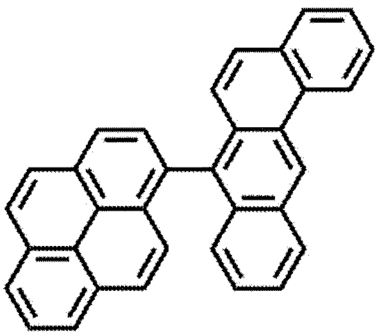

BH1-1

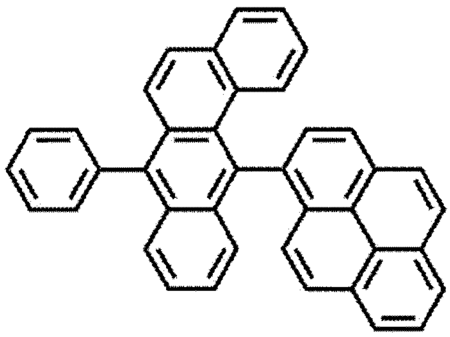

BH1-3

[Formula 281]

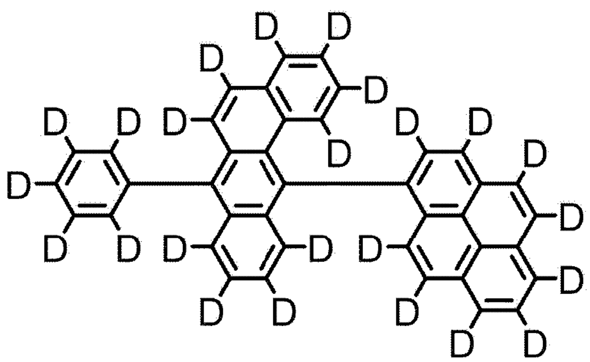

BH1-4

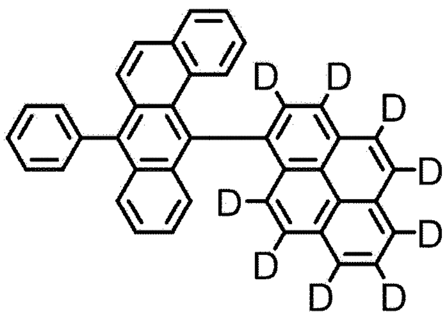

BH1-5

Structures of the compound represented by the formula (1C), the compound represented by the formula (1C-A), and the compound represented by the formula (1C-B) used for producing organic EL devices in Examples are given below.

[Formula 282]

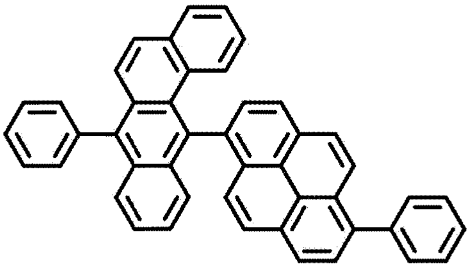

BH1-6

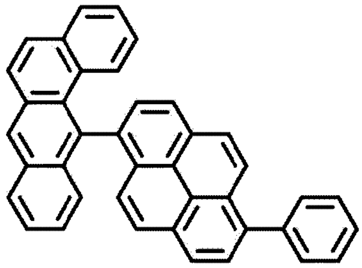

BH1-7

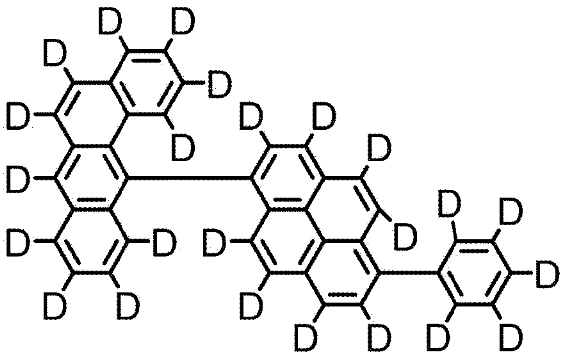

BH1-8

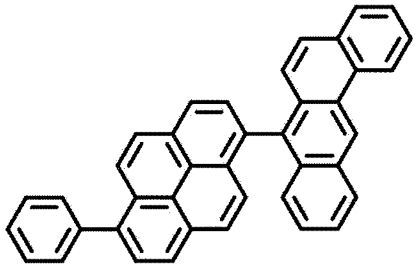

BH1-9

Structures of the compound represented by the formula (1C), the compound represented by the formula (1C-B), and the compound represented by the formula (1C-C) used for producing organic EL devices in Examples are given below.

[Formula 283]

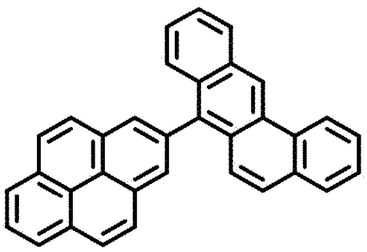

BH1-10

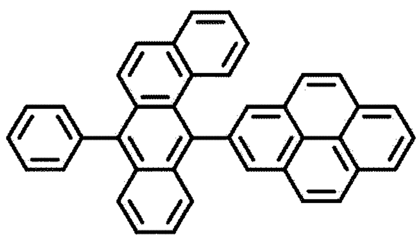

BH1-11

A Structure of a comparative compound used for producing organic EL devices in Comparatives is given below.

[Formula 284]

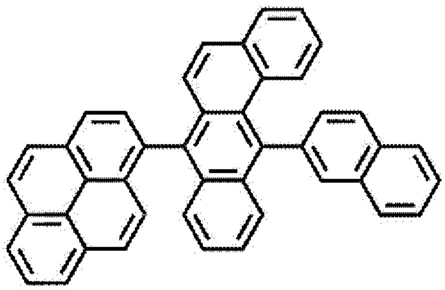

BH1-Ref1

Structures of other compounds used for producing organic EL devices in Examples and Comparatives are given below.

[Formula 285]

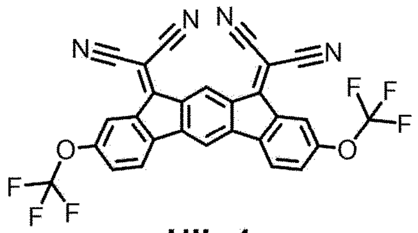

HIL-1

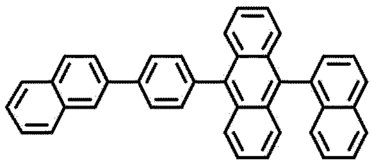

BH-2

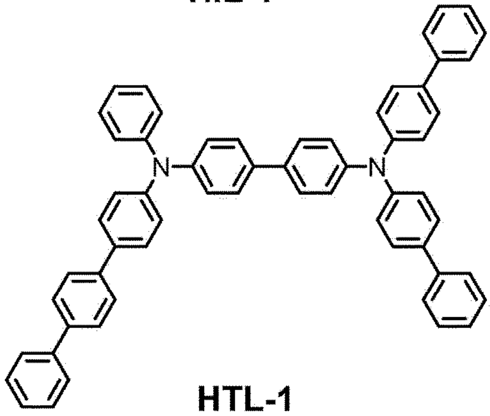

HTL-1

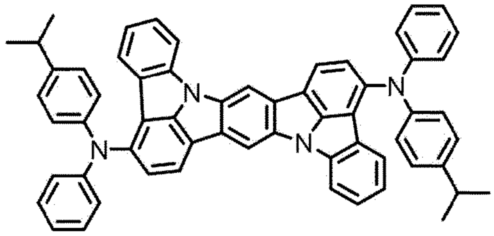

BD-1

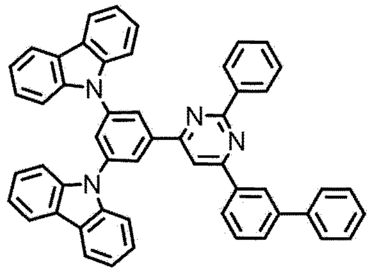

aET-1

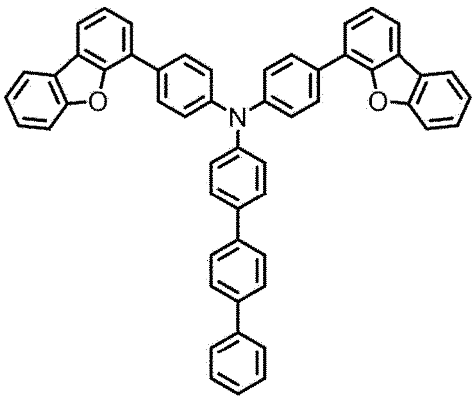

EBL-1

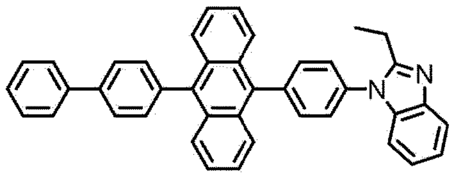

bET-1

[Formula 286]

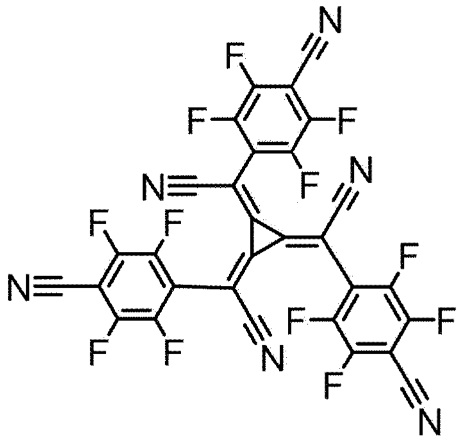

HIL-2

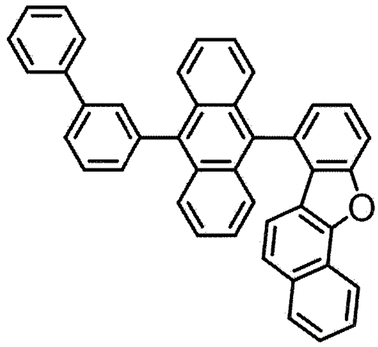

BH-2-4

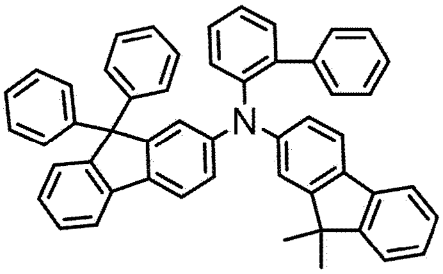

HTL-2

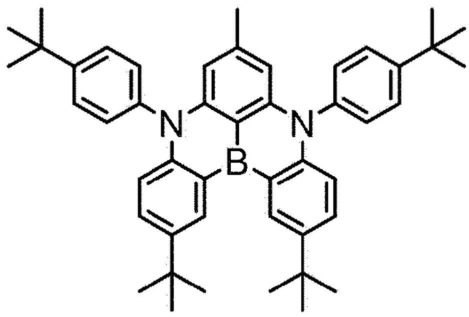

BD-2

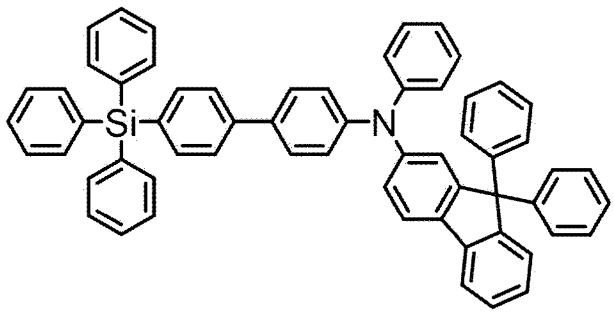

EBL-2

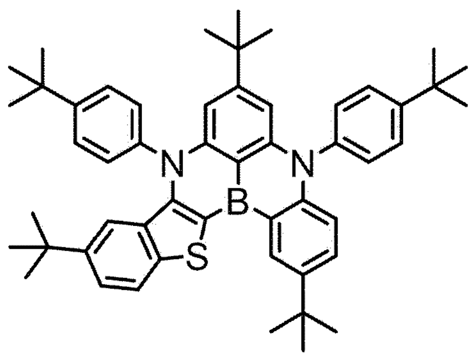

BD-3

[Formula 287]

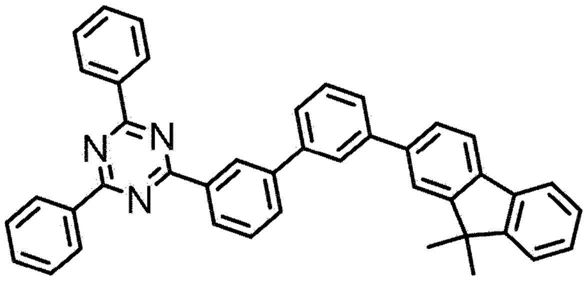

aET-2

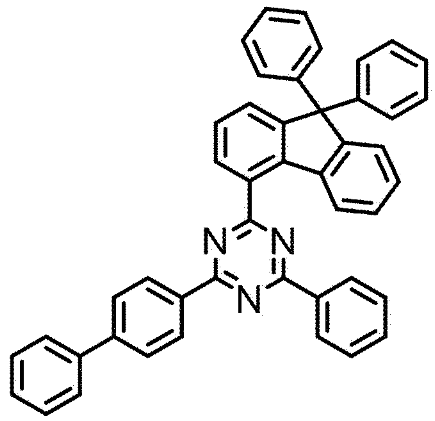

bET-2

Production of Organic EL Device

The organic EL devices were produced and evaluated as follows.

Example 1-1

A glass substrate (size: 25 mm × 75 mm × 1.1 mm thick, produced by Geomatec Co., Ltd.) having an ITO (indium tin oxide) transparent electrode (anode) was ultrasonic-cleaned in isopropyl alcohol for five minutes, and then UV-ozone-cleaned for 30 minutes. The film thickness of the ITO transparent electrode was 130 nm.

After the glass substrate having the transparent electrode line was cleaned, the glass substrate was mounted on a substrate holder of a vacuum deposition apparatus. Firstly, a compound HIL-1 was vapor-deposited on a surface of the glass substrate where the transparent electrode line was provided in a manner to cover the transparent electrode, thereby forming a 5-nm-thick hole injecting layer (HI).

After forming the hole injecting layer, a compound HTL-1 was vapor-deposited on the hole injecting layer to form an 80-nm-thick first hole transporting layer.

After forming the first hole transporting layer, a compound EBL-1 was vapor-deposited on the first hole transporting layer to form a 10-nm-thick second hole transporting layer (also referred to as an electron blocking layer).

A compound BH1-1 as the first compound and a compound BD-1 as the first luminescent compound were co-deposited on the second hole transporting layer such that the ratio of the compound BH1-1 accounted for 98 mass% and the ratio of the compound BD-1 accounted for 2 mass%, thereby forming a 12.5-nm-thick first emitting layer.

A compound BH-2 as the second compound and the compound BD-1 as the second luminescent compound were co-deposited on the first emitting layer such that the ratio of the compound BH-2 accounted for 98 mass% and the ratio of the compound BD-1 accounted for 2 mass%, thereby forming a 12.5-nm-thick second emitting layer.

A compound aET-1 was vapor-deposited on the second emitting layer to form a 10-nm-thick first electron transporting layer (also referred to as a hole blocking layer).

A compound bET-1 was vapor-deposited on the first electron transporting layer to form a 15-nm-thick second electron transporting layer.

LiF was vapor-deposited on the second electron transporting layer to form a 1-nm-thick electron injecting layer.

Metal Al was vapor-deposited on the electron injecting layer to form an 80-nm-thick cathode.

A device arrangement of the organic EL device in Example 1-1 is roughly shown as follows.

ITO(130)/HIL-1 (5)/HTL-1 (80)/EBL-1 (10)/BH1-1 :BD-1 (12.5,98%:2%)/BH-2:BD-1(12.5,98%:2%)/aET-1(10)/bET-1(15)/LiF(1)/Al(80)

Numerals in parentheses represent a film thickness (unit: nm).

The numerals (98%:2%) represented by percentage in the same parentheses indicate a ratio (mass%) between the compound BH1-1 or the compound BH-2 and the compound BD-1 in the first emitting layer or the second emitting layer.

Example 1-2 to Example 1-10

Organic EL devices in Examples 1-2 to 1-10 were each produced as in Example 1-1 except that the first compound of the first emitting layer was replaced with compounds listed in Table 1.

Comparative 1-1

An organic EL device in Comparative 1-1 was produced as in Example 1-1 except that the first compound of the first emitting layer was replaced with a compound listed in Table 1.

Example 2-1

A glass substrate (size: 25 mm × 75 mm × 1.1 mm thick, produced by Geomatec Co., Ltd.) having an ITO (indium tin oxide) transparent electrode (anode) was ultrasonic-cleaned in isopropyl alcohol for five minutes, and then UV-ozone-cleaned for 30 minutes. The film thickness of the ITO transparent electrode was 130 nm.

After the glass substrate having the transparent electrode line was cleaned, the glass substrate was mounted on a substrate holder of a vacuum deposition apparatus. Firstly, a compound HTL-2 and a compound HIL-2 were co-deposited on a surface of the glass substrate where the transparent electrode line was provided in a manner to cover the transparent electrode such that the ratio of the compound HTL-2 accounted for 90 mass% and the ratio of the compound HIL-2 accounted for 10 mass%, thereby forming a 10-nm-thick hole injecting layer (HI).

After forming the hole injecting layer, the compound HTL-2 was vapor-deposited thereon to form an 85-nm-thick first hole transporting layer.

After forming the first hole transporting layer, a compound EBL-2 was vapor-deposited thereon to form a 5-nm-thick second hole transporting layer (also referred to as an electron blocking layer).

The compound BH1-1 as the first compound and a compound BD-2 as the first luminescent compound were co-deposited on the second hole transporting layer such that the ratio of the compound BH1-1 accounted for 98 mass% and the ratio of the compound BD-2 accounted for 2 mass%, thereby forming a 10-nm-thick first emitting layer.

A compound BH-2-4 as the second compound and the compound BD-2 as the second luminescent compound were co-deposited on the first emitting layer such that the ratio of the compound BH-2-4 accounted for 98 mass% and the ratio of the compound BD-2 accounted for 2 mass%, thereby forming a 10-nm-thick second emitting layer.

A compound aET-2 was vapor-deposited on the second emitting layer to form a 5-nm-thick first electron transporting layer (also referred to as a hole blocking layer).

A compound bET-2 and a compound Liq were co-deposited on the first electron transporting layer such that the ratio of the compound bET-2 accounted for 50 mass% and the ratio of the compound Liq accounted for 50 mass%, thereby forming a 25-nm-thick electron transporting layer. Liq is an abbreviation of (8-quinolinolato)lithium ((8-Quinolinolato)lithium).

Liq was vapor-deposited on the second electron transporting layer to form a 1-nm-thick electron injecting layer.

Metal Al was vapor-deposited on the electron injecting layer to form an 80-nm-thick cathode.

A device arrangement of the organic EL device in Example 2-1 is roughly shown as follows.

ITO(130)/HTL-2:HIL-2(10,90%:1 0%)/HTL-2(85)/EBL-2(5)/BH1 -1 :BD-2(10,98%:2%)/BH-2-4:BD-2(10,98%:2%)/aET-2(5)/bET-2:Liq(25,50%:50%)/Liq(1)/Al(80)

Numerals in parentheses represent a film thickness (unit: nm).

The numerals (90%:10%) represented by percentage in the same parentheses indicate a ratio (mass%) between the compound HTL-2 and the compound HIL-2 (mass%).

The numerals (98%:2%) represented by percentage in the same parentheses indicate a ratio (mass%) between the compound BH1-1 or the compound BH-2-4 and the compound BD-2 in the first emitting layer or the second emitting layer.

Example 2-2 to Example 2-4

Organic EL devices in Examples 2-2 to 2-4 were each produced as in Example 2-1 except that the first compound of the first emitting layer was replaced with compounds listed in Table 2.

Comparative 2-1

An organic EL device in Comparative 2-1 was produced as in Example 2-1 except that the first compound of the first emitting layer was replaced with a compound listed in Table 2.

Example 3-1

A glass substrate (size: 25 mm × 75 mm × 1.1 mm thick, produced by Geomatec Co., Ltd.) having an ITO (indium tin oxide) transparent electrode (anode) was ultrasonic-cleaned in isopropyl alcohol for five minutes, and then UV-ozone-cleaned for 30 minutes. The film thickness of the ITO transparent electrode was 130 nm.

After the glass substrate having the transparent electrode line was cleaned, the glass substrate was mounted on a substrate holder of a vacuum deposition apparatus. Firstly, the compound HTL-2 and the compound HIL-2 were co-deposited on a surface of the glass substrate where the transparent electrode line was provided in a manner to cover the transparent electrode such that the ratio of the compound HTL-2 accounted for 90 mass% and the ratio of the compound HIL-2 accounted for 10 mass%, thereby forming a 10-nm-thick hole injecting layer (HI).

After forming the hole injecting layer, the compound HTL-2 was vapor-deposited thereon to form an 85-nm-thick first hole transporting layer.

After forming the first hole transporting layer, the compound EBL-2 was vapor-deposited thereon to form a 5-nm-thick second hole transporting layer (also referred to as an electron blocking layer).

The compound BH1-1 as the first compound and a compound BD-3 as the first luminescent compound were co-deposited on the second hole transporting layer such that the ratio of the compound BH1-1 accounted for 98 mass% and the ratio of the compound BD-3 accounted for 2 mass%, thereby forming a 10-nm-thick first emitting layer.

The compound BH-2-4 as the second compound and the compound BD-3 as the second luminescent compound were co-deposited on the first emitting layer such that the ratio of the compound BH-2-4 accounted for 98 mass% and the ratio of the compound BD-3 accounted for 2 mass%, thereby forming a 10-nm-thick second emitting layer.

The compound aET-2 was vapor-deposited on the second emitting layer to form a 5-nm-thick first electron transporting layer (also referred to as a hole blocking layer).

The compound bET-2 and the compound Liq were co-deposited on the first electron transporting layer such that the ratio of the compound bET-2 accounted for 50 mass% and the ratio of the compound Liq accounted for 50 mass%, thereby forming a 25-nm-thick electron transporting layer.

Liq was vapor-deposited on the second electron transporting layer to form a 1-nm-thick electron injecting layer.

Metal Al was vapor-deposited on the electron injecting layer to form an 80-nm-thick cathode.

A device arrangement of the organic EL device in Example 3-1 is roughly shown as follows.

ITO(130)/HTL-2:HIL-2(10,90%:1 0%)/HTL-2(85)/EBL-2(5)/BH1 -1 :BD-3(10,98%:2%)/BH-2-4:BD-3(10,98%:2%)/aET-2(5)/bET-2:Liq(25,50%:50%)/Liq(1)/Al(80)

Numerals in parentheses represent a film thickness (unit: nm).

The numerals (90%:10%) represented by percentage in the same parentheses indicate a ratio (mass%) between the compound HTL-2 and the compound HIL-2 (mass%).

The numerals (98%:2%) represented by percentage in the same parentheses indicate a ratio (mass%) between the compound BH1-1 or the compound BH-2-4 and the compound BD-3 in the first emitting layer or the second emitting layer.

Example 3-2 to Example 3-4

Organic EL devices in Examples 3-2 to 3-4 were each produced as in Example 3-1 except that the first compound of the first emitting layer was replaced with compounds listed in Table 3.

Comparative 3-1

An organic EL device in Comparative 3-1 was produced as in Example 3-1 except that the first compound of the first emitting layer was replaced with a compound listed in Table 3.

Evaluation of Organic EL Devices

The organic EL devices produced in Examples 1-1 to 1-10, Examples 2-1 to 2-4, Examples 3-1 to 3-4, and Comparatives 1-1, 2-1, and 3-1 were evaluated as follows. Tables 1, 2, and 3 show evaluation results.

Lifetime (LT95)

Voltage was applied to the produced organic EL device such that a current density was 50 mA/$cm^2$, where a time (LT95 (unit: hr)) elapsed before a luminance intensity was reduced to 95% of the initial luminance intensity was measured. The luminance intensity was measured by using a spectroradiometer CS-2000 (produced by Konica Minolta, Inc.).

LT95 (relative value) (unit: %), which was calculated based on the measurement value of LT95 in each Example (Examples 1-1 to 1-10 and Comparative 1-1) according to a numerical formula (Numerical Formula 1X) below, is shown in Table 1.

LT95 (relative value) = (LT95 of each Example / LT95 of Comparative 1-1) × 100 (Numerical Formula 1X)

LT95 (relative value) (unit: %), which was calculated based on the measurement value of LT95 in each Example (Examples 2-1 to 2-4 and Comparative 2-1) according to a numerical formula (Numerical Formula 2X) below, is shown in Table 2.

LT95 (relative value) = (LT95 of each Example / LT95 of Comparative 2-1) × 100 (Numerical Formula 2X)

LT95 (relative value) (unit: %), which was calculated based on the measurement value of LT95 in each Example (Examples 3-1 to 3-4 and Comparative 3-1) according to a numerical formula (Numerical Formula 3X) below, is shown in Table 3.

LT95 (relative value) = (LT95 of each Example / LT95 of Comparative 3-1) × 100 (Numerical Formula 3X)

Table 1

| | First emitting layer | | | | | | | Second emitting layer | | | | | Device evaluation |
|---|---|---|---|---|---|---|---|---|---|---|---|---|---|
| | First compound | | | First luminescent compound | | | Film thickness [nm] | Second compound | | | Second luminescent compound | Film thickness [nm] | LT95 (Relative value) [%] |
| | Name | $S_1$ [eV] | $T_1$ [eV] | Name | $S_1$ [eV] | $T_1$ [eV] | | Name | $S_1$ [eV] | $T_1$ [eV] | Name | | |
| Ex. 1-1 | BH1-1 | 3.07 | 2.07 | BD-1 | 2.73 | 2.29 | 12.5 | BH-2 | 3.01 | 1.87 | BD-1 | 12.5 | 380 |
| Ex. 1-2 | BH1-3 | 3.02 | 2.05 | BD-1 | 2.73 | 2.29 | 12.5 | BH-2 | 3.01 | 1.87 | BD-1 | 12.5 | 700 |
| Ex. 1-3 | BH1-4 | 3.02 | 2.05 | BD-1 | 2.73 | 2.29 | 12.5 | BH-2 | 3.01 | 1.87 | BD-1 | 12.5 | 750 |
| Ex. 1-4 | BH1-5 | 3.02 | 2.05 | BD-1 | 2.73 | 2.29 | 12.5 | BH-2 | 3.01 | 1.87 | BD-1 | 12.5 | 730 |
| Ex. 1-5 | BH1-6 | 3.01 | 204 | BD-1 | 2.73 | 2.29 | 12.5 | BH-2 | 3.01 | 1.87 | BD-1 | 12.5 | 770 |
| Ex. 1-6 | BH1-7 | 3.02 | 2.05 | BD-1 | 2.73 | 2.29 | 12.5 | BH-2 | 3.01 | 1.87 | BD-1 | 12.5 | 700 |
| Ex. 1-7 | BH1-8 | 3.02 | 2.05 | BD-1 | 2.73 | 2.29 | 12.5 | BH-2 | 3.01 | 1.87 | BD-1 | 12.5 | 740 |
| Ex. 1-8 | BH1-9 | 3.07 | 2.06 | BD-1 | 2.73 | 2.29 | 12.5 | BH-2 | 3.01 | 1.87 | BD-1 | 12.5 | 600 |
| Ex. 1-9 | BH1-10 | 3.11 | 2.08 | BD-1 | 2.73 | 2.29 | 12.5 | BH-2 | 3.01 | 1.87 | BD-1 | 12.5 | 400 |
| Ex. 1-10 | BH1-11 | 3.04 | 2.06 | BD-1 | 2.73 | 2.29 | 12.5 | BH-2 | 3.01 | 1.87 | BD-1 | 12.5 | 410 |
| Comp. 1-1 | BH1-Ref1 | 3.04 | 2.05 | BD-1 | 2.73 | 2.29 | 12.5 | BH-2 | 3.01 | 1.87 | BD-1 | 125 | 100 |

Table 2

| | First emitting layer | | | | | | | Second emitting layer | | | | | Device evaluation |
|---|---|---|---|---|---|---|---|---|---|---|---|---|---|
| | First compound | | | First luminescent compound | | | Film thickness [nm] | Second compound | | | Second luminescent compound | Film thickness [nm] | LT95 (Relative value) [%] |
| | Name | $S_1$ [eV] | $T_1$ [eV] | Name | $S_1$ [eV] | $T_1$ [eV] | | Name | $S_1$ [eV] | $T_1$ [eV] | Name | | |
| Ex. 2-1 | BH1-1 | 3.07 | 2.07 | BD-2 | 2.71 | 2.64 | 10 | BH-2-4 | 3.01 | 1.87 | BD-2 | 10 | 320 |
| Ex. 2-2 | BH1-3 | 3.02 | 2.05 | BD-2 | 2.71 | 2.64 | 10 | BH-2-4 | 3.01 | 1.87 | BD-2 | 10 | 640 |
| Ex. 2-3 | BH1-8 | 3.02 | 2.05 | BD-2 | 2.71 | 2.64 | 10 | BH-2-4 | 3.01 | 1.87 | BD-2 | 10 | 660 |
| Ex. 2-4 | BH1-11 | 3.04 | 2.06 | BD-2 | 2.71 | 2.64 | 10 | BH-2-4 | 3.01 | 1.87 | BD-2 | 10 | 350 |
| Comp. 2-1 | BH1-Ref1 | 3.04 | 2.05 | BD-2 | 2.71 | 2.64 | 10 | BH-2-4 | 3.01 | 1.87 | BD-2 | 10 | 100 |

Table 3

| | First emitting layer | | | | | | | Second emitting layer | | | | | Device evaluation |
|---|---|---|---|---|---|---|---|---|---|---|---|---|---|
| | First compound | | | First luminescent compound | | | Film thickness | Second compound | | | Second luminescent compound | Film thickness [nm] | LT95 (Relative value) [%] |
| | Name | $S_1$ [eV] | $T_1$ [eV] | Name | $S_1$ [eV] | $T_1$ [eV] | [nm] | Name | $S_1$ [eV] | $T_1$ [eV] | Name | | |
| Ex. 3-1 | BH1-1 | 3.07 | 2.07 | BD-3 | 2.80 | 2.45 | 10 | BH-2-4 | 3.01 | 1.87 | BD-3 | 10 | 340 |
| Ex. 3-2 | BH1-3 | 3.02 | 2.05 | BD-3 | 2.80 | 2.45 | 10 | BH-2-4 | 3.01 | 1.87 | BD-3 | 10 | 600 |
| Ex. 3-3 | BH1-8 | 3.02 | 2.05 | BD-3 | 2.80 | 2.45 | 10 | BH-2-4 | 3.01 | 1.87 | BD-3 | 10 | 620 |
| Ex. 3-4 | BH1-11 | 3.04 | 2.06 | BD-3 | 2.80 | 2.45 | 10 | BH-2-4 | 3.01 | 1.87 | BD-3 | 10 | 410 |
| Comp. 3-1 | BH1-Ref1 | 3.04 | 2.05 | BD-3 | 2.80 | 2.45 | 10 | BH-2-4 | 3.01 | 1.87 | BD-3 | 10 | 100 |

The organic EL devices in Examples 1-1 to 3-4 using the compound represented by the formula (1C-A), the compound represented by the formula (1C-C), and the compound represented by the formula (1C) had a longer lifetime than the organic EL devices in Comparatives 1-1 to 3-1.

## Evaluation on Compounds

### Triplet Energy $T_1$

A measurement target compound was dissolved in EPA (diethylether : isopentane : ethanol = 5:5:2 in volume ratio) at a concentration of 10 $\mu$mol/L, and the obtained solution was put in a quartz cell to provide a measurement sample. A phosphorescence spectrum (ordinate axis: phosphorescent luminous intensity, abscissa axis: wavelength) of the measurement sample was measured at a low temperature (77K). A tangent is drawn to the rise of the phosphorescence spectrum close to the short-wavelength region. An energy amount is calculated by a conversion equation (F1) below on a basis of a wavelength value $\lambda_{edge}$ [nm] at an intersection of the tangent and the abscissa axis. The calculated energy amount was defined as triplet energy $T_1$. Tables 1 and 2 show the results.

It should be noted that the triplet energy $T_1$ may have an error of about plus or minus 0.02 eV depending on measurement conditions.

$$\text{Conversion Equation (F1): } T_1 \text{ [eV]} = 1239.85/\lambda_{edge}$$

The tangent to the rise of the phosphorescence spectrum close to the short-wavelength region is drawn as follows. While moving on a curve of the phosphorescence spectrum from the short-wavelength region to the local maximum value closest to the short-wavelength region among the local maximum values of the phosphorescence spectrum, a tangent is checked at each point on the curve toward the long-wavelength of the phosphorescence spectrum. An inclination of the tangent is increased along the rise of the curve (i.e., a value of the ordinate axis is increased). A tangent drawn at a point of the local maximum inclination (i.e., a tangent at an inflection point) is defined as the tangent to the rise of the phosphorescence spectrum close to the short-wavelength region.

A local maximum point where a peak intensity is 15% or less of the maximum peak intensity of the spectrum is not counted as the above-mentioned local maximum peak intensity closest to the short-wavelength region. The tangent drawn at a point that is closest to the local maximum peak intensity closest to the short-wavelength region and where the inclination of the curve is the local maximum is defined as a tangent to the rise of the phosphorescence spectrum close to the short-wavelength region.

For phosphorescence measurement, a spectrophotofluorometer body F-4500 manufactured by Hitachi High-Technologies Corporation was used.

### Singlet Energy $S_1$

A toluene solution of a measurement target compound at a concentration of 10 $\mu$mol/L was prepared and put in a quartz cell. An absorption spectrum (ordinate axis: absorption intensity, abscissa axis: wavelength) of the thus-obtained sample was measured at a normal temperature (300K). A tangent was drawn to the fall of the absorption spectrum close to the long-wavelength region, and a wavelength value $\lambda_{edge}$ (nm) at an intersection of the tangent and the abscissa axis was assigned to a conversion equation (F2) below to calculate singlet energy $S_1$. Tables 1 and 2 show the results.

$$\text{Conversion Equation (F2): } S_1 \text{ [eV]} = 1239.85/\lambda edge$$

A spectrophotometer (U3310 manufactured by Hitachi, Ltd.) was used for measuring absorption spectrum.

The tangent to the fall of the absorption spectrum close to the long-wavelength region is drawn as follows. While moving on a curve of the absorption spectrum from the local maximum value closest to the long-wavelength region, among the local maximum values of the absorption spectrum, in a long-wavelength direction, a tangent at each point on the curve is checked. An inclination of the tangent is decreased and increased in a repeated manner as the curve falls (i.e., a value of the ordinate axis is decreased). A tangent drawn at a point where the inclination of the curve is the local minimum closest to the long-wavelength region (except when absorbance is 0.1 or less) is defined as the tangent to the fall of the absorption spectrum close to the long-wavelength region.

The local maximum absorbance of 0.2 or less is not counted as the above-mentioned local maximum absorbance closest to the long-wavelength region.

Maximum Peak Wavelength of Compounds

A maximum peak wavelength λ of each compound was measured as follows.

A toluene solution of a measurement target compound at a concentration of 5 μmol/L was prepared and put in a quartz cell. An emission spectrum (ordinate axis: luminous intensity, abscissa axis: wavelength) of each of the samples was measured at a normal temperature (300K). In Examples, an emission spectrum was measured with a spectrophotofluorometer (produced by Hitachi High-Tech Science Corporation: F-7000). It should be noted that the apparatus for measuring the emission spectrum is not limited to the apparatus used herein. A peak wavelength of the emission spectrum exhibiting the maximum luminous intensity was defined as the maximum peak wavelength λ.

The maximum peak wavelength λ of the compound BD-1 was 452 nm.

The maximum peak wavelength λ of the compound BD-2 was 455 nm.

The maximum peak wavelength λ of the compound BD-3 was 457 nm.

Synthesis of Compound

Synthesis Example 1: Synthesis of Compound BH1-1

The compound BH1-1 was synthesized through a synthesis pathway below.

[Formula 288]

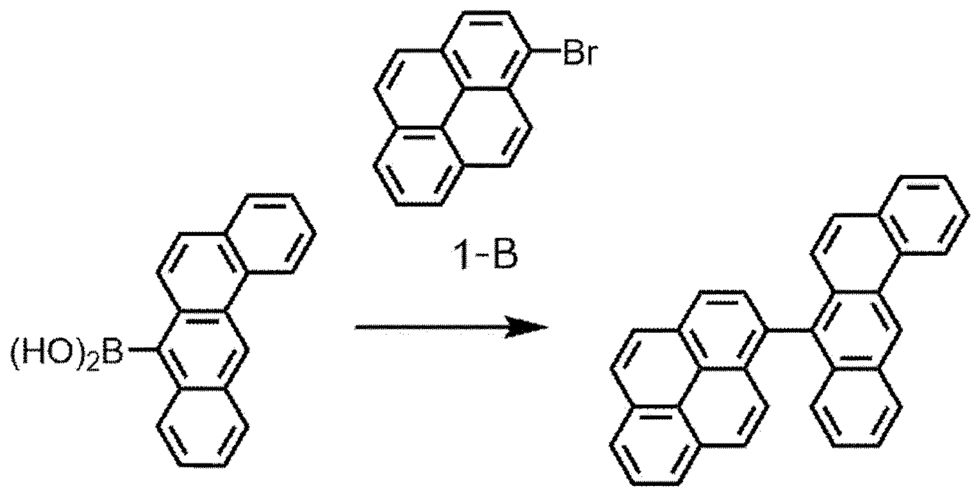

1-B

1-A

BH1-1

Synthesis of Compound BH1-1

Under an argon atmosphere, 5.44 g (20.0 mmol) of an intermediate 1-A, 5.62 g (20.0 mmol) of an intermediate 1-B, 0.46 g (0.4 mmol) of dichlorobisamphospalladium (II), 30.0 ml (60.0 mmol) of 2M sodium carbonate aqueous solution, and 100 ml of 1,2-dimethoxyethane were put into a flask and heated to reflux with stirring for eight hours. After stirring, the reaction solution was cooled to a room temperature (25 degrees C) and the solvent was distilled off. The obtained solid was purified by silica-gel column chromatography to obtain 5.4 g (a yield of 63%) of a white solid.

The white solid was identified as the compound BH1-1 by analysis according to liquid chromatography-mass spectrometry (LC-MS).

Synthesis Example 2: Synthesis of Compound BH1-3

A compound BH1-3 was synthesized through a synthesis pathway below.

[Formula 289]

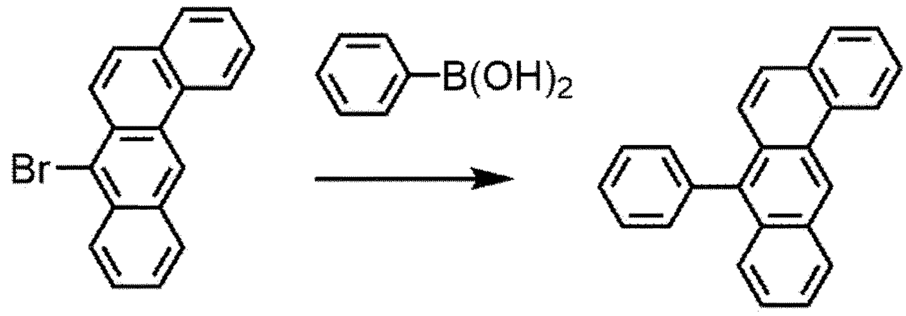

3-A 3-B

Synthesis of Intermediate 3-B

An intermediate 3-B was synthesized as in the compound BH1-1 except that the intermediate 1-A and the intermediate 1-B in the synthesis of the compound BH1-1 were replaced with an intermediate 3-A and phenylboronic acid, thereby obtaining 7.0 g (a yield of 87%) of a white solid.

The white solid was identified as the intermediate 3-B by analysis according to liquid chromatography-mass spectrometry (LC-MS).

[Formula 290]

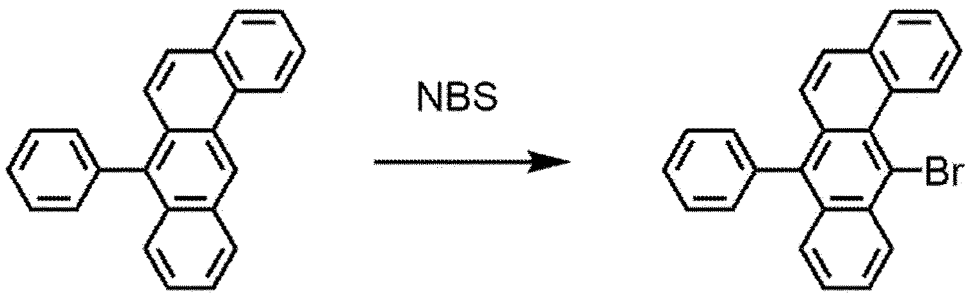


3-B 3-C

Synthesis of Intermediate 3-C

Under an argon atmosphere, 7.0 g (23.0 mmol) of the intermediate 3-B, 4.1 g (NBS, 23.0 mmol) of N-bromosuccinimide, and 115 ml of dichloromethane were put into a flask and heated to reflux with stirring for 48 hours. After the reaction solution was cooled to a room temperature (25 degrees C), the solvent was distilled off. The obtained residue was purified by silica-gel column chromatography to obtain 5.6 g (a yield of 64%) of a white solid.

The white solid was identified as an intermediate 3-C by analysis according to liquid chromatography-mass spectrometry (LC-MS).

[Formula 291]

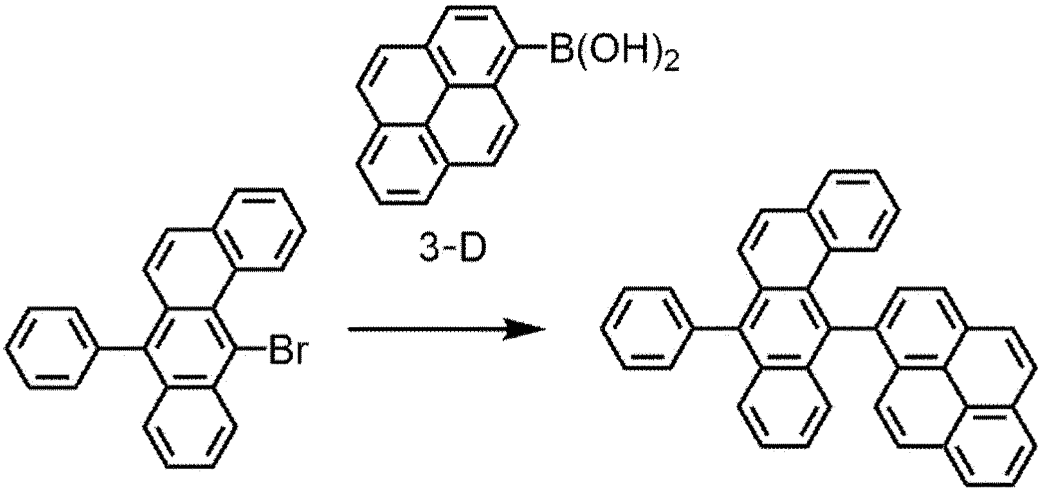

3-D

3-C

BH1-3

Synthesis of Compound BH1-3

The compound BH1-3 was synthesized as in the compound BH1-1 except that the intermediate 1-A and the intermediate 1-B in the synthesis of the compound BH1-1 were replaced with the intermediate 3-C and an intermediate 3-D, thereby obtaining 0.6 g (a yield of 25%) of a light yellow solid.

The light yellow solid was identified as the compound BH1-3 by analysis according to liquid chromatography-mass spectrometry (LC-MS).

Synthesis Example 3: Synthesis of Compound BH1-4

A compound BH1-4 was synthesized through a synthesis pathway below.

[Formula 292]

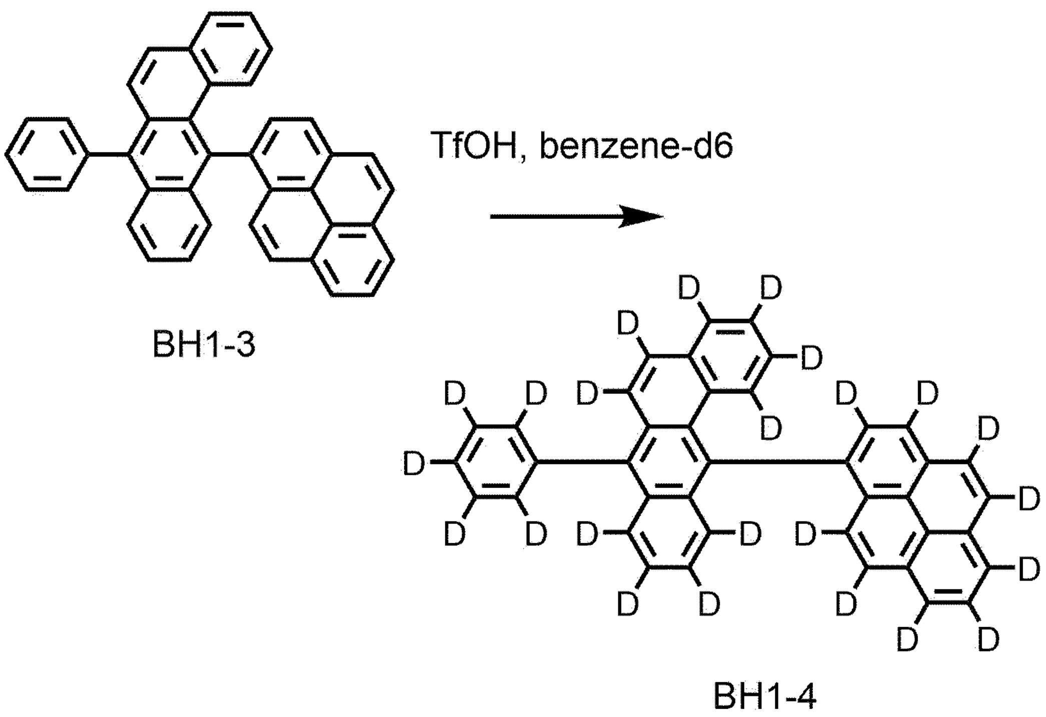


Synthesis of Compound BH1-4

Under an argon atmosphere, 5.7 g (11.3 mmol) of the compound BH1-3 and 100 ml of ortho-dichlorobenzene were put into a flask and completely dissolved with stirring at room temperature. Then, 50 ml of benzene-d6 was added thereto and the solution was stirred at 10 degrees C for five minutes. After that, 1.99 ml (22.5 mmol) of trifluoromethanesulfonic acid was added thereto, and the solution was stirred at 10 degrees C for two hours. 200 ml of heavy water was then added thereto, and the solution was stirred for further 15 minutes. After stirring, a water layer was removed and the remaining organic layer was concentrated. The obtained solid was purified by silica gel column chromatography to obtain 0.5 g (a yield of 24%) of a light yellow solid.

The light yellow solid was identified as the compound BH1-4 by analysis according to liquid chromatography-mass spectrometry (LC-MS).

Synthesis Example 4: Synthesis of Compound BH1-5

A compound BH1-5 was synthesized through a synthesis pathway below.

[Formula 293]

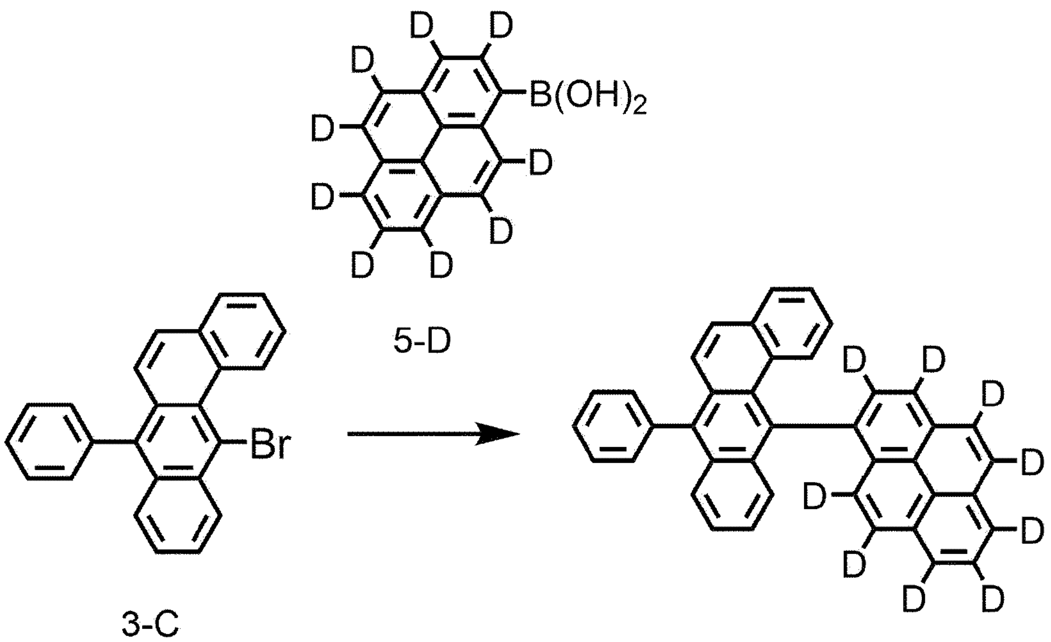

5-D

3-C

BH1-5

Synthesis of Compound BH1-5

The compound BH1-5 was synthesized as in the compound BH1-1 except that the intermediate 1-A and the intermediate 1-B in the synthesis of the compound BH1-1 were replaced with the intermediate 3-C and an intermediate 5-D, thereby obtaining 0.9 g (a yield of 47%) of a light yellow solid.

The light yellow solid was identified as the compound BH1-5 by analysis according to liquid chromatography-mass spectrometry (LC-MS).

Synthesis Example 5: Synthesis of Compound BH1-6

A compound BH1-6 was synthesized through a synthesis pathway below.

[Formula 294]

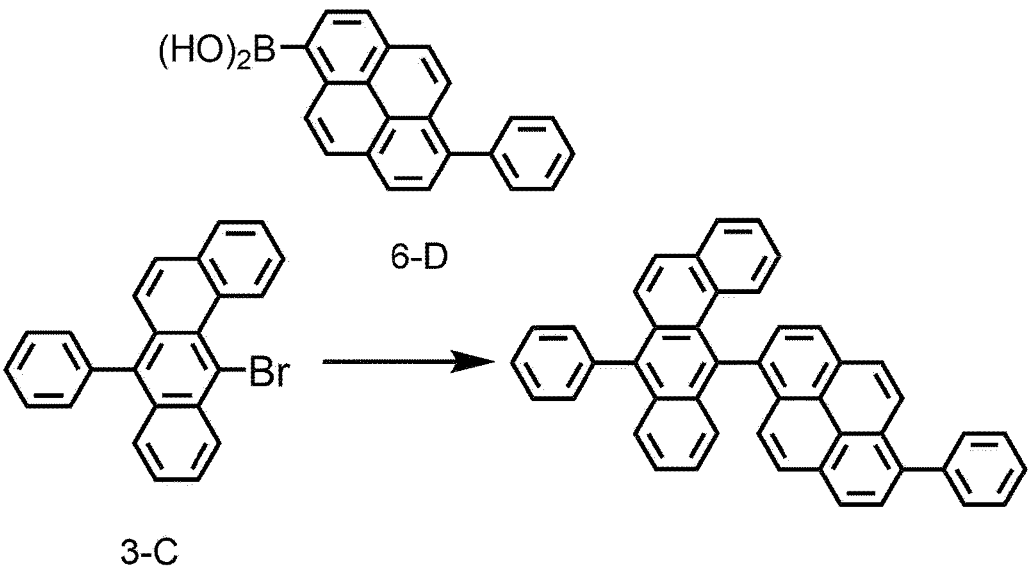

6-D

3-C

BH1-6

Synthesis of Compound BH1-6

The compound BH1-6 was synthesized as in the compound BH1-1 except that the intermediate 1-A and the intermediate 1-B in the synthesis of the compound BH1-1 were replaced with the intermediate 3-C and an intermediate 6-D, thereby obtaining 1.1 g (a yield of 49%) of a light yellow solid.

The light yellow solid was identified as the compound BH1-6 by analysis according to liquid chromatography-mass spectrometry (LC-MS).

Synthesis Example 6: Synthesis of Compound BH1-7

A compound BH1-7 was synthesized through a synthesis pathway below.

[Formula 295]

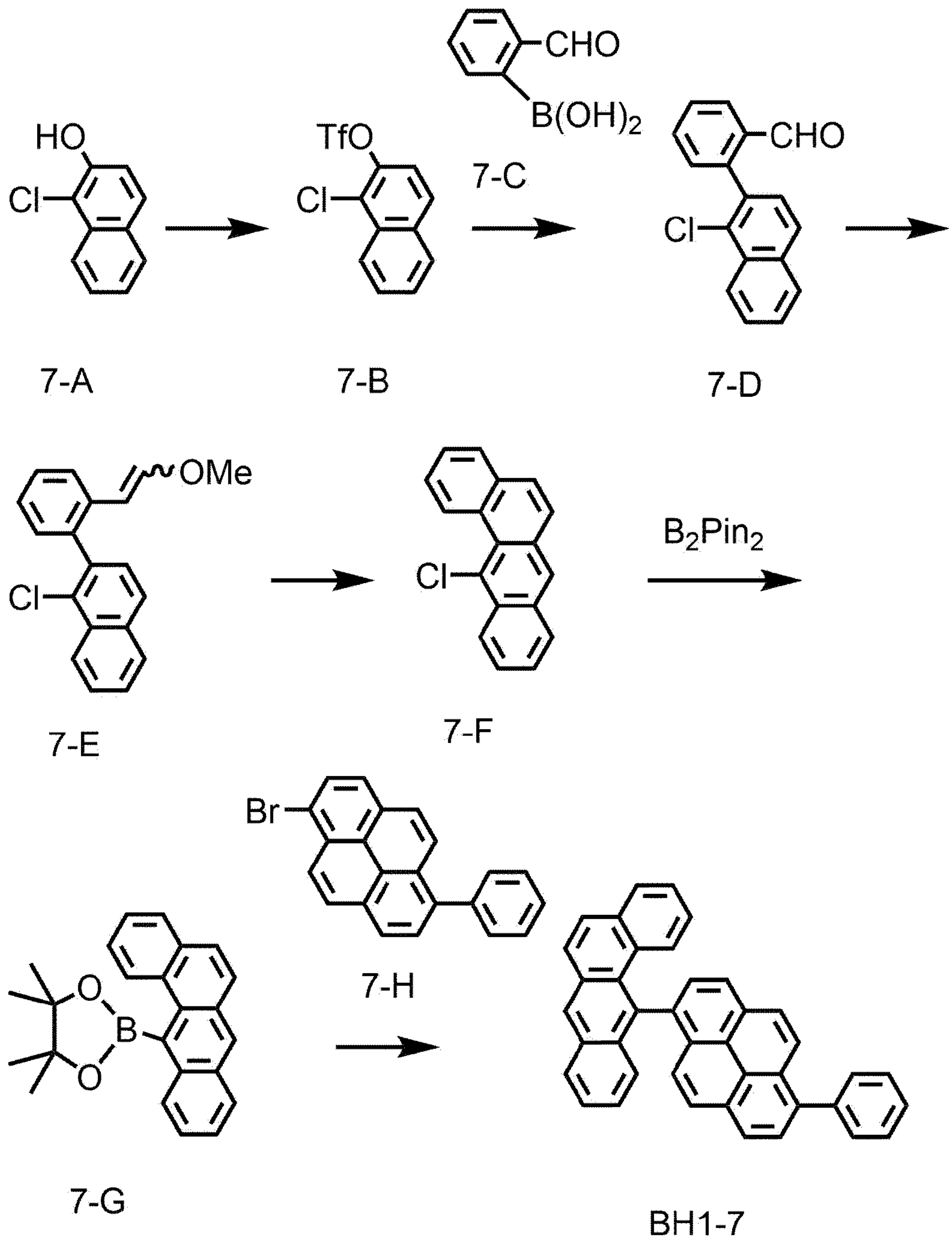


7-A 7-B 7-D

7-E 7-F

7-H

7-G BH1-7

Synthesis of Intermediate 7-B

Under an argon atmosphere, 11.6 g (43.2 mmol) of an intermediate 7-A and 215 ml of dichloromethane were put into a flask and the solution was cooled to 0 degrees C. After that, 4.4 ml (56.1 mmol) of pyridine, then 9.2 ml (56.1 mmol) of trifluoromethanesulfonic anhydride were added dropwise into the system, and stirred as it was for one hour.
The solution was stirred for further six hours while being returned to a room temperature (25 degrees C), followed by addition of ice water to the reaction solution. After sufficient stirring, a water layer was removed, the remaining organic layer was concentrated, and the residue was purified by silica gel column chromatography to obtain 21.3 g (a yield of 73%) of a white solid.
The white solid was identified as an intermediate 7-B by analysis according to liquid chromatography-mass spectrometry (LC-MS).

Synthesis of Intermediate 7-D

An intermediate 7-D was synthesized as in the compound BH1-1 except that the intermediate 1-A and the intermediate 1-B in the synthesis of the compound BH1-1 were replaced with the intermediate 7-B and an intermediate 7-C, thereby obtaining 19.3 g (yield of 87%) of a white solid.
The white solid was identified as an intermediate 7-D by analysis according to liquid chromatography-mass spectrometry (LC-MS).

Synthesis of Intermediate 7-E

Under an argon atmosphere, 28.1 g of (methoxymethyl)triphenylphosphonium chloride and 130 ml of tetrahydrofuran were put into a flask and stirred for five minutes, followed by addition of 82 ml of 1mol/L potassium tert-butoxide tetrahydrofuran solution thereto. Then, the solution was stirred at room temperature for 1.5 hours. After that, a solution obtained by dissolving 21.1g of the intermediate 7-D in 342 ml of tetrahydrofuran was added to the system, and the thus-obtained solution was stirred at room temperature for three hours. The deposited solid was filtered to concentrate the filtrate. The obtained residue was purified by silica gel column chromatography to obtain 22.5 g (a yield of 98%) of a white solid.
The white solid was identified as an intermediate 7-E by analysis according to liquid chromatography-mass spectrometry (LC-MS).

Synthesis of Intermediate 7-F

Under an argon atmosphere, 22.5 g of the intermediate 7-E and 684 ml of dichloromethane were put into a flask and the solution was stirred at 0 degrees C for five minutes. After that, 7 ml of methanesulfonic acid was added thereto and the solution was stirred as it was at 0 degrees C for three hours. After that, washing with a saturated aqueous sodium hydrogencarbonate solution was performed to remove a water layer, and then the organic layer was concentrated. The obtained residue was purified by silica gel column chromatography to obtain 9.4 g (a yield of 45%) of a white solid.
The white solid was identified as an intermediate 7-F by analysis according to liquid chromatography-mass spectrometry (LC-MS).

Synthesis of Intermediate 7-G

Under an argon atmosphere, 6.5 g (19.8 mmol) of the intermediate 7-F, 10.0 g (39.5 mmol) of bis(pinacolato)diboron, 0.09 g (0.4 mmol) of palladium (II) acetate, 0.75 g (1.6 mmol) of XPhos, 5.82 g (59.3 mmol) of potassium acetate, and 100 mL of 1,4-dioxane were put into a flask and the solution was stirred at 100 degrees C for seven hours. After stirring, the solution was cooled to room temperature. After the cooling, an organic layer was washed with saturated saline solution and then added with sodium sulfate to be dried. After the drying, filtration and concentration under reduced pressure were conducted. The crude product was purified by silica gel column chromatography to obtain 5.7 g (a yield of 41%) of a white solid.
The white solid was identified as an intermediate 7-G by analysis according to liquid chromatography-mass spectrometry (LC-MS).

Synthesis of Compound BH1-7

The compound BH1-7 was synthesized as in the compound BH1-1 except that the intermediate 1-A and the intermediate 1-B in the synthesis of the compound BH1-1 were replaced with the intermediate 7-G and an intermediate

7-H, thereby obtaining 1.9 g (a yield of 25%) of a light yellow solid.

The light yellow solid was identified as the compound BH1-7 by analysis according to liquid chromatography-mass spectrometry (LC-MS).

Synthesis Example 7: Synthesis of Compound BH1-8

A compound BH1-8 was synthesized through a synthesis pathway below.

[Formula 296]

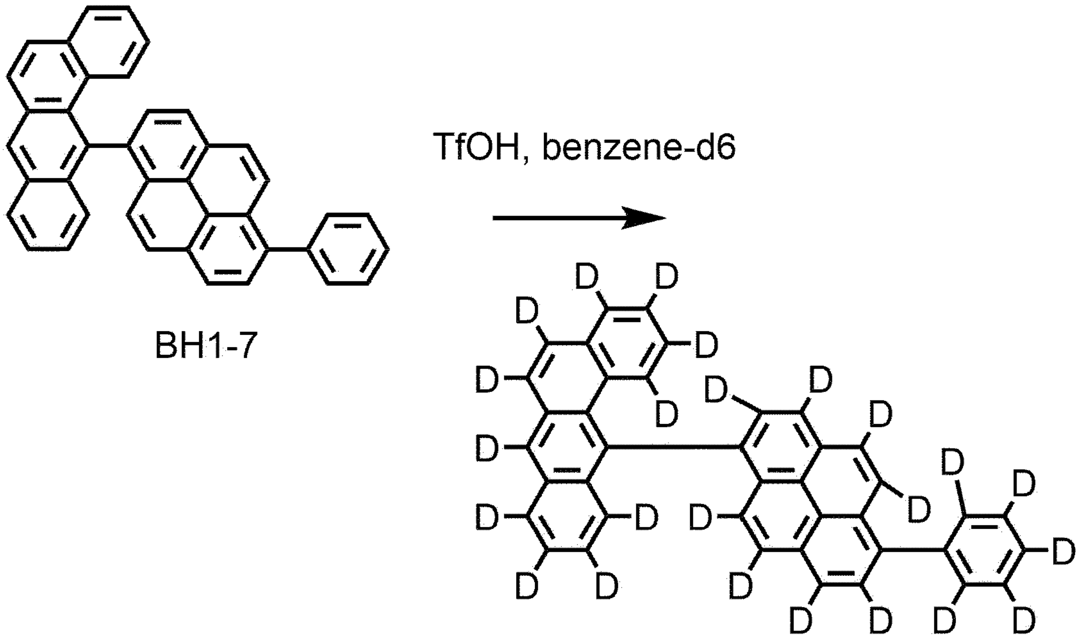


BH1-7

BH1-8

Synthesis of Compound BH1-8

The compound BH1-8 was synthesized as in the compound BH1-4 except that the compound BH1-3 in the synthesis of the compound BH1-4 was replaced with the intermediate BH1-7, thereby obtaining 0.2 g (a yield of 43%) of a light yellow solid.

The light yellow solid was identified as the compound BH1-8 by analysis according to liquid chromatography-mass spectrometry (LC-MS).

Synthesis Example 8: Synthesis of Compound BH1-9

A compound BH1-9 was synthesized through a synthesis pathway below.

[Formula 297]

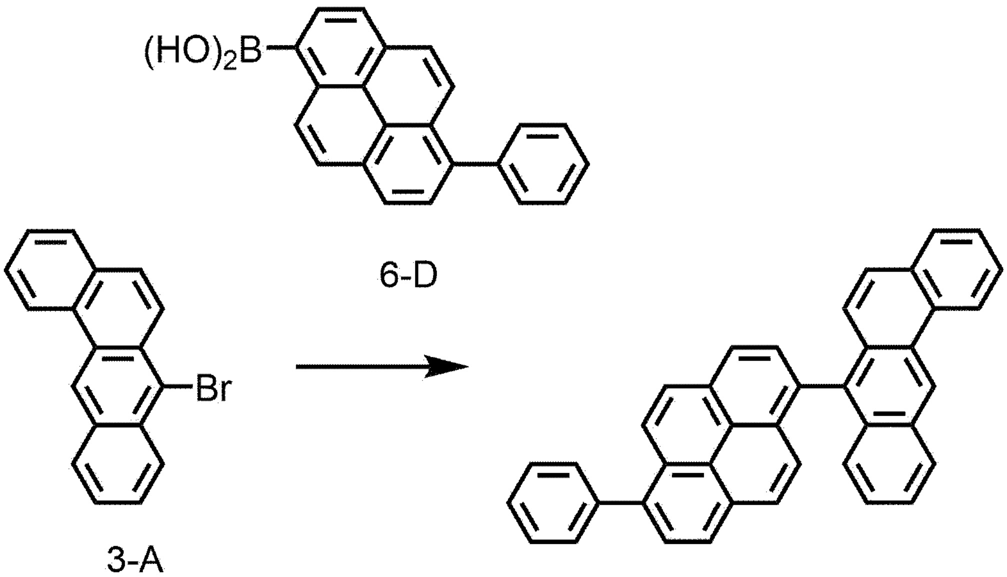

6-D

3-A

BH1-9

Synthesis of Compound BH1-9

The compound BH1-9 was synthesized as in the compound BH1-1 except that the intermediate 1-A and the intermediate 1-B in the synthesis of the compound BH1-1 were replaced with the intermediate 3-A and the intermediate 6-D, thereby obtaining 3.4 g (yield of 82%) of a light yellow solid.

The light yellow solid was identified as the compound BH1-9 by analysis according to liquid chromatography-mass spectrometry (LC-MS).

Synthesis Example 9: Synthesis of Compound BH1-10

A compound BH1-10 was synthesized through a synthesis pathway below.

[Formula 298]

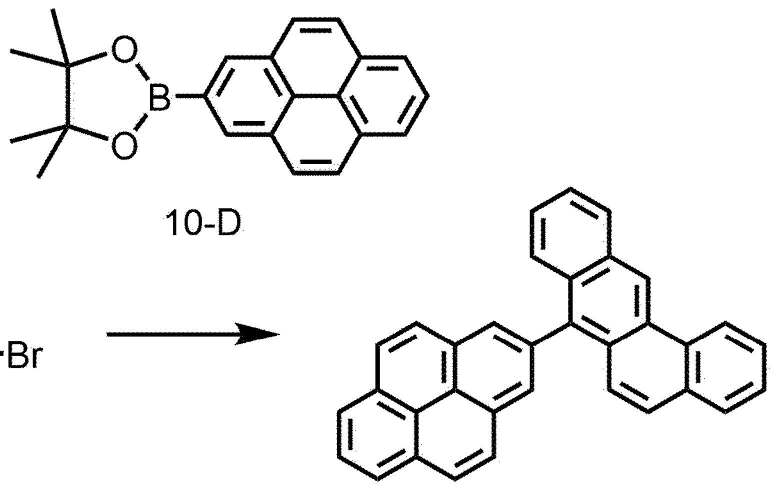

10-D

3-A

BH1-10

Synthesis of Compound BH1-10

The compound BH1-10 was synthesized as in the compound BH1-1 except that the intermediate 1-A and the intermediate 1-B in the synthesis of the compound BH1-1 were replaced with the intermediate 3-A and an intermediate 10-D, thereby obtaining 2.2 g (a yield of 77%) of a light yellow solid.
The light yellow solid was identified as the compound BH1-10 by analysis according to liquid chromatography-mass spectrometry (LC-MS).

Synthesis Example 10: Synthesis of Compound BH1-11

A compound BH1-11 was synthesized through a synthesis pathway below.

[Formula 299]

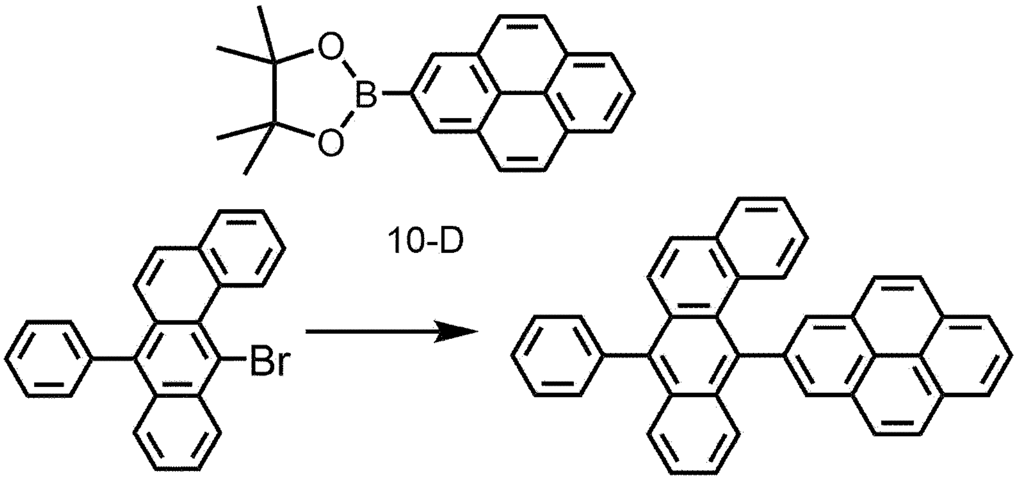

10-D

3-C

BH1-11

Synthesis of Compound BH1-11

The compound BH1-11 was synthesized as in the compound BH1-1 except that the intermediate 1-A and the intermediate 1-B in the synthesis of the compound BH1-1 were replaced with the intermediate 3-C and the intermediate 10-D, thereby obtaining 1.7 g (a yield of 40%) of a light yellow solid.
The light yellow solid was identified as the compound BH1-11 by analysis according to liquid chromatography-mass spectrometry (LC-MS).

EXPLANATION OF CODES

1A, 1B...organic EL device, 2... substrate, 3...anode, 4...cathode, 5A, 5B...emitting region, 5...emitting layer, 51...first emitting layer, 52...second emitting layer, 6... hole transporting zone, 61 ... hole injecting layer, 62... hole transporting layer, 7... electron transporting zone, 71...electron transporting layer, 72... electron injecting layer.

## Claims

**1.** A compound represented by a formula (1C-A) below,

[Formula 1]

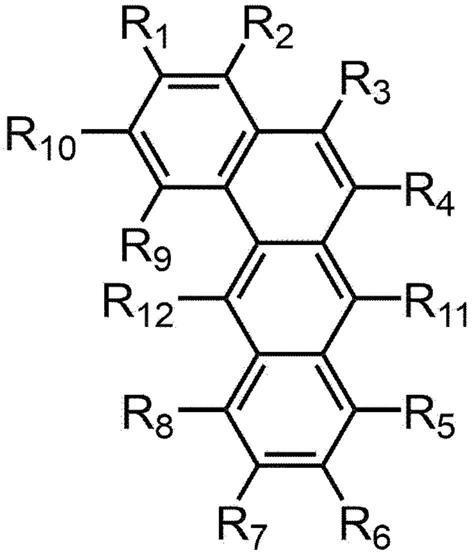

(1C-A)

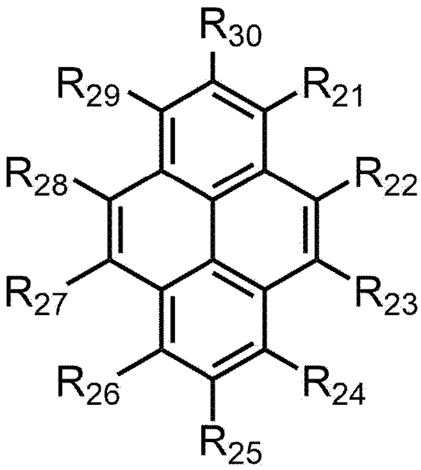

(1D-A)

where, in the formula (1C-A):

one of $R_4$ to $R_8$ and $R_{10}$ to $R_{12}$ is a group represented by a formula (1D-A) above;
$R_1$ to $R_3$, $R_9$, and $R_4$ to $R_8$ and $R_{10}$ to $R_{12}$ not being the group represented by the formula (1D-A) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a substituted or unsubstituted aralkyl group having 7 to 50 carbon atoms, a group represented by - $C(=O)R_{801}$, a group represented by $-COOR_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;
when $R_{11}$ is a group represented by the formula (1D-A), $R_{12}$ is a hydrogen atom or a substituted or unsubstituted phenyl group;
when $R_{12}$ is a group represented by the formula (1D-A), $R_{11}$ is a hydrogen atom or a substituted or unsubstituted phenyl group;
in the formula (1D-A), one of $R_{21}$ to $R_{30}$ represents a bonding position to a benz[a]anthracene ring in the formula (1C-A), and at least one combination of adjacent two or more of $R_{21}$ to $R_{30}$ not being the bonding position in the formula (1D-A) are mutually bonded to form a substituted or unsubstituted monocyclic ring, mutually bonded to form a substituted or unsubstituted fused ring, or not mutually bonded;
$R_{21}$ to $R_{30}$ not being the bonding position to the benz[a]anthracene ring in the formula (1C-A) and forming neither the substituted or unsubstituted monocyclic ring nor the substituted or unsubstituted fused ring in the formula (1D-A) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and
at least one of $R_{21}$ to $R_{30}$ is other than a hydrogen atom; and
in the compound represented by the formula (1C-A), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group

having 5 to 50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

2. The compound according to claim 1, wherein at least one of $R_{21}$ to $R_{30}$ in the formula (1D-A) not being the bonding position to the benz[a]anthracene ring in the formula (1C-A), forming neither the substituted or unsubstituted monocyclic ring nor the substituted or unsubstituted fused ring, and not being the hydrogen atom in the group represented by the formula (1D-A) is a substituted or unsubstituted aryl group having 6 to 50 ring carbon atoms.

3. The compound according to claim 1 or 2, wherein at least one of $R_{21}$ to $R_{30}$ in the formula (1D-A) not being the bonding position to the benz[a]anthracene ring in the formula (1C-A), forming neither the substituted or unsubstituted monocyclic ring nor the substituted or unsubstituted fused ring, and not being the hydrogen atom in the group represented by the formula (1D-A) is a substituted or unsubstituted phenyl group.

4. The compound according to any one of claims 1 to 3, wherein
the group represented by the formula (1D-A) is a group represented by a formula (1D-A1), (1D-A2), or (1D-A3) below,

[Formula 2]

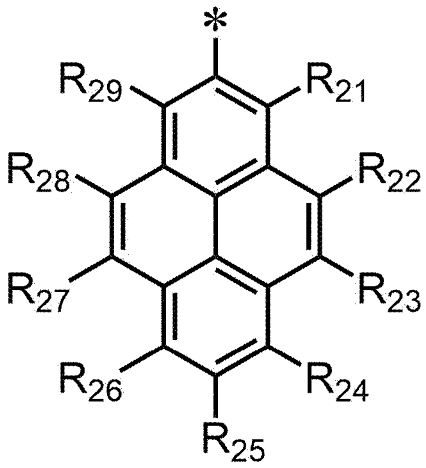

(1D-A1)

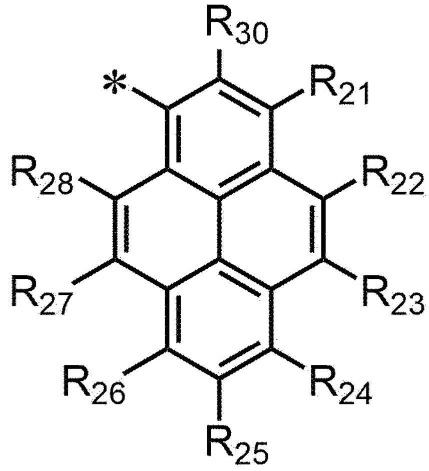

(1D-A2)

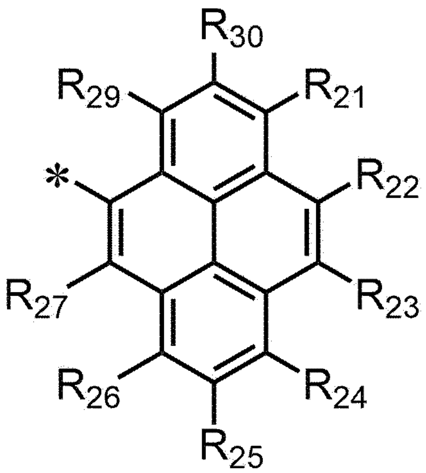

(1D-A3)

where, in the formula (1D-A1):

at least one combination of adjacent two or more of $R_{21}$ to $R_{29}$ are mutually bonded to form a substituted or unsubstituted monocyclic ring, mutually bonded to form a substituted or unsubstituted fused ring, or not mutually bonded;

$R_{21}$ to $R_{29}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by - S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms;

at least one of $R_{21}$ to $R_{29}$ is other than a hydrogen atom; and

* represents a bonding position to the benz[a]anthracene ring in the formula (1C-A),

in the formula (1D-A2):

at least one combination of adjacent two or more of $R_{21}$ to $R_{28}$, and $R_{30}$ are mutually bonded to form a

substituted or unsubstituted monocyclic ring, mutually bonded to form a substituted or unsubstituted fused ring, or not mutually bonded;
$R_{21}$ to $R_{28}$ and $R_{30}$ forming neither the substituted or unsubstituted monocyclic ring nor the substituted or unsubstituted fused ring each independently represent the same as $R_{21}$ to $R_{29}$ in the formula (1D-A1);
at least one of $R_{21}$ to $R_{28}$, or $R_{30}$ is other than a hydrogen atom; and
* represents a bonding position to the benz[a]anthracene ring in the formula (1C-A),
in the formula (1D-A3):

at least one combination of adjacent two or more of $R_{21}$ to $R_{27}$, $R_{29}$, and $R_{30}$ are mutually bonded to form a substituted or unsubstituted monocyclic ring, mutually bonded to form a substituted or unsubstituted fused ring, or not mutually bonded;
$R_{21}$ to $R_{27}$, $R_{29}$, and $R_{30}$ forming neither the substituted or unsubstituted monocyclic ring nor the substituted or unsubstituted fused ring each independently represent the same as $R_{21}$ to $R_{29}$ in the formula (1D-A1);
at least one of $R_{21}$ to $R_{27}$, $R_{29}$, or $R_{30}$ is other than a hydrogen atom; and
* represents a bonding position to the benz[a]anthracene ring in the formula (1C-A).

5. The compound according to claim 4, wherein at least one of $R_{21}$ to $R_{29}$ in the formula (1D-A1), at least one of $R_{21}$ to $R_{28}$, or $R_{30}$ in the formula (1D-A2), or at least one of $R_{21}$ to $R_{27}$, $R_{29}$, or $R_{30}$ in the formula (1D-A3) forming neither the substituted or unsubstituted monocyclic ring nor the substituted or unsubstituted fused ring and not being the hydrogen atom in the group represented by the formula (1D-A1), (1D-A2), or (1D-A3) is a substituted or unsubstituted aryl group having 6 to 50 ring carbon atoms.

6. The compound according to claim 4 or 5, wherein at least one of $R_{21}$ to $R_{29}$ in the formula (1D-A1), at least one of $R_{21}$ to $R_{28}$, or $R_{30}$ in the formula (1D-A2), or at least one of $R_{21}$ to $R_{27}$, $R_{29}$, or $R_{30}$ in the formula (1D-A3) forming neither the substituted or unsubstituted monocyclic ring nor the substituted or unsubstituted fused ring and not being the hydrogen atom in the group represented by the formula (1D-A1), (1D-A2), or (1D-A3) is a substituted or unsubstituted phenyl group.

7. The compound according to any one of claims 4 to 6, wherein the group represented by the formula (1D-A) is a group represented by the formula (1D-A1).

8. The compound according to any one of claims 1 to 7, wherein one of $R_5$, $R_6$, $R_7$, $R_8$, $R_{11}$, and $R_{12}$ is a group represented by the formula (1D-A).

9. The compound according to claim 8, wherein one of $R_6$, $R_7$, $R_{11}$, and $R_{12}$ is a group represented by the formula (1D-A).

10. The compound according to claim 9, wherein $R_{11}$ is a group represented by the formula (1D-A).

11. The compound according to claim 9, wherein $R_{12}$ is a group represented by the formula (1D-A).

12. The compound according to claim 9, wherein

$R_{11}$ is a group represented by the formula (1D-A) and $R_{12}$ is an unsubstituted phenyl group, or
$R_{12}$ is a group represented by the formula (1D-A) and $R_{11}$ is an unsubstituted phenyl group.

13. The compound according to claim 12, wherein $R_{11}$ is a group represented by the formula (1D-A) and $R_{12}$ is an unsubstituted phenyl group.

14. The compound according to claim 12, wherein $R_{12}$ is a group represented by the formula (1D-A) and $R_{11}$ is an unsubstituted phenyl group.

15. The compound according to any one of claims 1 to 14, wherein $R_{12}$ is a hydrogen atom.

16. The compound according to any one of claims 1 to 15, comprising at least one deuterium atom in a molecule.

17. The compound according to any one of claims 1 to 16, wherein when one of $R_5$, $R_6$, $R_7$, and $R_8$ is a group represented by the formula (1D-A), $R_2$ and $R_3$ are each a hydrogen atom.

**18.** A compound represented by a formula (1C-B) below,

[Formula 3]

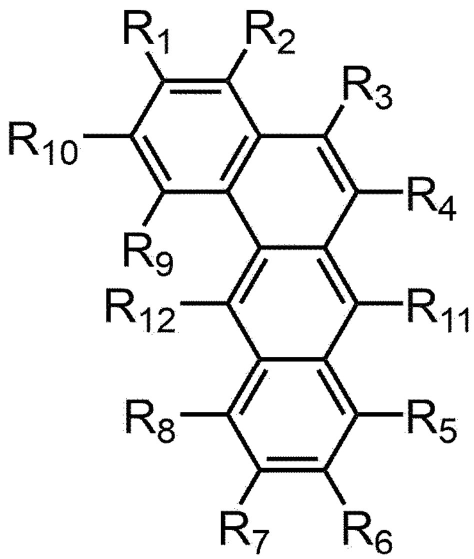

(1C-B)

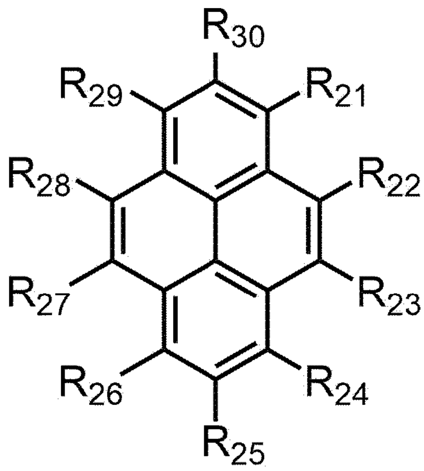

(1D-B)

where, in the formula (1C-B):

one of $R_4$ to $R_6$, $R_8$, and $R_{10}$ to $R_{12}$ is a group represented by a formula (1D-B) above;
$R_1$ to $R_3$, $R_7$, $R_9$, and $R_4$ to $R_6$, $R_8$, and $R_{10}$ to $R_{12}$ not being the group represented by the formula (1D-B) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by - S-($R_{905}$), a substituted or unsubstituted aralkyl group having 7 to 50 carbon atoms, a group represented by -C(=O)$R_{801}$, a group represented by -COO$R_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;
when $R_{11}$ is a group represented by the formula (1D-B), $R_{12}$ is a hydrogen atom or an unsubstituted phenyl group; and
when $R_{12}$ is a group represented by the formula (1D-B), $R_{11}$ is a substituted or unsubstituted phenyl group;
in the formula (1D-B), one of $R_{21}$ to $R_{30}$ represents a bonding position to a benz[a]anthracene ring in the formula (1C-B), and at least one combination of adjacent two or more of $R_{21}$ to $R_{30}$ not being the bonding position in the formula (1D-B) are mutually bonded to form a substituted or unsubstituted monocyclic ring, mutually bonded to form a substituted or unsubstituted fused ring, or not mutually bonded;
$R_{21}$ to $R_{30}$ not being the bonding position to the benz[a]anthracene ring in the formula (1C-B) and forming neither the substituted or unsubstituted monocyclic ring nor the substituted or unsubstituted fused ring in the formula (1D-B) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms;
in the compound represented by the formula (1C-B), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group

having 5 to 50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

**19.** The compound according to claim 18, wherein at least one of $R_{21}$ to $R_{30}$ in the formula (1D-B) not being the bonding position to the benz[a]anthracene ring in the formula (1C-B) and forming neither the substituted or unsubstituted monocyclic ring nor the substituted or unsubstituted fused ring in the group represented by the formula (1D-B) is other than a hydrogen atom.

**20.** The compound according to claim 18 or 19, wherein at least one of $R_{21}$ to $R_{30}$ in the formula (1D-B) not being the bonding position to the benz[a]anthracene ring in the formula (1C-B) and forming neither the substituted or unsubstituted monocyclic ring nor the substituted or unsubstituted fused ring in the group represented by the formula (1D-B) is a substituted or unsubstituted aryl group having 6 to 50 ring carbon atoms.

**21.** The compound according to claim 20, wherein at least one of $R_{21}$ to $R_{30}$ in the formula (1D-B) not being the bonding position to the benz[a]anthracene ring in the formula (1C-B) and forming neither the substituted or unsubstituted monocyclic ring nor the substituted or unsubstituted fused ring in the group represented by the formula (1D-B) is a substituted or unsubstituted phenyl group.

**22.** The compound according to any one of claims 18 to 21, wherein
the group represented by the formula (1D-B) is a group represented by a formula (1D-B1), (1D-B2), or (1D-B3) below,

[Formula 4]

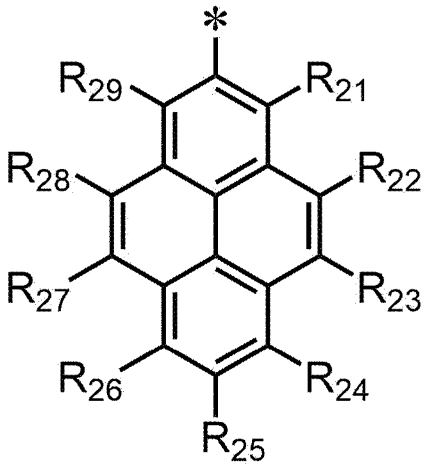

(1D-B1)

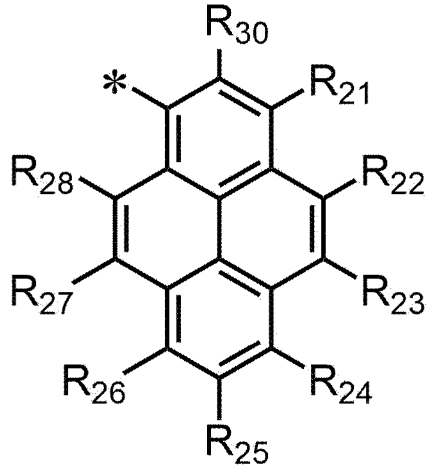

(1D-B2)

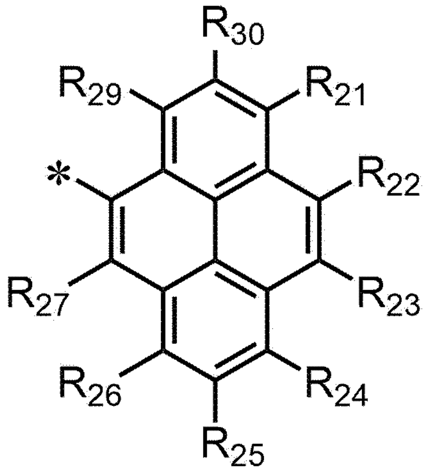

(1D-B3)

where, in the formula (1D-B1):

at least one combination of adjacent two or more of $R_{21}$ to $R_{29}$ are mutually bonded to form a substituted or unsubstituted monocyclic ring, mutually bonded to form a substituted or unsubstituted fused ring, or not mutually bonded;

$R_{21}$ to $R_{29}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $- S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and

* represents a bonding position to the benz[a]anthracene ring in the formula (1C-B);

in the formula (1D-B2):

at least one combination of adjacent two or more of $R_{21}$ to $R_{28}$, and $R_{30}$ are mutually bonded to form a substituted or unsubstituted monocyclic ring, mutually bonded to form a substituted or unsubstituted fused ring, or not mutually bonded;

$R_{21}$ to $R_{28}$ and $R_{30}$ forming neither the substituted or unsubstituted monocyclic ring nor the substituted or unsubstituted fused ring each independently represent the same as $R_{21}$ to $R_{29}$ in the formula (1D-B1); and

* represents a bonding position to the benz[a]anthracene ring in the formula (1C-B);
in the formula (1D-B3):

at least one combination of adjacent two or more of $R_{21}$ to $R_{27}$, $R_{29}$, and $R_{30}$ are mutually bonded to form a substituted or unsubstituted monocyclic ring, mutually bonded to form a substituted or unsubstituted fused ring, or not mutually bonded;
$R_{21}$ to $R_{27}$, $R_{29}$, and $R_{30}$ forming neither the substituted or unsubstituted monocyclic ring nor the substituted or unsubstituted fused ring each independently represent the same as $R_{21}$ to $R_{29}$ in the formula (1D-B1); and
* represents a bonding position to the benz[a]anthracene ring in the formula (1C-B).

**23.** The compound according to claim 22, wherein at least one of $R_{21}$ to $R_{29}$ in the formula (1D-B1), at least one of $R_{21}$ to $R_{28}$, or $R_{30}$ in the formula (1D-B2), or at least one of $R_{21}$ to $R_{27}$, $R_{29}$, or $R_{30}$ in the formula (1D-B3) forming neither the substituted or unsubstituted monocyclic ring nor the substituted or unsubstituted fused ring in the group represented by the formula (1D-B1), (1D-B2), or (1D-B3) is other than a hydrogen atom.

**24.** The compound according to claim 22 or 23, wherein at least one of $R_{21}$ to $R_{29}$ in the formula (1D-B1), at least one of $R_{21}$ to $R_{28}$, or $R_{30}$ in the formula (1D-B2), or at least one of $R_{21}$ to $R_{27}$, $R_{29}$, or $R_{30}$ in the formula (1D-B3) forming neither the substituted or unsubstituted monocyclic ring nor the substituted or unsubstituted fused ring in the group represented by the formula (1D-B1), (1D-B2), or (1D-B3) is a substituted or unsubstituted aryl group having 6 to 50 ring carbon atoms.

**25.** The compound according to claim 24, wherein at least one of $R_{21}$ to $R_{29}$ in the formula (1D-B1), at least one of $R_{21}$ to $R_{28}$, or $R_{30}$ in the formula (1D-B2), or at least one of $R_{21}$ to $R_{27}$, $R_{29}$, or $R_{30}$ in the formula (1D-B3) forming neither the substituted or unsubstituted monocyclic ring nor the substituted or unsubstituted fused ring in the group represented by the formula (1D-B1), (1D-B2), or (1D-B3) is a substituted or unsubstituted phenyl group.

**26.** The compound according to any one of claims 22 to 24, wherein
the group represented by the formula (1D-B) is a group represented by the formula (1D-B1).

**27.** The compound according to any one of claims 18 to 26, wherein one of $R_5$, $R_6$, $R_8$, $R_{11}$, and $R_{12}$ is a group represented by the formula (1D-B).

**28.** The compound according to claim 27, wherein one of $R_6$, $R_{11}$, and $R_{12}$ is a group represented by the formula (1D-B).

**29.** The compound according to claim 28, wherein $R_{11}$ is a group represented by the formula (1D-B).

**30.** The compound according to claim 28, wherein $R_{12}$ is a group represented by the formula (1D-B).

**31.** The compound according to claim 28, wherein

$R_{11}$ is a group represented by the formula (1D-B) and $R_{12}$ is an unsubstituted phenyl group, or
$R_{12}$ is a group represented by the formula (1D-B) and $R_{11}$ is an unsubstituted phenyl group.

**32.** The compound according to claim 31, wherein $R_{11}$ is a group represented by the formula (1D-B) and $R_{12}$ is an unsubstituted phenyl group.

**33.** The compound according to claim 31, wherein $R_{12}$ is a group represented by the formula (1D-B) and $R_{11}$ is an unsubstituted phenyl group.

**34.** The compound according to any one of claims 18 to 33, wherein $R_7$ and $R_{12}$ are each a hydrogen atom.

**35.** The compound according to any one of claims 18 to 34, comprising at least one deuterium atom in a molecule.

**36.** A compound represented by a formula (1C-C) below,

[Formula 5]

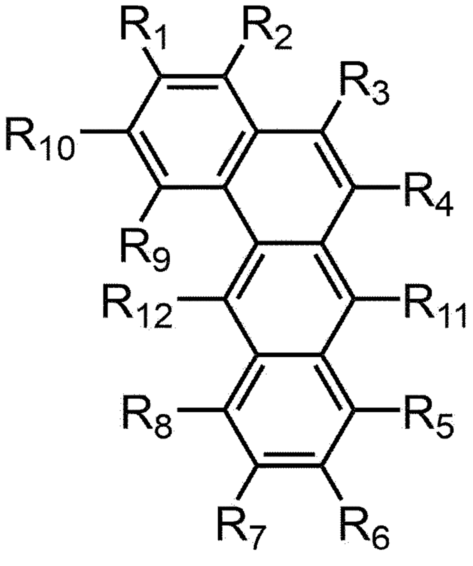

(1C-C)

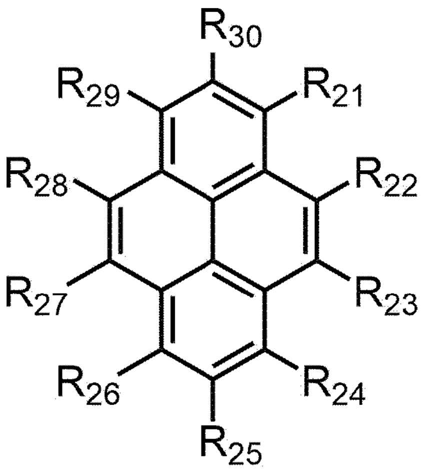

(1D-C)

where, in the formula (1C-C):

one of $R_4$ to $R_8$ and $R_{10}$ to $R_{12}$ is a group represented by a formula (1D-C) above;

$R_1$ to $R_3$, $R_9$, and $R_4$ to $R_8$ and $R_{10}$ to $R_{12}$ not being the group represented by the formula (1D-C) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a substituted or unsubstituted aralkyl group having 7 to 50 carbon atoms, a group represented by - $C(=O)R_{801}$, a group represented by $-COOR_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;

when $R_{11}$ is a group represented by the formula (1D-C), $R_{12}$ is a hydrogen atom or a substituted or unsubstituted phenyl group; and

when $R_{12}$ is a group represented by the formula (1D-C), $R_{11}$ is a hydrogen atom or a substituted or unsubstituted phenyl group;

in the formula (1D-C), one of $R_{22}$, $R_{23}$, $R_{25}$, $R_{27}$, $R_{28}$, and $R_{30}$ represents a bonding position to a benz[a]anthracene ring in the formula (1C-C), and at least one combination of adjacent two or more of $R_{22}$, $R_{23}$, $R_{25}$, $R_{27}$, $R_{28}$, and $R_{30}$ not being the bonding position, $R_{21}$, $R_{24}$, $R_{26}$, and $R_{29}$ in the formula (1D-C) are mutually bonded to form a substituted or unsubstituted monocyclic ring, mutually bonded to form a substituted or unsubstituted fused ring, or not mutually bonded;

$R_{21}$ to $R_{30}$ not being the bonding position to the benz[a]anthracene ring in the formula (1C-C) and forming neither the substituted or unsubstituted monocyclic ring nor the substituted or unsubstituted fused ring in the formula (1D-C) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

in the compound represented by the formula (1C-C), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group

having 5 to 50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

**37.** The compound according to claim 36, wherein at least one of $R_{21}$ to $R_{30}$ in the formula (1D-C) not being the bonding position to the benz[a]anthracene ring in the formula (1C-C) and forming neither the substituted or unsubstituted monocyclic ring nor the substituted or unsubstituted fused ring in the group represented by the formula (1D-C) is other than a hydrogen atom.

**38.** The compound according to claim 36 or 37, wherein at least one of $R_{21}$ to $R_{30}$ in the formula (1D-C) not being the bonding position to the benz[a]anthracene ring in the formula (1C-C) and forming neither the substituted or unsubstituted monocyclic ring nor the substituted or unsubstituted fused ring in the group represented by the formula (1D-C) is a substituted or unsubstituted aryl group having 6 to 50 ring carbon atoms.

**39.** The compound according to claim 38, wherein at least one of $R_{21}$ to $R_{30}$ in the formula (1D-C) not being the bonding position to the benz[a]anthracene ring in the formula (1C-C) and forming neither the substituted or unsubstituted monocyclic ring nor the substituted or unsubstituted fused ring in the group represented by the formula (1D-C) is a substituted or unsubstituted phenyl group.

**40.** The compound according to any one of claims 36 to 39, wherein
the group represented by the formula (1D-C) is a group represented by a formula (1D-C1) or (1D-C3) below,

[Formula 6]

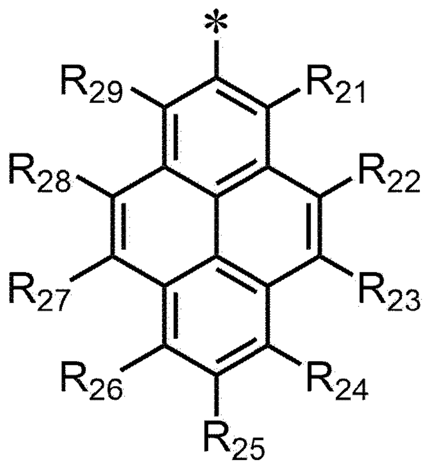

(1D-C1)

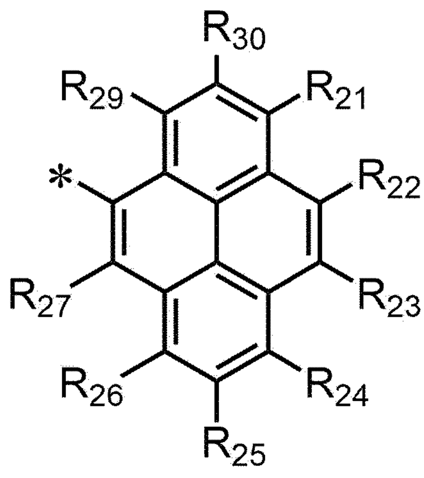

(1D-C3)

where, in the formula (1D-C1):

at least one combination of adjacent two or more of $R_{21}$ to $R_{29}$ are mutually bonded to form a substituted or unsubstituted monocyclic ring, mutually bonded to form a substituted or unsubstituted fused ring, or not mutually bonded;
$R_{21}$ to $R_{29}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or

unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by - S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and
* represents a bonding position to the benz[a]anthracene ring in the formula (1C-C);
in the formula (1D-C3):
at least one combination of adjacent two or more of $R_{21}$ to $R_{27}$, $R_{29}$, and $R_{30}$ are mutually bonded to form a substituted or unsubstituted monocyclic ring, mutually bonded to form a substituted or unsubstituted fused ring, or not mutually bonded;
$R_{21}$ to $R_{27}$, $R_{29}$, and $R_{30}$ forming neither the substituted or unsubstituted monocyclic ring nor the substituted or unsubstituted fused ring each independently represent the same as $R_{21}$ to $R_{29}$ in the formula (1D-C1); and
* represents a bonding position to the benz[a]anthracene ring in the formula (1C-C).

**41.** The compound according to claim 40, wherein at least one of $R_{21}$ to $R_{29}$ in the formula (1D-C1) or at least one of $R_{21}$ to $R_{27}$, $R_{29}$, or $R_{30}$ in the formula (1D-C3) forming neither the substituted or unsubstituted monocyclic ring nor the substituted or unsubstituted fused ring in the group represented by the formula (1D-C1) or (1D-C3) is other than a hydrogen atom.

**42.** The compound according to claim 40 or 41, wherein at least one of $R_{21}$ to $R_{29}$ in the formula (1D-C1) or at least one of $R_{21}$ to $R_{27}$, $R_{29}$, or $R_{30}$ in the formula (1D-C3) forming neither the substituted or unsubstituted monocyclic ring nor the substituted or unsubstituted fused ring in the group represented by the formula (1D-C1) or (1D-C3) is a substituted or unsubstituted aryl group having 6 to 50 ring carbon atoms.

**43.** The compound according to claim 42, wherein at least one of $R_{21}$ to $R_{29}$ in the formula (1D-C1) or at least one of $R_{21}$ to $R_{27}$, $R_{29}$, or $R_{30}$ in the formula (1D-C3) forming neither the substituted or unsubstituted monocyclic ring nor the substituted or unsubstituted fused ring in the group represented by the formula (1D-C1) or (1D-C3) is a substituted or unsubstituted phenyl group.

**44.** The compound according to any one of claims 40 to 43, wherein the group represented by the formula (1D-C) is a group represented by the formula (1D-C1).

**45.** The compound according to any one of claims 36 to 44, wherein one of $R_5$, $R_6$, $R_7$, $R_8$, $R_{11}$, and $R_{12}$ is a group represented by the formula (1D-C).

**46.** The compound according to claim 45, wherein one of $R_6$, $R_7$, $R_{11}$, and $R_{12}$ is a group represented by the formula (1D-C).

**47.** The compound according to claim 46, wherein $R_{11}$ is a group represented by the formula (1D-C).

**48.** The compound according to claim 46, wherein $R_{12}$ is a group represented by the formula (1D-C).

**49.** The compound according to claim 46, wherein

$R_{11}$ is a group represented by the formula (1D-C) and $R_{12}$ is an unsubstituted phenyl group, or
$R_{12}$ is a group represented by the formula (1D-C) and $R_{11}$ is an unsubstituted phenyl group.

**50.** The compound according to claim 49, wherein $R_{11}$ is a group represented by the formula (1D-C) and $R_{12}$ is an unsubstituted phenyl group.

**51.** The compound according to claim 49, wherein $R_{12}$ is a group represented by the formula (1D-C) and $R_{11}$ is an unsubstituted phenyl group.

**52.** The compound according to any one of claims 36 to 51, wherein $R_{12}$ is a hydrogen atom.

**53.** The compound according to any one of claims 36 to 52, comprising at least one deuterium atom in a molecule.

**54.** The compound according to any one of claims 1 to 53, wherein

the groups specified to be substituted or unsubstituted are each an unsubstituted group, and
the rings specified to be substituted or unsubstituted are each an unsubstituted ring.

**55.** An organic electroluminescence device, comprising the compound according to any one of claims 1 to 54.

**56.** The organic electroluminescence device according to claim 55, comprising:

an anode;
a cathode; and
an organic layer disposed between the anode and the cathode, wherein
at least one layer of the organic layer comprises the compound.

**57.** The organic electroluminescence device according to claim 56, wherein

the organic layer comprises an emitting region,
the emitting region comprises at least one emitting layer, and
the at least one emitting layer comprises the compound.

**58.** The organic electroluminescence device according to claim 57, wherein

the emitting region comprises a first emitting layer and a second emitting layer, and
the first emitting layer comprises the compound as a first compound and the second emitting layer comprises a second compound.

**59.** The organic electroluminescence device according to claim 58, wherein a triplet energy of the first compound $T_1(H1)$ and a triplet energy of the second compound $T_1(H2)$ satisfy a relationship of a numerical formula (Numerical Formula 1) below,

$$T_1(H1) > T_1(H2) \ldots \text{(Numerical Formula 1)}.$$

**60.** The organic electroluminescence device according to claim 58 or 59, wherein the first emitting layer is in direct contact with the second emitting layer.

**61.** The organic electroluminescence device according to any one of claims 58 to 60, wherein the first emitting layer is disposed between the anode and the second emitting layer.

**62.** The organic electroluminescence device according to any one of claims 58 to 61, wherein

the first emitting layer comprises a first luminescent compound,
the second emitting layer comprises a second luminescent compound, and
the first luminescent compound and the second luminescent compound are each independently a compound that emits light having a maximum peak wavelength of 500 nm or less.

**63.** The organic electroluminescence device according to any one of claims 57 to 62, further comprising a hole transporting layer between the anode and the emitting region.

**64.** The organic electroluminescence device according to any one of claims 57 to 63, further comprising an electron transporting layer between the cathode and the emitting region.

**65.** An organic electroluminescence device, comprising:

an anode;
a cathode; and
an organic layer disposed between the anode and the cathode, wherein
the organic layer comprises an emitting region,
the emitting region comprises a first emitting layer and a second emitting layer,
the first emitting layer comprises a first compound represented by a formula (1C) below, and

the second emitting layer comprises a second compound,

[Formula 7]

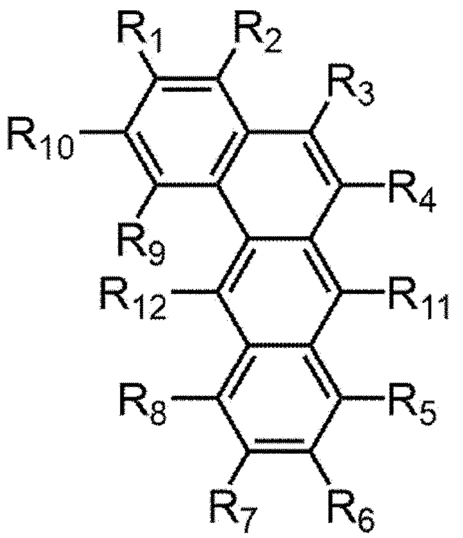

(1C)

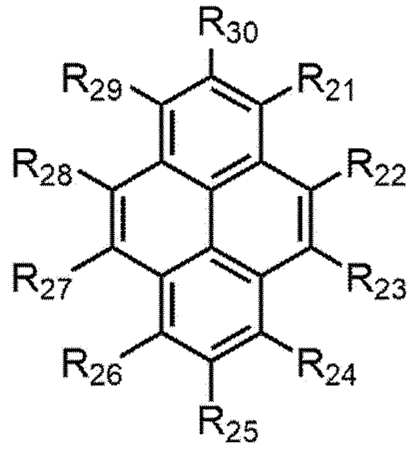

(1D)

where, in the formula (1C):

one of $R_4$ to $R_8$ and $R_{10}$ to $R_{12}$ is a group represented by a formula (1D) above;

$R_1$ to $R_3$, $R_9$, and $R_4$ to $R_8$ and $R_{10}$ to $R_{12}$ not being the group represented by the formula (1D) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a substituted or unsubstituted aralkyl group having 7 to 50 carbon atoms, a group represented by $-C(=O)R_{801}$, a group represented by $-COOR_{802}$, a halogen atom, a cyano group, a nitro group, a substituted or unsubstituted aryl group having 6 to 50 ring carbon atoms, or a substituted or unsubstituted heterocyclic group having 5 to 50 ring atoms;

when $R_{11}$ is a group represented by the formula (1D), $R_{12}$ is a hydrogen atom or a substituted or unsubstituted phenyl group; and

when $R_{12}$ is a group represented by the formula (1D), $R_{11}$ is a hydrogen atom or a substituted or unsubstituted phenyl group;

in the formula (1D), one of $R_{21}$ to $R_{30}$ represents a bonding position to a benz[a]anthracene ring in the formula (1C), and at least one combination of adjacent two or more of $R_{21}$ to $R_{30}$ not being the bonding position in the formula (1D) are mutually bonded to form a substituted or unsubstituted monocyclic ring, mutually bonded to form a substituted or unsubstituted fused ring, or not mutually bonded; and

$R_{21}$ to $R_{30}$ not being the bonding position to the benz[a]anthracene ring in the formula (1C) and forming neither the substituted or unsubstituted monocyclic ring nor the substituted or unsubstituted fused ring in the formula (1D) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

in the compound represented by the formula (1C), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to

50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

**66.** The organic electroluminescence device according to claim 65, wherein at least one of $R_{21}$ to $R_{30}$ in the formula (1D) not being the bonding position to the benz[a]anthracene ring in the formula (1C) and forming neither the substituted or unsubstituted monocyclic ring nor the substituted or unsubstituted fused ring in the group represented by the formula (1D) is other than a hydrogen atom.

**67.** The organic electroluminescence device according to claim 65 or 66, wherein at least one of $R_{21}$ to $R_{30}$ in the formula (1D) not being the bonding position to the benz[a]anthracene ring in the formula (1C) and forming neither the substituted or unsubstituted monocyclic ring nor the substituted or unsubstituted fused ring in the group represented by the formula (1D) is a substituted or unsubstituted aryl group having 6 to 50 ring carbon atoms.

**68.** The organic electroluminescence device according to claim 67, wherein at least one of $R_{21}$ to $R_{30}$ in the formula (1D) not being the bonding position to the benz[a]anthracene ring in the formula (1C) and forming neither the substituted or unsubstituted monocyclic ring nor the substituted or unsubstituted fused ring in the group represented by the formula (1D) is a substituted or unsubstituted phenyl group.

**69.** The organic electroluminescence device according to any one of claims 65 to 68, wherein the group represented by the formula (1D) is a group represented by a formula (1D-1), (1D-2), or (1D-3) below,

[Formula 8]

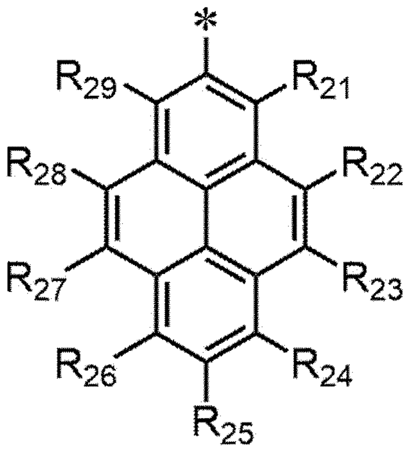

(1D-1)

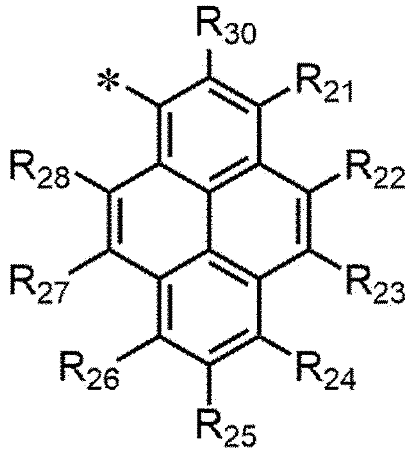

(1D-2)

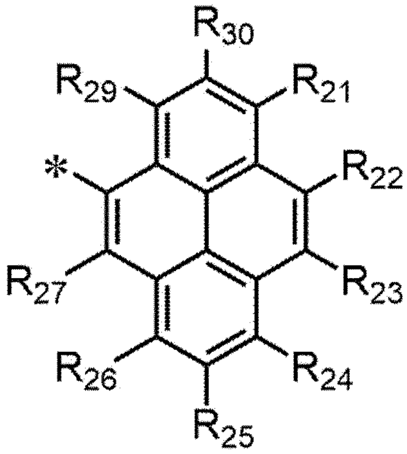

(1D-3)

where, in the formula (1D-1):

at least one combination of adjacent two or more of $R_{21}$ to $R_{29}$ are mutually bonded to form a substituted or unsubstituted monocyclic ring, mutually bonded to form a substituted or unsubstituted fused ring, or not mutually bonded;

$R_{21}$ to $R_{29}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by - S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and

* represents a bonding position to the benz[a]anthracene ring in the formula (1C); and

in the formula (1D-2):

at least one combination of adjacent two or more of $R_{21}$ to $R_{28}$, and $R_{30}$ are mutually bonded to form a substituted or unsubstituted monocyclic ring, mutually bonded to form a substituted or unsubstituted fused ring, or not mutually bonded;

$R_{21}$ to $R_{28}$ and $R_{30}$ forming neither the substituted or unsubstituted monocyclic ring nor the substituted or unsubstituted fused ring each independently represent the same as $R_{21}$ to $R_{29}$ in the formula (1D-1); and

* represents a bonding position to the benz[a]anthracene ring in the formula (1C),

in the formula (1D-3):

at least one combination of adjacent two or more of $R_{21}$ to $R_{27}$, $R_{29}$, and $R_{30}$ are mutually bonded to form a substituted or unsubstituted monocyclic ring, mutually bonded to form a substituted or unsubstituted fused ring, or not mutually bonded;
$R_{21}$ to $R_{27}$, $R_{29}$, and $R_{30}$ forming neither the substituted or unsubstituted monocyclic ring nor the substituted or unsubstituted fused ring each independently represent the same as $R_{21}$ to $R_{29}$ in the formula (1D-1); and
* represents a bonding position to the benz[a]anthracene ring in the formula (1C).

**70.** The organic electroluminescence device according to claim 69, wherein at least one of $R_{21}$ to $R_{29}$ in the formula (1D-1), at least one of $R_{21}$ to $R_{28}$, or $R_{30}$ in the formula (1D-2), or at least one of $R_{21}$ to $R_{27}$, $R_{29}$, or $R_{30}$ in the formula (1D-3) forming neither the substituted or unsubstituted monocyclic ring nor the substituted or unsubstituted fused ring in the group represented by the formula (1D-1), (1D-2), or (1D-3) is other than a hydrogen atom.

**71.** The organic electroluminescence device according to claim 69 or 70, wherein at least one of $R_{21}$ to $R_{29}$ in the formula (1D-1), at least one of $R_{21}$ to $R_{28}$, or $R_{30}$ in the formula (1D-2), or at least one of $R_{21}$ to $R_{27}$, $R_{29}$, or $R_{30}$ in the formula (1D-3) forming neither the substituted or unsubstituted monocyclic ring nor the substituted or unsubstituted fused ring in the group represented by the formula (1D-1), (1D-2), or (1D-3) is a substituted or unsubstituted aryl group having 6 to 50 ring carbon atoms.

**72.** The organic electroluminescence device according to claim 71, wherein at least one of $R_{21}$ to $R_{29}$ in the formula (1D-1), at least one of $R_{21}$ to $R_{28}$, or $R_{30}$ in the formula (1D-2), or at least one of $R_{21}$ to $R_{27}$, $R_{29}$, or $R_{30}$ in the formula (1D-3) forming neither the substituted or unsubstituted monocyclic ring nor the substituted or unsubstituted fused ring in the group represented by the formula (1D-1), (1D-2), or (1D-3) is a substituted or unsubstituted phenyl group.

**73.** The organic electroluminescence device according to any one of claims 69 to 72, wherein the group represented by the formula (1D) is a group represented by the formula (1D-1).

**74.** The organic electroluminescence device according to any one of claims 65 to 73, wherein one of $R_5$, $R_6$, $R_7$, $R_8$, $R_{11}$, and $R_{12}$ is a group represented by the formula (1D).

**75.** The organic electroluminescence device according to claim 74, wherein one of $R_6$, $R_7$, $R_{11}$, and $R_{12}$ is a group represented by the formula (1D).

**76.** The organic electroluminescence device according to claim 75, wherein $R_{11}$ is a group represented by the formula (1D).

**77.** The organic electroluminescence device according to claim 75, wherein $R_{12}$ is a group represented by the formula (1D).

**78.** The organic electroluminescence device according to claim 75, wherein

$R_{11}$ is a group represented by the formula (1D) and $R_{12}$ is an unsubstituted phenyl group, or
$R_{12}$ is a group represented by the formula (1D) and $R_{11}$ is an unsubstituted phenyl group.

**79.** The organic electroluminescence device according to claim 78, wherein $R_{11}$ is a group represented by the formula (1D) and $R_{12}$ is an unsubstituted phenyl group.

**80.** The organic electroluminescence device according to claim 78, wherein $R_{12}$ is a group represented by the formula (1D) and $R_{11}$ is an unsubstituted phenyl group.

**81.** The organic electroluminescence device according to any one of claims 65 to 81, wherein $R_{12}$ is a hydrogen atom.

**82.** The organic electroluminescence device according to any one of claims 65 to 81, comprising at least one deuterium atom in a molecule.

**83.** The organic electroluminescence device according to any one of claims 65 to 82, wherein a triplet energy of the

first compound $T_1(H1)$ and a triplet energy of the second compound $T_1(H2)$ satisfy a relationship of a numerical formula (Numerical Formula 1) below,

$$T_1(H1) > T_1(H2) \ldots \text{(Numerical Formula 1)}.$$

**84.** The organic electroluminescence device according to any one of claims 65 to 83, wherein the first emitting layer is in direct contact with the second emitting layer.

**85.** The organic electroluminescence device according to any one of claims 65 to 84, wherein the first emitting layer is disposed between the anode and the second emitting layer.

**86.** The organic electroluminescence device according to any one of claims 65 to 85, wherein

the first emitting layer comprises a first luminescent compound,
the second emitting layer comprises a second luminescent compound, and
the first luminescent compound and the second luminescent compound are each independently a compound that emits light having a maximum peak wavelength of 500 nm or less.

**87.** The organic electroluminescence device according to any one of claims 65 to 86, further comprising a hole transporting layer between the anode and the emitting region.

**88.** The organic electroluminescence device according to any one of claims 65 to 87, further comprising an electron transporting layer between the cathode and the emitting region.

**89.** An electronic device comprising the organic electroluminescence device according to any one of claims 55 to 88.

FIG.1

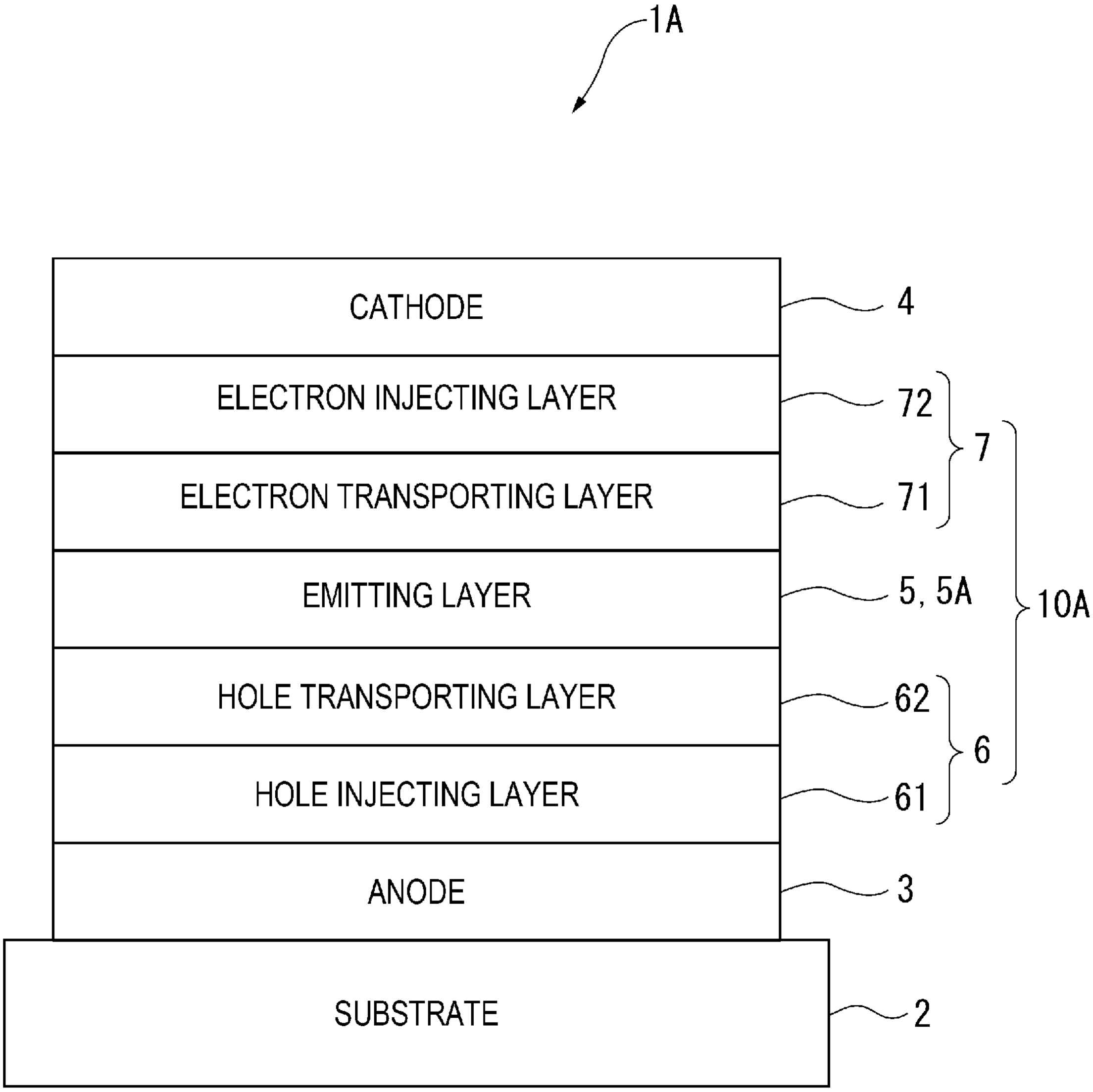
1A
CATHODE
4
ELECTRON INJECTING LAYER
72
7
ELECTRON TRANSPORTING LAYER
71
EMITTING LAYER
5, 5A
10A
HOLE TRANSPORTING LAYER
62
6
HOLE INJECTING LAYER
61
ANODE
3
SUBSTRATE
2

FIG.2

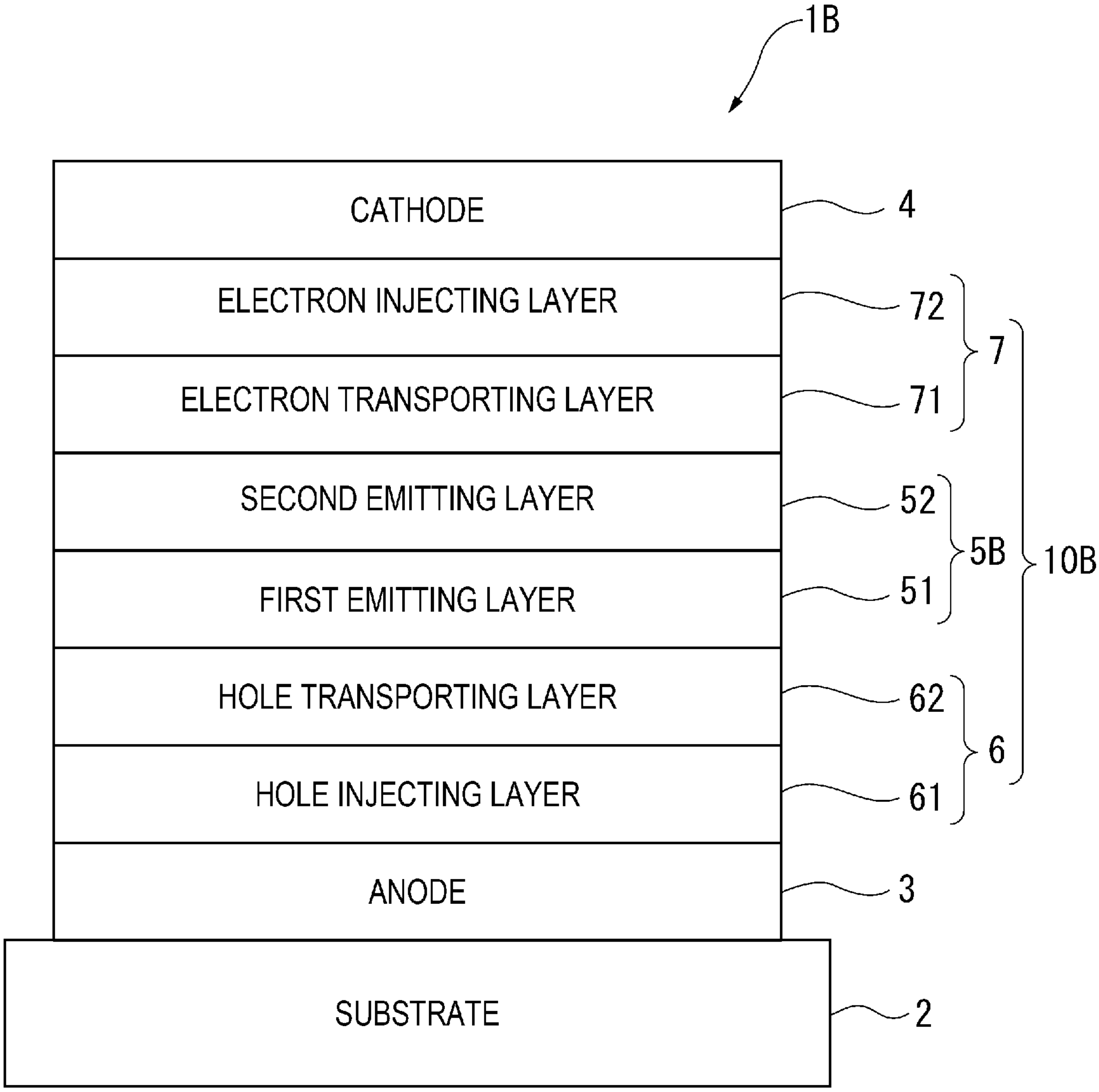
1B
CATHODE
4
ELECTRON INJECTING LAYER
72
ELECTRON TRANSPORTING LAYER
71
7
SECOND EMITTING LAYER
52
FIRST EMITTING LAYER
51
5B
10B
HOLE TRANSPORTING LAYER
62
HOLE INJECTING LAYER
61
6
ANODE
3
SUBSTRATE
2

**INTERNATIONAL SEARCH REPORT**

International application No.
**PCT/JP2022/048063**

**A. CLASSIFICATION OF SUBJECT MATTER**

***C07C 15/38***(2006.01)i; ***C09K 11/06***(2006.01)i; ***C07D 307/77***(2006.01)i; ***H05B 33/12***(2006.01)i; ***H10K 50/00***(2023.01)i; ***H10K 50/16***(2023.01)i; ***H10K 50/15***(2023.01)i
FI: C07C15/38 CSP; H05B33/14 B; H05B33/12 C; C09K11/06 690; H05B33/22 C; H05B33/22 A; C07D307/77 CSP

According to International Patent Classification (IPC) or to both national classification and IPC

**B. FIELDS SEARCHED**

Minimum documentation searched (classification system followed by classification symbols)

C07C15/38; C09K11/06; C07D307/77; H05B33/12; H10K50/00; H10K50/16; H10K50/15

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2009/081774 A1 (IDEMITSU KOSAN CO., LTD.) 02 July 2009 (2009-07-02) claims | 1-89 |
| A | WO 2021/132535 A1 (IDEMITSU KOSAN CO., LTD.) 01 July 2021 (2021-07-01) claims | 1-89 |
| A | JP 2009-16693 A (IDEMITSU KOSAN CO., LTD.) 22 January 2009 (2009-01-22) claims | 1-89 |
| A | KR 10-2013-0101449 A (CS ELSOLAR CO., LTD.) 13 September 2013 (2013-09-13) claims | 1-89 |
| A | CN 102911656 A (JILIN OPTICAL AND ELECTRONIC MATERIALS CO., LTD.) 06 February 2013 (2013-02-06) claims | 1-89 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 January 2023** | **31 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/048063**

| Patent document cited in search report | Publication date (day/month/year) | Patent family member(s) | Publication date (day/month/year) |
|---|---|---|---|
| WO 2009/081774 A1 | 02 July 2009 | JP H9-81774 A1<br>claims<br>US 2010/0289013 A1<br>claims | |
| WO 2021/132535 A1 | 01 July 2021 | CN 114514625 A<br>claims<br>KR 10-2022-0119596 A<br>claims | |
| JP 2009-16693 A | 22 January 2009 | (Family: none) | |
| KR 10-2013-0101449 A | 13 September 2013 | (Family: none) | |
| CN 102911656 A | 06 February 2013 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021132535 A
- WO 2009081774 A
- WO 2014104144 A
- WO 2010134350 A


### Non-patent literature cited in the description


- **S. M. BACHILO et al.** *J. Phys. Chem. A,* 2000, vol. 104, 7711